# EUROPEAN PATENT APPLICATION

(11) **EP 1 829 968 A1**
(43) Date of publication of application: **05.09.2007**
(21) Application number: 05820283.9
(22) Date of filing: 20.12.2005
(51) Int. Cl.: C12N 15/09, C12N 9/00, C12P 21/02, C12P 13/00

(54) **MUTANT PROTEIN HAVING PEPTIDE-PRODUCTION ACTIVITY**

(30) Priority: 20.12.2004 JP 2004368503; 27.12.2004 US 638370 P
(71) Applicant: Ajinomoto Co., Inc., Tokyo 104-8315 (JP)
(72) Inventor: ABE, Isao, c/o Ajinomoto Co., Inc., Kawasaki-shi, Kanagawa 2108681 (JP); TAKESHITA, Rie, c/o Ajinomoto Co., Inc., Kawasaki-shi, Kanagawa 2108681 (JP); HARA, Seiichi, c/o Ajinomoto Co., Inc., Kawasaki-shi, Kanagawa 2108681 (JP); SUZUKI, Sonoko, c/o Ajinomoto Co., Inc., Kawasaki-shi, Kanagawa 2108681 (JP); YOKOZEKI, Kenzo, c/o Ajinomoto Co., Inc., Kawasaki-shi, Kanagawa 2108681 (JP); SUGIYAMA, Masakazu, c/o Ajinomoto Co., Inc., Kawasaki-shi, Kanagawa 2108681 (JP); SUZUKI, Shunichi, c/o Ajinomoto Co., Inc., Kawasaki-shi, Kanagawa 2108681 (JP); WATANABE, Kunihiko c/o Ajinomoto Co., Inc., Kawasaki-shi, Kanagawa 2108681 (JP); SHIMBA, Nobuhisa c/o Ajinomoto Co., Inc., Kawasaki-shi, Kanagawa 2108681 (JP); NAKAMURA, Takefumi c/o Ajinomoto Co., Inc., Kawasaki-shi, Kanagawa 2108681 (JP); TAGAMI, Uno c/o Ajinomoto Co., Inc., Kawasaki-shi, Kanagawa 2108681 (JP); KODAMA, Yuya c/o Ajinomoto Co., Inc., Kawasaki-shi, Kanagawa 2108681 (JP); ONOYE, Hiromi c/o Ajinomoto Co., Inc., Kawasaki-shi, Kanagawa 2108681 (JP); YUUJI, Reiko c/o Ajinomoto Co., Inc., Kawasaki-shi, Kanagawa 2108681 (JP); SUZUKI, Eiichiro c/o Ajinomoto Co., Inc., Kawasaki-shi, Kanagawa 2108681 (JP); KASHIWAGI, Tatsuki c/o Ajinomoto Co., Inc., Kawasaki-shi, Kanagawa 2108681 (JP); XU, Ningchun c/o Ajinomoto Co., Inc., Kawasaki-shi, Kanagawa 2108681 (JP); KAI, Yuko c/o Ajinomoto Co., Inc., Kawasaki-shi, Kanagawa 2108681 (JP)
(74) Representative: Strehl Schübel-Hopf & Partner
(86) International application number: PCT/JP2005/023400
(87) International publication number: WO 2006/075486

(57) **Abstract**

The present invention aims at providing an excellent peptide-synthesizing protein and a method for efficiently producing a peptide. The peptide is synthesized by reacting an amine component and a carboxy component in the presence of at least one of proteins shown in the following (I) and (II).
(I) The mutant protein having an amino acid sequence comprising one or more mutations from any of the mutations 1 to 68, and the mutations 239 to 290 and 324 to 377 in an amino acid sequence of SEQ ID NO:2.
(II) The mutant protein having an amino acid sequence comprising one or more mutations from any of the mutations
L1 to L335 and M1 to M642 in an amino acid sequence of SEQ ID NO:208

## Description

### Technical Field

The present invention relates to a mutant protein having a peptide-synthesizing activity, and more particularly relates to a mutant protein having an excellent peptide-synthesizing activity and a method for producing a peptide using this protein.

### Background Art

Peptides have been used in a variety of fields such as pharmaceuticals and foods. For example, L-alanyl-L-glutamine is widely used as a component for infusions and serum-free media taking advantage of its higher stability and water-solubility than that of L-glutamine.

Peptides have hitherto been produced by chemical synthesis methods. However, the chemical synthesis has not always been satisfactory in terms of simplicity and efficiency.
On the other hand, methods for producing the peptide using an enzyme have been developed (e.g., Patent documents 1 and 2). However, the conventional enzymological method for producing the peptide still had room for improvement such as slow synthesis rate and low yield of the peptide products. In such a context, it has been desired to develop a method for efficiently producing peptides on an industrial scale.

The present inventors have already been found an enzyme derived from *Sphingobacterium* as an enzyme having an excellent peptide-synthesizing activity (Patent documents 3 to 6).

[Patent document 1]
   EP 0278787 A1
[Patent document 2]
   EP 359399 A1
[Patent document 3]
   WO2004/011653
[Patent document 4]
   JP 2005-040037 A
[Patent document 5]
   JP 2005-058212 A
[Patent document 6]
   JP 2005-168405 A

### Disclosure of Invention

### Problem to be Solved by the Invention

It is an object of the present invention to provide a more excellent peptide-synthesizing protein and a method for efficiently producing the peptide.

### Means for Solving Problem

As a result of an extensive study, the present inventors have found that a protein having a more excellent peptide-synthesizing activity is obtainable by modifying a specific position in an amino acid sequence or a nucleotide sequence of a protein derived from a microorganism belonging to genus *Sphingobacterium* and having a peptide-synthesizing activity, and completed the present invention. That is, the present invention provides the following protein and method for producing a peptide using this protein.

[1] A mutant protein having an amino acid sequence comprising one or more mutations selected from any of the following mutations 1 to 68 in an amino acid sequence of SEQ ID NO:2.
mutation 1 F207V, mutation 2 Q441E, mutation 3 K83A,
mutation 4 A301V, mutation 5 V257I, mutation 6 A537G,
mutation 7 A324V, mutation 8 N607K, mutation 9 D313E,
mutation 10 Q229H, mutation 11 M208A, mutation 12 E551K,
mutation 13 F207H, mutation 14 T72A, mutation 15 A137S,
mutation 16 L439V, mutation 17 G226S, mutation 18 D619E,
mutation 19 Y339H, mutation 20 W327G, mutation 21 V184A,
mutation 22 V184C, mutation 23 V184G, mutation 24 V184I,
mutation 25 V184L, mutation 26 V184M, mutation 27 V184P,
mutation 28 V184S, mutation 29 V184T, mutation 30 Q441K,
mutation 31 N442K, mutation 32 D203N, mutation 33 D203S,
mutation 34 F207A, mutation 35 F207S, mutation 36 Q441N,
mutation 37 F207T, mutation 38 F207I, mutation 39 T210K,
mutation 40 W187A, mutation 41 S209A, mutation 42 F211A,
mutation 43 F211V, mutation 44 V257A, mutation 45 V257G,
mutation 46 V257H, mutation 47 V257M, mutation 48 V257N,
mutation 49 V257Q, mutation 50 V257S, mutation 51 V257T,
mutation 52 V257W, mutation 53 V257Y, mutation 54 K47G,
mutation 55 K47E, mutation 56 N442F, mutation 57 N607R,
mutation 58 P214T, mutation 59 Q202E, mutation 60 Y494F,
mutation 61 R117A, mutation 62 F207G, mutation 63 S209D,
mutation 64 S209G, mutation 65 Q441D, mutation 66 R445D,
mutation 67 R445F, mutation 68 N442D.
[2] The mutant protein according to [1] above wherein, in said amino acid sequence comprising one or more mutations selected from any of the mutations 1 to 68, said amino acid sequence further comprises at other than the mutated position(s) one or several amino acid mutations selected from the group consisting of substitutions, deletions, insertions, additions and inversions, said mutant protein having a peptide-synthesizing activity.
[3] The mutant protein according to [1] or [2] above comprising at least the mutation 2.
[4] The mutant protein according to any one of [1] to [3] above comprising at least the mutation 14.
[5] A mutant protein having an amino acid sequence comprising one or more mutations selected from any of the following mutations 239 to 290 and 324 to 377 in an amino acid sequence of SEQ ID NO:2:
   mutation 239 F207V/Q441E
   mutation 240 F207V/K83A
   mutation 241 F207V/E551K
   mutation 242 K83A/Q441E
   mutation 243 M208A/E551K
   mutation 244 V257I/Q441E
   mutation 245 V257I/A537G
   mutation 246 F207V/S209A
   mutation 247 K83A/S209A
   mutation 248 K83A/F207V/Q441E
   mutation 249 L439V/F207V/Q441E
   mutation 250 A537G/F207V/Q441E
   mutation 251 A301V/F207V/Q441E
   mutation 252 G226S/F207V/Q441E
   mutation 253 V257I/F207V/Q441E
   mutation 254 D619E/F207V/Q441E
   mutation 255 Y339H/F207V/Q441E
   mutation 256 N607K/F207V/Q441E
   mutation 257 A324V/F207V/Q441E
   mutation 258 Q229H/F207V/Q441E
   mutation 259 W327G/F207V/Q441E
   mutation 260 A301V/L439V/A537G/N607K
   mutation 261 K83A/Q229H/A301V/D313E/A324V/L439V/A537G/N607K
   mutation 262 Q229H/V257I/A301V/A324V/Q441E/A537G/N607K
   mutation 263 Q229H/A301V/A324V/Q441E/A537G/N607K
   mutation 264 Q229H/V257I/A301V/D313E/A324V/Q441E/A537G/N607K
   mutation 265 T72A/A137S/A301V/L439V/Q441E/A537G/N607K
   mutation 266 T72A/A137S/A301V/Q441E/A537G/N607K
   mutation 267 T72A/A137S/Q229H/A301V/A324V/L439V/A537G/N607K
   mutation 268 T72A/A137S/Q229H/A301V/A324V/L439V/Q441E/A537G/N607K
   mutation 269 T72A/ Q229H/V257I/A301V/D313E/A324V/L439V/Q441E/A537G/N607K
   mutation 270 T72A/ Q229H/V257I/A301V/D313E/A324V/Q441E/A537G/N607K
   mutation 271 T72A/A137S/Q229P/A301V/L439V/Q441E/A537G/N607K
   mutation 272 T72A/A137S/Q229L/A301V/L439V/Q441E/A537G/N607K
   mutation 273 T72A/A137S/Q229G/A301V/L439V/Q441E/A537G/N607K
   mutation 274 T72A/Q229I/V257I/A301V/D313E/A324V/L439V/Q441E/A537G/N607K
   mutation 275 T72A/A137S/I228G/Q229P/A301V/L439V/Q441E/A537G/N607K
   mutation 276 T72A/A137S/I228L/Q229P/A301V/L439V/Q441E/A537G/N607K
   mutation 277 T72A/A137S/I228D/Q229P/A301V/L439V/Q441E/A537G/N607K
   mutation 278 T72A/A137S/Q229P/I230D/A301V/L439V/Q441E/A537G/N607K
   mutation 279 T72A/A137S/Q229P/I230V/A301V/L439V/Q441E/A537G/N607K
   mutation 280 T72A/I228S/Q229H/V257I/A301V/D313E/A324V/L439V/Q441E/A537G/ N607K
   mutation 281 T72A/Q229H/S256C/V257I/A301V/D313E/A324V/L439V/Q441E/A537G/ N607K
   mutation 282 T72A/A137S/Q229P/V257I/A301V/A324V/L439V/Q441E/A537G/N607K
   mutation 283 T72A/A137S/Q229P/A301V/A324V/L439V/Q441E/A537G/N607K
   mutation 284 T72A/Q229P/V257I/A301G/D313E/A324V/Q441E/A537G/N607K
   mutation 285 T72A/Q229P/V257I/A301V/D313E/A324V/Q441E/A537G/N607K
   mutation 286 T72A/A137S/V184A/Q229P/V257I/A301V/A324V/L439V/Q441E/A537G/ N607K
   mutation 287 T72A/A137S/V184G/Q229P/V257I/A301V/A324V/L439V/Q441E/A537G/ N607K
   mutation 288 T72A/A137S/V184N/Q229P/V257I/A301V/A324V/L439V/Q441E/A537G/ N607K
   mutation 289 T72A/A137S/V184S/Q229P/V257I/A301V/A324V/L439V/Q441E/A537G/ N607K
   mutation 290 T72A/A137S/V184T/Q229P/V257I/A301V/A324V/L439V/Q441E/A537G/ N607K
   mutation 324 V184A/V257Y
   mutation 325 V184A/W187A
   mutation 326 V184A/N442D
   mutation 327 V184P/N442D
   mutation 328 V184A/N442D/L439V
   mutation 329 A301V/L439V/A537G/N607K/V184A
   mutation 330 A301V/L439V/A537G/N607K/V184P
   mutation 331 A301V/L439V/A537G/N607K/V257Y
   mutation 332 A301V/L439V/A537G/N607K/W187A
   mutation 333 A301V/L439V/A537G/N607K/F211A
   mutation 334 A301V/L439V/A537G/N607K/Q441E
   mutation 335 A301V/L439V/A537G/N607K/N442D
   mutation 336 A301V/L439V/A537G/N607K/V184A/F207V
   mutation 337 A301V/L439V/A537G/N607K/V184A/A182G
   mutation 338 T72A/A137S/Q229P/V257I/A301V/A324V/L439V/A537G/N607K/V184A/ N442D
   mutation 339 T72A/A137S/Q229P/V257I/A301V/A324V/L439V/A537G/N607K/V184A/ N442D/T185F
   mutation 340 T72A/A137S/Q229P/V257I/A301V/A324V/L439V/Q441E/A537G/N607K/ V184A/K83A
   mutation 341 T72A/A137S/Q229P/V257I/A301V/A324V/L439V/Q441E/A537G/N607K/ V184A/W187A
   mutation 342 T72A/A137S/Q229P/V257I/A301V/A324V/L439V/Q441E/A537G/N607K/ V184A/F211A
   mutation 343 T72A/A137S/Q229P/V257I/A301V/A324V/L439V/Q441E/A537G/N607K/ V184A/V178G
   mutation 344 T72A/A137S/Q229P/V257I/A301V/A324V/L439V/Q441E/A537G/N607K/ V184A/T185A
   mutation 345 T72A/A137S/Q229P/V257I/A301V/A324V/L439V/Q441E/A537G/N607K/ V184A/A182G
   mutation 346 T72A/A137S/Q229P/V257I/A301V/A324V/L439V/Q441E/A537G/N607K/ V184A/K314R
   mutation 347 T72A/A137S/Q229P/V257I/A301V/A324V/L439V/Q441E/A537G/N607K/ V184A/A515V
   mutation 348 T72A/A137S/Q229P/V257I/A301V/A324V/L439V/Q441E/A537G/N607K/ V184A/L66F
   mutation 349 T72A/A137S/Q229P/V257I/A301V/A324V/L439V/Q441E/A537G/N607K/ V184A/S315R
   mutation 350 T72A/A137S/Q229P/V257I/A301V/A324V/L439V/Q441E/A537G/N607K/ V184A/K484I
   mutation 351 T72A/A137S/Q229P/V257I/A301V/A324V/L439V/0441E/A537G/N607K/ V184A/V213A
   mutation 352 T72A/A137S/Q229P/V257I/A301V/A324V/L439V/Q441E/A537G/N607K/ V184A/A245S
   mutation 353 T72A/A137S/Q229P/V257I/A301V/A324V/L439V/Q441E/A537G/N607K/ V184A/P214H
   mutation 354 T72A/A137S/Q229P/V2S7I /A301V/A324V/L439V/Q441E /A537G/N607K/V184A/L263M
   mutation 355 T72A/A137S/Q229P/V257I /A301V/A324V/L439V/Q441E/A537G/N607K/V184A/P183A
   mutation 356 T72A/A137S/Q229P/V257I/A301V/A324V/L439V/Q441E/A537G/N607K/ V184A/T185K
   mutation 357 T72A/A137S/Q229P/V257I/A301V/A324V/L439V/Q441E/A537G/N607K/ V184A/T185D
   mutation 358 T72A/A137S/Q229P/V257I/A301V/A324V/L439V/Q441E/A537G/N607K/ V184A/T185C
   mutation 359 T72A/A137S/Q229P/V257I/A301V/A324V/L439V/Q441E/A537G/N607K/ V184A/T185S
   mutation 360 T72A/A137S/Q229P/V257I/A301V/A324V/L439V/Q441E/A537G/N607K/ V184A/T185F
   mutation 361 T72A/A137S/Q229P/V257I/A301V/A324V/L439V/Q441E/A537G/N607K/ V184A/T185P
   mutation 362 T72A/A137S/Q229P/V257I/A301V/A324V/L439V/Q441E/A537G/N607K/ V184A/T185N
   mutation 363 T72A/A137S/Q229P/V257I/A301V/A324V/L439V/Q441E/A537G/N607K/ V184A/P183A/A182G
   mutation 364 T72A/A137S/Q229P/V257I/A301V/A324V/L439V/Q441E/A537G/N607K/ V184A/P183A/A182S
   mutation 365 T72A/A137S/Q229P/V257I /A301V/A324V/L439V/Q441E/A537G/N607K/V184A/T185F/N442D
   mutation 366 T72A/A137S/Q229P/V257I /A301V/A324V/L439V/Q441E/A537G/N607K/V184A/L66F/E80K/I157L/ A182G/P214H/L263M
   mutation 367 T72A/A137S/Q229P/V257I/A301V/A324V/L439V/Q441E/A537G/N607K/ V184A/L66F/E80K/I157L/A182G/P214H/L263M/Y328F
   mutation 368 T72A/A137S/Q229P/V257I /A301V/A324V/L439V/Q441E/A537G/N607K/V184A/L66F/Y81A/I157L/ A182G/P214H/L263M/Y328F
   mutation 369 T72A/A137S/Q229P/V257I/A301V/A324V/L439V/Q441E/A537G/N607K/ V184A/L66F/E80K/I157L/A182G/T210L/L263M/Y328F
   mutation 370 A301V/L439V/A537G/N607K/Q441K
   mutation 371 T72A/A137S/Q229P/V257I/A301V/A324V/L439V/Q441E/A537G/N607K/ V184A/I157L
   mutation 372 T72A/A137S/Q229P/V257I/A301V/A324V/L439V/Q441E/A537G/N607K/ V184A/G161A
   mutation 373 T72A/A137S/Q229P/V257I/A301V/A324V/L439V/Q441E/A537G/N607K/ V184A/Y328F
   mutation 374 F207V/G226S
   mutation 375 F207V/W327G
   mutation 376 F207V/Y339H
   mutation 377 F207V/D619E.
[6] The mutant protein according to [5] above wherein, in said amino acid sequence comprising one or more mutations selected from any of the mutations 239 to 290 and 324 to 377, said amino acid sequence further comprises at other than the mutated position(s) one or more amino acid mutations selected from the group consisting of substitutions, deletions, insertions, additions and inversions, said mutant protein having a peptide-synthesizing activity.
[7] The mutant protein according to [5] or [6] above comprising at least the mutation 260.
[8] The mutant protein according to any one of [5] to [7] above comprising at least the mutation 286.
[9] A polynucleotide encoding the amino acid sequence of the mutant protein according to any one of [1] to [8] above.
[10] A recombinant polynucleotide comprising the polynucleotide according to [9] above.
[11] A transformed microorganism comprising the recombinant polynucleotide according to [10] above.
[12] A method for producing a mutant protein comprising culturing the transformed microorganism according to [11] above in a medium, to accumulate the mutant protein in the medium and/or the transformed microorganism.
[13] A method for producing a peptide comprising performing a peptide-synthesizing reaction in the presence of the mutant protein according to any one of [1] to [8] above.
[14] A method for producing a peptide comprising culturing the transformed microorganism according to [11] above in a medium to accumulate the mutant protein in the medium and/or the transformed microorganism for performing a peptide-synthesizing reaction.
[15] A method for producing α-L-aspartyl-L-phenylalanine-β-ester comprising reacting L-aspartic acid-α,β-diester and L-phenylalanine in the presence of the mutant protein according to any one of [1] to [8] above.
[16] A method for producing α-L-aspartyl-L-phenylalanine-β-ester comprising culturing the transformed microorganism according to [11] above in a medium to accumulate the mutant protein in the medium and/or the transformed microorganism for performing a reaction of L-aspartic acid-a,β-diester and L-phenylalanine.
[17] A method for designing and producing a mutant protein having a peptide-synthesizing activity comprising:
   analyzing a protein having an amino acid sequence of SEQ ID NO:208 by X-ray crystal structure analysis to obtain a tertiary structure thereof;
   predicting a substrate binding site of the protein based on said tertiary structure; and
   substituting, inserting or deleting an amino acid residue located at said substrate binding site.
[18] A mutant protein having an amino acid sequence comprising one or more amino acid substitutions, insertions or deletions at positions 67 to 70, 72 to 88, 100, 102, 103, 106, 107, 113 to 117, 130, 155 to 163, 165, 166, 180 to 188, 190 to 195, 200 to 235, 259, 273, 276, 278, 292 to 294, 296, 298, 299, 300 to 304, 325 to 328, 330 to 340, and 437 to 447 in an amino acid sequence in a tertiary structure of a protein having an amino acid sequence of SEQ ID NO:208, and having a peptide-synthesizing activity.
[19] A mutant protein of a protein having a peptide-synthesizing activity wherein:
   three dimensional structures of the mutant protein and a protein having an amino acid sequence of SEQ ID NO:209 are similar as a result of determination by a threading method;
   in alignment obtained upon the determination, at least one or more amino acid residues are substituted, inserted or deleted at positions corresponding to positions 67 to 70, 72 to 88, 100, 102, 103, 106, 107, 113 to 117, 130, 155 to 163, 165, 166, 180 to 188, 190 to 195, 200 to 235, 259, 273, 276, 278, 292 to 294, 296, 298, 299, 300 to 304, 325 to 328, 330 to 340 and 437 to 447 in the amino acid sequence of SEQ ID NO:209; and
   said mutant protein has the peptide-synthesizing activity.
[20] A mutant protein of a protein having a peptide-synthesizing activity wherein:
   when an alignment of primary sequences of the mutant protein and a protein having an amino acid sequence of SEQ ID NO:209 or an alignment of three dimensional structures of the mutant protein and the protein having the amino acid sequence of SEQ ID NO:209 is performed, homology of the primary sequences is 25% or more, and at least one or more amino acid residues are substituted, inserted or deleted at positions corresponding to positions 67 to 70, 72 to 88, 100, 102, 103, 106, 107, 113 to 117, 130, 155 to 163, 165, 166, 180 to 188, 190 to 195, 200 to 235, 259, 273, 276, 278, 292 to 294, 296, 298, 299, 300 to 304, 325 to 328, 330 to 340 and 437 to 447 in the amino acid sequence of SEQ ID NO:209; and
   said mutant protein has the peptide-synthesizing activity.
[21] A mutant protein having one or more changes in a tertiary structure selected from the following (a) to (i) in the tertiary structure of a protein having an amino acid sequence of SEQ ID NO:208, said mutant protein having a peptide-synthesizing activity:
   (a) at least one or more amino acid residue substitutions, insertions or deletions at any of positions 79 to 82 in the amino acid sequence of SEQ ID NO:208;
   (b) at least one or more amino acid residue substitutions, insertions or deletions at any of positions 84, 88, 89 and 92 in the amino acid sequence of SEQ ID NO:208;
   (c) at least one or more amino acid residue substitutions, insertions or deletions at any of positions 72, 75 and 77 in the amino acid sequence of SEQ ID NO:208;
   (d) at least one or more amino acid residue substitutions, insertions or deletions at any of positions 159, 161, 162, 184, 187 and 276 in the amino acid sequence of SEQ ID NO:208;
   (e) at least one or more amino acid residue substitutions, insertions or deletions at any of positions 70, 106, 113, 115, 193, 207, 209 to 212, 216 and 259 in the amino acid sequence of SEQ ID NO:208;
   (f) at least one or more amino acid residue substitutions, insertions or deletions at any of positions 200, 202 to 205, 207 and 228 in the amino acid sequence of SEQ ID NO:208;
   (g) at least one or more amino acid residue substitutions, insertions or deletions at any of positions 233, 234 and 439 in the amino acid sequence of SEQ ID NO:208;
   (h) at least one or more amino acid residue substitutions, insertions or deletions at any of positions 328, 339, 340, 445 and 446 in the amino acid sequence of SEQ ID NO:208; and
   (i) at least one or more amino acid residue substitutions, insertions or deletions at any of positions 87, 155, 157 and 160 in the amino acid sequence of SEQ ID NO:208.
[22] A mutant protein of a protein having a peptide-synthesizing activity wherein:
   three dimensional structures of the mutant protein and a protein having an amino acid sequence of SEQ ID NO:209 are similar as a result of determination by a threading method, and in alignment obtained upon the determination, one or more changes selected from the following (a') to (i') are present; and
   the mutant protein has a peptide-synthesizing activity:
      (a') at least one or more amino acid residue substitutions, insertions or deletions in the tertiary structure corresponding to any of positions 79 to 82 in the amino acid sequence of SEQ ID NO:209;
      (b') at least one or more amino acid residue substitutions, insertions or deletions in the tertiary structure corresponding to any of positions 84, 88, 89 and 92 in the amino acid sequence of SEQ ID NO:209;
      (c') at least one or more amino acid residue substitutions, insertions or deletions in the tertiary structure corresponding to any of positions 72, 75 and 77 in the amino acid sequence of SEQ ID NO:209;
      (d') at least one or more amino acid residue substitutions, insertions or deletions in the tertiary structure corresponding to any of positions 159, 161, 162, 184, 187 and 276 in the amino acid sequence of SEQ ID NO:209;
      (e') at least one or more amino acid residue substitutions, insertions or deletions in the tertiary structure corresponding to any of positions 70, 106, 113, 115, 193, 207, 209 to 212, 216 and 259 in the amino acid sequence of SEQ ID NO:209;
      (f') at least one or more amino acid residue substitutions, insertions or deletions in the tertiary structure corresponding to any of positions 200, 202 to 205, 207 and 228 in the amino acid sequence of SEQ ID NO:209;
      (g') at least one or more amino acid residue substitutions, insertions or deletions in the tertiary structure corresponding to any of positions 233, 234 and 439 in the amino acid sequence of SEQ ID NO:209;
      (h') at least one or more amino acid residue substitutions, insertions or deletions in the tertiary structure corresponding to any of positions 328, 339, 340, 445 and 446 in the amino acid sequence of SEQ ID NO:209; and
      (i') at least one or more amino acid residue substitutions, insertions or deletions in the tertiary structure corresponding to any of positions 87, 155, 157 and 160 in the amino acid sequence of SEQ ID NO:209.
[23] A mutant protein of a protein having a peptide-synthesizing activity wherein:
   when an alignment of primary sequences of the mutant protein and a protein having an amino acid sequence of SEQ ID NO:209 or an alignment of three dimensional structures of the mutant protein and the protein having the amino acid sequence of SEQ ID NO:209 is performed, homology of the primary sequences is 25% or more, and one or more changes selected from the following (a") to (i'') are present; and
   said mutant protein has the peptide-synthesizing activity:
      (a'') at least one or more amino acid residue substitutions, insertions or deletions in the tertiary structure corresponding to any of positions 79 to 82 in the amino acid sequence of SEQ ID NO:209;
      (b'') at least one or more amino acid residue substitutions, insertions or deletions in the tertiary structure corresponding to any of positions 84, 88, 89 and 92 in the amino acid sequence of SEQ ID NO:209;
      (c'') at least one or more amino acid residue substitutions, insertions or deletions in the tertiary structure corresponding to any of positions 72, 75 and 77 in the amino acid sequence of SEQ ID NO:209;
      (d'') at least one or more amino acid residue substitutions, insertions or deletions in the tertiary structure corresponding to any of positions 159, 161, 162, 184, 187 and 276 in the amino acid sequence of SEQ ID NO:209;
      (e'') at least one or more amino acid residue substitutions, insertions or deletions in the tertiary structure corresponding to any of positions 70, 106, 113, 115, 193, 207, 209 to 212, 216 and 259 in the amino acid sequence of SEQ ID NO:209;
      (f'') at least one or more amino acid residue substitutions, insertions or deletions in the tertiary structure corresponding to any of positions 200, 202 to 205, 207 and 228 in the amino acid sequence of SEQ ID NO:209;
      (g'') at least one or more amino acid residue substitutions, insertions or deletions in the tertiary structure corresponding to any of positions 233, 234 and 439 in the amino acid sequence of SEQ ID NO:209;
      (h'') at least one or more amino acid residue substitutions, insertions or deletions in the tertiary structure corresponding to any of positions 328, 339, 340, 445 and 446 in the amino acid sequence of SEQ ID NO:209; and
      (i'') at least one or more amino acid residue substitutions, insertions or deletions in the tertiary structure corresponding to any of positions 87, 155, 157 and 160 in the amino acid sequence of SEQ ID NO:209.
[24] A mutant protein having at least one or more amino acid residue substitutions, insertions or deletions at positions 67, 69, 70, 72 to 85, 103, 106, 107, 113 to 116, 165, 182, 183, 185, 187, 188, 190, 200, 202, 204 to 206, 209 to 211, 213 to 235, 301, 328, 338 to 340, 440 to 442 and 446 in a tertiary structure of a protein having an amino acid sequence of SEQ ID NO:208, said mutant protein having a peptide-synthesizing activity.
[25] A mutant protein having at least one or more amino acid residue substitutions, insertions or deletions at positions 67, 69, 70, 72 to 84, 106, 107, 114, 116, 183, 185, 187, 188, 202, 204 to 206, 209, 211, 213 to 233, 235, 328, 338 to 442 and 446 in a tertiary structure of a protein having an amino acid sequence of SEQ ID NO:208, said mutant protein having a peptide-synthesizing activity.
[26] A mutant protein having at least one or more amino acid residue substitutions, insertions or deletions at positions 67, 70, 72 to 75, 77 to 79, 81 to 84, 114, 116, 185, 188, 202, 204, 206, 209, 211, 213 to 215, 218 to 224, 226 to 233, 235, 328, 338 to 441 and 446 in a tertiary structure of a protein having an amino acid sequence of SEQ ID NO:208, said mutant protein having a peptide-synthesizing activity.
[27] A mutant protein having an amino acid sequence comprising one or more mutations selected from any of the following mutations L1 to L335 in an amino acid sequence of SEQ ID NO:208:
   mutation L1 N67K
   mutation L2 N67L
   mutation L3 N67S
   mutation L4 T69I
   mutation L5 T69M
   mutation L6 T69Q
   mutation L7 T69R
   mutation L8 T69V
   mutation L9 P70G
   mutation L10 P70N
   mutation L11 P70S
   mutation L12 P70T
   mutation L13 P70V
   mutation L14 A72C
   mutation L15 A72D
   mutation L16 A72E
   mutation L17 A72I
   mutation L18 A72L
   mutation L19 A72M
   mutation L20 A72N
   mutation L21 A72Q
   mutation L22 A72S
   mutation L23 A72V
   mutation L24 V73A
   mutation L25 V73I
   mutation L26 V73L
   mutation L27 V73M
   mutation L28 V73N
   mutation L29 V73S
   mutation L30 V73T
   mutation L31 S74A
   mutation L32 S74F
   mutation L33 S74K
   mutation L34 S74N
   mutation L35 S74T
   mutation L36 S74V
   mutation L37 P75A
   mutation L38 P75D
   mutation L39 P75L
   mutation L40 P75S
   mutation L41 Y76F
   mutation L42 Y76H
   mutation L43 Y76I
   mutation L44 Y76V
   mutation L45 Y76W
   mutation L46 G77A
   mutation L47 G77F
   mutation L48 G77K
   mutation L49 G77M
   mutation L50 G77N
   mutation L51 G77P
   mutation L52 G77S
   mutation L53 G77T
   mutation L54 Q78F
   mutation L55 Q78L
   mutation L56 N79D
   mutation L57 N79L
   mutation L58 N79R
   mutation L59 N79S
   mutation L60 E80D
   mutation L61 E80F
   mutation L62 E80L
   mutation L63 E80P
   mutation L64 E80S
   mutation L65 Y81A
   mutation L66 Y81C
   mutation L67 Y81D
   mutation L68 Y81E
   mutation L69 Y81F
   mutation L70 Y81H
   mutation L71 Y81K
   mutation L72 Y81L
   mutation L73 Y81N
   mutation L74 Y81S
   mutation L75 Y81T
   mutation L76 Y81W
   mutation L77 K82D
   mutation L78 K82L
   mutation L79 K82P
   mutation L80 K82S
   mutation L81 K83D
   mutation L82 K83F
   mutation L83 K83L
   mutation L84 K83P
   mutation L85 K83S
   mutation L86 K83V
   mutation L87 S84D
   mutation L88 S84F
   mutation L89 S84K
   mutation L90 S84L
   mutation L91 S84N
   mutation L92 S84Q
   mutation L93 L85F
   mutation L94 L85I
   mutation L95 L85P
   mutation L96 L85V
   mutation L97 N87E
   mutation L98 N87Q
   mutation L99 F88E
   mutation L100 V103I
   mutation L101 V103L
   mutation L102 K106A
   mutation L103 K106F
   mutation L104 K106L
   mutation L105 K106Q
   mutation L106 K106S
   mutation L107 W107A
   mutation L108 W107Y
   mutation L109 F113A
   mutation L110 F113W
   mutation L111 F113Y
   mutation L112 E114A
   mutation L113 E114D
   mutation L114 D115E
   mutation L115 D115Q
   mutation L116 D115S
   mutation L117 I116F
   mutation L118 I116K
   mutation L119 I116L
   mutation L120 I116M
   mutation L121 I116N
   mutation L122 I116T
   mutation L123 I116V
   mutation L124 I157K
   mutation L125 I157L
   mutation L126 Y159G
   mutation L127 Y159N
   mutation L128 Y159S
   mutation L129 P160G
   mutation L130 G161A
   mutation L131 F162L
   mutation L132 F162Y
   mutation L133 Y163I
   mutation L134 T165V
   mutation L135 Q181F
   mutation L136 A182G
   mutation L137 A182S
   mutation L138 P183A
   mutation L139 P183G
   mutation L140 P183S
   mutation L141 T185A
   mutation L142 T185G
   mutation L143 T185V
   mutation L144 W187A
   mutation L145 W187F
   mutation L146 W187H
   mutation L147 W187Y
   mutation L148 Y188F
   mutation L149 Y188L
   mutation L150 Y188W
   mutation L151 G190A
   mutation L152 G190D
   mutation L153 F193W
   mutation L154 H194D
   mutation L155 F200A
   mutation L156 F200L
   mutation L157 F200S
   mutation L158 F200V
   mutation L159 L201Q
   mutation L160 L201S
   mutation L161 Q202A
   mutation L162 Q202D
   mutation L163 Q202F
   mutation L164 Q202S
   mutation L165 Q202T
   mutation L166 Q202V
   mutation L167 D203E
   mutation L168 A204G
   mutation L169 A204L
   mutation L170 A204S
   mutation L171 A204T
   mutation L172 A204V
   mutation L173 F205L
   mutation L174 F205Q
   mutation L175 F205V
   mutation L176 F205W
   mutation L177 T206F
   mutation L178 T206K
   mutation L179 T206L
   mutation L180 F207I
   mutation L181 F207W
   mutation L182 F207Y
   mutation L183 M208A
   mutation L184 M208L
   mutation L185 S209F
   mutation L186 S209K
   mutation L187 S209L
   mutation L188 S209N
   mutation L189 S209V
   mutation L190 T210A
   mutation L191 T210L
   mutation L192 T210Q
   mutation L193 T210V
   mutation L194 F211A
   mutation L195 F211I
   mutation L196 F211L
   mutation L197 F211M
   mutation L198 F211V
   mutation L199 F211W
   mutation L200 F211Y
   mutation L201 G212A
   mutation L202 V213D
   mutation L203 V213F
   mutation L204 V213K
   mutation L205 V213S
   mutation L206 P214D
   mutation L207 P214F
   mutation L208 P214K
   mutation L209 P214S
   mutation L210 R215A
   mutation L211 R215I
   mutation L212 R215K
   mutation L213 R215Q
   mutation L214 R215S
   mutation L215 R215T
   mutation L216 R215Y
   mutation L217 P216D
   mutation L218 P216K
   mutation L219 K217D
   mutation L220 P218F
   mutation L221 P218L
   mutation L222 P218Q
   mutation L223 P218S
   mutation L224 I219D
   mutation L225 I219F
   mutation L226 I219K
   mutation L227 T220A
   mutation L228 T220D
   mutation L229 T220F
   mutation L230 T220K
   mutation L231 T220L
   mutation L232 T220S
   mutation L233 P221A
   mutation L234 P221D
   mutation L235 P221F
   mutation L236 P221K
   mutation L237 P221L
   mutation L238 P221S
   mutation L239 D222A
   mutation L240 D222F
   mutation L241 D222L
   mutation L242 D222R
   mutation L243 Q223F
   mutation L244 Q223K
   mutation L245 Q223L
   mutation L246 Q223S
   mutation L247 F224A
   mutation L248 F224D
   mutation L249 F224G
   mutation L250 F224K
   mutation L251 F224L
   mutation L252 K225D
   mutation L253 K225G
   mutation L254 K225S
   mutation L255 G226A
   mutation L256 G226F
   mutation L257 G226L
   mutation L258 G226N
   mutation L259 G226S
   mutation L260 K227D
   mutation L261 K227F
   mutation L262 K227S
   mutation L263 I228A
   mutation L264 I228F
   mutation L265 I228K
   mutation L266 I228S
   mutation L267 P229A
   mutation L268 P229D
   mutation L269 P229K
   mutation L270 P229L
   mutation L271 P229S
   mutation L272 I230A
   mutation L273 I230F
   mutation L274 I230K
   mutation L275 I230S
   mutation L276 K231F
   mutation L277 K231L
   mutation L278 K231S
   mutation L279 E232D
   mutation L280 E232F
   mutation L281 E232G
   mutation L282 E232L
   mutation L283 E232S
   mutation L284 A233D
   mutation L285 A233F
   mutation L286 A233H
   mutation L287 A233K
   mutation L288 A233L
   mutation L289 A233N
   mutation L290 A233S
   mutation L291 D234L
   mutation L292 D234S
   mutation L293 K235D
   mutation L294 K235F
   mutation L295 K235L
   mutation L296 K235S
   mutation L297 F259Y
   mutation L298 R276A
   mutation L299 R276Q
   mutation L300 A298S
   mutation L301 D300N
   mutation L302 V301M
   mutation L303 Y328F
   mutation L304 Y328H
   mutation L305 Y328M
   mutation L306 Y328W
   mutation L307 W332H
   mutation L308 E336A
   mutation L309 N338A
   mutation L310 N338F
   mutation L311 Y339K
   mutation L312 Y339L
   mutation L313 Y339T
   mutation L314 L340A
   mutation L315 L340I
   mutation L316 L340V
   mutation L317 V439P
   mutation L318 I440F
   mutation L319 I440V
   mutation L320 E441F
   mutation L321 E441M
   mutation L322 E441N
   mutation L323 N442A
   mutation L324 N442L
   mutation L325 R443S
   mutation L326 T444W
   mutation L327 R445G
   mutation L328 R445K
   mutation L329 E446A
   mutation L330 E446F
   mutation L331 E446Q
   mutation L332 E446S
   mutation L333 E446T
   mutation L334 Y447L
   mutation L335 Y447S.
[28] The mutant protein according to [20] above wherein, in said amino acid sequence comprising one or more mutations selected from any of the mutations L1 to L335, said amino acid sequence further comprises at other than the mutated position(s) one or several amino acid mutations selected from the group consisting of substitutions, deletions, insertions, additions and inversions, said mutant protein having a peptide-synthesizing activity.
[29] The mutant protein according to [27] or [28] above comprising at least the mutation L124 or L125.
[30] The mutant protein according to any one of [27] to [29] above comprising at least the mutation L303.
[31] The mutant protein according to any one of [27] to [30] above comprising at least the mutation L12.
[32] The mutant protein according to any one of [27] to [31] above comprising at least the mutation L127.
[33] The mutant protein according to any one of [27] to [32] above comprising at least the mutation L195 or L199.
[34] The mutant protein according to any one of [27] to [33] above comprising at least the mutation L130.
[35] The mutant protein according to any one of [27] to [34] above comprising at least the mutation L115.
[36] The mutant protein according to any one of [27] to [35] above comprising at least the mutation L316.
[37] The mutant protein according to any one of [27] to [36] above comprising at least the mutation L99.
[38] The mutant protein according to any one of [27] to [37] above comprising at least the mutation L15 or L16.
[39] The mutant protein according to any one of [27] to [38] above comprising at least the mutation L131.
[40] The mutant protein according to any one of [27] to [39] above comprising at least the mutation L284.
[41] The mutant protein according to any one of [27] to [40] above comprising at least the mutation L191.
[42] The mutant protein according to any one of [27] to [41] above comprising at least the mutation L65.
[43] The mutant protein according to any one of [27] to [42] above comprising at least the mutation L265.
[44] The mutant protein according to any one of [27] to [43] above comprising at least the mutation L317.
[45] The mutant protein according to any one of [27] to [44] above comprising at least the mutation L255.
[46] The mutant protein according to any one of [27] to [45] above comprising at least the mutation L52.
[47] The mutant protein according to any one of [27] to [46] above comprising at least the mutation L155.
[48] The mutant protein according to any one of [27] to [47] above comprising at least the mutation L298.
[49] The mutant protein according to any one of [27] to [48] above comprising at least the mutation L201.
[50] The mutant protein according to any one of [27] to [49] above comprising at least the mutation L145.
[51] The mutant protein according to any one of [27] to [50] above comprising at least the mutation L170.
[52] The mutant protein according to any one of [27] to [51] above comprising at least the mutation L87.
[53] The mutant protein according to any one of [27] to [52] above comprising at least the mutation L60.
[54] The mutant protein according to any one of [27] to [53] above comprising at least the mutation L110.
[55] A mutant protein having an amino acid sequence comprising one or more mutations selected from any of the following mutations M1 to M642 in an amino acid sequence of SEQ ID NO:208:
   mutation M1 T69N/I157L
   mutation M2 T69Q/I157L
   mutation M3 T69S/I157L
   mutation M4 P70A/I157L
   mutation M5 P70G/I157L
   mutation M6 P70I/I157L
   mutation M7 P70L/I157L
   mutation M8 P70N/I157L
   mutation M9 P70S/I157L
   mutation M10 P70T/I157L
   mutation M11 P70T/T210L
   mutation M12 P70T/Y328F
   mutation M13 P70V/I157L
   mutation M14 A72E/G77S
   mutation M15 A72E/E80D
   mutation M16 A72E/Y81A
   mutation M17 A72E/S84D
   mutation M18 A72E/F113W
   mutation M19 A72E/I157L
   mutation M20 A72E/G161A
   mutation M21 A72E/F162L
   mutation M22 A72E/A184G
   mutation M23 A72E/W187F
   mutation M24 A72E/F200A
   mutation M25 A72E/A204S
   mutation M26 A72E/T210L
   mutation M27 A72E/F211L
   mutation M28 A72E/F211W
   mutation M29 A72E/G226A
   mutation M30 A72E/I228K
   mutation M31 A72E/A233D
   mutation M32 A72E/Y328F
   mutation M33 A72S/I157L
   mutation M34 A72V/Y328F
   mutation M35 V73A/I157L
   mutation M36 V73I/I157L
   mutation M37 S74A/I157L
   mutation M38 S74N/I157L
   mutation M39 S74T/I157L
   mutation M40 S74V/I157L
   mutation M41 G77A/I157L
   mutation M42 G77F/I157L
   mutation M43 G77M/I157L
   mutation M44 G77P/I157L
   mutation M45 G77S/E80D
   mutation M46 G77S/Y81A
   mutation M47 G77S/S84D
   mutation M48 G77S/F113W
   mutation M49 G77S/I157L
   mutation M50 G77S/Y159N
   mutation M51 G77S/Y159S
   mutation M52 G77S/G161A
   mutation M53 G77S/F162L
   mutation M54 G77S/A184G
   mutation M55 G77S/W187F
   mutation M56 G77S/F200A
   mutation M57 G77S/A204S
   mutation M58 G77S/T210L
   mutation M59 G77S/F211L
   mutation M60 G77S/F211W
   mutation M61 G77S/I228K
   mutation M62 G77S/A233D
   mutation M63 G77S/R276A
   mutation M64 G77S/Y328F
   mutation M65 E80D/Y81A
   mutation M66 E80D/F113W
   mutation M67 E80D/I157L
   mutation M68 E80D/Y159N
   mutation M69 E80D/G161A
   mutation M70 E80D/A184G
   mutation M71 E80D/F211W
   mutation M72 E80D/Y328F
   mutation M73 E80S/I157L
   mutation M74 Y81A/F113W
   mutation M75 Y81A/I157L
   mutation M76 Y81A/Y159N
   mutation M77 Y81A/Y159S
   mutation M78 Y81A/G161A
   mutation M79 Y81A/A184G
   mutation M80 Y81A/W187F
   mutation M81 Y81A/F200A
   mutation M82 Y81A/T210L
   mutation M83 Y81A/F211W
   mutation M84 Y81A/F211Y
   mutation M85 Y81A/G226A
   mutation M86 Y81A/I228K
   mutation M87 Y81A/A233D
   mutation M88 Y81A/Y328F
   mutation M89 Y81H/I157L
   mutation M90 Y81N/I157L
   mutation M91 K83P/I157L
   mutation M92 S84A/I157L
   mutation M93 S84D/F113W
   mutation M94 S84D/I157L
   mutation M95 S84D/Y159N
   mutation M96 S84D/G161A
   mutation M97 S84D/A184G
   mutation M98 S84D/Y328F
   mutation M99 S84E/I157L
   mutation M100 S84F/I157L
   mutation M101 S84K/I157L
   mutation M102 L85F/I157L
   mutation M103 L85I/I157L
   mutation M104 L85P/I157L
   mutation M105 L85V/I157L
   mutation M106 N87A/I157L
   mutation M107 N87D/I157L
   mutation M108 N87E/I157L
   mutation M109 N87G/I157L
   mutation M110 N87Q/I157L
   mutation M111 N87S/I157L
   mutation M112 F88A/I157L
   mutation M113 F88D/I157L
   mutation M114 F88E/I157L
   mutation M115 F88E/Y328F
   mutation M116 F88L/I157L
   mutation M117 F88T/I157L
   mutation M118 F88V/I157L
   mutation M119 F88Y/I157L
   mutation M120 K106H/I157L
   mutation M121 K106L/I157L
   mutation M122 K106M/I157L
   mutation M123 K106Q/I157L
   mutation M124 K106R/I157L
   mutation M125 K106S/I157L
   mutation M126 K106V/I157L
   mutation M127 W107A/I157L
   mutation M128 W107A/Y328F
   mutation M129 W107Y/I157L
   mutation M130 W107Y/T206Y
   mutation M131 W107Y/K217D
   mutation M132 W107Y/P218L
   mutation M133 W107Y/T220L
   mutation M134 W107Y/P221D
   mutation M135 W107Y/Y328F
   mutation M136 F113A/I157L
   mutation M137 F113H/I157L
   mutation M138 F113N/I157L
   mutation M139 F113V/I157L
   mutation M140 F113W/I157L
   mutation M141 F113W/Y159N
   mutation M142 F113W/Y159S
   mutation M143 F113W/G161A
   mutation M144 F113W/F162L
   mutation M145 F113W/A184G
   mutation M146 F113W/W187F
   mutation M147 F113W/F200A
   mutation M148 F113W/T206Y
   mutation M149 F113W/T210L
   mutation M150 F113W/F211L
   mutation M151 Fl13W/F211W
   mutation M152 F113W/F211Y
   mutation M153 F113W/V213D
   mutation M154 F113W/K217D
   mutation M155 F113W/T220L
   mutation M156 F113W/P221D
   mutation M157 F113W/G226A
   mutation M158 F113W/I228K
   mutation M159 F113W/A233D
   mutation M160 F113W/R276A
   mutation M161 F113Y/I157L
   mutation M162 F113Y/F211W
   mutation M163 E114D/I157L
   mutation M164 D115A/I157L
   mutation M165 D115E/I157L
   mutation M166 D115M/I157L
   mutation M167 D115N/I157L
   mutation M168 D115Q/I157L
   mutation M169 D115S/I157L
   mutation M170 D115V/I157L
   mutation M171 I157L/Y159I
   mutation M172 I157L/Y159L
   mutation M173 I157L/Y159N
   mutation M174 I157L/Y159S
   mutation M175 I157L/Y159V
   mutation M176 I157L/P160A
   mutation M177 I157L/P160S
   mutation M178 I157L/G161A
   mutation M179 I157L/F162L
   mutation M180 I157L/F162M
   mutation M181 I157L/F162N
   mutation M182 I157L/F162Y
   mutation M183 I157L/T165L
   mutation M184 I157L/T165V
   mutation M185 I157L/Q181A
   mutation M186 I157L/Q181F
   mutation M187 I157L/Q181N
   mutation M188 I157L/A184G
   mutation M189 I157L/A184L
   mutation M190 I157L/A184M
   mutation M191 I157L/A184S
   mutation M192 I157L/A184T
   mutation M193 I157L/W187F
   mutation M194 I157L/W187Y
   mutation M195 I157L/F193H
   mutation M196 I157L/F193I
   mutation M197 I157L/F193W
   mutation M198 I157L/F200A
   mutation M199 I157L/F200H
   mutation M200 I157L/F200L
   mutation M201 I157L/F200Y
   mutation M202 I157L/A204G
   mutation M203 I157L/A204I
   mutation M204 I157L/A204L
   mutation M205 I157L/A204S
   mutation M206 I157L/A204T
   mutation M207 I157L/A204V
   mutation M208 I157L/F205A
   mutation M209 I157L/F207I
   mutation M210 I157L/F207M
   mutation M211 I157L/F207V
   mutation M212 I157L/F207W
   mutation M213 I157L/F207Y
   mutation M214 I157L/M208A
   mutation M215 I157L/M208K
   mutation M216 I157L/M208L
   mutation M217 I157L/M208T
   mutation M218 I157L/M208V
   mutation M219 I157L/S209F
   mutation M220 I157L/S209N
   mutation M221 I157L/T210A
   mutation M222 I157L/T210L
   mutation M223 I157L/F211I
   mutation M224 I157L/F211L
   mutation M225 I157L/F211V
   mutation M226 I157L/F211W
   mutation M227 I157L/G212A
   mutation M228 I157L/G212D
   mutation M229 I157L/G212S
   mutation M230 I157L/R215K
   mutation M231 I157L/R215L
   mutation M232 I157L/R215T
   mutation M233 I157L/R215Y
   mutation M234 I157L/T220L
   mutation M235 I157L/G226A
   mutation M236 I157L/G226F
   mutation M237 I157L/I228K
   mutation M238 I157L/A233D
   mutation M239 I157L/R276A
   mutation M240 I157L/Y328A
   mutation M241 I157L/Y328F
   mutation M242 I157L/Y328H
   mutation M243 I157L/Y328I
   mutation M244 I157L/Y328L
   mutation M245 I157L/Y328P
   mutation M246 I157L/Y328V
   mutation M247 I157L/Y328W
   mutation M248 I157L/L340F
   mutation M249 I157L/L340I
   mutation M250 I157L/L340V
   mutation M251 I157L/V439A
   mutation M252 I157L/V439P
   mutation M253 I157L/R445A
   mutation M254 I157L/R445F
   mutation M255 I157L/R445G
   mutation M256 I157L/R445K
   mutation M257 I157L/R445V
   mutation M258 Y159N/G161A
   mutation M259 Y159N/A184G
   mutation M260 Y159N/A204S
   mutation M261 Y159N/T210L
   mutation M262 Y159N/F211W
   mutation M263 Y159N/F211Y
   mutation M264 Y159N/G226A
   mutation M265 Y159N/I228K
   mutation M266 Y159N/A233D
   mutation M267 Y159N/Y328F
   mutation M268 Y159S/G161A
   mutation M269 Y159S/F211W
   mutation M270 G161A/F162L
   mutation M271 G161A/A184G
   mutation M272 G161A/W187F
   mutation M273 G161A/F200A
   mutation M274 G161A/A204S
   mutation M275 G161A/T210L
   mutation M276 G161A/F211L
   mutation M277 G161A/F211W
   mutation M278 G161A/G226A
   mutation M279 G161A/I228K
   mutation M280 G161A/A233D
   mutation M281 G161A/Y328F
   mutation M282 F162L/A184G
   mutation M283 F162L/F211W
   mutation M284 F162L/A233D
   mutation M285 P183A/Y328F
   mutation M286 A184G/W187F
   mutation M287 A184G/F200A
   mutation M288 A184G/A204S
   mutation M289 A184G/T210L
   mutation M290 A184G/F211L
   mutation M291 A184G/F211W
   mutation M292 A184G/I228K
   mutation M293 A184G/A233D
   mutation M294 A184G/R276A
   mutation M295 V184G/Y328F
   mutation M296 T185A/Y328F
   mutation M297 T185N/Y328F
   mutation M298 W187F/F211W
   mutation M299 W187F/Y328F
   mutation M300 F193W/F211W
   mutation M301 F200A/F211W
   mutation M302 F200A/Y328F
   mutation M303 L201Q/Y328F
   mutation M304 L201S/Y328F
   mutation M305 A204S/F211W
   mutation M306 A204S/Y328F
   mutation M307 T210L/F211W
   mutation M308 T210L/Y328F
   mutation M309 F211L/A233D
   mutation M310 F211L/Y328F
   mutation M311 F211W/I228K
   mutation M312 F211W/A233D
   mutation M313 F211W/Y328F
   mutation M314 R215A/Y328F
   mutation M315 R215L/Y328F
   mutation M316 T220L/A233D
   mutation M317 T220L/D300N
   mutation M318 P221L/A233D
   mutation M319 P221L/Y328F
   mutation M320 F224A/A233D
   mutation M321 G226A/Y328F
   mutation M322 G226F/A233D
   mutation M323 G226F/Y328F
   mutation M324 I228K/Y328F
   mutation M325 A233D/K235D
   mutation M326 A233D/Y328F
   mutation M327 R276A/Y328F
   mutation M328 Y328F/Y339F
   mutation M329 A27T/Y81A/S84D
   mutation M330 P70T/A72E/I157L
   mutation M331 P70T/G77S/I157L
   mutation M332 P70T/E80D/F88E
   mutation M333 P70T/Y81A/I157L
   mutation M334 P70T/S84D/I157L
   mutation M335 P70T/F88E/Y328F
   mutation M336 P70T/F113W/I157L
   mutation M337 P70T/I157L/A204S
   mutation M338 P70T/I157L/T210L
   mutation M339 P70T/I157L/A233D
   mutation M340 P70T/I157L/Y328F
   mutation M341 P70T/I157L/V439P
   mutation M342 P70T/I157L/I440F
   mutation M343 P70T/G161A/T210L
   mutation M344 P70T/G161A/Y328F
   mutation M345 P70T/A184G/W187F
   mutation M346 P70T/A204S/Y328F
   mutation M347 P70T/F211W/Y328F
   mutation M348 P70V/A72E/I157L
   mutation M349 A72E/S74T/I157L
   mutation M350 A72E/G77S/Y328F
   mutation M351 A72E/E80D/Y328F
   mutation M352 A72E/Y81H/I157L
   mutation M353 A72E/K83P/I157L
   mutation M354 A72E/S84D/Y328F
   mutation M355 A72E/L85P/I157L
   mutation M356 A72E/F113W/I157L
   mutation M357 A72E/F113W/Y328F
   mutation M358 A72E/F113Y/I157L
   mutation M359 A72E/D115Q/I157L
   mutation M360 A72E/I157L/G161A
   mutation M361 A72E/I157L/F162L
   mutation M362 A72E/I157L/A184G
   mutation M363 A72E/I157L/F200A
   mutation M364 A72E/I157L/A204S
   mutation M365 A72E/I157L/A204T
   mutation M366 A72E/I157L/T210L
   mutation M367 A72E/I157L/F211W
   mutation M368 A72E/I157L/G226A
   mutation M369 A72E/I157L/A233D
   mutation M370 A72E/I157L/Y328F
   mutation M371 A72E/I157L/L340V
   mutation M372 A72E/I157L/V439P
   mutation M373 A72E/G161A/Y328F
   mutation M374 A72E/F162L/Y328F
   mutation M375 A72E/A184G/Y328F
   mutation M376 A72E/W187F/Y328F
   mutation M377 A72E/F200A/Y328F
   mutation M378 A72E/A204S/Y328F
   mutation M379 A72E/T210L/Y328F
   mutation M380 A72E/I228K/Y328F
   mutation M381 A72E/A233D/Y328F
   mutation M382 A72E/Y328F/Y159N
   mutation M383 A72E/Y328F/F211W
   mutation M384 A72E/Y328F/F211Y
   mutation M385 A72E/Y328F/G226A
   mutation M386 A72V/Y81A/Y328F
   mutation M387 A72V/G161A/Y328F
   mutation M388 G77M/I157L/T210L
   mutation M389 G77P/I157L/F162L
   mutation M390 G77P/I157L/A184G
   mutation M391 G77P/F211W/Y328F
   mutation M392 G77S/Y81A/Y328F
   mutation M393 G77S/S84D/I157L
   mutation M394 G77S/F88E/I157L
   mutation M395 G77S/F113W/I157L
   mutation M396 G77S/F113Y/I157L
   mutation M397 G77S/Dl15Q/I157L
   mutation M398 G77S/I157L/G161A
   mutation M399 G77S/I157L/F200A
   mutation M400 G77S/I157L/A204S
   mutation M401 G77S/I157L/T210L
   mutation M402 G77S/I157L/F211W
   mutation M403 G77S/I157L/G226A
   mutation M404 G77S/I157L/A233D
   mutation M405 G77S/I157L/L340V
   mutation M406 G77S/I157L/V439P
   mutation M407 G77S/G161A/Y328F
   mutation M408 E80D/Y81A/Y328F
   mutation M409 Y81A/S84D/Y328F
   mutation M410 Y81A/F113W/Y328F
   mutation M411 Y81A/I157L/T210L
   mutation M412 Y81A/I157L/Y328F
   mutation M413 Y81A/G161A/Y328F
   mutation M414 Y81A/F162L/Y328F
   mutation M415 Y81A/A184G/Y328F
   mutation M416 Y81A/W187F/Y328F
   mutation M417 Y81A/A204S/Y328F
   mutation M418 Y81A/T210L/Y328F
   mutation M419 Y81A/I228K/Y328F
   mutation M420 Y81A/A233D/Y328F
   mutation M421 Y81A/Y328F/Y159N
   mutation M422 Y81A/Y328F/Y159S
   mutation M423 Y81A/Y328F/F211W
   mutation M424 Y81A/Y328F/F211Y
   mutation M425 Y81A/Y328F/G226A
   mutation M426 Y81A/Y328F/R276A
   mutation M427 K83P/I157L/A184G
   mutation M428 K83P/I157L/T210L
   mutation M429 K83P/F211W/Y328F
   mutation M430 S84D/F113W/I157L
   mutation M431 S84D/I157L/T210L
   mutation M432 F88E/I157L/F162L
   mutation M433 F88E/I157L/A184G
   mutation M434 F88E/I157L/F200A
   mutation M435 F88E/I157L/T210L
   mutation M436 F88E/I157L/Y328F
   mutation M437 F88E/I157L/Y328Q
   mutation M438 F88E/I157L/L340V
   mutation M439 F88E/T210L/Y328F
   mutation M440 F88E/F211W/Y328F
   mutation M441 F113W/I157L/G161A
   mutation M442 F113W/I157L/A184G
   mutation M443 F113W/I157L/W187F
   mutation M444 F113W/I157L/F200A
   mutation M445 F113W/I157L/A204S
   mutation M446 F113W/I157L/A204T
   mutation M447 F113W/I157L/T210L
   mutation M448 F113W/I157L/F211W
   mutation M449 F113W/I157L/G226A
   mutation M450 F113W/I157L/A233D
   mutation M451 F113W/I157L/Y328F
   mutation M452 F113W/I157L/L340V
   mutation M453 F113W/I157L/V439P
   mutation M454 F113W/G161A/T210L
   mutation M455 F113W/G161A/Y328F
   mutation M456 F113W/A184G/W187F
   mutation M457 F113Y/I157L/T210L
   mutation M458 F113Y/I157L/Y328F
   mutation M459 F113Y/G161A/T210L
   mutation M460 D115Q/I157L/T210L
   mutation M461 D115Q/I157L/Y328F
   mutation M462 I157L/Y159N/T210L
   mutation M463 I157L/Y159N/Y328F
   mutation M464 I157L/G161A/W187F
   mutation M465 I157L/G161A/F200A
   mutation M466 I157L/G161A/A204S
   mutation M467 I157L/G161A/T210L
   mutation M468 I157L/G161A/A233D
   mutation M469 I157L/G161A/Y328F
   mutation M470 I157L/F162L/A184G
   mutation M471 I157L/F162L/T210L
   mutation M472 I157L/F162L/L340V
   mutation M473 I157L/A184G/W187F
   mutation M474 I157L/A184G/F200A
   mutation M475 I157L/A184G/A204T
   mutation M476 I157L/A184G/T210L
   mutation M477 I157L/A184G/F211W
   mutation M478 I157L/A184G/L340V
   mutation M479 I157L/W187F/T210L
   mutation M480 I157L/W187F/Y328F
   mutation M481 I157L/F200A/T210L
   mutation M482 I157L/F200A/Y328F
   mutation M483 I157L/A204S/T210L
   mutation M484 I157L/A204S/Y328F
   mutation M485 I157L/A204T/T210L
   mutation M486 I157L/A204T/Y328F
   mutation M487 I157L/T210L/F211W
   mutation M488 I157L/T210L/G212A
   mutation M489 I157L/T210L/G226A
   mutation M490 I157L/T210L/A233D
   mutation M491 I157L/T210L/Y328F
   mutation M492 I157L/T210L/L340V
   mutation M493 I157L/T210L/V439P
   mutation M494 I157L/F211W/Y328F
   mutation M495 I157L/G226A/Y328F
   mutation M496 I157L/A233D/Y328F
   mutation M497 I157L/Y328F/L340V
   mutation M498 I157L/Y328F/V439P
   mutation M499 Y159N/F211W/Y328F
   mutation M500 G161A/A184G/W187F
   mutation M501 G161A/T210L/Y328F
   mutation M502 G161A/F211W/Y328F
   mutation M503 A182G/P183A/Y328F
   mutation M504 A182S/P183A/Y328F
   mutation M505 A184G/W187F/F200A
   mutation M506 A184G/W187F/A204S
   mutation M507 A184G/W187F/F211W
   mutation M508 A184G/W187F/I228K
   mutation M509 A184G/W187F/A233D
   mutation M510 F200A/F211W/Y328F
   mutation M511 A204S/F211W/Y328F
   mutation M512 A204T/F211W/Y328F
   mutation M513 F211W/Y328F/L340V
   mutation M514 P70T/A72E/I157L/Y328F
   mutation M515 P70T/A72E/T210L/Y328F
   mutation M516 P70T/G77M/I157L/Y328F
   mutation M517 P70T/Y81A/I157L/T210L
   mutation M518 P70T/Y81A/I157L/Y328F
   mutation M519 P70T/S84D/I157L/Y328F
   mutation M520 P70T/F88E/I157L/Y328F
   mutation M521 P70T/F88E/T210L/Y328F
   mutation M522 P70T/F113W/I157L/T210L
   mutation M523 P70T/F113W/G161A/Y328F
   mutation M524 P70T/F113Y/I157L/Y328F
   mutation M525 P70T/D115Q/I157L/T210L
   mutation M526 P70T/D115Q/I157L/Y328F
   mutation M527 P70T/I157L/G161A/T210L
   mutation M528 P70T/I157L/A184G/W187F
   mutation M529 P70T/I157L/A184G/T210L
   mutation M530 P70T/I157L/W187F/T210L
   mutation M531 P70T/I157L/W187F/Y328F
   mutation M532 P70T/I157L/A204T/T210L
   mutation M533 P70T/I157L/A204T/Y328F
   mutation M534 P70T/I157L/A204T/T210L
   mutation M535 P70T/I157L/T210L/F211W
   mutation M536 P70T/I157L/T210L/G226A
   mutation M537 P70T/I157L/T210L/A233D
   mutation M538 P70T/I157L/T210L/Y328F
   mutation M539 P70T/I157L/T210L/L340V
   mutation M540 P70T/I157L/T210L/V439P
   mutation M541 P70T/I157L/Y328F/V439P
   mutation M542 P70T/G161A/T210L/Y328F
   mutation M543 P70T/G161A/A233D/Y328F
   mutation M544 A72E/S74T/I157L/Y328F
   mutation M545 A72E/G77S/F113W/I157L
   mutation M546 A72E/Y81H/I157L/Y328F
   mutation M547 A72E/K83P/I157L/Y328F
   mutation M548 A72E/F88E/F113W/I157L
   mutation M549 A72E/F88E/I157L/Y328F
   mutation M550 A72E/F88E/G161A/Y328F
   mutation M551 A72E/F113W/I157L/Y328F
   mutation M552 A72E/F113W/G161A/Y328F
   mutation M553 A72E/F113Y/I157L/Y328F
   mutation M554 A72E/F113Y/G161A/Y328F
   mutation M555 A72E/F113Y/G226A/Y328F
   mutation M556 A72E/I157L/G161A/Y328F
   mutation M557 A72E/I157L/F162L/Y328F
   mutation M558 A72E/I157L/A184G/Y328F
   mutation M559 A72E/I157L/F200A/Y328F
   mutation M560 A72E/I157L/A204T/Y328F
   mutation M561 A72E/I157L/F211W/Y328F
   mutation M562 A72E/I157L/F211Y/Y328F
   mutation M563 A72E/I157L/A233D/Y328F
   mutation M564 A72E/I157L/Y328F/L340V
   mutation M565 A72E/G161A/A204T/Y328F
   mutation M566 A72E/G161A/T210L/Y328F
   mutation M567 A72E/G161A/F211W/Y328F
   mutation M568 A72E/G161A/F211Y/Y328F
   mutation M569 A72E/G161A/A233D/Y328F
   mutation M570 A72E/G161A/Y328F/L340V
   mutation M571 A72E/A184G/W187F/Y328F
   mutation M572 A72E/T210L/Y328F/L340V
   mutation M573 A72V/I157L/W187F/Y328F
   mutation M574 G77P/I157L/T210L/Y328F
   mutation M575 Y81A/S84D/I157L/Y328F
   mutation M576 Y81A/F88E/I157L/Y328F
   mutation M577 Y81A/F113W/I157L/Y328F
   mutation M578 Y81A/I157L/G161A/Y328F
   mutation M579 Y81A/I157L/W187F/Y328F
   mutation M580 Y81A/I157L/A204S/Y328F
   mutation M581 Y81A/I157L/T210L/Y328F
   mutation M582 Y81A/I157L/A233D/Y328F
   mutation M583 Y81A/I157L/Y328F/V439P
   mutation M584 Y81A/A184G/W187F/Y328F
   mutation M585 F88E/I157L/T210L/Y328F
   mutation M586 F88E/I157L/A233D/Y328F
   mutation M587 F113W/I157L/A204T/T210L
   mutation M588 F113W/I157L/T210L/Y328F
   mutation M589 I157L/G161A/A184G/W187F
   mutation M590 I157L/G161A/T210L/Y328F
   mutation M591 I157L/A184G/W187F/T210L
   mutation M592 I157L/A204S/T210L/Y328F
   mutation M593 I157L/A204T/T210L/Y328F
   mutation M594 I157L/T210L/A233D/Y328F
   mutation M595 G161A/A184G/W187F/Y328F
   mutation M596 P70T/A72E/S84D/I157L/Y328F
   mutation M597 P70T/A72E/A204S/I157L/Y328F
   mutation M598 P70T/A72E/T210L/I157L/Y328F
   mutation M599 P70T/A72E/G226A/I157L/Y328F
   mutation M600 P70T/A72E/A233D/I157L/Y328F
   mutation M601 P70T/Y81A/I157L/T210L/Y328F
   mutation M602 P70T/Y81A/I157L/A233D/Y328F
   mutation M603 P70T/Y81A/I157L/T210L/Y328F
   mutation M604 P70T/Y81A/A233D/I157L/Y328F
   mutation M605 P70T/S84D/I157L/T210L/Y328F
   mutation M606 P70T/F113W/I157L/T210L/Y328F
   mutation M607 P70T/I157L/A184G/W187F/A233D
   mutation M608 P70T/I157L/W187F/T210L/Y328F
   mutation M609 P70T/I157L/A204S/T210L/Y328F
   mutation M610 P70T/G161A/A184G/W187F/Y328F
   mutation M611 P70V/A72E/F113Y/I157L/Y328F
   mutation M612 P70V/A72E/I157L/F211W/Y328F
   mutation M613 A72E/S74T/F113Y/I157L/Y328F
   mutation M614 A72E/S74T/I157L/F211W/Y328F
   mutation M615 A72E/Y81H/I157L/F211W/Y328F
   mutation M616 A72E/K83P/F113Y/I157L/Y328F
   mutation M617 A72E/W17F/F113Y/I157L/Y328F
   mutation M618 A72E/F113Y/D115Q/I157L/Y328F
   mutation M619 A72E/F113Y/I157L/Y328F/L340V
   mutation M620 A72E/F113Y/I157L/Y328F/V439P
   mutation M621 A72E/F113Y/G161A/I157L/Y328F
   mutation M622 A72E/F113Y/A204S/I157L/Y328F
   mutation M623 A72E/F113Y/A204T/I157L/Y328F
   mutation M624 A72E/F113Y/T210L/I157L/Y328F
   mutation M625 A72E/F113Y/A233D/I157L/Y328F
   mutation M626 A72E/I157L/G161A/F162L/Y328F
   mutation M627 A72E/I157L/N187F/F211N/Y328F
   mutation M628 A72E/I157L/A204S/F211W/Y328F
   mutation M629 A72E/I157L/A204T/F211W/Y328F
   mutation M630 A72E/I157L/F211W/Y328F/L340V
   mutation M631 A72E/I157L/F211W/Y328F/V439P
   mutation M632 A72E/I157L/G226A/F211W/Y328F
   mutation M633 A72E/I157L/A233D/F211W/Y328F
   mutation M634 Y81A/S84D/I157L/T210L/Y328F
   mutation M635 Y81A/I157L/A184G/W187F/Y328F
   mutation M636 Y81A/I157L/A184G/W187F/T210L
   mutation M637 Y81A/I157L/A233D/T210L/Y328F
   mutation M638 F88E/I157L/A184G/W187F/T210L
   mutation M639 F113Y/I157L/Y159N/F211W/Y328F
   mutation M640 I157L/A184G/W187F/T210L/Y328F
   mutation M641 P70T/I157L/A184G/W187F/T210L/Y328F
   mutation M642 Y81A/I157L/A184G/W187F/T210L/Y328F.
[56] The mutant protein according to [55] above wherein, in said amino acid sequence comprising one or more mutations selected from any of the mutations M1 to M642, said amino acid sequence further comprises at other than the mutated position(s) one or several amino acid mutations selected from the group consisting of substitutions, deletions, insertions, additions and inversions, said mutant protein having a peptide-synthesizing activity.
[57] The mutant protein according to any one of [55] to [56] above comprising at least the mutation M241.
[58] The mutant protein according to any one of [55] to [57] above comprising at least the mutation M340.
[59] The mutant protein according to any one of [55] to [58] above comprising at least the mutation M412.
[60] The mutant protein according to any one of [55] to [59] above comprising at least the mutation M491.
[61] The mutant protein according to any one of [55] to [60] above comprising at least the mutation M496.
[62] The mutant protein according to any one of [55] to [61] above comprising at least the mutation M581.
[63] The mutant protein according to any one of [55] to [62] above comprising at least the mutation M582.
[64] The mutant protein according to any one of [55] to [63] above comprising at least the mutation M594.
[65] A polynucleotide encoding an amino acid sequence of the mutant protein according to any one of [18] to [64] above.
[66] A recombinant polynucleotide comprising the polynucleotide according to [65] above.
[67] A transformed microorganism comprising the recombinant polynucleotide according to [66] above.
[68] A method for producing a mutant protein comprising culturing the transformed microorganism according to [67] above in a medium, to accumulate the mutant protein in the medium and/or the transformed microorganism.
[69] A method for producing a peptide comprising performing a peptide-synthesizing reaction in the presence of the mutant protein according to any one of [18] to [64] above.
[70] A method for producing a peptide comprising culturing the transformed microorganism according to [67] above in a medium to accumulate the mutant protein in the medium and/or the transformed microorganism for performing a peptide-synthesizing reaction.
[71] A method for producing α-L-aspartyl-L-phenylalanine-β-ester comprising reacting L-aspartic acid-α,β-diester and L-phenylalanine in the presence of the mutant protein according to any one of [18] to [64] above.
[72] A method for producing α-L-aspartyl-L-phenylalanine-β-ester comprising culturing the transformed microorganism according to [67] above in a medium to accumulate the mutant protein in the medium and/or the transformed microorganism for performing a reaction of L-aspartic acid-α,β-diester and L-phenylalanine.

### EFFECT OF THE INVENTION

According to the present invention, a protein having an excellent peptide-synthesizing activity and a method for efficient peptide production are provided.

### BEST MODE FOR CARRYING OUT THE INVENTION

Embodiments for carrying out the invention will be described below along with the best mode thereof.
Concerning various genetic engineering techniques described below, many standard experimental manuals such as Molecular Cloning, 2nd edition, Cold Spring Harbor Press (1989); Saibo Kogaku Handbook (Cellular Engineering Handbook) edited by Toshio Kuroda et al., Yodosha (1992); and Shin Idenshi Kogaku Handbook (New Genetic Engineering Handbook) revised 3rd version, edited by Muramatsu et al., Yodosha (1999) are available, and the techniques may be carried out by those skilled in the art with reference to these literatures.

Abbreviations as used herein for amino acids, peptides, nucleic acids, nucleotide sequences and the like are in conformity with definitions by IUPAC (International Union of Pure and Applied Chemistry) or IUBMB (International Union of Biochemistry and Molecular Biology), or conventional legends used in "Guideline for the preparation of specification and others containing a base sequence and an amino acid sequence" (edited by Japanese Patent Office) and in this field of art. Sequence numbers used herein indicate the sequence numbers in Sequence Listing unless otherwise specified. With respect to amino acids other than glycine, when a D-amino acid or an L-amino acid is not specified, the amino acid refers to the L-amino acid.

### 1. Proteins having a peptide-synthesizing activity of the present invention (mutant proteins based on the amino acid sequence of SEQ ID NO:2)

The protein of the present invention is a mutant protein having an amino acid sequence in which one or more mutations from any of the following mutations 1 to 68 have been introduced in the amino acid sequence of SEQ ID NO:2, and has a peptide-synthesizing activity (this protein may be referred to hereinbelow as the "mutant protein (I)"). The mutations 1 to 68 are as shown in Tables 1-1 and 1-2.

### Table 1-1

**Table 1-1: MUTATION**

| MUTATION No. | MUTATION |
|---|---|
| 1 | F207V |
| 2 | Q441 E |
| 3 | K83A |
| 4 | A301V |
| 5 | V257I |
| 6 | A537G |
| 7 | A324V |
| 8 | N607K |
| 9 | D313E |
| 10 | Q229H |
| 11 | M208A |
| 12 | E551 K |
| 13 | F207H |
| 14 | T72A |
| 15 | A137S |
| 16 | L439V |
| 17 | G226S |
| 18 | D619E |
| 19 | Y339H |
| 20 | W327G |
| 21 | V184A |
| 22 | V184C |
| 23 | V184G |
| 24 | V184I |
| 25 | V184L |
| 26 | V184M |
| 27 | V184P |
| 28 | V184S |
| 29 | V184T |
| 30 | Q441K |
| 31 | N442K |
| 32 | D203N |
| 33 | D203S |
| 34 | F207A |
| 35 | F207S |
| 36 | Q441N |
| 37 | F207T |
| 38 | F207I |

### Table 1-2

**Table 1-2: MUTATION**

| MUTATION No. | MUTATION |
|---|---|
| 39 | T210K |
| 40 | W187A |
| 41 | S209A |
| 42 | F211A |
| 43 | F211V |
| 44 | V257A |
| 45 | V257G |
| 46 | V257H |
| 47 | V257M |
| 48 | V257N |
| 49 | V257Q |
| 50 | V257S |
| 51 | V257T |
| 52 | V257W |
| 53 | V257Y |
| 54 | K47G |
| 55 | K47E |
| 56 | N442F |
| 57 | N607R |
| 58 | P214T |
| 59 | Q202E |
| 60 | Y494F |
| 61 | R117A |
| 62 | F207G |
| 63 | S209D |
| 64 | S209G |
| 65 | Q441 D |
| 66 | R445D |
| 67 | R445F |
| 68 | N442D |

As shown in Tables 1-1 and 1-2, each mutation in the present specification is specified by the abbreviation of the amino acid residue and the position in the amino acid sequence in SEQ ID NOS:1 or 2. For example, "F207V" which is designated as the mutation 1 indicates that the amino acid residue, phenylalanine at position 207 in the sequence of SEQ ID NO:2 has been substituted with valine. That is, the mutation is represented by the type of the amino acid residue in a wild type (amino acid specified in SEQ ID NO:2), the position of the amino acid residue in the amino acid sequence of SEQ ID NO:2, and the type of the amino acid residue after introduction of the mutation. Other mutations are represented in the same fashion.

Each of the mutations 1 to 68 may be introduced alone or in combination of two or more. One or more of the mutations 1 to 68 may be introduced in combination with one or more mutations selected from the mutations other than those in Tables 1-1 and 1-2, for example, mutations in V184N, Q229P, Q229L, Q229G, Q229I, I228G, I228L, I228D, I228S, I230D, I230V, I230S, S256C, A301G, L66F, E80K, Y81A, I157L, V178G, A182G, A182S, P183A, V184P, T185F, T185A, T185K, T185D, T185C, T185S, T185P, T185N, T210L, V213A, P214T, P214H, A245S, L263M, K314R, S315R, Y328F, K484I, and A515V. Specifically, the combinations as shown in the following Tables 1-3 and 1-4 are preferable. The mutant protein comprising at least the mutation 2: Q441E and the mutant protein comprising at least the mutation 14: T72A are preferable in terms of enhanced peptide-synthesizing activity. In addition, the mutant proteins comprising the combination of M7-35, and M35-4+V184A (A1) are also preferable in terms of enhanced peptide-synthesizing activity.

### Table 1-3

**Table 1-3: MUTATION (COMBINATION OF TWO OR MORE MUTATIONS)**

| MUTATION No. | MUTATION | ABBREVIATED NAME |
|---|---|---|
| 239 | F207V+Q441E | |
| 240 | F207V+K83A | |
| 241 | F207V+E551K | |
| 242 | K83A+Q441E | |
| 243 | M208A+E551K | |
| 244 | V257I+Q441E | |
| 245 | V257I+A537G | |
| 246 | F207V+S209A | |
| 247 | K83A+S209A | |
| 248 | K83A+F207V+Q441E | |
| 249 | L439V+F207V+0441E | |
| 250 | A537G+F207V+Q441E | |
| 251 | A301V+F207V+Q441E | |
| 252 | G226S+F207V+Q441E | |
| 253 | V257I+F207V+Q441E | |
| 254 | D619E+F207V+Q441E | |
| 255 | Y339H+F207V+Q441E | |
| 256 | N607K+F207V+Q441E | |
| 257 | A324V+F207V+Q441E | |
| 258 | Q229H+F207V+Q441E | |
| 259 | W327G+F207V+Q441 E | |
| 260 | A301V+L439V+A537G+N607K | M7-35 |
| 261 | K83A+Q229H+A301V+D313E+A324V+L439V+A537G+N607K | M7-46 |
| 262 | Q229H+V257I+A301V+A324V+Q441E+A537G+N607K | M7-54 |
| 263 | Q229H+A301V+A324V+Q441E+A537G+N607K | M7-63 |
| 264 | Q229H+V257I+A301V+D313E+A324V+Q441E+A537G+N607K | M7-95 |
| 265 | T72A+A137S+A301V+L439V+Q441E+A537G+N607K | M9-9 |
| 266 | T72A+A137S+A301V+Q441E+A537G+N607K | M9-10 |
| 267 | T72A+A137S+Q229H+A301V+A324V+L439V+A537G+N607K | M11-2 |
| 268 | T72A+A137S+Q229H+A301V+A324V+L439V+Q441E+A537G+N607K | M11-3 |
| 269 | T72A+ Q229H+V2571+A301V+D313E+A324V+L439V+Q441E+A537G+N607K | M12-1 |
| 270 | T72A+ Q229H+V257I+A301V+D313E+A324V+Q441E+A537G+N607K | M12-3 |
| 271 | T72A+A137S+Q229P+A301V+L439V+Q441E +A537G+N607K | M21-18 |
| 272 | T72A+A137S+0229L+A301V+L439V+Q441E +A537G+N607K | M21-22 |
| 273 | T72A+A137S+Q229G+A301V+L439V+Q441E +A537G+N607K | M21-25 |
| 274 | T72A+ Q229I+V257I+A301V+D313E+A324V+L439V+Q441E+A537G+N607K | M22-25 |
| 275 | T72A+A137S+1228G+Q229P+A301V+L439V+Q441E +A537G+N607K | M24-1 |
| 276 | T72A+A137S+1228L+Q229P+A301V+L439V+Q441E +A537G+N607K | M24-2 |
| 277 | T72A+A137S+I228D+Q229P+A301V+L439V+Q441E +A537G+N607K | M24-5 |
| 278 | T72A+A137S+ Q229P+I230D+A301V+L439V+Q441E +A537G+N607K | M26-3 |
| 279 | T72A+A137S+ Q229P+I230V+A301V+L439V+Q441E +A537G+N607K | M26-5 |
| 280 | T72A+I228S+Q229H+V257I+A301V+D313E+A324V+L439V+Q441E+A537G+N607K | M29-3 |
| 281 | T72A+Q229H+S256C+V257I+A301V+D313E+A324V+L439V+Q441E+A537G+N607K | M33-1 |
| 282 | T72A+A137S+Q229P+V257I+A301V+A324V+L439V+Q441E +A537G+N607K | M35-4 |
| 283 | T72A+A137S+Q229P+A301V+A324V+L439V+Q441E +A537G+N607K | M37-5 |
| 284 | T72A+ Q229P+V257I+A301G+D313E+A324V+0441E+A537G+N607K | M39-4 |
| 285 | T72A+ Q229P+V2571+A301V+D313E+A324V+Q441E+A537G+N607K | M41-2 |
| 286 | T72A+A137S+V184A+Q229P+V257I+A301V+A324V+L439V+Q441E+A537G+N607K | M35-4/ V184A |
| 287 | T72A+A137S+V184G+Q229P+V257I+A301V+A324V+L439V+Q441E+A537G+N607K | M35-4/ V184G |
| 288 | T72A+A137S+V184N+Q229P+V257I+A301V+A324V+L439V+Q441E+A537G+N607K | M35-4/ V184N |
| 289 | T72A+A137S+V184S+Q229P+V257I+A301V+A324V+L439V+Q441E+A537G+N607K | M35-4/ V184S |
| 290 | T72A+A137S+V184T+Q229P+V257I+A301V+A324V+L439V+Q441E+A537G+N607K | M35-4/ V184T |

### Table 1-4

**Table 1-4: MUTATION (COMBINATION OF TWO OR MORE MUTATIONS)**

| MUTANT No. | MUTATION | | ABBREVIATED NAME |
|---|---|---|---|
| 324 | V184A+V257Y | | |
| 325 | V184A+W187A | | |
| 326 | V184A+N442D | | |
| 327 | V184P+N442D | | |
| 328 | V184A+N442D+L439V | | |
| 329 | A301V+L439V+A537G+N607K+V184A | | M7-35/V184A |
| 330 | A301V+L439V+A537G+N607K+V184P | | M7-35/V184P |
| 331 | A301V+L439V+A537G+N607K+V257Y | | M7-35/V257Y |
| 332 | A301V+L439V+A537G+N607K+W187A | | M7-35/VV187A |
| 333 | A301V+L439V+A537G+N607K+F211A | | M7-35/F211A |
| 334 | A301V+L439V+A537G+N607K+Q441E | | M7-35/Q441E |
| 335 | A301V+L439V+A537G+N607K+N442D | | M7-35/N442D |
| 336 | A301V+L439V+A537G+N607K+V184A+F207V | | M7-35/V184A/F207V |
| 337 | A301V+L439V+A537G+N607K+V184A+A182G | | M7-35/V184A/A182G |
| 338 | T72A+A137S+Q229P+V257I+A301V+A324V+L439V +A537G+N607K+V184A+N442D | | M35-4/-Q441E/V184A/N442D |
| 339 | T72A+A137S+Q229P+V257I+A301V+A324V+L439V +A537G+N607K+V184A+N442D+T185F | | M35-4/-Q441E/V184A/N442D/T185F |
| 340 | T72A+A137S+Q229P+V257I+A301V+A324V+L439V+Q441E +A537G+N607K+V184A+K83A | | A1(M35-4/V184A)/K83A |
| 341 | T72A+A137S+Q229P+V257I+A301V+A324V+L439V+Q441E +A537G+N607K+V184A+W1B7A | | A1(M35-4/V184A)/W187A |
| 342 | T72A+A137S+Q229P+V257I+A301V+A324V+L439V+Q441E+A537G+N607K+V184A+F211A | | A1(M35-4/V184A)/F211A |
| 343 | T72A+A137S+Q229P+V257I+A301V+A324V+L439V+Q441E+A537G+N607K+V184A+V178G | | A1(M35-4/V184A)/V178G |
| 344 | T72A+A137S+Q229P+V257I+A301V+A324V+L439V+Q441E+A537G+N607K+V184A+T185A | | A1(M35-4/V184A)/T185A |
| 345 | T72A+A137S+Q229P+V257I+A301V+A324V+L439V+Q441E+A537G+N607K+V184A+A182G | | A1(M35-4/V184A)/A182G |
| 346 | T72A+A137S+Q229P+V257I+A301V+A324V+L439V+Q441E+A537G+N607K+V184A+K314R | | A1(M35-4/V184A)/K314R |
| 347 | T72A+A137S+Q229P+V257I+A301V+A324V+L439V+Q441E+A537G+N607K+V184A+A515V | | A1(M35-4/V184A)/A515V |
| 348 | T72A+A137S+Q229P+V25TI+A301V+A324V+L439V+Q441E+A537G+N607K+V184A+L66F | | A1(M35-4N184A)/L66F |
| 349 | T72A+A137S+Q229P+V257I+A301V+A324V+L439V+Q441E+A537G+N607K+V184A+S315R | | A1(M35-4/V184A)/S315R |
| 350 | T72A+A137S+Q229P+V257I+A301V+A324V+L439V+Q441E+A537G+N607K+V184A+K4841 | | A1(M35-4/V184A)/K484I |
| 351 | T72A+A137S+Q229P+V257I+A301V+A324V+L439V+Q441E+A537G+N607K+V184A+V213A | | A1(M35-4/V184A)/V213A |
| 352 | T72A+A137S+Q229P+V257I+A301V+A324V+L439V+Q441E+A537G+N607K+V184A+A245S | | A1(M35-4/V184A)/A245S |
| 353 | T72A+A137S+Q229P+V257I+A301V+A324V+L439V+Q441E+A537G+N607K+V184A+P214H | | A1(M35-4/V184A)/P214H |
| 354 | T72A+A137S+Q229P+V257I+A301V+A324V+L439V+Q441E+A537G+N607K+V184A+L263M | | A1(M35-4/V184A)/L263M |
| 355 | T72A+A137S+Q229P+V257I+A301V+A324V+L439V+Q441E+A537G+N607K+V184A+P183A | | A1(M35-4/V184A)/P183A |
| 356 | T72A+A137S+Q229P+V257I+A301V+A324V+L439V+Q441E+A537G+N607K+V184A+T185K | | A1(M35-4/V184A)/T185K |
| 357 | T72A+A137S+Q229P+V257I+A301V+A324V+L439V+Q441E+A537G+N607K+V184A+T185D | | A1(M35-4/V184A)/T185D |
| 358 | T72A+A137S+Q229P+V257I+A301V+A324V+L439V+Q441E+A537G+N607K+V184A+T185C | | A1(M35-4/V184A)/T185C |
| 359 | T72A+A137S+Q229P+V257I+A301V+A324V+L439V+Q441E+A537G+N607K+V184A+T185S | | A1(M35-4/V184A)/T185S |
| 360 | T72A+A137S+Q229P+V257I+A301V+A324V+L439V+Q441E+A537G+N607K+V184A+T185F | | A1(M35-4/V184A)/T185F |
| 361 | T72A+A137S+Q229P+V257I+A301V+A324V+L439V+Q441E+A537G+N607K+V184A+T185P | | A1(M35-4/V184A)/T185P |
| 362 | T72A+A137S+Q229P+V257I+A301V+A324V+L439V+Q441E+A537G+N607K+V184A+T185N | | A1(M35-4/V184A)/T185N |
| 363 | T72A+A137S+Q229P+V257I+A301V+A324V+L439V+Q441E+A537G+N607K+V184A+P183A+A182G | | A1(M35-4/V184A)/P183A/A182G |
| 364 | T72A+A137S+Q229P+V257I+A301V+A324V+L439V+Q441E+A537G-N607K+V184A+P183A+A182S | | A1(M35-4/V184A)/P183A/A182S |
| 365 | T72A+A137S+Q229P+V257I+A301V+A324V+L439V+Q441E+A537G+N607K+V184A+T185F+N442D | | A1(M35-4/V184A)/T185F/N442D |
| 366 | T72A+A137S+Q229P+V257I+A301V+A324V+L439V+Q441E+A537G+N607K+V184A+L66F | | F22 |
| | E80K+I157L+A182G+P214H+L263M | | |
| 367 | T72A+A137S+Q229P+V257I+A301V+A324V+L439V+Q441E+A537G+N607K+V184A+L66F | | F22/Y328F |
| | E80K+1157L+A182G+P214H+L263M+Y328F | | |
| 368 | T72A+A137S+Q229P+V257I+A301V+A324V+L439V+Q441E +A537G+N607K+V184A+L66F | | F22/-E80K/Y328F/Y81A |
| | Y81A+1157L+A182G+P214H+L263M+Y328F | | |
| 369 | T72A+A137S+Q229P+V257I+A301V+A324V+L439V+Q441E+A537G+N607K+V184A+L66F | | F22/-P214H/Y328F/T210L |
| | E80K+I157L+A182G+T210L+L263M+Y328F | | |
| 370 | A301V+L439V+A537G+N607K+Q441K | | M7-35/Q441K |
| 371 | T72A+A137S+Q229P+V257I+A301V+A324V+L439V+Q441E+A537G+N607K+V184A+I157L | | A1(M35-4/V184A)/I157L |
| 372 | T72A+A137S+Q229P+V257I+A301V+A324V+L439V+Q441E +A537G+N607K+V184A+G161A | | A1(M35-4/V184A)/G161A |
| 373 | T72A+A137S+Q229P+V257I+A301V+A324V+L439V+Q441E +A537G+N607K+V184A+Y328F | | A1(M35-4/V184A)/Y328F |
| 374 | F207V+G226S | | F207V/G226S |
| 375 | F207V+W327G | | F207V/W327G |
| 376 | F207V+Y339H | | F207V/Y339H |
| 377 | F207V+D619E | | F207V/339H |

| | | | |
|---|---|---|---|
| M7-35 : A301V+L439V+A537G+N607K M35-4*N*184A =A1 : T72A+A137S+Q229P+V257I+A301V+A324V+L439V+Q441E+A537G+N607K+V184A | | | |

The mutant protein of the present invention has an excellent peptide-synthesizing activity. That is, the mutant protein exert more excellent performance as to capability to catalyze a peptide-synthesizing reaction than the wild type protein having the amino acid sequence of SEQ ID NO:2. More specifically, each mutant protein of the present invention exert more excellent performance as to any of the properties required for the peptide-synthesizing reaction, such as a reaction rate, a yield, a substrate specificity, a pH property and a temperature stability, than the wild type protein when the peptide is synthesized from a specific carboxy component and a specific amine component (specifically, see the following Examples). Thus, the mutant protein of the present invention may be used suitably for production of the peptide on an industrial scale. A preferable embodiment of the mutant protein may be those having the ability to achieve preferably 1.3 times or more, more preferably 1.5 times or more and still more preferably 2 times or more peptide concentration when the peptide concentration achieved by the wild type protein is "1" .

In the present specification, the peptide-synthesizing activity refers to an activity to synthesize a new compound having a peptide bond by forming the peptide bond from two or more substances, and more specifically refers to the activity to synthesize a peptide compound obtained by increasing at least one peptide bond from, e.g., two amino acids or esters thereof.

The mutation shown in the mutations 1 to 68 and the mutations 239 to 290 and 324 to 377 may be introduced by modifying the nucleotide sequence of the gene encoding the protein having the amino acid sequence of SEQ ID NO:2 by, e.g., a site-directed mutagenesis such that the amino acid at specific position is substituted. The nucleotide sequence corresponding to the position to be mutated in the amino acid sequence of SEQ ID NO:2 may easily be identified by referring to SEQ ID NO:1. A polypeptide encoded by the nucleotide sequence modified as the above may be obtained by conventional mutagenesis. Examples of the mutagenesis may include a method of *in vitro* treatment of a DNA encoding the protein with hydroxylamine, a method of introduction of the mutation by error-prone PCR, and a method of amplification of a DNA in a host which lacks a mutation repair system and subsequent retrieval of the mutated DNA.

According to the present invention, substantially the same protein as the mutant protein comprising one or more mutations selected from the above mutations 1 to 68 and the mutations 239 to 290 and 324 to 377 is also provided. That is, the present invention also provides a mutant protein wherein, in the mutant protein comprising one or more mutations selected from the mutations 1 to 68 and the mutations 239 to 290 and 324 to 377, the amino acid sequence thereof further comprises at other than the mutated position(s) one or more amino acid mutations selected from the group consisting of substitutions, deletions, insertions, additions and inversions; and wherein the mutant protein has the peptide-synthesizing activity (the protein may be referred to hereinbelow as the "mutant protein (II)"). That is, the mutant protein of the present invention may contain the mutation at the position other than positions of the mutations 1 to 68, 239 to 290 and 324 to 377 of the amino acids shown in SEQ ID NO:2. Therefore, when the mutation such as deletions and insertions has been introduced at the position other than the positions of the mutations 1 to 68, 239 to 290 and 324 to 377, the number of amino acid residues from the position specified by the mutations 1 to 68, 239 to 290 and 324 to 377 to the N terminus or the C terminus may be sometimes different from that before introducing the mutation.

As used herein, "several amino acids" may vary depending on the position and the type in the tertiary structure of the protein of amino acid residues, but may be in a range so as not to significantly impair the tertiary structure and the activity of the protein of amino acid residues. Specifically, "several" may refer to 2 to 50, preferably 2 to 30 and more preferably 2 to 10 amino acids. In the case of the mutant protein comprising the mutated position other than the positions of the mutations 1 to 68, 239 to 290 and 324 to 377, it is desirable to retain the peptide-synthesizing activity at about a half or more, more preferably 80% or more and still more preferably 90% or more of that of the protein comprising one or more mutations from the mutations 1 to 68, 239 to 290 and 324 to 377 (i.e.. the mutant protein (I)) under a condition at 50°C and pH 8.

The mutation other than the mutations 1 to 68, 239 to 290 and 324 to 377 may also be obtained by, e.g., the site-directed mutagenesis method for modifying the nucleotide sequence so that an amino acid at a specific position of the present protein is substituted, deleted, inserted, added or inverted. The polypeptide encoded by the nucleotide sequence modified as the above may also be obtained by the conventional mutagenesis. Examples of the mutagenesis may include the method of *in vitro* treating the DNA encoding the mutant protein (I) with hydroxylamine, and the method of treating *Escherichia* bacteria which carries the DNA encoding the mutant protein (I) with ultraviolet ray or with a conventional mutagen for artificial mutagenesis such as N-methyl-N'-nitro-N-nitrosoguanidine (NTG) and nitrous acid.

The mutations such as substitutions, deletions, insertions, additions and inversions of nucleotides as the above encompass naturally occurring mutations such as those owing to difference of species or microbial strains of the microorganism. A DNA encoding substantially the same protein as the protein of SEQ ID NO:2 may be obtained by expressing the DNA having the mutation as the above in an appropriate cell and examining the enzyme activity of the expressed products.

### 2. Design and preparation of mutant protein based on amino acid sequence of SEQ ID NO:208

The present inventor found out that the mutant peptide which is more excellent in peptide-synthesizing activity may be designed and prepared by further adding the mutation to the aforementioned mutant protein. In particular, the inventors found out that the mutant protein which exerts the remarkable peptide-synthesizing activity is obtainable by further adding the mutation to the M35-4/V184A mutant (A1) (mutation 286; see Table 1-3). The present invention also provides the method for designing and producing the mutant protein based on such an M35-4/V184A mutant (A1).

The amino acid sequence corresponding to the M35-4/V184A is as shown in SEQ ID NO:208. That is, in the amino acid sequence of SEQ ID NO:208, the amino acid residues at 11 positions have been substituted with other amino acid residues corresponding to the M35-4/V184A mutation (see Table 1-3) based on the amino acid sequence of SEQ ID NO:2.

The mutant protein may be designed and produced based on tertiary structure determination by X-ray crystal structure analysis and the structural information determined thereby. That is, the mutant protein having the peptide-synthesizing activity may be designed and produced by predicting the substrate binding site based on the tertiary structure obtained by analyzing the X-ray crystal structure of the protein, and changing at least a part of the substrate binding site of the protein.

The determination of the protein tertiary structure by analyzing the X-ray crystal structure may be performed by, for example, the following procedure.

(1) A protein is crystallized. Crystallization is essential for the determination of the tertiary structure, and is industrially useful as the method for purifying the protein at high purity and the method for stably storing the protein with high density and high protease resistance.

(2) The prepared crystal is then irradiated with an X-ray, and diffraction data are collected. The protein crystal is often damaged by X-ray irradiation and lose diffraction quality. In order to avoid such a phenomenon, the low-temperature measurement where the crystal is rapidly cooled to about -173°C and the diffraction data are collected in that state has become common recently. To finally collect high resolution data used for the structure determination, synchrotron radiation with high luminance may be utilized.

(3) Subsequently, a crystal structure is analyzed. To analyze the crystal structure, phase information is required in addition to the diffraction data. For example, for the protein having the amino acid sequence of SEQ ID NO:209, the structure can be determined by a molecular replacement method because the crystal structure of an analogous protein, the S205A mutant of α-amino acid ester hydrolase (Entry Number of Protein Data Bank: 1NX9), has been known publicly. The model of the protein is then fit to the electron density map calculated using the determined phase. This process is performed on computer graphics using a program such as QUANTA supplied from Accelrys (USA). Subsequently, the structure is refined using the program such as CNX supplied from Accelrys to complete the structural analysis.
The substrate binding site of the protein may be predicted based on the tertiary structure analyzed as a result of the aforementioned processing. As used herein, the "substrate binding site" means the site on the protein surface at which the substrate (e.g., the amino acid or amino acid ester in the case of the protein having the peptide-synthesizing activity) interacts, and is generally present around an active center of the protein.

In the method for design and production of the present invention, the protein having the amino acid sequence of SEQ ID NO:208 is used as the subject of the crystal structure analysis. The protein having the amino acid sequence of SEQ ID NO:208 is the mutant protein M35-4/V184A as already described. That is, the amino acid sequence of SEQ ID NO:208 is the same as the amino acid sequence of SEQ ID NO:2 except that the amino acid residues at 11 positions have been substituted with the specific amino acid residues corresponding to the mutation M35-4/V184A described in Table 1-3.

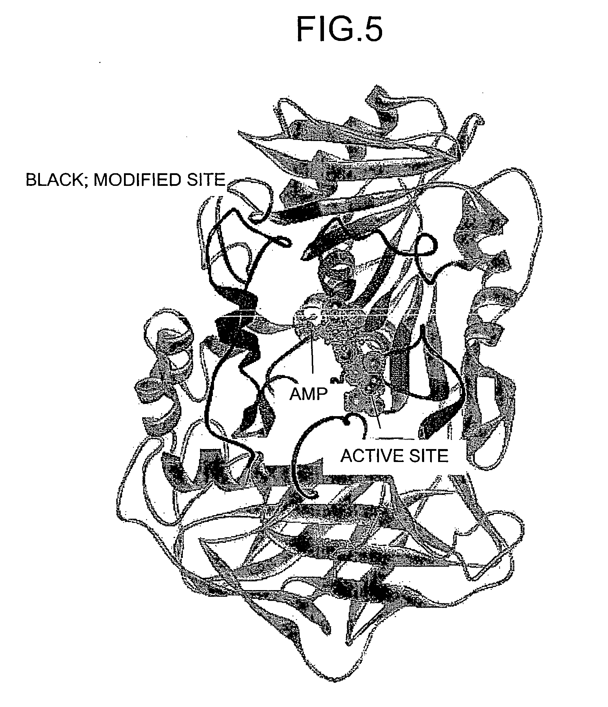

The amino acid sequence of SEQ ID NO:209 and the amino acid sequence of SEQ ID NO:208 are very highly homologous, and only 4 amino acid residues have been substituted. Therefore, the substrate binding site of the protein having the amino acid sequence of SEQ ID NO:208 may be predicted by analyzing the crystal structure of the protein having the amino acid sequence of SEQ ID NO:209, and referring to the resulting tertiary structure. The substrate binding site of the protein having the amino acid sequence of SEQ ID NO:208 was predicted as a region within 15 angstroms from an active residue serine (position 158 in the amino acid sequence of SEQ ID NO:208, which may be abbreviated hereinbelow as "Ser158" ; see an "active site" in FIG. 5) on the basis of the result of the aforementioned structural analysis of the protein having the amino acid sequence of SEQ ID NO:209.

In the method for design and production of the present invention, it is possible to obtain a mutant having a enhanced peptide-synthesizing activity by changing at least a part of the predicted substrate binding site. As used herein, "changing at least a part of the substrate binding site" means modification of one or more residues in the amino acid residues which configure the substrate binding site, particularly substituting, inserting or deleting, and preferably substituting with the other amino acid residues, with a proviso that the mutant protein after changing has the peptide-synthesizing activity. The number of the amino acid residues subjected to the modification may vary depending on the position and the type of the amino acid residues, and may be suitably determined in the range in which the tertiary structure and the activity of the resulting mutant protein are not significantly impaired.

For example, in order to obtain the mutant protein having the peptide-synthesizing activity from the protein having the amino acid sequence of SEQ ID NO:208, at least one or more amino acid residues may be substituted, inserted or deleted at positions in at least a part of the region within 15 angstroms from the active residue Ser158 in the protein, i.e., at positions 67 to 70, 72 to 88, 100, 102, 103, 106, 107, 113 to 117, 130, 155 to 163, 165, 166, 180 to 188, 190 to 195, 200 to 235, 259, 273, 276, 278, 292 to 294, 296, 298, 299, 300 to 304, 325 to 328, 330 to 340, and 437 to 447 in the amino acid sequence of SEQ ID NO:208. Specifically, the desired mutant protein may be obtained by substituting at least one residue among the foregoing amino acid residues with another amino acid residue.

In particular, the mutant protein obtained by substituting, inserting or deleting at least one or more amino acid residues at positions 67, 69, 70, 72 to 85, 103, 106, 107, 113 to 116, 165, 182, 183, 185, 187, 188, 190, 200, 202, 204 to 206, 209 to 211, 213 to 235, 301, 328, 338 to 340, 440 to 442 and 446 in the amino acid sequence of SEQ ID NO:208 may have a high peptide-synthesizing activity and particularly have an enhanced AMP-synthesizing activity. Specifically, AMP yield enhancement probability of these mutant proteins compared with the A1 mutant protein is 20% or more.

Particularly, the mutant protein obtained by substituting, inserting or deleting at least one or more amino acid residues at positions 67, 69, 70, 72 to 84, 106, 107, 114, 116, 183, 185, 187, 188, 202, 204 to 206, 209, 211, 213 to 233, 235, 328, 338 to 442, and 446 in the amino acid sequence of SEQ ID NO:208 and having the peptide-synthesizing activity may have a high peptide-synthesizing activity and a particularly enhanced AMP-synthesizing activity . Specifically, AMP yield enhancement probability of these mutant proteins compared with the A1 mutant protein is 30% or more.

Further, the mutant protein obtained by substituting, inserting or deleting at least one or more amino acid residues at positions 67, 70, 72 to 75, 77 to 79, 81 to 84, 114, 116, 185, 188, 202, 204, 206, 209, 211, 213 to 215, 218 to 224, 226 to 233, 235, 328, 338 to 441 and 446 in the amino acid sequence of SEQ ID NO:208 and having the peptide-synthesizing activity may have a high peptide-synthesizing activity, and a particularly enhanced AMP-synthesizing activity. Specifically, AMP yield enhancement probability of these mutant proteins compared with the A1 mutant protein is 40% or more.

It is preferable that the designed mutant protein has homology in terms of its primary sequence (i.e., amino acid sequences) to some extent with the A1 mutant protein. The homology may be, for example, 25% or more, more preferably 50% or more, still more preferably 80% or more and particularly preferably 90% or more.

It is possible to find out the mutant protein having the enhanced peptide-synthesizing activity by changing at least a part of the amino acid positions, i.e., substituting one or more amino acid residue, in the aforementioned range of the amino acid residues. It is also possible to combine mutations each of which has brought about the enhanced activity, to create a mutant protein having further enhanced peptide-synthesizing activity by their synergistic effect. Meanwhile, in the enhancement of the peptide-synthesizing activity by the mutation, changing of even one atom of a side chain in the amino acid residue may possibly result in a drastic change. Therefore, there are various possibilities for the optimization. For example, if mutation of a certain position reveals that the position is involved in enhancement of the activity, random mutation on several residues neighboring the position in the tertiary structure may result in discovery of a mutant having a further enhanced activity. That is, it is possible to obtain a mutant protein having a peptide-synthesizing activity by modification of at least a part of positions which configure a continuous surface in terms of a tertiary structure with an amino acid residue whose modification brings about enhancement of the peptide-synthesizing activity.

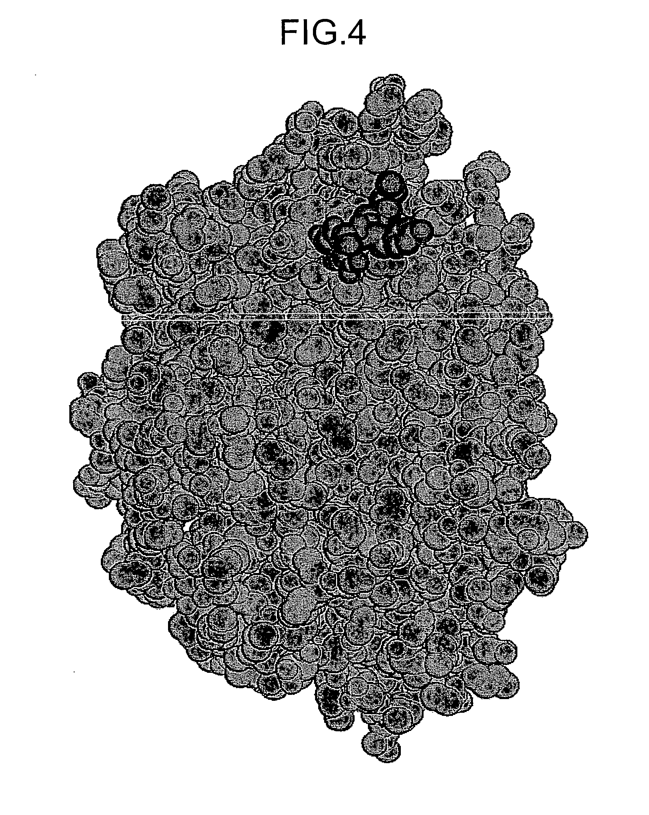

The surface of a protein is an envelop surface of the part exposed to a solvent when constitutive atoms are represented as a sphere with van der Waals radius, and may be figured by a space-filling view as shown in FIG. 4. In the protein having the amino acid sequence of SEQ ID NO:208, "the position which configures a continuous surface in terms of a tertiary structure with an amino acid residue whose modification brings about enhancement of the peptide-synthesizing activity" is the part which constitutes a continuous patch on the protein surface described above, for example, two or more positions in the positions 67 to 70, 72 to 88, 100, 102, 103, 106, 107, 113 to 117, 130, 155 to 163, 165, 166, 180 to 188, 190 to 195, 200 to 235, 259, 273, 276, 278, 292 to 294, 296, 298, 299, 300 to 304, 325 to 328, 330 to 340, and 437 to 447 in the amino acid sequence of SEQ ID NO:208. Specifically, for example, the location at which the amino acid residues at positions 79 to 82 in the amino acid sequence of SEQ ID NO:208 are the part shown by a gray color in FIG. 4. Specifically, the mutant protein having the peptide-synthesizing activity may be obtained by causing one or more changes in the tertiary structure selected from the following (a) to (i).
(a) One or more amino acid residue substitutions, insertions or deletions at any of positions 79 to 82 in the amino acid sequence of SEQ ID NO:208
(b) One or more amino acid residue substitutions, insertions or deletions at any of positions 84, 88, 89 and 92 in the amino acid sequence of SEQ ID NO:208
(c) One or more amino acid residue substitutions, insertions or deletions at any of positions 72, 75 and 77 in the amino acid sequence of SEQ ID NO:208
(d) One or more amino acid residue substitutions, insertions or deletions at any of positions 159, 161, 162, 184, 187 and 276 in the amino acid sequence of SEQ ID NO:208
(e) One or more amino acid residue substitutions, insertions or deletions at any of positions 70, 106, 113, 115, 193, 207, 209-212, 216 and 259 in the amino acid sequence of SEQ ID NO:208
(f) One or more amino acid residue substitutions, insertions or deletions at any of positions 200, 202-205, 207 and 228 in the amino acid sequence of SEQ ID NO:208
(g) One or more amino acid residue substitutions, insertions or deletions at any of positions 233, 234 and 439 in the amino acid sequence of SEQ ID NO:208
(h) One or more amino acid residue substitutions, insertions or deletions at any of positions 328, 339, 340, 445 and 446 in the amino acid sequence of SEQ ID NO:208
(i) One or more amino acid residue substitutions, insertions or deletions at any of positions 87, 155, 157 and 160 in the amino acid sequence of SEQ ID NO:208

### 3. Design and preparation of a mutant protein on the basis of other proteins than the mutant protein of SEQ ID NO:208

The tertiary structure of the protein having the amino acid sequence of SEQ ID NO:209 obtained by the X-ray crystal structure analysis described above may be practically applied to designing and producing a mutant protein on the basis of other proteins than the protein having the amino acid sequence of SEQ ID NO:208. The present invention also provides a mutant protein derived from such other proteins and having the peptide-synthesizing activity equal to or higher than that of the protein having the amino acid sequence of SEQ ID NO:208.

The mutant protein on the basis of other proteins than the protein having the amino acid sequence of SEQ ID NO:208 may be designed and produced by the alignment of the tertiary structure with the protein having the amino acid sequence of SEQ ID NO:209 by the threading method, and giving the same amino acid mutations as the protein having the amino acid sequence of SEQ ID NO:208. As already described, the amino acid residues at only 3 positions are different between the protein having the amino acid sequence of SEQ ID NO:208 and the protein having the amino acid sequence of SEQ ID NO:209. Thus, their three dimensional structures may be regarded to be almost the same.

The protein to which mutation is introduced with the threading method is a protein other than the protein having the amino acid sequence of SEQ ID NO:208, and preferably a protein having the peptide-synthesizing activity. Furthermore, it is preferable to use the protein whose amino acid sequence has been already known. It is preferable that the protein to be mutated has a tertiary structure similar to that of the mutant protein having the amino acid sequence of SEQ ID NO:209. As used herein, "having a similar tertiary structure" means that secondary structures or three dimensional structures are similar, and specifically means the similarity in distances between the amino acid residues and angles of backbones and side chains which configure the peptides.

The threading method may be used for determining whether the protein other than the protein having the amino acid sequence of SEQ ID NO:208 has the similar tertiary structure to that of the protein having the amino acid sequence of SEQ ID NO:209 or not. The threading method is a method in which what tertiary structure the amino acid sequence has is assessed and predicted on the basis of the similarity with known tertiary structures in the database (Science 253:164-170, 1991).

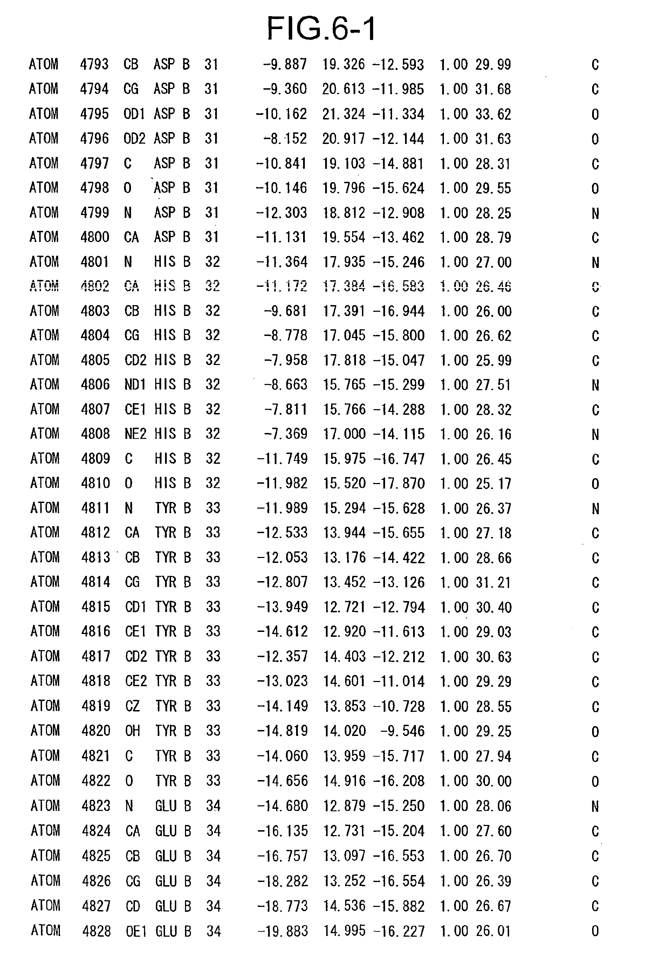

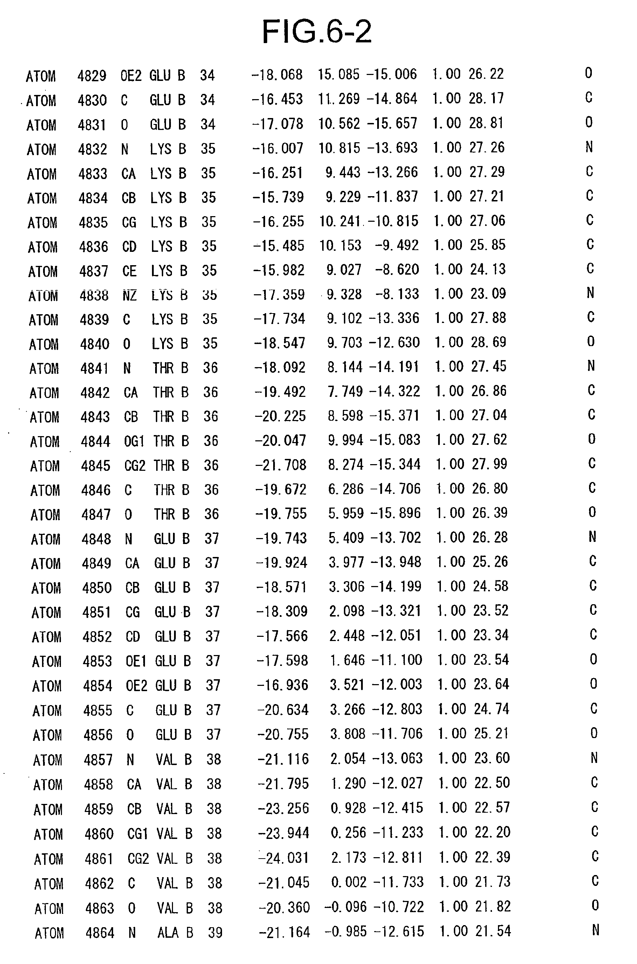

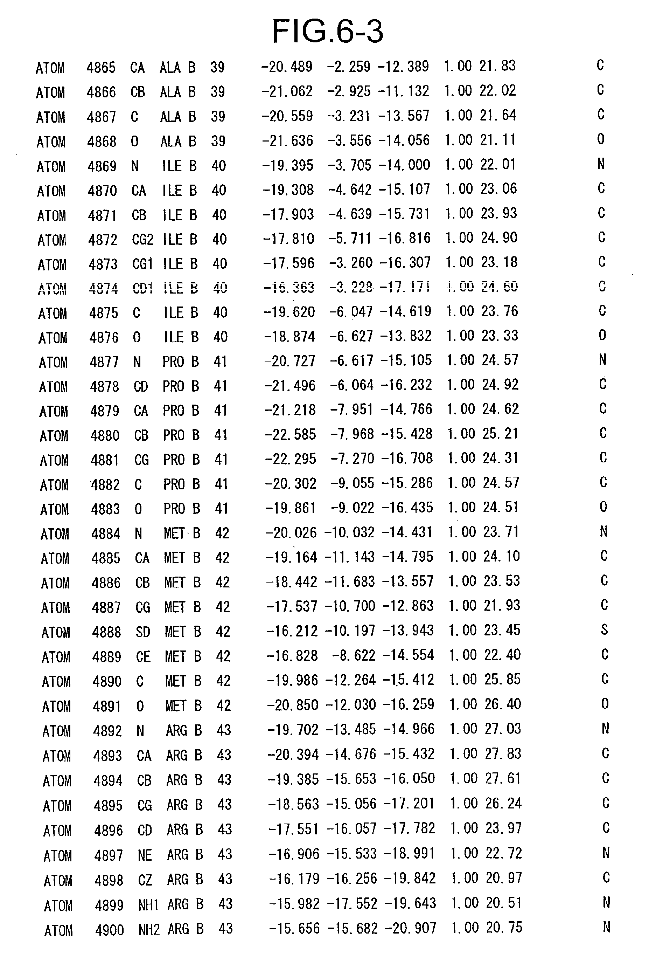

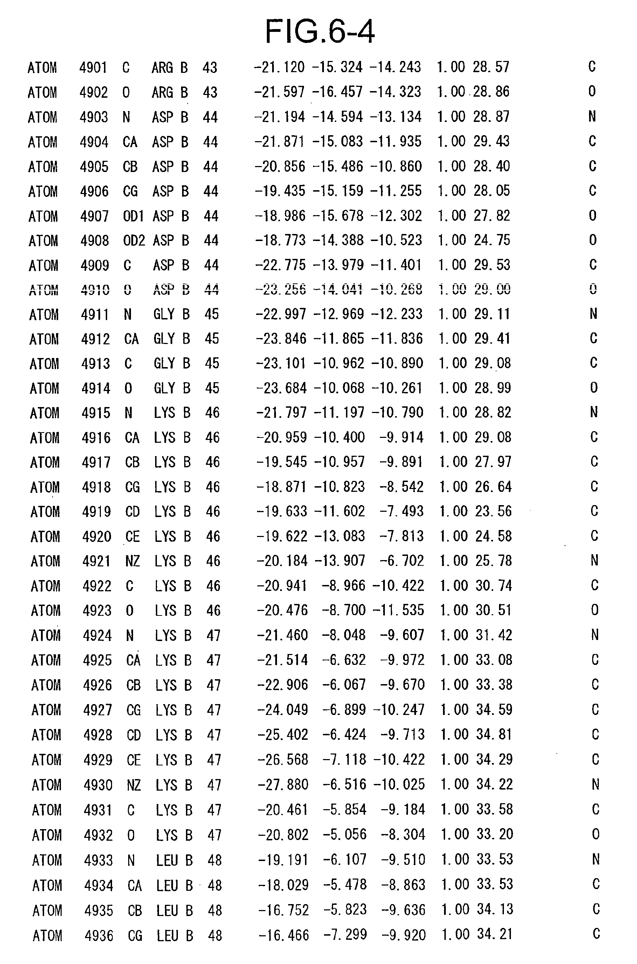

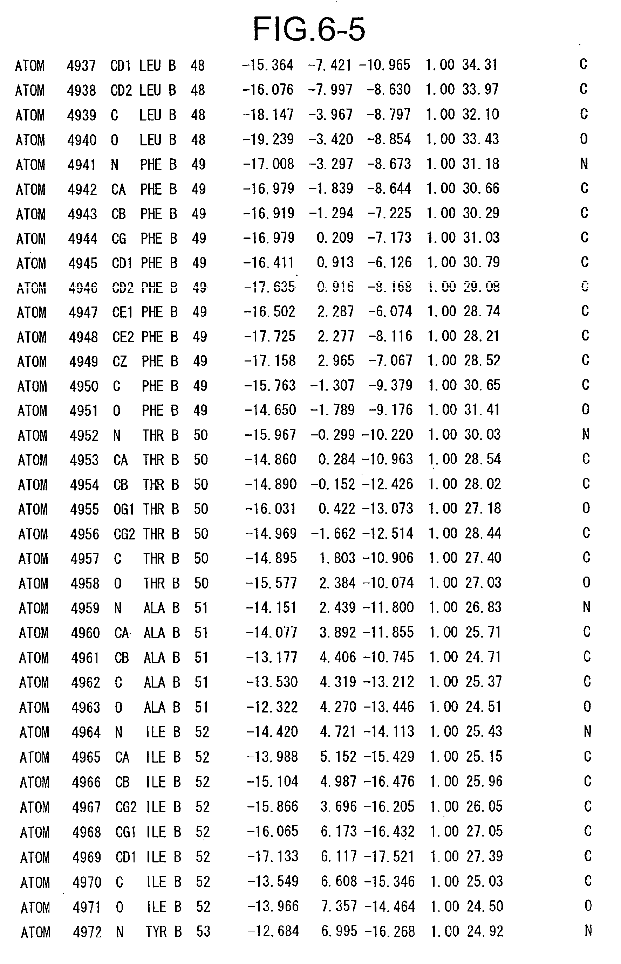

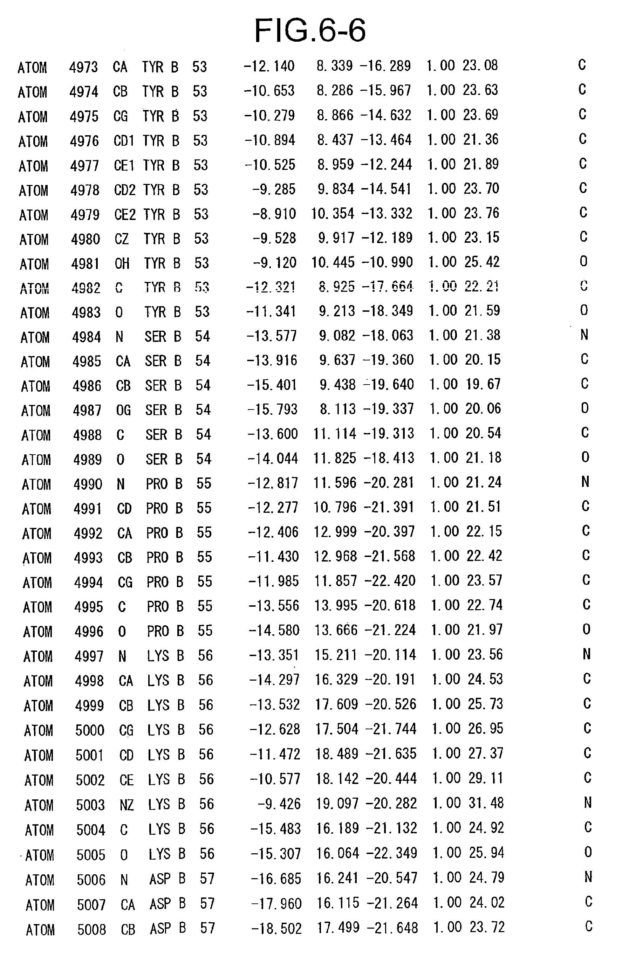

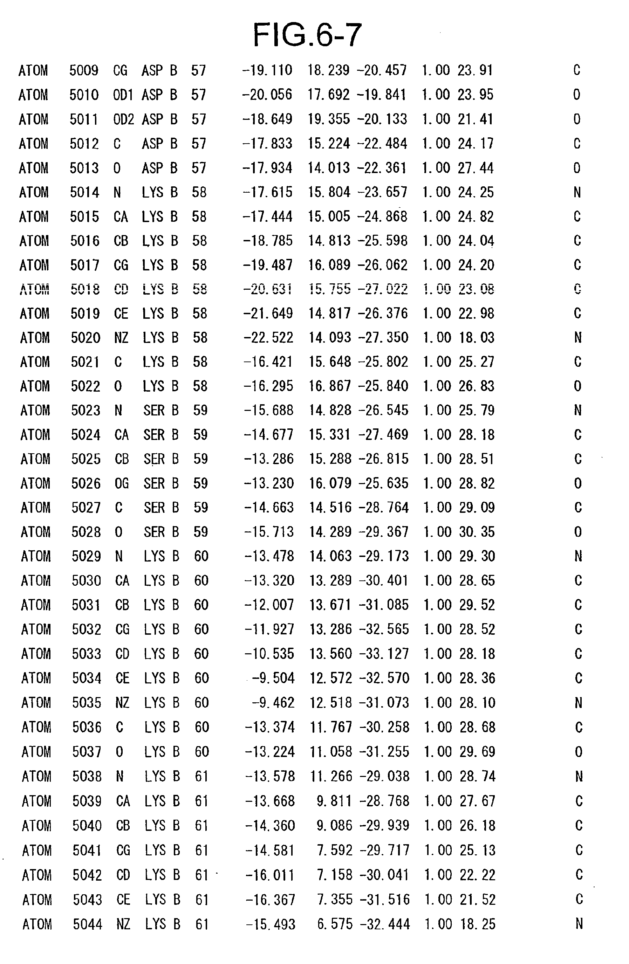

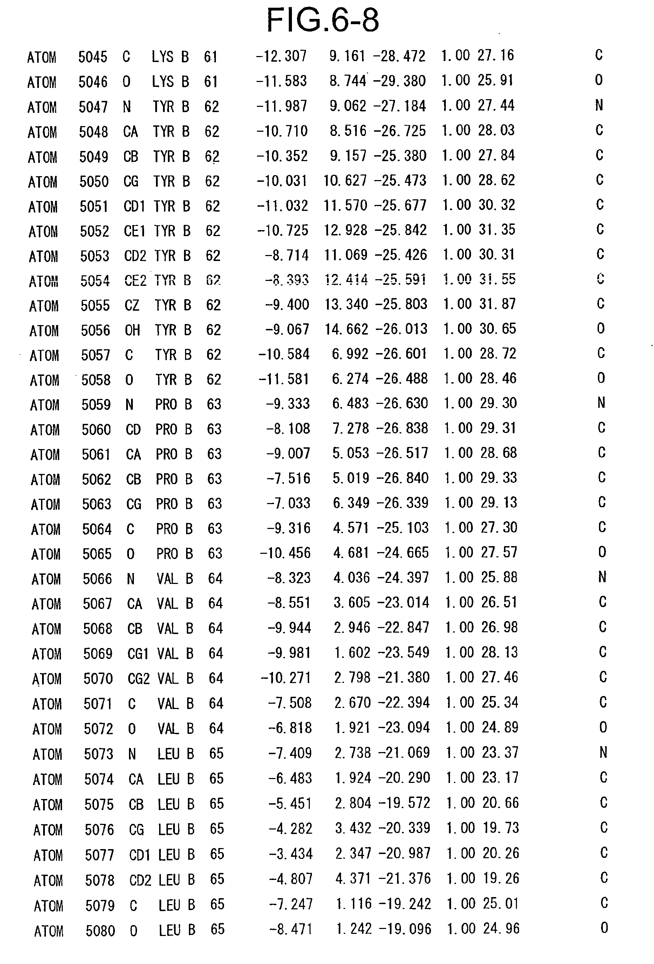

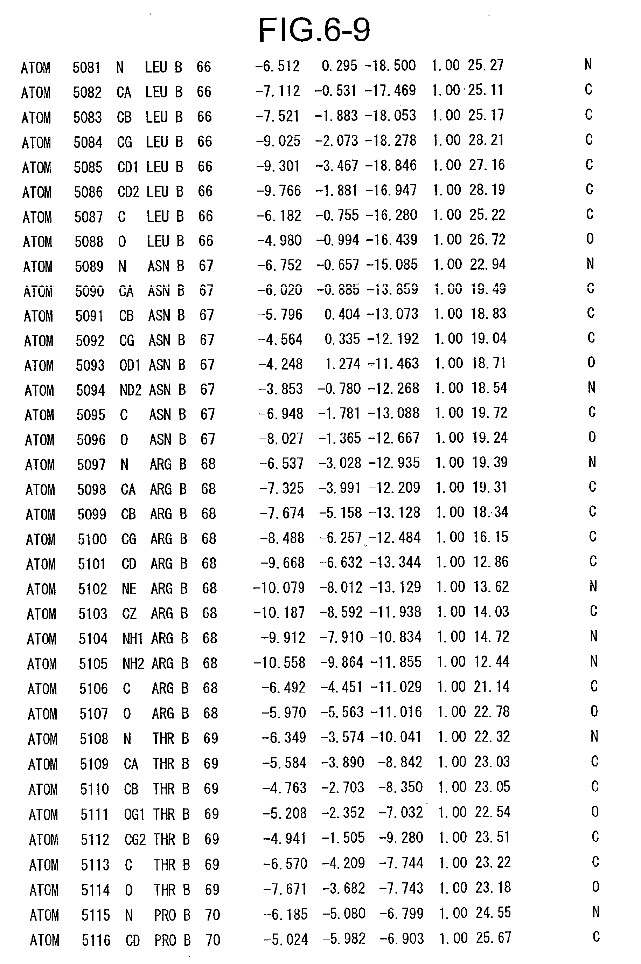

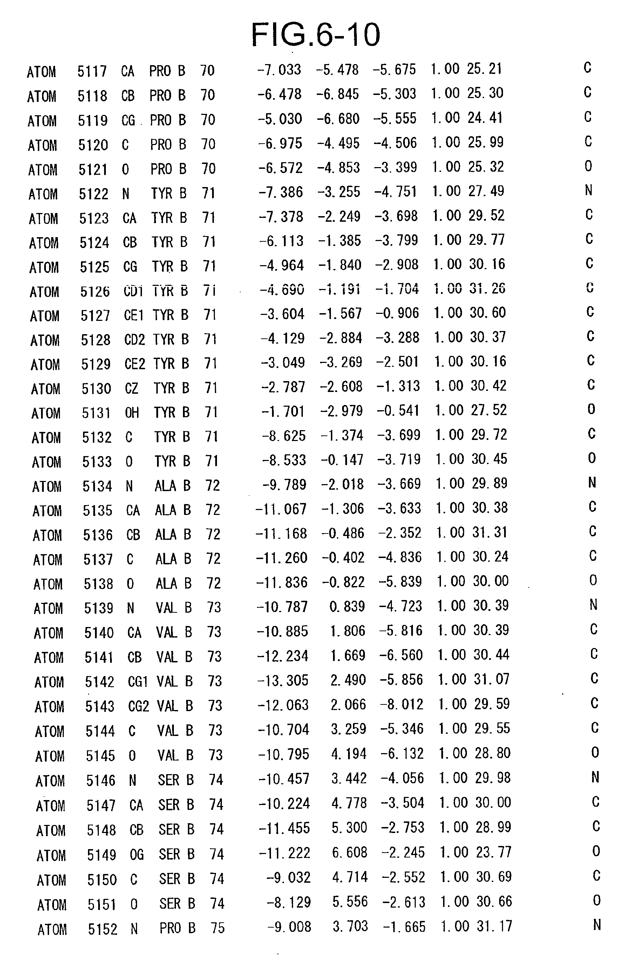

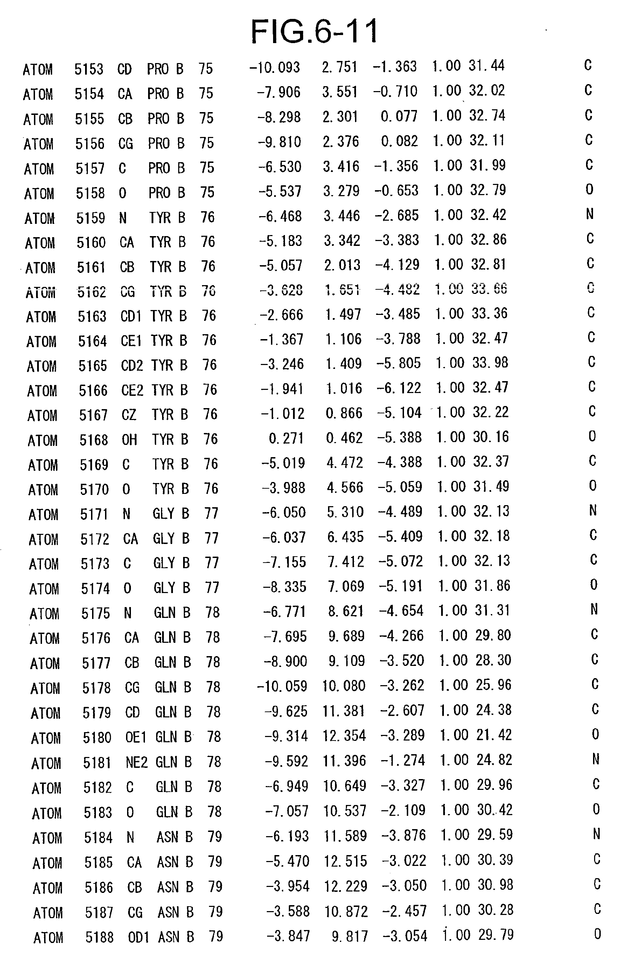

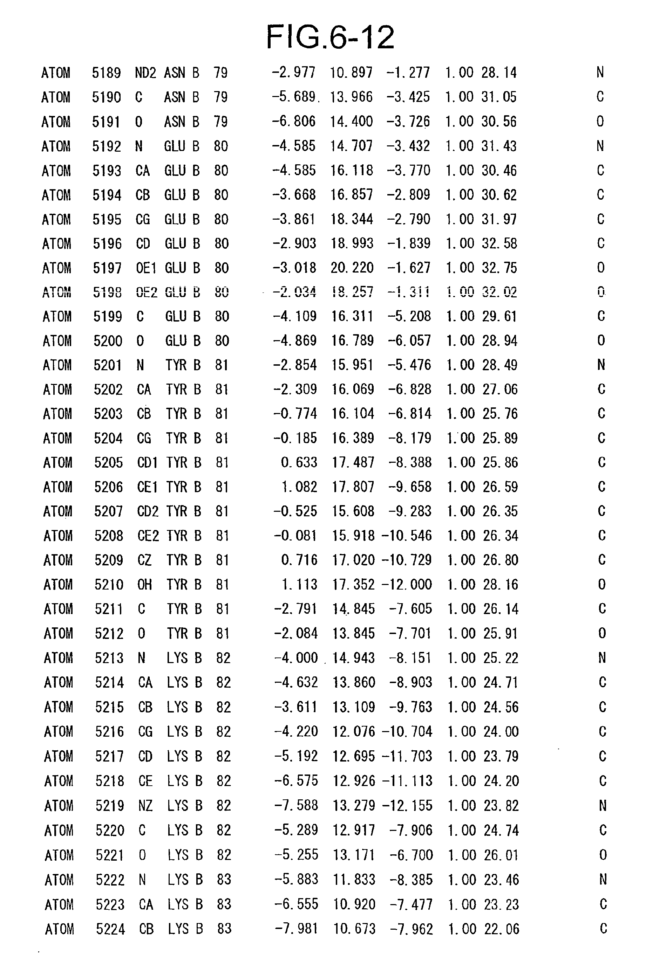

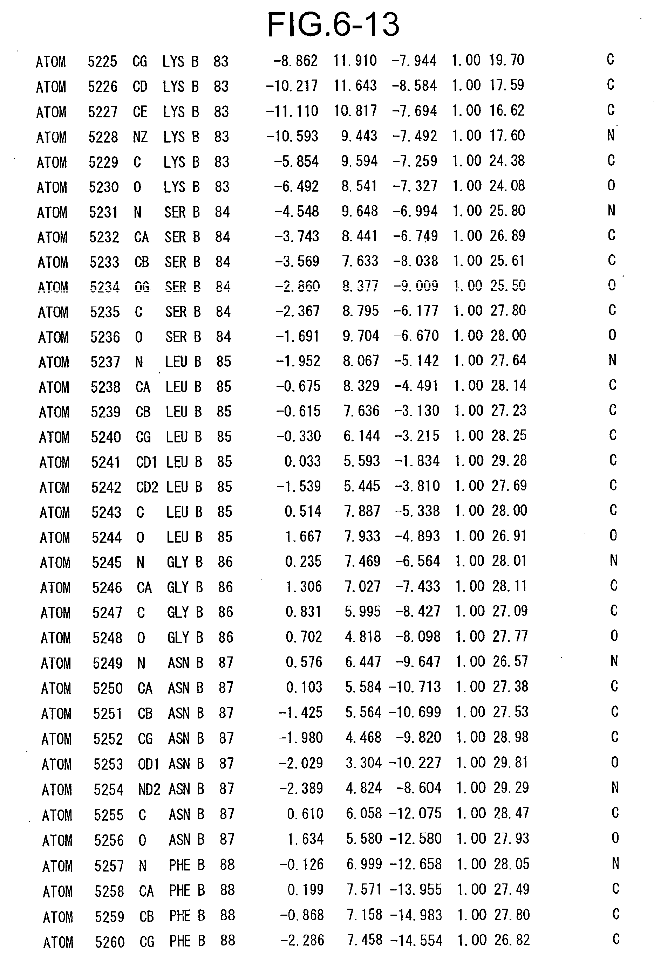

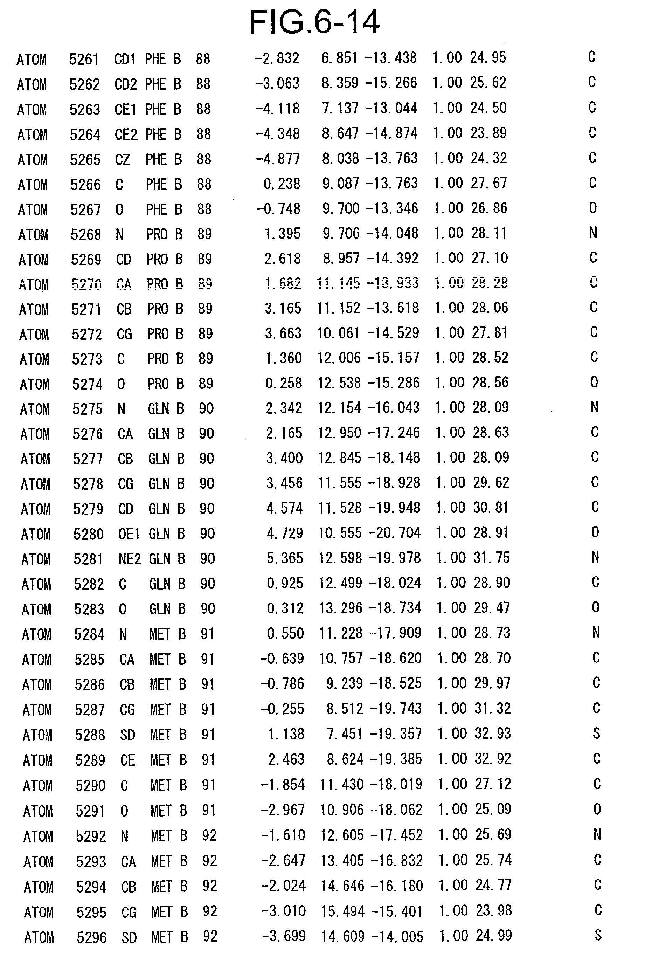

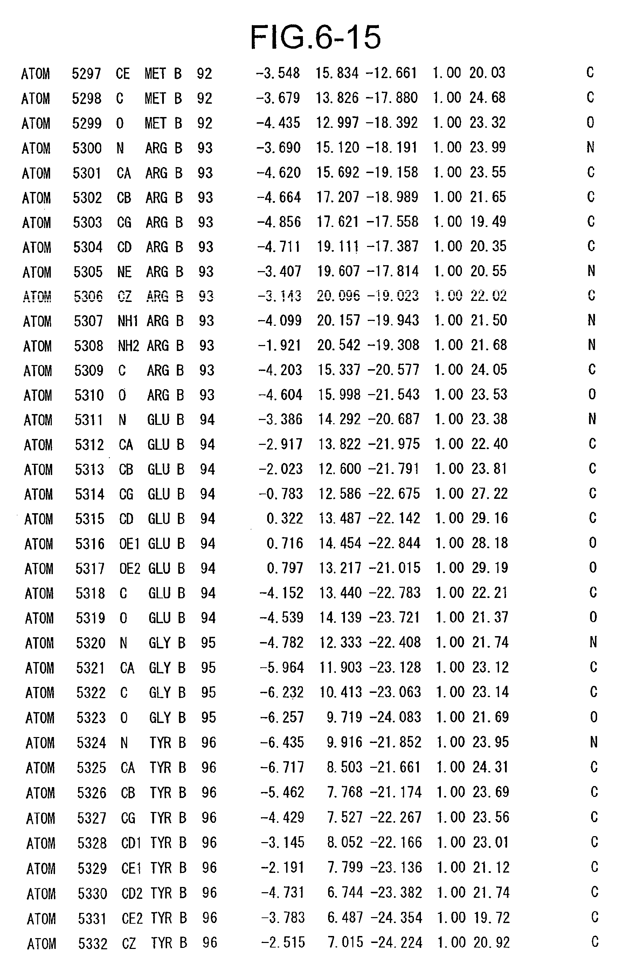

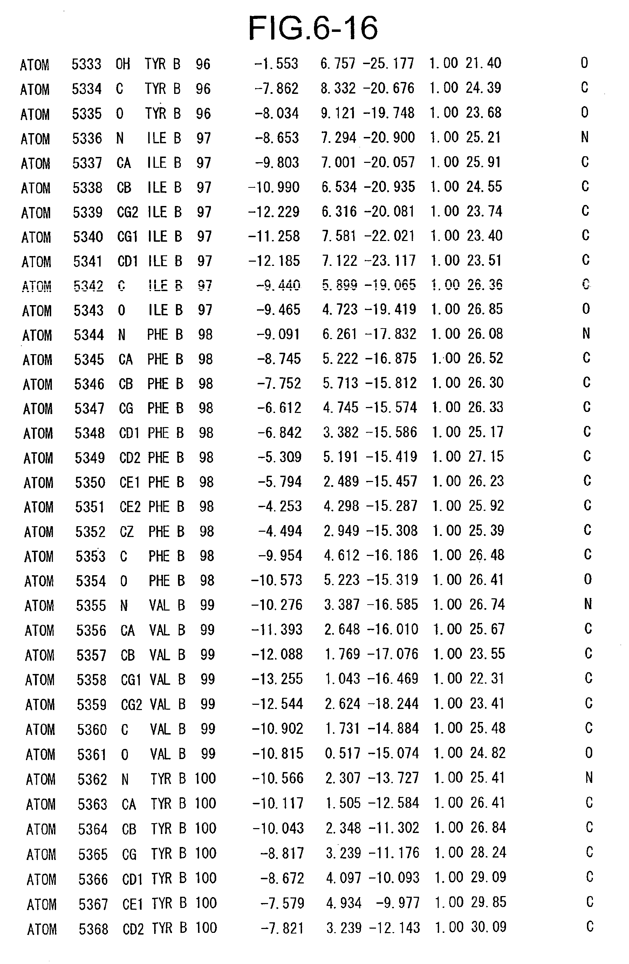

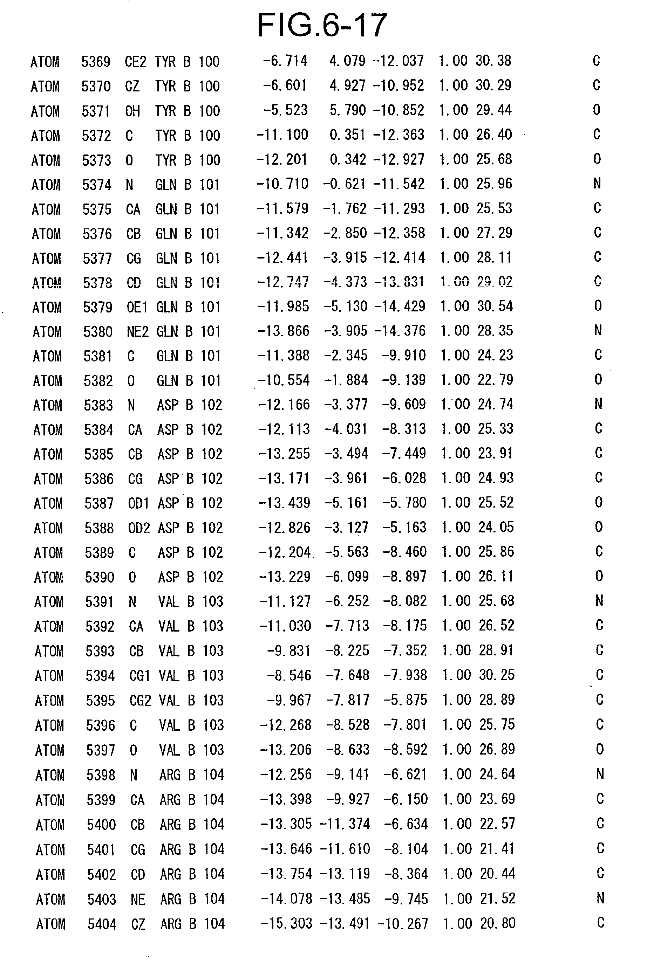

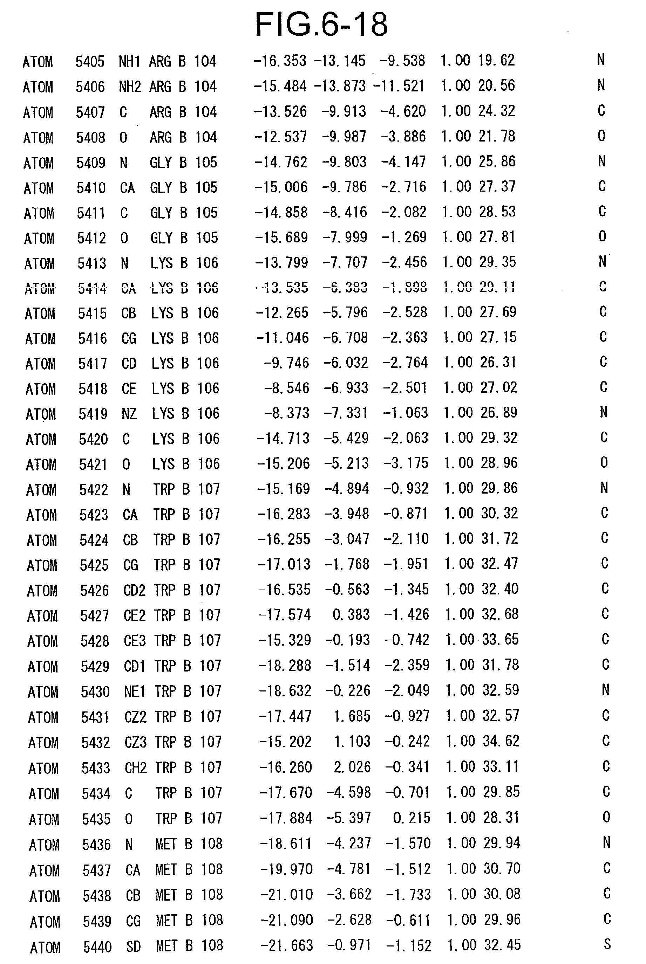

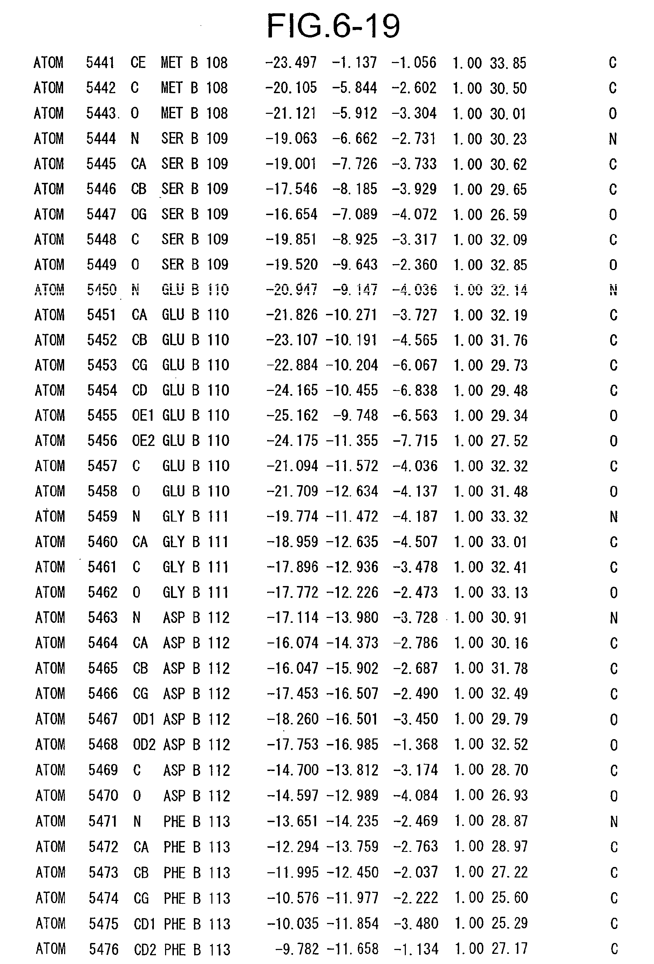

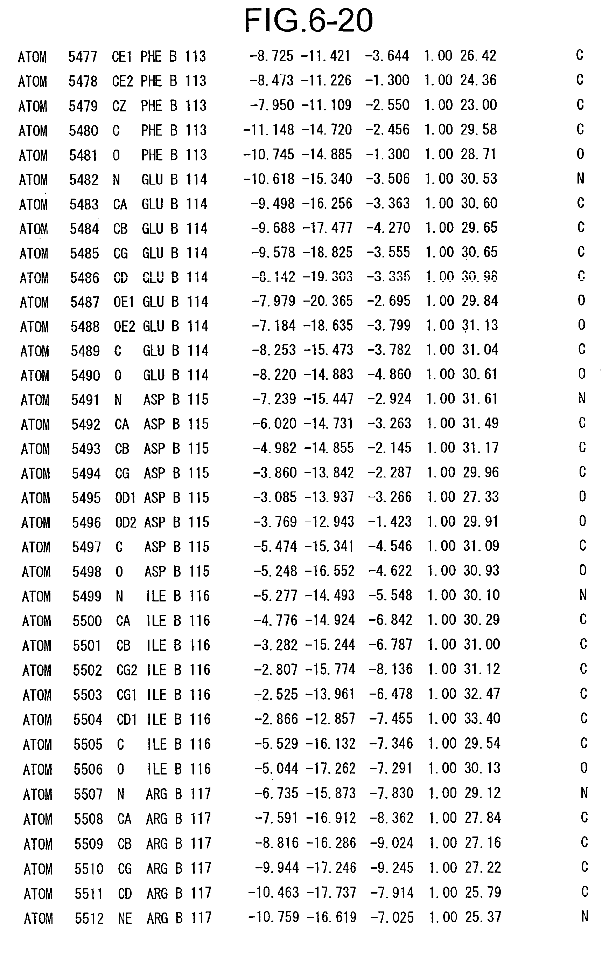

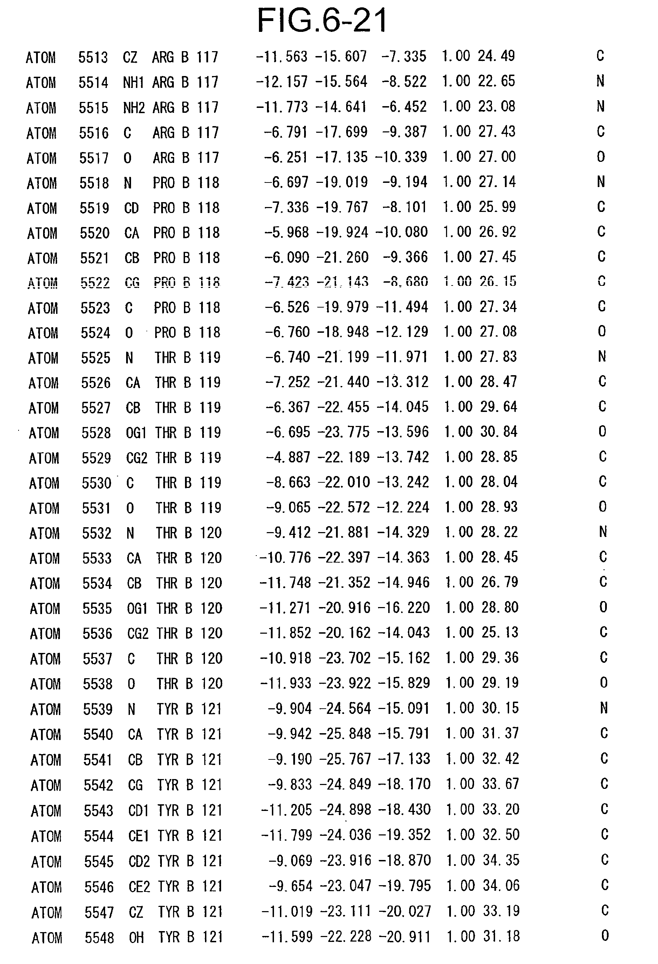

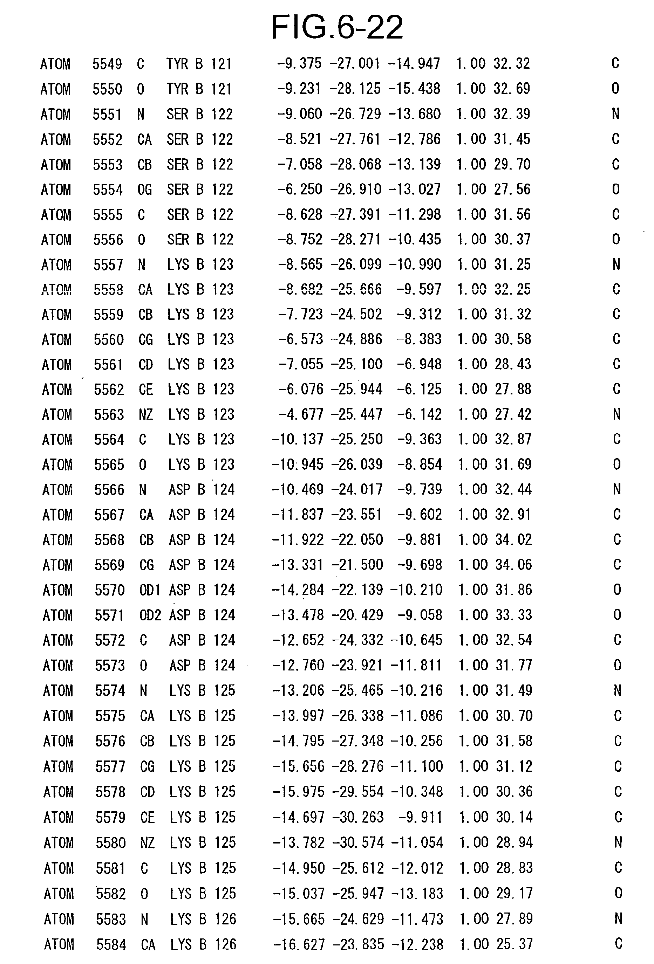

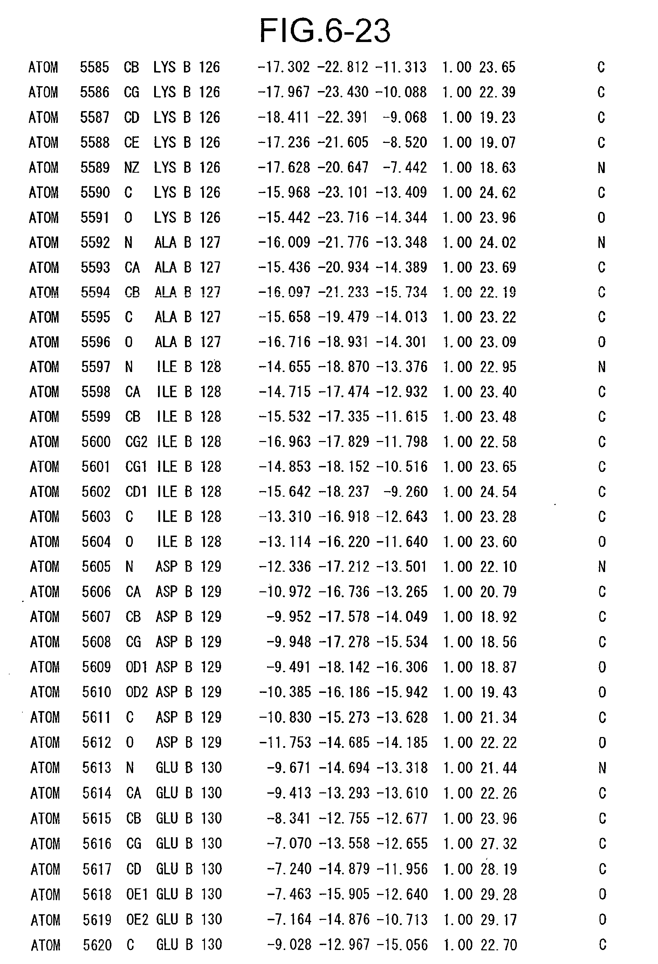

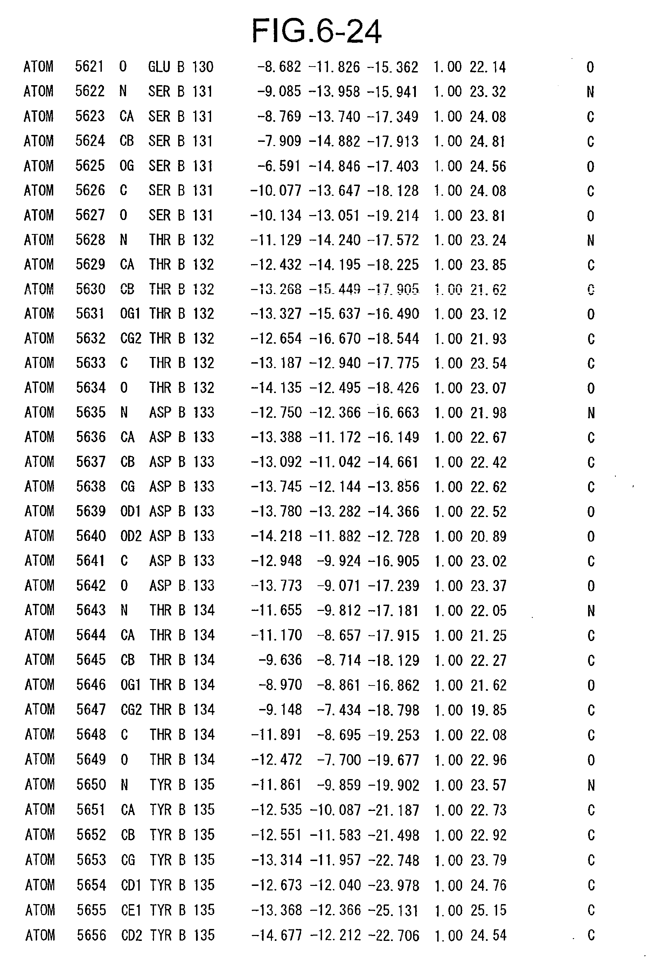

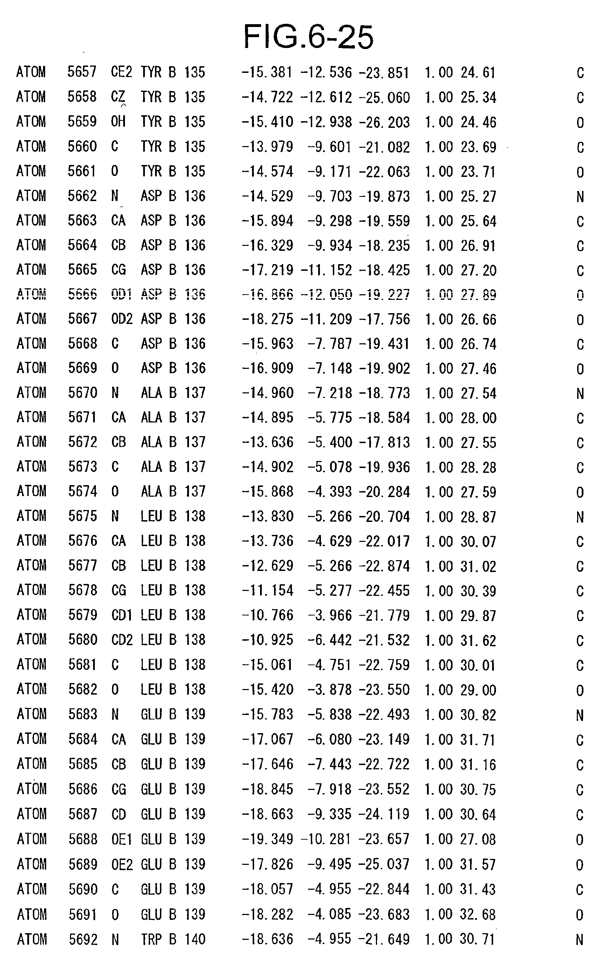

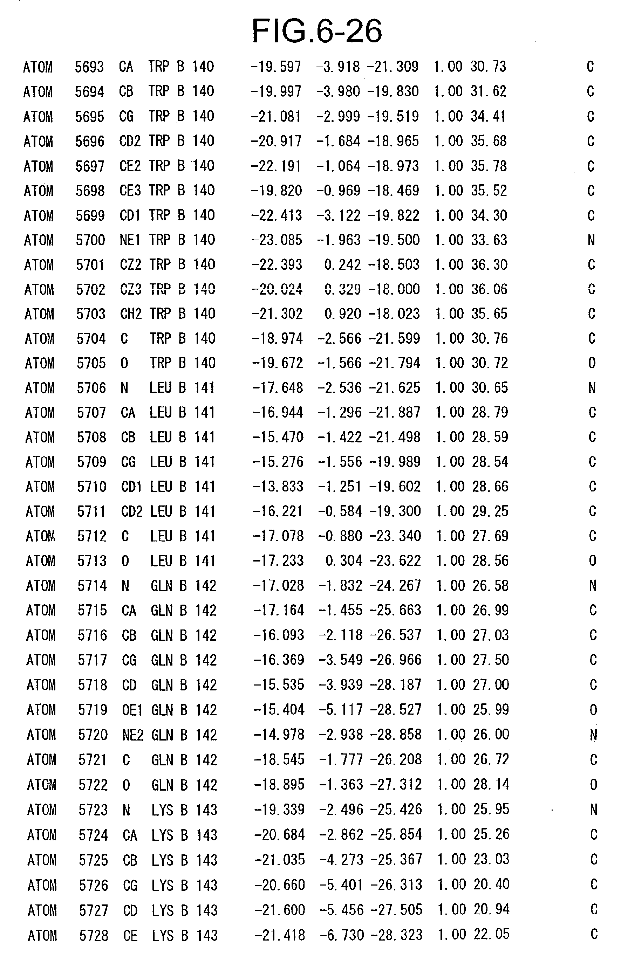

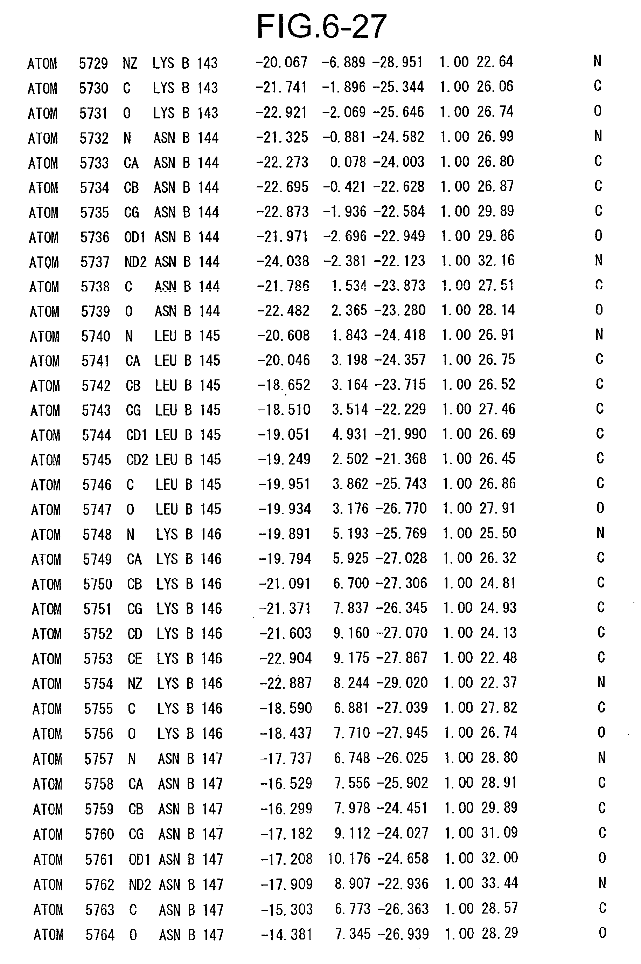

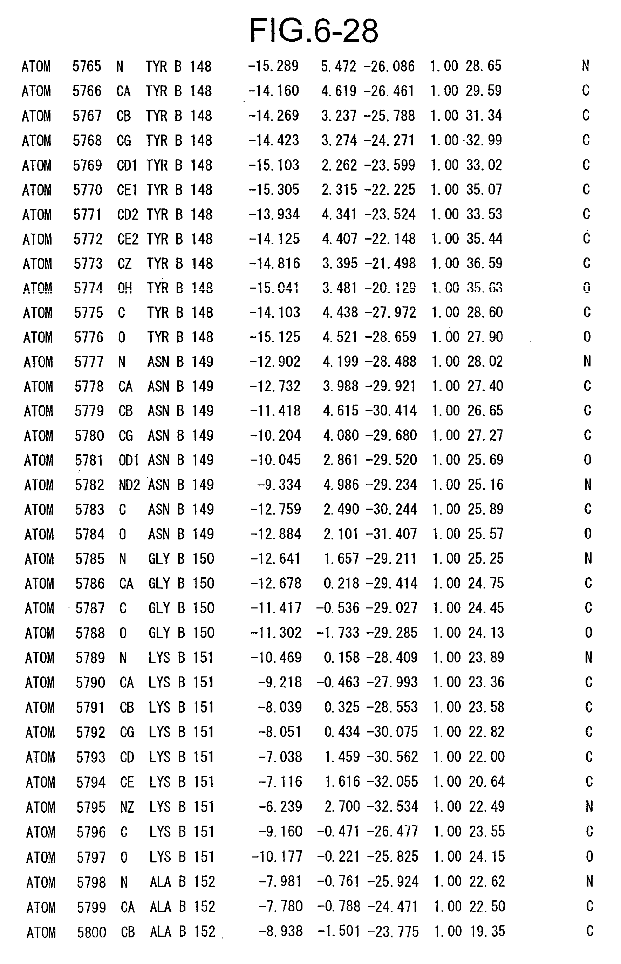

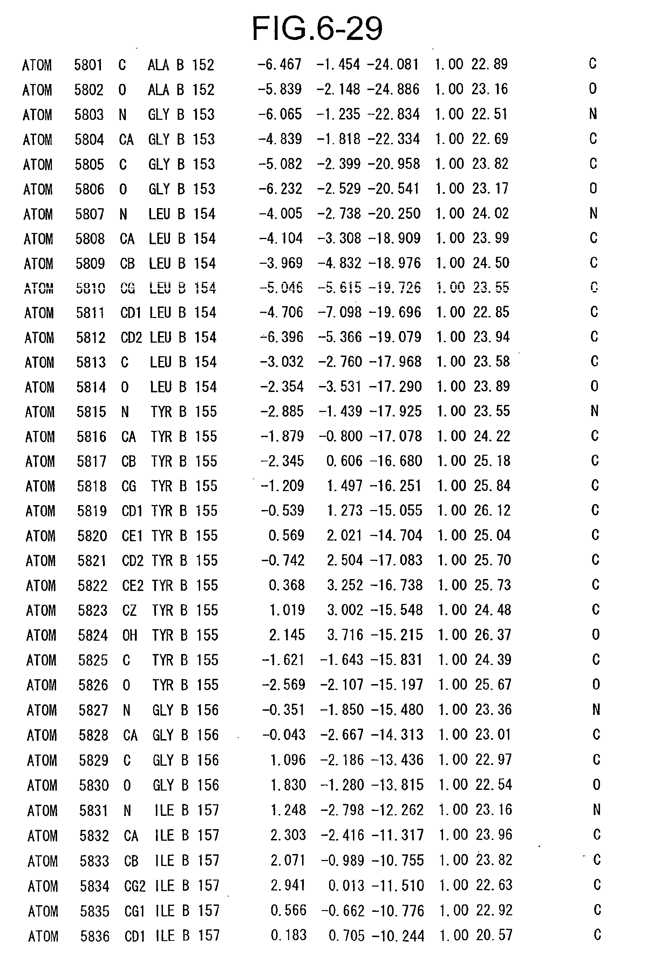

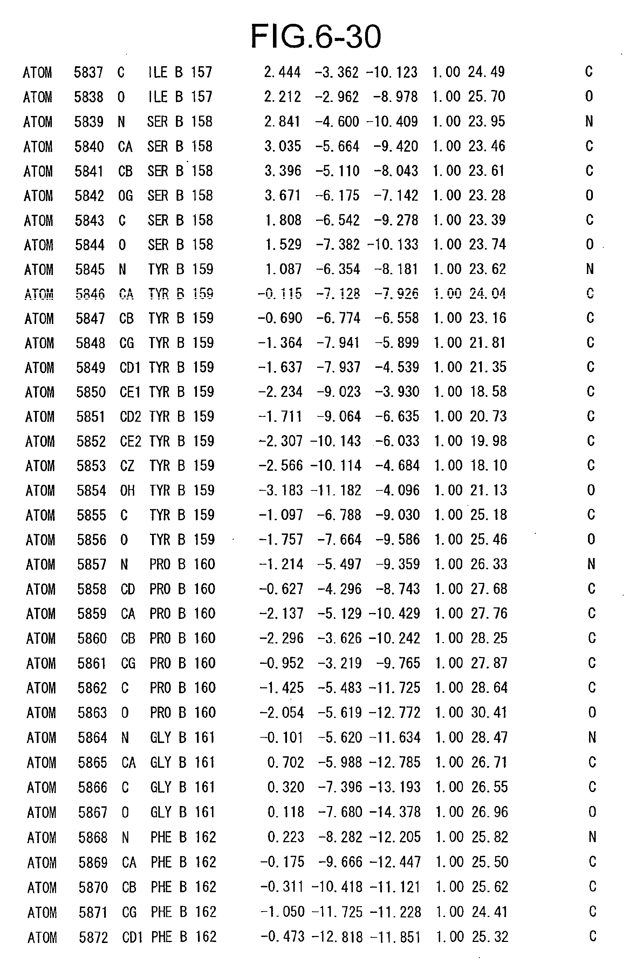

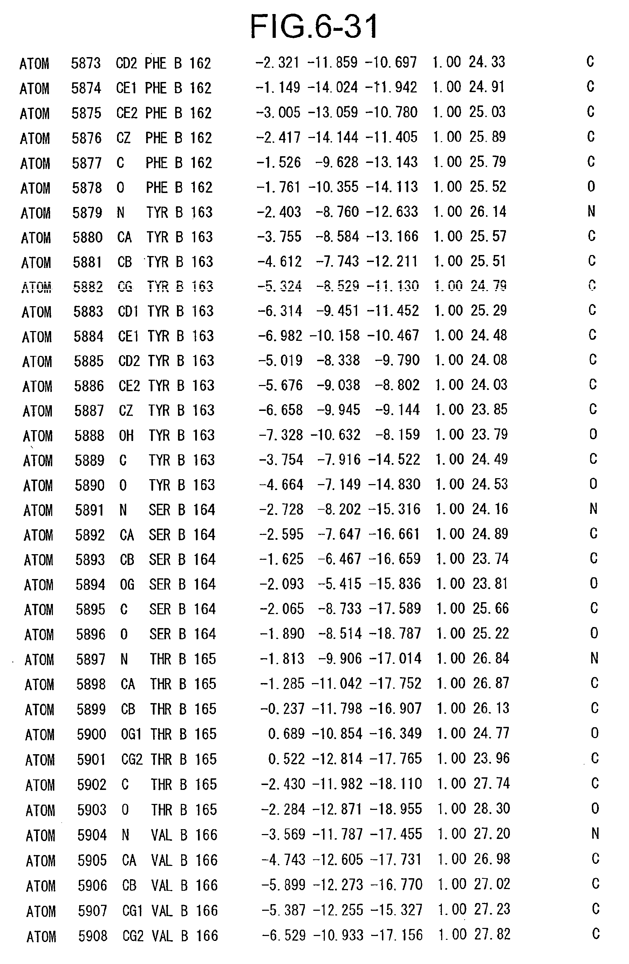

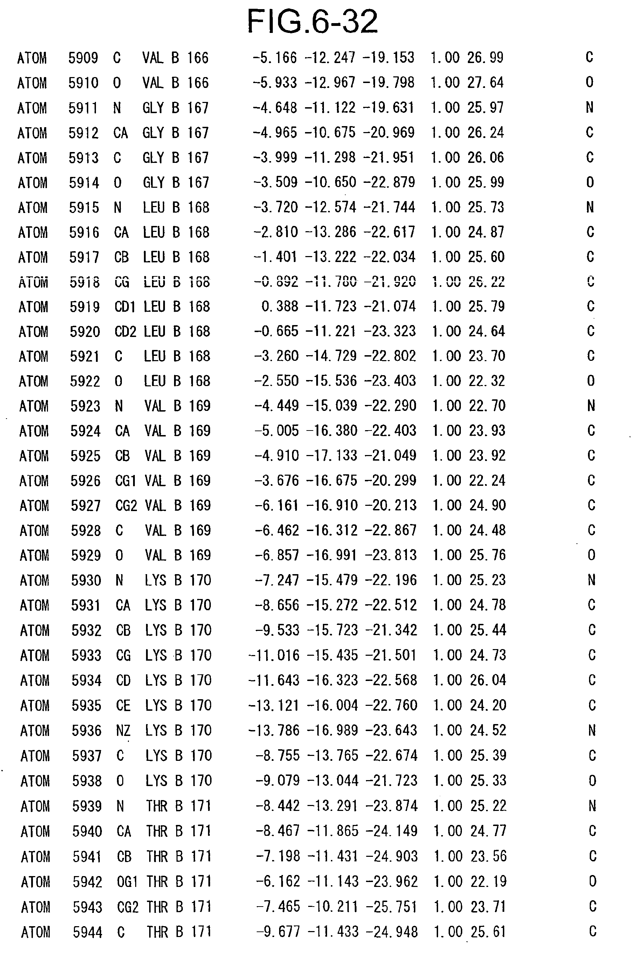

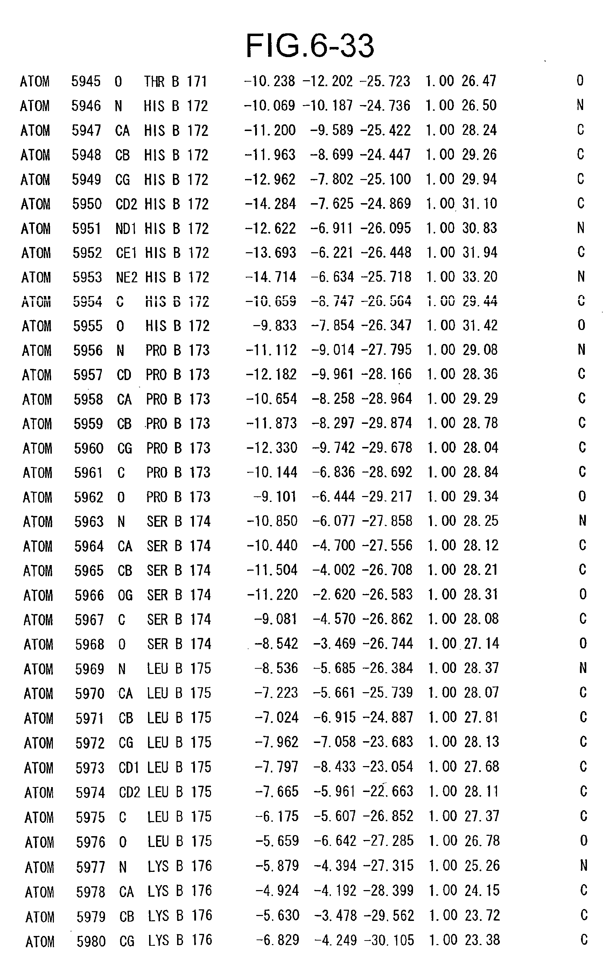

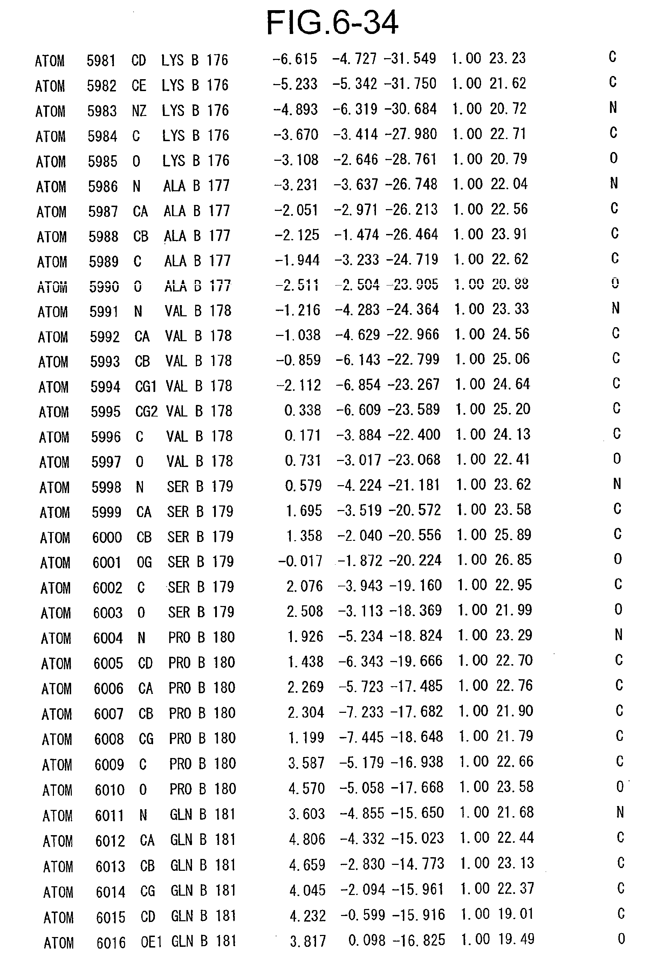

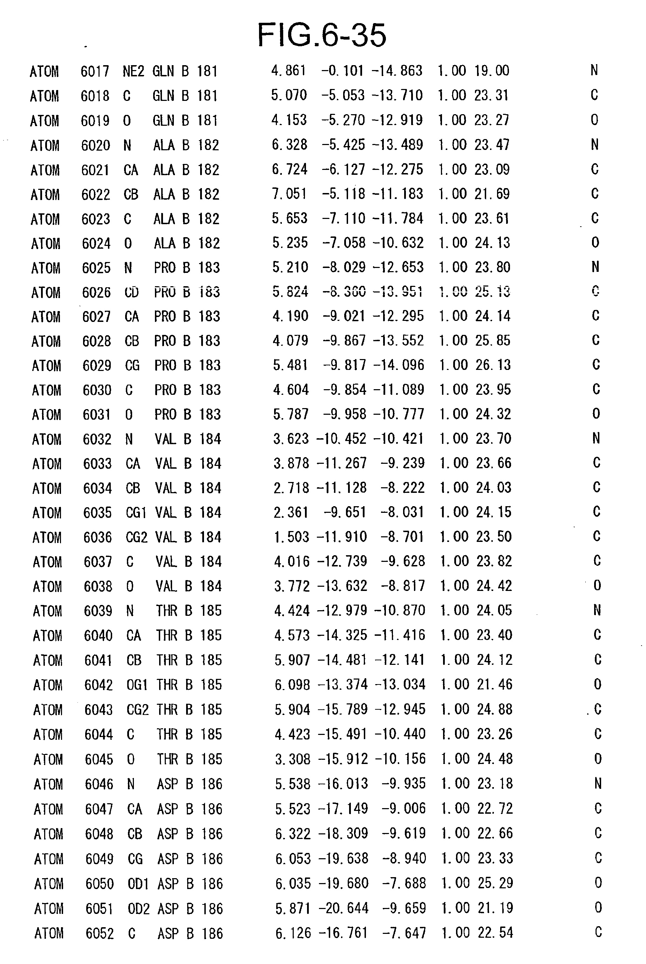

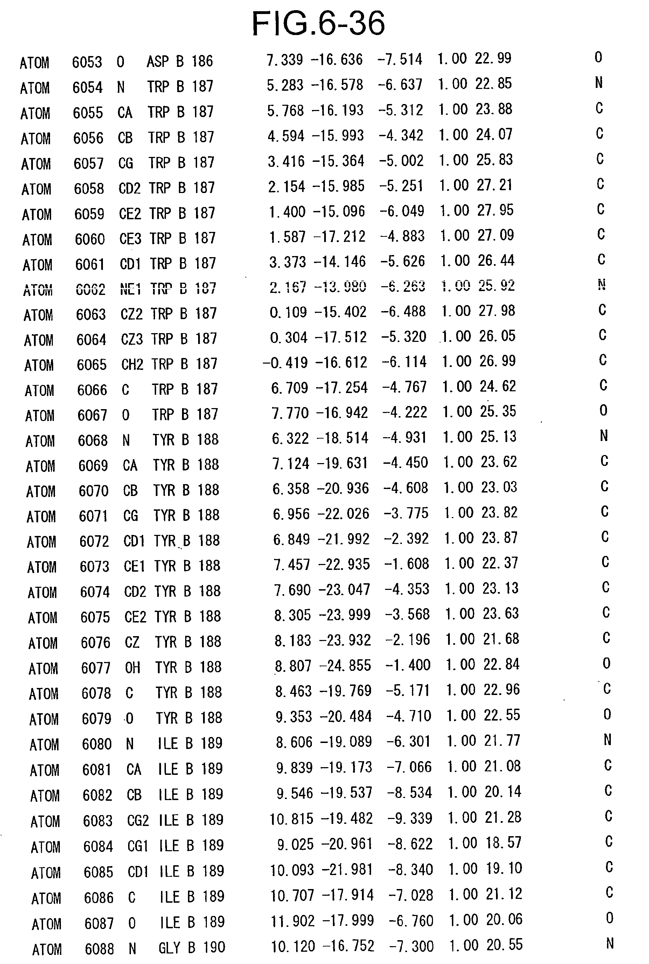

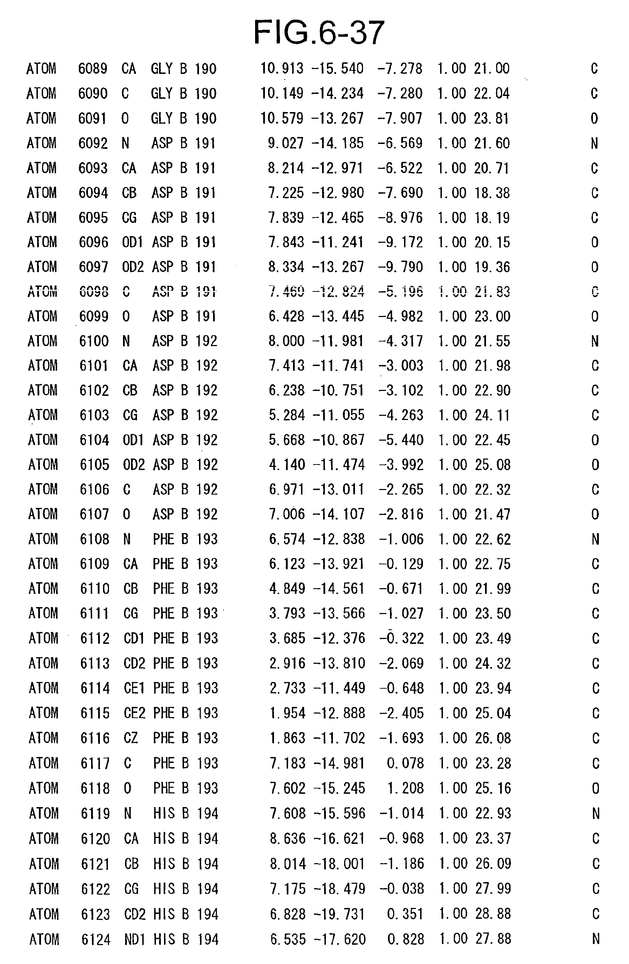

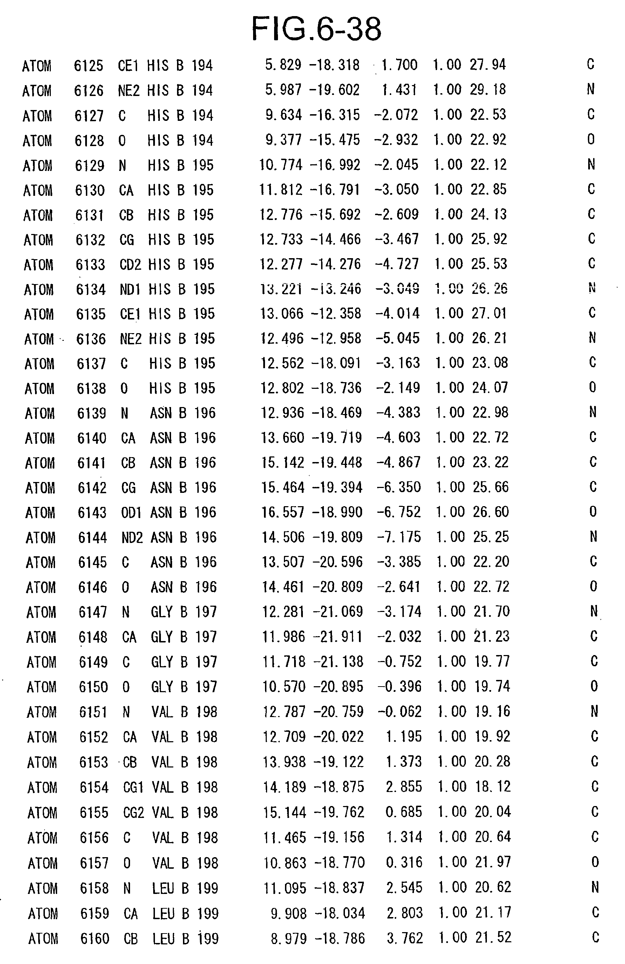

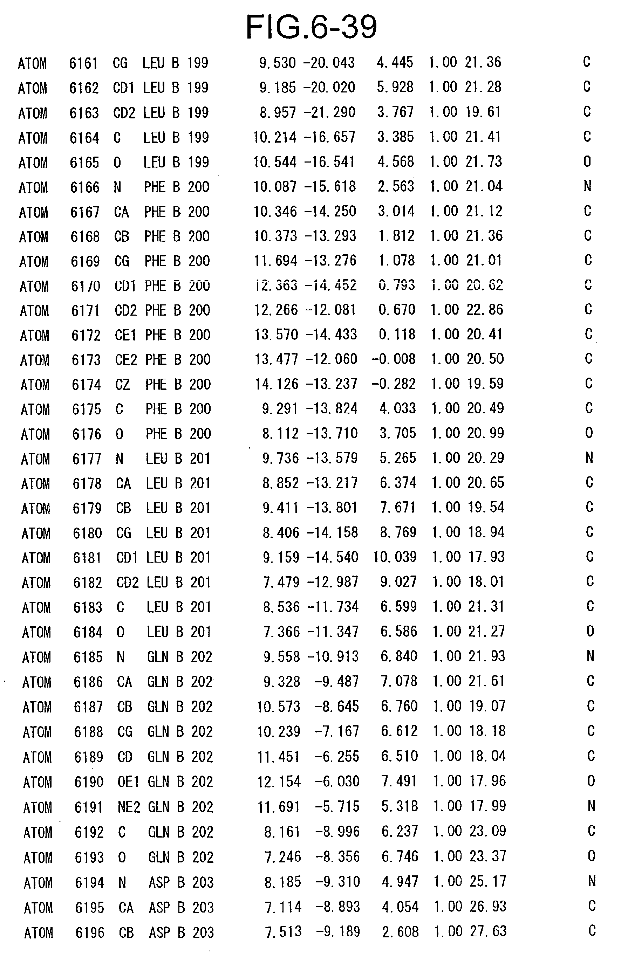

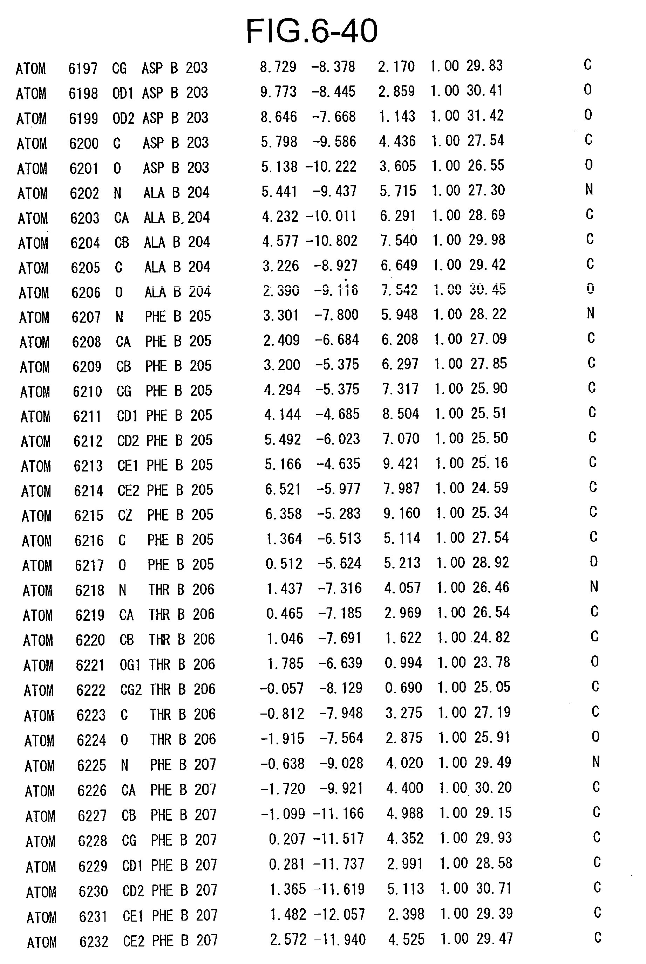

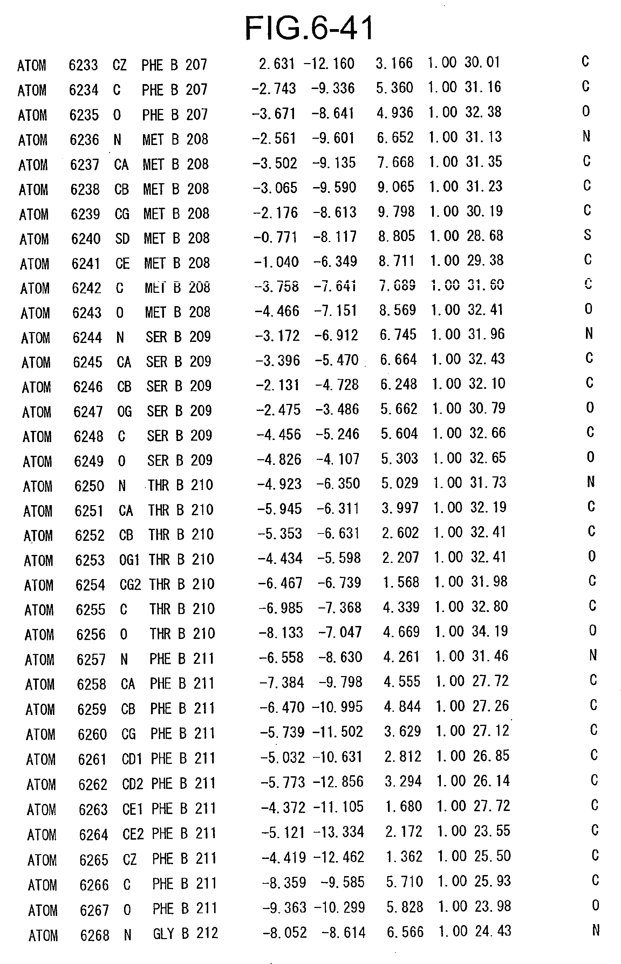

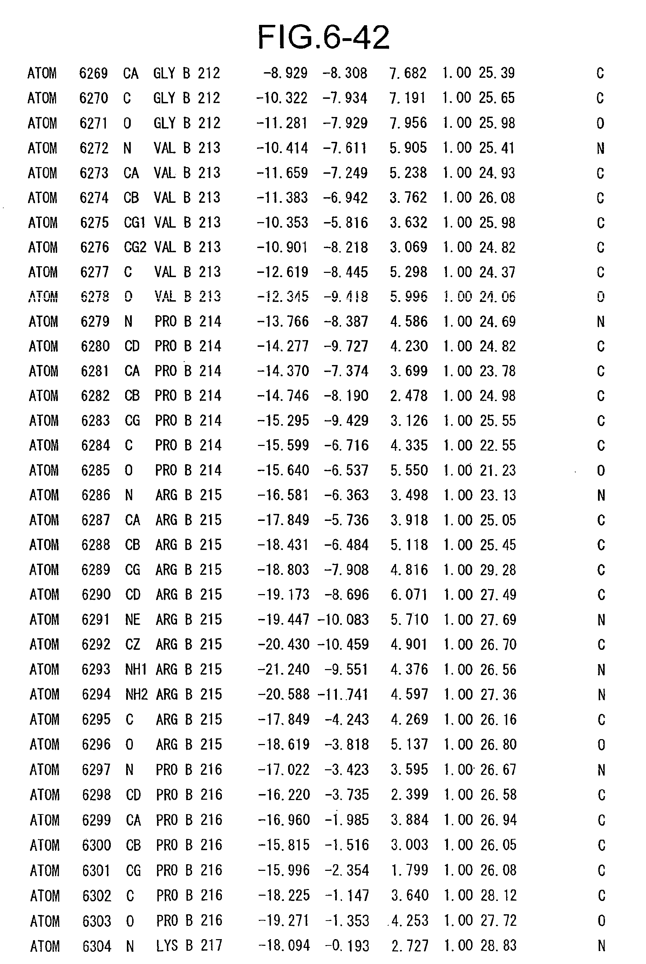

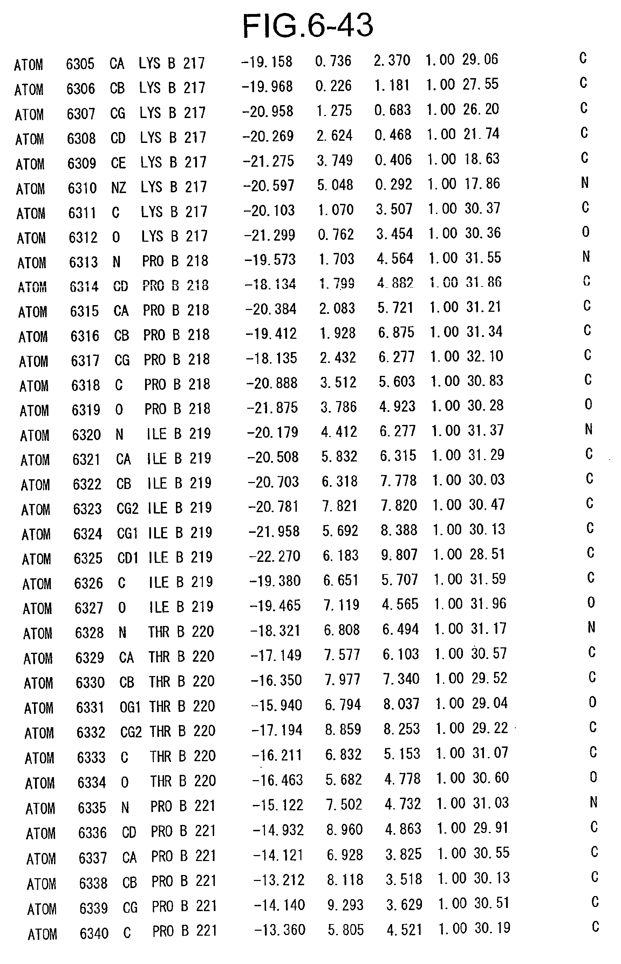

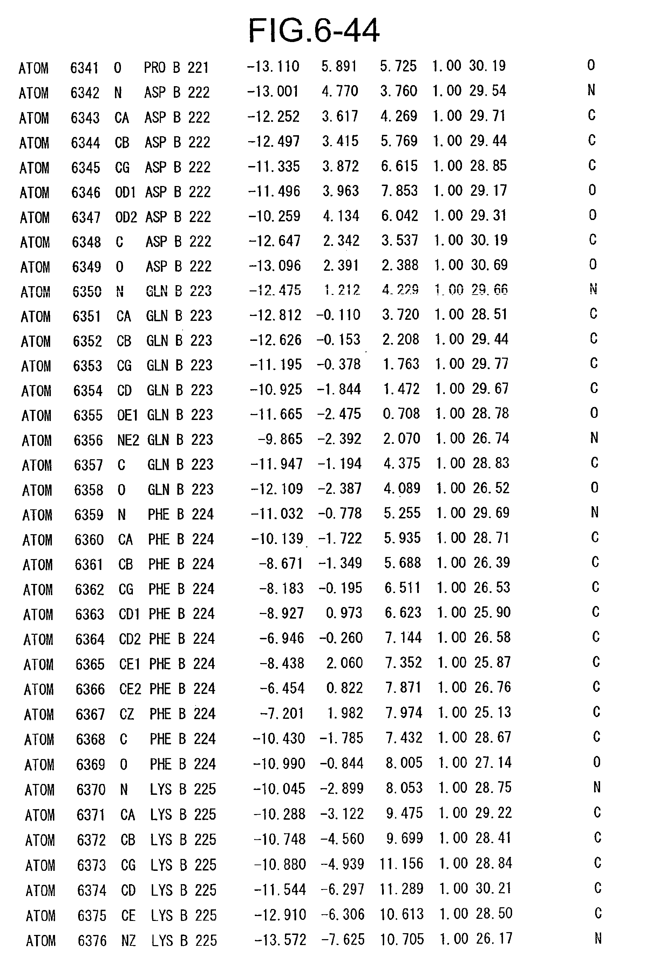

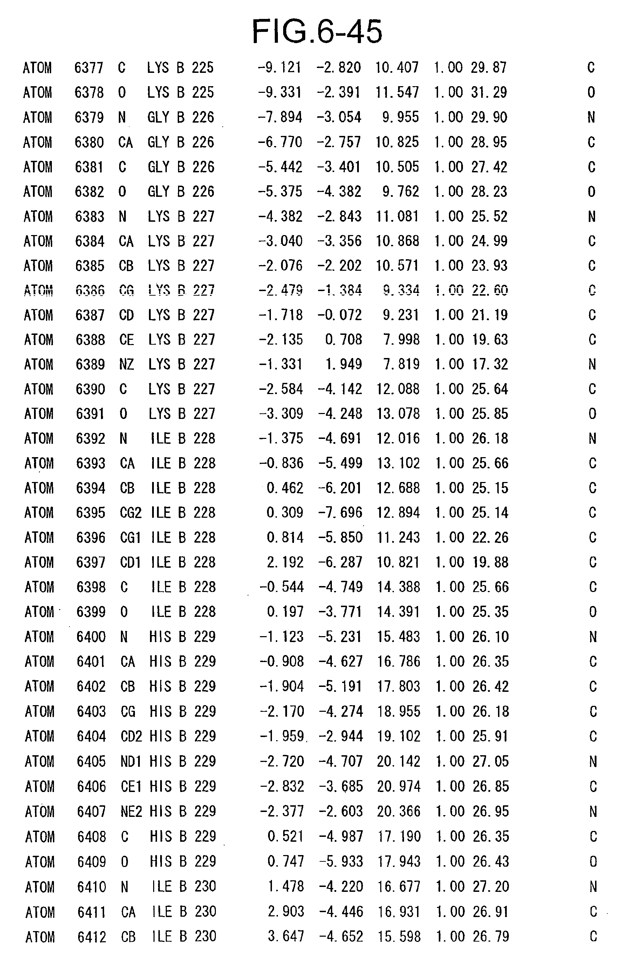

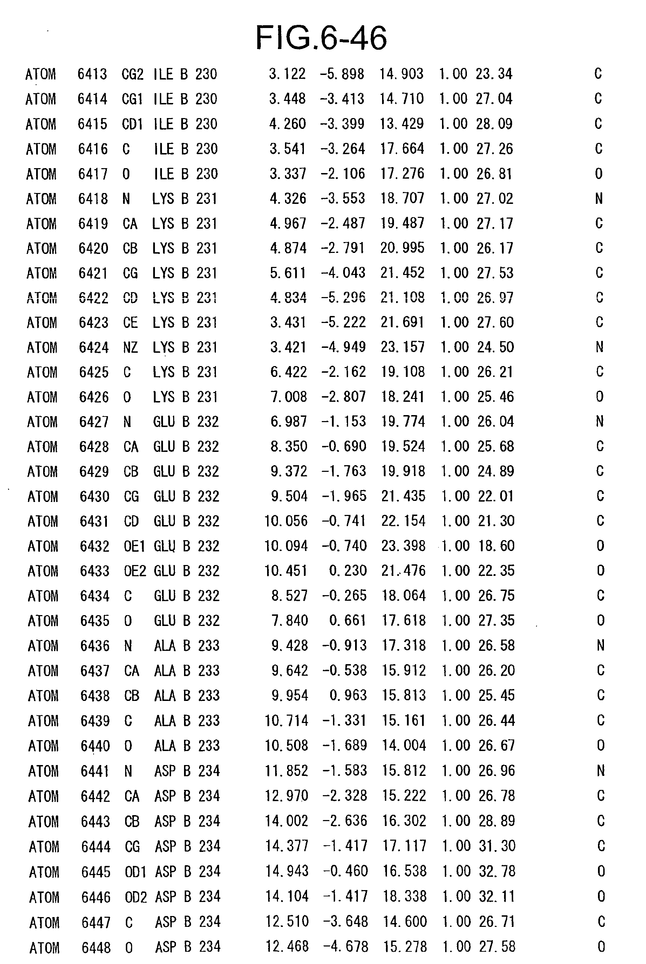

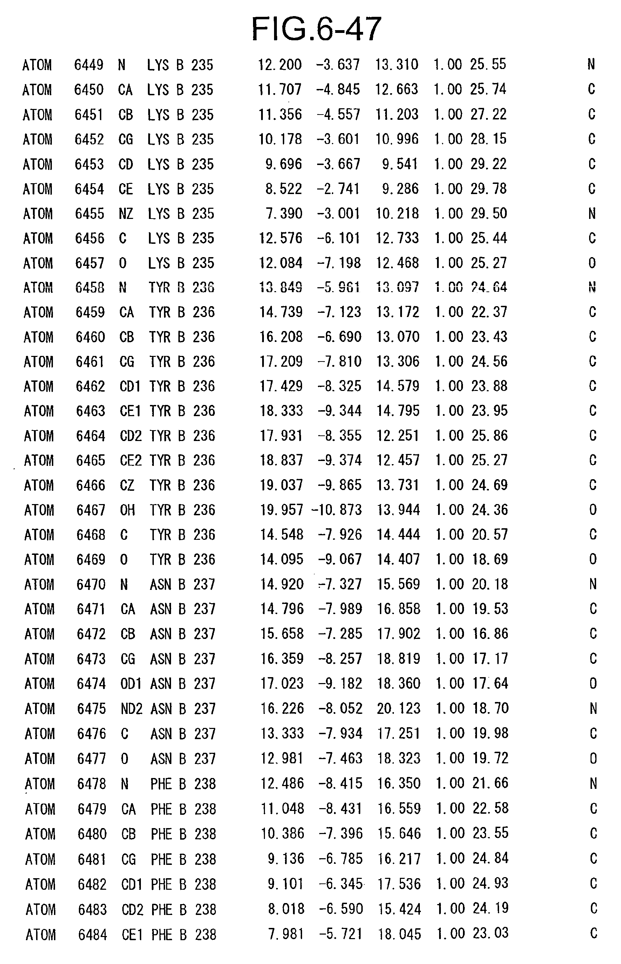

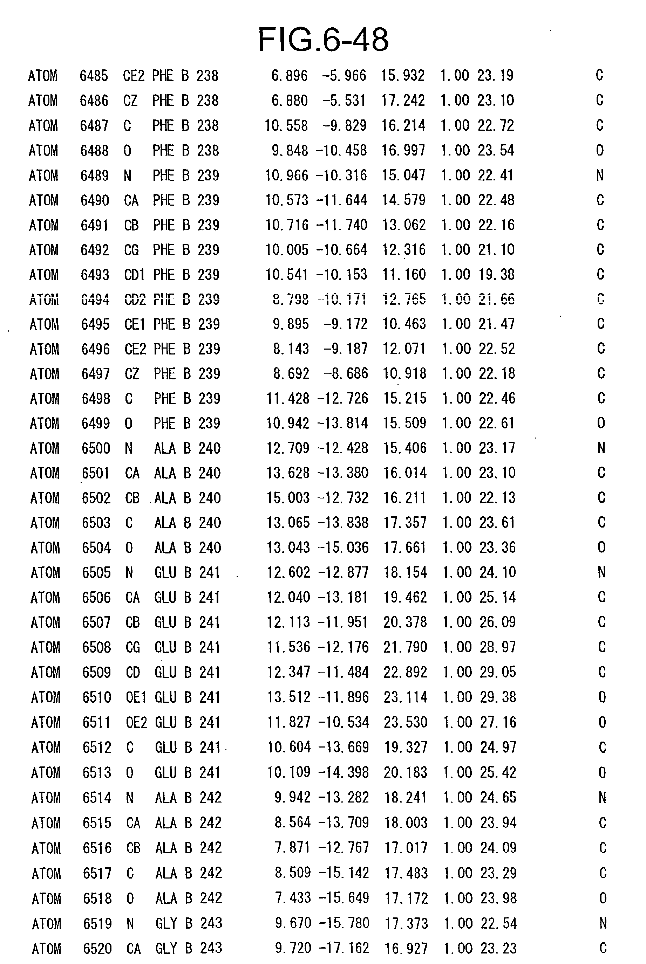

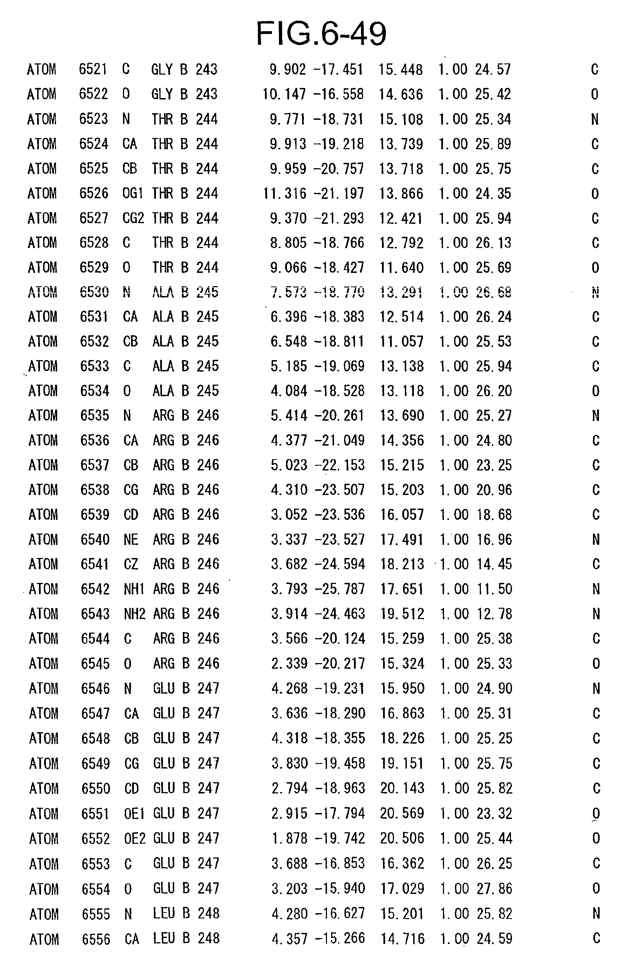

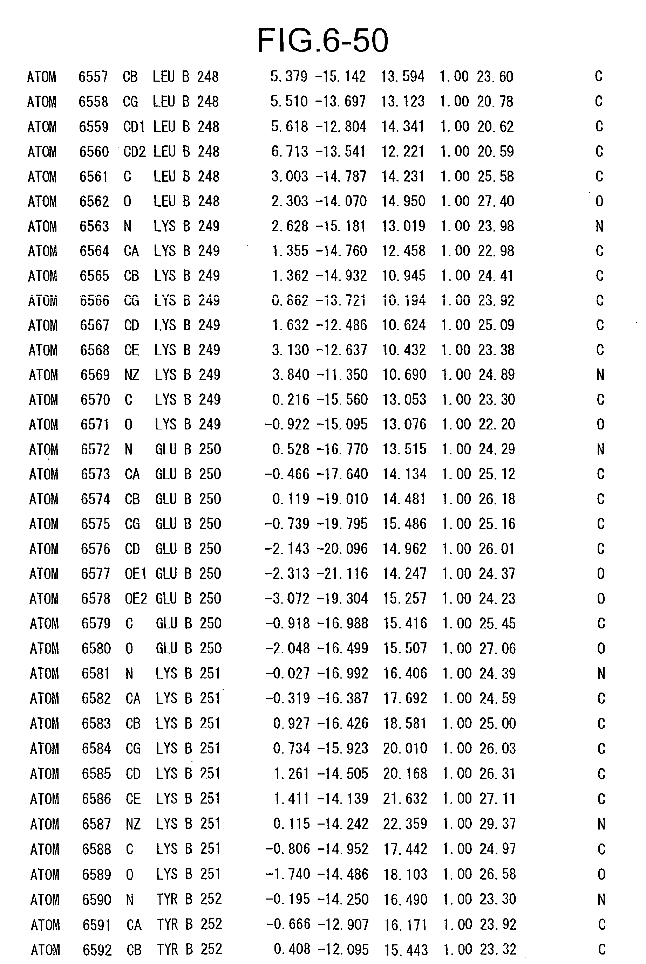

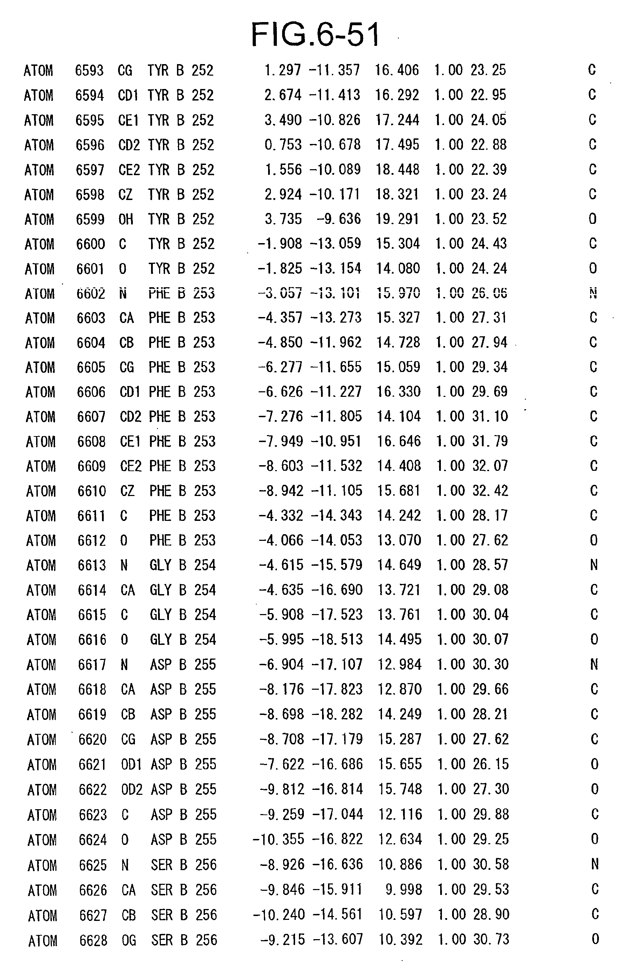

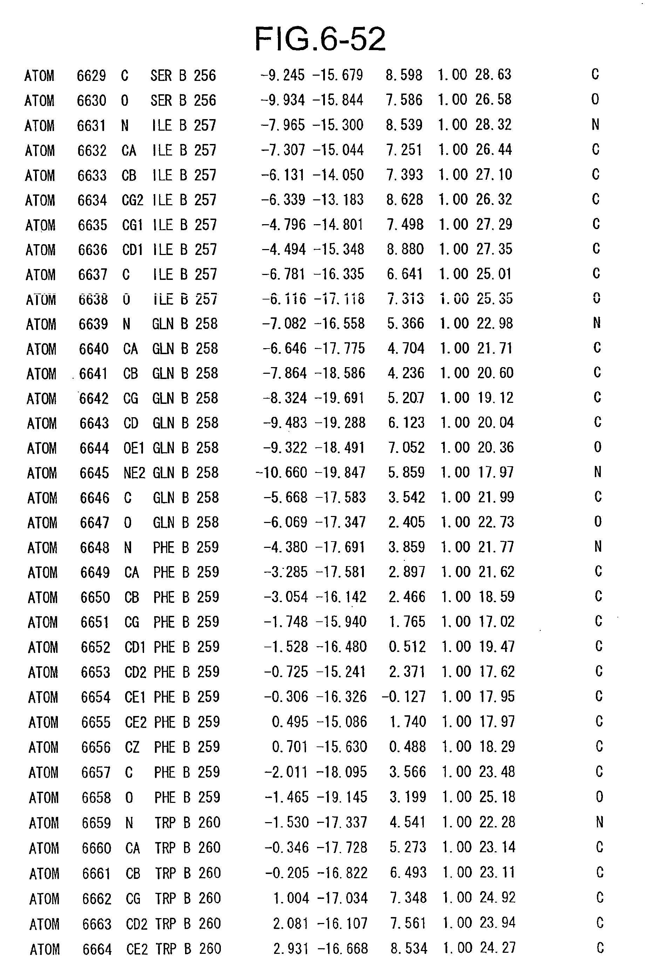

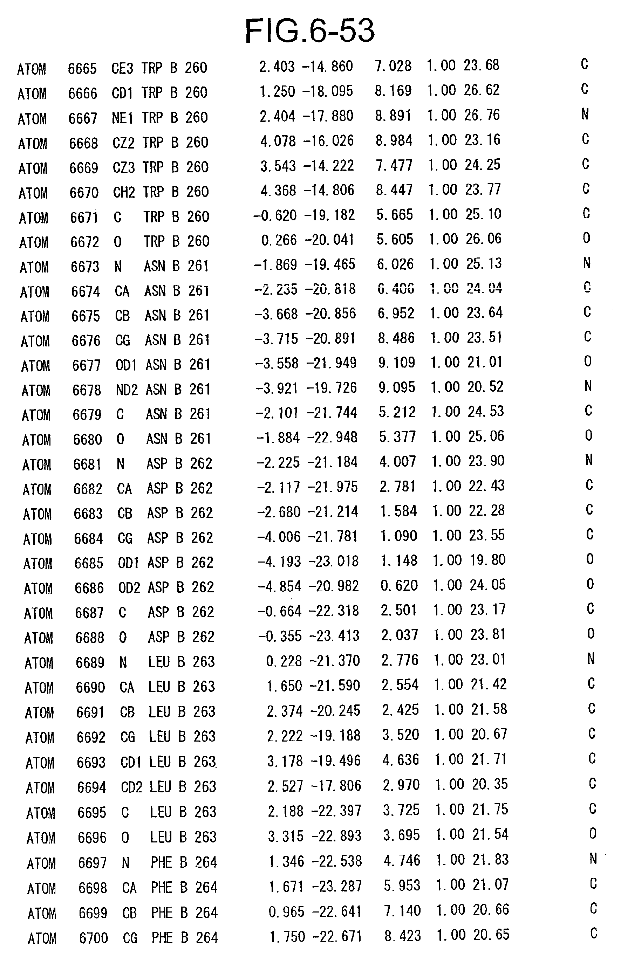

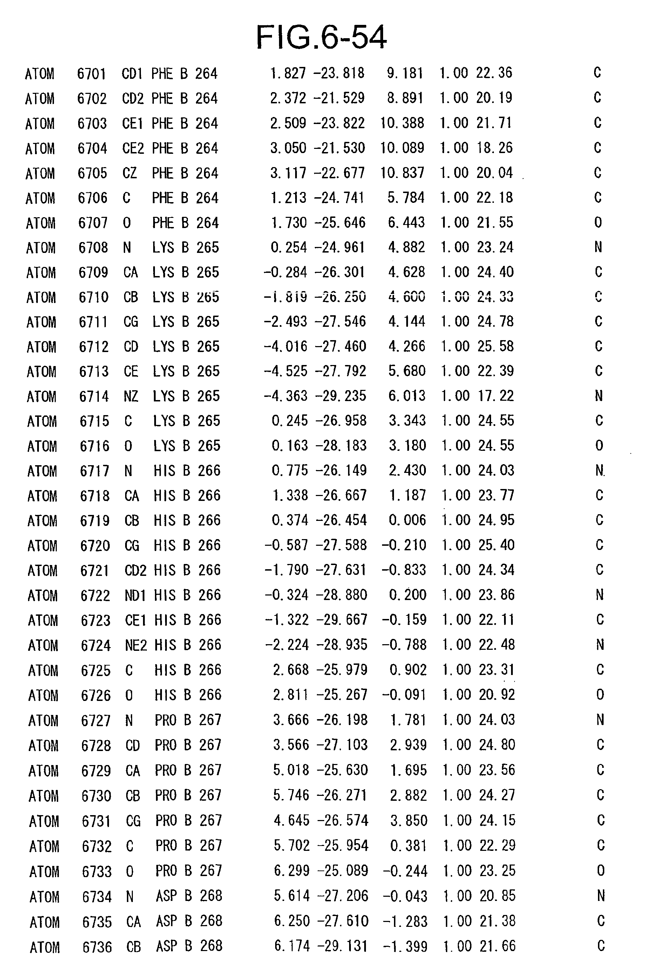

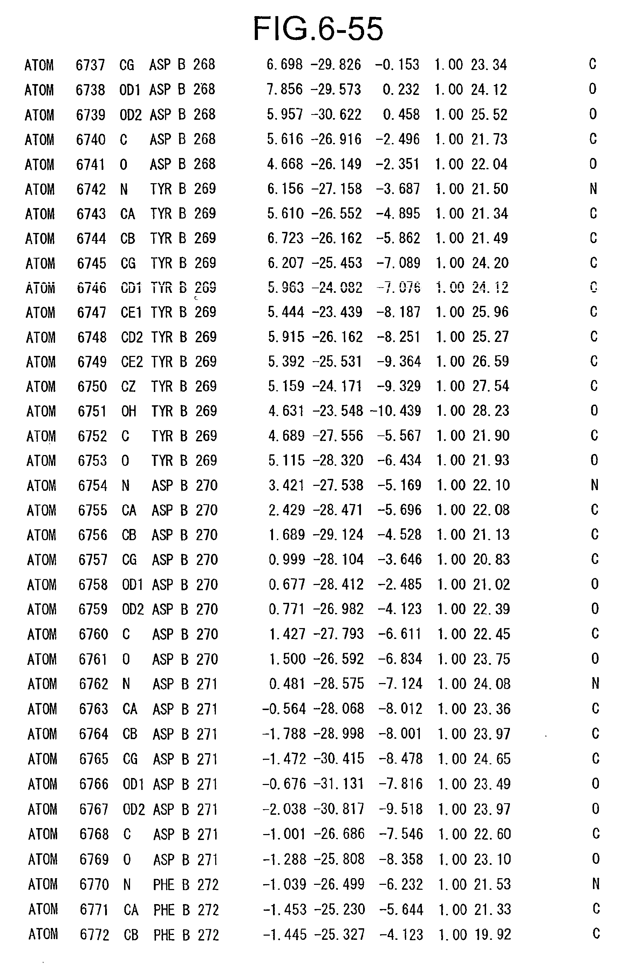

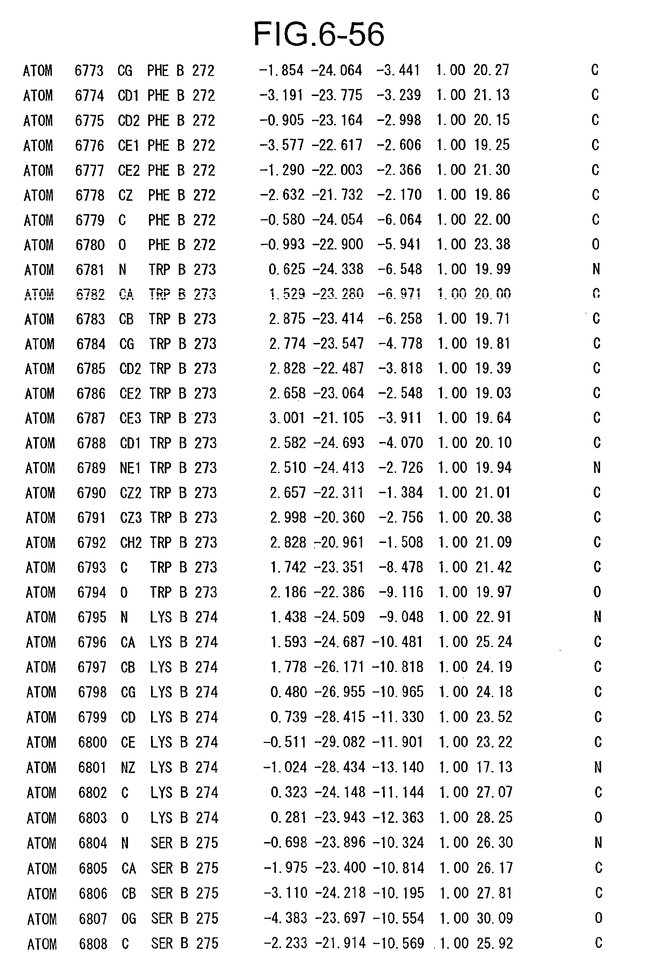

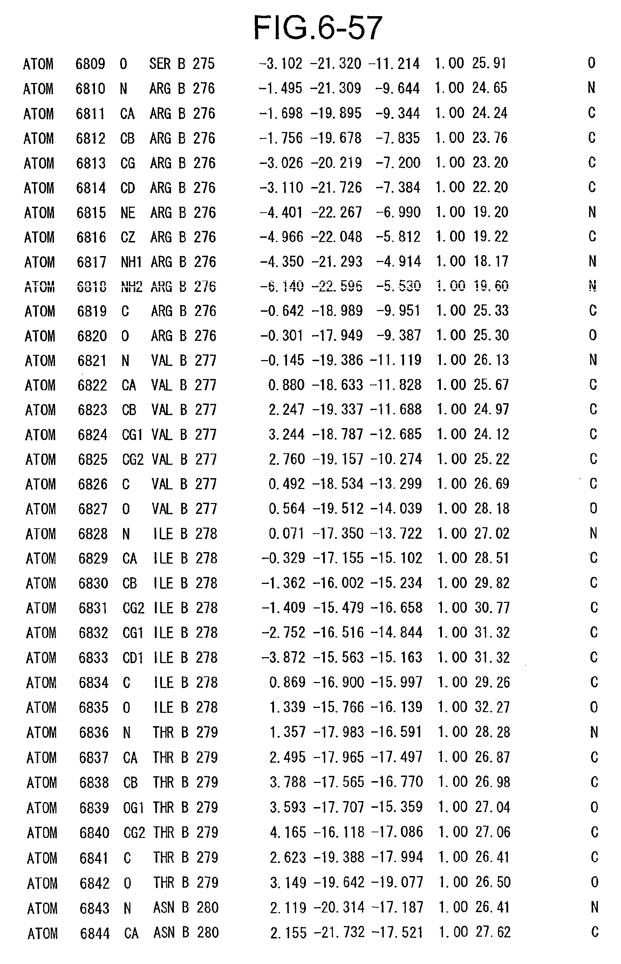

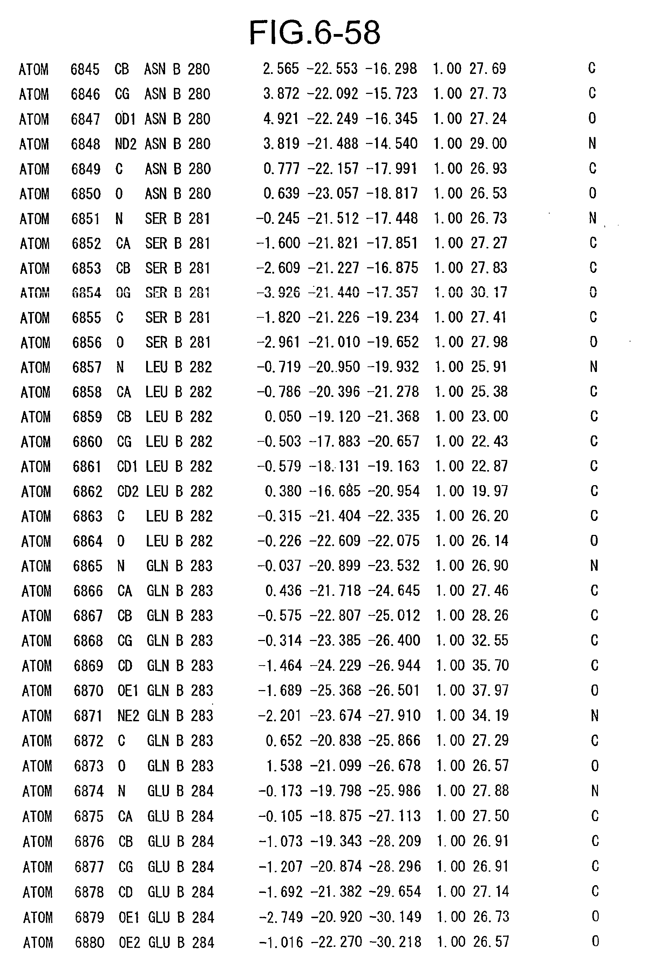

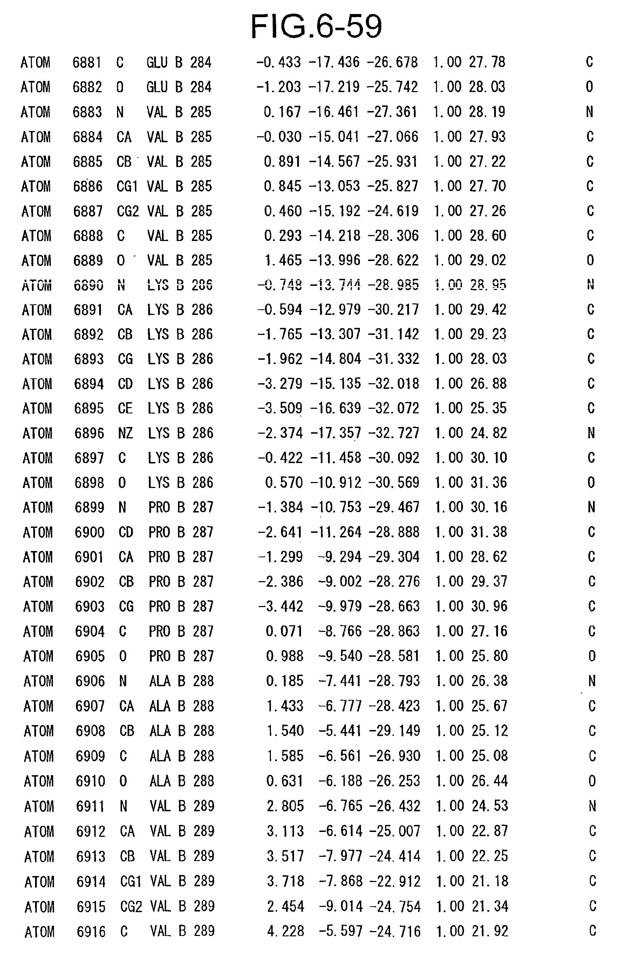

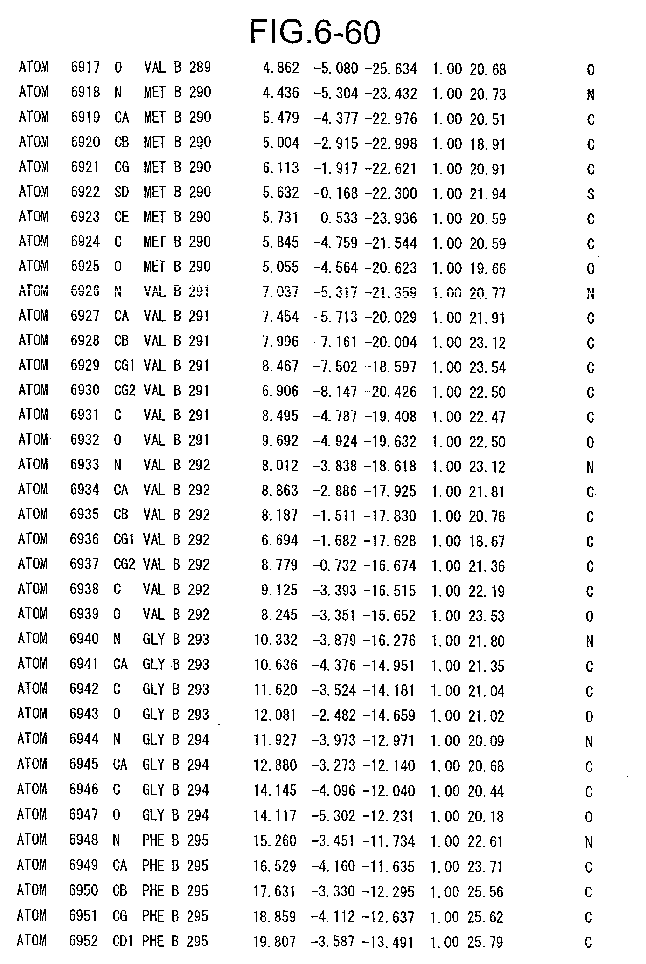

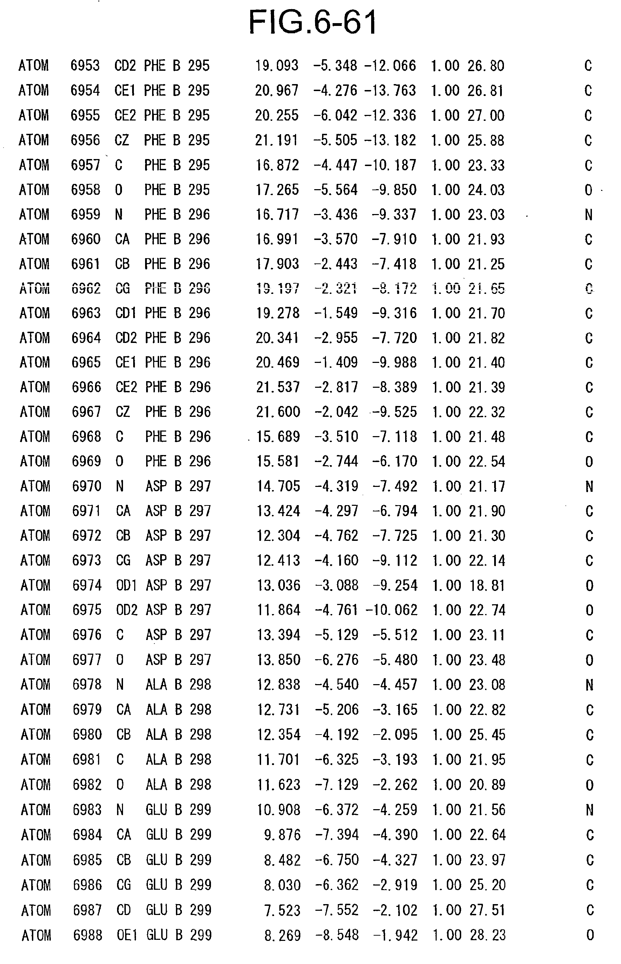

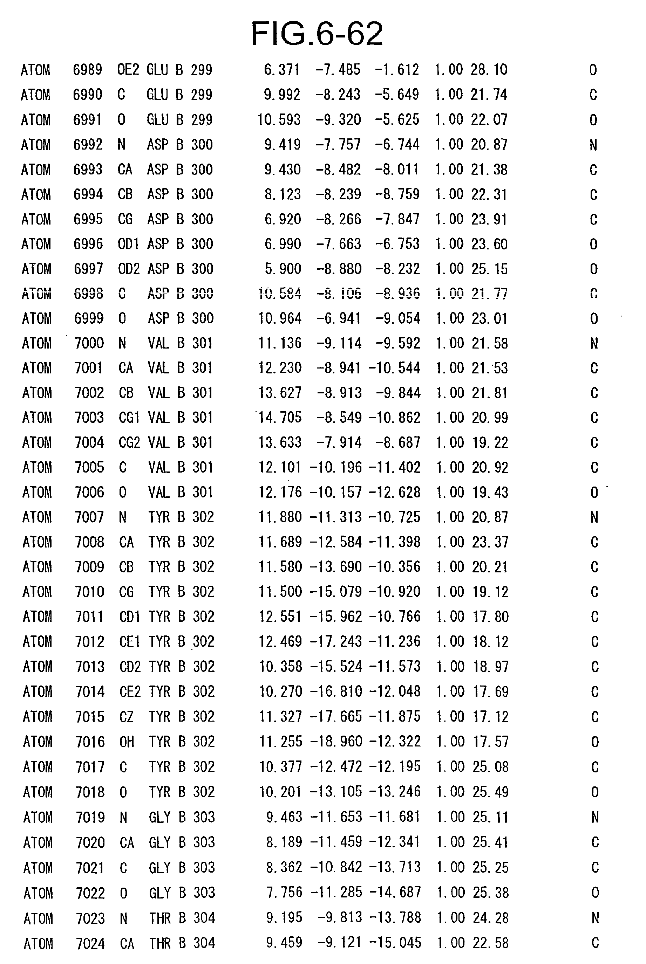

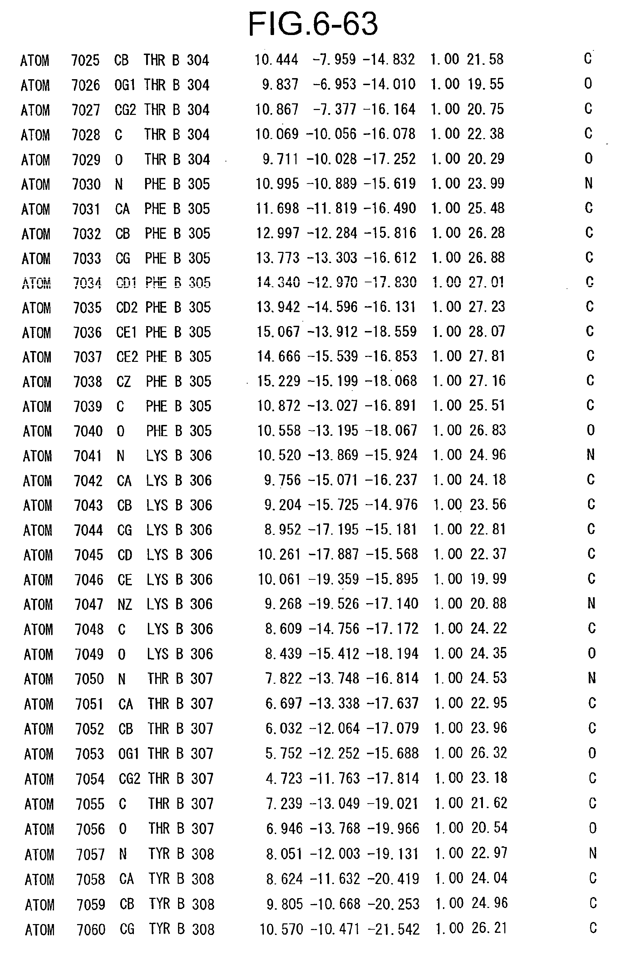

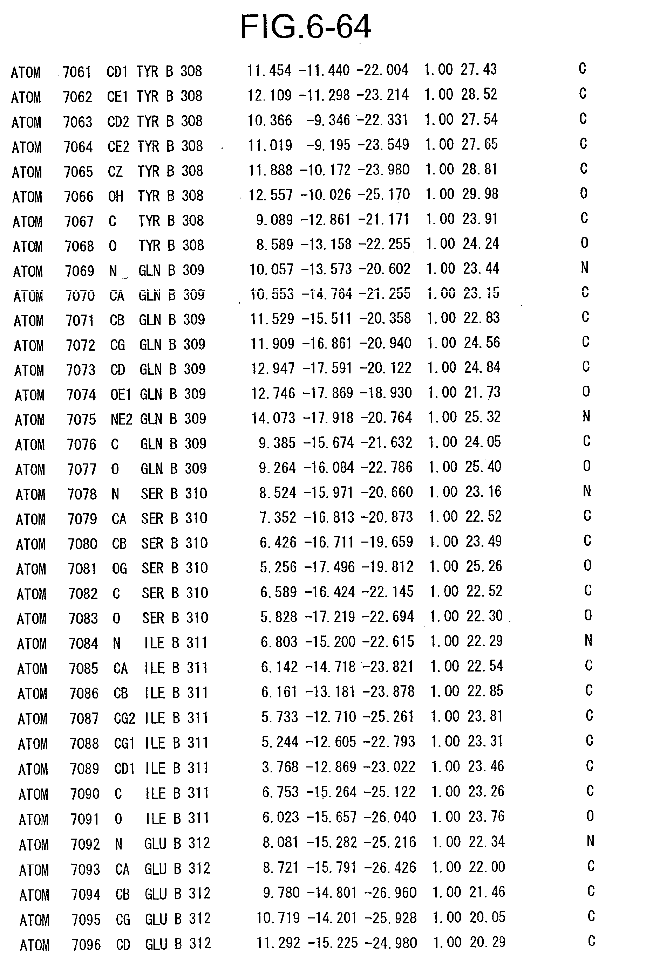

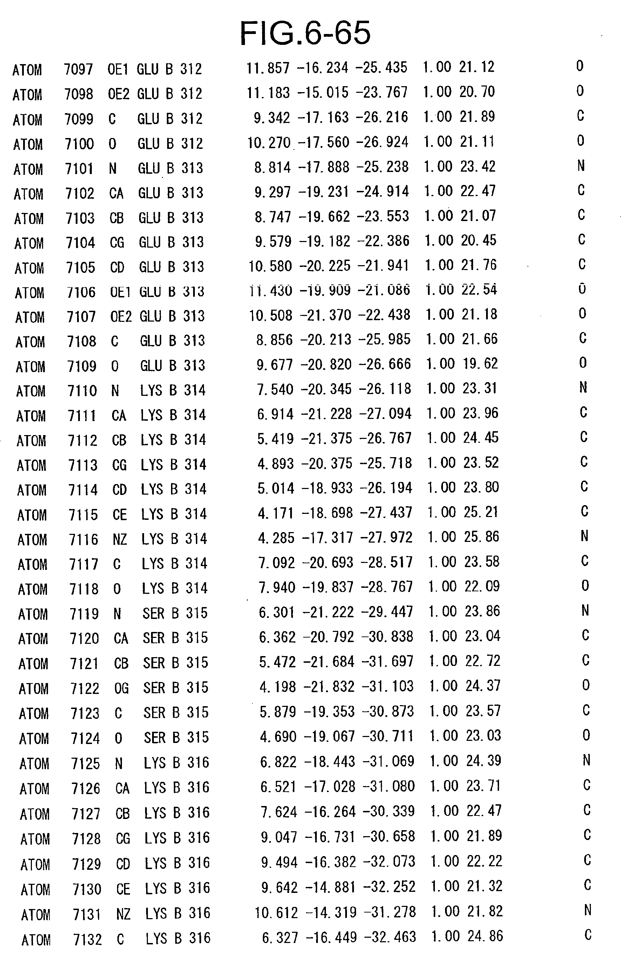

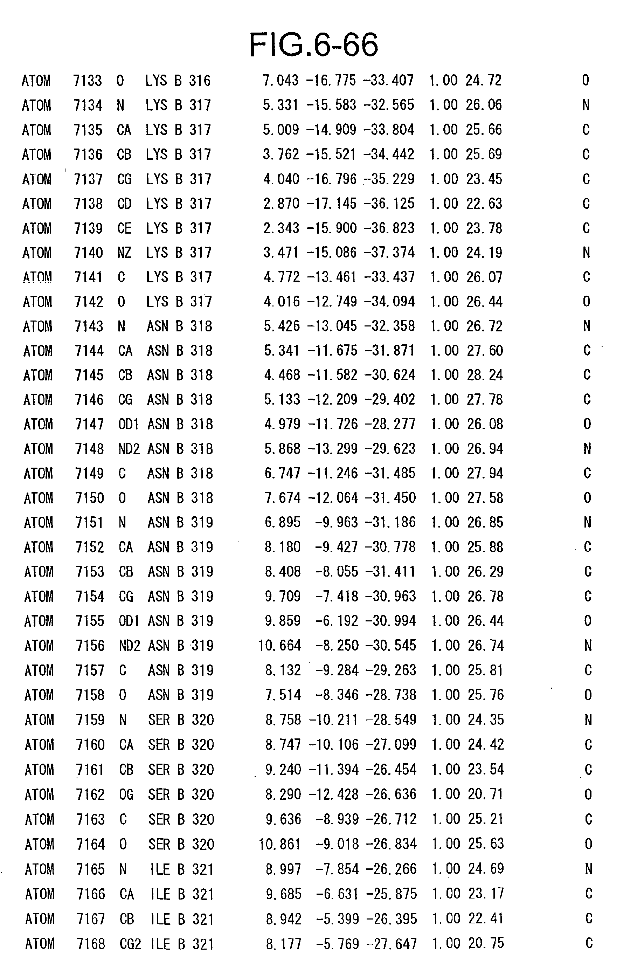

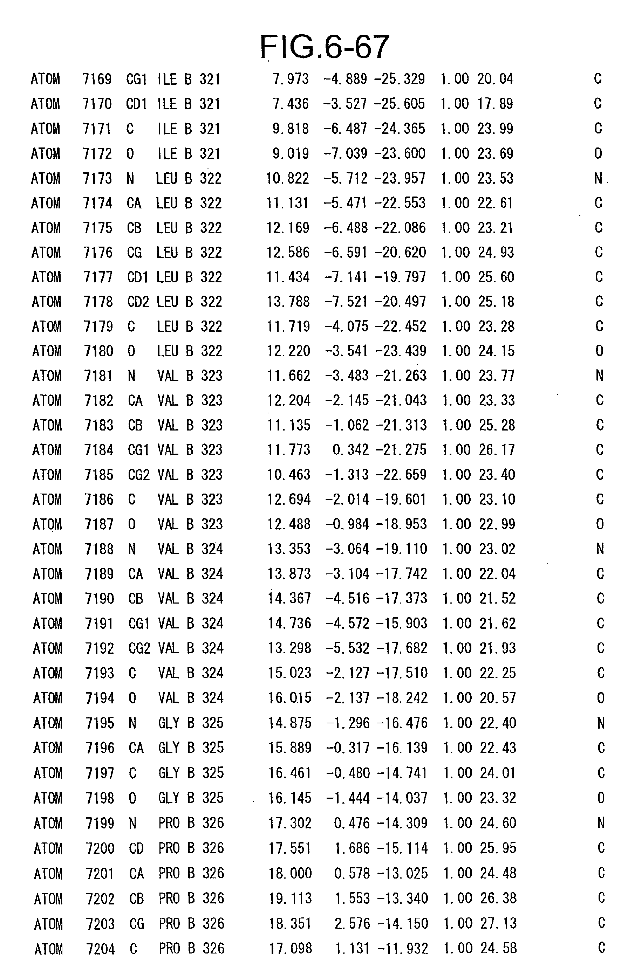

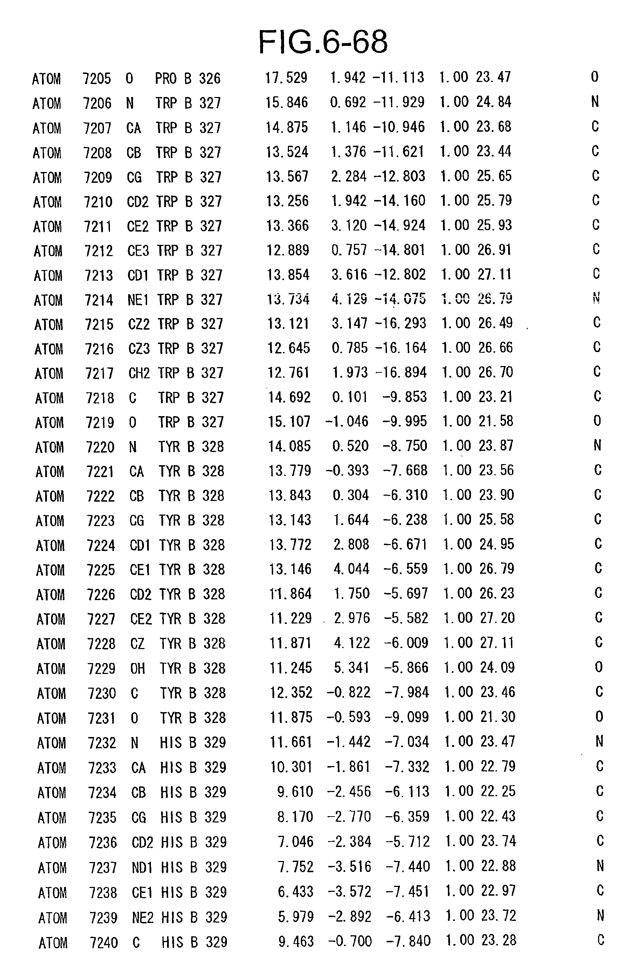

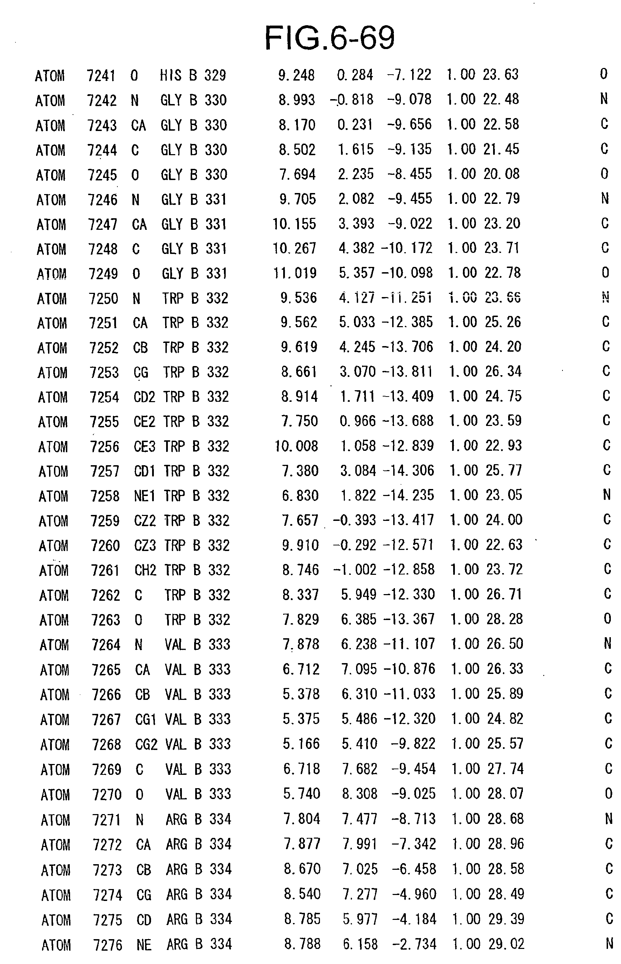

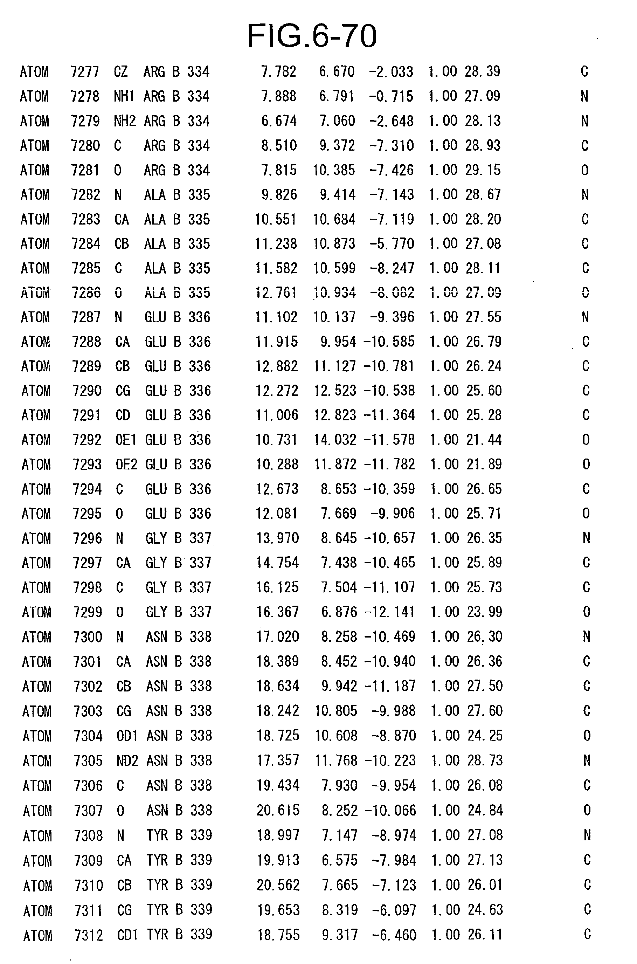

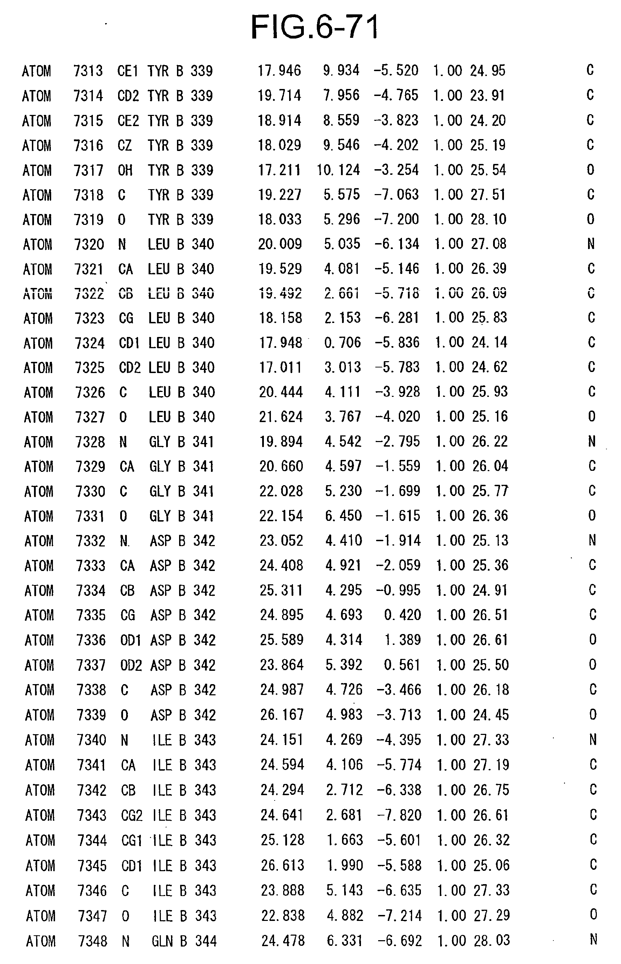

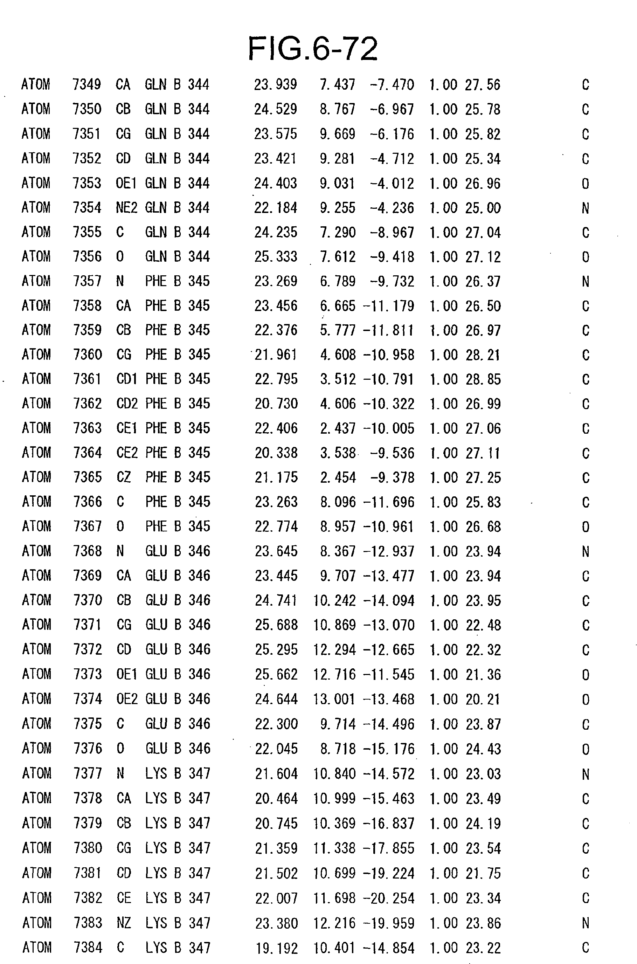

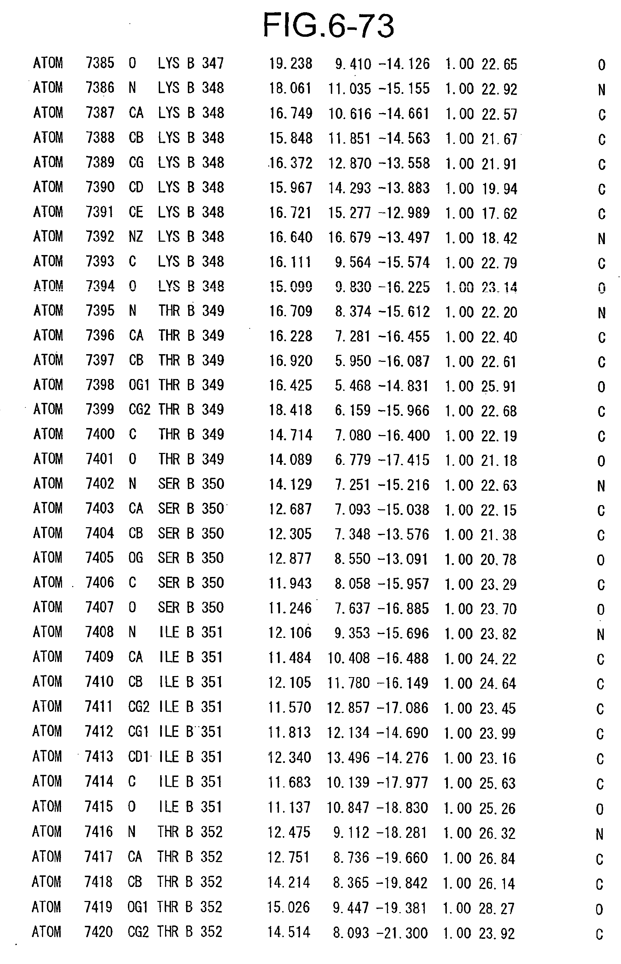

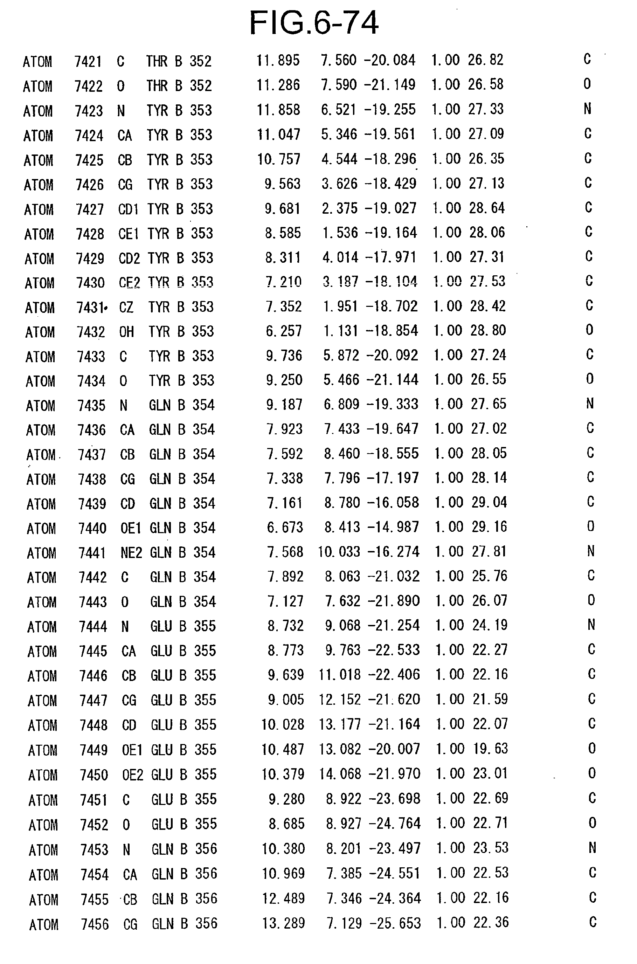

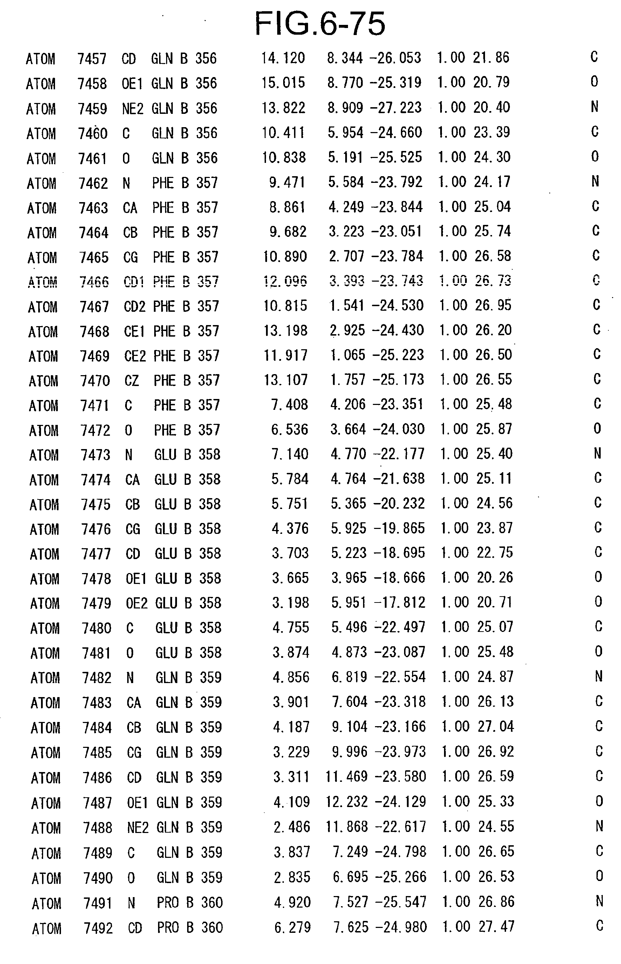

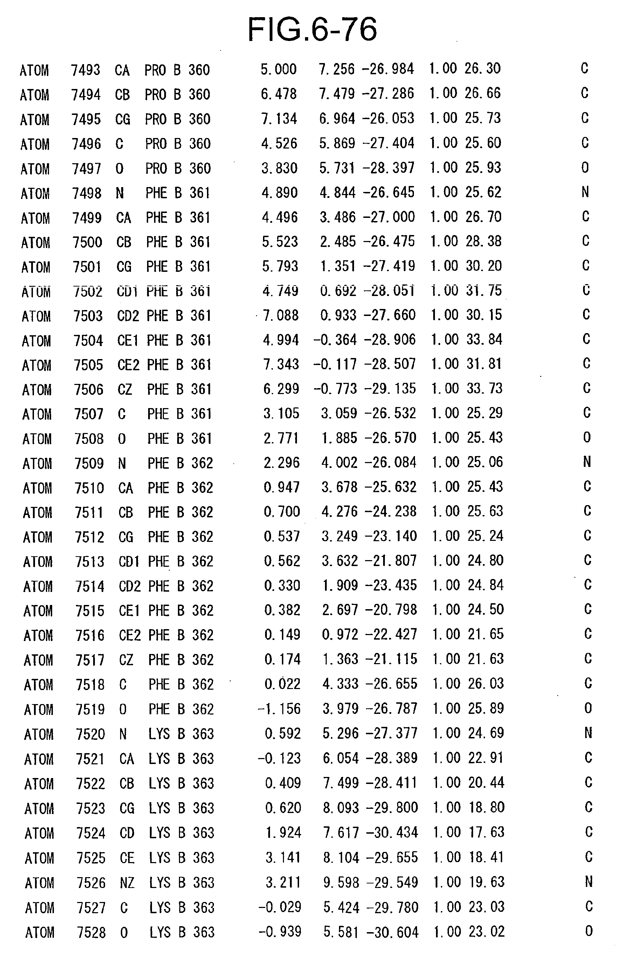

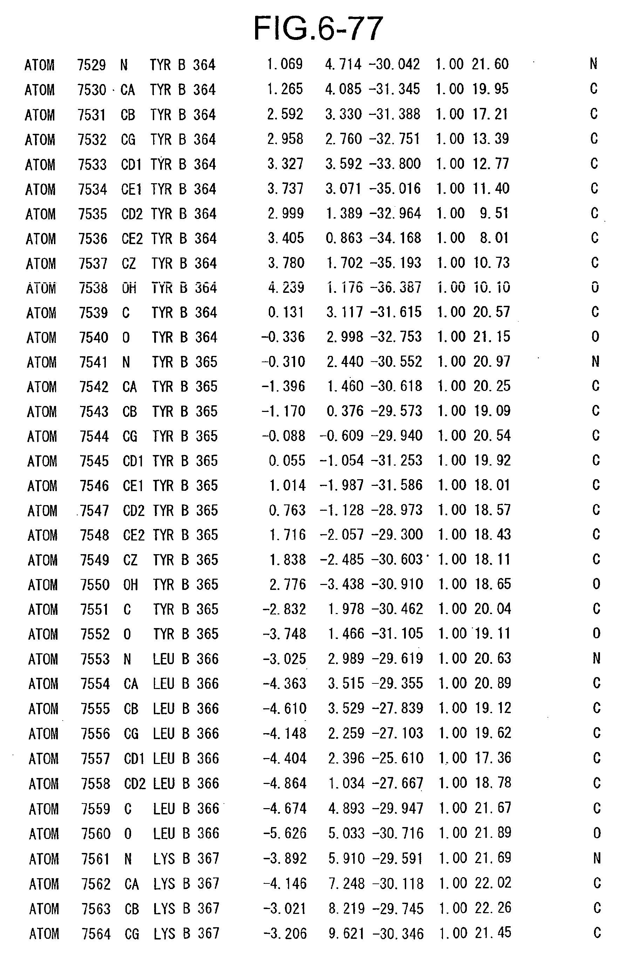

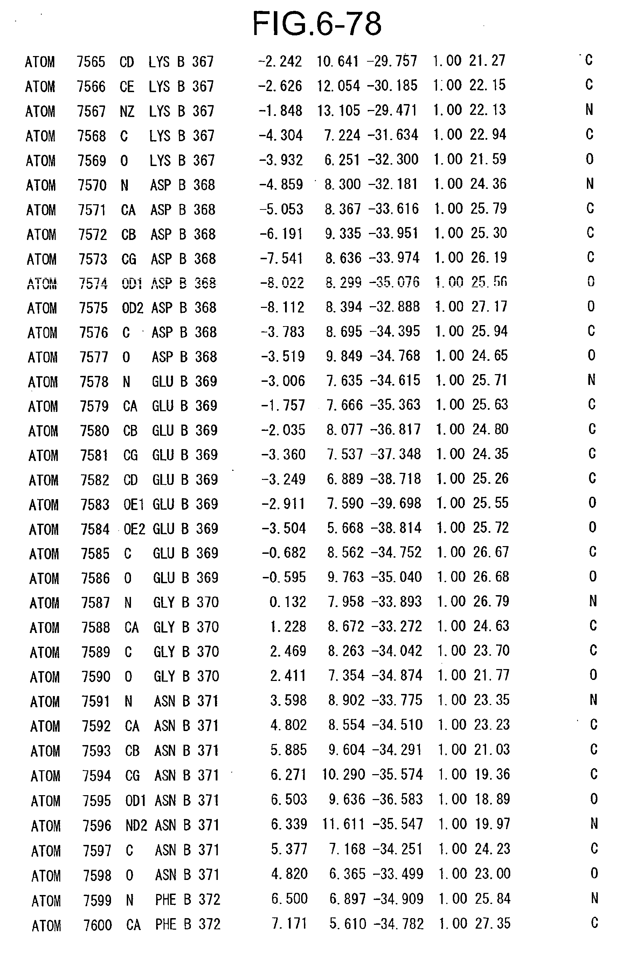

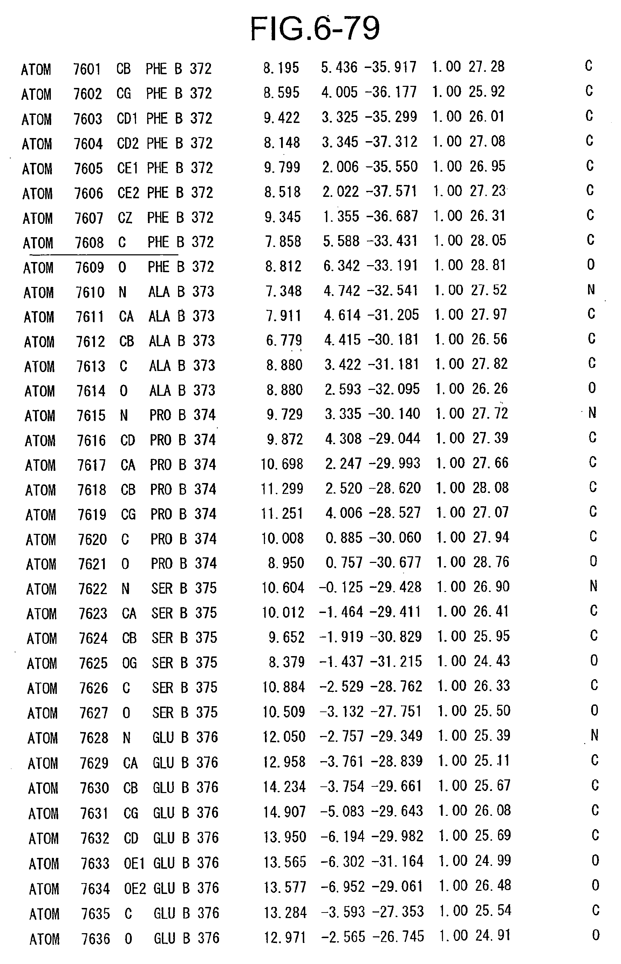

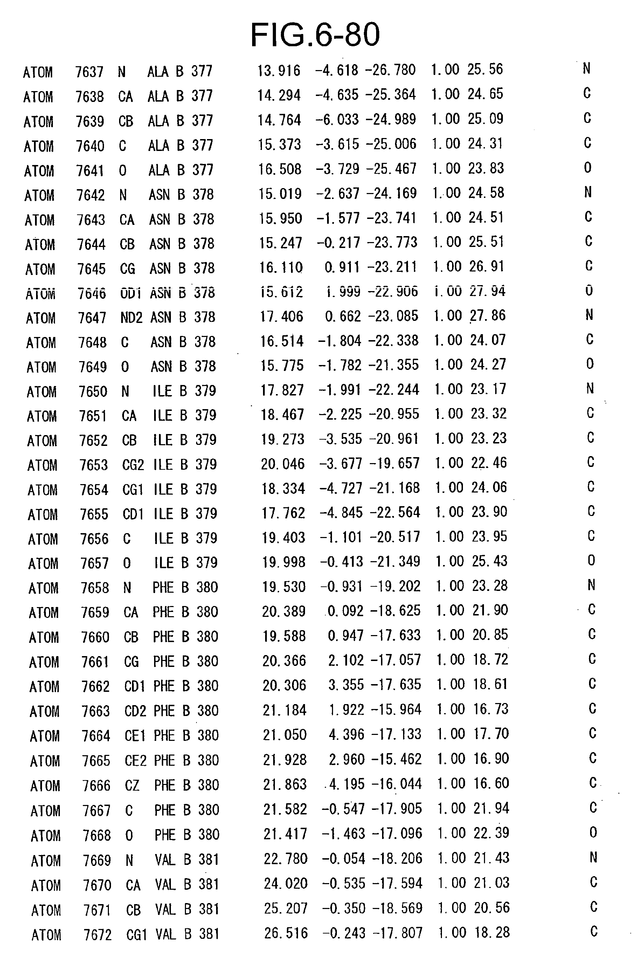

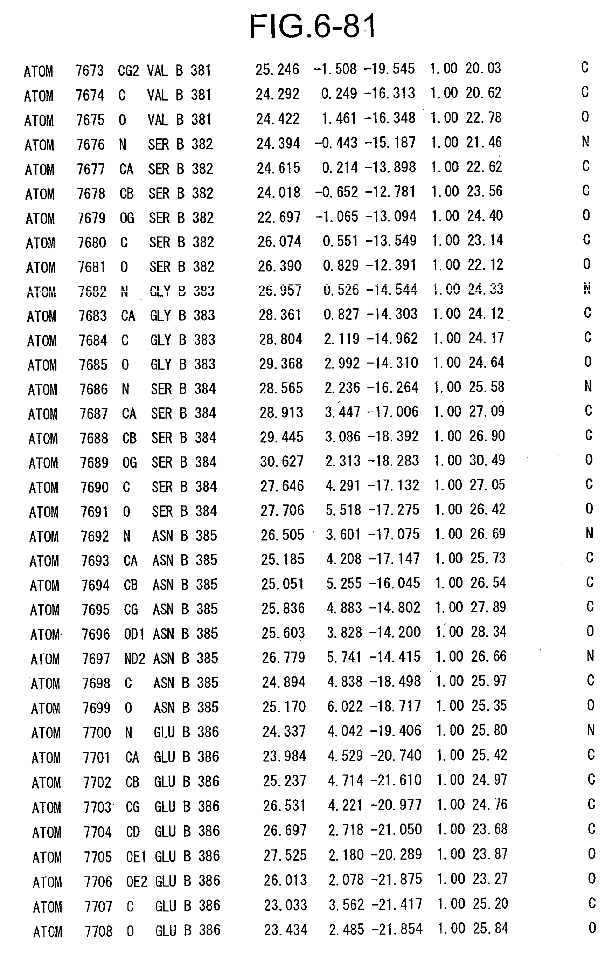

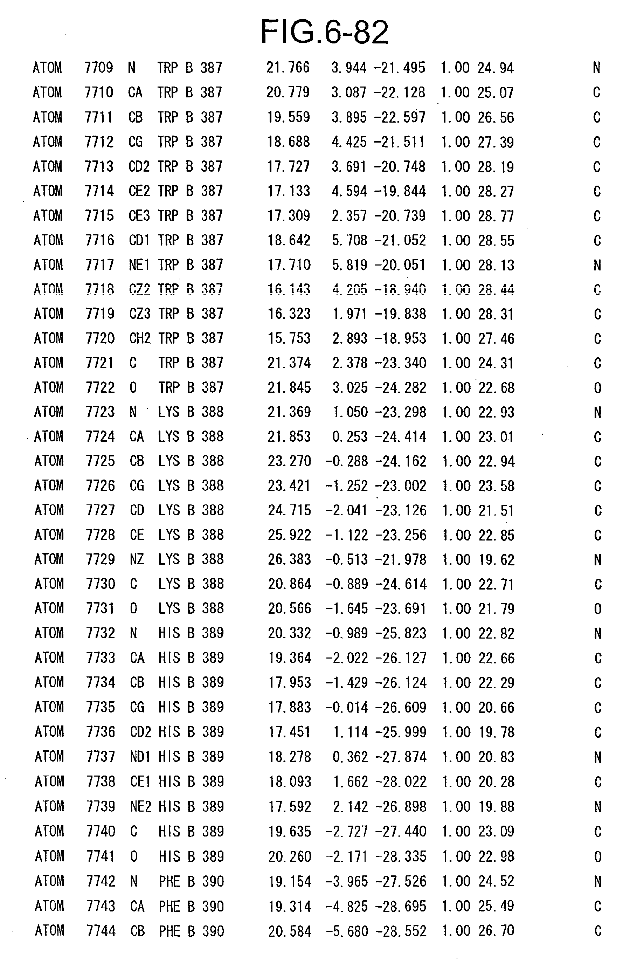

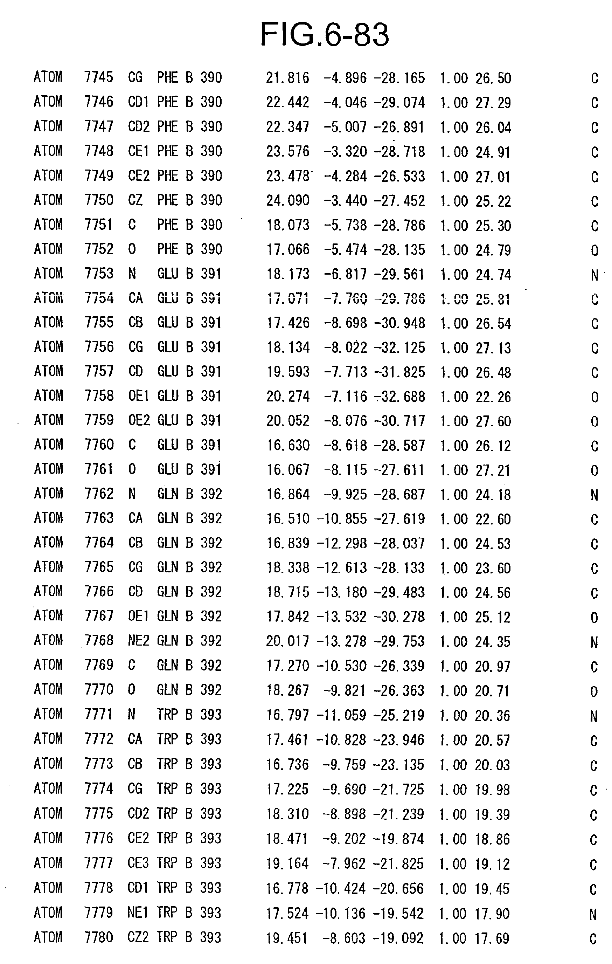

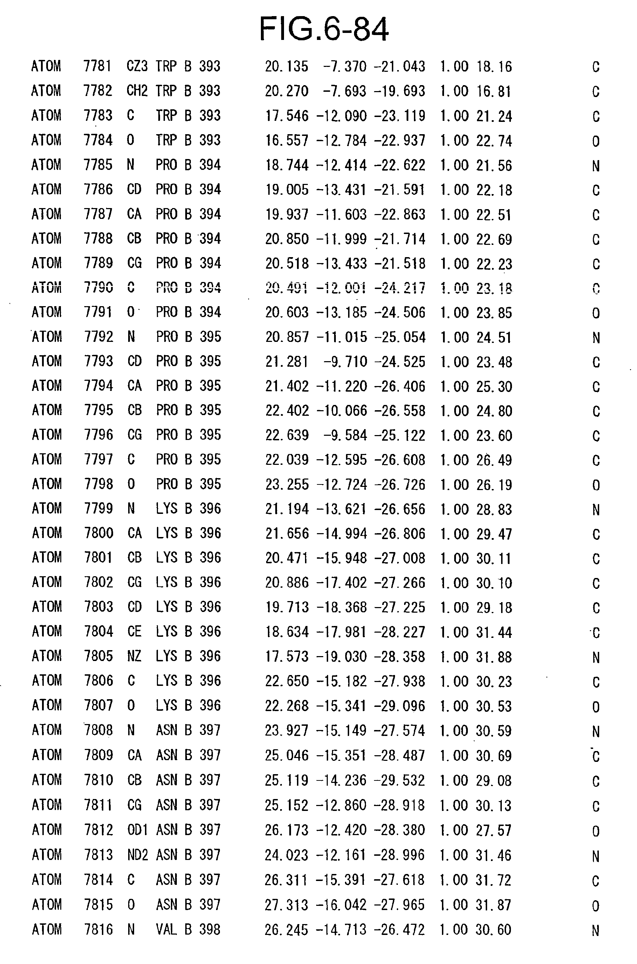

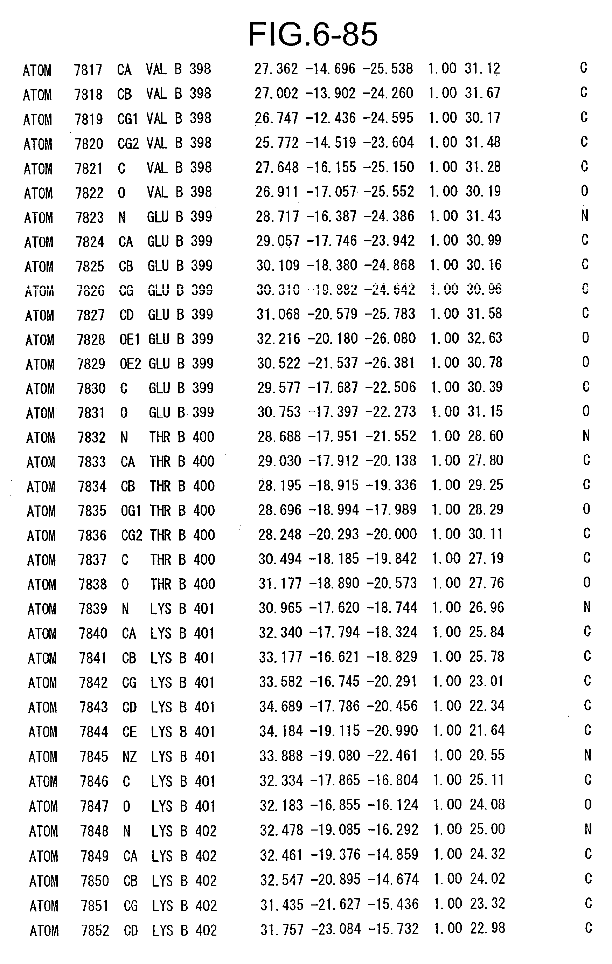

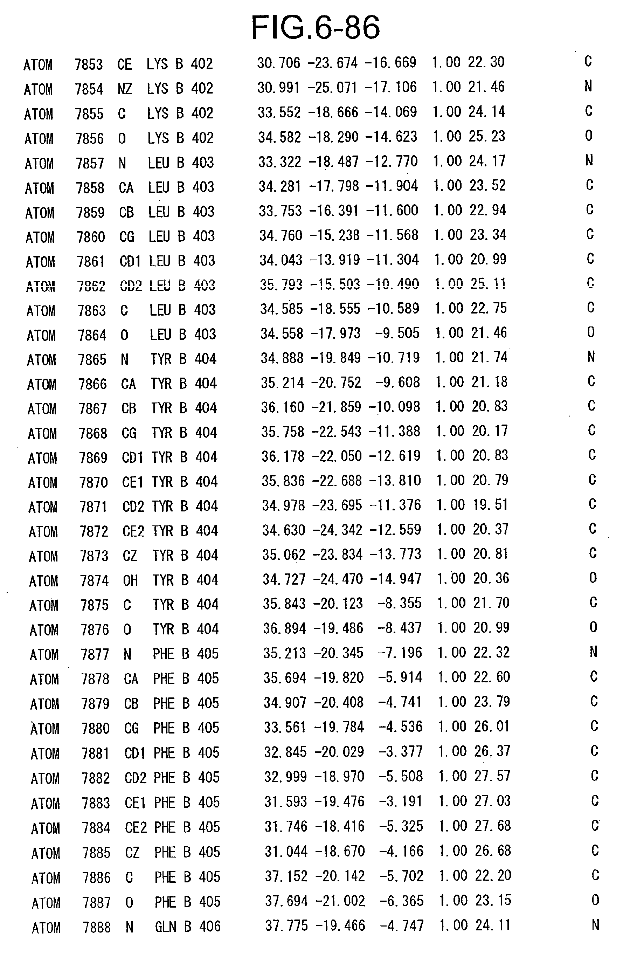

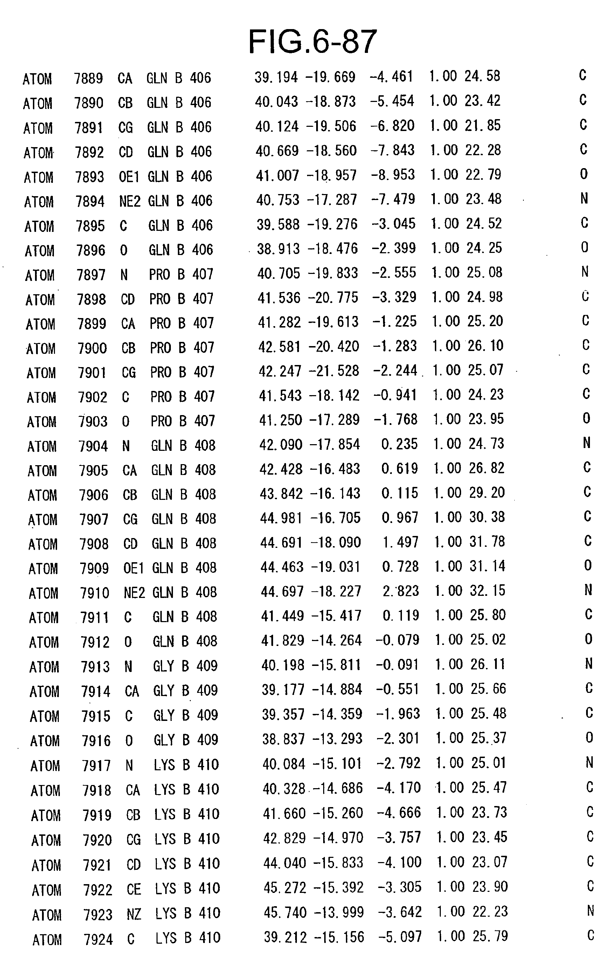

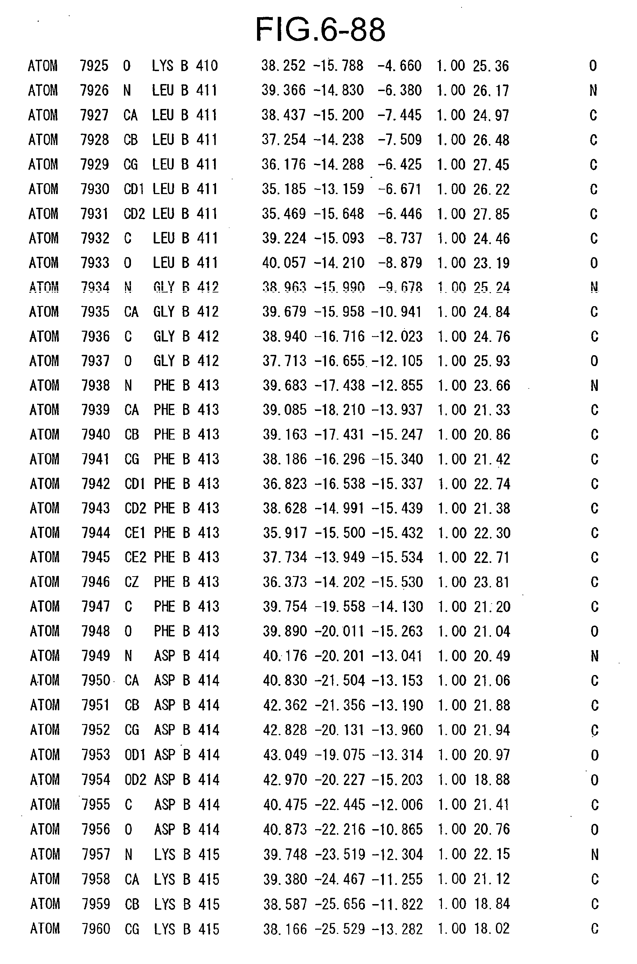

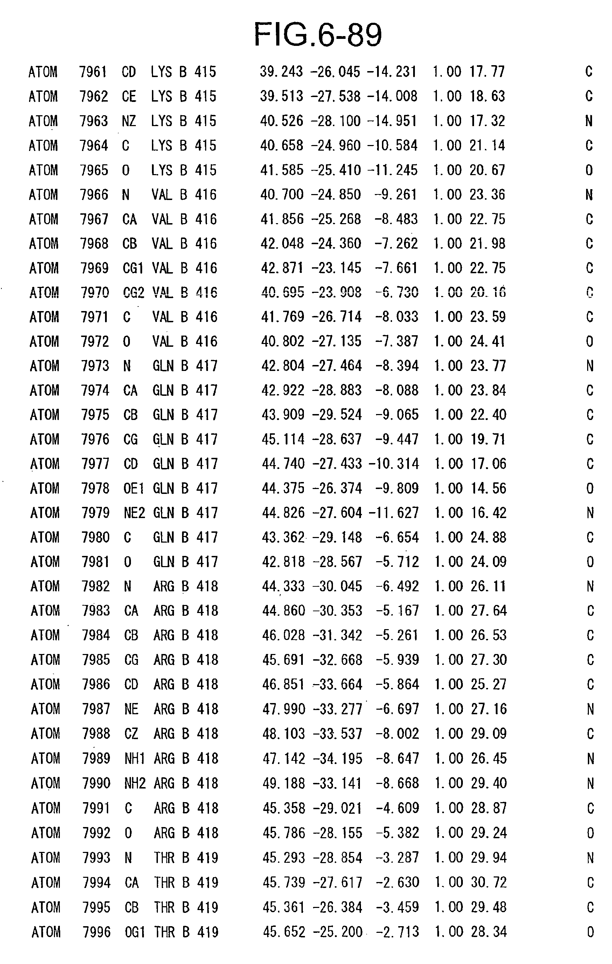

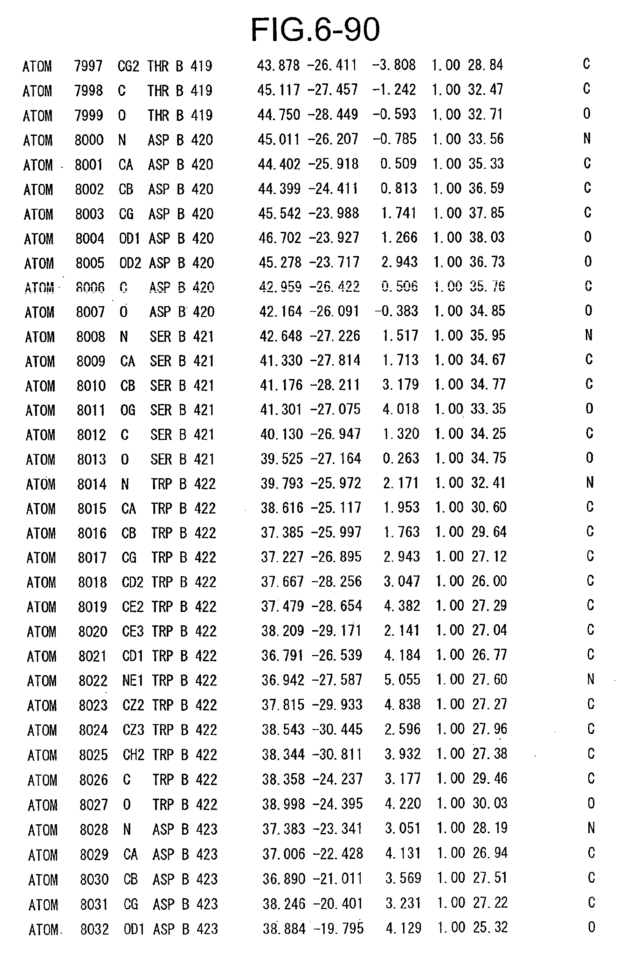

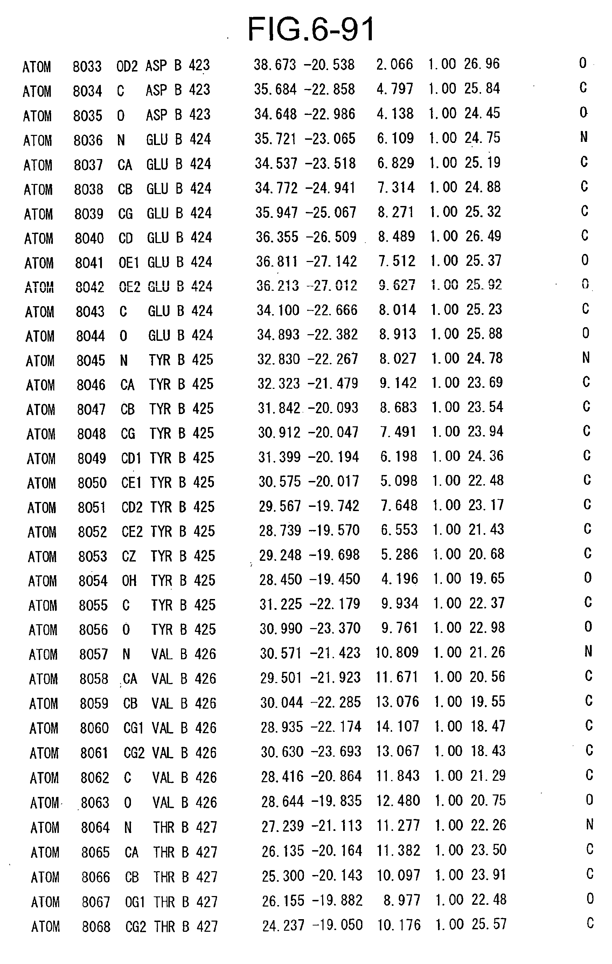

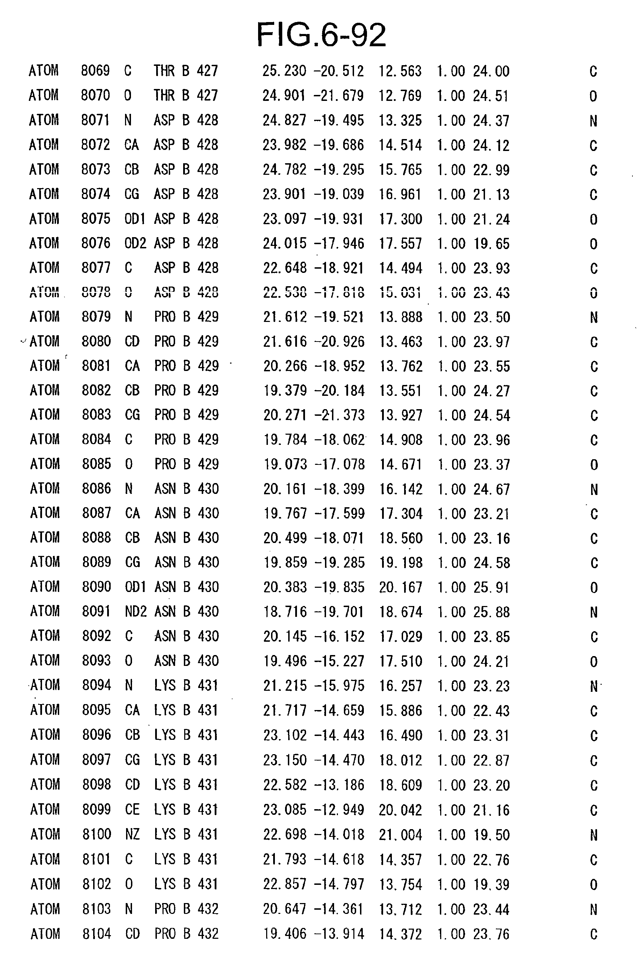

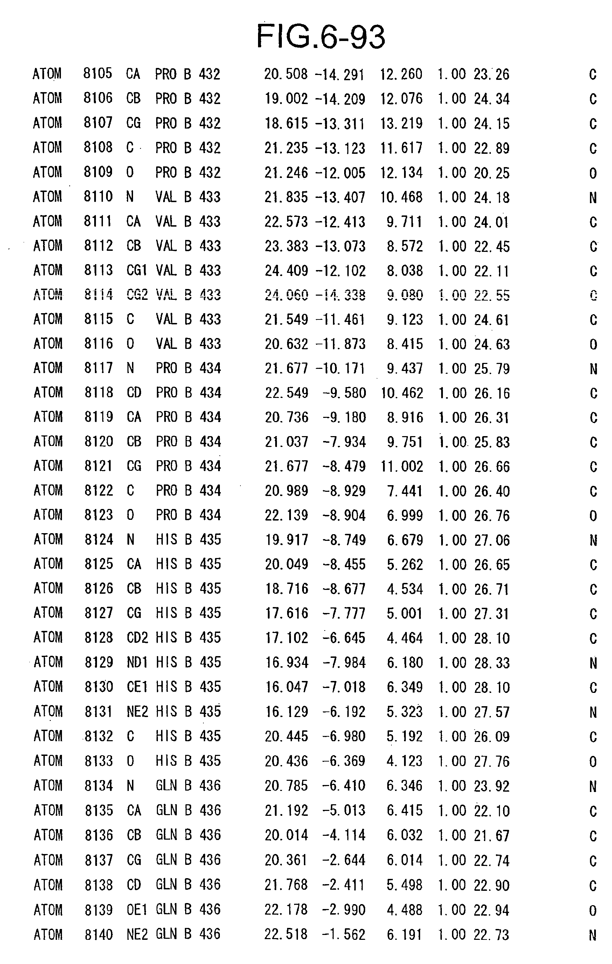

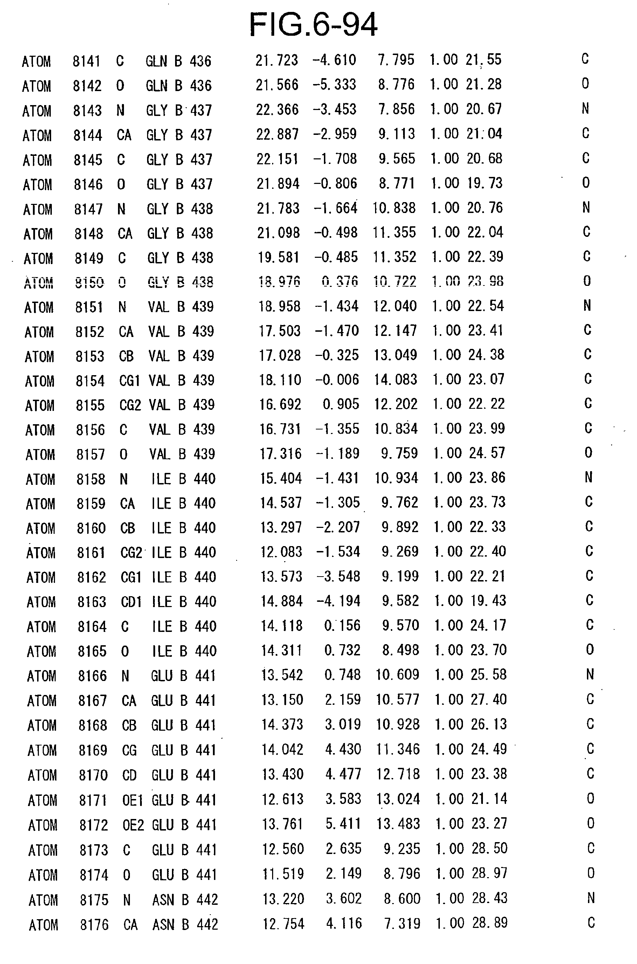

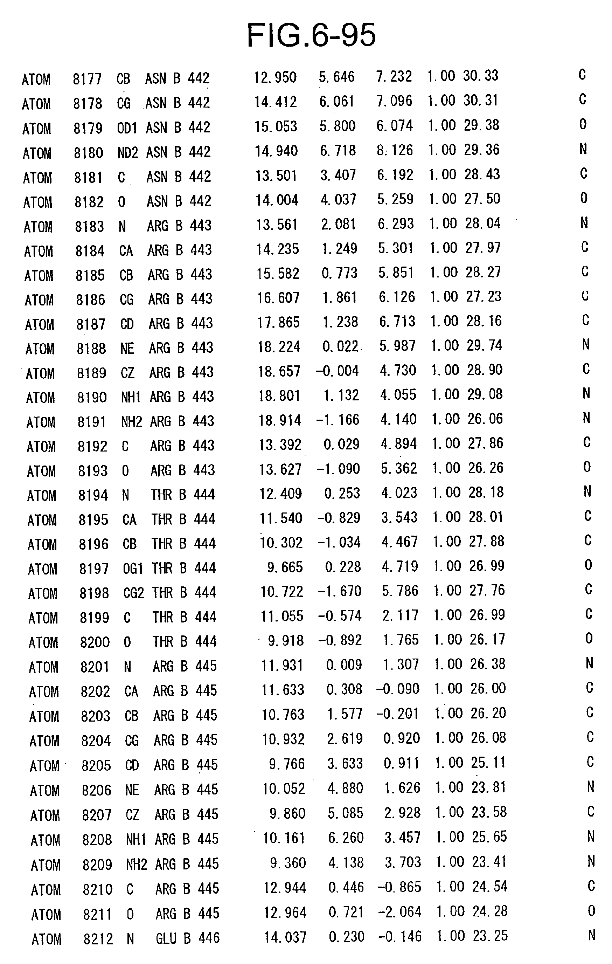

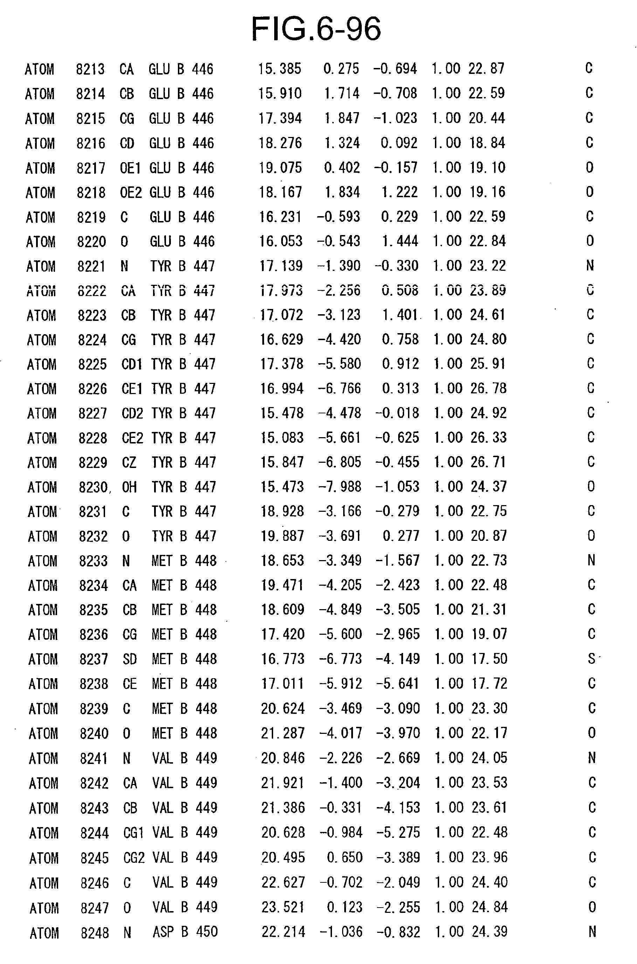

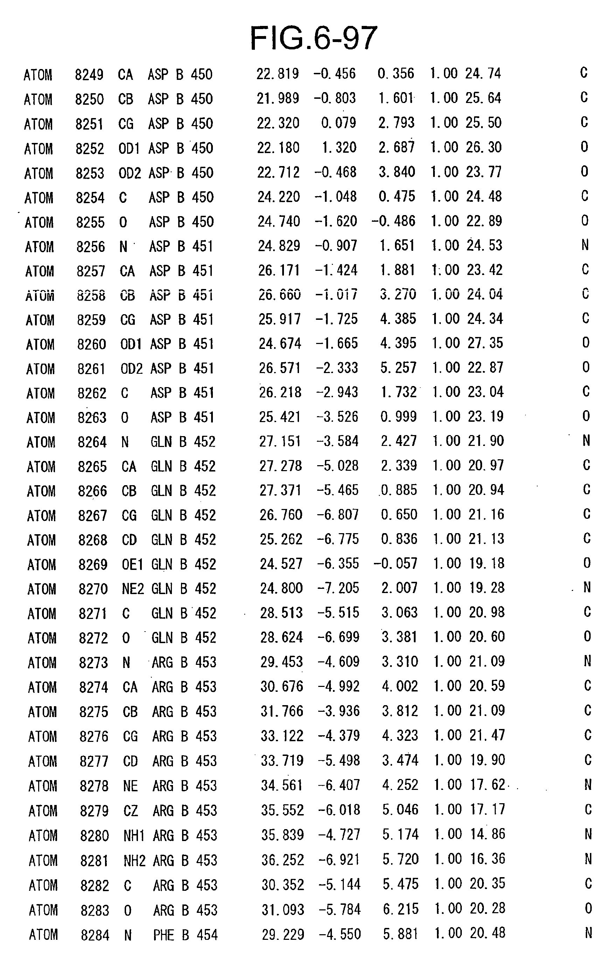

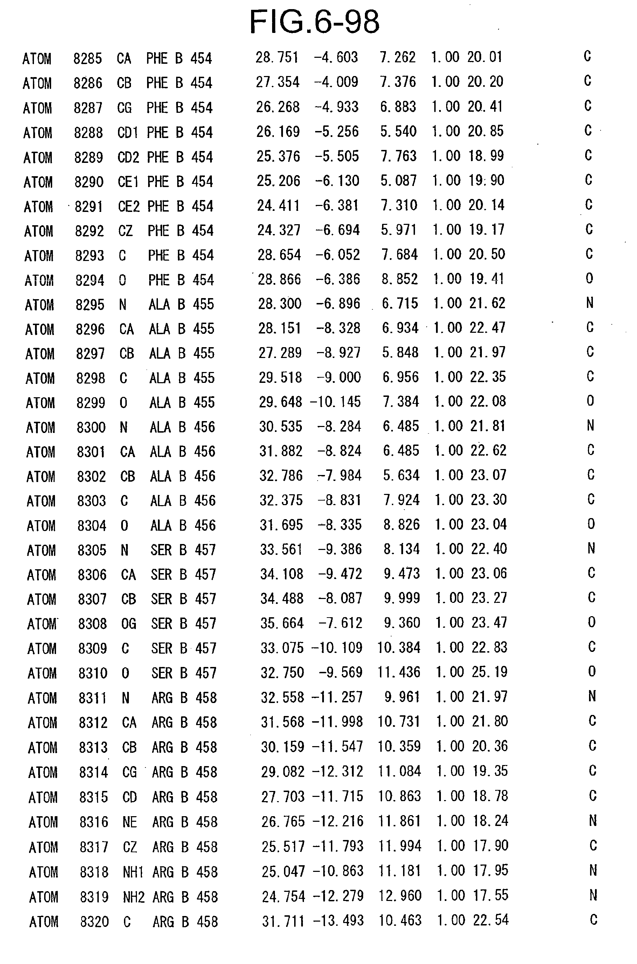

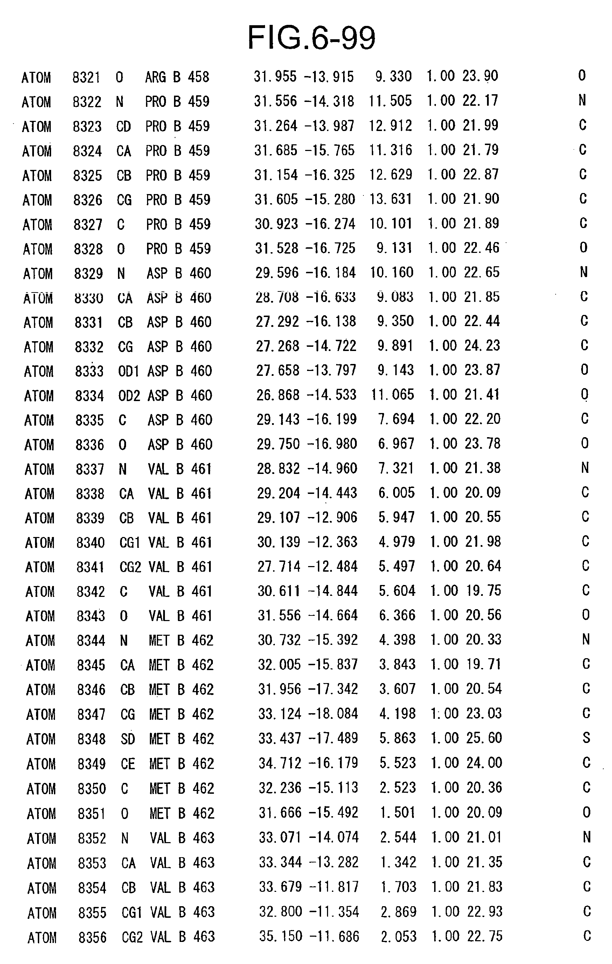

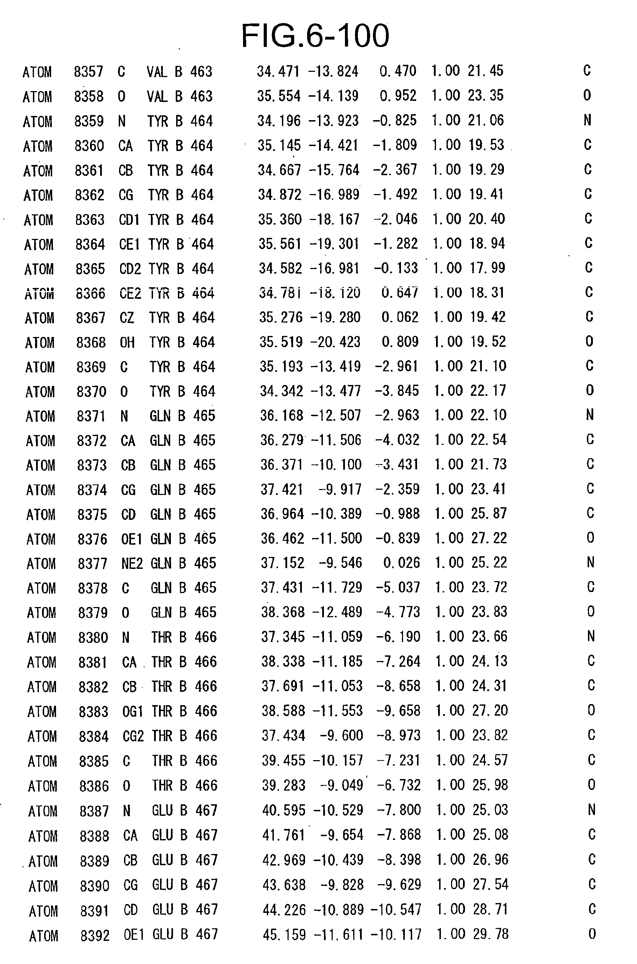

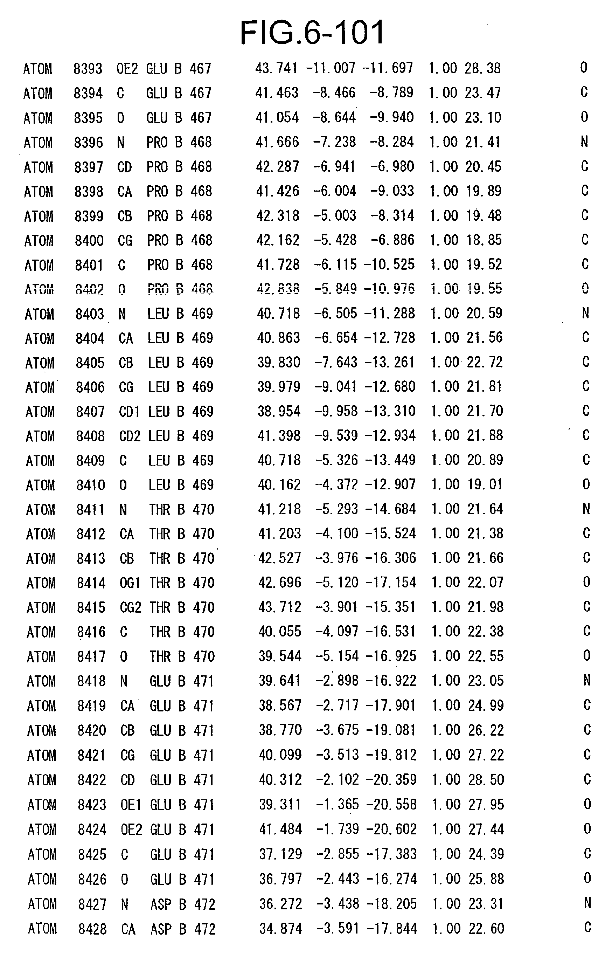

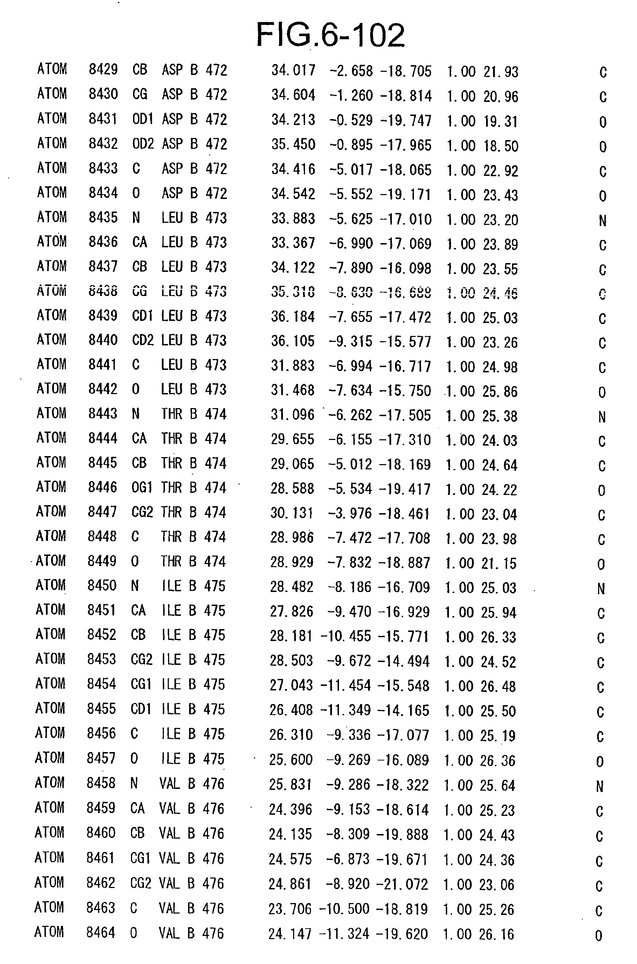

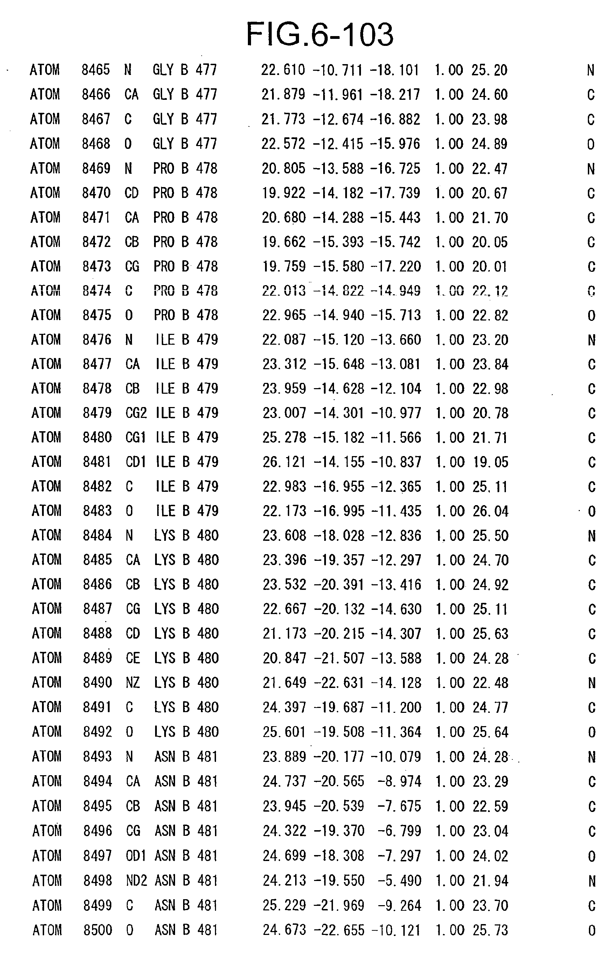

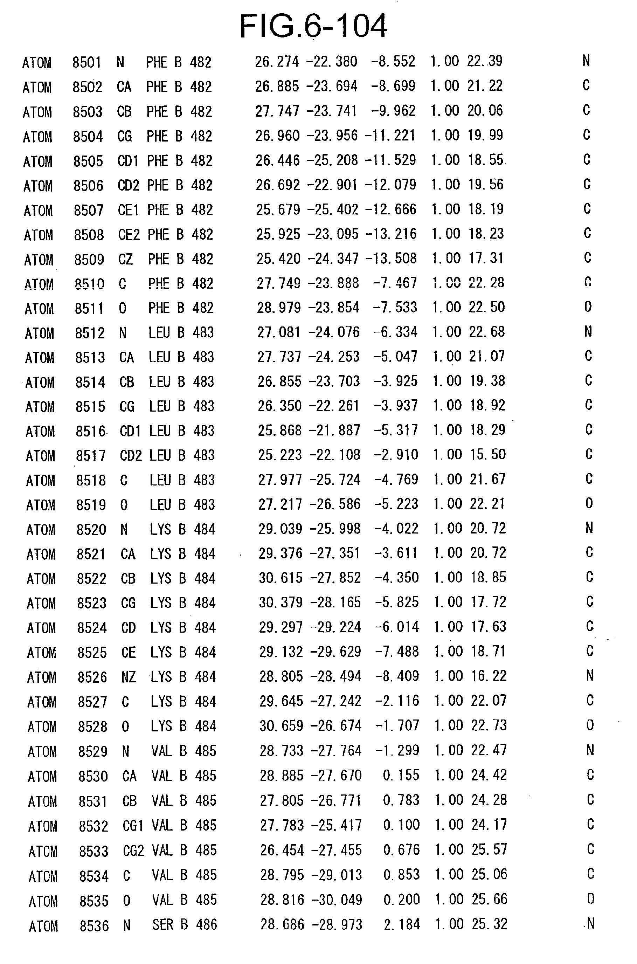

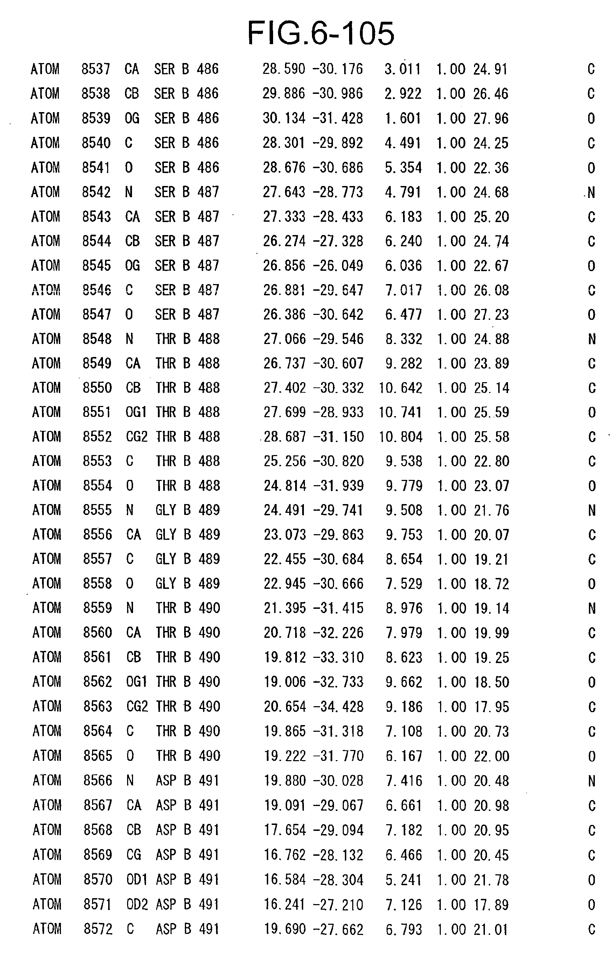

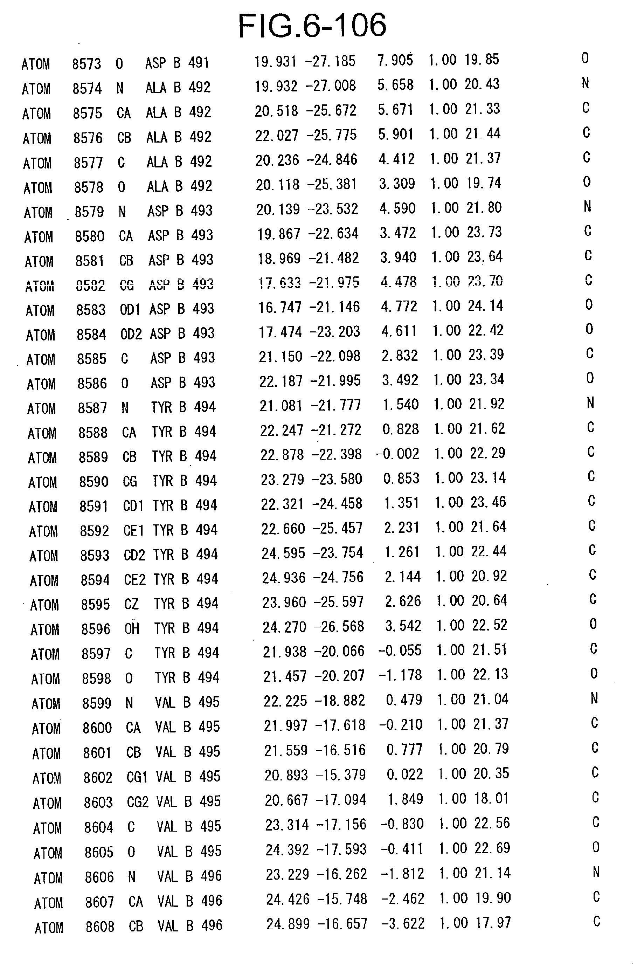

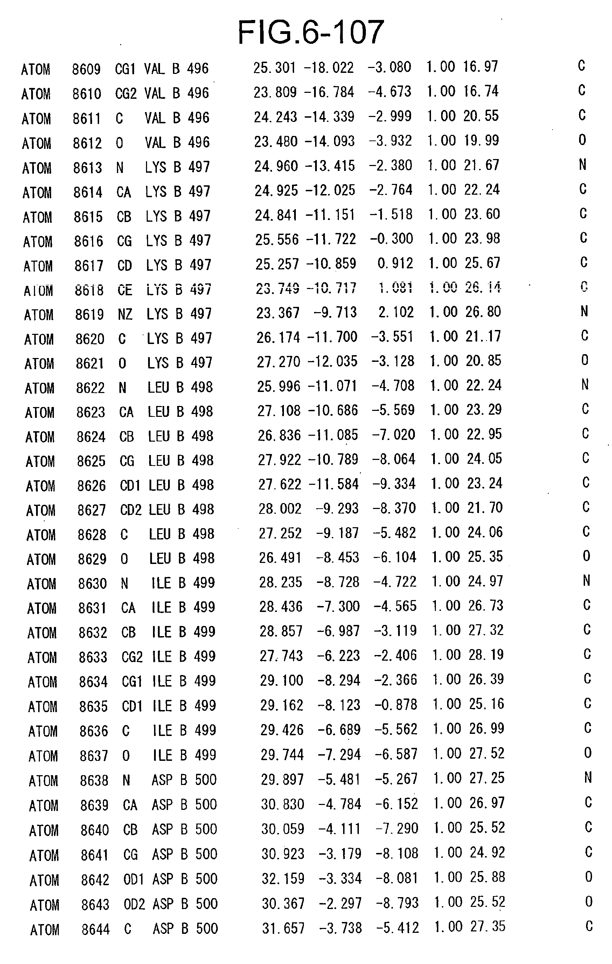

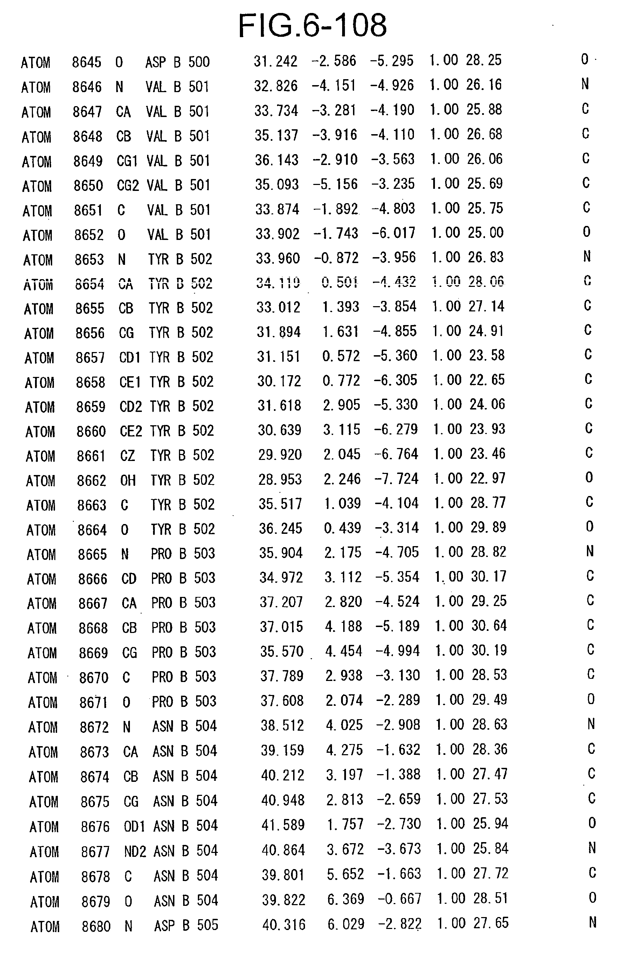

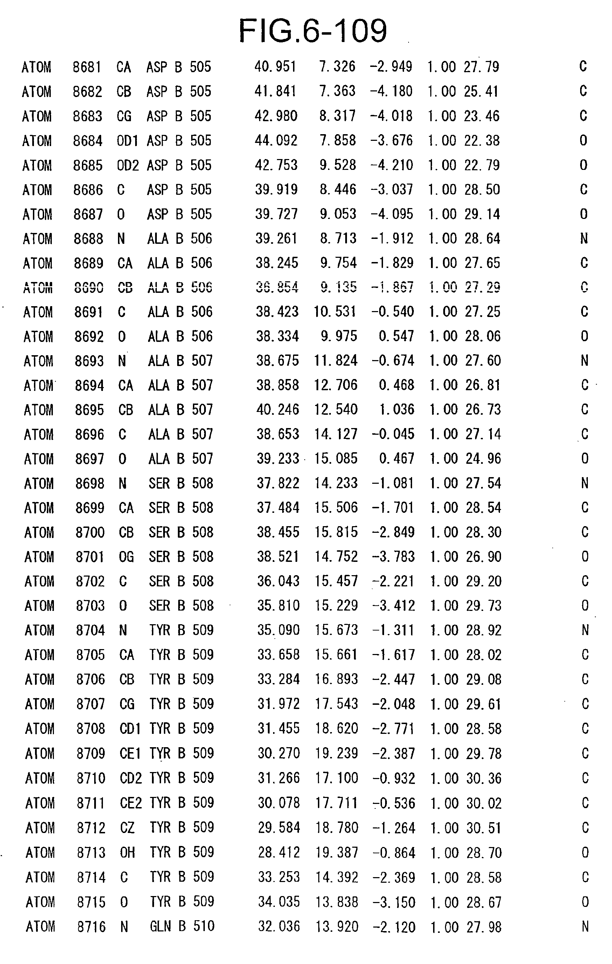

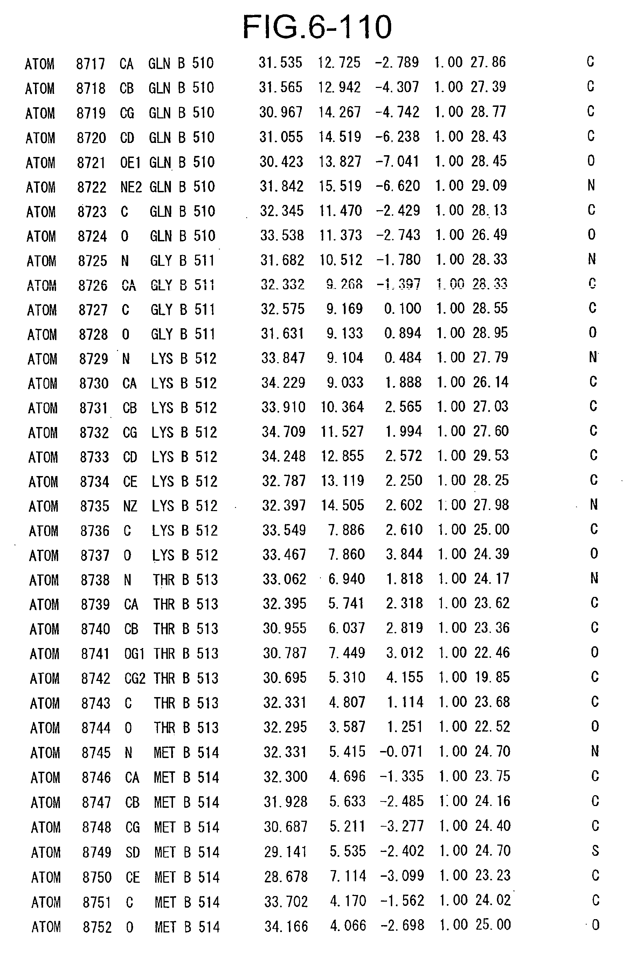

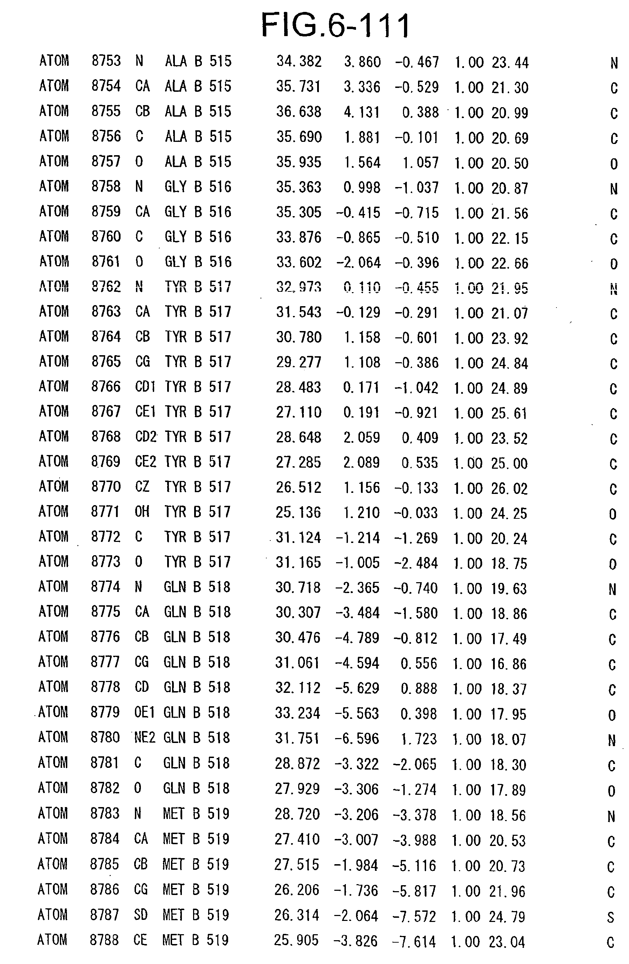

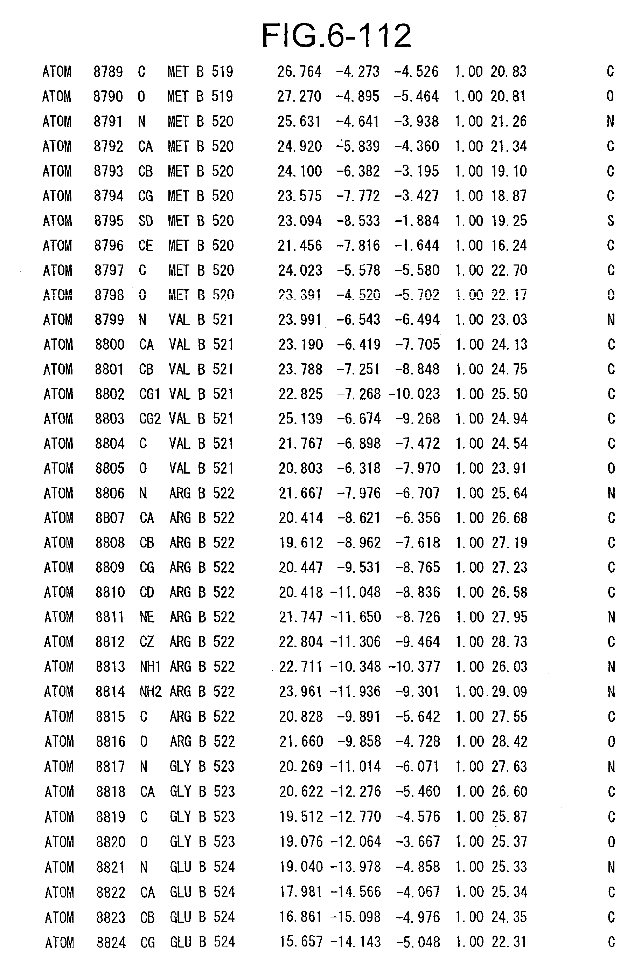

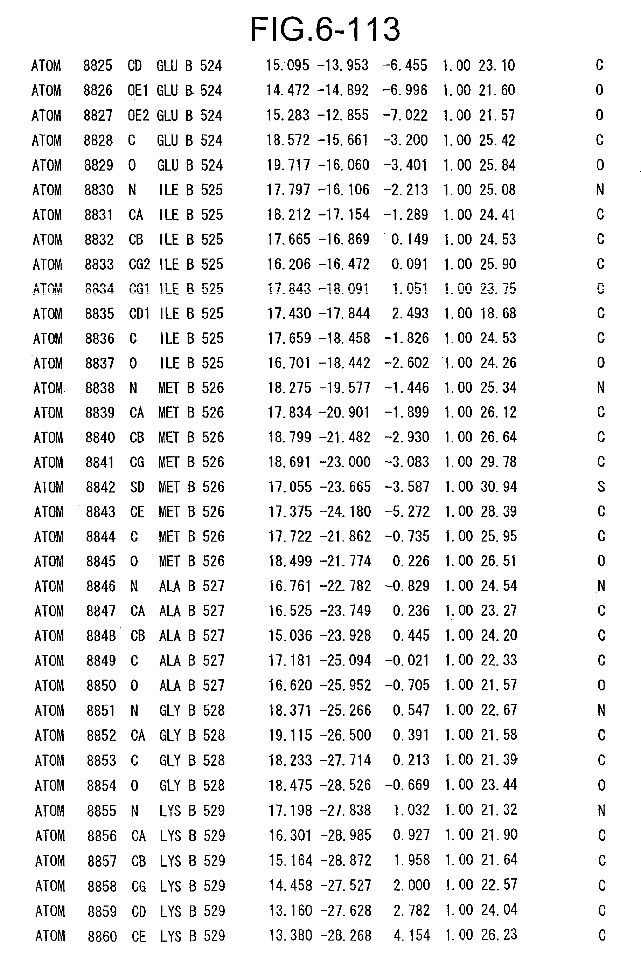

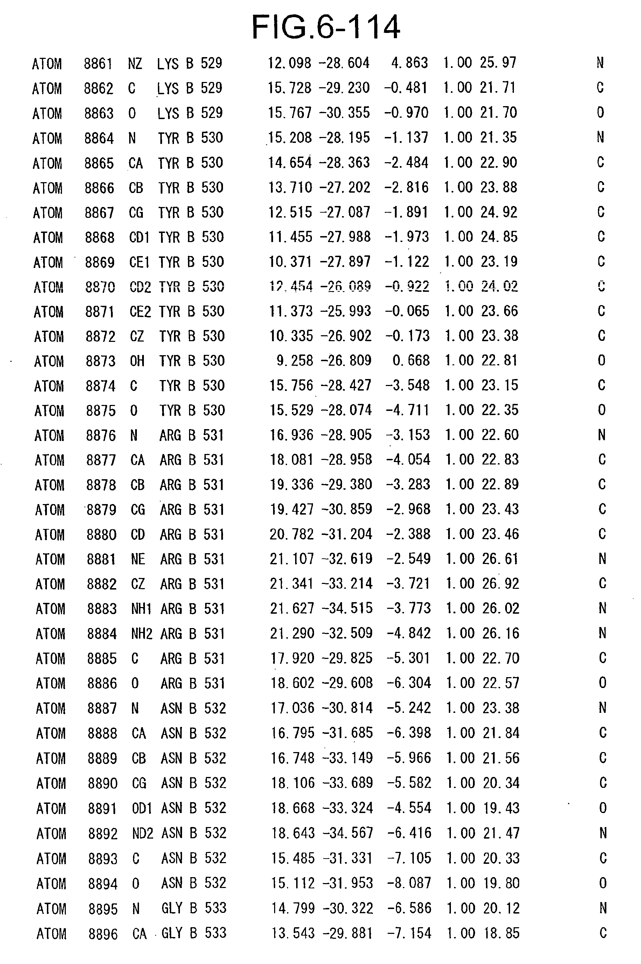

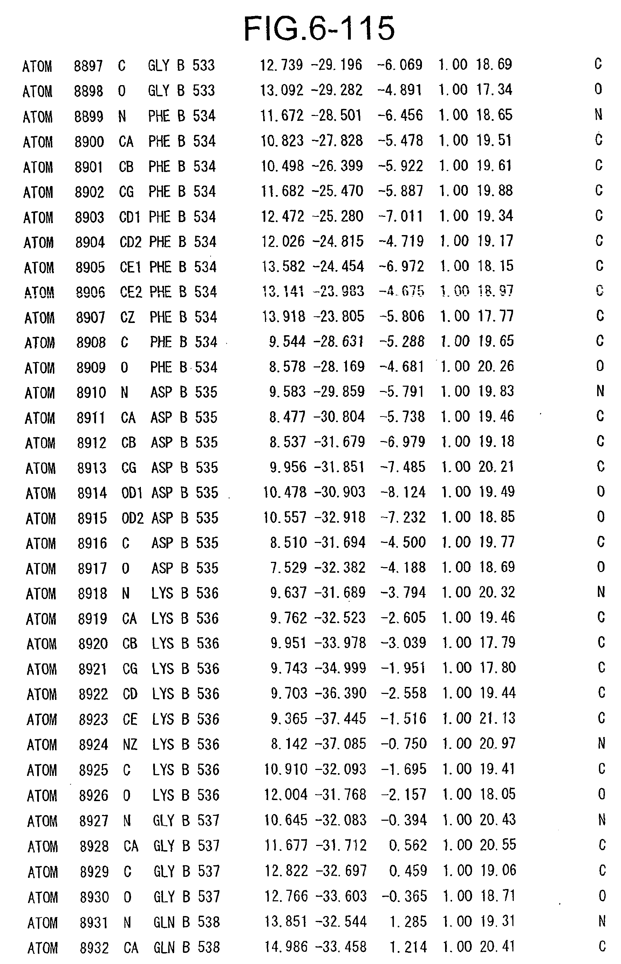

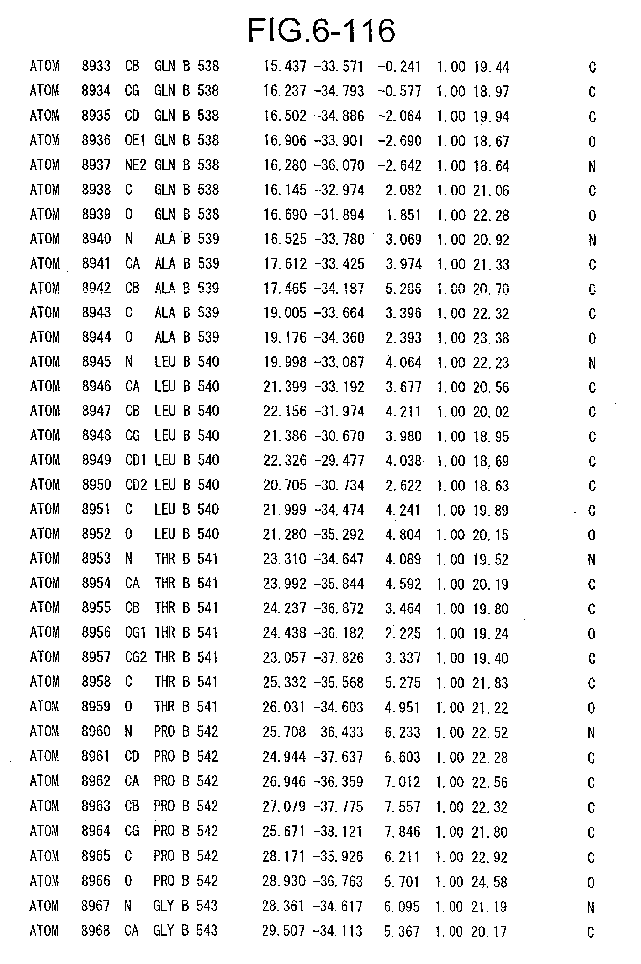

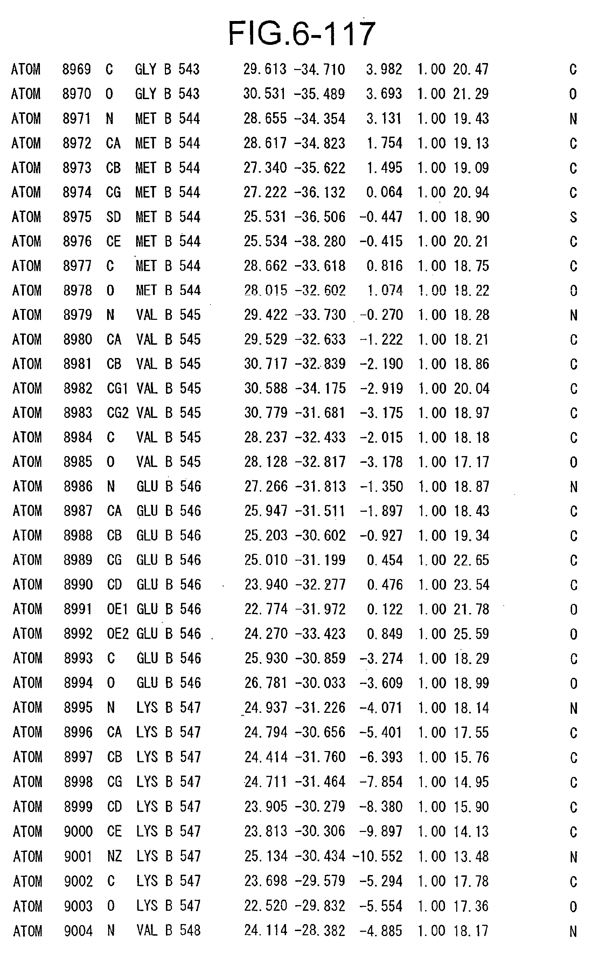

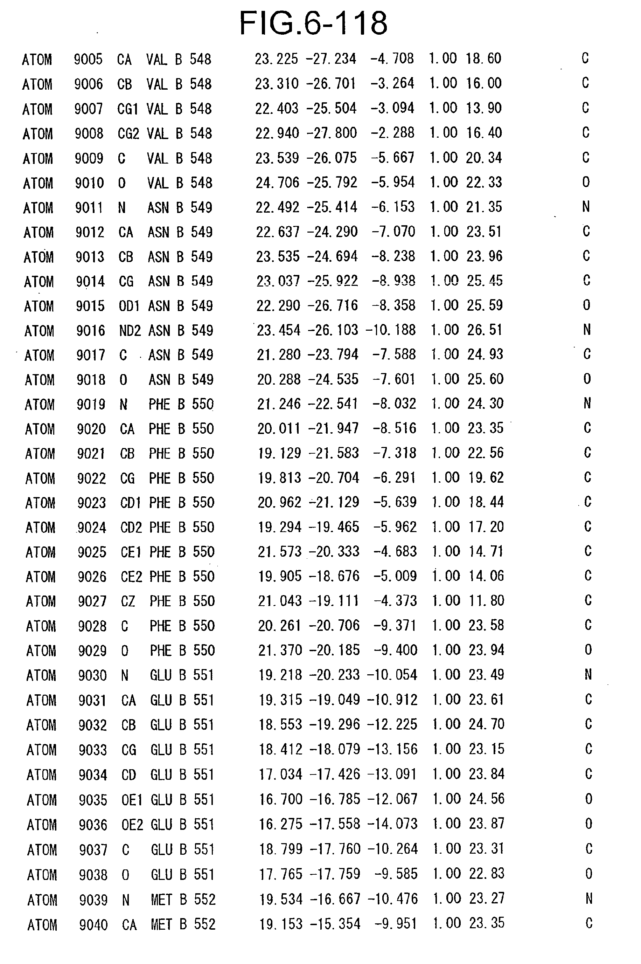

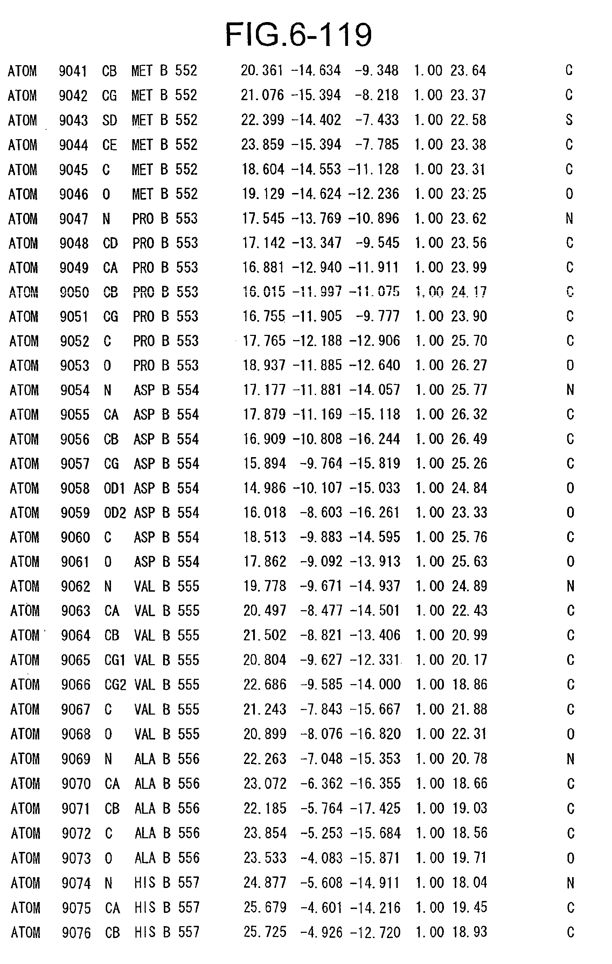

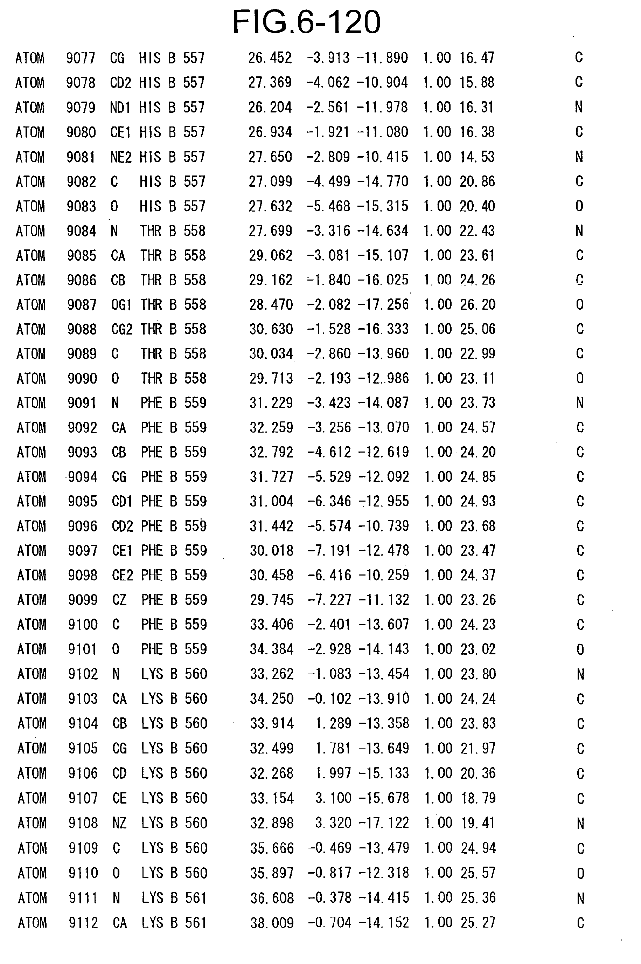

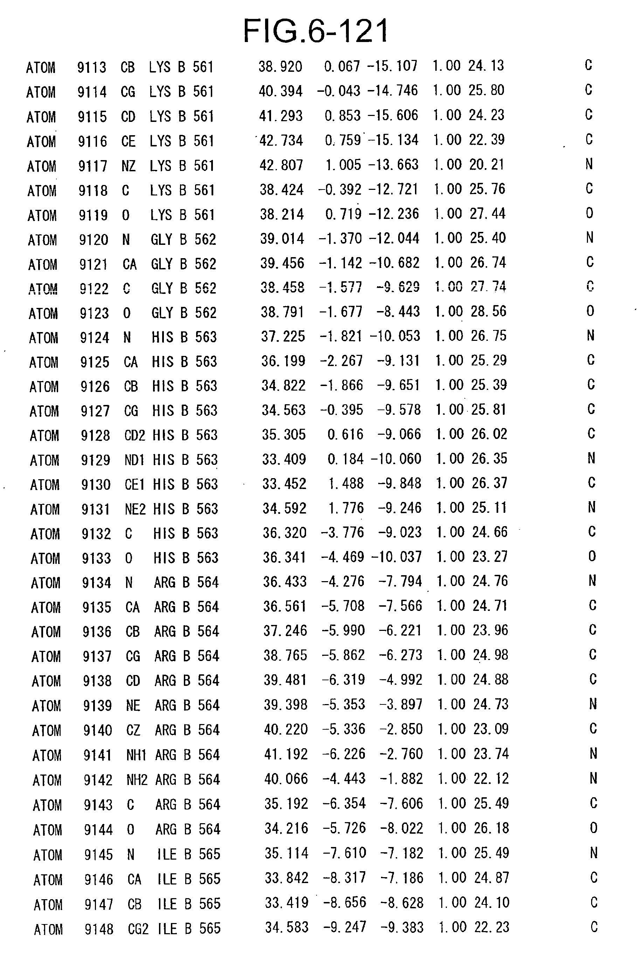

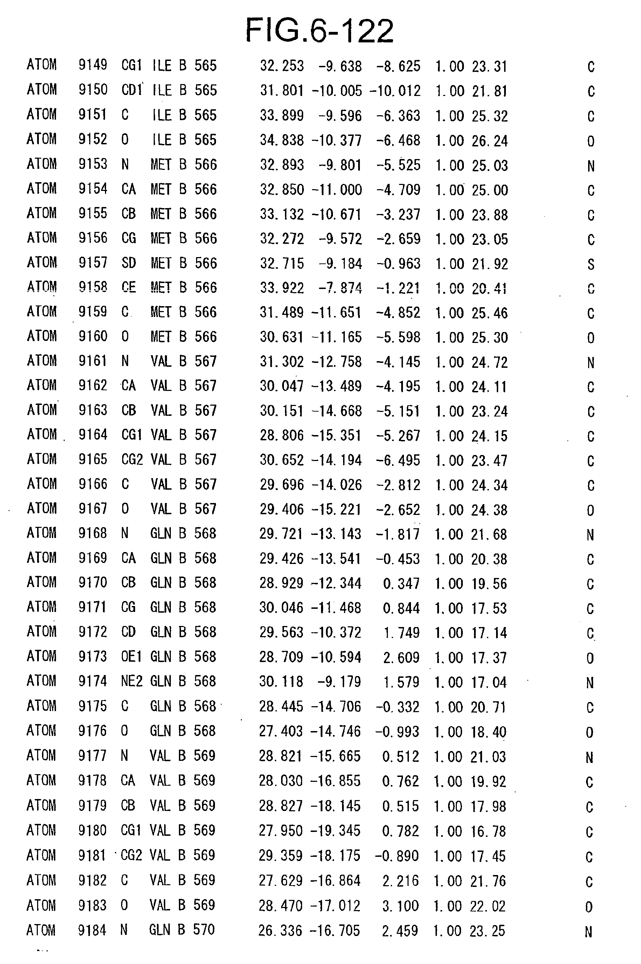

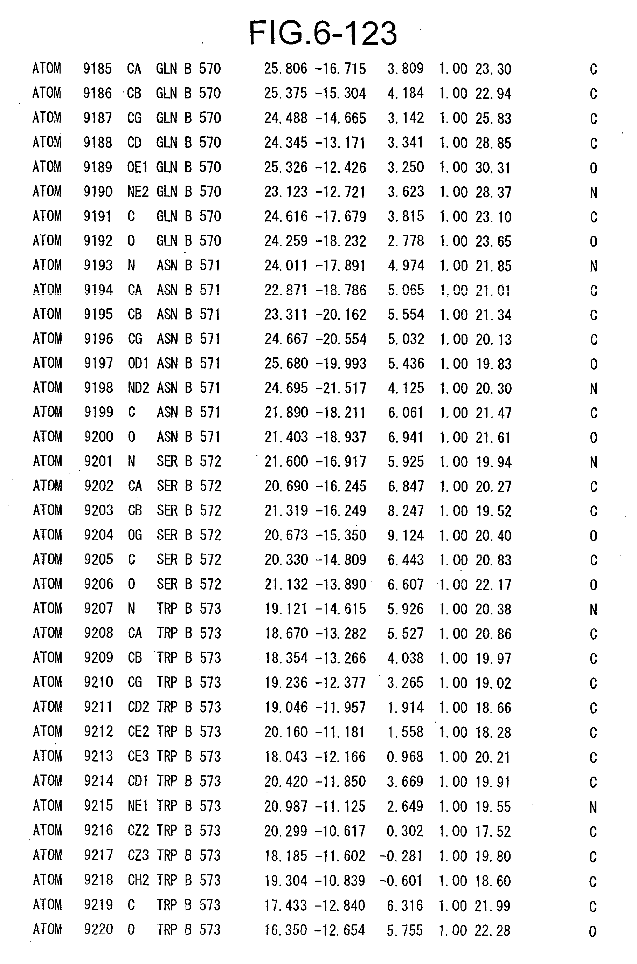

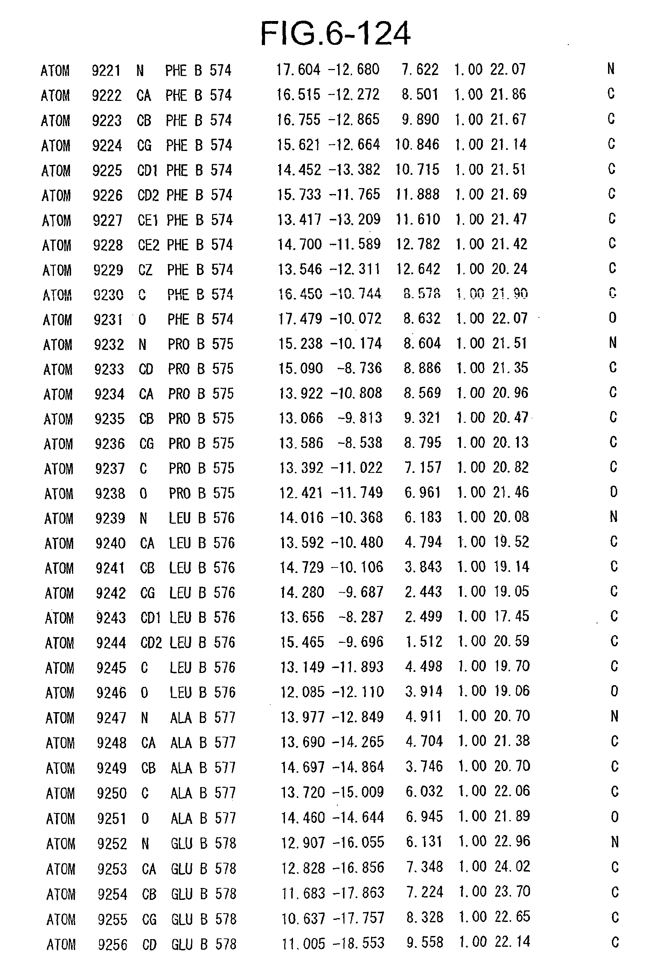

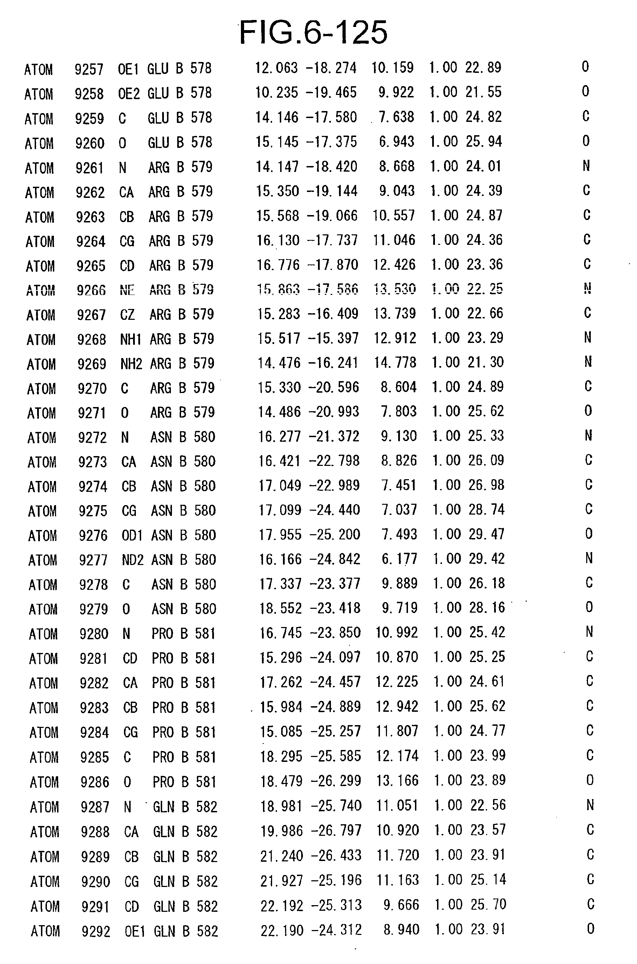

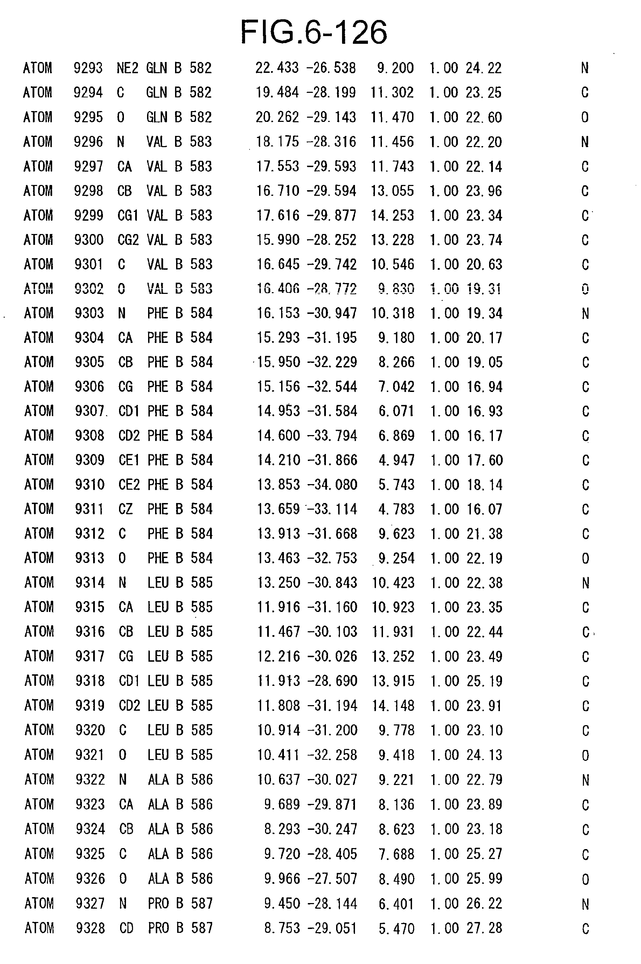

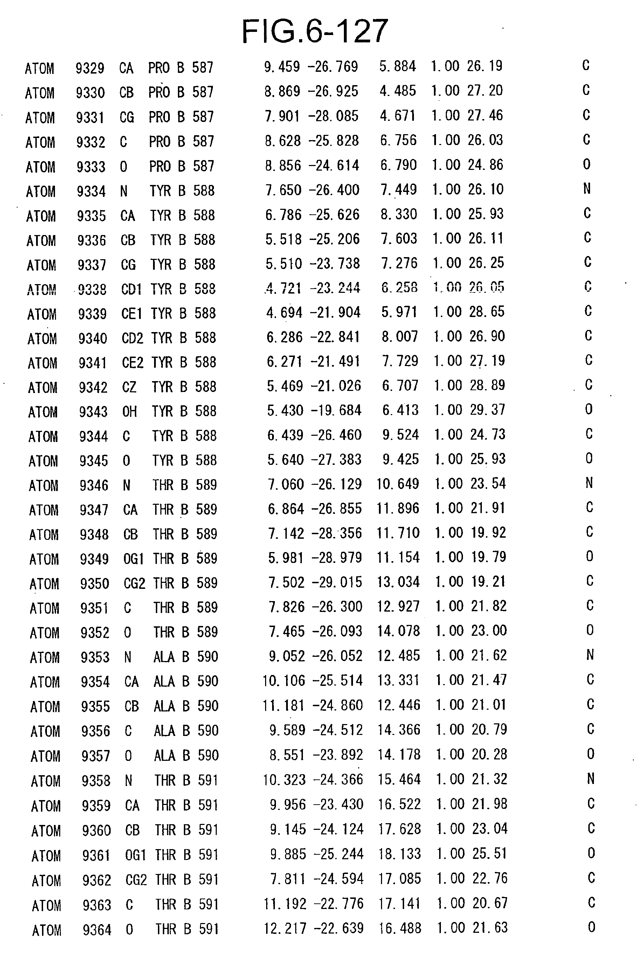

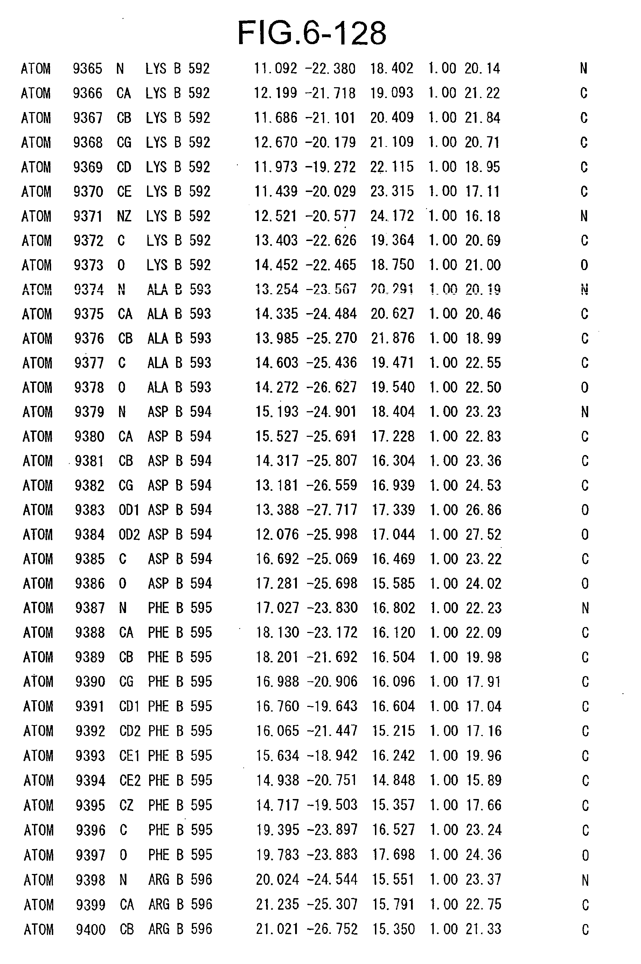

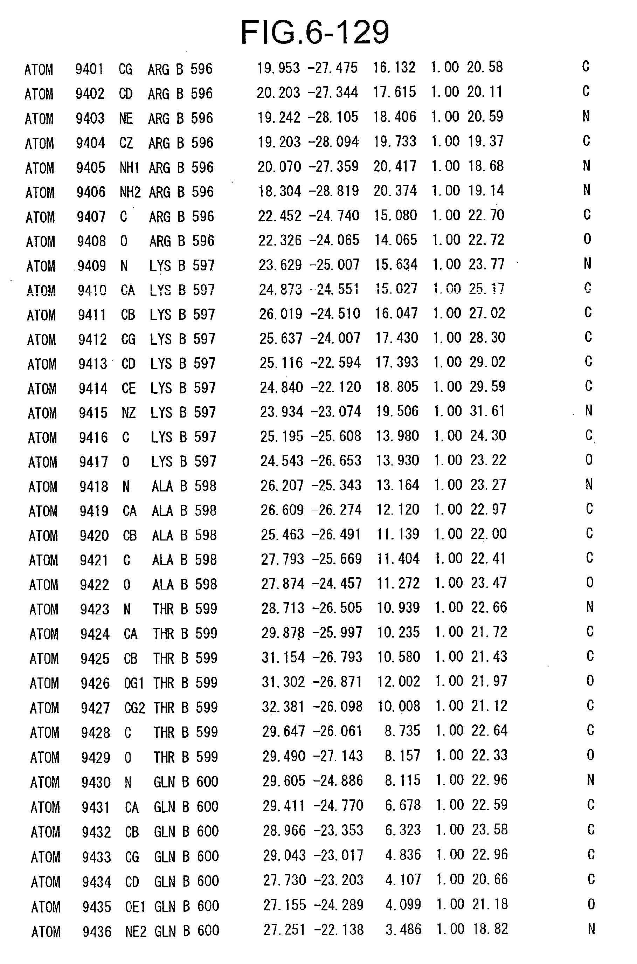

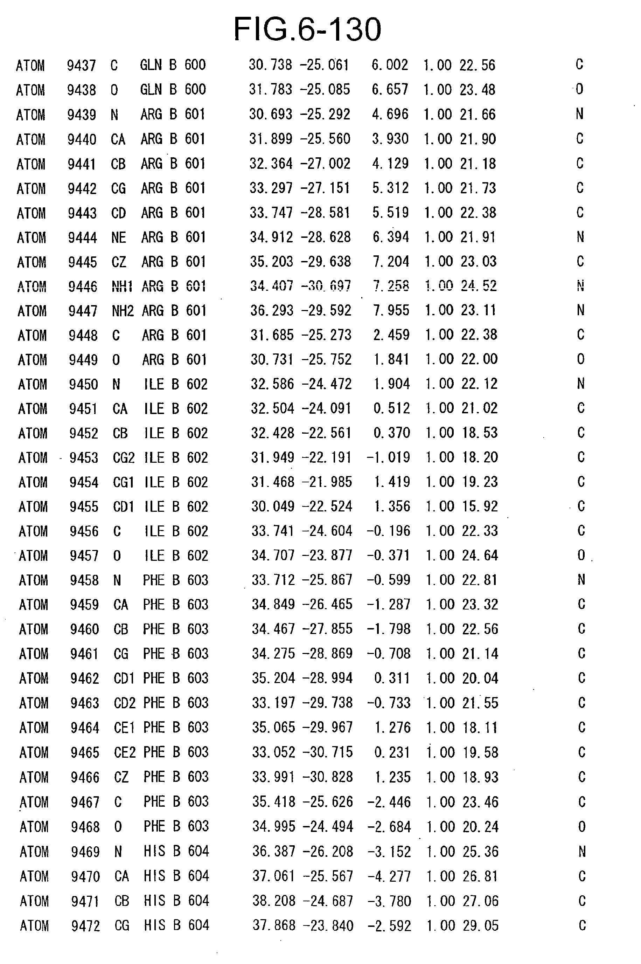

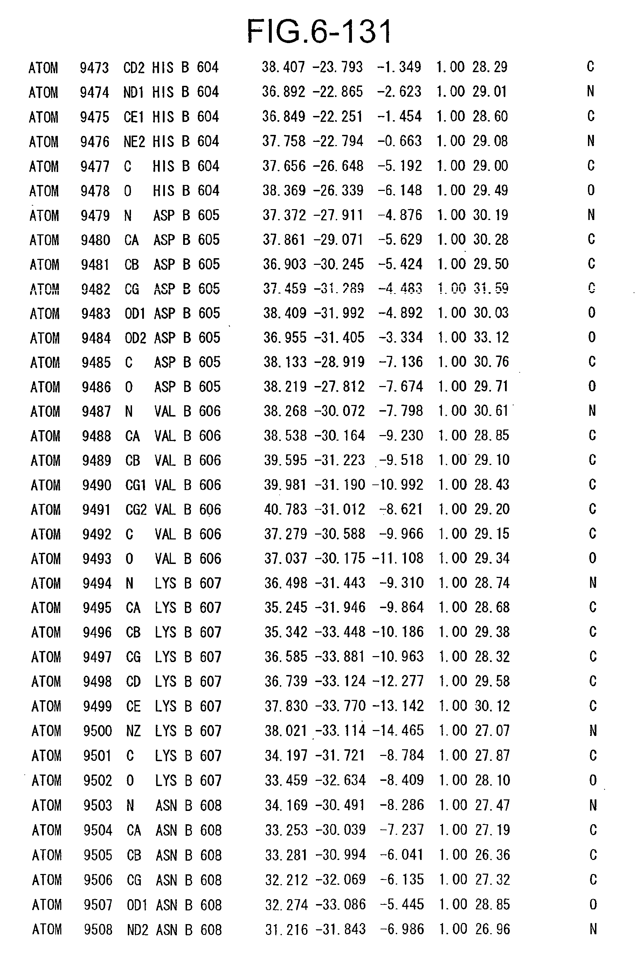

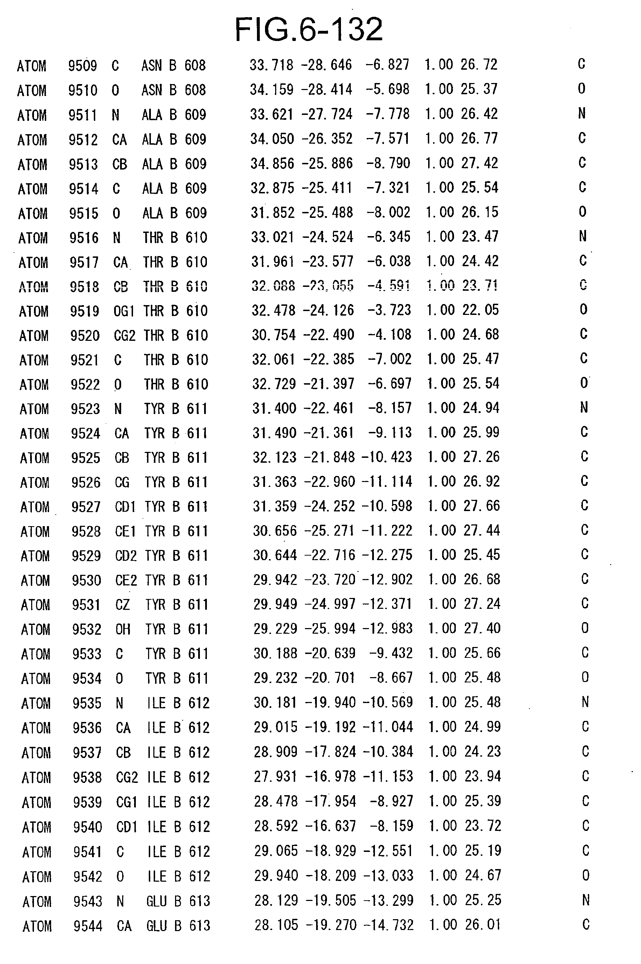

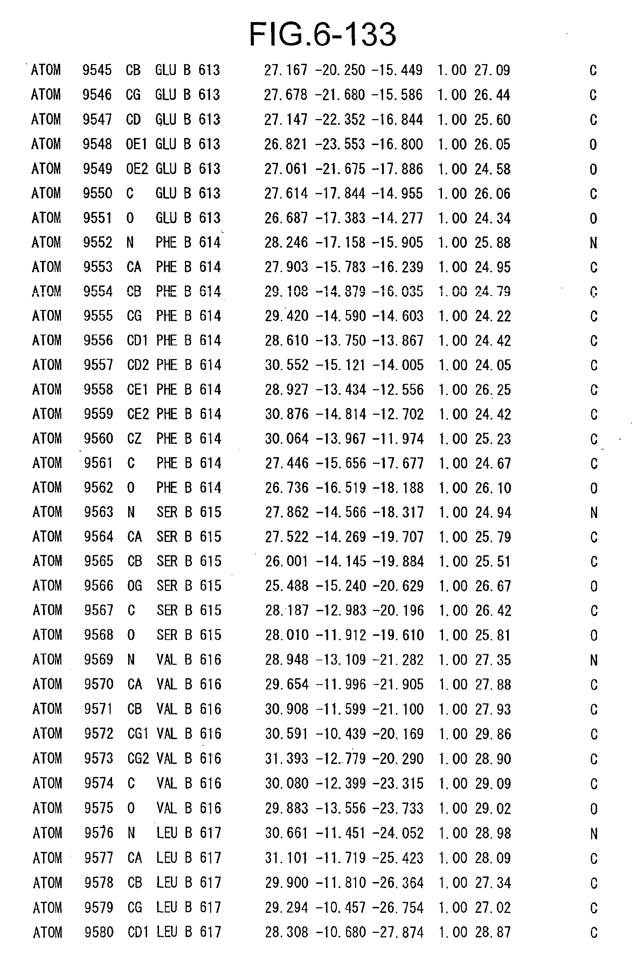

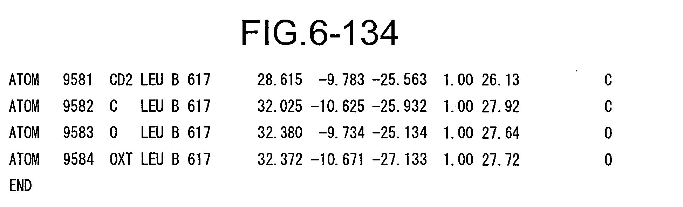

The similarity of the tertiary structures is determined and assessed in the threading method by aligning the amino acid sequence of the subject protein with the tertiary structure of the protein having the amino acid sequence of SEQ ID NO:209, calculating an objective function which quantifies fitness of these structures as to, e.g. easiness to make the secondary structure, and comparing/examining the results. The data described in FIG. 6-1 to FIG. 6-134 may be used as the data (coordinates) of the tertiary structure (three dimensional structure) of the protein having the amino acid sequence of SEQ ID NO:209.

The threading method may be carried out by the use of the program such as INSIGHT II and LIBRA. INSIGHT II is available from Accelrys in USA. To carry out the threading method using INSIGHT II, SeqFold module in the program may be utilized. Meanwhile, LIBRA may be used by using the Internet and accessing the address of a homepage of DDBJ (http://www.ddbj.nig.ac.jp/search/libra_i-j.html).

As a standard to determine whether the certain protein has the similarity in the tertiary structure with the protein having the amino acid sequence of SEQ ID NO:209 or not, it is preferable to use a total assessment value (SeqFold total score (bits)) calculated by gathering up all assessment functions by the threading method when using INSIGHT II-SeqFold. It is possible to determine by calculating SeqFold total score (bits) whether the tertiary structures of the proteins are generally similar. When the threading method is carried out using the program SeqFold, various assessment values such as SeqFold (LIB) P value, SeqFold (LIB) P-value, SeqFold (LEN) P-value, SeqFold (LOW) P-value, SeqFold (High) P-value, SeqFold Total Score (raw), and SeqFold Alignment Score (raw) are calculated, and SeqFold Total Score (bits) is the total assessment value calculated by gathering up all these assessment values. The larger the value of SeqFold Total Score (bits) means that the higher the similarity between the tertiary structures of compared two proteins is. For example, when the threading method is carried out using INSIGHT II, it seems to be reasonable that a threshold for determining whether or not the protein has the similar tertiary structure to that of the protein having the amino acid sequence of SEQ ID NO:209 is about 90 as the value of SeqFold Total Score (bits). That is, if the value of SeqFold Total Score (bits) is 90 or more, it may be appropriate to determine that the tertiary structure of the protein having the amino acid sequence of SEQ ID NO:209 and the tertiary structure of the protein in question have the similarity. The more preferable threshold is 110 or more, still more preferably 130 or more and particularly preferably 150 or more as the value of SeqFold Total Score.

When it is determined that the protein in question has the similar tertiary structure to that of the protein having the amino acid sequence of SEQ ID NO:209, the amino acid residues in the sequence of the determined protein corresponding to the amino acid residues present within 15 angstroms from the active residue Ser158 of the protein having the amino acid sequence of SEQ ID NO:209 are specified. The objective amino acid residues may be specified by the alignment of the three dimensional structure of the objective protein with the protein having the amino acid sequence of SEQ ID NO:209, which is obtained in the process of determining the similarity of the three dimensional structure by the threading method.

In the method for the design and production of the present invention, the peptide other than the peptide having the amino acid sequence of SEQ ID NO:208 may also be subjected to the changing of at least a part of the predicted substrate binding site, to find out the mutant protein having the enhanced peptide-synthesizing activity. It is possible combine mutations each of which has brought about the enhanced activity, to create a mutant having a further enhanced activity by their synergistic effect. As used herein, "changing of at least a part of the substrate binding site" means modification of one or more residues in the amino acid residues which configure the substrate binding site, particularly substituting, inserting or deleting, and preferably substituting with the other amino acid residues, with a proviso that the mutant protein after changing has the peptide-synthesizing activity. The number of the amino acid residues subjected to the modification varies depending on the position and the type of the amino acid residues, and may be suitably determined in the range in which the tertiary structure and the activity of the resulting mutant protein are not significantly impaired.

For example, one or more amino acid residues in the amino acid sequence of the protein in question may be substituted, inserted or deleted at the position(s) corresponding to the positions 67 to 70, 72 to 88, 100, 102, 103, 106, 107, 113 to 117, 130, 155 to 163, 165, 166, 180 to 188, 190 to 195, 200 to 235, 259, 273, 276, 278, 292 to 294, 296, 298, 299, 300 to 304, 325 to 328, 330 to 340 and 437 to 447 in the amino acid sequence of SEQ ID NO:209, the correspondence being made in the three-dimensional alignment of the protein in question with the protein having the amino acid sequence of SEQ ID NO:209 upon the determination by the threading method. Specifically, the desired mutant protein may be obtained by substituting one or more amino acid residues among the amino acid residues at the aforementioned corresponding (overlapping) positions as a result of the alignment, with another amino acid residue.

It is preferable that the mutant protein to be designed has the homology to some extent with the protein having the amino acid sequence of SEQ ID NO:207 in terms of their primary sequences. The homology may be, for example, 25% or more, more preferably 50% or more, still more preferably 80% or more and particularly preferably 90% or more.

It is possible to find out the mutant protein having the enhanced peptide-synthesizing activity by changing at least a part of the amino acid positions, i.e., substituting one or more amino acid residue, in the aforementioned range of the amino acid residues. It is also possible to combine mutations each of which has brought about the enhanced activity, to create a mutant protein having further enhanced peptide-synthesizing activity by their synergistic effect. Meanwhile, in the enhancement of the peptide-synthesizing activity by the mutation, changing of even one atom of a side chain in the amino acid residue may possibly result in a drastic change. Therefore, there are various possibilities for the optimization. For example, if mutation of a certain position reveals that the position is involved in enhancement of the activity, random mutation on several residues neighboring the position in the tertiary structure may result in discovery of a mutant having a further enhanced activity. That is, it is possible to obtain a mutant protein having a peptide-synthesizing activity by modification of at least a part of positions which configure a continuous surface in terms of a tertiary structure with an amino acid residue whose modification brings about enhancement of the peptide-synthesizing activity.

In the protein other than the protein having the amino acid sequence of SEQ ID NO:208, "the position which configures a continuous surface in terms of the tertiary structure with an amino acid residue whose modification brings about enhancement of the peptide-synthesizing activity" is a position which configures a surface (plane) facing the substrate binding site (Ser158) with base positions that are the positions of the amino acid residues which correspond to the positions 67 to 70, 72 to 88, 100, 102, 103, 106, 107, 113 to 117, 130, 155 to 163, 165, 166, 180 to 188, 190 to 195, 200 to 235, 259, 273, 276, 278, 292 to 294, 296, 298, 299, 300 to 304, 325 to 328, 330 to 340 and 437 to 447 in the amino acid sequence of SEQ ID NO:209, the correspondence being made in the three-dimensional threading alignment of the protein in question with the protein having the amino acid sequence of SEQ ID NO:209. Specifically, it is possible to obtain the mutant protein having the peptide-synthesizing activity by causing one or more changes selected from the following (a') to (i').
(a') At least one or more amino acid residue substitutions, insertions or deletions in the tertiary structure corresponding to any of positions 79 to 82 in the amino acid sequence of SEQ ID NO:209
(b') At least one or more amino acid residue substitutions, insertions or deletions in the tertiary structure corresponding to any of positions 84, 88, 89 and 92 in the amino acid sequence of SEQ ID NO:209
(c') At least one or more amino acid residue substitutions, insertions or deletions in the tertiary structure corresponding to any of positions 72, 75 and 77 in the amino acid sequence of SEQ ID NO:209
(d') At least one or more amino acid residue substitutions, insertions or deletions in the tertiary structure corresponding to any of positions 159, 161, 162, 184, 187 and 276 in the amino acid sequence of SEQ ID NO:209
(e') At least one or more amino acid residue substitutions, insertions or deletions in the tertiary structure corresponding to any of positions 70, 106, 113, 115, 193, 207, 209 to 212, 216 and 259 in the amino acid sequence of SEQ ID NO:209
(f') At least one or more amino acid residue substitutions, insertions or deletions in the tertiary structure corresponding to any of positions 200, 202 to 205, 207 and 228 in the amino acid sequence of SEQ ID NO:209
(g') At least one or more amino acid residue substitutions, insertions or deletions in the tertiary structure corresponding to any of positions 233, 234 and 439 in the amino acid sequence of SEQ ID NO:209
(h') At least one or more amino acid residue substitutions, insertions or deletions in the tertiary structure corresponding to any of positions 328, 339, 340, 445 and 446 in the amino acid sequence of SEQ ID NO:209
(i') At least one or more amino acid residue substitutions, insertions or deletions in the tertiary structure corresponding to any of positions 87, 155, 157 and 160 in the amino acid sequence of SEQ ID NO:209

It is also possible to obtain a mutant protein having a peptide-synthesizing activity by causing one or more changes selected from the following (a'') to (i'') in those having the homology of 25% or more in the primary sequence when the primary sequence alignment or the tertiary structure alignment of the protein in question with the protein having the amino acid sequence of SEQ ID NO:209 is performed.
(a'') At least one or more amino acid residue substitutions, insertions or deletions in the tertiary structure corresponding to any of positions 79 to 82 in the amino acid sequence of SEQ ID NO:209
(b'') At least one or more amino acid residue substitutions, insertions or deletions in the tertiary structure corresponding to any of positions 84, 88, 89 and 92 in the amino acid sequence of SEQ ID NO:209
(c'') At least one or more amino acid residue substitutions, insertions or deletions in the tertiary structure corresponding to any of positions 72, 75 and 77 in the amino acid sequence of SEQ ID NO:209
(d'') At least one or more amino acid residue substitutions, insertions or deletions in the tertiary structure corresponding to any of positions 159, 161, 162, 184, 187 and 276 in the amino acid sequence of SEQ ID NO:209
(e'') At least one or more amino acid residue substitutions, insertions or deletions in the tertiary structure corresponding to any of positions 70, 106, 113, 115, 193, 207, 209 to 212, 216 and 259 in the amino acid sequence of SEQ ID NO:209
(f'') At least one or more amino acid residue substitutions, insertions or deletions in the tertiary structure corresponding to any of positions 200, 202 to 205, 207 and 228 in the amino acid sequence of SEQ ID NO:209
(g'') At least one or more amino acid residue substitutions, insertions or deletions in the tertiary structure corresponding to any of positions 233, 234 and 439 in the amino acid sequence of SEQ ID NO:209
(h'') At least one or more amino acid residue substitutions, insertions or deletions in the tertiary structure corresponding to any of positions 328, 339, 340, 445 and 446 in the amino acid sequence of SEQ ID NO:209
(i'') At least one or more amino acid residue substitutions, insertions or deletions in the tertiary structure corresponding to any of positions 87, 155, 157 and 160 in the amino acid sequence of SEQ ID NO:209

### 4. Proteins having peptide-synthesizing activity of the present invention (mutant proteins based on amino acid sequence of SEQ ID NO:208)

The protein of the present invention is the mutant protein designed and produced by the methods for the design and production described in the sections 2 and 3 above, and specifically is the mutant protein having the amino acid sequence where one or more mutations from any of the following mutations L1 to L335 or the following mutations M1 to M642 have been introduced into the amino acid sequence of SEQ ID NO:208 and having the peptide-synthesizing activity (these proteins may be referred to hereinbelow as the "mutant protein (I') of the protein having the amino acid sequence of SEQ ID NO:208"). The mutations L1 to L335, and the mutations M1 to M642 are as shown in Tables 2-1 to 2-19.

### Table 2-1

**Table 2-1**

| MUTATION ID | MUTATION |
|---|---|
| MUTATION L1 | N67K |
| MUTATION L2 | N67L |
| MUTATION L3 | N67S |
| MUTATION L4 | T69I |
| MUTATION L5 | T69M |
| MUTATION L6 | T69Q |
| MUTATION L7 | T69R |
| MUTATION L8 | T69V |
| MUTATION L9 | P70G |
| MUTATION L10 | P70N |
| MUTATION L11 | P70S |
| MUTATION L12 | P70T |
| MUTATION L13 | P70V |
| MUTATION L14 | A72C |
| MUTATION L15 | A72D |
| MUTATION L16 | A72E |
| MUTATION L17 | A721 |
| MUTATION L18 | A72L |
| MUTATION L19 | A72M |
| MUTATION L20 | A72N |
| MUTATION L21 | A72Q |
| MUTATION L22 | A72S |
| MUTATION L23 | A72V |
| MUTATION L24 | V73A |
| MUTATION L25 | V731 |
| MUTATION L26 | V73L |
| MUTATION L27 | V73M |
| MUTATION L28 | V73N |
| MUTATION L29 | V73S |
| MUTATION L30 | V73T |
| MUTATION L31 | S74A |
| MUTATION L32 | S74F |
| MUTATION L33 | S74K |
| MUTATION L34 | S74N |
| MUTATION L35 | S74T |
| MUTATION L36 | S74V |
| MUTATION L37 | P75A |
| MUTATION L38 | P75D |
| MUTATION L39 | P75L |
| MUTATION L40 | P75S |
| MUTATION L41 | Y76F |
| MUTATION L42 | Y76H |
| MUTATION L43 | Y76I |
| MUTATION L44 | Y76V |
| MUTATION L45 | Y76W |
| MUTATION L46 | G77A |
| MUTATION L47 | G77F |
| MUTATION L48 | G77K |
| MUTATION L49 | G77M |
| MUTATION L50 | G77N |
| MUTATION L51 | G77P |
| MUTATION L52 | G77S |
| MUTATION L53 | G77T |

### Table 2-2

**Table 2-2**

| MUTATION ID | MUTATION |
|---|---|
| MUTATION L54 | Q78F |
| MUTATION L55 | Q78L |
| MUTATION L56 | N79D |
| MUTATION L57 | N79L |
| MUTATION L58 | N79R |
| MUTATION L59 | N79S |
| MUTATION L60 | E80D |
| MUTATION L61 | E80F |
| MUTATION L62 | E80L |
| MUTATION L63 | E80P |
| MUTATION L64 | E80S |
| MUTATION L65 | Y81A |
| MUTATION L66 | Y81C |
| MUTATION L67 | Y81 D |
| MUTATION L68 | Y81 E |
| MUTATION L69 | Y81F |
| MUTATION L70 | Y81H |
| MUTATION L71 | Y81 K |
| MUTATION L72 | Y81 L |
| MUTATION L73 | Y81 N |
| MUTATION L74 | Y81S |
| MUTATION L75 | Y81T |
| MUTATION L76 | Y81W |
| MUTATION L77 | K82D |
| MUTATION L78 | K82L |
| MUTATION L79 | K82P |
| MUTATION L80 | K82S |
| MUTATION L81 | K83D |
| MUTATION L82 | K83F |
| MUTATION L83 | K83L |
| MUTATION L84 | K83P |
| MUTATION L85 | K83S |
| MUTATION L86 | K83V |
| MUTATION L87 | S84D |
| MUTATION L88 | S84F |
| MUTATION L89 | S84K |
| MUTATION L90 | S84L |
| MUTATION L91 | S84N |
| MUTATION L92 | S84Q |
| MUTATION L93 | L85F |
| MUTATION L94 | L85I |
| MUTATION L95 | L85P |
| MUTATION L96 | L85V |
| MUTATION L97 | N87E |
| MUTATION L98 | N87Q |
| MUTATION L99 | F88E |
| MUTATION L100 | V103I |
| MUTATION L101 | V103L |
| MUTATION L102 | K106A |
| MUTATION L103 | K106F |
| MUTATION L104 | K106L |
| MUTATION L105 | K106Q |
| MUTATION L106 | K106S |
| MUTATION L107 | W107A |

### Table 2-3

**Table 2-3**

| MUTATION ID | MUTATION |
|---|---|
| MUTATION L108 | W107Y |
| MUTATION L109 | F113A |
| MUTATION L110 | F113W |
| MUTATION L111 | F113Y |
| MUTATION L112 | E114A |
| MUTATION L113 | E114D |
| MUTATION L114 | D115E |
| MUTATION L115 | D115Q |
| MUTATION L116 | D115S |
| MUTATION L117 | I116F |
| MUTATION L118 | I116K |
| MUTATION L119 | I116L |
| MUTATION L120 | I116M |
| MUTATION L121 | I116N |
| MUTATION L122 | I116T |
| MUTATION L123 | I116V |
| MUTATION L124 | I157K |
| MUTATION L125 | I157L |
| MUTATION L126 | Y159G |
| MUTATION L127 | Y159N |
| MUTATION L128 | Y159S |
| MUTATION L129 | P160G |
| MUTATION L130 | G161A |
| MUTATION L131 | F162L |
| MUTATION L132 | F162Y |
| MUTATION L133 | Y1631 |
| MUTATION L134 | T165V |
| MUTATION L135 | Q181F |
| MUTATION L136 | A182G |
| MUTATION L137 | A182S |
| MUTATION L138 | P183A |
| MUTATION L139 | P183G |
| MUTATION L140 | P183S |
| MUTATION L141 | T185A |
| MUTATION L142 | T185G |
| MUTATION L143 | T185V |
| MUTATION L144 | W187A |
| MUTATION L145 | W187F |
| MUTATION L146 | W187H |
| MUTATION L147 | W187Y |
| MUTATION L148 | Y188F |
| MUTATION L149 | Y188L |
| MUTATION L150 | Y188W |
| MUTATION L151 | G190A |
| MUTATION L152 | G190D |
| MUTATION L153 | F193W |
| MUTATION L154 | H194D |
| MUTATION L155 | F200A |
| MUTATION L156 | F200L |
| MUTATION L157 | F200S |
| MUTATION L158 | F200V |
| MUTATION L159 | L201Q |
| MUTATION L160 | L201S |
| MUTATION L161 | Q202A |

### Table 2-4

**Table 2-4**

| MUTATION ID | MUTATION |
|---|---|
| MUTATION L162 | Q202D |
| MUTATION L163 | Q202F |
| MUTATION L164 | Q202S |
| MUTATION L165 | Q202T |
| MUTATION L166 | Q202V |
| MUTATION L167 | D203E |
| MUTATION L168 | A204G |
| MUTATION L169 | A204L |
| MUTATION L170 | A204S |
| MUTATION L171 | A204T |
| MUTATION L172 | A204V |
| MUTATION L173 | F205L |
| MUTATION L174 | F205Q |
| MUTATION L175 | F205V |
| MUTATION L176 | F205W |
| MUTATION L177 | T206F |
| MUTATION L178 | T206K |
| MUTATION L179 | T206L |
| MUTATION L180 | F207I |
| MUTATION L181 | F207W |
| MUTATION L182 | F207Y |
| MUTATION L183 | M208A |
| MUTATION L184 | M208L |
| MUTATION L185 | S209F |
| MUTATION L186 | S209K |
| MUTATION L187 | S209L |
| MUTATION L188 | S209N |
| MUTATION L189 | S209V |
| MUTATION L190 | T210A |
| MUTATION L191 | T210L |
| MUTATION L192 | T210Q |
| MUTATION L193 | T210V |
| MUTATION L194 | F211A |
| MUTATION L195 | F211I |
| MUTATION L196 | F211L |
| MUTATION L197 | F211M |
| MUTATION L198 | F211V |
| MUTATION L199 | F211W |
| MUTATION L200 | F211Y |
| MUTATION L201 | G212A |
| MUTATION L202 | V213D |
| MUTATION L203 | V213F |
| MUTATION L204 | V213K |
| MUTATION L205 | V213S |
| MUTATION L206 | P214D |
| MUTATION L207 | P214F |
| MUTATION L208 | P214K |
| MUTATION L209 | P214S |
| MUTATION L210 | R215A |
| MUTATION L211 | R2151 |
| MUTATION L212 | R215K |
| MUTATION L213 | R215Q |
| MUTATION L214 | R215S |
| MUTATION L215 | R215T |

### Table 2-5

**Table 2-5**

| MUTATION ID | MUTATION |
|---|---|
| MUTATION L216 | R215Y |
| MUTATION L217 | P216D |
| MUTATION L218 | P216K |
| MUTATION L219 | K217D |
| MUTATION L220 | P218F |
| MUTATION L221 | P218L |
| MUTATION L222 | P218Q |
| MUTATION L223 | P218S |
| MUTATION L224 | I219D |
| MUTATION L225 | I219F |
| MUTATION L226 | I219K |
| MUTATION L227 | T220A |
| MUTATION L228 | T220D |
| MUTATION L229 | T220F |
| MUTATION L230 | T220K |
| MUTATION L231 | T220L |
| MUTATION L232 | T220S |
| MUTATION L233 | P221 A |
| MUTATION L234 | P221 D |
| MUTATION L235 | P221F |
| MUTATION L236 | P221K |
| MUTATION L237 | P221L |
| MUTATION L238 | P221 S |
| MUTATION L239 | D222A |
| MUTATION L240 | D222F |
| MUTATION L241 | D222L |
| MUTATION L242 | D222R |
| MUTATION L243 | Q223F |
| MUTATION L244 | Q223K |
| MUTATION L245 | Q223L |
| MUTATION L246 | Q223S |
| MUTATION L247 | F224A |
| MUTATION L248 | F224D |
| MUTATION L249 | F224G |
| MUTATION L250 | F224K |
| MUTATION L251 | F224L |
| MUTATION L252 | K225D |
| MUTATION L253 | K225G |
| MUTATION L254 | K225S |
| MUTATION L255 | G226A |
| MUTATION L256 | G226F |
| MUTATION L257 | G226L |
| MUTATION L258 | G226N |
| MUTATION L259 | G226S |
| MUTATION L260 | K227D |
| MUTATION L261 | K227F |
| MUTATION L262 | K227S |
| MUTATION L263 | I228A |
| MUTATION L264 | I228F |
| MUTATION L265 | I228K |
| MUTATION L266 | I228S |
| MUTATION L267 | P229A |
| MUTATION L268 | P229D |
| MUTATION L269 | P229K |

### Table 2-6

**Table 2-6**

| MUTATION ID | MUTATION |
|---|---|
| MUTATION L270 | P229L |
| MUTATION L271 | P229S |
| MUTATION L272 | I230A |
| MUTATION L273 | I230F |
| MUTATION L274 | 1230K |
| MUTATION L275 | I230S |
| MUTATION L276 | K231F |
| MUTATION L277 | K231L |
| MUTATION L278 | K231S |
| MUTATION L279 | E232D |
| MUTATION L280 | E232F |
| MUTATION L281 | E232G |
| MUTATION L282 | E232L |
| MUTATION L283 | E232S |
| MUTATION L284 | A233D |
| MUTATION L285 | A233F |
| MUTATION L286 | A233H |
| MUTATION L287 | A233K |
| MUTATION L288 | A233L |
| MUTATION L289 | A233N |
| MUTATION L290 | A233S |
| MUTATION L291 | D234L |
| MUTATION L292 | D234S |
| MUTATION L293 | K235D |
| MUTATION L294 | K235F |
| MUTATION L295 | K235L |
| MUTATION L296 | K235S |
| MUTATION L297 | F259Y |
| MUTATION L298 | R276A |
| MUTATION L299 | R276Q |
| MUTATION L300 | A298S |
| MUTATION L301 | D300N |
| MUTATION L302 | V301M |
| MUTATION L303 | Y328F |
| MUTATION L304 | Y328H |
| MUTATION L305 | Y328M |
| MUTATION L306 | Y328W |
| MUTATION L307 | W332H |
| MUTATION L308 | E336A |
| MUTATION L309 | N338A |
| MUTATION L310 | N338F |
| MUTATION L311 | Y339K |
| MUTATION L312 | Y339L |
| MUTATION L313 | Y339T |
| MUTATION L314 | L340A |
| MUTATION L315 | L3401 |
| MUTATION L316 | L340V |
| MUTATION L317 | V439P |
| MUTATION L318 | I440F |
| MUTATION L319 | I440V |
| MUTATION L320 | E441 F |
| MUTATION L321 | E441M |
| MUTATION L322 | E441N |
| MUTATION L323 | N442A |

### Table 2-7

**Table 2-7**

| MUTATION ID | MUTATION |
|---|---|
| MUTATION L324 | N442L |
| MUTATION L325 | R443S |
| MUTATION L326 | T444W |
| MUTATION L327 | R445G |
| MUTATION L328 | R445K |
| MUTATION L329 | E446A |
| MUTATION L330 | E446F |
| MUTATION L331 | E446Q |
| MUTATION L332 | E446S |
| MUTATION L333 | E446T |
| MUTATION L334 | Y447L |
| MUTATION L335 | Y447S |

### Table 2-8

**Table 2-8**

| MUTATION ID | MUTATION | | | | | |
|---|---|---|---|---|---|---|
| MUTATION M1 | T69N | I157L | | | | |
| MUTATION M2 | T69Q | I157L | | | | |
| MUTATION M3 | T69S | I157L | | | | |
| MUTATION M4 | P70A | I157L | | | | |
| MUTATION M5 | P70G | I157L | | | | |
| MUTATION M6 | P70I | I157L | | | | |
| MUTATION M7 | P70L | I157L | | | | |
| MUTATION M8 | P70N | I157L | | | | |
| MUTATION M9 | P70S | I157L | | | | |
| MUTATION M10 | P70T | I157L | | | | |
| MUTATION M11 | P70T | T210L | | | | |
| MUTATION M12 | P70T | Y328F | | | | |
| MUTATION M13 | P70V | l157L | | | | |
| MUTATION M14 | A72E | G77S | | | | |
| MUTATION M15 | A72E | E80D | | | | |
| MUTATION M16 | A72E | Y81A | | | | |
| MUTATION M17 | A72E | S84D | | | | |
| MUTATION M18 | A72E | F113W | | | | |
| MUTATION M19 | A72E | 1157L | | | | |
| MUTATION M20 | A72E | G161A | | | | |
| MUTATION M21 | A72E | F162L | | | | |
| MUTATION M22 | A72E | A184G | | | | |
| MUTATION M23 | A72E | W187F | | | | |
| MUTATION M24 | A72E | F200A | | | | |
| MUTATION M25 | A72E | A204S | | | | |
| MUTATION M26 | A72E | T210L | | | | |
| MUTATION M27 | A72E | F211L | | | | |
| MUTATION M28 | A72E | F211W | | | | |
| MUTATION M29 | A72E | G226A | | | | |
| MUTATION M30 | A72E | 1228K | | | | |
| MUTATION M31 | A72E | A233D | | | | |
| MUTATION M32 | A72E | Y328F | | | | |
| MUTATION M33 | A72S | I157L | | | | |
| MUTATION M34 | A72V | Y328F | | | | |
| MUTATION M35 | V73A | I157L | | | | |
| MUTATION M36 | V731 | 1157L | | | | |
| MUTATION M37 | S74A | 1157L | | | | |
| MUTATION M38 | S74N | I157L | | | | |
| MUTATION M39 | S74T | 1157L | | | | |
| MUTATION M40 | S74V | 1157L | | | | |
| MUTATION M41 | G77A | 1157L | | | | |
| MUTATION M42 | G77F | 1157L | | | | |
| MUTATION M43 | G77M | 1157L | | | | |
| MUTATION M44 | G77P | I157L | | | | |
| MUTATION M45 | G77S | E80D | | | | |
| MUTATION M46 | G77S | Y81A | | | | |
| MUTATION M47 | G77S | S84D | | | | |
| MUTATION M48 | G77S | F113W | | | | |
| MUTATION M49 | G77S | I157L | | | | |
| MUTATION M50 | G77S | Y159N | | | | |
| MUTATION M51 | G77S | Y159S | | | | |
| MUTATION M52 | G77S | G161A | | | | |
| MUTATION M53 | G77S | F162L | | | | |

### Table 2-9

**Table 2-9**

| MUTATION ID | MUTATION | | | | | |
|---|---|---|---|---|---|---|
| MUTATION M54 | G77S | A184G | | | | |
| MUTATION M55 | G77S | W187F | | | | |
| MUTATION M56 | G77S | F200A | | | | |
| MUTATION M57 | G77S | A204S | | | | |
| MUTATION M58 | G77S | T210L | | | | |
| MUTATION M59 | G77S | F211L | | | | |
| MUTATION M60 | G77S | F211W | | | | |
| MUTATION M61 | G77S | 1228K | | | | |
| MUTATION M62 | G77S | A233D | | | | |
| MUTATION M63 | G77S | R276A | | | | |
| MUTATION M64 | G77S | Y328F | | | | |
| MUTATION M65 | E80D | Y81A | | | | |
| MUTATION M66 | E80D | F113W | | | | |
| MUTATION M67 | E80D | I157L | | | | |
| MUTATION M68 | E80D | Y159N | | | | |
| MUTATION M69 | E80D | G161A | | | | |
| MUTATION M70 | E80D | A184G | | | | |
| MUTATION M71 | E80D | F211W | | | | |
| MUTATION M72 | E80D | Y328F | | | | |
| MUTATION M73 | E80S | l157L | | | | |
| MUTATION M74 | Y81A | F113W | | | | |
| MUTATION M75 | Y81A | I157L | | | | |
| MUTATION M76 | Y81A | Y159N | | | | |
| MUTATION M77 | Y81A | Y159S | | | | |
| MUTATION M78 | Y81A | G161A | | | | |
| MUTATION M79 | Y81A | A184G | | | | |
| MUTATION M80 | Y81A | W187F | | | | |
| MUTATION M81 | Y81A | F200A | | | | |
| MUTATION M82 | Y81A | T210L | | | | |
| MUTATION M83 | Y81A | F211W | | | | |
| MUTATION M84 | Y81A | F211Y | | | | |
| MUTATION M85 | Y81A | G226A | | | | |
| MUTATION M86 | Y81A | I228K | | | | |
| MUTATION M87 | Y81A | A233D | | | | |
| MUTATION M88 | Y81A | Y328F | | | | |
| MUTATION M89 | Y81H | I157L | | | | |
| MUTATION M90 | Y81N | I157L | | | | |
| MUTATION M91 | K83P | l157L | | | | |
| MUTATION M92 | S84A | I157L | | | | |
| MUTATION M93 | S84D | F113W | | | | |
| MUTATION M94 | S84D | I157L | | | | |
| MUTATION M95 | S84D | Y159N | | | | |
| MUTATION M96 | S84D | G161A | | | | |
| MUTATION M97 | S84D | A184G | | | | |
| MUTATION M98 | S84D | Y328F | | | | |
| MUTATION M99 | S84E | I157L | | | | |
| MUTATION M100 | S84F | I157L | | | | |
| MUTATION M101 | S84K | I157L | | | | |
| MUTATION M102 | L85F | I157L | | | | |
| MUTATION M103 | L85I | I157L | | | | |
| MUTATION M104 | L85P | I157L | | | | |
| MUTATION M105 | L85V | I157L | | | | |
| MUTATION M106 | N87A | I157L | | | | |
| MUTATION M107 | N87D | I157L | | | | |

### Table 2-10

**Table 2-10**

| MUTATION ID | MUTAION | | | | | |
|---|---|---|---|---|---|---|
| MUTATION M108 | N87E | I157L | | | | |
| MUTATION M109 | N87G | I157L | | | | |
| MUTATION M110 | N87Q | I157L | | | | |
| MUTATION M111 | N87S | I157L | | | | |
| MUTATION M112 | F88A | I157L | | | | |
| MUTATION M113 | F88D | I157L | | | | |
| MUTATION M114 | F88E | I157L | | | | |
| MUTATION M115 | F88E | Y328F | | | | |
| MUTATION M116 | F88L | I157L | | | | |
| MUTATION M117 | F88T | I157L | | | | |
| MUTATION M118 | F88V | l157L | | | | |
| MUTATION M119 | F88Y | l157L | | | | |
| MUTATION M120 | K106H | l157L | | | | |
| MUTATION M121 | K106L | I157L | | | | |
| MUTATION M122 | K106M | l157L | | | | |
| MUTATION M123 | K106Q | l157L | | | | |
| MUTATION M124 | K106R | 1157L | | | | |
| MUTATION M125 | K106S | I157L | | | | |
| MUTATION M126 | K106V | l157L | | | | |
| MUTATION M127 | W107A | 1157L | | | | |
| MUTATION M128 | W107A | Y328F | | | | |
| MUTATION M129 | W107Y | I157L | | | | |
| MUTATION M130 | W107Y | T206Y | | | | |
| MUTATION M131 | W107Y | K217D | | | | |
| MUTATION M132 | W107Y | P218L | | | | |
| MUTATION M133 | W107Y | T220L | | | | |
| MUTATION M134 | W107Y | P221 D | | | | |
| MUTATION M135 | W107Y | Y328F | | | | |
| MUTATION M136 | F113A | I157L | | | | |
| MUTATION M137 | F113H | I157L | | | | |
| MUTATION M138 | F113N | I157L | | | | |
| MUTATION M139 | F113V | I157L | | | | |
| MUTATION M140 | F113W | I157L | | | | |
| MUTATION M141 | F113W | Y159N | | | | |
| MUTATION M142 | F113W | Y159S | | | | |
| MUTATION M143 | F113W | G161A | | | | |
| MUTATION M144 | F113W | F162L | | | | |
| MUTATION M145 | F113W | A184G | | | | |
| MUTATION M146 | F113W | W187F | | | | |
| MUTATION M147 | F113W | F200A | | | | |
| MUTATION M148 | F113W | T206Y | | | | |
| MUTATION M149 | F113W | T210L | | | | |
| MUTATION M150 | F113W | F211 L | | | | |
| MUTATION M151 | F113W | F211W | | | | |
| MUTATION M152 | F113W | F211Y | | | | |
| MUTATION M153 | F113W | V213D | | | | |
| MUTATION M154 | F113W | K217D | | | | |
| MUTATION M155 | F113W | T220L | | | | |
| MUTATION M156 | F113W | P221 D | | | | |
| MUTATION M157 | F113W | G226A | | | | |
| MUTATION M158 | F113W | 1228K | | | | |
| MUTATION M159 | F113W | A233D | | | | |
| MUTATION M160 | F113W | R276A | | | | |
| MUTATION M161 | F113Y | I157L | | | | |

### Table 2-11

**Table 2-11**

| MUTATION ID | MUTAITON | | | | | |
|---|---|---|---|---|---|---|
| MUTATION M162 | F113Y | F211W | | | | |
| MUTATION M163 | E114D | I157L | | | | |
| MUTATION M164 | D115A | I157L | | | | |
| MUTATION M165 | D115E | I157L | | | | |
| MUTATION M166 | D115M | I157L | | | | |
| MUTATION M167 | D115N | I157L | | | | |
| MUTATION M168 | D115Q | I157L | | | | |
| MUTATION M169 | D115S | I157L | | | | |
| MUTATION M170 | D115V | I157L | | | | |
| MUTATION M171 | I157L | Y1591 | | | | |
| MUTATION M172 | I157L | Y159L | | | | |
| MUTATION M173 | I157L | Y159N | | | | |
| MUTATION M174 | I157L | Y159S | | | | |
| MUTATION M175 | I157L | Y159V | | | | |
| MUTATION M176 | I157L | P160A | | | | |
| MUTATION M177 | I157L | P160S | | | | |
| MUTATION M178 | I157L | G161A | | | | |
| MUTATION M179 | I157L | F162L | | | | |
| MUTATION M180 | I157L | F162M | | | | |
| MUTATION M181 | I157L | F162N | | | | |
| MUTATION M182 | I157L | F162Y | | | | |
| MUTATION M183 | I157L | T165L | | | | |
| MUTATION M184 | I157L | T165V | | | | |
| MUTATION M185 | I157L | Q181A | | | | |
| MUTATION M186 | I157L | Q181F | | | | |
| MUTATION M187 | I157L | Q181N | | | | |
| MUTATION M188 | I157L | A184G | | | | |
| MUTATION M189 | I157L | A184L | | | | |
| MUTATION M190 | I157L | A184M | | | | |
| MUTATION M191 | I157L | A184S | | | | |
| MUTATION M192 | I157L | A184T | | | | |
| MUTATION M193 | I157L | W187F | | | | |
| MUTATION M194 | I157L | W187Y | | | | |
| MUTATION M195 | I157L | F193H | | | | |
| MUTATION M196 | I157L | F193I | | | | |
| MUTATION M197 | I157L | F193W | | | | |
| MUTATION M198 | I157L | F200A | | | | |
| MUTATION M199 | I157L | F200H | | | | |
| MUTATION M200 | I157L | F200L | | | | |
| MUTATION M201 | I157L | F200Y | | | | |
| MUTATION M202 | I157L | A204G | | | | |
| MUTATION M203 | I157L | A2041 | | | | |
| MUTATION M204 | I157L | A204L | | | | |
| MUTATION M205 | I157L | A204S | | | | |
| MUTATION M206 | I157L | A204T | | | | |
| MUTATION M207 | I157L | A204V | | | | |
| MUTATION M208 | I157L | F205A | | | | |
| MUTATION M209 | I157L | F2071 | | | | |
| MUTATION M210 | I157L | F207M | | | | |
| MUTATION M211 | I157L | F207V | | | | |
| MUTATION M212 | I157L | F207W | | | | |
| MUTATION M213 | I157L | F207Y | | | | |
| MUTATION M214 | I157L | M208A | | | | |
| MUTATION M215 | I157L | M208K | | | | |

### Table 2-12

**Table 2-12**

| MUTATION ID | MUTATION | | | | | |
|---|---|---|---|---|---|---|
| MUTATION M216 | 1157L | M208L | | | | |
| MUTATION M217 | l157L | M208T | | | | |
| MUTATION M218 | I157L | M208V | | | | |
| MUTATION M219 | I157L | S209F | | | | |
| MUTATION M220 | I157L | S209N | | | | |
| MUTATION M221 | I157L | T210A | | | | |
| MUTATION M222 | I157L | T210L | | | | |
| MUTATION M223 | I157L | F211I | | | | |
| MUTATION M224 | I157L | F211L | | | | |
| MUTATION M225 | 1157L | F211V | | | | |
| MUTATION M226 | I157L | F211W | | | | |
| MUTATION M227 | I157L | G212A | | | | |
| MUTATION M228 | I157L | G212D | | | | |
| MUTATION M229 | I157L | G212S | | | | |
| MUTATION M230 | I157L | R215K | | | | |
| MUTATION M231 | I157L | R215L | | | | |
| MUTATION M232 | I157L | R215T | | | | |
| MUTATION M233 | I157L | R215Y | | | | |
| MUTATION M234 | I157L | T220L | | | | |
| MUTATION M235 | I157L | G226A | | | | |
| MUTATION M236 | I157L | G226F | | | | |
| MUTATION M237 | I157L | I228K | | | | |
| MUTATION M238 | I157L | A233D | | | | |
| MUTATION M239 | I157L | R276A | | | | |
| MUTATION M240 | I157L | Y328A | | | | |
| MUTATION M241 | I157L | Y328F | | | | |
| MUTATION M242 | I157L | Y328H | | | | |
| MUTATION M243 | I157L | Y328I | | | | |
| MUTATION M244 | I157L | Y328L | | | | |
| MUTATION M245 | I157L | Y328P | | | | |
| MUTATION M246 | I157L | Y328V | | | | |
| MUTATION M247 | I157L | Y328W | | | | |
| MUTATION M248 | I157L | L340F | | | | |
| MUTATION M249 | I157L | L340I | | | | |
| MUTATION M250 | I157L | L340V | | | | |
| MUTATION M251 | I157L | V439A | | | | |
| MUTATION M252 | I157L | V439P | | | | |
| MUTATION M253 | I157L | R445A | | | | |
| MUTATION M254 | I157L | R445F | | | | |
| MUTATION M255 | I157L | R445G | | | | |
| MUTATION M256 | I157L | R445K | | | | |
| MUTATION M257 | I157L | R445V | | | | |
| MUTATION M258 | Y159N | G161A | | | | |
| MUTATION M259 | Y159N | A184G | | | | |
| MUTATION M260 | Y159N | A204S | | | | |
| MUTATION M261 | Y159N | T210L | | | | |
| MUTATION M262 | Y159N | F211W | | | | |
| MUTATION M263 | Y159N | F211Y | | | | |
| MUTATION M264 | Y159N | G226A | | | | |
| MUTATION M265 | Y159N | I228K | | | | |
| MUTATION M266 | Y159N | A233D | | | | |
| MUTATION M267 | Y159N | Y328F | | | | |
| MUTATION M268 | Y159S | G161A | | | | |
| MUTATION M269 | Y159S | F211W | | | | |

### Table 2-13

**Table 2-13**

| MUTATION ID | MUTATION | | | | | |
|---|---|---|---|---|---|---|
| MUTATION M270 | G161A | F162L | | | | |
| MUTATION M271 | G161A | A184G | | | | |
| MUTATION M272 | G161A | W187F | | | | |
| MUTATION M273 | G161A | F200A | | | | |
| MUTATION M274 | G161A | A204S | | | | |
| MUTATION M275 | G161A | T210L | | | | |
| MUTATION M276 | G161A | F211L | | | | |
| MUTATION M277 | G161A | F211W | | | | |
| MUTATION M278 | G161A | G226A | | | | |
| MUTATION M279 | G161A | I228K | | | | |
| MUTATION M280 | G161A | A233D | | | | |
| MUTATION M281 | G161A | Y328F | | | | |
| MUTATION M282 | F162L | A184G | | | | |
| MUTATION M283 | F162L | F211W | | | | |
| MUTATION M284 | F162L | A233D | | | | |
| MUTATION M285 | P183A | Y328F | | | | |
| MUTATION M286 | A184G | W187F | | | | |
| MUTATION M287 | A184G | F200A | | | | |
| MUTATION M288 | A184G | A204S | | | | |
| MUTATION M289 | A184G | T210L | | | | |
| MUTATION M290 | A184G | F211L | | | | |
| MUTATION M291 | A184G | F211W | | | | |
| MUTATION M292 | A184G | I228K | | | | |
| MUTATION M293 | A184G | A233D | | | | |
| MUTATION M294 | A184G | R276A | | | | |
| MUTATION M295 | V184G | Y328F | | | | |
| MUTATION M296 | T185A | Y328F | | | | |
| MUTATION M297 | T185N | Y328F | | | | |
| MUTATION M298 | W187F | F211W | | | | |
| MUTATION M299 | W187F | Y328F | | | | |
| MUTATION M300 | F193W | F211W | | | | |
| MUTATION M301 | F200A | F211W | | | | |
| MUTATION M302 | F200A | Y328F | | | | |
| MUTATION M303 | L201Q | Y328F | | | | |
| MUTATION M304 | L201S | Y328F | | | | |
| MUTATION M305 | A204S | F211W | | | | |
| MUTATION M306 | A204S | Y328F | | | | |
| MUTATION M307 | T210L | F211W | | | | |
| MUTATION M308 | T210L | Y328F | | | | |
| MUTATION M309 | F211L | A233D | | | | |
| MUTATION M310 | F211L | Y328F | | | | |
| MUTATION M311 | F211W | I228K | | | | |
| MUTATION M312 | F211W | A233D | | | | |
| MUTATION M313 | F211W | Y328F | | | | |
| MUTATION M314 | R215A | Y328F | | | | |
| MUTATION M315 | R215L | Y328F | | | | |
| MUTATION M316 | T220L | A233D | | | | |
| MUTATION M317 | T220L | D300N | | | | |
| MUTATION M318 | P221L | A233D | | | | |
| MUTATION M319 | P221L | Y328F | | | | |
| MUTATION M320 | F224A | A233D | | | | |
| MUTATION M321 | G226A | Y328F | | | | |
| MUTATION M322 | G226F | A233D | | | | |
| MUTATION M323 | G226F | Y328F | | | | |

### Table 2-14

**Table 2-14**

| MUTATION ID | MUTATION | | | | | |
|---|---|---|---|---|---|---|
| MUTATION M324 | I228K | Y328F | | | | |
| MUTATION M325 | A233D | K235D | | | | |
| MUTATION M326 | A233D | Y328F | | | | |
| MUTATION M327 | R276A | Y328F | | | | |
| MUTATION M328 | Y328F | Y339F | | | | |
| MUTATION M329 | A27T | Y81A | S84D | | | |
| MUTATION M330 | P70T | A72E | I157L | | | |
| MUTATION M331 | P70T | G77S | I157L | | | |
| MUTATION M332 | P70T | E80D | F88E | | | |
| MUTATION M333 | P70T | Y81A | I157L | | | |
| MUTATION M334 | P70T | S84D | I157L | | | |
| MUTATION M335 | P70T | F88E | Y328F | | | |
| MUTATION M336 | P70T | F113W | I157L | | | |
| MUTATION M337 | P70T | I157L | A204S | | | |
| MUTATION M338 | P70T | I157L | T210L | | | |
| MUTATION M338 | P70T | I157L | T210L | | | |
| MUTATION M339 | P70T | I157L | A233D | | | |
| MUTATION M340 | P70T | I157L | Y328F | | | |
| MUTATION M341 | P70T | I157L | V439P | | | |
| MUTATION M342 | P70T | I157L | I440F | | | |
| MUTATION M343 | P70T | G161A | T210L | | | |
| MUTATION M344 | P70T | G161A | Y328F | | | |
| MUTATION M345 | P70T | A184G | W187F | | | |
| MUTATION M346 | P70T | A204S | Y328F | | | |
| MUTATION M347 | P70T | F211W | Y328F | | | |
| MUTATION M348 | P70V | A72E | I157L | | | |
| MUTATION M349 | A72E | S74T | I157L | | | |
| MUTATION M350 | A72E | G77S | Y328F | | | |
| MUTATION M351 | A72E | E80D | Y328F | | | |
| MUTATION M352 | A72E | Y81H | I157L | | | |
| MUTATION M353 | A72E | K83P | I157L | | | |
| MUTATION M354 | A72E | S84D | Y328F | | | |
| MUTATION M355 | A72E | L85P | I157L | | | |
| MUTATION M356 | A72E | F113W | I157L | | | |
| MUTATION M357 | A72E | F113W | Y328F | | | |
| MUTATION M358 | A72E | F113Y | I157L | | | |
| MUTATION M359 | A72E | D115Q | I157L | | | |
| MUTATION M360 | A72E | I157L | G161A | | | |
| MUTATION M361 | A72E | I157L | F162L | | | |
| MUTATION M362 | A72E | I157L | A184G | | | |
| MUTATION M363 | A72E | I157L | F200A | | | |
| MUTATION M364 | A72E | I157L | A204S | | | |
| MUTATION M365 | A72E | I157L | A204T | | | |
| MUTATION M366 | A72E | I157L | T210L | | | |
| MUTATION M367 | A72E | I157L | F211W | | | |
| MUTATION M368 | A72E | I157L | G226A | | | |
| MUTATION M369 | A72E | I157L | A233D | | | |
| MUTATION M370 | A72E | I157L | Y328F | | | |
| MUTATION M371 | A72E | I157L | L340V | | | |
| MUTATION M372 | A72E | I157L | V439P | | | |
| MUTATION M373 | A72E | G161A | Y328F | | | |
| MUTATION M374 | A72E | F162L | Y328F | | | |
| MUTATION M375 | A72E | A184G | Y328F | | | |
| MUTATION M376 | A72E | W187F | Y328F | | | |
| MUTATION M377 | A72E | F200A | Y328F | | | |

### Table 2-15

**Table 2-15**

| MUTATION ID | MUTATION | | | | | |
|---|---|---|---|---|---|---|
| MUTATION M378 | A72E | A204S | Y328F | | | |
| MUTATION M379 | A72E | T210L | Y328F | | | |
| MUTATION M380 | A72E | I228K | Y328F | | | |
| MUTATION M381 | A72E | A233D | Y328F | | | |
| MUTATION M382 | A72E | Y328F | Y159N | | | |
| MUTATION M383 | A72E | Y328F | F211W | | | |
| MUTATION M384 | A72E | Y328F | F211Y | | | |
| MUTATION M385 | A72E | Y328F | G226A | | | |
| MUTATION M386 | A72V | Y81A | Y328F | | | |
| MUTATION M387 | A72V | G161A | Y328F | | | |
| MUTATION M388 | G77M | I157L | T210L | | | |
| MUTATION M389 | G77P | I157L | F162L | | | |
| MUTATION M390 | G77P | I157L | A184G | | | |
| MUTATION M391 | G77P | F211W | Y328F | | | |
| MUTATION M392 | G77S | Y81A | Y328F | | | |
| MUTATION M393 | G77S | S84D | l157L | | | |
| MUTATION M394 | G77S | F88E | l157L | | | |
| MUTATION M395 | G77S | F113W | l157L | | | |
| MUTATION M396 | G77S | F113Y | l157L | | | |
| MUTATION M397 | G77S | D115Q | l157L | | | |
| MUTATION M398 | G77S | I157L | G161A | | | |
| MUTATION M399 | G77S | I157L | F200A | | | |
| MUTATION M400 | G77S | I157L | A204S | | | |
| MUTATION M401 | G77S | I157L | T210L | | | |
| MUTATION M402 | G77S | I157L | F211 W | | | |
| MUTATION M403 | G77S | I157L | G226A | | | |
| MUTATION M404 | G77S | I157L | A233D | | | |
| MUTATION M405 | G77S | I157L | L340V | | | |
| MUTATION M406 | G77S | I157L | V439P | | | |
| MUTATION M407 | G77S | G161A | Y328F | | | |
| MUTATION M408 | E80D | Y81A | Y328F | | | |
| MUTATION M409 | Y81A | S84D | Y328F | | | |
| MUTATION M410 | Y81A | F113W | Y328F | | | |
| MUTATION M411 | Y81A | I157L | T210L | | | |
| MUTATION M412 | Y81A | I157L | Y328F | | | |
| MUTATION M413 | Y81A | G161A | Y328F | | | |
| MUTATION M414 | Y81A | F162L | Y328F | | | |
| MUTATION M415 | Y81A | A184G | Y328F | | | |
| MUTATION M416 | Y81A | W187F | Y328F | | | |
| MUTATION M417 | Y81A | A204S | Y328F | | | |
| MUTATION M418 | Y81A | T210L | Y328F | | | |
| MUTATION M419 | Y81A | 1228K | Y328F | | | |
| MUTATION M420 | Y81A | A233D | Y328F | | | |
| MUTATION M421 | Y81A | Y328F | Y159N | | | |
| MUTATION M422 | Y81A | Y328F | Y159S | | | |
| MUTATION M423 | Y81A | Y328F | F211W | | | |
| MUTATION M424 | Y81A | Y328F | F211Y | | | |
| MUTATION M425 | Y81A | Y328F | G226A | | | |
| MUTATION M426 | Y81A | Y328F | R276A | | | |
| MUTATION M427 | K83P | I157L | A184G | | | |
| MUTATION M428 | K83P | I157L | T210L | | | |
| MUTATION M429 | K83P | F211W | Y328F | | | |
| MUTATION M430 | S84D | F113W | I157L | | | |
| MUTATION M431 | S84D | I157L | T210L | | | |

### Table 2-16

**Table 2-16**

| MUTATION ID | MUTATION | | | | | |
|---|---|---|---|---|---|---|
| MUTATION M432 | F88E | I157L | F162L | | | |
| MUTATION M433 | F88E | I157L | A184G | | | |
| MUTATION M434 | F88E | I157L | F200A | | | |
| MUTATION M435 | F88E | I157L | T210L | | | |
| MUTATION M436 | F88E | 1157L | Y328F | | | |
| MUTATION M437 | F88E | I157L | Y328Q | | | |
| MUTATION M438 | F88E | I157L | L340V | | | |
| MUTATION M439 | F88E | T210L | Y328F | | | |
| MUTATION M440 | F88E | F211W | Y328F | | | |
| MUTATION M441 | F113W | I157L | G161A | | | |
| MUTATION M442 | F113W | I157L | A184G | | | |
| MUTATION M443 | F113W | 1157L | W187F | | | |
| MUTATION M444 | F113W | I157L | F200A | | | |
| MUTATION M445 | F113W | I157L | A204S | | | |
| MUTATION M446 | F113W | I157L | A204T | | | |
| MUTATION M447 | F113W | I157L | T210L | | | |
| MUTATION M448 | F113W | I157L | F211W | | | |
| MUTATION M449 | F113W | l157L | G226A | | | |
| MUTATION M450 | F113W | I157L | A233D | | | |
| MUTATION M451 | F113W | I157L | Y328F | | | |
| MUTATION M452 | F113W | I157L | L340V | | | |
| MUTATION M453 | F113W | I157L | V439P | | | |
| MUTATION M454 | F113W | G161A | T210L | | | |
| MUTATION M455 | F113W | G161A | Y328F | | | |
| MUTATION M456 | F113W | A184G | W187F | | | |
| MUTATION M457 | F113Y | I157L | T210L | | | |
| MUTATION M458 | F113Y | I157L | Y328F | | | |
| MUTATION M459 | F113Y | G161A | T210L | | | |
| MUTATION M460 | D115Q | 1157L | T210L | | | |
| MUTATION M461 | D115Q | I157L | Y328F | | | |
| MUTATION M462 | I157L | Y159N | T210L | | | |
| MUTATION M463 | I157L | Y159N | Y328F | | | |
| MUTATION M464 | I157L | G161A | W187F | | | |
| MUTATION M465 | I157L | G161A | F200A | | | |
| MUTATION M466 | I157L | G161A | A204S | | | |
| MUTATION M467 | I157L | G161A | T210L | | | |
| MUTATION M468 | I157L | G161A | A233D | | | |
| MUTATION M469 | I157L | G161A | Y328F | | | |
| MUTATION M470 | I157L | F162L | A184G | | | |
| MUTATION M471 | I157L | F162L | T210L | | | |
| MUTATION M472 | I157L | F162L | L340V | | | |
| MUTATION M473 | I157L | A184G | W187F | | | |
| MUTATION M474 | I157L | A184G | F200A | | | |
| MUTATION M475 | I157L | A184G | A204T | | | |
| MUTATION M476 | I157L | A184G | T210L | | | |
| MUTATION M477 | I157L | A184G | F211W | | | |
| MUTATION M478 | I157L | A184G | L340V | | | |
| MUTATION M479 | I157L | W187F | T210L | | | |
| MUTATION M480 | I157L | W187F | Y328F | | | |
| MUTATION M481 | I157L | F200A | T210L | | | |
| MUTATION M482 | I157L | F200A | Y328F | | | |
| MUTATION M483 | I157L | A204S | T210L | | | |
| MUTATION M484 | I157L | A204S | Y328F | | | |
| MUTATION M485 | I157L | A204T | T210L | | | |

### Table 2-17

**Table 2-17**

| MUTATION ID | MUTATION | | | | | |
|---|---|---|---|---|---|---|
| MUTATION M486 | I157L | A204T | Y328F | | | |
| MUTATION M487 | I157L | T210L | F211W | | | |
| MUTATION M488 | I157L | T210L | G212A | | | |
| MUTATION M489 | I157L | T210L | G226A | | | |
| MUTATION M490 | I157L | T210L | A233D | | | |
| MUTATION M491 | I157L | T210L | Y328F | | | |
| MUTATION M492 | I157L | T210L | L340V | | | |
| MUTATION M493 | I157L | T210L | V439P | | | |
| MUTATION M494 | I157L | F211W | Y328F | | | |
| MUTATION M495 | I157L | G226A | Y328F | | | |
| MUTATION M496 | I157L | A233D | Y328F | | | |
| MUTATION M497 | I157L | Y328F | L340V | | | |
| MUTATION M498 | I157L | Y328F | V439P | | | |
| MUTATION M499 | Y159N | F211W | Y328F | | | |
| MUTATION M500 | G161A | A184G | W187F | | | |
| MUTATION M501 | G161A | T210L | Y328F | | | |
| MUTATION M502 | G161A | F211W | Y328F | | | |
| MUTATION M503 | A182G | P183A | Y328F | | | |
| MUTATION M504 | A182S | P183A | Y328F | | | |
| MUTATION M505 | A184G | W187F | F200A | | | |
| MUTATION M506 | A184G | W187F | A204S | | | |
| MUTATION M507 | A184G | W187F | F211W | | | |
| MUTATION M508 | A184G | W187F | 1228K | | | |
| MUTATION M509 | A184G | W187F | A233D | | | |
| MUTATION M510 | F200A | F211W | Y328F | | | |
| MUTATION M511 | A204S | F211W | Y328F | | | |
| MUTATION M512 | A204T | F211W | Y328F | | | |
| MUTATION M513 | F211W | Y328F | L340V | | | |
| MUTATION M514 | P70T | A72E | I157L | Y328F | | |
| MUTATION M515 | P70T | A72E | T210L | Y328F | | |
| MUTATION M516 | P70T | G77M | I157L | Y328F | | |
| MUTATION M517 | P70T | Y81A | I157L | T210L | | |
| MUTATION M518 | P70T | Y81A | I157L | Y328F | | |
| MUTATION M519 | P70T | S84D | I157L | Y328F | | |
| MUTATION M520 | P70T | F88E | I157L | Y328F | | |
| MUTATION M521 | P70T | F88E | T210L | Y328F | | |
| MUTATION M522 | P70T | F113W | 1157L | T210L | | |
| MUTATION M523 | P70T | F113W | G161A | Y328F | | |
| MUTATION M524 | P70T | F113Y | I157L | Y328F | | |
| MUTATION M525 | P70T | D115Q | I157L | T210L | | |
| MUTATION M526 | P70T | D1150 | I157L | Y328F | | |
| MUTATION M527 | P70T | I157L | G161A | T210L | | |
| MUTATION M528 | P70T | I157L | A184G | W187F | | |
| MUTATION M529 | P70T | I157L | A184G | T210L | | |
| MUTATION M530 | P70T | I157L | W187F | T210L | | |
| MUTATION M531 | P70T | I157L | W187F | Y328F | | |
| MUTATION M532 | P70T | I157L | A204T | T210L | | |
| MUTATION M533 | P70T | I157L | A204T | Y328F | | |
| MUTATION M534 | P70T | I157L | A204T | T210L | | |
| MUTATION M535 | P70T | I157L | T210L | F211W | | |
| MUTATION M536 | P70T | I157L | T210L | G226A | | |
| MUTATION M537 | P70T | I157L | T210L | A233D | | |
| MUTATION M538 | P70T | I157L | T210L | Y328F | | |
| MUTATION M539 | P70T | I157L | T210L | L340V | | |

### Table 2-18

**Table 2-18**

| MUTATION ID | MUTATION | | | | | |
|---|---|---|---|---|---|---|
| MUTATION M540 | P70T | I157L | T210L | V439P | | |
| MUTATION M541 | P70T | I157L | Y328F | V439P | | |
| MUTATION M542 | P70T | G161A | T210L | Y328F | | |
| MUTATION M543 | P70T | G161A | A233D | Y328F | | |
| MUTATION M544 | A72E | S74T | I157L | Y328F | | |
| MUTATION M545 | A72E | G77S | F113W | I157L | | |
| MUTATION M546 | A72E | Y81H | I157L | Y328F | | |
| MUTATION M547 | A72E | K83P | I157L | Y328F | | |
| MUTATION M548 | A72E | F88E | F113W | 1157L | | |
| MUTATION M549 | A72E | F88E | l157L | Y328F | | |
| MUTATION M550 | A72E | F88E | G161A | Y328F | | |
| MUTATION M551 | A72E | F113W | I157L | Y328F | | |
| MUTATION M552 | A72E | F113W | G161A | Y328F | | |
| MUTATION M553 | A72E | F113Y | I157L | Y328F | | |
| MUTATION M554 | A72E | F113Y | G161A | Y328F | | |
| MUTATION M555 | A72E | F113Y | G226A | Y328F | | |
| MUTATION M556 | A72E | I157L | G161A | Y328F | | |
| MUTATION M557 | A72E | I157L | F162L | Y328F | | |
| MUTATION M558 | A72E | I157L | A184G | Y328F | | |
| MUTATION M559 | A72E | I157L | F200A | Y328F | | |
| MUTATION M560 | A72E | I157L | A204T | Y328F | | |
| MUTATION M561 | A72E | I157L | F211W | Y328F | | |
| MUTATION M562 | A72E | I157L | F211Y | Y328F | | |
| MUTATION M563 | A72E | I157L | A233D | Y328F | | |
| MUTATION M564 | A72E | I157L | Y328F | L340V | | |
| MUTATION M565 | A72E | G161A | A204T | Y328F | | |
| MUTATION M566 | A72E | G161A | T210L | Y328F | | |
| MUTATION M567 | A72E | G161A | F211W | Y328F | | |
| MUTATION M568 | A72E | G161A | F211Y | Y328F | | |
| MUTATION M569 | A72E | G161A | A233D | Y328F | | |
| MUTATION M570 | A72E | G161A | Y328F | L340V | | |
| MUTATION M571 | A72E | A184G | W187F | Y328F | | |
| MUTATION M572 | A72E | T210L | Y328F | L340V | | |
| MUTATION M573 | A72V | I157L | W187F | Y328F | | |
| MUTATION M574 | G77P | I157L | T210L | Y328F | | |
| MUTATION M575 | Y81A | S84D | I157L | Y328F | | |
| MUTATION M576 | Y81A | F88E | I157L | Y328F | | |
| MUTATION M577 | Y81A | F113W | I157L | Y328F | | |
| MUTATION M578 | Y81A | I157L | G161A | Y328F | | |
| MUTATION M579 | Y81A | I157L | W187F | Y328F | | |
| MUTATION M580 | Y81A | I157L | A204S | Y328F | | |
| MUTATION M581 | Y81A | I157L | T210L | Y328F | | |
| MUTATION M582 | Y81A | I157L | A233D | Y328F | | |
| MUTATION M583 | Y81A | I157L | Y328F | V439P | | |
| MUTATION M584 | Y81A | A184G | W187F | Y328F | | |
| MUTATION M585 | F88E | I157L | T210L | Y328F | | |
| MUTATION M586 | F88E | I157L | A233D | Y328F | | |
| MUTATION M587 | F113W | I157L | A204T | T210L | | |
| MUTATION M588 | F113W | I157L | T210L | Y328F | | |
| MUTATION M589 | I157L | G161A | A184G | W187F | | |
| MUTATION M590 | I157L | G161A | T210L | Y328F | | |
| MUTATION M591 | I157L | A184G | W187F | T210L | | |
| MUTATION M592 | I157L | A204S | T210L | Y328F | | |
| MUTATION M593 | I157L | A204T | T210L | Y328F | | |

### Table 2-19

**Table 2-19**

| MUTATION ID | MUTATION | | | | | |
|---|---|---|---|---|---|---|
| MUTATION M594 | l157L | T210L | A233D | Y328F | | |
| MUTATION M595 | G161A | A184G | W187F | Y328F | | |
| MUTATION M596 | P70T | A72E | S84D | I157L | Y328F | |
| MUTATION M597 | P70T | A72E | A204S | I157L | Y328F | |
| MUTATION M598 | P70T | A72E | T210L | I157L | Y328F | |
| MUTATION M599 | P70T | A72E | G226A | I157L | Y328F | |
| MUTATION M600 | P70T | A72E | A233D | I157L | Y328F | |
| MUTATION M601 | P70T | Y81A | I157L | T210L | Y328F | |
| MUTATION M602 | P70T | Y81A | I157L | A233D | Y328F | |
| MUTATION M603 | P70T | Y81A | I157L | T210L | Y328F | |
| MUTATION M604 | P70T | Y81A | A233D | I157L | Y328F | |
| MUTATION M605 | P70T | S84D | I157L | T210L | Y328F | |
| MUTATION M606 | P70T | F113W | I157L | T210L | Y328F | |
| MUTATION M607 | P70T | I157L | A184G | W187F | A233D | |
| MUTATION M608 | P70T | I157L | W187F | T210L | Y328F | |
| MUTATION M609 | P70T | I157L | A204S | T210L | Y328F | |
| MUTATION M610 | P70T | G161A | A184G | W187F | Y328F | |
| MUTATION M611 | P70V | A72E | F113Y | I157L | Y328F | |
| MUTATION M612 | P70V | A72E | I157L | F211W | Y328F | |
| MUTATION M613 | A72E | S74T | F113Y | l157L | Y328F | |
| MUTATION M614 | A72E | S74T | I157L | F211W | Y328F | |
| MUTATION M615 | A72E | Y81 H | I157L | F211W | Y328F | |
| MUTATION M616 | A72E | K83P | F113Y | I157L | Y328F | |
| MUTATION M617 | A72E | W17F | F113Y | I157L | Y328F | |
| MUTATION M618 | A72E | F113Y | D115Q | I157L | Y328F | |
| MUTATION M619 | A72E | F113Y | I157L | Y328F | L340V | |
| MUTATION M620 | A72E | F113Y | I157L | Y328F | V439P | |
| MUTATION M621 | A72E | F113Y | G161A | I157L | Y328F | |
| MUTATION M622 | A72E | F113Y | A204S | I157L | Y328F | |
| MUTATION M623 | A72E | F113Y | A204T | I157L | Y328F | |
| MUTATION M624 | A72E | F113Y | T210L | I157L | Y328F | |
| MUTATION M625 | A72E | F113Y | A233D | I157L | Y328F | |
| MUTATION M626 | A72E | I157L | G161A | F162L | Y328F | |
| MUTATION M627 | A72E | I157L | W187F | F211W | Y328F | |
| MUTATION M628 | A72E | I157L | A204S | F211W | Y328F | |
| MUTATION M629 | A72E | I157L | A204T | F211W | Y328F | |
| MUTATION M630 | A72E | I157L | F211W | Y328F | L340V | |
| MUTATION M631 | A72E | I157L | F211W | Y328F | V439P | |
| MUTATION M632 | A72E | I157L | G226A | F211W | Y328F | |
| MUTATION M633 | A72E | I157L | A233D | F211W | Y328F | |
| MUTATION M634 | Y81A | S84D | l157L | T210L | Y328F | |
| MUTATION M635 | Y81A | I157L | A184G | W187F | Y328F | |
| MUTATION M636 | Y81A | I157L | A184G | W187F | T210L | |
| MUTATION M637 | Y81A | I157L | A233D | T210L | Y328F | |
| MUTATION M638 | F88E | I157L | A184G | W187F | T210L | |
| MUTATION M639 | F113Y | I157L | Y159N | F211W | Y328F | |
| MUTATION M640 | I157L | A184G | W187F | T210L | Y328F | |
| MUTATION M641 | P70T | I157L | A184G | W187F | T210L | Y328F |
| MUTATION M642 | Y81A | I157L | A184G | W187F | T210L | Y328F |

Each mutation in the present specification is specified, as is the case with the mutant protein based on the amino acid sequence of SEQ ID NO:2 described above, by the abbreviations of the amino acid residues and the position in the amino acid sequence in SEQ ID NO:208, as shown in Tables 2-1 to 2-19. For example, the mutation L1, "N67K" represents that the amino acid residue, asparagine at position 67 in the sequence of SEQ ID NO:208 has been substituted with lysine. That is, the mutation is represented by the type of amino acid residue in M35-4/VT184A mutant (amino acid specified by SEQ ID NO:208); the position of the amino acid residue in the amino acid sequence of SEQ ID NO:208; and the type of the amino acid residue after the introduction of the mutation. Other mutations are represented in the same fashion.

Each of the mutations L1 to L335 may be introduced alone or in combination of two or more. One or more of the mutations L1 to L335 may be introduced in combination with one or more selected from the mutations other than the mutations in Tables 2-1 to 2-7, for example, the mutations shown in Table 33 which will be described later. Specifically, the combinations M1 to M642 as shown in Tables 2-8 to 2-19 described above are suitable. Particularly, mutant proteins having any of the following mutations are preferable in terms of improving peptide-synthesizing activity: mutation L125:I157L, mutation L124:I157K, mutation L303:Y328F, mutation L12:P70T, mutation L127:Y159N, mutation L199:F211W, mutation L195:F211I, mutation L130:G161A, mutation L115:D115Q, mutation L316:L340V, mutation L99:F88E, mutation L16:A72E, mutation L15:A72D, mutation L131:F162L, mutation L284:A233D, mutation L191:T210L, mutation L65:Y81A, mutation L265:I228K, mutation L317:V439P, mutation L255:G226A, mutation L52:G77S, mutation L155:F200A, mutation L298:R276A, mutation L201:G212A, mutation L145:W187F, mutation L170:A204S, mutation L87:S84D, mutation L60:E80D, mutation L110 : F113W, mutation M241:I157L/Y328F, mutation M340:P70T/I157L/Y328F, mutation M412:Y81A/I157L/Y328F, mutation M491:I157L/T210L/Y328F, mutation M496:I157L/A233D/ Y328F, mutation M581:Y81A/I157L/T210L/Y328F, mutation M582:Y81A/I157L/A233D/Y328F, and mutation M594:I157L/T210L/A233D/Y328F.

The present mutant protein has the excellent peptide-synthesizing activity. That is, these mutant protein exert a more excellent performance as to an ability to catalyze a peptide-synthesizing reaction than the protein (M35-4/V184A mutant protein) having the amino acid sequence of SEQ ID NO:208. More specifically, each mutant protein of the present invention exert more excellent performance for any of properties required for the peptide-synthesizing reaction, such as a reaction rate, a yield, a substrate specificity, a pH property and a temperature stability, than the protein shown in SEQ ID NO:208 when the peptide is synthesized from a specific carboxy component and amine component(specifically, see the following Examples). Thus, the mutant protein of the present invention may be used suitably for production of the peptide on an industrial scale.

The mutation shown in the mutations L1 to L335 and the mutations M1 to M642 may be introduced by modifying the nucleotide sequence of the gene encoding the protein having the amino acid sequence of SEQ ID NO:208 by site-directed mutagenesis such that the amino acid at the specific position is substituted. The nucleotide sequence corresponding to the positions to be mutated in the amino acid sequence of SEQ ID NO:208 may easily be identified with reference to SEQ ID NO:207.

The present invention also provides substantially the same protein as the mutant protein comprising one or more mutations shown in the above mutations L1 to L335 or the mutations M1 to M642. That is, the present invention also provides the mutant protein wherein, in the mutant protein comprising one or more of the mutations selected from the mutations L1 to L335 and M1 to M624, the amino acid sequence thereof further comprises, at other than the mutated position(s) in accordance with one or more of the mutations L1 to L335 and M1 to M624, one or more amino acid mutations selected from the group consisting of substitutions, deletions, insertions, additions and inversions; and wherein the mutant protein has the peptide-synthesizing activity (this protein may be referred to hereinbelow as the "mutant protein (II') of the protein having the amino acid sequence of SEQ ID NO:208). That is, the mutant protein of the present invention may contain the mutation at position other than the positions of the mutations L1 to L335 and M1 to M624 in the amino acid sequence shown in SEQ ID NO:208. Therefore, when the mutation such as deletions and insertions has been introduced at the position other than the positions of the mutations L1 to L335 and M1 to M624, the number of amino acid residues from the position specified by the mutations L1 to L335 and M1 to M624 to the N terminus or the C terminus may be sometimes different from that before introducing the mutation.

As used herein, "several amino acids" vary depending on the position and the type of the tertiary structure of the protein of amino acid residues, but may be in a range so as not to significantly impair the tertiary structure and the activity. Specifically, "several" may refer to 2 to 50, preferably 2 to 30 and more preferably 2 to 10 amino acids. It is desirable that the mutated protein retains the peptide-synthesizing activity at about a half or more, more preferably 80% or more, still more preferably 90% or more and particularly preferably 95% or more of the protein comprising one or more mutations selected from the mutations L1 to L335 and M1 to M624 (i.e., the mutant protein (I') of the protein having the amino acid sequence of SEQ ID NO:208).

The mutation other than those in the mutations L1 to L335 and M1 to M624 may be obtained by, e.g., site-directed mutagenesis for modifying the nucleotide sequence so that an amino acid at a specific position of the present protein is substituted, deleted, inserted, added or inverted. The polypeptide encoded by the nucleotide sequence modified as the above may also be obtained by conventional mutagenesis. The mutagenesis treatment and the meanings of the substitution, deletion, insertion, addition and inversion of the nucleotide are the same as defined in the foregoing section 1. The DNA encoding substantially the same protein as the protein described in DEQ ID NO:208 is obtainable by expressing the DNA having the above mutation in an appropriate cell and examining the present enzyme activity among the expressed products.

### 4. Polynucleotides of the present invention

The present invention provides a polynucleotide encoding the amino acid sequence of the above mutant protein of the present invention. Owing to codon degeneracy, the multiple nucleotide sequences may be present for defining one amino acid sequence. That is, the polynucleotides of the present invention encompass the following polynucleotides.
(i) The polynucleotide encoding the mutant protein having the amino acid sequence comprising one or more mutations from any of the mutations 1 to 68, and the mutations 239 to 290 and 324 to 377 in the amino acid sequence of SEQ ID NO:2.
(ii) The polynucleotide encoding the mutant protein having the amino acid sequence wherein, in the amino acid sequence comprising one or more mutations from any of the mutations 1 to 68, and the mutations 239 to 290 and 324 to 377 of the mutant protein (I), the amino acid sequence further comprises at other than the mutated positions one or several amino acid mutations selected from the group consisting of substitutions, deletions, insertions, additions and inversions; and having the peptide-synthesizing activity.
   The amino acid sequence of SEQ ID NO:2 is encoded by, e.g., the nucleotide sequence of SEQ ID NO:1.

The present invention also provides a polynucleotide encoding the amino acid sequence of the mutant protein based on the protein having the amino acid sequence of SEQ ID NO:208 of the present invention. Owing to codon degeneracy, the multiple nucleotide sequences may be present for defining one amino acid sequence. That is, the polynucleotides of the present invention encompass the following polynucleotides.
(i') The polynucleotide encoding the mutant protein having the amino acid sequence comprising one or more mutations from any of the mutations L1 to L335 and the mutations M1 to M624 in the amino acid sequence of SEQ ID NO:208.
(ii') The polynucleotide encoding the mutant protein having the amino acid sequence further comprising one or more amino acid mutations selected from the group consisting of substitutions, deletions, insertions, additions and inversions at positions other than the mutated positions in the amino acid sequence comprising one or more mutations from any of the mutations 1 to L335 and the mutations M1 to M624 in the amino acid sequence in the mutant protein described in the above (I'), and having the peptide-synthesizing activity.
   The amino acid sequence of SEQ ID NO:208 is encoded by, e.g., the nucleotide sequence of SEQ ID NO:207.

Substantially the same polynucleotide as the DNA having the nucleotide sequence shown in SEQ ID NO:1 may include the following polynucleotides. The specific polynucleotide to be separated may be a polynucleotide composed of a nucleotide sequence which hybridizes under a stringent condition with a polynucleotide complementary to the nucleotide sequence described in SEQ ID NO:1, or a probe prepared from the nucleotide sequence; and encodes a protein having the peptide-synthesizing activity. The specific polynucleotide may be isolated from the polynucleotide encoding the protein having the amino acid sequence described in SEQ ID NO:2 or from cells keeping the same. The polynucleotide which is substantially the same as the polynucleotide having the nucleotide sequence described in SEQ ID NO:1 may thus be obtained.
Meanwhile, the substantially the same polynucleotide as the DNA having the nucleotide sequence of SEQ ID NO:207 may also be obtained in the similar way to the aforementioned case with DNA of SEQ ID NO:1, i.e., may be obtained by isolating the polynucleotide from the polynucleotide encoding the protein having the amino acid sequence of SEQ ID NO:208 or from the cell having the same.

Likewise, the present invention provides the following polynucleotide (iii) or (iv) which is substantially the same as the polynucleotide encoding the mutant protein of the present invention.
(iii) The polynucleotide which hybridizes with the polynucleotide having the nucleotide sequence complementary to the nucleotide sequence of the aforementioned polynucleotide (i) under the stringent condition, and encodes the protein keeping one or more mutations selected from the mutations 1 to 68, 239 to 290 and 324 to 377 and having the peptide-synthesizing activity.
(iv) The polynucleotide which hybridizes with the polynucleotide having the nucleotide sequence complementary to the nucleotide sequence of the aforementioned polynucleotide (ii) under the stringent condition, and encodes the protein keeping one or more mutations selected from the mutations 1 to 68, 239 to 290 and 324 to 377 and having the peptide-synthesizing activity.

Likewise, the present invention provides the following polynucleotide (iii') or (iv') which is substantially the same as the polynucleotide encoding the mutant protein of the present invention.
(iii') The polynucleotide which hybridizes with the polynucleotide having the nucleotide sequence complementary to the nucleotide sequence of the aforementioned polynucleotide (i') under the stringent condition, and encodes the protein keeping one or more mutations selected from the mutations L1 to L335 and M1 to M642 and having the peptide-synthesizing activity.
(iv') The polynucleotide which hybridizes with the polynucleotide having the nucleotide sequence complementary to the nucleotide sequence of the aforementioned polynucleotide (ii') under the stringent condition, and encodes the protein keeping one or more mutations selected from the mutations L1 to L335 and M1 to M642 and having the peptide-synthesizing activity.

The probe for obtaining substantially the same polynucleotide may be prepared by standard methods based on the nucleotide sequence of SEQ ID NO:1 or SEQ ID NO:207 or the nucleotide sequence encoding the mutant protein. The method of isolating the objective polynucleotide by using the probe and taking the polynucleotide which hybridizes therewith may be performed in accordance with the standard method. For example, the DNA probe may be prepared by amplifying the nucleotide sequence cloned in a plasmid or phage vector, cutting out the nucleotide sequence to be used as the probe with restriction enzymes, and extracting it. The cut out site may be controlled depending on the objective DNA.

As used herein, the "stringent condition" refers to the condition where a so-called specific hybrid is formed whereas non-specific hybrid is not formed. Although it is difficult to clearly quantify this condition, examples thereof may include the condition where a pair of DNA sequences with high homology, e.g., DNA sequences having the homology of 50% or more, more preferably 80% or more, still more preferably 90% or more and particularly preferably 95% or more are hybridized whereas DNA with lower homology than that are not hybridized, and a washing condition of an ordinary Southern hybridization, i.e., hybridization at salt concentrations equivalent to 1 x SSC and 0.1% SDS, and preferably 0.1 x SSC and 0.1% SDS at 60°C. Among the genes which hybridize under such a condition, those having a stop codon in the middle of the sequence and which has lost the activity because of the mutation of the active center may be included. However, those may be easily removed by ligating them to the commercially available vector, expressing in an appropriate host, and measuring the enzyme activity of the expressed product by the method described below.

In the case of the polynucleotide in the above (ii), (iii) or (iv), it is desirable that the protein encoded by the polynucleotide retains the peptide-synthesizing activity at about a half or more, more preferably 80% or more and still more preferably 90% or more of the mutant protein in the above (I) under the condition at 50°C and pH 8. Meanwhile, in the case of the polynucleotide in the above (ii'), (iii') or (iv'), it is desirable that the protein encoded by the polynucleotide retains the peptide-synthesizing activity at about a half or more, more preferably 80% or more and still more preferably 90% or more of the mutant protein in the above (I) under the condition at 22°C and pH 8.5.

### 5. Protein having amino acid sequence of SEQ ID NO:2, and protein having amino acid sequence of SEQ ID NO:208

As described above, the mutant protein (I) and the mutant protein of the protein (I') having amino acid sequence of SEQ ID NO:208 may be obtained by modifying the proteins having amino acid sequences of SEQ ID NO:2 and SEQ ID NO:208. The protein which was used as a source of the protein of the invention will be described below. However, the mutant protein of the present invention is not limited to the source of the protein.

The DNA described in SEQ ID NO:1 and the protein having the amino acid sequence described in SEQ ID NO:2, as well as the DNA described in SEQ ID NO:207 and the protein having the amino acid sequence described in SEQ ID NO:208 are derived from *Sphingobacterium multivorum* FERM BP-10163 strain (indication given by the depositor for identification: *Sphingobacterium multivorum* AJ 2458). Microbial strains having an FERM number have been deposited to International Patent Organism Depositary, National Institute of Advanced Industrial Science and Technology, (Central No. 6, 1-1-1 Higashi, Tsukuba, Ibaraki Prefecture, Japan), and can be furnished with reference to the accession number.

A homogeneous protein to the protein having the amino acid sequence described in SEQ ID NO:2 or SEQ ID NO:208 may be isolated from *Sphingobacterium sp.* FERM BP-8124 strain. The protein where leucine, the amino acid residue at position 439 in the protein having the amino acid sequence described in SEQ ID NO:2 has been substituted with valine is isolated from *Sphingobacterium sp.* FERM BP-8124 strain. *Sphingobacterium sp.* FERM BP-8124 strain (indication given by the depositor for identification: *Sphingobacterium sp.* AJ 110003) was deposited on July 22, 2002 to International Patent Organism Depositary, National Institute of Advanced Industrial Science and Technology, and the accession number was given. Microbial strains having the FERM number have been deposited to International Patent Organism Depositary, National Institute of Advanced Industrial Science and Technology, (Central No. 6, 1-1-1 Higashi, Tsukuba, Ibaraki Prefecture, Japan), and can be furnished with reference to the accession number.

The aforementioned microbial strain of *Sphingobacterium multivorum* was identified to be of *Sphingobacterium multivorum* by the following classification experiments. The aforementioned microbial strain had the following natures: bacillus (0.6 to 0.7 x 1.2 to 1.5 µm), gram negative, no sporogenesis, no mobility, circular colony form, smooth entire fringe, low convex, lustrous shining, yellow color, grown at 30°C, catalase positive, oxidase positive and OF test (glucose) negative, and was thereby identified to be of genus *Sphingobacterium.* Furthermore, the microbial strain was proven to be similar to *Sphingobacterium multivorum* in characterization by the following natures: nitrate reduction negative, indole production negative, negative for acid generation from glucose, arginine dihydrase negative, urease positive, aesculin hydrolysis positive, gelatin hydrolysis negative, β-galactosidase positive, glucose utilization positive, L-arabinose utilization positive, D-mannose utilization positive, D-mannitol utilization negative, N-acetyl-D-glucosamine utilization positive, maltose utilization positive, potassium gluconate utilization negative, n-capric acid utilization negative, adipic acid utilization negative, dl-malic acid utilization negative, sodium citrate utilization negative, phenyl acetate utilization negative and cytochrome oxidase positive. In addition, as a result of a homology analysis of a nucleotide sequence of 16S rRNA gene, the highest homology (98.5%) to *Sphingobacterium multivorum* was exhibited, and thus, the present microbial strain was identified as *Sphingobacterium multivorum.*

A DNA consisting of a nucleotide sequence of the base numbers 61 to 1917 in SEQ ID NO:1 is a code sequence portion. The nucleotide sequence of the base numbers 61 to 1917 includes a signal sequence region and a mature protein region. The signal sequence region is the region of the base numbers 61 to 120, and the mature protein region is the region of the base numbers 121 to 1917. That is, the present invention provides both a peptide enzyme protein gene containing the signal sequence and a peptide enzyme protein gene as the mature protein. The signal sequence containing the sequence described in SEQ ID NO:1 is a class of a leader sequence, and a major function of a leader peptide encoded in the leader sequence region is presumed to be secretion thereof from a cell membrane inside to a cell membrane outside. The protein encoded by the nucleotide sequence of the base numbers 121 to 1917, i.e., the region except the leader peptide sequence corresponds to the mature protein, and is presumed to have the high peptide-synthesizing activity.

The DNA having the nucleotide sequence of SEQ ID NO:1 may be obtained from a chromosomal DNA of *sphingobacterium multivorum* or a DNA library by PCR (polymerase chain reaction, see White, T. J. et al ;Trends Genet., 5, 185(1989)) or hybridization. Primers for PCR may be designed based on an internal amino acid sequence determined on the basis of the purified protein having the peptide-synthesizing activity. The primer or a probe for the hybridization may be designed based on the nucleotide sequence described in SEQ ID NO:1, or may also be isolated using a probe. When the primers having the sequences corresponding to a 5'-untranslated region and a 3'-untranslated region as the PCR primers, a full length coding region of the present protein may be amplified. Explaining as an example the primers for amplifying the region including the region encoding both the leader sequence and the mature protein described in SEQ ID NO:1, a primer having the nucleotide sequence of the upstream of the base number 61 in SEQ ID NO:1 may be used as the 5'-primer, and a primer having a sequence complementary to the nucleotide sequence of the downstream of the base number 1917 may be used as the 3'-primer.

The primers may be synthesized in accordance with standard methods, for example, by a phosphoamidite method (see Tetrahedron Letters, 22:1859, 1981) using a DNA synthesizer model 380B supplied from Applied Biosystems.
The PCR reaction may be performed, for example, using Gene Amp PCR System 9600 (supplied from Perkin Elmer) and TaKaRa LA PCR in vitro Cloning Lit (supplied from Takara Shuzo Co., Ltd.) in accordance with instructions from the supplier such as manufacturer.

### 6. Method for producing mutant protein of the present invention

The method for producing the protein of the present invention and the methods for producing recombinants and transformants used therefor will be subsequently described.

A transformant which expresses the aforementioned mutant protein can produce the mutant protein having the peptide-synthesizing activity. For example, the mutant protein having the activity may be produced by introducing the mutation corresponding to any of the mutations 1 to 38, 239 to 290 and 324 to 377 into a recombinant DNA such as an expression vector having the nucleotide sequence shown in SEQ ID NO:1, and introducing the expression vector into an appropriate host to express the mutant protein. A transformant which expresses the mutant protein of SEQ ID NO:208 can also produce the mutant protein having the peptide-synthesizing activity. For example, the mutant protein having the activity may be produced by introducing the mutation corresponding to any of the mutations L1 to L335, and M1 to M642 into a recombinant DNA such as an expression vector having the nucleotide sequence shown in SEQ ID NO:207, and introducing the expression vector into an appropriate host to express the mutant protein. As the host for expressing the mutant protein specified by the DNA having the nucleotide sequence of SEQ ID NO:1 or No:207, it is possible to use various prokaryotic cells such as microorganisms belonging genera *Escherichia* (e.g., *Escherichia coli), Empedobacter, Sphingobacterium* and *Flavobacterium,* and *Bacillus subtilis* as well as various eukaryotic cells such as *Saccharomyces cerevisiae, Pichia stipitis,* and *Aspergillus oryzae.*

The recombinant DNA for introducing a foreign DNA into the host may be prepared by inserting a predetermined DNA into the vector selected depending on the type of the host in a manner whereby a protein encoded by the DNA can be expressed. When a promoter inherent for a gene encoding the protein produced by *Empedobacter brevis* works in the host cell, that promoter may be used as the promoter for expressing the protein. If necessary, another promoter which works in the host cell may be ligated to the DNA encoding the mutant protein, which may be then expressed under the control of that promoter.

Examples of a transformation method for introducing the recombinant DNA into the host cell may include D. M. Morrison's method (Methods in Enzymology 68, 326 (1979)) or a method of enhancing permeability of the DNA by treating recipient microorganisms with calcium chloride (Mandel, M. and Higa, A., J. Mol. Biol., 53, 159 (1970)).

In the case of producing a protein on a large scale using the recombinant DNA technology, one of the preferable embodiments therefor may be formation of an inclusion body of the protein. The inclusion body is configured by aggregation of the protein in the protein-producing transformant. The advantages of this expression production method may be protection of the objective protein from digestion by protease which is present in the microbial cells, and ready purification of the objective protein that may be performed by disruption of the microbial cells and following centrifugation.

The protein inclusion body obtained in this way may be solubilized by a protein denaturing agent, which is then subjected to activation regeneration mainly by removing the denaturing agent, to be converted into correctly refolded and physiologically active protein. There are many examples of such procedures, such as activity regeneration of human interleukin 2 (JP-S61-257931 A).

To obtain the active protein from the protein inclusion body, a series of the manipulations such as solubilization and activity regeneration is required, and thus, the manipulations are more complicate than those in the case of directly producing the active protein. However, when a protein which affects microbial cell growth is produced on a large scale in the microbial cells, effects thereof may be inhibited by accumulating the protein as the inactive inclusion body in the microbial cells.

Examples of the methods for producing the objective protein on a large scale as the inclusion body may include methods of expressing the protein alone under control of a strong promoter, as well as methods of expressing the objective protein as a fusion protein with a protein known to be expressed in a large amount.

As an example, a method for preparing transformed *Escherichia coli* and producing a mutant protein using this will be described more specifically hereinbelow. When the mutant protein is produced by microorganisms such as E. *coli,* a DNA encoding a precursor protein including the leader sequence may be incorporated or a DNA for a mature protein region without including the leader sequence may be incorporated as a code sequence of the protein. Either one may be appropriately selected depending on the production condition, the form and the use condition of the enzyme to be produced.

As the promoter for expressing the DNA encoding the mutant protein, the promoter typically used for producing xenogenic proteins in E. *coli* may be used, and examples thereof may include strong promoters such as T7 promoter, lac promoter, trp promoter, trc promoter, tac promoter, and PR promoter and PL promoter of lambda phage. As the vector, pUC19, pUC18, pBR322, pHSG299, pHSG298, pHSG399, pHSG398, RSF1010, pMW119, pMW118, pMW219, and pMW218 may be used. Other vectors of phage DNA may also be used. In addition, expression vectors which contains a promoter and can express the inserted DNA sequence may also be used.

In order to produce the mutant protein as a fusion protein inclusion body, a fusion protein gene is made by linking a gene encoding another protein, preferably a hydrophilic peptide to upstream or downstream of the mutant protein gene. Such a gene encoding the other protein may be those which increase an amount of the accumulated fusion protein and enhance solubility of the fusion protein after denaturation and regeneration steps. Examples of candidates thereof may include T7 gene 10, β-galactosidase gene, dehydrofolic acid reductase gene, interferon γ gene, interleukin-2 gene and prochymosin gene.

Such a gene may be ligated to the gene encoding the mutant protein so that reading frames of codons are matched. This may be effected by ligating at an appropriate restriction enzyme site or using a synthetic DNA having an appropriate sequence.

In some cases, it is preferable to ligate a terminator, i.e. the transcription termination sequence, to downstream of the fusion protein in order to increase the production amount. Examples of this terminator may include T7 terminator, fd phage terminator, T4 terminator, tetracycline resistant gene terminator and *E*. *coli* trpA gene terminator.

The vector for introducing the gene encoding the mutant protein or the fusion protein of the mutant protein with the other protein into E. *coli* may preferably be of a so-called multicopy type. Examples thereof may include plasmids having a replication origin derived from ColE1, such as pUC based plasmids, pBR322 based plasmids or derivatives thereof. As used herein, the "derivative" means the plasmid modified by the substitution, deletion, insertion, addition and/or inversion of a base(s). "Modified" referred to herein includes the modification by mutagenesis with the mutagen or UV irradiation and natural mutation.

In order to select the transformants, it is preferable that the vector has a marker such as an ampicillin resistant gene. As such a plasmid, expression vectors having the strong promoter are commercially available (pUC series: Takara Shuzo Co., Ltd., pPROK series and pKK233-2: Clontech, etc.).

A DNA fragment where the promoter, the gene encoding the protein having the peptide-synthesizing activity or the fusion protein of the protein having the peptide-synthesizing activity with the other protein, and in some cases the terminator are ligeted sequentially is then ligeted to the vector DNA to obtain a recombinant DNA.

The mutated protein or the fusion protein of the mutated protein with the other protein is expressed and produced by transforming *E. coli* with the resulting recombinant DNA and culturing this *E*. *coli.* Strains commonly used for the expression of the xenogenic gene may be used as the host to be transformed. *E. coli* JM 109 strain which is a subspecies of *E*. *coli* K12 strain is preferable. The methods for transformation and for selecting transformants are described in Molecular Cloning, 2nd edition, Cold Spring Harbor press, 1989.

In the case of expressing as the fusion protein, the fusion protein may be composed so as to be able to cleave the peptide-synthesizing enzyme therefrom using a restriction protease which recognizes a sequence of blood coagulation factor Xa, kallikrein or the like which is not present in the peptide-synthesizing enzyme.

As production media, the media such as M9-casamino acid medium and LB medium typically used for cultivation of *E. coli* may be used. The conditions for cultivation and a production induction may be appropriately selected depending on types of the marker and the promoter of the vector and the host used.

The following methods are available for recovering the mutant protein or the fusion protein of the mutant protein with the other protein. If the mutant protein or the fusion protein thereof is solubilized in the microbial cells, the cells may be collected and then disrupted or lysed to thereby obtain a crude enzyme solution. If necessary, the crude solution may be purified using techniques such as ordinary precipitation, filtration and column chromatography, to obtain purified mutant protein or the fusion protein. In this case, the purification may be performed using an antibody against the mutant protein or the fusion protein.

In the case where the protein inclusion body is formed, this may be solubilized with a denaturing agent. The inclusion body may be solubilized together with the microbial cells. However, considering the following purification process, it is preferable to take up the inclusion body before solubilization. Collection of the inclusion body from the microbial cells may be performed in accordance with conventionally and publicly known methods. For example, the microbial cells are disrupted, and the inclusion body is then collected by centrifugation and the like. Examples of the denaturing agent which solubilizes the protein inclusion body may include guanidine-hydrochloric acid (e.g., 6 M, pH 5 to 8), urea (e.g., 8 M), and the like.

As a result of removal of the denaturing agent by dialysis and the like, the protein may be regenerated as the protein having the activity. Dialysis solutions used for the dialysis may include Tris hydrochloric acid buffer, phosphate buffer and the like. The concentration thereof may be 20 mM to 0.5 M, and pH thereof may be 5 to 8.

It is preferred that the protein concentration at a regeneration step is kept at about 500 µg/ml or less. In order to inhibit self-crosslinking of the regenerated peptide-synthesizing enzyme, it is preferred that dialysis temperature is kept at 5°C or below. Methods for removing the denaturing agent other than the dialysis method may include a dilution method and an ultrafiltration method. The regeneration of the activity is anticipated by using any of these methods.

### 7. Method for producing peptide

In the method for producing the peptide of the present invention, the peptide is synthesized using the foregoing mutant protein. That is, in the method for producing the peptide of the present invention, the peptide is synthesized by reacting an amine component and a carboxy component in the presence of at least one of the following proteins (I) and (II).
(I) The mutant protein having the amino acid sequence comprising one or more mutations selected from any of the mutations 1 to 68, and the mutations 239 to 290 and 324 to 377 in the amino acid sequence of SEQ ID NO:2.
(II) The mutant protein having the amino acid sequence further comprising one or several amino acid mutations selected from substitutions, deletions, insertions, additions and inversions at positions other than the mutated positions of one or more mutations selected from any of the mutations 1 to 68, and the mutations 239 to 290 and 324 to 377 in the mutant protein (I); and having the peptide-synthesizing activity.

In the method for producing the peptide of the present invention, the peptide may also be synthesized using the mutant protein based on the protein having the amino acid sequence of SEQ ID NO:208. That is, in the method for producing the peptide of the present invention, the peptide may be synthesized by reacting the amine component and the carboxy component in the presence of at least one of the following proteins (I') and (II').
(I') The mutant protein having the amino acid sequence comprising one or more mutations selected from any of the mutations L1 to L335, and the mutations M1 to M642 in the amino acid sequence of SEQ ID NO:208.
(II') The mutant protein having the amino acid sequence further comprising one or several amino acid mutations selected from substitutions, deletions, insertions, additions and inversions at positions other than the mutated positions of one or more mutations selected from any of the mutations L1 to L335, and the mutations M1 to M642 in the mutant protein described in the above (I'); and having the peptide-synthesizing activity.

In the method for producing the peptide of the present invention, the mutant protein is placed in the peptide-synthesizing reaction system. The mutant protein may be supplied as a mixture containing the protein (I) and/or (II), or (I') and/or (II') in a biochemically acceptable solvent (the mixture will be referred to hereinbelow as "mutant protein-containing material"). More specifically, the peptide may be synthesized from the amine component and the carboxy component using one or more selected from the group consisting of a cultured product of a microorganism that has been transformed so as to express the mutant protein of the present invention, a microbial cell separated from the cultured product and the treated microbial cells of the microorganism.

As used herein, the "mutant protein-containing material" may be any material containing the mutant protein of the present invention, and specifically includes a cultured product of microorganisms which produce the mutant protein, microbial cells separated from the cultured product, and the treated microbial cells. The cultured product of microorganisms refers to one obtained by cultivation of the microorganisms, and more specifically refers to, e.g., a mixture of microbial cells, the medium used for culturing the microorganisms and substances produced by the cultured microorganisms. Alternatively, the microbial cells may be washed, and used as the washed microbial cells. The treated microbial cells may include ones obtained by disrupting, lysing and lyophilizing the microbial cells, as well as crude purified proteins recovered by further treating the microbial cells, and purified proteins obtained by further purification. As the purified proteins, partially purified proteins obtained by various purification methods may be used, and immobilized proteins obtained by immobilizing by a covalent bond method, an absorption method or an entrapment method may also be used. Depending on the microorganism to be used, bacteriolysis may partially occurs during the cultivation. In this case, a cultured supernatant may also be used as the mutant protein-containing material.

As the microorganism containing the mutant protein of the present invention, a gene recombinant strain which expresses the mutant protein may be used. Alternatively, treated microbial cells such as microbial cells treated with acetone and lyophilized microbial cells may be used. These may further be immobilized by a variety of methods such as the covalent bond method, the absorption method or the entrapment method, to produce immobilized microbial cells or immobilized treated microbial cells for use.

When the cultured product, the cultured microbial cells, the washed microbial cells and the treated microbial cells such as disrupted or lysed microbial cells are used, these materials tend to contain enzymes which are not involved in peptide production and degrade produced peptides. In this case, it is sometimes preferable to add a metal protease inhibitor such as ethylenediamine tetraacetatic acid (EDTA). The amount of such an inhibitor to be added may be in the range of 0.1 mM to 300 mM, and preferably from 1 mM to 100 mM.

The mutant protein or the mutant protein-containing material may be allowed to act upon a carboxy component and an amine component merely by mixing the mutant protein or the mutant protein-containing material, the carboxy component and the amine component. More specifically, the mutant protein or the mutant protein-containing material may be added to a solution containing the carboxy component and the amine component to react. Alternatively, in the case of using microorganisms which produce the mutant protein, the microorganisms which produce the mutant protein may be cultured to generate and accumulate the enzyme in the microorganisms or a cultured medium of the microorganisms, and the carboxy component and the amine component may then be added into the cultured medium. The produced peptide may be recovered in accordance with standard methods, and purified as needed.

To obtain microbial cells (cells of the microorganisms), the microorganisms may be cultured and grown in an appropriate cultivation medium which may be selected depending on the type of the microorganisms. The medium therefor is not particularly limited as long as the microorganisms can be grown in the medium, and may be an ordinary medium containing carbon sources, nitrogen sources, phosphorus sources, sulfur sources, inorganic ions, and, if necessary, organic nutrient sources.

Any carbon sources may be used as long as the microorganism can utilize. Examples of the carbon sources may include sugars such as glucose, fructose, maltose and amylose, alcohols such as sorbitol, ethanol and glycerol, organic acids such as fumaric acid, citric acid, acetic acid and propionic acid and salts thereof, carbohydrates such as paraffin, and mixtures thereof.

As the nitrogen sources, ammonium salts of inorganic acids such as ammonium sulfate and ammonium chloride, ammonium salts of organic acids such as ammonium fumarate and ammonium citrate, nitrate salts such as sodium nitrate and potassium nitrate, organic nitrogen compounds such as peptone, yeast extract, meat extract and corn steep liquor, or mixtures thereof may be used.

If necessary, nutrient sources such as inorganic salts, trace metal salts and vitamins commonly used in the medium may be admixed for use.

A cultivation condition is not particularly limited, and the cultivation may be performed under an aerobic condition at pH 5 to 9 and at a temperature ranging from about 15 to 55°C for about 12 to 48 hours while appropriately controlling pH and the temperature.

A preferable embodiment of the method for producing the peptide of the present invention may be a method in which the transformed microorganisms are cultured in the medium to accumulate the mutated protein in the medium and/or the transformed microorganisms. Employment of the transformants enables production of the mutant protein readily on a large scale, and thus the peptide may thereby be rapidly synthesized in a large amount.

The amount of the mutant protein or the mutant protein-containing material to be used may be the amount by which an objective effect is exerted (i.e., effective amount). Those skilled in the art can easily determine this effective amount by a simple preliminary experiment. For example, the effective amount is about 0.01 to 100 units (U) or about 0.1 to 500 g/L in the case of using the enzyme or the washed microbial cells, respectively.

Any carboxy component may be used as long as it can be condensed with the amine component, the other substrate, to generate the peptide. Examples of the carboxy component may include L-amino acid ester, D-amino acid ester, L-amino acid amide, D-amino acid amide, and organic acid ester having no amino group. As amino acid ester, not only amino acid esters corresponding to natural amino acids but also amino acid esters corresponding to non-natural amino acids and derivatives thereof are also exemplified. In addition, as amino acid esters, β-, γ-, and ω-amino acid esters in addition to α-amino acid ester having different binding sites of amino groups are also exemplified. Representative examples of amino acid esters may include methyl ester, ethyl ester, n-propyl ester, iso-propyl ester, n-butyl ester, iso-butyl ester and tert-butyl ester of amino acids.

Any amine component may be used as long as it can be condensed with the carboxy component, the other substrate, to generate the peptide. Examples of the amine component may include L-amino acid, C-protected L-amino acid, D-amino acid, C-protected D-amino acid and amines. As amines, not only natural amine but also non-natural amine and derivatives thereof are exemplified. As amino acids, not only natural amino acids but also non-natural amino acids and derivatives thereof are exemplified. β-, γ-, and ω-Amino acids in addition to α-amino acids having different binding sites of amino groups are also exemplified.

Concentrations of the carboxy component and the amine component which are starting materials may be 1 mM to 10 M and preferably 0.05 M to 2 M. In some cases, it is preferable to add the amine component in the amount equal to or more than the amount of the carboxy component. When the reaction is inhibited by the high concentration of the substrate, the concentrations may be kept to a certain level in order to avoid inhibition of the reaction and the components may be sequentially added.

A reaction temperature may be 0 to 60°C at which the peptide can be synthesized, and preferably 5 to 40°C. A reaction pH may be 6.5 to 10.5 at which the peptide can be synthesized, and preferably pH 7.0 to 10.0.

The method for producing the peptide of the present invention is suitable as the method for producing various peptides. Examples of the peptide may include dipeptides such as α-L-aspartyl-L-phenylalanine-β-methyl ester (i.e., α-L-(β-O-methyl aspartyl)-L-phenylalanine(abbreviation: α-AMP)), L-alanyl-L-glutamine (Ala-Gln), L-alanyl-L-phenylalanine (Ala-Phe), L-phenylalanyl-L-methionine (Phe-Met), L-leucyl-L-methionine (Leu-Met), L-isoleucyl-L-methionine (IIe-Met), L-methionyl-L-methionine (Met-Met), L-prolyl-L-methionine (Pro-Met), L-tryptophyl-L-methionine (Trp-Met), L-valyl-L-methionine (Val-Met), L-asparaginyl-L-methionine (Asn-Met), L-cysteinyl-L-methionine (Cys-Met), L-glutaminyl-L-methionine (Gln-Met), glycyl-L-methionine (Gly-Met), L-seryl-L-methionine (Ser-Met), L-threonyl-L-methionine (Thr-Met), L-tyrosyl-L-methionine (Tyr-Met), L-aspartyl-L-methionine (Asp-Met), L-arginyl-L-methiomne (Arg-Met), L-histidyl-L-methionine (His-Met), L-lysyl-L-methionine (Lys-Met), L-alanyl-glycine (Ala-Gly), L-alanyl-L-threonine (Ala-Thr), L-alanyl-L-glutamic acid (Ala-Glu), L-alanyl-L-alanine (Ala-Ala), L-alanyl-L-aspartic acid (Ala-Asp), L-alanyl-L-serine (Ala-Ser), L-alanyl-L-methionine (Ala-Met), L-alanyl-L-valine (Ala-Val), L-alanyl-L-lysine (Ala-Lys), L-alanyl-L-asparagine (Ala-Asn), L-alanyl-L-cysteine (Ala-Cys), L-alanyl-L-tyrosine (Ala-Tyr), L-alanyl-L-isoleucine (Ala-Ile), L-arginyl-L-glutamine (Arg-Gln), glycyl-L-serine (Gly-Ser), glycyl-L-(t-butyl)serine (Gly-Ser(tBu)), and (2S,3R,4S)-4-hydroxylisoleucyl-phenylalanine (HIL-Phe); tripeptides such as L-alanyl-L-phenylalanyl-L-alanine (AFA), L-alanyl-glycyl-L-alanine (AGA), L-alanyl-L-histidyl-L-alanine (AHA), L-alanyl-L-leucyl-L-alanine (ALA), L-alanyl-L-alanyl-L-alanine (AAA), L-alanyl-L-alanyl-glycine (AAG), L-alanyl-L-alanyl-L-proline (AAP), L-alanyl-L-alanyl-L-glutamine (AAQ), L-alanyl-L-alanyl-L-tyrosine (AAY), glycyl-L-phenylalanyl-L-alanine (GFA), L-alanyl-glycyl-glycine (AGG), L-threonyl-glycyl-glycine (TGG), glycyl-glycyl-glycine (GGG), and L-alanyl-L-phenylalanyl-glycine (AFG); tetrapeptides such as glycyl-glycyl-L-phenylalanyl-L-methionine (GGFM); and pentapeptides such as L-tyrosyl-glycyl-glycyl-L-phenylalanyl-L-methionine (YGGFM).
The method for producing the peptide of the present invention is also suitable for the method for producing, for example, α-L-aspartyl-L-phenylalanine-β-methyl ester (i.e., α-L-(β-O-methyl aspartyl)-L-phenylalanine, abbreviated as α-AMP). α-AMP is an important intermediate for producing α-L-aspartyl-L-phenylalanine-α-methyl ester (product name: Aspartame) which has a large demand as a sweetener.

### [Examples]

The present invention will be described in detail with reference to the following Examples, but the invention is not limited thereto.

### (Example 1) Expression of peptide-synthesizing enzyme gene in E. coli

An objective gene encoding a protein having a peptide-synthesizing activity was amplified by PCR with a chromosomal DNA from *Sphingobacterium multivorum* FERM BP-10163 strain as a template using oligonucleotides shown in SEQ ID NOS:5 and 6 as primers. An amplified DNA fragment was treated with NdeI/XbaI, and a resulting DNA fragment was ligated to pTrpT that had been treated with NdeI/XbaI. *Escherichia coli* JM109 was transformed with this solution containing the ligated product, and a strain having an objective plasmid was selected with ampicillin resistance as an indicator, and this plasmid was designated as pTrpT_Sm_Aet. *Escherichia coli* JM109 having pTrpT_Sm_Aet is also represented as pTrpT_Sm_Aet/JM109 strain.

One platinum loopful of pTrpT_Sm_Aet/JM109 strain was inoculated into a general test tube in which 3 mL of a medium (2 g/L of glucose, 10 g/L of yeast extract, 10 g/L of casamino acid, 5 g/L of ammonium sulfate, 3 g/L of potassium dihydrogen phosphate, 1 g/L of dipotassium hydrogen phosphate, 0.5 g/L of magnesium sulfate 7-hydrate, 100 mg/L of ampicillin) had been placed, and a main cultivation was performed at 25°C for 20 hours. An AMP-synthesizing activity of 2.1 U per 1 mL of the cultured medium was found, thereby confirming that the cloned gene had been expressed in *Escherichia coli.* No activity was detected in transformants into which pTrpT alone had been introduced as a control.

### (Example 2) Construction of rational mutant strain using pKF vector

### (1) Construction of pKF_Sm_Aet

An objective gene was amplified by PCR with pTrpT_Sm_Aet plasmid as a template using the oligonucleotides shown in SEQ ID NOS:3 and 4 as the primers. This DNA fragment was treated with EcoRI/PstI, and the resulting DNA fragment was ligated to pKF18k2 (suppled from Takara Shuzo Co., Ltd.) that had been treated with EcoRI/PstI. *Escherichia coli* JM109 was transformed with this solution containing the ligated product, and a strain having an objective plasmid was selected with kanamycin resistance as the indicator, and this plasmid was designated as pKF_Sm_Aet. *Escherichia coli* JM109 having pKF_Sm_Aet is also represented as pKF_Sm_Aet/JM109 strain.

### (2) Introduction of rational mutation into pKF_Sm_Aet

In order to construct mutant Aet, pKF_Sm_Aet plasmid was used as the template for site-directed mutagenesis using an ODA method. Mutations were introduced using "site-directed mutagenesis system Mutan Super Express kit" supplied from Takara Shuzo Co., Ltd. (Japan) in accordance with the protocol of the manufacturer using the primers (SEQ ID NOS:12 to 33) corresponding to each mutant enzyme. The 5' terminus of the primers were phosphorylated before use with T4 polynucleotide kinase supplied from Takara Shuzo Co., Ltd. The primers were phosphorylated by adding 100 µmol DNA (primer) and 10 units of T4 polynucleotide kinase to 20 µL of 50 mM tris-hydrochloric acid buffer (pH 8.0) containing 0.5 mM ATP, 10 mM magnesium chloride and 5 mM DTT and warming at 37°C for 30 minutes followed by heating at 70°C for 5 minutes. Subsequently, 1 µL (5 pmol) of this reaction solution was used for PCR by which the mutation was introduced. The PCR was performed by adding 10 ng of ds DNA (pKF_Sm_Aet plasmid) as the template, 5 pmol each of Selection Primer and 5'-phosphorylated mutagenic oligonucleotides shown above as the primers and 40 units of LA-Taq to 50 µL of LA-Taq buffer II (Mg²⁺ plus) containing 250 µM each of dATP, dCTP, dGTP and dTTP, which was then subjected to 25 cycles of heating at 94°C for one minute, 55°C for one minute and 72°C for 3 minutes. After the PCR for introducing the mutation was completed, a DNA fragment was collected by ethanol precipitation, and *Escherichia coli* MV1184 strain was transformed with the resulting DNA fragment. A strain having an objective plasmid: pKF_Sm_AetM containing a mutant Aet gene was selected with kanamycin resistance as the indicator.

In the present specification, *Escherichia coli* MV1184 strain having pKF_Sm_AetM is also represented as pKF_Sm_AetM/MV1184 strain. When referring to a specific mutant of pKF_Sm_AetM, the mutation thereof may be represented by replacing "AetM" with the type of mutation, e.g., pKF_Sm_F207V. When a mutant contains two or more mutations, the mutations may be stated continuously with "/" dividing each mutation. For example, pKF_Sm_F207V/Q441E represents a mutant in which the mutations F207V and Q441E have been introduced into the Aet gene which pKF_Sm_Aet plasmid carries.

### (3) Construction of pHSG_Sm_Aet

An objective gene was amplified by PCR with pTrpT_Sm_Aet plasmid as a template using the oligonucleotides shown in SEQ ID NO:3 and 4 as primers. This DNA fragment was treated with EcoRI/PstI, and a resulting DNA fragment was ligated to pHSG298 (suppled from Takara Shuzo Co., Ltd.) that had been treated with EcoRI/PstI. *Escherichia coli* MV1184 strain was transformed with this solution containing the ligated product, and a strain having an objective plasmid was selected with kanamycin resistance as an indicator, and this plasmid was designated as pHSG_Sm_Aet. *Escherichia coli* MV1184 having pHSG_Sm_Aet is also represented as pHSG_Sm_Aet/MV1184 strain.

### (4) Obtaining microbial cells: A

Each of pKF_Sm_Aet/JM109 strain, pKF_Sm_Aet/MV1184 strain and pHSG_Sm_Aet/MV1184 strain was precultured in an LB agar medium (10g/L of yeast extract, 10 g/L of peptone, 5 g/L of sodium chloride, 20 g/L of agar, pH 7.0) at 30°C for 24 hours. One platinum loopful of microbial cells of each strain obtained from the above cultivation was inoculated into a general test tube in which 3 mL of the LB medium (0.1 M IPTG and 20 mg/L of kanamycin were added to the above medium from which the agar had been omitted) had been placed, and a main cultivation was performed at 25°C at 150 reciprocatings/minute for 20 hours.

### (5) Production of peptide using microbial cells <synthesis of AMP>

400 µL of each cultured medium obtained in Example 2 (4) was centrifuged to collect the microbial cells. The collected cells were then suspended in 200 µL of 100 mM borate buffer (pH 9.0) containing 10 mM EDTA, 50 mM dimethyl aspartate and 100 mM phenylalanine, and reacted at 25°C for 30 minutes. The concentration of α-AMP produced by the strain which expressed the wild type enzyme (such a strain will be referred to hereinbelow as the "wild strain") in this reaction is shown in Table 3. For the dipeptide production by the strains which expressed various mutant enzymes (mutant strains), their ratios of production concentrations to those of the wild strain are shown in Table 3.

### (6) Production of peptide using microbial cells <synthesis of Ala-Gln>

100 µL of each cultured medium obtained in Example 2 (4) was centrifuged to collect the microbial cells. The collected cells were then suspended in 200 µL of 100 mM borate buffer (pH 9.0) containing 10 mM EDTA, 100 mM L-alanine methyl ester and 200 mM glutamine, and reacted at 25°C for 30 minutes. The concentration of L-alanyl-L-glutamine (Ala-Gln) produced by the wild strain in this reaction is shown in Table 3. For the dipeptide production by the various mutant strains, the ratio of production concentration to that of the wild strain is shown in Table 3.

### (7) Production of peptide using microbial cells <synthesis of Phe-Met, Leu-Met>

800 µL of each cultured medium obtained in Example 2 (4) was centrifuged to collect the microbial cells. The collected cells were then suspended in 400 µL of 100 mM borate buffer (pH 9.0) containing 10 mM EDTA, 50 mM L-phenylalanine methyl ester hydrochloride or L-leucine methyl ester hydrochloride, and 100 mM L-methionine, and reacted at 25°C for 20 minutes. The concentration of L-phenylalanyl-L-methionine (Phe-met) or L-leucyl-L-methionine (Leu-Met) produced by the wild strain in this reaction is shown in Table 3. For the dipeptide synthesized by the various mutant strains, the ratio of production concentration with respect to that by the wild strain is shown in Table 3.

### Table 3

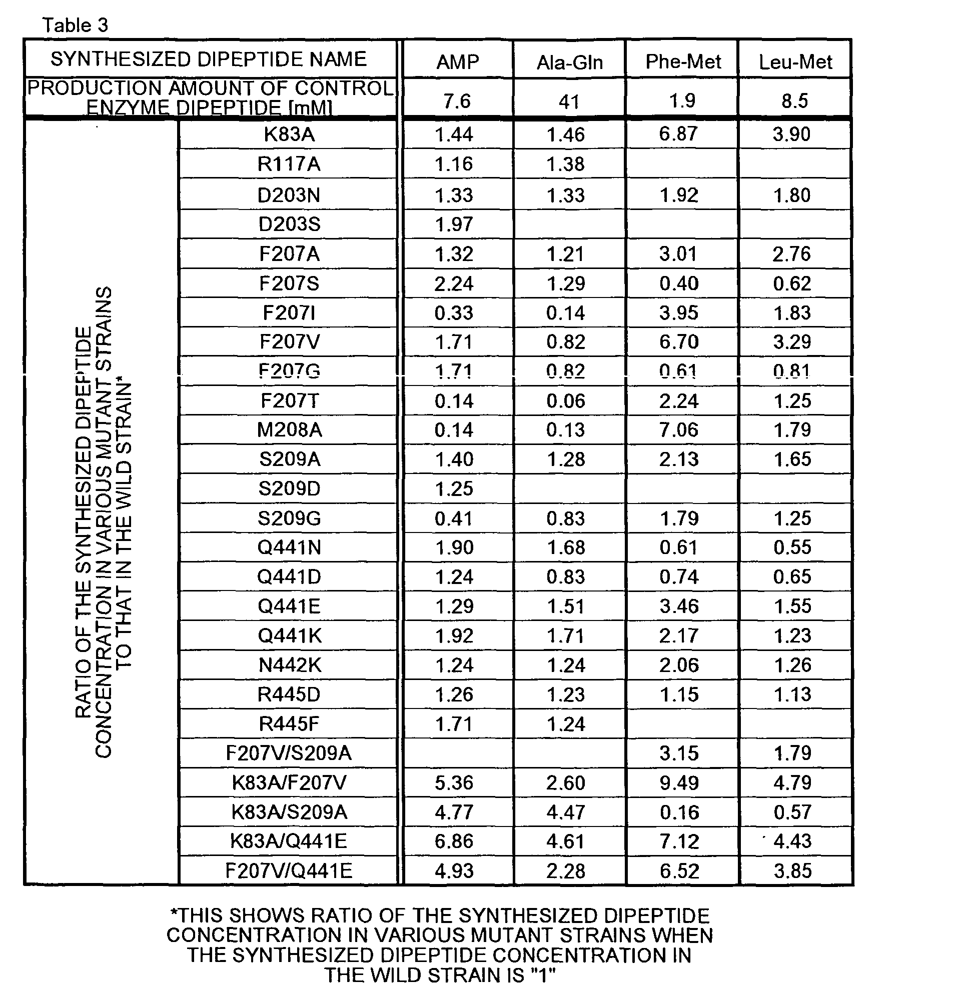

### (Example 3) Random screening 1

### (8) Preparation of pTrpT_Sm_Aet random library

In order to construct mutant Aet, pTrpT_Sm_Aet plasmid was used as the template for random mutagenesis using error prone PCR. The mutation was introduced using "GeneMorph PCR Mutagenesis Kit" supplied from Stratagene (USA) in accordance with the protocol of the manufacturer.

The PCR was performed using the oligonucleotides shown in SEQ ID NOS:5 and 6 as primers. That is, 500 ng of ds DNA (pTrpT_Sm_Aet or pTrpT_Sm_F207V plasmid) as the template, 125 ng each of the primers and 2.5 units of Mutazyme DNA polymerase were added to 50 µL of Mutazyme reaction buffer containing 200 µM each of dATP, dCTP, dGTP and dTTP, which was then subjected to the PCR using 30 cycles at 95°C for 30 seconds, 52°C for 30 seconds and 72°C for 2 minutes.

The PCR product was treated with NdeI/XbaI, and the resulting DNA fragment was ligated to pTrpT that had been treated with NdeI/XbaI. *Escherichia coli* JM109 (suppled from Takara Shuzo Co., Ltd.) was transformed with this solution containing the ligated product in accordance with standard methods. This was plated on an LB agar medium containing 100 µg/mL of ampicillin to make a library into which the random mutation had been introduced.

### (9) Screening from pTrpT_Sm_Aet random library: A

*Escherichia coli* JM109 strain transformed with the plasmid (pTrpT_Sm_AetM) containing each mutant Aet gene and *Escherichia coli* JM109 strain transformed with the plasmid containing the wild type Aet were inoculated to 150 µL (dispensed in wells of 96-well plate) of the medium containing 100 µg/mL of ampicillin (2 g/L of glucose, 10 g/L of yeast extract, 10 g/L of casamino acid, 5 g/L of ammonium sulfate, 1 g/L of potassium dihydrogen phosphate, 3 g/L of dipotassium hydrogen phosphate, 0.5 g/L of magnesium sulfate 7-hydrate, pH 7.5, 100 µg/mL of ampicillin), and cultured at 25°C for 16 hours with shaking. The cultivation was performed with shaking at 1000 rotations/minute using a bio-shaker (M/BR-1212FP) supplied from TITEC.

### (10) Primary screening

The primary screening was performed using the cultured medium obtained in Example 3 (9). Selection was performed as follows. 200 µL of a reaction solution (pH 8.2) containing 10 mM phenol, 6 mM AP, 5 mM Asp (OMe)₂, 7.5 mM Phe, 3.6 U/mL of peroxidase, 0.16 U/mL of alcohol oxidase, 10 mM EDTA and 100 mM borate was added to 5 µL of the cultured medium, which was then reacted at 25°C for about 20 minutes. After the reaction, an absorbance at 500 nm was measured, and an amount of released methanol was calculated. Those showing the large amount of released methanol were selected as those having the enzyme with high AMP-synthesizing activity.

### (11) Obtaining microbial cells

One platinum loopful of the strain selected in the primary screening was precultured in the LB agar medium at 25°C for 16 hours. One platinum loopful of each strain expressing the enzyme was inoculated to 2 mL of terrific medium (12 g/L of tryptone, 24 g/L of yeast extract, 2.3 g/L of potassium dihydrogen phosphate, 12.5 g/L of dipotassium hydrogen phosphate, 4 g/L glycerol, 100 mg/L of ampicillin) in a general test tube, and the main cultivation was performed at 25°C at 150 reciprocatings/minute for 18 hours.

### (12) Secondary screening

25 µL of the cultured broth was suspended in 500 µL of 100 mM borate buffer (pH 8.5 or pH 9.0) containing 10 mM EDTA, 50 mM dimethyl aspartate and 75 mM phenylalanine, which was then reacted at 20°C or 25°C for 10 or 15 minutes to measure the amount of synthesized AMP. Among the secondary screened strains, the strains which exerted improved specific activity was analyzed as to their mutation points. As a result, the following mutation points were specified. The mutant strains comprising the mutants 4, 5, 6, 7, 8, 9, 10, 14, 15 and 16 were obtained from the library derived from the wild strain as a parent strain (template), and the mutant strains comprising the mutants 17, 18, 19 and 20 were obtained from the library derived from the F207V mutant strain as the parent strain.

### (13) Production of peptide using microbial cells

The concentrations of AMP produced with the wild strain in the aforementioned reaction are shown in Table 4 (reaction time: 10 minutes), and the concentration of AMP produced with the mutant strain F207V is shown in Table 5 (reaction time: 15 minutes). For the dipeptide synthesized by each mutant strain, the ratio of the concentrations of the dipeptides synthesized by the mutant strain with respect to that by the parent strain are shown in Tables 4 and 5. Other conditions for the AMP synthesis reaction were the same as in the above Example 2 (5).

### Table 4

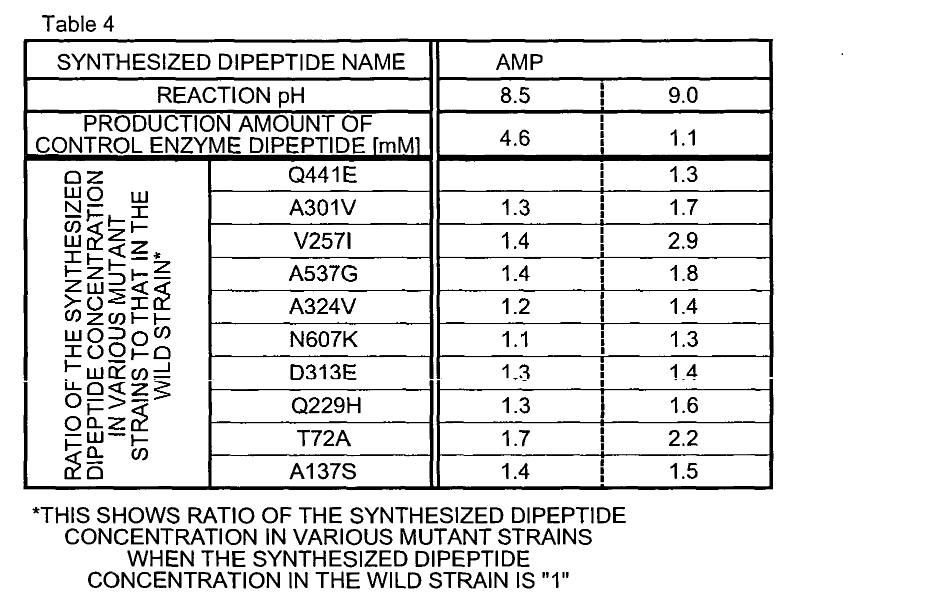

### Table 5

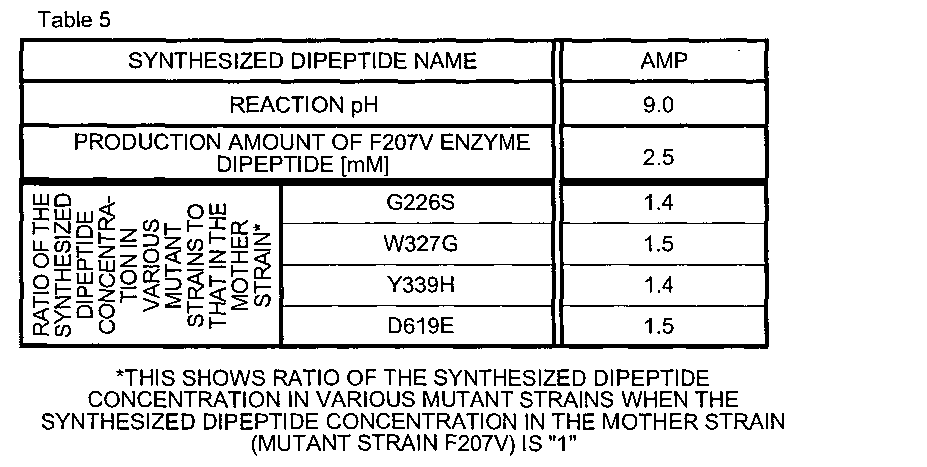

### (Example 4) Evaluation of specified mutation point by introducing it into pKF

### (14) Construction of strain in which specified mutation point has been introduced into pKF

The mutation point specified in Example 3 (12) was combined with already constructed pKF_Sm_F207V/Q441E to construct a triple mutant strain. The mutation was introduced in the same way as in Example 2 (2) using pKF_Sm_F207V/Q441E as the template and using the primers corresponding to various mutant enzymes (SEQ ID NOS:34 to 44 and 77). Resulting strains and the already constructed strains were cultured in the same way as in Example 2 (4).

### (15) Production of peptide using microbial cells <AMP>

500 µL of the cultured medium obtained in Example 4 (14) was centrifuged to collect microbial cells. The collected cells were then suspended in 500 µL of 100 mM borate buffer (pH 8.5 or pH 9.0) containing 10 mM EDTA, 50 mM dimethyl aspartate and 100 mM phenylalanine, and reacted at 25°C for 30 minutes. The concentrations of AMP synthesized with the wild strain in this reaction are shown in Table 6. For the dipeptide synthesized by various mutant strains, the ratio of the concentration of the dipeptide synthesized by the mutant strain with respect to that by the wild strain is shown in Table 6.

### (16) Production of peptide using microbial cells <Ala-Gln>

100 µL of the cultured medium obtained in Example 4 (14) was centrifuged to collect the microbial cells. The collected cells were then suspended in 1000 µL of 100 mM borate buffer (pH 8.5 or pH 9.0) containing 10 mM EDTA, 100 mM L-alanine methyl ester and 200 mM glutamine, and reacted at 25°C for 10 minutes. The concentrations of Ala-Gln synthesized with the wild strain in this reaction are shown in Table 6. For the dipeptide synthesized by various mutant strains, the ratio of the concentration of the dipeptide synthesized by the mutant strain with respect to that by the wild strain is shown in Table 6.

### (17) Production of peptide using microbial cells <Phe-Met, Leu-Met>

800 µL of the cultured medium obtained in Example 4 (14) was centrifuged to collect the microbial cells. The collected cells were then suspended in 400 µL of 100 mM borate buffer (pH 8.5 or pH 9.0) containing 10 mM EDTA, 50 mM L-phenylalanine methyl ester hydrochloride or L-leucine methyl ester hydrochloride, and 100 mM L-methionine, and reacted at 25°C for 20 minutes. The concentrations of Phe-Met and Leu-Met synthesized with the wild strain in this reaction are shown in Table 6. For the dipeptides synthesized by various mutant strains, the ratio of the concentration of the dipeptide synthesized by the mutant strain with respect to that by the wild strain is shown in Table 6.

### Table 6

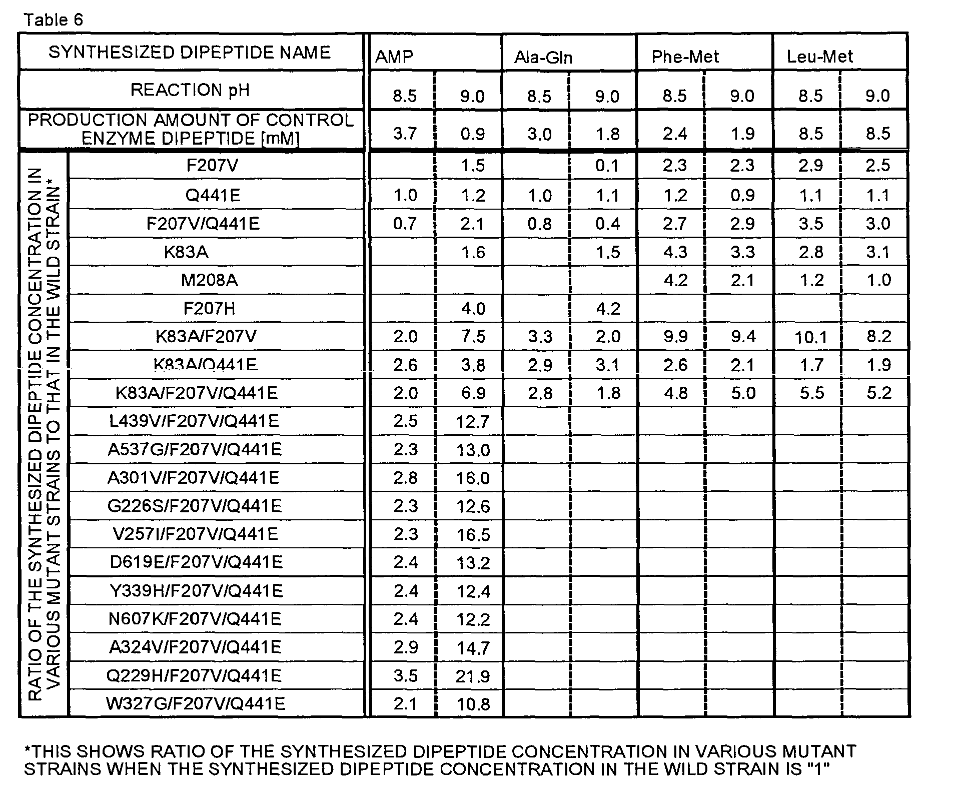

### (Example 5) Random screening 2

### (18) Preparation of pSTV_Sm_Aet random library

In order to construct mutant Aet, pHSG_Sm_Aet plasmid was used as the template for random mutagenesis using error prone PCR. The mutation was introduced using "GeneMorph PCR Mutagenesis Kit" supplied from Stratagene (USA) in accordance with the protocol of the manufacturer.

The PCR was performed using the oligonucleotides shown in SEQ ID NOS:3 and 4. That is, 100 ng of ds DNA (pHSG_Sm__Aet plasmid) as the template, 1.25 pmol each of the primers 1 and 2 and 2.5 units of Murazyme DNA polymerase were added to 50 µL of Mutazyme reaction buffer containing 200 µM each of dATP, dCTP, dGTP and dTTP. The mixture was heated at 95°C for 30 seconds and then subjected to the PCR using 25 cycles at 95°C for 30 seconds, 52°C for 30 seconds and 72°C for 2 minutes.

The PCR product was treated with EcoRI/PstI, and the resulting DNA fragment was ligated to pSTV28 (suppled from Takara Shuzo Co., Ltd.) that had been treated with EcoRI/PstI. *Escherichia coli* JM109 was transformed with this solution containing the ligated product. This transformed strain was plated on M9 agar medium (200 mL/L of 5*M9, 1 mL/L of 0.1 M CaCl₂, 1 mL/L of 1 M MgSO₄, 10 mL/L of 50% glucose, 10 g/L of casamino acid, 15 g/L of agar) containing 50 µg/mL of chloramphenicol and 0.1 mM IPTG to make a library in which random mutation was introduced. At that time, for the sake of simplicity of the subsequent screening, the transformants were applied so that about 100 colonies per plate would be grown. The above "5*M9" is a solution containing 64 g/L of Na₂HPO₄•7H₂O, 15 g/L of KH₂PO₄, 2.5 g/L of NaCl and 5 g/L of NH₄Cl.

### (19) Primary screening from pSTV based random library

In order to efficiently select the strain whose activity had been enhanced from the resulting transformants (library from mutant enzyme-expressing strain), Phe-pNA hydrolytic activity of each transformant was examined. A reaction solution (10 mM Phe-pNA, 10 mM OPT, 20 mM Tris-HCl (pH 8.2), 0.8% agar) (5 mL) was overlaid on the plate for transformant growth made in Example 5 (18), and color development by pNA produced by hydrolysis of Phe-pNA was examined (microbial cells are colored in yellow by liberation of pNA). The strongly colored colony was selected as the strain whose activity had been enhanced.

### (20) Obtaining microbial cells

The selected strains were cultured on the LB agar medium at 30°C for 24 hours. One platinum loopful of microbial cells of each strain was inoculated to 3 mL of the LB medium (agar was omitted from the above medium) containing 0.1 mM IPTG and 50 mg/L of chloramphenicol, and the main cultivation was performed at 25°C at 150 reciprocatings/minute for 20 hours.

### (21) Secondary screening

Microbial cells were collected from 400 µL of the cultured broth obtained in Example 5 (20). The collected cells were suspended in 400 µL of 100 mM borate buffer (pH 9.0) containing 10 mM EDTA, 50 mM Phe-OMe and 100 mM Met, and reacted at 25°C for 30 minutes. The amount of synthesized Phe-Met was measured, and the strains whose initial rate of the reaction was fast were selected. For the selected strains whose activity had been enhanced, the mutation point was analyzed, and the mutation points 11 and 12 were specified.

### (22) Production of peptide using microbial cells <Phe-Met, Leu-Met>

800 µL of the cultured medium obtained in Example 5 (20) was centrifuged to collect the microbial cells. The collected cells were then suspended in 400 µL of 100 mM borate buffer (pH 9.0) containing 10 mM EDTA, 25 mM L-phenylalanine methyl ester hydrochloride or L-leucine methyl ester hydrochloride, and 50 mM L-methionine, and reacted at 25°C for 20 minutes. The concentrations of Phe-Met and Leu-Met synthesized with the wild strain in this reaction are shown in Table 7. For the dipeptide synthesized by various mutant strains, the ratio of the concentration of the dipeptide synthesized by the mutant strain with respect to that by the wild strain is shown in Table 7.

### Table 7

**Table 7**

| SYNTHESIZED DIPEPTIDE NAME | | Phe-Met | Leu-Met |
|---|---|---|---|
| PRODUCTION AMOUNT OF CONTROL ENZYME DIPEPTIDE | | 1.35 mM | 4.86 mM |
| RATIO OF THE SYNTHESIZED DIPEPTIDE CONCENTRATION IN VARIOUS MUTANT STRAINS TO THAT IN THE WILD STRAIN* | F207V | 1.6 | 1.6 |
| | E551 K | 2.2 | 1.4 |
| | K83A/Q441E | 1.4 | 1.4 |
| | M208A/E551K | 5.3 | 2.4 |

| | | | |
|---|---|---|---|
| *THIS SHOWS RATIO OF THE SYNTHESIZED DIPEPTIDE CONCENTRATION IN VARIOUS MUTANT STRAINS WHEN THE SYNTHESIZED DIPEPTIDE CONCENTRATION IN THE WILD STRAIN IS "1" | | | |

### (Example 6) High expression of peptide-synthesizing enzyme gene in pSF_Sm_Aet

### (23) Construction of plasmid with high expression

An expression plasmid was constructed by ligating the mature peptide-synthesizing enzyme gene derived from *Sphingobacterium* to downstream of a modified promoter and a signal sequence of acid phosphatase derived from *Enterobacter aerogenes* by PCR.

The peptide-synthesizing enzyme gene was amplified by PCR using 50 µL of a reaction solution containing 0.4 mM pTrpT_Sm_Aet (Example 1) as a template, 0.4 mM each of Esp-S1 (5'- CCG TAA GGA GGA ATG TAG ATG AAA AAT ACA ATT TCG TGC C; SEQ ID NO:121) and S-AS1 (5'-GGC TGC AGT TTG CGG GAT GGA AGG CCG GC; SEQ ID NO:122) oligonucleotides as the primers, KOD plus buffer (suppled from Toyobo Co., Ltd.), 0.2 mM each of dATP, dCTP, dGTP and dTTP, 1 mM magnesium sulfate and 1 unit of KOD plus polymerase (suppled from Toyobo Co., Ltd.), by heating at 94°C for 30 seconds followed by 25 cycles at 94°C for 15 seconds, 55°C for 30 seconds and 68°C for two minutes and 30 seconds. The promoter and signal sequences of acid phosphatase were amplified by PCR using pEAP130 plasmid (see the following Reference Example 1, related patent application: JP 2004-83481) as the template, and E-S1 (5'-CCT CTA GAA TTT TTT CAA TGT GAT TT; SEQ ID NO:123) and Esp-AS1 (5'-GCA GGA AAT TGT ATT TTT CAT CTA CAT TCC TCC TTA CGG TGT TAT; SEQ ID NO:124) oligonucleotides as the primers under the same condition as the above. The reaction solutions were subjected to agarose electrophoresis, and the amplified DNA fragments were recovered using Microspin column (supplied from Amersham Pharmacia Biotech).

Then, a chimeric enzyme gene was constructed by PCR using the amplified DNA fragment mixture as the template, E-S1 and S-AS1 oligonucleotides as the primer, and the reaction solution having the same composition as the above, for 25 cycles of 94°C for 15 seconds, 55°C for 30 seconds and 68°C for two minutes and 30 seconds. The amplified DNA fragment was recovered using Microspin column (supplied from Amersham Pharmacia Biotech), and digested with XbaI and PstI. This was ligated to XbaI-PstI site of pCU18 plasmid. The nucleotide sequence was determined by a dye terminator method using a DNA sequencing kit, Dye Terminator Cycle Sequencing Ready Reaction (supplied from Perkin Elmer) and 310 Genetic Analyzer (ABI) to confirm that the objective mutations had been introduced, and then this plasmid was designated as pSF_Sm_Aet plasmid.

### (24) Construction of strain in which pSF_Sm_Aet rational mutation has been introduced

To construct the mutant Aet, pSF_Sm_Aet was used as the template of site-directed mutagenesis using the PCR. The mutation was introduced using QuikChange Site-Directed Mutagenesis Kit supplied from Stratagene (USA) and the primers corresponding to each mutant enzyme (SEQ ID NOS:45 to 78) in accordance with the protocol of the manufacturer. *Escherichia coli* JM109 strain was transformed with PCR products, and strains having objective plasmids were selected with ampicillin resistance as the indicator.
*Escherichia coli* JM109 strain having pSF_Sm_Aet is also represented as pSF_Sm_Aet/JM109 strain.

### (25) Obtaining microbial cells

Each mutant strain obtained in Example 6 (24) was precultured in the LB agar medium at 25°C for 16 hours. One platinum loopful of each strain expressing the enzyme was inoculated to 2 mL of terrific medium (12 g/L of tryptone, 24 g/L of yeast extract, 2.3 g/L of potassium dihydrogen phosphate, 12.5 g/L of dipotassium hydrogen phosphate, 4 g/L glycerol, 100 mg/L of ampicillin) in a general test tube, and the main cultivation was performed at 25°C at 150 reciprocatings/minute for 18 hours.

### (26) Production of peptide using microbial cells <Ala-Gln>

The cultured broth (5 µL) obtained in (25) was added to 500 µL of borate buffer (pH 8.5 or pH 9.0) containing 50 mM L-alanine methyl ester hydrochloride (A-OMe HCl), 100 mM L-glutamine and 10 mM EDTA" and reacted at 25°C for 10 minutes. The concentrations of Ala-Gln synthesized with the wild strain in this reaction are shown in Table 8. For the dipeptide synthesized by various mutant strains, the ratio of the concentration of the dipeptide synthesized by the mutant strain with respect to that by the wild strain is shown in Table 8.

### (27) Production of peptide using microbial cells <AMP>

The cultured broth (25 µL) obtained in the above was suspended in 500 µL of 100 mM borate buffer (pH 8.5 or pH 9.0) containing 10 mM EDTA, 50 mM dimethyl aspartate and 75 mM phenylalanine, and reacted at 20°C or 25°C for 15 minutes. The concentrations of AMP synthesized with the wild strain in this reaction are shown in Table 8. For the dipeptide synthesized by various mutant strains, the ratio of the concentration of the dipeptide synthesized by the mutant strain with respect to that by the wild strain is shown in Table 8.

### (28) Production of peptide using microbial cells <Phe-Met, Leu-Met>

The cultured broth (25 µL) obtained in the above was suspended in 500 µL of 100 mM borate buffer (pH 8.5 or pH 9.0) containing 10 mM EDTA, 25 mM L-phenylalanine methyl ester hydrochloride or L-leucine methyl ester hydrochloride, and 50 mM L-methionine, and reacted at 25°C for 15 minutes. The concentrations of Phe-Met and Leu-Met synthesized with the wild strain in this reaction are shown in Table 8. For the dipeptides synthesized by various mutant strains, the ratio of the concentration of the dipeptide synthesized by the mutant strain with respect to that by the wild strain is shown in Table 8.

### Table 8

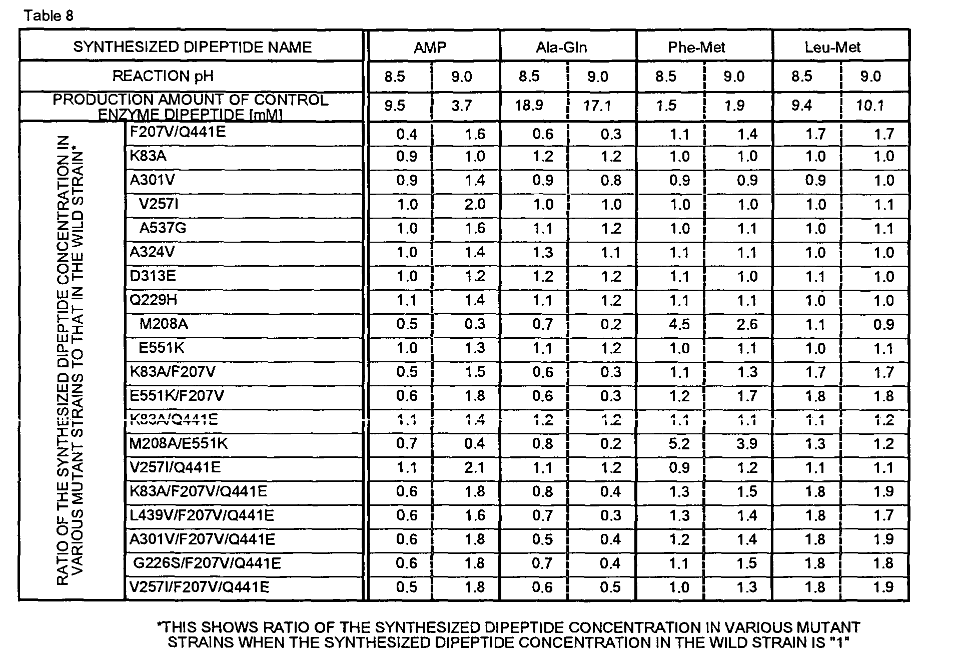

### (Example 7) Construction of strain having high activity by combination of mutations

### (29) Construction of random screening mutation-combining strain

To construct strains where various mutations were combined, pSF_Sm_Aet was used as the template for site-directed mutagenesis using the PCR.

The mutation was introduced using "QuikChange Multi" supplied from Stratagene (USA) in accordance with the protocol of the manufacturer and using the primers (99 to 120) corresponding to each mutant enzyme. The 5' terminus of the primers were phosphorylated before use with T4 polynucleotide kinase supplied from Takara Shuzo Co., Ltd. The primer was phosphorylated by adding 100 µmol DNA (primer) and 10 units of T4 polynucleotide kinase to 20 µL of 50 mM tris hydrochloric acid buffer (pH 8.0) containing 0.5 mM ATP, 10 mM magnesium chloride and 5 mM DTT and warming at 37°C for 30 minutes followed by heating at 70°C for 5 minutes.

The PCR was performed by adding 50 ng of ds DNA (pSF_Sm_Aet plasmid) as the template, 50 or 100 ng each of the 5'-phosphorylated mutagenic oligonucleotides (100 ng when the number of sort of primers in the combination is up to 3 types, and 50 ng when the number of sort of the primers in the combination is 4 types or more), 0.375 µL of Quik solution and 1.25 units of QuikChange Multi enzyme blend to 12,5 µL of QuikChange Multi reaction buffer containing 0.5 µL of dNTP mix, which was then subjected to the reaction of 30 cycles at 95°C for one minute, 53.5°C for one minute and 65°C for 10 minutes.

*Escherichia coli* JM109 strain was transformed with 2 µL of the reaction solution obtained by adding 5 unites of DpnI to the PCR product (total amount: 12.5 µL) and treating at 37°C for one hour. Transformed microbial cells were plated on the LB medium containing 100 µg/mL of ampicillin to obtain a library of randomly combined strains as ampicillin resistant strains.

### (30) Screening from library having combined mutations

*Escherichia coli* JM109 strain transformed with the plasmid (pTrpT_Sm_AetM) containing each mutant Aet gene and *Escherichia coli* JM109 strain transformed with the plasmid containing the wild type Aet were inoculated to 150 µL (dispensed in wells of 96-well plate) of the medium containing 100 µg/mL of ampicillin, and cultured at 25°C for 16 hours with shaking. The cultivation was performed with shaking at 1000 rotations/minute using a bio-shaker (M/BR-1212FP) supplied from TITEC. Using the resulting cultured medium, the selection was performed by screening.

### (31) Primary screening

A reaction solution (200 µL) (pH 8.2) containing 10 mM phenol, 6 mM AP, 5 mM Asp (OMe)₂, 7.5 mM Phe, 3.6 U/mL of peroxidase, 0.16 U/mL of alcohol oxidase, 10 mM EDTA and 100 mM borate was added to 5 µL of resulting microbial medium, which was then reacted at 25°C for about 20 minutes. After the reaction, the absorbance at 500 nm was measured, and the amount of released methanol was calculated. Those showing the large amount of released methanol were selected as those having the enzyme with high AMP-synthesizing activity.

### (32) Secondary screening

After the primary screening described above, the selected strains were cultured by the method described in Example 6 (25). 10 µL or 50 µL of each cultured broth was suspended in 1 mL of 100 mM borate buffer (pH 8.5) containing 10 mM EDTA, 50 mM Asp(OMe)₂ and 75 mM Phe, and reacted at 20°C or 25°C for 10 minutes. The amount of synthesized AMP was measured and strains that exerted a large synthesis amount were selected. The combination of the mutation points was determined in the selected strains by sequencing. The obtained strains and the combinations of the primers used for obtaining the strains are shown in Table 9.

### Table 9

**Table 9**

| OBTAINED STRAIN | MOTHER STRAIN | PRIMER USED |
|---|---|---|
| M7-35 (260) | pSF 2458 | 2458 K83A F, 2458 Q229H F, 2458 V257I F, 2458 A301V F, 2458 D313E F, 2458 A324V F, 2458 L439V F, 2458 Q441E F, 2458 A537G F, 2458 N607K F |
| M7-46 (261) | pSF 2458 | 2458 K83A F, 2458 Q229H F, 2458 V257I F, 2458 A301V F, 2458 D313E F, 2458 A324V F, 2458 L439V F, 2458 Q441E F, 2458 A537G F, 2458 N607K F |
| M7-54 (262) | pSF 2458 | 2458 K83A F, 2458 Q229H F, 2458 V257I F, 2458 A301V F, 2458 D313E F, 2458 A324V F , 2458 L439V F, 2458 Q441E F, 2458 A537G F, 2458 N607K F |
| M7-63 (263) | pSF 2458 | 2458 K83A F, 2458 Q229H F, 2458 V257I F, 2458 A301V F, 2458 D313E F, 2458 A324V F, 2458 L439V F, 2458 Q441E F, 2458 A537G F, 2458 N607K F |
| M7-95 (264) | pSF 2458 | 2458 K83A F, 2458 Q229H F, 2458 V257I F, 2458 A301V F, 2458 D313E F, 2458 A324V F, 2458 L439V F, 2458 Q441 EF, 2458 A537G F, 2458 N607K F |
| M9-9 (265) | M7-35 | T72A F, A137S F, 2458 Q441 E F |
| M9-10 (266) | M7-35 | T72A F, A137S F, 2458 Q441 E F |
| M11 -2(267) | M7-63 | T72A F, A137S F, 2458 L439V F |
| M11-3 (268) | M7-63 | T72A F, A137S F. 2458 L439V F |
| M12-1 (269) | M7-95 | T72A F, A137S F, 2458 L439V F |
| M12-3 (270) | M7-95 | T72A F, A137S F, 2458 L439V F |
| M21-18(271) | M9-9 | Q229X F |
| M21-22 (272) | M9-9 | Q229X F |
| M21-25 (273) | M9-9 | Q229X F |
| M22-25 (274) | M12-1 | Q229X F |
| M24-1 (275) | M9-9 | I228X F + Q229P F |
| M24-2 (276) | M9-9 | I228X F + Q229P F |
| M24-5 (277) | M9-9 | I228X F + Q229P F |
| M26-3 (278) | M9-9 | I230X F + Q229P F |
| M26-5 (279) | M9-9 | I230X F + Q229P F |
| M29-3 (280) | M12-1 | I228X F + Q229H F |
| M33-1 (281) | M12-1 | S256X F + V257I F |
| M35 - 4 (282) | M11-3 | A137X F, 2458 V257I F 2458 Q229P F |
| M37-5 (283) | M11-3 | 2458 V257I F, 2458 Q229P F, A324X F |
| M39-4 (284) | M12-3 | 2458 Q229P F, A301X F |
| M41-2 (285) | M12-3 | 2458 Q229P F, A537X F |

### (33) Production of peptide using microbial cells

The combination strains obtained in the above were evaluated. The cultured broth (25 µL) obtained in the above was suspended in 500 µL of 100 mM borate buffer (pH 8.5) containing 10 mM EDTA, 50 mM dimethyl aspartate and 75 mM phenylalanine, and reacted at 20°C for 15 minutes. The concentration of AMP synthesized with the wild strain in this reaction is shown in Table 10. For the dipeptide synthesized by various mutant strains, the ratio of the specific activity of the dipeptide synthesized by the mutant strain with respect to the specific activity as to the wild strain being 1 is shown in Table 10.

### Table 10

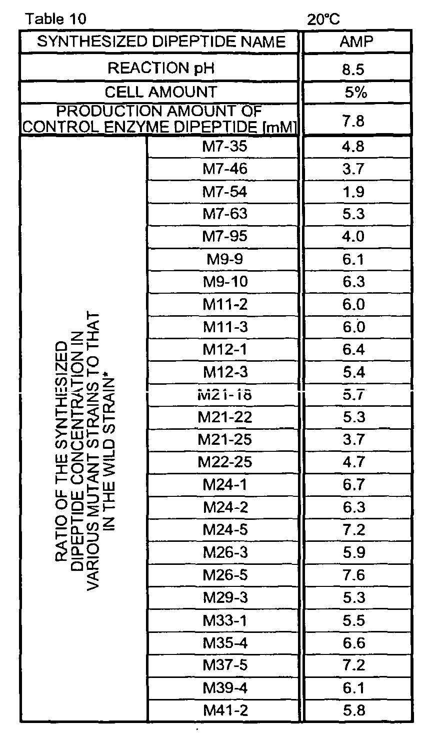

### (Example 8) Study of substrate specificity

### (34) Study of substrate specificity using mutant enzyme

The production of peptides was examined in the cases of using various amino acid methyl ester for the carboxy component and L-methionine for the amine component. The cultured broth (25 µL) prepared by the method described in Example 6 (25) was added to 500 µL of borate buffer (pH 8.5) containing 25 mM L-amino acid methyl ester hydrochloride (X-OMe-HCl) shown in Table 11, 50 mM L-methionine and 10 mM EDTA. The mixture was then reacted at 25°C for 15 minutes or 3 hours. The amounts of various peptides synthesized with the wild strain in this reaction are shown in Tables 11-1 and 11-2. The amount of the produced peptide with a mark "+" was shown in terms of estimated reference value of the peak, tentatively determining an area value of 8000 in HPLC being 1 mg/L. For the dipeptides synthesized by various mutant strains, the ratio of the concentration of the dipeptide synthesized by the mutant strain with respect to that by the wild strain is shown in Tables 11-1 and 11-2.

### Table 11-1

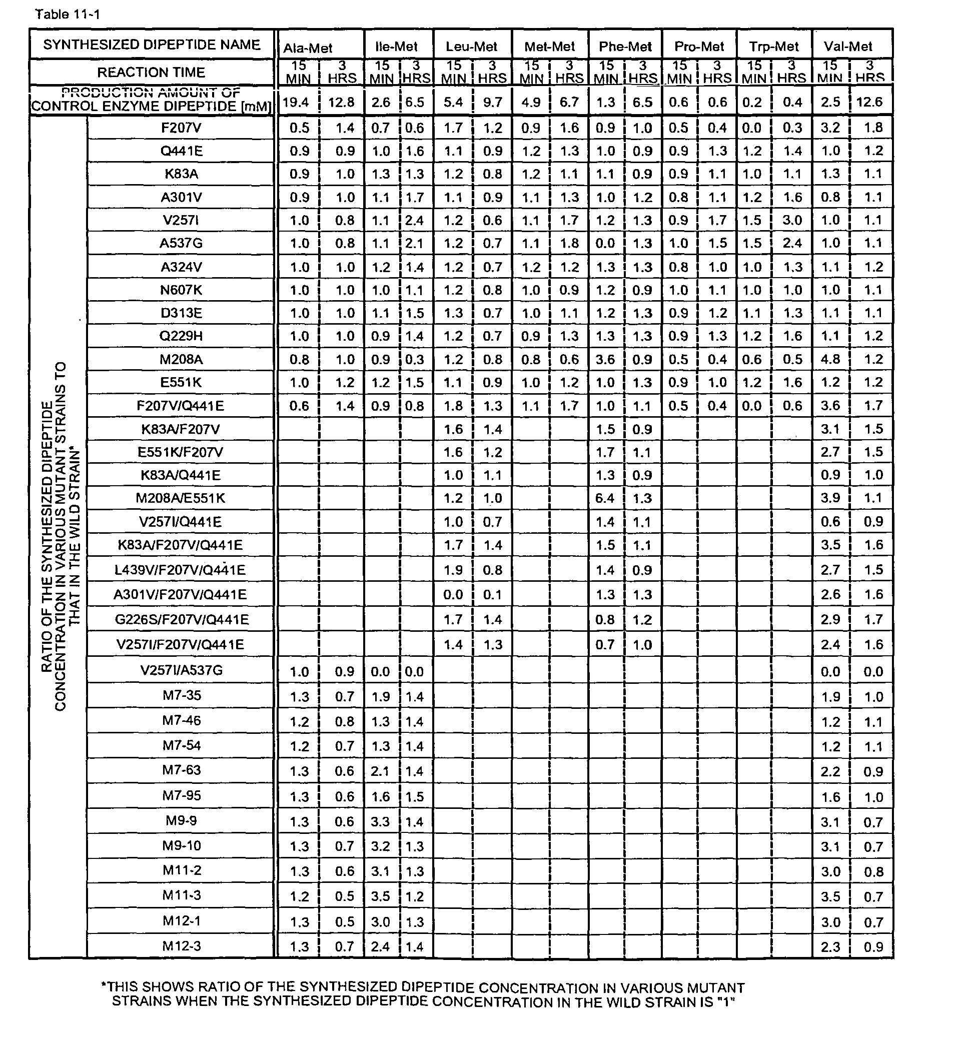

### Table 11-2

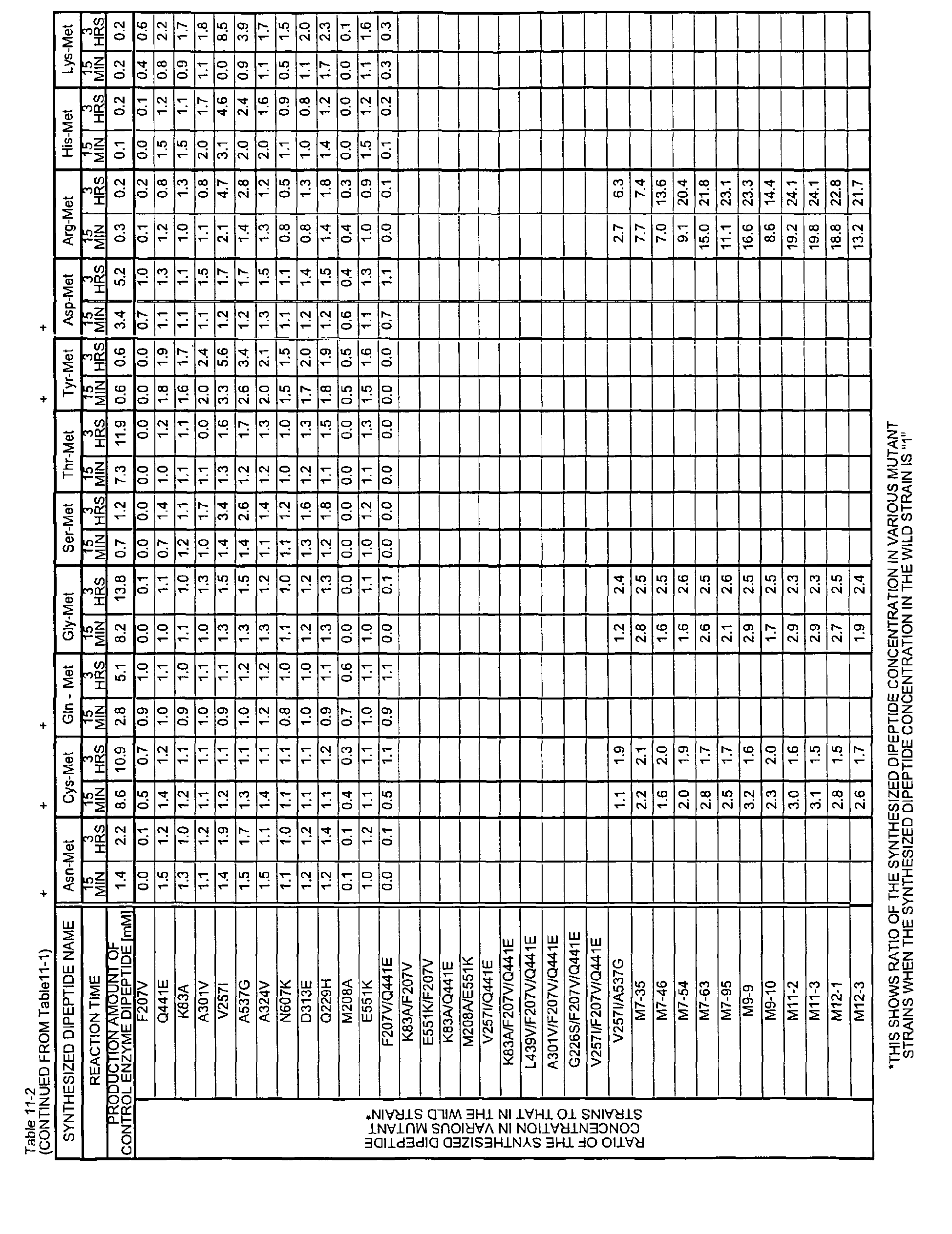

### (Example 9) Random screening

### (35) Screening from pTrpT_Sm_Aet random library: B

The library produced in Example 3 (8) was cultured in the same way as in Example 3 (9), and two types of screenings were performed using the cultured medium.

### (36) Primary screening: A

A reaction solution (200 µL) (pH 8.2) containing 10 mM phenol, 6 mM AP, 5 mM Asp(OMe)₂, 5mM Ala-OEt, 7.5 mM Phe, 3.6 U/mL of peroxidase, 0.16 U/mL of alcohol oxidase, 10 mM EDTA and 100 mM borate was added to 5 µL of the resulting microbial medium, which was then reacted at 25°C for about 20 minutes. After the reaction, the absorbance at 500 nm was measured, and an amount of released methanol was calculated. Herein, those showing the large amount of released methanol were selected as those having the enzyme which tends to synthesize AMP more abundantly than Ala-Phe.

### (37) Primary screening: B

A reaction solution (200 µL) (pH 8.2) containing 10 mM phenol, 6 mM AP, 5 mM Asp (OMe)₂, 5mM A(M), 3.6 U/mL of peroxidase, 0.16 U/mL of alcohol oxidase, 10 mM EDTA and 100 mM borate was added to 5 µL of the resulting microbial medium, which was then reacted at 25°C for about 20 minutes. After the reaction, the absorbance at 500 nm was measured, and an amount of released methanol was calculated. Herein, those showing the small amount of released methanol were selected as enzymes which has less tendency to produce AM (AM) .

### (38) Secondary screening

The strains selected in Example 9 (36) and (37) were cultured in the same way as in Example 6 (25), and 50 µL of each cultured broth was suspended in 1 mL of 100 mM borate buffer (pH 8.5) containing 10 mM EDTA, 50 mM Asp (OMe)₂, 50 mM Ala-OMe and 75 mM Phe, and reacted 20°C for 10 minutes. The amounts of synthesized AMP and Ala-Phe were measured, and the strains whose initial rate of the reaction was fast were selected. Likewise, 50 µL of each cultured broth was suspended in 1 mL of 100 mM borate buffer (pH 9.0) containing 10 mM EDTA, 50 mM Asp(OMe)₂, and 75 mM Phe, and reacted at 20°C for 10 minutes. The yields of synthesized AMP were measured, and the strains exerting the high yield were selected. The mutation 21 was selected as the valid mutation point.

### (Example 10) Evaluation of specified mutation point by introducing it into pSF

### (39) Introduction of mutation into V184

The mutation point, V184A obtained in Example 9 was introduced into pSF_Sm_Aet, and also introduced into an existing construct, pSF_Sm_M35-4. V184X strains were also constructed by substituting V184 with other amino acids. The mutation was introduced in the same way as in (2) using pSF_Sm_Aet or pSF_Sm_M35-4 as the template and using the primers (SEQ ID NO:79 to 98) corresponding to each mutant enzyme. The resulting strains were cultured by the method described in Example 6 (25).

### (40) Production of peptide using microbial cells <AMP>

The cultured broth (25 µL) prepared by the method described in Example 6 (24) was suspended in 500 µL of 100 mM borate buffer (pH 8.5 or pH 9.0) containing 10 mM EDTA, 50 mM dimethyl aspartate and 75 mM phenylalanine, and reacted at 20°C for 10 minutes. The concentrations of AMP synthesized with the wild strain in this reaction are shown in Table 12. For the dipeptide synthesized by various mutant strains, the ratio of the concentration of the dipeptide synthesized by the mutant strain with respect to that by the wild strain is shown in Table 12.

### Table 12

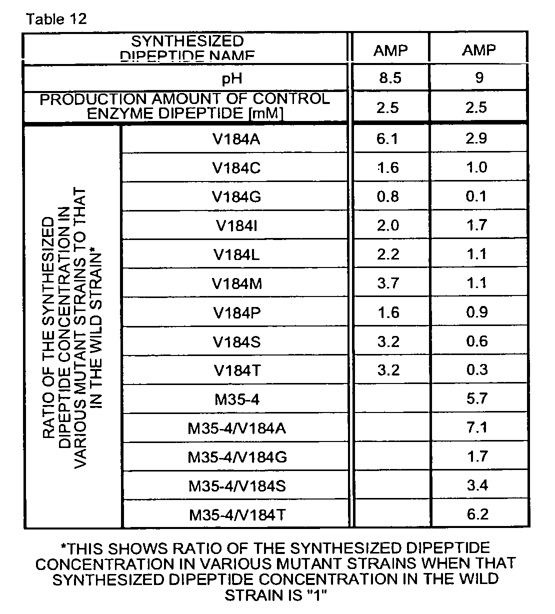

### (41) Production of peptide using microbial cells <AMP>

The cultured broth obtained by the method described in Example 6 (25) was suspended in 100 mM borate buffer (pH 8.5 or pH 9.0) containing 10 mM EDTA, 50 mM dimethyl aspartate and 75 mM phenylalanine, and reacted at 20°C. The yields of AMP synthesized with the wild strain and various mutant strains in this reaction are shown in Table 13.

### Table 13

**Table 13**

| SYNTHESIZED DIPEPTIDE NAME | | AMP | AMP |
|---|---|---|---|
| pH | | 8.5 | 9 |
| YIELD | | 36.8 | 57.0% |
| | V184A | 55.5 | 73.3 |
| | V184C | 54.9 | |
| | V184G | 64.3 | |
| | V1841 | 46.0 | |
| | V184L | 44.5 | |
| | V184M | 56.3 | |
| | V184P | 54.6 | |
| | V184S | 61.6 | |
| | V184T | 60.3 | |
| | M35-4 | | 57.5 |
| | M35-4/V184A | | 68.8 |
| | M35-4/V184G | | 77.2 |
| | M 35-4/V184N | | 77.3 |
| | M35-4/V184S | | 70.8 |
| | M35-4/V184T | | 67.7 |

### (Example 11) Change of natures in mutant enzymes

### (42) pH Stability of enzymes

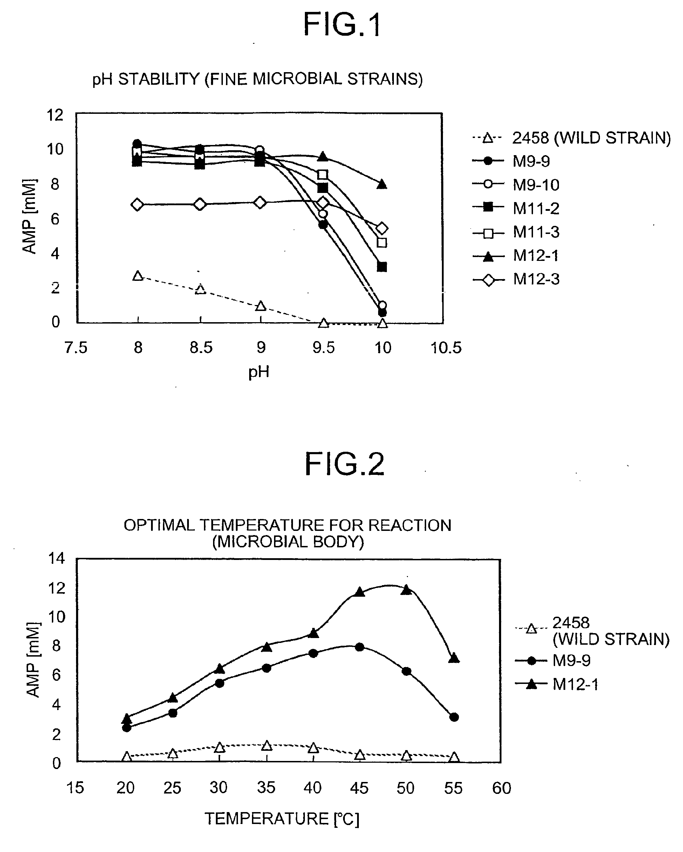

pH Stability was examined by incubating the enzyme at a certain pH for a certain period of time and subsequently synthesizing AMP from dimethyl L-aspartate hydrochloride and L-phenylalanine. The cultured broth (10 µL) prepared by the method described in Example 6 (25) was mixed with 190 µL of each of buffers at a variety of pH's (8.5, 9.0, 9.5) (as to M9-9 and M12-1, pH 8.0 was also tested), incubated for 30 minutes, and subsequently added to 400 µL of 450 mM borate buffer containing 75 mM dimethyl L-aspartate, 112.5 mM L-phenylalanine and 15 mM EDTA, which was then reacted at 20°C for 20 minutes. The concentrations of synthesized AMP are shown in Fig. 1.

### (43) Optimal reaction temperature of enzymes

Effects of the reaction temperature on the reaction to synthesize AMP from dimethyl L-aspartate hydrochloride and L-phenylalanine were examined. The cultured broth (20 µL) prepared by the method described in Example 6 (25) was added to 980 µL of 100 mM borate buffer (pH 8.5) containing 50 mM dimethyl L-aspartate, 75 mM L-phenylalanine and 10 mM EDTA, and reacted at each temperature (20, 25, 30, 35, 40, 45, 50, 55, 60°C) for 5 minutes. The concentrations of synthesized AMP are shown in Fig. 2. As a result, the optimal temperatures of the present enzymes were 35°C, 45°C and 50°C for 2458, M9-9 and M12-1, respectively.

### (44) Temperature stability of enzymes

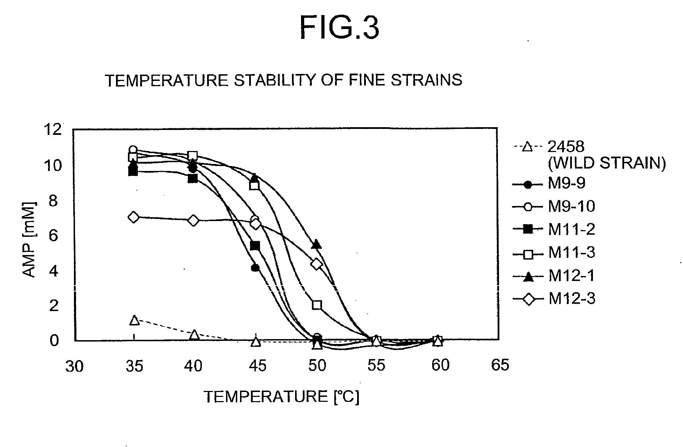

Temperature stability was examined by incubating the enzymes at a certain temperature for a certain period of time and subsequently synthesizing AMP from dimethyl L-aspartate hydrochloride and L-phenylalanine. The cultured broth (20 µL) that had been prepared by the method described in Example 6 (25) was incubated at each temperature (35, 40, 45, 50, 55, 60°C) for 30 minutes, and was subsequently added to 980 µL of 100 mM borate buffer (pH 8.5) containing 50 mM dimethyl L-aspartate, 100 mM L-phenylalanine and 10 mM EDTA, which was then reacted at 20°C for 5 minutes. The concentrations of AMP synthesized thereby are shown in Fig. 3.

### <Analysis of products>

In the aforementioned Examples, the products were quantified by the high performance liquid chromatography, details of which are as follows. Column: Inertsil ODS-3 (supplied from GL Sciences), eluants: i) aqueous solution of phosphoric acid containing 5.0 mM sodium 1-octanesulfonate (pH 2.1): methanol=100:15 to 50, ii) aqueous solution of phosphoric acid containing 5.0 mM sodium 1-octanesulfonate (pH 2.1): acetonitrile=100:15 to 30, flow rate: 1.0 mL/minute, and detection: 210 nm.

### <Reference Example: Preparation of pEAP130 plasmid - Modification of promoter sequence of acid phosphatase gene derived from Enterobacter aerogenes>

In accordance with the description of Journal of Bioscience and Bioengineering, 92(1):50-54, 2001 (or JP H10-201481 A publication), a DNA fragment of 1.6 kbp which contains an acid phosphatase gene region was cleaved out and isolated with restriction enzymes SalI and KpnI from a chromosomal DNA derived from *Enterobacter aerogenes* IFO 12010 strain. The fragment was ligated to pUC118 to construct a plasmid DNA which was designated as pEAP120. The nucleotide sequences encoding the promoter and the signal peptide of acid phosphatase were incorporated into the plasmid pEAP120. The strain to which IFO number was given has been deposited to Institute for Fermentation (17-85 Joso-honnmachi, Yodogawa-ku, Osaka, Japan), but, its operation has been transferred to NITE Biological Resource Center (NBRC), Department of Biotechnology (DOB), National Institute of Technology and Evaluation since June 30, 2002, and the strain can be furnished from NBRC with reference to the above IFO number.

Subsequently, it was attempted to enhance the activity by partially modifying the promoter sequence present upstream of this gene. The site-directed mutation was introduced using QuikChange Site-Directed Mutagenesis Kit (supplied from Stratagene) to replace -10 region of the putative promoter sequence of the acid phosphatase gene from AAAAAT to TATAAT. Oligonucleotide primers for PCR, EM1 (5'-CTT ACA GAT GAC TAT AAT GTG ACT AAA AAC: SEQ ID NO:125) and EMR1 (5'-GTT TTT AGT CAC ATT ATA GTC ATC TGT AAG: SEQ ID NO:126) designed for introducing the mutation were synthesized. In accordance with the method of the instructions, the mutation was introduced using pEAP120 as the template. The nucleotide sequence was determined by the dye termination method using DNA Sequencing Kit Dye Terminator Cycle Sequencing Ready Reaction (supplied from Perkin Elmer) and using 310 Genetic analyzer (ABI) to confirm that the objective mutation had been introduced, and this plasmid was designated as pEAP130. The plasmid pEAP130 has the nucleotide sequences encoding the signal peptide and the modified promoter derived from the N terminal region of acid phosphatase.

### (Example 12) Construction of rational mutant strain using pSFN vector

### (45) Construction of pSFN_Sm_Aet strain

In order to construct a plasmid pSFN_Sm_Aet from which a fragment of an Aet enzyme gene can be cut out by the treatment with restriction enzymes, pSF_Sm_Aet (Example 6) was used as a template of the site-directed mutagenesis using PCR. The mutation was introduced using "QuikChange Site-Directed Mutagenesis Kit" supplied from Stratagene (USA) in accordance with the manufacturer's protocol and using various primers. First, the base at position 4587 on pSF_Sm_Aet plasmid was substituted (from "a" to "g") by introducing the mutation using the oligonucleotides shown in SEQ ID NOS:127 and 128 as the primers, to delete NdeI site. Subsequently, the base at position 2363 on pSF_Sm_Aet plasmid was substituted (from "tag" to "atg") by introducing the mutation using the oligonucleotides shown in SEQ ID NOS:129 and 130, to introduce NdeI site. *Escherichia coli* JM109 was transformed with the PCR product, and a strain having the objective plasmid pSFN_Sm_Aet was selected using ampicillin resistance as an indicator.

### (46) Introduction of pKF_Sm_Aet rational mutation

In order to construct a mutant Aet, pKF_Sm_Aet plasmid (Example 2 (1)) was used as the template of the site-directed mutagenesis using the ODA method. The mutation was introduced by the same method as in Example 2 (2) using the primers (SEQ ID NOS:131 to 137) corresponding to various mutant enzymes, and the strains having the objective plasmid pKF_Sm_Aet containing the mutant Aet gene was selected.

### (47) Introduction into pSFN_Sm_Aet

The objective gene was amplified by PCR with the plasmid pKF_Sm_AetM containing the mutant Aet gene as the template using the oligonucleotides shown in SEQ ID NOS:129 and 122 as the primers. This DNA fragment was treated with NdeI/PstI, and the resulting DNA fragment was ligated to pSFN_Sm_Aet which had been treated with NdeI/PstI. *Escherichia coli* JM109 was transformed with this solution containing the ligated product, and a strain having the objective plasmid was selected using ampicillin resistance as the indicator. The resulting strain and the already constructed strains were cultured by the same method as in Example 6 (25).

### (48) Production of peptide using microbial cells <X-Met>

A cultured broth (40 µL) obtained in (47) was suspended in 400 µL of 100 mM borate buffer (pH 8.5 or 9.0) containing 10 mM EDTA, 50 mM amino acid methylester and 100 mM Met, and reacted at 20°C for one hour. Concentrations of various dipeptides synthesized in this reaction with the wild strain are shown in Table 14. For the dipeptide synthesized by various mutant enzyme-expressing strains (referred to as mutant strains), the ratio of the concentration of the dipeptides synthesized thereby with respect to that by the wild strain is shown in Table 14.

### Table 14

**Table 14**

| SYNTHESIZED DIPEPTIDE NAME | | Pro-Met | Val-Met | His-Met | Arg-Met | Val-Met |
|---|---|---|---|---|---|---|
| pH | | 9.0 | 9.0 | 8.5 | 8.5 | 8.5 |
| PRODUCTION AMOUNT OF CONTROL ENZYME DIPEPTIDE [mM] | | 3.46 | 11.48 | 7.64 | 4.62 | 12.06 |
| RATIO OF THE SYNTHESIZED DIPEPTIDE CONCENTRATION IN VARIOUS MUTANT STRAINS TO THAT IN THE WILD STRAIN* | W187A | 0.00 | 1.23 | 0.11 | 0.22 | 2.40 |
| | S209A | 1.70 | 1.53 | 1.49 | 1.48 | 0.92 |
| | S209G | 1.30 | 1.29 | 0.00 | 0.06 | 0.00 |
| | F211A | 0.00 | 1.83 | 0.88 | 1.04 | 0.74 |

| | | | | | | |
|---|---|---|---|---|---|---|
| *THIS SHOWS RATION OF THE SYNTHESIZED DIPEPTIDE CONCENTRATION IN VARIOUS MUTANT STRAINS WHEN THE SYNTHESIZED DIPEPTIDE CONCENTRATION [mM] IN THE WILD STRAIN IS "1" | | | | | | |

### (Example 13) Study of substrate specificity of various rational mutant strains

### (49) Production of dipeptide using microbial cells <Ala-X>

The production of the peptide when alanine methyl ester was used as the carboxy component and various L-amino acids were used as the amine component was examined. As the mutant enzymes, the mutant strains made in Examples 7 (32), 10 (39) and 12 (47) were used. The cultured broth (20 µL) obtained by the cultivation method described in Example 6 (25) was added to 400 µL of borate buffer (pH 8.5) containing 50 mM alanine methyl ester hydrochloride (Ala-OMe HCl), 100 mM L-amino acid and 10 mM EDTA, and reacted at 20°C. The concentrations (mM) of various dipeptides synthesized in this reaction with the wild strain are shown in Table 15. For the dipeptide synthesized by various mutant strains, the ratio of the concentration of the dipeptides synthesized thereby with respect to that by the wild strain is shown in Table 15. In Table 15, the synthesis of Ala-Gly and Ala-Thr was measured by the reaction for 10 minutes, and the synthesis of the other dipeptides was measured by the reaction for 15 minutes.

### Table 15

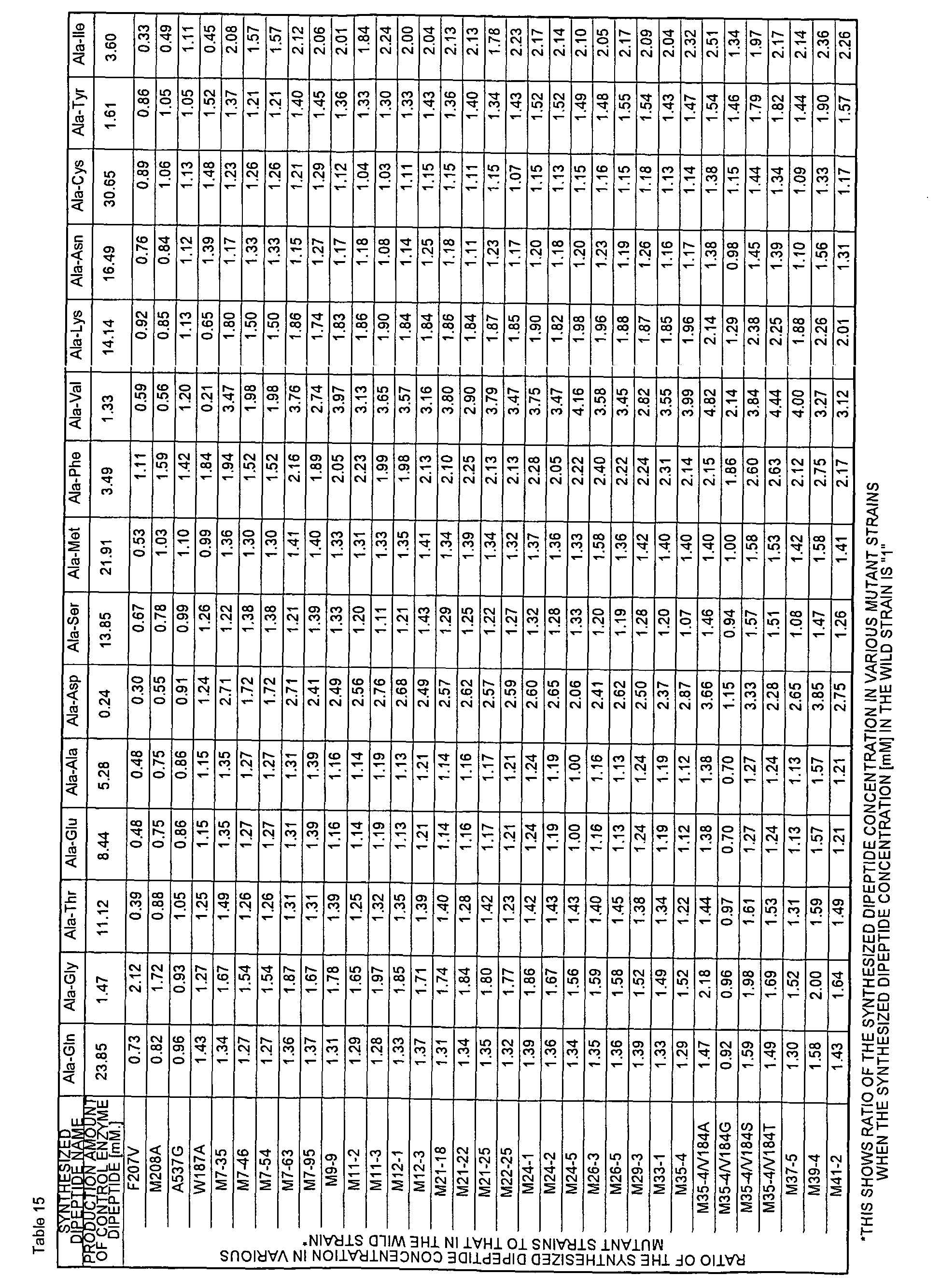

### (50) Production of dipeptide using microbial cells <Ala-X>

The cultured broth (20 µL) obtained in Example 12 (47) was added to 400 µL of 100 mM borate buffer (pH 8.5) containing 10 mM EDTA, 50 mM alanine methyl ester, and 100 mM L-amino acid, and reacted at 20°C for 15 minutes. The concentrations (mM/O.D.) of various dipeptides synthesized in this reaction with the wild strain are shown in Table 16. For the dipeptides synthesized by various mutant strains, the ratio of the concentration of the dipeptides synthesized thereby to that by the wild strain is shown in Table 16.

### Table 16

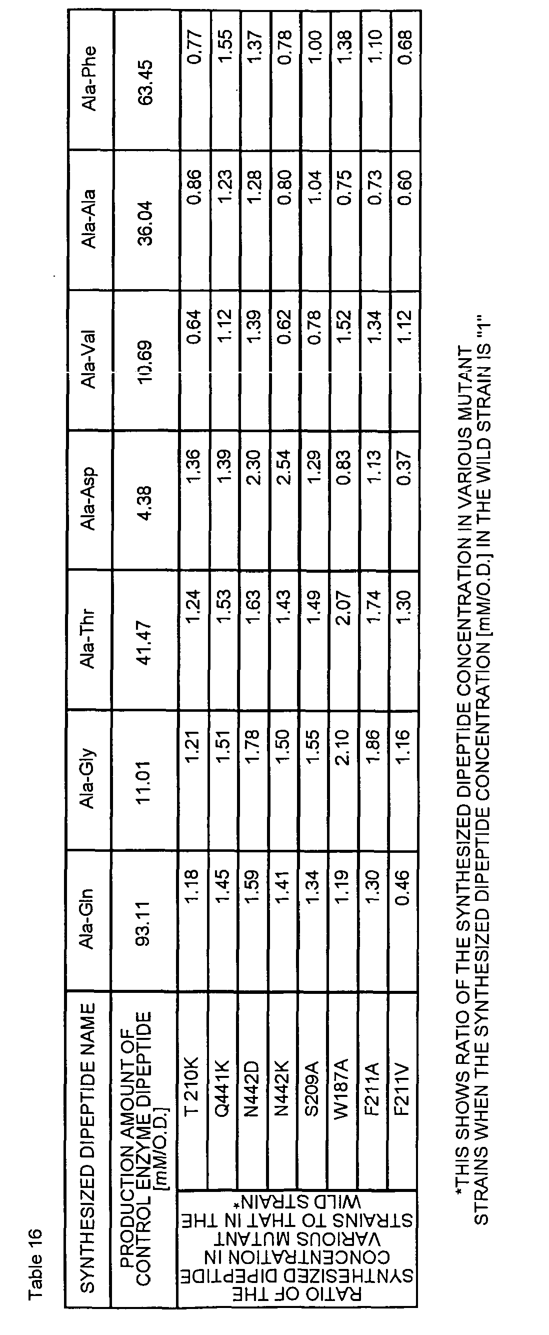

### (Example 14) Construction of strain having high activity by combination of mutations: A

### (51) Construction of pSF_Sm_Aet rational mutant strain

In order to construct mutant Aet, pSF_Sm_Aet was used as the template of the site-directed mutagenesis using PCR. The mutation was introduced by the same method as in Example 12 (45) using the primers (SEQ ID NOS:138 to 157, 160 to 167) corresponding to various mutant enzymes. *Escherichia coli* JM109 was transformed with the PCR product, and strains having the objective plasmid were selected using ampicillin resistance as the indicator. The resulting strain and the already constructed strains (Example 10 (39)) were cultured by the same method as in Example 6 (25).

### (52) Production of peptide using microbial cells <Ala-X>

The cultured broth (20 µL) obtained in (51) was added to 400 µL of borate buffer (pH 8.5) containing 50 mM alanine methyl ester hydrochloride (Ala-OMe HCl), 100 mM L-amino acid and 10 mM EDTA, and reacted at 20°C for 15 minutes. The concentrations (mM/O.D.) of various dipeptides (Ala-X) synthesized in this reaction with the wild strain are shown in Table 17. For the dipeptides synthesized by various mutant strains, the ratio of the concentration of the dipeptides synthesized thereby with respect to that by the wild strain is shown in Table 17.

### Table 17

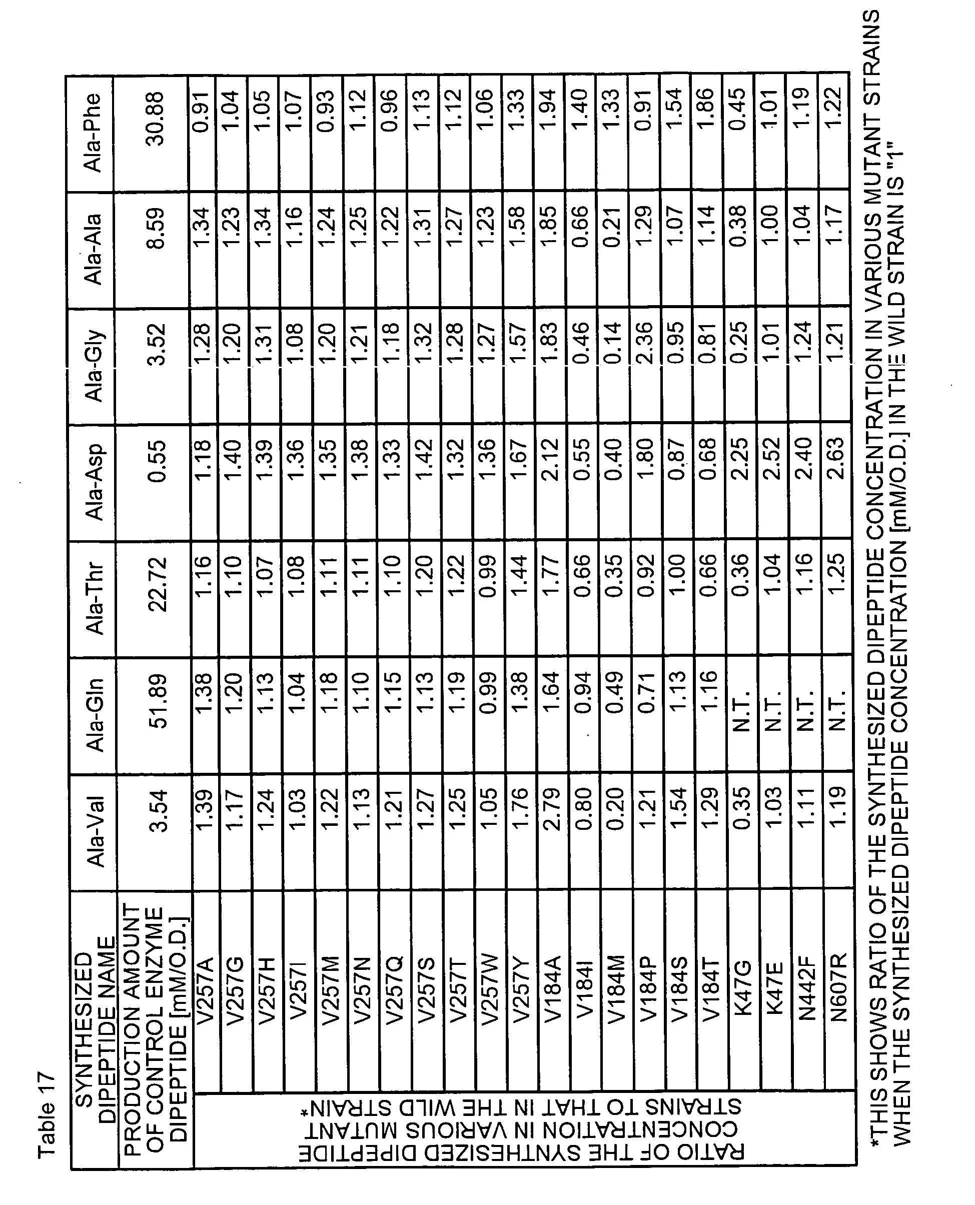

### (53) Production of peptide using microbial cells <Ala-X>

Mutation points V184A and V184P whose effects had been observed in (52) were introduced into pSF_Sm_M7-35. V257Y was introduced into pSF_Sm_M7-35 and pSF_Sm_V184A. The mutation was introduced by the same method as in (45) using pSF_Sm_M7-35 or pSF_Sm_V184A as the template and using the primers corresponding to various mutant enzymes (SEQ ID NOS:79, 80, 93, 94, 156, 157). The resulting strains were cultured by the method described in Example 6 (25).

### (54) Production of peptide using microbial cells <Ala-X>

The mutation points W187A, F211A, Q441E, Q441K and N442D whose effects had been observed in Table 11 in Example 8 (34) and Table 16 in Example 13 (50) were introduced into the already-constructed pSF_Sm_M7-35. Double substitution and a triple substitution such as pSF_Sm_V184A/W187A, V184A/N442D and V184A/N442D/L439V were also constructed. In addition, the mutant strain obtained by introducing F207V into pSF_Sm_M7-35/V184A was also constructed. The mutation was introduced by the same method as in Example 12 (45) using pSF_Sm_M7-35, pSF_Sm_V184A or pSF_Sm_M7-35/V184A as the template and using the primers (SEQ ID NOS:131, 158, 134, 159, 14, 170, 168, 169) corresponding to various mutant enzymes. The resulting strains and already-constructed strains were cultured by the method described in Example 6 (25).

### (55) Production of peptide using microbial cells <Ala-X>

The cultured broth (20 µL) obtained in (53) or (54) was added to 400 µL of borate buffer (pH 8.5) containing 50 mM alanine methyl ester hydrochloride (Ala-OMe HCl), 100 mM L-amino acid and 10 mM EDTA, and reacted at 20°C for 15 minutes. The concentrations (mM/O.D.) of various dipeptides (Ala-X) synthesized in this reaction with the wild strain are shown in Table 18. For the dipeptides synthesized by various mutant strains, the ratio of the concentration of the dipeptide synthesized thereby with respect to that by the wild strain is shown in Table 18.

### Table 18

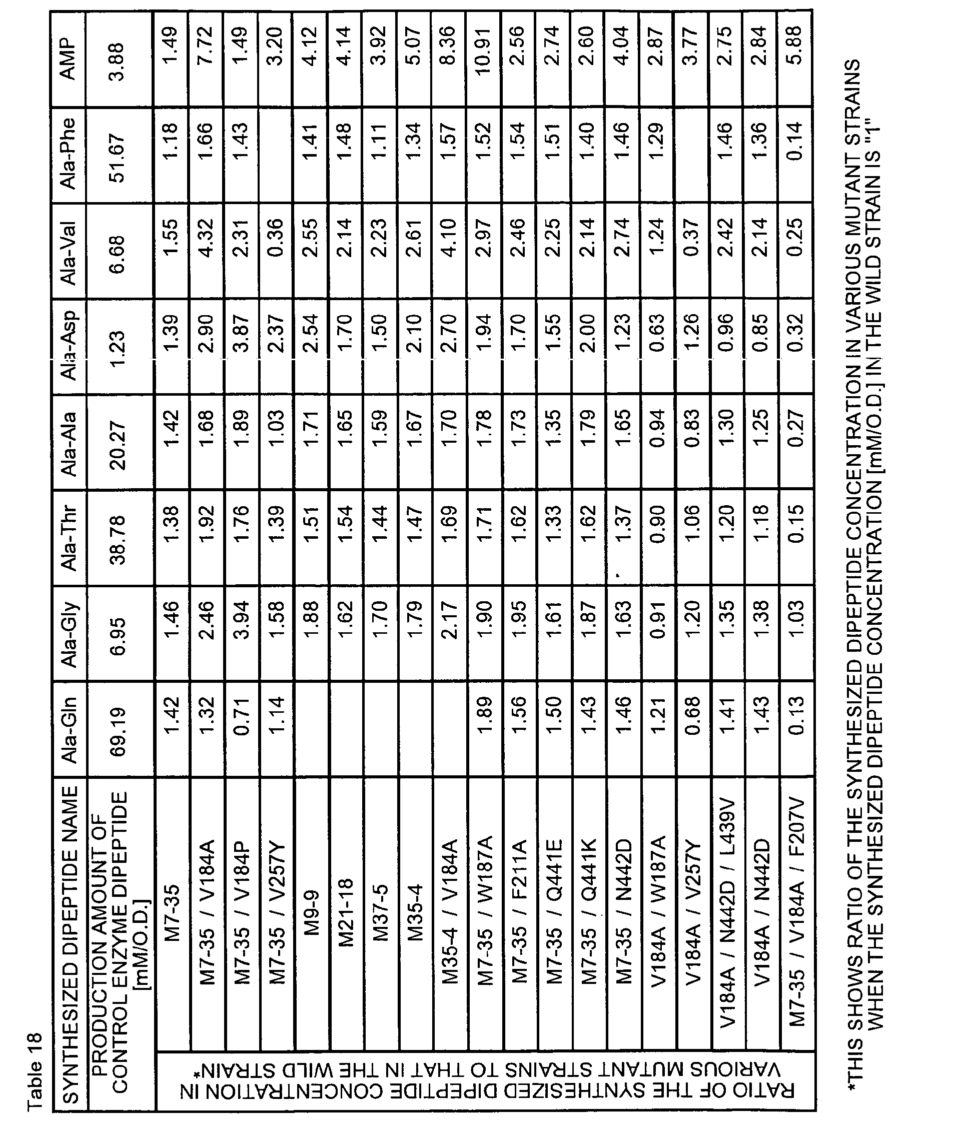

### (56) Production of peptide using microbial cells <Ala-X>

The mutation points K83A, W187A, F211A, and N442D whose effects had been observed in Example 14 (49) were introduced into pSF_Sm_M7-35/V184A. Double substitution obtained by introducing N442D into pSF_Sm_V184P was also constructed. The mutation was introduced by the same method as in (45) using pSF_Sm_M35-4/V184A or pSF_Sm_V184P as the template and using the primers corresponding to various mutant enzymes. The resulting strains were cultured by the method described in Example 6 (25).

### (57) Production of peptide using microbial cells <Ala-X>

The cultured broth (20 µL) obtained in (56) was added to 400 µL of borate buffer (pH 8.5) containing 50 mM alanine methyl ester hydrochloride (Ala-OMe HCl), 100 mM L-amino acid and 10 mM EDTA, and reacted at 20°C for 15 minutes. The concentrations (mM) of various dipeptides (Ala-X) synthesized in this reaction with the wild strain are shown in Table 19. For the dipeptides synthesized by various mutant strains, the ratio of the concentration of the dipeptide synthesized thereby with respect to that by the wild strain is shown in Table 19.

### Table 19

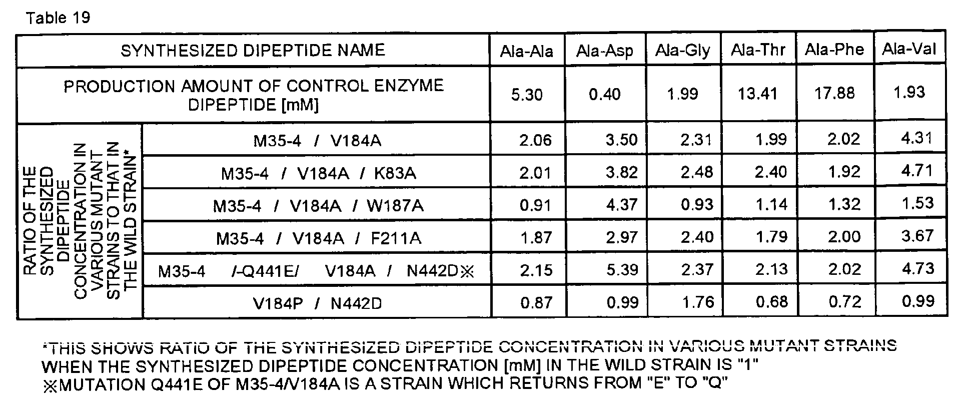

### (Example 15) Random screening

### (58) Preparation of pTrpT_Sm_Aet random library

In order to construct mutant Aet, pTrpT_Sm_Aet or pSF_Sm_M35-4/V184A plasmid was used as the template for random mutagenesis using error prone PCR. The library in which the mutation had been introduced was made by the same method as in Example 3 (8).

### (59) Screening of pSFN_Sm_Aet random library

Selection was performed by performing two screenings (A/B or A/C) selected from the primary screenings (A) to (C) shown below using the cultured solution obtained by culturing the library made in (58) by the same method as in Example 3 (9).

### (60) Primary screening (A)

A reaction solution (pH 8.2) (200 µL) containing 10 mM phenol, 6 mM AP, 5 mM Asp(OMe)₂, 5 mM Ala-OEt, 7.5 mM Phe, 3.6 U/mL of peroxidase, 0.16 U/mL of alcohol oxidase, 10 mM EDTA and 100 mM borate was added to 5 µL of the resulting microbial solution, and reacted at 25°C for about 20 minutes. Subsequently, absorbance at 500 nm was measured to calculate the released amount of methanol. Those in which methanol had been abundantly released were selected as the enzyme which tend to produce AMP rather than Ala-Phe.

### (61) Primary screening (B)

In the same manner as in (60), the reaction solution (pH 8.2) (200 µL) containing 10 mM phenol, 6 mM AP, 5 mM Asp(OMe)₂, 5 mM A(M), 3.6 U/mL of peroxidase, 0.16 U/mL of alcohol oxidase, 10 mM EDTA and 100 mM berate was added to 5 µL of the resulting microbial solution, and reacted at 25°C for about 20 minutes. Subsequently, absorbance at 500 nm was measured to calculate the released amount of methanol. Those in which the amount of released methanol had been low were selected as the enzyme which has less tendency to produce AM(AM).

### (62) Primary screening (C)

In the same manner as in (60), the reaction solution (pH 8.2) (200 µL) containing 10 mM phenol, 6 mM AP, 5 mM Asp(OMe)₂, 3.6 U/mL of peroxidase, 0.16 U/mL of alcohol oxidase, 10 mM EDTA and 100 mM borate was added to 5 µL of the resulting microbial solution, and reacted at 25°C for about 20 minutes. Subsequently, absorbance at 500 nm was measured to calculate a released amount of methanol. Those in which the amount of released methanol had been low were selected as the enzyme which has less tendency to decompose Asp(OMe)₂.

### (63) Secondary screening

The strains selected in (60), (61) and (62) were cultured by the same method as in Example 6 (25). 50 µL of each cultured broth was suspended in 1 mL of 100 mM borate buffer (pH 8.5) containing 10 mM EDTA, 50 mM Asp(OMe)₂, 50 mM Ala-OMe and 75 mM Phe. The mixture was reacted at 20°C for 10 minutes, and the amounts of produced AMP and Ala-Phe were measured. The strain which had exhibited a fast initial reaction rate was selected.

The cultured broth obtained in the same way as the above was also suspended (2.2 U/mL reaction solution) in 100 mM borate buffer (pH 9.0) containing 10 mM EDTA, 50 mM Asp(OMe)₂ and 75 mM Phe. The mixture was reacted at 20°C, and the yield of produced AMP was measured. The mutation point was analyzed in the strains which exhibited the high yield, and the following mutation points were specified. The mutant strains having the mutations 21, 22 and 23 (P214T, Q202E and Y494F) were obtained from the library using pTrpT_Sm_Aet as the template. The mutant strains having the mutations 354, 346, 347, 350, 351, 352, 343, 354, 348, 349 and 353 (combining each mutation of A182G, K314R, A515V, K484I, V213A, A245S, V178G, L263M, L66F, S315R and P214H with M35-4/V184A) were obtained from the library using pSF_Sm_M35-4/V184A as the template. The yields of AMP in this reaction 20, 40 and 70 minutes after the onset of the reaction in each mutant strain are shown in Tables 20-1 and 20-2. M35-4/V184A may be referred to hereinbelow as "A1".

### Table 20-1

**Table 20-1**

| | AMP YIELD [%] | | |
|---|---|---|---|
| | 20 min | 40 min | 70 min |
| A1 | 60.8 | 71.6 | 69.8 |
| A1/A182G | 56.3 | 72.7 | 69.9 |
| A1/K314R | 61.2 | 73.3 | 68.5 |
| A1/A515V | 60.7 | 74.7 | 69.7 |
| A1/K484l | 61.0 | 75.1 | 71.1 |
| A1N213A | 59.1 | 74.3 | 69.3 |
| A1/A245S | 61.6 | 73.3 | 69.5 |
| A1N178G | 63.6 | 74.6 | 72.7 |
| A1/L263M | 59.9 | 72.3 | 71.1 |

### Table 20-2

**Table 20-2**

| | AMP YIELD [%] | | |
|---|---|---|---|
| | 20 min | 40 min | 60 min |
| WILD STRAIN | 49.9 | 55.6 | 54.9 |
| P214T | 49.6 | 59.0 | 61.0 |
| Q202E | 54.6 | 60.2 | 57.7 |
| Y494F | 55.2 | 62.2 | 63.2 |

### (Example 16) Construction of rational mutant strains

### (64) Introduction of mutation into A182, P183 and T185

Since the yield was enhanced in the strain carrying the V184A mutation, the strains carrying the mutation at around position 184 were constructed. The mutation was introduced by the same method as in (45) using pSF_Sm_M35-4/V184A as the template and using the primers (SEQ ID NOS:171 to 192) corresponding to various mutant enzymes.

### (65) Production of peptides using microbial cells <AMP>

The strains obtained in Example 15 (63) and the aforementioned (64) were cultured by the method described in Example 6 (25). The cultured broth was suspended (10U/mL reaction solution) in 100 mM borate buffer (pH 8.5) containing 400 mM Asp(OMe)₂ hydrochloride and 600 mM Phe, and reacted at 25°C with keeping pH 8.5 using NaOH. The yields of produced AMP was measured 20, 40 and 80 minutes after the onset of the reaction. The AMP yields in this reaction are shown in Table 21.

### Table 21

**Table 21**

| | AMP YIELD [%] | | |
|---|---|---|---|
| | 40 min | 60 min | 80 min |
| A1 | 47.7 | 47.5 | 48.7 |
| A1/V178G | 48.9 | 48.4 | |
| A1/K484I | 47.8 | 49.3 | |
| A1/A515V | 49.6 | 49.1 | |
| A1/V213A | 50.8 | 50.7 | |
| A1/A245S | 49.3 | 49.1 | |
| A1/K314R | 49.2 | 48.1 | |
| A1/A182G | 51.5 | 51.3 | |
| A1/P183A | 51.8 | 52.6 | 51.9 |
| A1/T185A | 50.8 | 53.3 | 51.8 |
| A1/T185N | 49.3 | 50.2 | 50.1 |
| A1/P183A/A182G | 53.4 | 56.1 | 54.8 |
| A1/P183A/A182S | 54.1 | 54.8 | 56.0 |

### (66) Production of peptides using microbial cells <Ala-X>

The strains obtained in Example 15 (63) and the aforementioned (64) were cultured by the method described in Example 6 (25). The cultured broth (20 µL) was added to 400 µL of borate buffer (pH 8.5) containing 50 mM Ala-OMe•HCl, 100 mM L-amino acid and 10 mM EDTA, and reacted at 20°C for 15 minutes. The concentrations (mM) of various dipeptides (Ala-X) synthesized in this reaction with pSF_Sm_M35-4/V184A are shown in Table 22. For the dipeptides synthesized by various mutant strains, the ratio of the concentration of the dipeptide synthesized thereby with respect to that by pSF_Sm_M35-4/V184A is shown in Table 22.

### Table 22

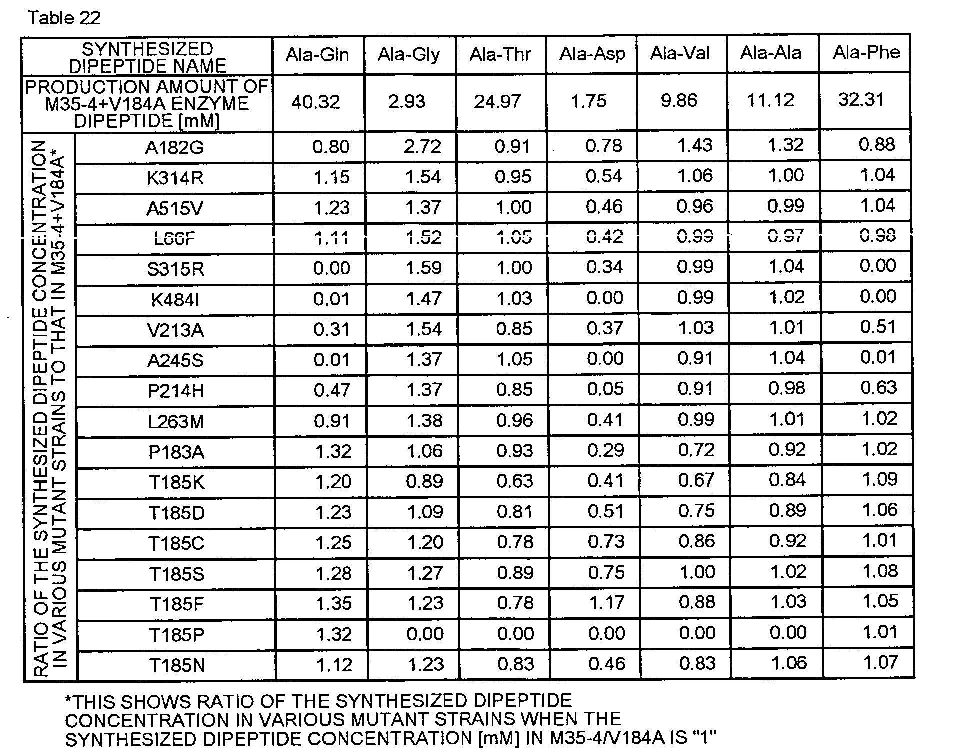

### (Example 17) Construction of strains having high activity by combining mutations: B

### (67) Construction of combined mutant strain

The mutation points T185F and A182G which had exhibited the effect when combined with M35-4/V184A (A1) were introduced into pSF_Sm_M35-4/V184A, pSF_Sm_M7-35/V184A and pSF_Sm_M35-4/V184A/N442D. The mutation was introduced by the same method as in (45) using the primers (SEQ ID NOS:185, 186, 193, 194, 199, 200) corresponding to various mutant enzymes. The resulting strains were cultured by the method described in Example 6 (25).

### (68) Production of peptides using microbial cells <Ala-X>

The cultured broth (20 µL) obtained in (67) was added to 400 µL of borate buffer (pH 8.5) containing 50 mM Ala-OMe HCl, 100 mM L-amino acid and 10 mM EDTA, and reacted at 20°C for 15 minutes. The concentrations (mM) of the dipeptides (Ala-X) synthesized in this reaction with the wild strain are shown in Table 23. For the dipeptides synthesized by various mutant strains, the ratio of the concentration of the dipeptide synthesized thereby with respect to that by the wild strain is shown in Table 23.

### Table 23

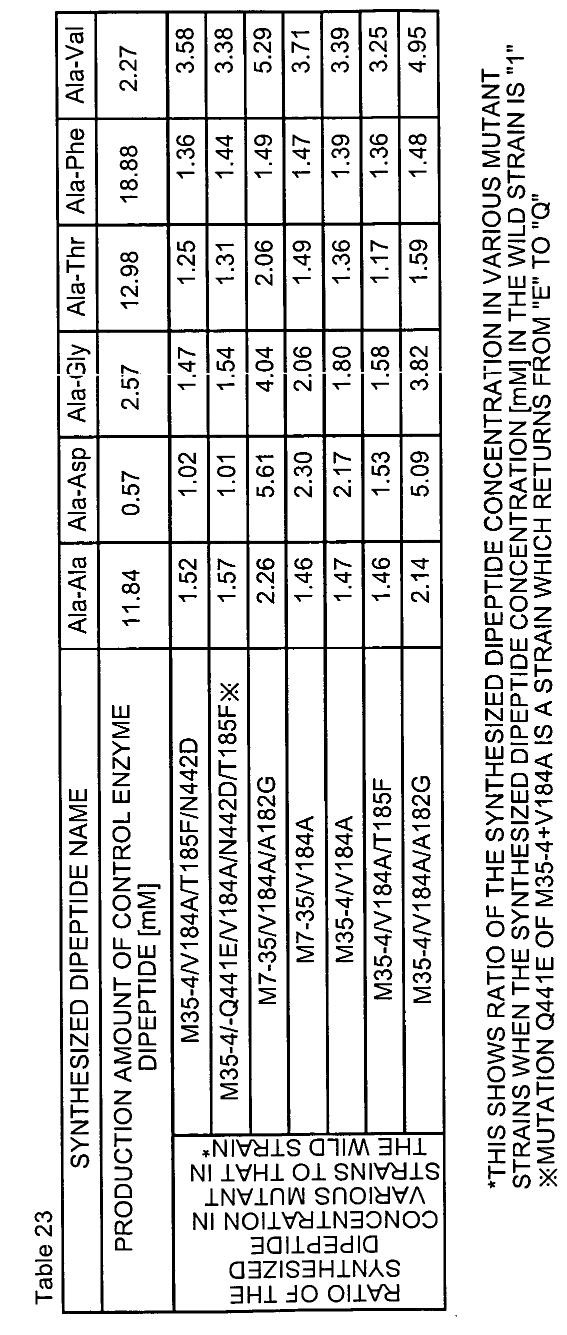

### (69) Production of peptides with increased amount of substrate <Ala-X>

pSF_Sm_Aet, pSF_Sm_M35-4/V184A and pSF_Sm_M7-35/V184A/A182G were cultured by the method shown in Example 6 (25). The cultured broth (5 µL or 20 µL) was added to 400 µL of borate buffer (pH 8.5) containing 50 mM Ala-OMe HCl, 100 mM to 400 mM L-amino acid and 10 mM EDTA, and reacted at 20°C for one hour. The concentrations (mM) of the dipeptides (Ala-X) synthesized in this reaction are shown in Table 24.

### Table 24

**Table 24**

| Concentration N [mM] | Strain | Dipeptide [Mm] | | | | |
|---|---|---|---|---|---|---|
| | | Ala-Ala | Ala-Asp | Ala-Gly | Ala-Thr | Ala-Val |
| 100 | control | 14.1 | 0.8 | 4.8 | 16.5 | 5.5 |
| | M35-4 / V184A | 14.8 | 1.3 | 6.2 | 18.8 | 7.5 |
| | M7-35 / V184A / A182G | 23.1 | 3.3 | 15.9 | 25.4 | 12.9 |
| 200 | control | 21.7 | 1.1 | 7.5 | 24.0 | 7.9 |
| | M35-4 / V184A | 22.6 | 1.7 | 11.0 | 25.7 | 10.9 |
| | M7-35 / V184A / A182G | 34.0 | 5.4 | 23.2 | 31.3 | 18.5 |
| 400 | control | 30.2 | 2.6 | 14.5 | 33.6 | 8.4 |
| | M35-4 / V184A | 33.5 | 4.0 | 18.5 | 33.2 | 17.2 |
| | M7-35/V184A/A182G | 47.2 | 11.2 | 33.1 | 36.7 | 25.7 |

### (Example 18) Study of substrate specificity

### (70) Production of various dipeptides using mutant enzymes

The production of the peptide with various L-amino acid methyl esters as the carboxy component and L-amino acid as the amine component was examined. The cultured broth (20 µL or 40 µL) cultured by the method described in Example 6 (25) was added to 400 µL of borate buffer (pH 8.5 or 9.0) containing 50 mM L-amino acid methyl ester hydrochloride (X-OMe HCl), 100 mM L-amino acid shown in Table 25 and 10 mM EDTA, and reacted at 20°C. The amounts of various dipeptides produced in this reaction are shown in Table 25. As the enzymes, those derived from pSF_Sm_Aet, pSF_Sm_M12-1 (Example 7 (32)) and pSF_Sm_M35-4/V184A (Example 10 (39)) were used. In the synthesis reaction of Val-Met and Met-Met, enzymes derived from pSF_Sm_F207V (Example 6 (24)) and pSF_Sm_M35-4/V184A/F20TV were also used.

### Table 25

**Table 25**

| C (X-OMe) | N (x) | Yield [%] | | | |
|---|---|---|---|---|---|
| | | control | M12-1 | M35-4/V184A | Others |
| Gly | Met | 66.1 | 61.7 | 66.5 | |
| Ala | Met | 60.0 | | | |
| Val | Met | 52.7 | 61.7 | 76.2 | 81.6^{*1} |
| Leu | Met | 80.4 | | | |
| Ile | Gln | 46.8 | 58.6 | 64.5 | |
| Pro | Met | 4.8 | 17.4 | 13.5 | |
| Ser | Met | 73.1 | 83.1 | 85.4 | |
| Thr | Met | 63.9 | 65.1 | 71.0 | |
| Cys | Gly | 17.8 | 25.1 | 23.7 | |
| Met | Met | 25.1 | 36.7 | 36.4 | 48.2 ^{*2} |
| Asp^{*3} | Phe | | 60.0 | 70.0 | |
| Asn | Glu | 14.5 | 23.9 | 12.6 | |
| Lys | Met | 6.6 | 36.6 | 44.0 | |
| Arg | Met | 3.3 | 39.2 | 58.9 | |
| His | Met | 3.6 | 32.7 | 38.6 | |
| Phe | Met | 22.4 | 38.8 | 59.2 | |
| Tyr | Gln | 17.0 | 48.5 | 53.9 | |
| Trp | Met | 0.9 | 40.6 | 47.1 | |

| | | | | | |
|---|---|---|---|---|---|
| ^{*1} ; F207V ^{*2} ; M35-4/V184A/F207V ^{*3} ; Asp(OMe)₂ | | | | | |

### (Example 19) Production of Arg-Gln

### (71) Production of peptides using microbial cells <Arg-Gln>

pSF_Sm_Aet and pSF_Sm_M35-4/V184A were cultured in the method described in Example 6 (25). The cultured broth (1 mL) was suspended in 9 mL of 100 mM borate buffer (pH 9.0) containing 10 mM EDTA, 100 or 200 mM arginine methyl ester and 150 to 300 mL Gln, and reacted at 20°C for 3 hours. As the reaction proceeds, a pH value was lowered. Thus, the reaction was performed with keeping pH to 9.0 using a 25% NaOH solution. The concentrations and the yields of Arg-Gln produced in this reaction are shown in Table 26.

### Table 26

**Table 26**

| ArgOMe [mM] | Gln [mM] | pH | strain | broth vol. | Arg-Gln [mM] | Yield [%] |
|---|---|---|---|---|---|---|
| 100 | 150 | 9.0 | control | 10% | 1.3 | 1.3 |
| | | 9.0 | M35-4+V184A | 10% | 80.5 | 80.1 |
| 200 | 200 | 9.0 | M35-4+V184A | 10% | 127.3 | 61.9 |
| | 300 | 9.0 | M35-4+V184A | 10% | 144.0 | 70.8 |

| | | | | | | |
|---|---|---|---|---|---|---|
| Reaction time ; 180 min | | | | | | |

### (Example 20) Production of peptides using purified enzyme

### (72) Purification of enzymes

The wild strain, the pSF_Sm_M35-4/V184A strain and the pSF_Sm_M7-35/V184A/A182G strain were refreshed on LB plates. One platinum loopful thereof was inoculated to 50 mL of terrific broth, and cultured at 25°C for 18 hours. Microbial cells were collected from the cultured solution, suspended in 100 mM KPB (pH 6.5) and disrupted by a sonicator (180 W/30 minutes). The solution was collected and the supernatant was collected as a soluble fraction by ultracentrifugation at 200,000 g at 4°C for 20 minutes. The following manipulations were performed at 4°C or on ice unless otherwise particularly specified. AKTA explorer 100 was used for the following column fractionation.

The resulting soluble fraction was subjected to CHT5-1 (5 mL, 10 x 64 mm) which had previously been equilibrated with 100 mM KPB (pH 6.5). Unabsorbed proteins were eluted with 100 mM KPB buffer at a flow rate of 1 mL/minute, and subsequently the absorbed protein was eluted with 25 times volume of the column volume of 100 to 500 mM KPB buffer having a linear gradient. The active fraction separated by hydroxyapatite chromatography was subjected to preparation so that the final ammonium sulfate concentration became 2 M, and then subjected to Hic-resource-Phe (1 mL) which had previously been equilibrated with 100 mM KPB (pH 6.5) and 2 M ammonium sulfate. The unabsorbed proteins were eluted at a flow rate of 1 mL/minute, and subsequently the absorbed protein was eluted with KPB buffer (60 times volume of the column volume) containing 2M to 0M ammonium sulfate in a linear gradient.

The fraction separated by hydrophobic chromatography was subjected to HiLoad 16/60 Superdex-200 pg (column volume: 120 mL, 16 mm x 600 mm) which had previously been equilibrated with 20 mM Hepes (pH 6.5) and 500 mM NaCl. The protein was eluted at a flow rate of 0.75 mL/minute to collect the active fraction. The active fraction was concentrated, and then dialyzed against 20 mM Hepes (pH 6.5). The "unit" shown below indicates the unit in Ala-Gln synthesis reaction.

### (73) Production of peptides using purified enzyme <HIL-Phe>

The purified enzyme (0.84 or 4.2 U, 1 or 5 µL) obtained from pSF_Sm_M35-4/V184A was added to 150 µL of borate buffer (pH 9.0) containing 50 mM lactonized HIL [{2S, 3R, 4S)-hydroxyisoleucine], 100 mM Phe and 10 mM EDTA, and reacted at 20°C for one hour. The concentrations of HIL-Phe synthesized in this reaction are shown in Table 27.

### Table 27

**Table 27**

| U/system | Reac. time [min] | HIL-Phe Conc. [mM] |
|---|---|---|
| 4.20 | 15 | 0.21 |
| | 120 | 1.77 |
| 0.84 | 15 | 0.02 |
| | 120 | 0.33 |

### (74) Production of peptides using purified enzyme <Gly-Ser(tBu)>

The purified enzyme (0.84 or 4.2 U, 1 or 5 µL) obtained from pSF_Sm_M35-4/V184A was added to 150 µL of borate buffer (pH 8.5) containing 50 mM Gly-OMe, 100 mM Ser(tBu) and 10 mM EDTA, and reacted at 20°C. The concentrations of Gly-Ser(tBu) synthesized in this reaction calculated in terms of Gly-Ser are shown in Table 28.

### Table 28

**Table 28**

| U/system | Reac. time [min] | Gly-Ser(tBu) Conc. [mM] |
|---|---|---|
| 0.84 | 15 | 7.6 |
| | 60 | 21.4 |
| | 120 | 28.2 |
| 4.2 | 15 | 24.7 |
| | 60 | 28.9 |
| | 120 | 27.8 |

| | | |
|---|---|---|
| *Gly-Ser conversion | | |

### (75) Production of tripeptides using purified enzymes <Ala-X-X>

The purified enzyme (0.84 or 4.2 U, 1 or 5 µL) obtained from pSF_Sm_M35-4/V184A or pSF_Sm_M7-35/V184A/A182G was added to 150 µL of borate buffer (pH 9.0) containing 50 mM Ala-OMe, 100 X-X and 10 mM EDTA, and reacted at 20°C. The concentrations of tripeptides (Ala-X-X) synthesized in this reaction are shown in Table 29.

### Table 29

**Table 29**

| Enzyme vol. (U/system) | Enzyme | Production amount of tripeptide [mM] | | | | | |
|---|---|---|---|---|---|---|---|
| | | M35-4/V184A | | | M7-35/V184A/A182G | | |
| | | 5 min | 15 min | 60 min | 5 min | 15 min | 60 min |
| 0.84 | AFA | 22.7 | 29.8 | 27.2 | 10.4 | 23.2 | 31.3 |
| | AGA | 1.1 | 10.7 | 19.4 | 13.9 | 27.3 | 29.7 |
| | AHA | 12.0 | 27.5 | 30.7 | 15.8 | | 13.6 |
| | ALA | 20.4 | 26.9 | 23.3 | 14.6 | 26.3 | 25.7 |
| | AAA | 13.2 | 21.9 | 25.3 | 14.7 | 25.6 | 29.2 |
| | AAG | 7.8 | 13.8 | 17.0 | 10.3 | 17.5 | 17.0 |
| | AAP | 3.2 | 5.3 | 6.5 | 4.9 | 7.3 | 8.1 |
| | AAQ | 3.7 | 5.0 | 7.2 | 4.1 | 7.1 | 8.9 |
| | AAY | 2.0 | 6.6 | 11.4 | 5.6 | 10.0 | 17.3 |
| 4.2 | AFA | 29.4 | 30.1 | 25.1 | 31.7 | 30.9 | 20.6 |
| | AGA | 21.5 | 21.2 | 20.5 | 30.0 | 30.2 | 28.7 |
| | AHA | 33.5 | 27.9 | 23.7 | 15.3 | 13.5 | 12.3 |
| | ALA | 27.0 | 25.3 | 22.7 | 27.6 | 24.6 | 19.0 |
| | AAA | 25.6 | 26.4 | 26.1 | 25.6 | 26.4 | 26.1 |
| | AAG | 18.3 | 17.8 | 17.7 | 18.3 | 17.8 | 17.7 |
| | AAP | 6.6 | 6.7 | 7.5 | 6.6 | 6.7 | 7.5 |
| | AAQ | 6.8 | 7.4 | 7.8 | 6.8 | 7.4 | 7.8 |
| | AAY | 8.5 | 13.6 | 14.4 | 8.5 | 13.6 | 14.4 |

### (76) Production of tripeptides using purified enzyme

The purified enzyme (0.84 or 4.2 U, 1 or 5 µL) obtained from pSF_Sm_M35-4/V184A was added to 150 µL of borate buffer (pH 9.0) containing 50 mM Ala-OMe, 50 mM X-X and 10 mM EDTA, and reacted at 20°C. The concentrations of the tripeptides synthesized in this reaction are shown in Table 30.

### Table 30

**Table 30**

| Enzyme vol. (U/system) | Reaction time [min] | Synthesized tripeptide [mM] | | | | |
|---|---|---|---|---|---|---|
| | | AFA | GFA | AGG | TGG | GGG |
| 0.84 | 15 | 31.0 | 5.9 | 19.8 | 13.8 | 3.8 |
| | 60 | 25.2 | 13.6 | 17.7 | 30.5 | 9.9 |
| | 120 | 22.5 | 16.0 | 20.0 | 33.9 | 12.5 |

| | | | | | | |
|---|---|---|---|---|---|---|
| **Substrate 50mM XOMe + 50mM XX** | | | | | | |

### (77) Production of peptides using purified enzyme <Ala-X-X>

The purified enzyme (0.84 or 4.2 U, 1 or 5 µL) obtained from pSF_Sm_M35-4/V184A was added to 150 µL of borate buffer (pH 9.0) containing 100 mM Ala-OMe, 100 mM X-X and 10 mM EDTA, and reacted at 20°C. The concentrations of the tripeptides (Ala-X-X) synthesized in this reaction are shown in Table 31.

### Table 31

**Table 31**

| Enzyme vol. (U/system) | Reaction time [min] | Synthesized tripeptide [mM] | |
|---|---|---|---|
| | | AFG | AGG |
| 0.84U | 15 | 29.4 | 6.0 |
| | 30 | 39.0 | 15.1 |
| | 60 | 40.1 | 24.3 |
| 4.2U | 15 | 40.6 | 29.3 |
| | 30 | 38.5 | 35.1 |
| | 60 | 34.0 | 35.7 |

| | | | |
|---|---|---|---|
| **Substrate 100mM AlaOMe + 100mM XX** | | | |

### (78) Production of tetrapeptide using purified enzyme <GGFM>

The purified enzyme (4.2 U, 5 µL) obtained from pSF_Sm_M35-4/V184A was added to 150 µL of borate buffer (pH 9.0) containing 100 mM Gly-OMe, 40 mM GFM and 10 mM EDTA, and reacted at 20°C. The concentrations of the tetrapeptide (GGFM) synthesized in this reaction are shown in Table 32.

### Table 32

**Table 32**

| Reaction Time [min] | GGFM [mM] |
|---|---|
| 5 | 6.0 |
| 15 | 12.3 |
| 30 | 16.0 |
| 60 | 17.1 |

### (79) Production of pentapeptide using purified enzyme <Met-Enkephalin>

The purified enzyme (4.2 U, 5 µL) obtained from pSF_Sm_M35-4/V184A was added to 150 µL of borate buffer (pH 8.5) containing 50 mM Tyr-OMe, 5 mM GGFM and 10 mM EDTA, and reacted at 20°C. The concentrations of the pentapeptide (YGGFM) synthesized in this reaction are shown in Table 33.

### Table 33

**Table 33**

| Reaction time [min] | YGGFM [mM] | |
|---|---|---|
| | 4.2U | 8.4U |
| 5 | 0.5 | 1.0 |
| 15 | 1.1 | 1.7 |
| 30 | 1.6 | 2.1 |
| 60 | 2.0 | 2.3 |
| 120 | 2.2 | 2.4 |

### (Example 21) X-ray crystal structure analysis

### (1) 1 L of Escherichia coli (E. coli) JM109 strain in which the protein having the amino acid sequence of SEQ ID NO:209 was expressed at high level was cultured, and the protein was purified from microbial cells by the following procedure.

### (1-1) Hydroxyapatite chromatography

The microbial cells obtained in the above were disrupted in "100 mM potassium phosphate buffer (pH 6.5)" (buffer A), and 100 mL of the soluble fraction was subjected to a hydroxyapatite column Bio-Scale CHT-I (supplied from Bio-Rad, CV=5 mL) which had been equilibrated with the buffer A, to absorb to the carrier. The absorbed protein was eluted by linearly changing the concentration of potassium phosphate buffer from 100 mM to 500 mM (25CV). A peak of the protein was detected by absorbance at 280 nm, and the fraction was collected.

### (1-2) Hydrophobic chromatography

The fraction fractionated in (1-1) was mixed with the 5 time volume of "100 mM potassium phosphate buffer (pH 6.5) containing 2M ammonium sulfate" (buffer B). This solution was subjected to a hydrophobic chromatographic column RESOURCE PHE (supplied from Amersham, CV=1 mL) which had been equilibrated with the buffer B. The objective protein was absorbed to the carrier by this manipulation. Subsequently, the protein was eluted by a linear gradient from 2M to 0 M of ammonium sulfate (60CV), and the fraction was fractionated.

### (1-3) Cation exchange chromatography: Resource S

The fraction fractionated in (1-2) was dialyzed against "20 mM sodium acetate buffer (pH 5.0)" (buffer C) overnight. This solution was subjected to a cation exchange column RESOURCE S (supplied from Amersham, CV=1 mL) which had been equilibrated with the buffer C. The absorbed protein was eluted by linearly changing the concentration of sodium chloride from 0 mM to 500 mM (50CV). The peak of the protein was detected by absorbance at 280nm, and the fraction was fractionated.

The fractions in respective purification stages were confirmed by SDS-PAGE. As a result, the purified protein obtained after (1-3) was detected as an almost single band at a position of about 70 kDa by CBBR staining. The solution the protein thus obtained was dialyzed against 20 mM HEPES buffer (pH 7.0) at 4°C overnight. About 30 mg of the purified protein was obtained by the aforementioned manipulations.

### (2) Crystallization of protein having amino acid sequence of SEQ ID NO:209

The purified protein solution obtained in (1) was concentrated to about 40 mg/mL at 4°C using an ultrafiltrator AmiconUltra (supplied from Millipore, fractioning molecular weight: 10 kDa). Using the obtained concentrated protein solution, crystallization conditions were searched by changing various parameters such as a protein concentration, a precipitating agent, pH, temperature and additives. As a result, hexagonal-cylindrical crystals were obtained which had grown to the 0.2 mm x 0.2 mm x 0.2 mm crystal in about one week by the hanging drop vapor diffusion method in which a droplet which is a mixture of 1 µL of the protein solution and 1 µL of the precipitating agent containing 0.2% octyl β D-glucopyranoside is equilibrated at 20°C in the precipitating agent having the composition of 12 to 18% PEG 6000 and 0.1 M Tris-HCl (pH 8.0).

### (3) X-ray crystal structure analysis of protein having amino acid sequence of SEQ ID NO:209

X-ray diffraction intensity was measured at low temperature because the protein crystal is deteriorated in the measurement by X-ray damage at ambient temperature and the resolution thereby gradually decreases. The crystal was transferred into the solution containing 20% glycerol, 20% PEG 6000, 0.1 M Tris-HCl (pH 8.0) and 0.4% octyl β D-glucopyranoside. Then nitrogen gas at -173°C was sprayed thereto for rapid cooling. X-ray diffraction data of the crystal were obtained using a CCD detector of 315 type supplied from ADSC, placed in the beam line 5 in Photon Factory in Inter-University Research Institute Corporation, High Energy Accelerator Research Organization (Tsukuba-shi). The wavelength of the X-ray was set up to 1.0 angstrom, and a distance from the crystal to the CCD detector was 450 mm. Image data per one frame was taken with exposure for 20 seconds and an oscillation angle of 1.0°. The data for 150 frames were collected. Crystallographic parameters were as follows: a space group was P6₅22, and lattice constants were a=104.324 angstroms and c=615.931 angstroms. Given that two protein molecules are contained in an asymmetric unit, a water content rate of the crystal is 65%. The crystal was diffracted to about 3.0 angstroms. The data were processed using the program HKL 2000 (Methods Enzymol., 276:307-326, 1997). The values of R_{merge} which is the indicator of data quality were 0.106 at the resolution of 50.0 to 3.0 angstroms and 0.450 at the outmost shell at the resolution of 3.11 to 3.00 angstroms. Completeness of the data were 97.2% at the resolution of 50.0 to 3.0 angstroms and 81.1% at the outmost shell at the resolution of 3.11 to 3.00 angstroms.

The structure was analyzed by a molecular replacement method. The program for the molecular replacement AMORE (Acta Crystallogr., Sect. A, 50:157-163, 1994) included in program package CCP4 for protein structure analysis (Acta Crystallogr., Sect. D, 50:760-763, 1994) was used. As a reference structure, the S205A mutant of α-amino acid ester hydrolase (entry number of Protein Data Bank: 1NX9) was utilized. The α-amino acid ester hydrolase has a tetramer structure whereas the protein having the amino acid sequence of SEQ ID NO:209 has a dimer structure. When a monomer structure of the α-amino acid ester hydrolase was used as a model, no promising solution was obtained. It is possible to cut out 3 types of the dimer structures from the α-amino acid ester hydrolase tetramer. Thus, the molecular replacement was attempted using these three types of dimers. As a result, when the dimer composed of A molecule and D molecule in 1NX9 coordinate data was used as the model, the promising solution was found from several standpoints (good contrast in the first solution, clear difference in space groups, no bad contact between the molecules). The electron density map at the resolution of 3.0 angstroms was calculated based on the resulting initial phase, and the electron density map was depicted on a computer graphic program QUANTA supplied from Accelrys. The structural analysis was carried forward by repeating modification of the molecular model on the graphics and by refinement using the program CNX supplied from Accelrys.

### (4) Crystallization of protein having the amino acid sequence of SEQ ID NO:209 in which Lys residues were reductively dimethylated

It has been reported that the crystal quality is sometimes improved when the Lys residue of the protein is reductively dimethylated (Biochemistry 32:9851-9858, 1993). In accordance with this method, the Lys residues of the purified protein solution obtained in the above were reductively dimethylated using hydrogenated sodium boron and formaldehyde, and subsequently this protein was subjected to the crystallization experiment. As a result, platy crystals were obtained which had grown to the 0.4 mm x 0.2 mm x 0.1 mm crystal in about one week by the hanging drop vapor diffusion method in which a droplet which is a mixture of 1 µL of the protein solution and 1 µL of the precipitating agent containing 0.2% octyl β D-glucopyranoside is equilibrated in the precipitating agent having the composition of 15% PEG 6000 and 0.1 M Tris-HCl (pH 8.0) .

### (5) X-ray crystal structure analysis of protein having the amino acid sequence of SEQ ID NO:209 in which Lys residues were reductively dimethylated

The crystal was transferred into the solution containing 20% glycerol, 20% PEG 6000, 0.1 M Tris-HCl (pH 8.0) and 0.4% octyl β D-glucopyranoside. Then nitrogen gas at -173°C was sprayed thereto for rapid cooling. X-ray diffraction data of the crystal were obtained using R-AXIS V type imaging plate detector supplied from Rigaku and placed in beam line 24XU in Synchrotron Orbit Radiation Facility, SPring 8 in Japan Synchrotron Radiation Research Institute (Hyogo Prefecture, Sayo-gun). The wavelength of the X-ray was set up to 0.827 angstrom, and the distance from the crystal to the imaging plate detector was 500 mm. Image data per one frame was taken with exposure for 90 seconds and an oscillation angle of 1.0°. The data for 180 frames were collected. Crystallographic parameters were as follows: the space group was P2₁, and lattice constants were a=74.476 angstroms, b=213.892 angstroms and c=90.427 angstroms. Given that four protein molecules are contained in the asymmetric unit, the water content rate of the crystal is 53%. The crystal was diffracted to about 3.0 angstroms. The data were processed using the program CrystalClear supplied from Rigaku. The values of R_{merge} which is the indicator of data quality were 0.097 at a resolution of 40.0 to 3.0 angstroms and 0.309 at the outermost shell at a resolution of 3.11 to 3.00 angstroms. Completeness of the data were 96.8% at a resolution of 40.0 to 3.0 angstroms and 95.8% at the outmost shell at a resolution of 3.11 to 3.00 angstroms.

The structure was analyzed by the molecular replacement method. The program for the molecular replacement AMORE (Acta Crystallogr., Sect. A, 50:157-163, 1994) included in program package CCP4 for protein structure analysis (Acta Crystallogr., Sect. D, 50:760-763, 1994) was used. As a reference structure, the S205A mutant of α-amino acid ester hydrolase (entry number of Protein Data Bank: 1NX9) was utilized. When the monomer structure of the α-amino acid ester hydrolase was used as the model, no promising solution was obtained. Thus, the molecular replacement was attempted using three types of dimers cut out from the α-amino acid ester hydrolase tetramer. As a result, when the dimer composed of A molecule and D molecule in 1NX9 coordinate data was used as the model as with the above, the solution was found. This result indicates success of the molecular replacement method as well as the dimer structure of the protein having the amino acid sequence of SEQ ID NO:209. The electron density map at the resolution of 3.0 angstroms was calculated based on the resulting initial phase, and the electron density map was depicted on the computer graphic program QUANTA supplied from Accelrys. The structural analysis was carried forward by repeating modification of the molecular model on the graphics and by refinement using the program CNX supplied from Accelrys. Atomic coordinates of the present crystal structure were are in FIGS. 4 and 5. In FIG. 4, the residues at positions 79 to 82 were represented by dark gray and the other residues were represented by light gray. In FIG. 5, α-L-aspartyl-L-phenylalanine-β-methylester (i.e., α-L-(β-O-methyl aspartyl)-L-phenylalanine (abbreviated as α-AMP) was represented as "AMP" (gray represented by ball-and-stick), and catalytic triad was represented as the "active site" (CPK representation).

### (Example 22) Preparation of rational mutant strains using tertiary structure information

Modified proteins were made by introducing rational mutation concerning 134 residues which are close to the active site (colored in black) in the amino acid sequence of SEQ ID NO:208, in accordance with the following Example 22.

### (1) Rational mutation method based on tertiary structure information

In order to increase the production amount of AMP, the site-directed mutation was introduced into the amino acid sequence of SEQ ID NO:208 (referred to hereinbelow as pAl) based on the tertiary structure information. The protein having the amino acid sequence of SEQ ID NO:209 has high homology with the protein having the amino acid sequence of SEQ ID NO:208, i.e., only four substitutions are given. Thus, the tertiary structure information of mutant peptide-synthesizing enzymes expressed by pA1 (represented as A1) was predicted from the protein having the amino acid sequence of SEQ ID NO:209, and 134 amino acid residues (colored in black in FIG. 5) at positions 67 to 70, 72 to 88, 100, 102, 103, 106, 107, 113 to 117, 130, 155 to 163, 165, 166, 180 to 188, 190 to 195, 200 to 235, 259, 273, 276, 278 292 to 291, 296, 298, 299, 300 to 304, 325 to 328, 330 to 340, and 437 to 447 located within 15 angstroms from Ser158 of the catalytic triad which was the active center were selected as possible residues contributing to the synthesis of AMP. Thus, the site-directed mutation was introduced into these positions. Types of substituted amino acids in these positions are shown in Tables 34-1 and 34-2.

### Table 34-1

**Table 34-1**

| RESIDUE No. | MUTATED RESIDUE | | | | | | | | | | | | | | | | | | | |
|---|---|---|---|---|---|---|---|---|---|---|---|---|---|---|---|---|---|---|---|---|
| | A | C | D | E | F | G | H | I | K | L | M | N | P | Q | R | S | T | V | W | Y |
| N67 | A | | D | | F | | | | K | L | | | | | | S | T | | | |
| R68 | A | | D | | F | | H | | | L | | | | | | S | | | | |
| T69 | A | | D | | F | G | H | I | K | L | M | N | P | Q | R | S | | V | | |
| P70 | A | | D | | F | G | | I | K | L | | N | | Q | | S | T | V | | |
| A72 | A | C | D | E | | G | | I | K | L | M | N | | Q | | S | | V | | |
| V73 | A | | D | E | F | G | | I | K | L | M | N | P | Q | | S | T | | W | |
| S74 | A | | D | | F | G | | | K | | | N | P | | | | T | V | | |
| P75 | A | | D | | F | G | | | | L | | | | | | S | T | V | W | |
| Y76 | A | | D | | F | G | H | I | | L | M | N | P | Q | R | S | T | V | W | |
| G77 | A | | D | | F | | H | I | K | L | M | N | P | Q | | S | T | V | W | |
| Q78 | A | | | | F | | | | | L | | N | | | | | | | | |
| N79 | A | | D | | F | | | | | L | | | | | R | S | | | | |
| E80 | A | | D | | F | G | | | K | L | | N | P | Q | | S | T | | W | Y |
| Y81 | A | C | D | E | F | G | H | I | K | L | | N | P | Q | | S | T | V | W | |
| K82 | A | | D | | | | | | | L | | | P | | | S | | | | |
| K83 | A | | D | | F | | | | | L | | | P | | | S | | V | | |
| S84 | A | | D | E | F | | H | | K | L | M | N | | Q | R | | T | | | |
| L85 | A | | D | | F | G | H | I | K | | M | | P | | | S | T | V | W | Y |
| G86 | A | | D | | | | | | K | L | | N | | Q | | S | | | | |
| N87 | A | | D | E | F | G | H | I | K | L | M | | P | Q | | S | T | V | W | Y |
| F88 | A | | D | E | | | H | I | K | L | M | N | P | Q | | | T | V | W | Y |
| Y100 | A | | D | | F | | H | | K | L | | | | Q | | S | | | W | |
| D102 | A | | | E | | | | | | L | | N | | | | | | | | |
| V103 | A | | D | | F | | | I | | L | | | | | | | | | W | Y |
| K106 | A | | D | | F | | H | | | L | M | N | P | Q | R | S | | V | W | Y |
| W107 | A | | D | | F | | | | K | | | | | | | S | | | | Y |
| F113 | A | | | | | | H | | | L | | N | P | Q | R | S | T | V | W | Y |
| E114 | A | | D | | | | | | | | | | | | | | | V | | |
| D115 | A | | | E | F | G | | I | K | L | M | | P | Q | | S | T | V | W | Y |
| I116 | A | | D | | F | G | | | K | L | M | N | P | | | S | T | V | | Y |
| R117 | A | | | | | | | | | | | | | | | | | | | |
| E130 | A | | | | | | | | | | | | | | | | | | | |
| Y155 | A | | | | F | | H | I | | | | | | | | | T | | W | |
| G156 | A | | D | | F | | | | | L | | | | | | S | | | | |
| I157 | A | | D | E | F | | H | | K | L | M | N | P | Q | | S | T | V | W | Y |
| S158 | | C | | | | | | | | | | | | | | | | | | |
| Y159 | A | | D | | F | G | H | I | K | L | M | N | P | Q | | S | T | V | W | |
| P160 | A | | D | E | F | G | | | K | L | | N | | Q | | S | T | V | | |
| G161 | A | | D | | F | | | I | | L | M | N | P | Q | | S | T | V | | |
| F162 | A | | D | | | G | H | I | | L | M | N | | Q | R | S | T | V | W | Y |
| Y163 | A | | D | | F | | | I | K | L | M | | P | Q | | | T | V | W | |
| T165 | A | | | | | | | I | | L | | | | | | | | V | | |
| V166 | A | | | | F | | | | | L | | | | | | | | | | |
| P180 | A | | | | | | | | | | | | | | | | | | | |
| Q181 | A | | D | E | F | | H | I | K | L | M | N | | | | S | T | V | W | Y |
| A182 | | | | | | G | | I | | L | M | | | | | S | T | V | | |
| P183 | A | | | | | G | | I | | L | | | | Q | | S | T | V | | |
| T185 | A | | | | | G | | I | | L | | | | | | S | | V | | |
| D186 | A | | | | | G | H | I | | L | M | | | Q | | | T | V | | |
| W187 | A | | D | | F | G | H | I | K | L | M | | P | | | S | | V | | Y |
| Y188 | | | | | F | | | | | L | | | | | | | | | W | |
| G190 | A | | D | | F | | | | K | L | | | P | | | S | | | | |
| D191 | A | | | E | F | | | | K | L | | N | | Q | | S | T | V | | |
| D192 | A | | | E | F | G | | | K | L | | N | | Q | | S | T | V | | |
| F193 | | | D | | | | H | I | K | L | M | | | | | S | | V | W | Y |
| H194 | A | | D | | F | | | | K | L | | | | | | S | | | | |
| H195 | A | | D | | F | | | | K | L | | N | | | | | | | W | Y |
| F200 | A | | D | | | G | H | I | | L | M | N | P | | R | S | T | V | W | Y |
| L201 | A | | D | | F | | | I | K | | | N | P | Q | | S | T | V | | Y |
| Q202 | A | | D | E | F | G | | | | L | M | N | | | R | S | T | V | W | |
| D203 | A | C | | E | | G | | | K | L | M | N | P | Q | | S | T | V | | Y |
| A204 | | | D | | F | G | | I | K | L | M | N | P | | | S | T | V | | |
| F205 | A | | D | | | | | I | K | L | M | N | P | Q | | S | T | V | W | |
| T206 | A | | D | | F | | | | K | L | | | | | | S | | | | Y |
| F207 | A | | D | | | G | H | I | K | L | M | N | P | Q | R | S | | V | W | Y |
| M208 | A | | D | | F | G | | I | K | L | | | P | Q | R | S | T | V | W | Y |

### Table 34-2

**Table 34-2**

| RESIDUE No. | MUTATED RESIDUE | | | | | | | | | | | | | | | | | | | |
|---|---|---|---|---|---|---|---|---|---|---|---|---|---|---|---|---|---|---|---|---|
| | A | C | D | E | F | G | H | I | K | L | M | N | P | Q | R | S | T | V | W | Y |
| S209 | A | | D | | F | G | | | K | L | | N | P | Q | | S | T | V | | |
| T210 | A | | D | | F | G | | I | K | L | M | | P | Q | | S | | V | W | Y |
| F211 | A | | D | | | | H | I | K | L | M | N | | Q | | S | T | V | W | Y |
| G212 | A | | D | | F | | | | K | L | | | | | | S | T | | | |
| V213 | A | | D | | F | | | | K | | | | | | | S | | V | | |
| P214 | A | | D | | F | | | | K | L | | | | | | S | | | | |
| R215 | A | | D | | F | | H | I | K | L | | N | | Q | | S | T | V | W | Y |
| P216 | A | | D | | F | | | | K | L | | | | | | S | | | | |
| K217 | A | | D | | | | | | | L | | | | | | | | | | |
| P218 | A | | D | | F | | | | K | L | | | | Q | | S | | | | |
| I219 | | | D | | F | | | | K | | | | | | | S | | | | |
| T220 | A | | D | | F | | | | K | L | | | | | | S | | | | |
| P221 | A | | D | | F | | | | K | L | | | | | | S | | | | |
| D222 | A | | | | F | | | | | L | | | | | R | | | | | |
| Q223 | | | | | F | G | | | K | L | | | | | | S | | | | |
| F224 | A | | D | | | G | | | K | L | | | | | | S | | | | |
| K225 | A | | D | | F | G | | | | L | M | | | | R | S | | | | |
| G226 | A | | D | | F | | | | K | L | | N | | | | S | | | | |
| K227 | A | | D | | F | G | | | | L | | | | | | S | | | | |
| I228 | A | | D | | F | | H | | K | L | | | | | R | S | | | | |
| P229 | A | | D | | F | | | | K | L | | | | | | S | | | | |
| I230 | A | | D | | F | | | | K | | | | | | | S | | | | |
| K231 | A | | D | | F | | | | | L | | | | Q | | S | | | | |
| E232 | A | | D | | F | G | | | K | L | | | | | | S | | | | |
| A233 | | | D | E | F | G | H | | K | L | | N | | Q | | S | | V | | |
| D234 | A | | | E | F | | | | K | L | | N | | | | S | | | | |
| K235 | A | | D | | F | | | | | L | | | | | | S | | | | |
| F259 | A | | D | | | | H | I | K | L | M | | p | | | S | | V | W | Y |
| W273 | A | | | | F | | | | | L | | | | | | | | | | |
| R276 | A | | D | | F | G | H | I | K | L | M | N | | Q | | S | T | V | W | Y |
| 1278 | A | | | | F | | | | | L | | | | | | | | V | | |
| V292 | A | | D | E | F | | | I | K | L | | N | | | | S | | V | | |
| G293 | A | | D | | F | | | | K | L | | | | Q | | S | | | | |
| G294 | A | | D | | F | | | | K | L | | | | | | | | | | |
| F296 | A | | | | | | | | | L | | | | | | | | V | W | |
| A298 | | | | | F | G | | I | | L | M | N | P | Q | | S | T | V | | |
| E299 | A | | D | | | | | | | | M | N | | Q | | | | | | |
| D300 | A | | | E | | | | | | L | | N | | | | S | T | V | | |
| V301 | | | D | | F | G | | | | L | M | | | | | | | | | |
| Y302 | A | | | | F | | | | | | | | | | | | | | W | |
| G303 | A | | | | | | | | | | | | | | | | | | | |
| T304 | A | | D | | F | | | | | L | | | | | | | | | | |
| G325 | A | | | | | | | | | | | | | | | | | | | |
| P326 | A | | | | | G | | | | | | | | | | | | | | |
| W327 | A | | | E | F | | | | | L | | | | | R | | | | W | Y |
| Y328 | A | | | | F | | H | | K | L | M | | P | | R | | | V | W | |
| G330 | A | | D | | F | | | I | | L | | | P | | | S | T | V | | |
| G331 | A | | D | | | | | | K | L | | N | P | Q | | S | | V | | |
| W332 | | | | | F | | H | I | | L | M | | P | | R | | | V | W | Y |
| V333 | A | | D | | F | G | H | I | K | | M | N | P | | | | T | | | |
| R334 | A | | D | | F | | H | I | K | L | M | | | Q | | | | V | | Y |
| A335 | | | D | | F | G | | I | K | L | M | N | P | Q | | S | T | V | W | |
| E336 | A | | D | | F | | | I | K | L | M | | | Q | | | | V | | |
| G337 | A | | | | | | | | | | | | P | | | S | | | | |
| N338 | A | | D | | F | | | | K | | | | | | | S | | | | |
| Y339 | A | | D | | | | | | K | L | | | | | | S | T | | W | |
| L340 | A | | | | F | | | I | | | | | | | | S | T | V | | |
| G437 | A | | | | | | | | | | | | | | | | | | | |
| G438 | A | | | | | | | | | | | | | | | | | | | |
| V439 | A | | D | | F | | | I | K | | | | P | | | S | | | | |
| 1440 | A | | D | | F | | | | K | L | | | | | | S | | V | | |
| E441 | A | | D | | F | | | | | L | M | N | | | | | | V | | |
| N442 | A | | D | | F | | | | | L | | | | | | S | | | W | |
| R443 | A | | D | | F | G | H | | K | L | M | N | P | Q | | S | T | V | | |
| T444 | A | | D | | F | | | I | K | L | M | N | | | | S | | V | W | Y |
| R445 | A | C | D | E | F | G | H | I | K | L | M | N | P | Q | | S | T | V | W | Y |
| E446 | A | | D | | F | | | | K | L | | | P | Q | | S | T | | | |
| Y447 | | | D | | F | | H | | K | L | | | P | | | | | | W | |

### (2) Preparation of single mutation strains

In order to obtain the mutant A1, pA1 was used as the template of the site-directed mutagenesis using PCR. The mutation was introduced using "QuikChange Site-Directed Mutagenesis Kit" supplied from Stratagene (USA) in accordance with the manufacturer's protocol. The primer of 33mer comprising a mutation codon at a center and 15mers sandwiching the mutation codon was used for the introduction of the site-directed mutagenesis in each residue. The primers used for each mutation point are shown in Table 46. The nucleotide sequences which configure the primers in Table 46 are also shown in Sequence Listing. SEQ ID NOS:210 to 483 correspond to primers in Table 46 in the order of the forward primer and the reverse primer in the direction from upper to lower rows in the table. The codon corresponding to each amino acid to be substituted is placed as the mutation codon "xxx" in the center of each primer sequence ("nnn" part in nucleotide sequences of SEQ ID NOS:210 to 483). That is, depending on the type of amino acid residue to be introduced, each primer includes the corresponding codon sequence introduced into "xxx" part. Each codon corresponding to the amino acid residue is as shown in Table 44. *Escherichia coli* JM109 was transformed with the PCR product, and the strain having the objective plasmid was selected using ampicillin resistance as the indicator.

### (3) Obtaining microbial cells

One platinum loopful of each mutant strain was inoculated into a usual test tube in which 2 mL of terrific medium (12 g/L of tryptone, 24 g/L of yeast extract, 2.3 g/L of potassium dihydrogen phosphate, 12.5 g/L of dipotassium hydrogen phosphate, 4 g/L of glycerol and 100 mg/L of ampicillin) had been placed, and main cultivation was performed at 25°C at 150 reciprocations/minute for 18 hours.

### (4) Measurement of specific activity in each mutant strain

The broth (50 µL) of each mutant strain was added to 1 mL of a low concentration reaction solution (50 mM dimethyl aspartate, 75 mM phenylalanine), and reacted at 20°C at initial pH of 8.5. The amount of produced AMP 15 minutes after the start of the reaction was quantified by HPLC, and the specific activity (U/mL) in each single mutation strain was calculated. For the unit (U) of the enzyme, the amount of the enzyme which can produce 1 µmol of the product AMP in one minute was defined as 1 U.

### (5) Measurement of AMP yield in each single mutation strain in low concentration reaction solution

Based on the resulting specific activity data, the amount of the broth necessary for obtaining 2 U was calculated as to each mutant strain. Subsequently, the calculated amount of the broth was added to 1 mL of the low concentration reaction solution, and reacted at a temperature of 20°C at initial pH of 8.5. The amounts of produced AMP 25 and 45 minutes after the start of the reaction were quantified by HPLC, and the mutant strains listed on Tables 32-1 to 35-7 exhibited higher yield than A1. These were found out to be the important mutant strains which contribute to the reaction of AMP synthesis.

### Table 35-1

**Table 35-1**

| MUTATION ID | MUTATION | YIELD [%] |
|---|---|---|
| MUTATION L1 | N67K | 54.9 |
| MUTATION L2 | N67L | 54.1 |
| MUTATION L3 | N67S | 55.1 |
| MUTATION L4 | T69I | 55.3 |
| MUTATION L5 | T69M | 54.6 |
| MUTATION L6 | T69Q | 58.2 |
| MUTATION L7 | T69R | 56.0 |
| MUTATION L8 | T69V | 54.6 |
| MUTATION L9 | P70G | 54.6 |
| MUTATION L10 | P70N | 59.9 |
| MUTATION L11 | P70S | 59.5 |
| MUTATION L12 | P70T | 59.5 |
| MUTATION L13 | P70V | 57.5 |
| MUTATION L14 | A72C | 59.4 |
| MUTATION L15 | A72D | 58.6 |
| MUTATION L16 | A72E | 61.8 |
| MUTATION L17 | A721 | 56.3 |
| MUTATION L18 | A72L | 55.6 |
| MUTATION L19 | A72M | 57.3 |
| MUTATION L20 | A72N | 60.8 |
| MUTATION L21 | A72Q | 55.1 |
| MUTATION L22 | A72S | 58.4 |
| MUTATION L23 | A72V | 55.1 |
| MUTATION L24 | V73A | 54.4 |
| MUTATION L25 | V731 | 57.0 |
| MUTATION L26 | V73L | 58.4 |
| MUTATION L27 | V73M | 57.9 |
| MUTATION L28 | V73N | 57.6 |
| MUTATION L29 | V73S | 56.1 |
| MUTATION L30 | V73T | 57.7 |
| MUTATION L31 | S74A | 58.4 |
| MUTATION L32 | S74F | 58.5 |
| MUTATION L33 | S74K | 54.0 |
| MUTATION L34 | S74N | 58.6 |
| MUTATION L35 | S74T | 59.6 |
| MUTATION L36 | S74V | 56.8 |
| MUTATION L37 | P75A | 59.4 |
| MUTATION L38 | P75D | 54.8 |
| MUTATION L39 | P75L | 55.1 |
| MUTATION L40 | P75S | 54.6 |
| MUTATION L41 | Y76F | 54.9 |
| MUTATION L42 | Y76H | 56.5 |
| MUTATION L43 | Y761 | 55.9 |
| MUTATION L44 | Y76V | 58.5 |
| MUTATION L45 | Y76W | 54.3 |
| MUTATION L46 | G77A | 59.7 |
| MUTATION L47 | G77F | 56.4 |
| MUTATION L48 | G77K | 57.2 |
| MUTATION L49 | G77M | 54.5 |
| MUTATION L50 | G77N | 59.1 |
| MUTATION L51 | G77P | 55.2 |
| MUTATION L52 | G77S | 57.8 |
| MUTATION L53 | G77T | 55.4 |

### Table 35-2

**Table 35-2**

| MUTATION ID | MUTATION YIELD [%] | |
|---|---|---|
| MUTATION L54 | Q78F | 54.5 |
| MUTATION L55 | Q78L | 58.0 |
| MUTATION L56 | N79D | 55.8 |
| MUTATION L57 | N79L | 54.4 |
| MUTATION L58 | N79R | 56.0 |
| MUTATION L59 | N79S | 55.7 |
| MUTATION L60 | E80D | 56.1 |
| MUTATION L61 | E80F | 56.9 |
| MUTATION L62 | E80L | 59.7 |
| MUTATION L63 | E80P | 57.9 |
| MUTATION L64 | E80S | 57.5 |
| MUTATION L65 | Y81A | 58.7 |
| MUTATION L66 | Y81C | 57.2 |
| MUTATION L67 | Y81 D | 57.3 |
| MUTATION L68 | Y81 E | 59.9 |
| MUTATION L69 | Y81 F | 57.9 |
| MUTATION L70 | Y81 H | 59.7 |
| MUTATION L71 | Y81 K | 60.8 |
| MUTATION L72 | Y81 L | 56.2 |
| MUTATION L73 | Y81 N | 59.0 |
| MUTATION L74 | Y81S | 56.7 |
| MUTATION L75 | Y81T | 56.1 |
| MUTATION L76 | Y81W | 57.7 |
| MUTATION L77 | K82D | 55.2 |
| MUTATION L78 | K82L | 57.5 |
| MUTATION L79 | K82P | 56.6 |
| MUTATION L80 | K82S | 54.3 |
| MUTATION L81 | K83D | 55.8 |
| MUTATION L82 | K83F | 58.0 |
| MUTATION L83 | K83L | 56.4 |
| MUTATION L84 | K83P | 59.8 |
| MUTATION L85 | K83S | 56.7 |
| MUTATION L86 | K83V | 54.8 |
| MUTATION L87 | S84D | 56.7 |
| MUTATION L88 | S84F | 56.4 |
| MUTATION L89 | S84K | 56.6 |
| MUTATION L90 | S84L | 54.3 |
| MUTATION L91 | S84N | 55.5 |
| MUTATION L92 | S84Q | 56.2 |
| MUTATION L93 | L85F | 60.1 |
| MUTATION L94 | L851 | 59.5 |
| MUTATION L95 | L85P | 57.6 |
| MUTATION L96 | L85V | 59.2 |
| MUTATION L97 | N87E | 58.7 |
| MUTATION L98 | N87Q | 58.5 |
| MUTATION L99 | F88E | 62.7 |
| MUTATION L100 | V103I | 57.3 |
| MUTATION L101 | V103L | 56.7 |
| MUTATION L102 | K106A | 57.7 |
| MUTATION L103 | K106F | 59.3 |
| MUTATION L104 | K106L | 57.3 |
| MUTATION L105 | K106Q | 59.1 |
| MUTATION L106 | K106S | 58.9 |
| MUTATION L107 | W107A | 57.3 |

### Table 35-3

**Table 35-3**

| MUTATION ID | MUTATION YIELD [%] | |
|---|---|---|
| MUTATION L108 | W107Y | 55.3 |
| MUTATION L109 | F113A | 55.4 |
| MUTATION L110 | F113W | 58.0 |
| MUTATION L111 | F113Y | 57.6 |
| MUTATION L112 | E114A | 57.6 |
| MUTATION L113 | E114D | 58.8 |
| MUTATION L114 | D115E | 54.2 |
| MUTATION L115 | D115Q | 55.0 |
| MUTATION L116 | D115S | 54.6 |
| MUTATION L117 | I116F | 57.0 |
| MUTATION L118 | I116K | 56.1 |
| MUTATION L119 | I116L | 58.3 |
| MUTATION L120 | I116M | 57.1 |
| MUTATION L121 | I116N | 56.1 |
| MUTATION L122 | I116T | 54.8 |
| MUTATION L123 | I116V | 54.8 |
| MUTATION L124 | I157K | 60.1 |
| MUTATION L125 | I157L | 63.3 |
| MUTATION L126 | Y159G | 55.6 |
| MUTATION L127 | Y159N | 58.5 |
| MUTATION L128 | Y159S | 56.4 |
| MUTATION L129 | P160G | 58.3 |
| MUTATION L130 | G161A | 58.9 |
| MUTATION L131 | F162L | 58.7 |
| MUTATION L132 | F162Y | 63.0 |
| MUTATION L133 | Y163I | 56.1 |
| MUTATION L134 | T165V | 54.6 |
| MUTATION L135 | Q181F | 57.2 |
| MUTATION L136 | A182G | 61.4 |
| MUTATION L137 | A182S | 55.6 |
| MUTATION L138 | P183A | 55.3 |
| MUTATION L139 | P183G | 54.1 |
| MUTATION L140 | P183S | 54.9 |
| MUTATION L141 | T185A | 57.4 |
| MUTATION L142 | T185G | 54.7 |
| MUTATION L143 | T185V | 55.0 |
| MUTATION L144 | W187A | 54.3 |
| MUTATION L145 | W187F | 57.3 |
| MUTATION L146 | W187H | 55.3 |
| MUTATION L147 | W187Y | 61.9 |
| MUTATION L148 | Y188F | 54.5 |
| MUTATION L149 | Y188L | 57.9 |
| MUTATION L150 | Y188W | 54.2 |
| MUTATION L151 | G190A | 57.7 |
| MUTATION L152 | G190D | 55.8 |
| MUTATION L153 | F193W | 56.7 |
| MUTATION L154 | H194D | 55.0 |
| MUTATION L155 | F200A | 57.4 |
| MUTATION L156 | F200L | 57.6 |
| MUTATION L157 | F200S | 55.3 |
| MUTATION L158 | F200V | 57.3 |
| MUTATION L159 | L201Q | 54.3 |
| MUTATION L160 | L201S | 59.6 |
| MUTATION L161 | Q202A | 57.1 |

### Table 35-4

**Table 35-4**

| MUTATION ID | MUTATION YIELD [%] | |
|---|---|---|
| MUTATION L162 | Q202D | 62.8 |
| MUTATION L163 | Q202F | 55.9 |
| MUTATION L164 | Q202S | 55.1 |
| MUTATION L165 | Q202T | 55.0 |
| MUTATION L166 | Q202V | 56.1 |
| MUTATION L167 | D203E | 55.7 |
| MUTATION L168 | A204G | 62.2 |
| MUTATION L169 | A204L | 55.2 |
| MUTATION L170 | A204S | 58.0 |
| MUTATION L171 | A204T | 55.7 |
| MUTATION L172 | A204V | 57.2 |
| MUTATION L173 | F205L | 59.1 |
| MUTATION L174 | F205Q | 55.6 |
| MUTATION L175 | F205V | 54.7 |
| MUTATION L176 | F205W | 64.6 |
| MUTATION L177 | T206F | 57.9 |
| MUTATION L178 | T206K | 54.3 |
| MUTATION L179 | T206L | 60.3 |
| MUTATION L180 | F2071 | 55.9 |
| MUTATION L181 | F207W | 58.8 |
| MUTATION L182 | F207Y | 57.5 |
| MUTATION L183 | M208A | 57.4 |
| MUTATION L184 | M208L | 58.9 |
| MUTATION L185 | S209F | 61.7 |
| MUTATION L186 | S209K | 60.5 |
| MUTATION L187 | S209L | 59.9 |
| MUTATION L188 | S209N | 60.3 |
| MUTATION L189 | S209V | 60.1 |
| MUTATION L190 | T210A | 56.6 |
| MUTATION L191 | T210L | 59.3 |
| MUTATION L192 | T210Q | 55.1 |
| MUTATION L193 | T210V | 54.5 |
| MUTATION L194 | F211A | 59.3 |
| MUTATION L195 | F211I | 60.6 |
| MUTATION L196 | F211L | 56.3 |
| MUTATION L197 | F21 1 M | 54.3 |
| MUTATION L198 | F211V | 57.8 |
| MUTATION L199 | F211W | 58.3 |
| MUTATION L200 | F211Y | 57.8 |
| MUTATION L201 | G212A | 56.8 |
| MUTATION L202 | V213D | 54.9 |
| MUTATION L203 | V213F | 56.0 |
| MUTATION L204 | V213K | 56.1 |
| MUTATION L205 | V213S | 57.3 |
| MUTATION L206 | P214D | 54.0 |
| MUTATION L207 | P214F | 56.3 |
| MUTATION L208 | P214K | 54.9 |
| MUTATION L209 | P214S | 54.1 |
| MUTATION L210 | R215A | 55.6 |
| MUTATION L211 | R2151 | 57.4 |
| MUTATION L212 | R215K | 56.9 |
| MUTATION L213 | R215Q | 55.4 |
| MUTATION L214 | R215S | 55.6 |
| MUTATION L215 | R215T | 56.9 |

### Table 35-5

**Table 35-5**

| MUTATION ID | MUTATION YIELD [%] | |
|---|---|---|
| MUTATION L216 | R215Y | 57.4 |
| MUTATION L217 | P216D | 54.7 |
| MUTATION L218 | P216K | 55.6 |
| MUTATION L219 | K217D | 55.3 |
| MUTATION L220 | P218F | 55.5 |
| MUTATION L221 | P218L | 54.1 |
| MUTATION L222 | P218Q | 54.9 |
| MUTATION L223 | P218S | 54.6 |
| MUTATION L224 | I219D | 57.1 |
| MUTATION L225 | I219F | 54.4 |
| MUTATION L226 | I219K | 55.8 |
| MUTATION L227 | T220A | 54.6 |
| MUTATION L228 | T220D | 54.6 |
| MUTATION L229 | T220F | 55.3 |
| MUTATION L230 | T220K | 55.8 |
| MUTATION L231 | T220L | 54.6 |
| MUTATION L232 | T220S | 54.6 |
| MUTATION L233 | P221A | 57.8 |
| MUTATION L234 | P221 D | 56.7 |
| MUTATION L235 | P221F F | 54.8 |
| MUTATION L236 | P221K | 58.0 |
| MUTATION L237 | P221 L | 55.2 |
| MUTATION L238 | P221S | 56.5 |
| MUTATION L239 | D222A | 54.7 |
| MUTATION L240 | D222F | 56.5 |
| MUTATION L241 | D222L | 58.1 |
| MUTATION L242 | D222R | 54.0 |
| MUTATION L243 | Q223F | 54.7 |
| MUTATION L244 | Q223K | 54.8 |
| MUTATION L245 | Q223L | 55.2 |
| MUTATION L246 | Q223S | 57.9 |
| MUTATION L247 | F224A | 55.9 |
| MUTATION L248 | F224D | 55.7 |
| MUTATION L249 | F224G | 54.2 |
| MUTATION L250 | F224K | 55.2 |
| MUTATION L251 | F224L | 54.8 |
| MUTATION L252 | K225D | 54.8 |
| MUTATION L253 | K225G | 54.4 |
| MUTATION L254 | K225S | 55.4 |
| MUTATION L255 | G226A | 56.6 |
| MUTATION L256 | G226F | 55.2 |
| MUTATION L257 | G226L | 55.7 |
| MUTATION L258 | G226N | 55.6 |
| MUTATION L259 | G226S | 54.5 |
| MUTATION L260 | K227D | 55.1 |
| MUTATION L261 | K227F F | 57.6 |
| MUTATION L262 | K227S | 61.3 |
| MUTATION L263 | I228A | 54.5 |
| MUTATION L264 | I228F | 59.3 |
| MUTATION L265 | I228K | 58.2 |
| MUTATION L266 | I228S | 54.3 |
| MUTATION L267 | P229A | 54.6 |
| MUTATION L268 | P229D | 57.0 |
| MUTATION L269 | P229K | 54.8 |

### Table 35-6

**Table 35-6**

| MUTATION ID | MUTATION | YIELD [%] |
|---|---|---|
| MUTATION L270 | P229L | 60.6 |
| MUTATION L271 | P229S | 54.1 |
| MUTATION L272 | I230A | 56.9 |
| MUTATION L273 | I230F | 58.2 |
| MUTATION L274 | I230K | 55.3 |
| MUTATION L275 | I230S | 57.8 |
| MUTATION L276 | K231 F | 56.2 |
| MUTATION L277 | K231 L | 60.4 |
| MUTATION L278 | K231 S | 56.3 |
| MUTATION L279 | E232D | 59.0 |
| MUTATION L280 | E232F | 56.5 |
| MUTATION L281 | E232G | 57.5 |
| MUTATION L282 | E232L | 55.6 |
| MUTATION L283 | E232S | 55.0 |
| MUTATION L284 | A233D | 56.4 |
| MUTATION L285 | A233F | 54.1 |
| MUTATION L286 | A233H | 56.8 |
| MUTATION L287 | A233K | 55.4 |
| MUTATION L288 | A233L | 55.6 |
| MUTATION L289 | A233N | 54.9 |
| MUTATION L290 | A233S | 55.4 |
| MUTATION L291 | D234L | 56.3 |
| MUTATION L292 | D234S | 55.4 |
| MUTATION L293 | K235D | 54.9 |
| MUTATION L294 | K235F | 55.4 |
| MUTATION L295 | K235L | 56.0 |
| MUTATION L296 | K235S | 55.4 |
| MUTATION L297 | F259Y | 55.3 |
| MUTATION L298 | R276A | 57.4 |
| MUTATION L299 | R276Q | 56.2 |
| MUTATION L300 | A298S | 59.0 |
| MUTATION L301 | D300N | 54.5 |
| MUTATION L302 | V301 M | 56.6 |
| MUTATION L303 | Y328F | 62.4 |
| MUTATION L304 | Y328H | 56.8 |
| MUTATION L305 | Y328M | 55.0 |
| MUTATION L306 | Y328W | 59.3 |
| MUTATION L307 | W332H | 57.6 |
| MUTATION L308 | E336A | 56.5 |
| MUTATION L309 | N338A | 54.0 |
| MUTATION L310 | N338F | 56.4 |
| MUTATION L311 | Y339K | 54.7 |
| MUTATION L312 | Y339L | 57.1 |
| MUTATION L313 | Y339T | 55.0 |
| MUTATION L314 | L340A | 54.7 |
| MUTATION L315 | L340I | 54.4 |
| MUTATION L316 | L340V | 55.4 |
| MUTATION L317 | V439P | 56.2 |
| MUTATION L318 | I440F | 56.3 |
| MUTATION L319 | I440V | 56.3 |
| MUTATION L320 | E441 F | 54.1 |
| MUTATION L321 | E441 M | 57.2 |
| MUTATION L322 | E441 N | 55.1 |
| MUTATION L323 | N442A | 57.3 |

### Table 35-7

**Table 35-7**

| MUTATION ID | MUTATION | YIELD [%] |
|---|---|---|
| MUTATION L324 | N442L | 56.6 |
| MUTATION L325 | R443S | 55.2 |
| MUTATION L326 | T444W | 55.3 |
| MUTATION L327 | R445G | 54.2 |
| MUTATION L328 | R445K | 55.9 |
| MUTATION L329 | E446A | 54.3 |
| MUTATION L330 | E446F | 55.3 |
| MUTATION L331 | E446Q | 55.1 |
| MUTATION L332 | E446S | 55.8 |
| MUTATION L333 | E446T | 55.2 |
| MUTATION L334 | Y447L | 54.9 |
| MUTATION L335 | Y447S | 54.1 |

### (6) Calculation of yield enhancement probability

Among 1137 single mutation mutants, 335 mutants were found to be the mutants exhibiting improved yield when compared with A1. The yield enhancement probability was 335x1137=0.29. Meanwhile, the results of calculating the yield enhancement probability for each residue are summarized in Tables 36 and 37. The values of yield enhancement probability were largely different depending on the residues. For example, probability of yield increase by mutation at each of 47 positions was 40% or more, at each of 59 positions was 30% or more, and at each of 71 positions was 20% or more. The position which brings about the yield enhancement probability of 20% or more can enhance the yield with very high probability and may be determined to be an industrially very important mutation point.

### Table 36-1

**Table 36-1**

| RESIDUE No. | Ratio of mutations resulted in 54% or more improvement in yield |
|---|---|
| N67 | 42.9 |
| R68 | 0.0 |
| T69 | 33.3 |
| P70 | 41.7 |
| A72 | 76.9 |
| V73 | 46.7 |
| S74 | 66.7 |
| P75 | 44.4 |
| Y76 | 31.3 |
| G77 | 53.3 |
| Q78 | 50.0 |
| N79 | 66.7 |
| E80 | 38.5 |
| Y81 | 70.6 |
| K82 | 80.0 |
| K83 | 85.7 |
| S84 | 46.2 |
| L85 | 28.6 |
| G86 | 0.0 |
| N87 | 11.8 |
| F88 | 6.7 |
| Y100 | 0.0 |
| D102 | 0.0 |
| V103 | 28.6 |
| K106 | 35.7 |
| W107 | 33.3 |
| F113 | 25.0 |
| E114 | 66.7 |
| D115 | 20.0 |
| I116 | 53.8 |
| R117 | 0.0 |
| E130 | 0.0 |
| Y155 | 0.0 |
| G156 | 0.0 |
| I157 | 12.5 |
| S158 | 0.0 |
| Y159 | 18.8 |
| P160 | 8.3 |
| G161 | 8.3 |
| F162 | 13.3 |
| Y163 | 8.3 |
| T165 | 25.0 |
| V 166 | 0.0 |
| P180 | 0.0 |
| Q 181 | 6.7 |
| A182 | 28.6 |
| P183 | 37.5 |
| T185 | 50.0 |
| D 186 | 0.0 |
| W187 | 30.8 |
| Y188 | 100.0 |
| G190 | 28.6 |
| D191 | 0.0 |
| D192 | 0.0 |
| F193 | 10.0 |
| H194 | 16.7 |
| H195 | 0.0 |
| F200 | 26.7 |
| L201 | 16.7 |
| Q202 | 46.2 |
| D203 | 7.1 |
| A204 | 41.7 |
| F205 | 30.8 |
| T206 | 42.9 |
| F207 | 18.8 |

### Table 36-2

**Table 36-2**

| RESIDUE No. RESIDUE No. | Ratio of mutations resulted in 54% or more improvement in yield |
|---|---|
| M208 | 13.3 |
| S209 | 41.7 |
| T210 | 28.6 |
| F211 | 50.0 |
| G212 | 14.3 |
| V213 | 66.7 |
| P214 | 66.7 |
| R215 | 50.0 |
| P216 | 33.3 |
| K217 | 33.3 |
| P218 | 57.1 |
| I219 | 75.0 |
| T220 | 100.0 |
| P221 | 100.0 |
| D222 | 100.0 |
| Q223 | 80.0 |
| F224 | 83.3 |
| K225 | 37.5 |
| G226 | 71.4 |
| K227 | 50.0 |
| I228 | 50.0 |
| P229 | 83.3 |
| I230 | 80.0 |
| K231 | 50.0 |
| E232 | 71.4 |
| A233 | 63.6 |
| D234 | 28.6 |
| K235 | 80.0 |
| F259 | 8.3 |
| W273 | 0.0 |
| R276 | 12.5 |
| I278 | 0.0 |
| V292 | 0.0 |
| G293 | 0.0 |
| G294 | 0.0 |
| F296 | 0.0 |
| A298 | 9.1 |
| E299 | 0.0 |
| D300 | 14.3 |
| V301 | 20.0 |
| Y302 | 0.0 |
| G303 | 0.0 |
| T304 | 0.0 |
| G325 | 0.0 |
| P326 | 0.0 |
| W327 | 0.0 |
| Y328 | 40.0 |
| G330 | 0.0 |
| G331 | 0.0 |
| W332 | 10.0 |
| V333 | 0.0 |
| R334 | 0.0 |
| A335 | 0.0 |
| E336 | 11.1 |
| G337 | 0.0 |
| N338 | 40.0 |
| Y339 | 42.9 |
| L340 | 50.0 |
| G437 | 0.0 |
| G438 | 0.0 |
| V439 | 14.3 |
| I440 | 28.6 |
| E441 | 42.9 |
| N442 | 33.3 |
| R443 | 7.1 |
| T444 | 8.3 |
| R445 | 10.5 |
| E446 | 55.6 |
| Y447 | 12.5 |

### Table 37-1

**Table 37-1**

| Position at which 20% or more of mutations resulted in 54% or more improvement in yield | Position at which 30% or more of mutations resulted in 54% or more improvement in yield | Position at which 40% or more of mutations resulted in 54% or more improvement in yield |
|---|---|---|
| (71 RESIDUES) | (59 RESIDUES) | (47 RESIDUES) |
| N67 | N67 | N67 |
| T69 | T69 | P70 |
| P70 | P70 | A72 |
| A72 | A72 | V73 |
| V73 | V73 | S74 |
| S74 | S74 | P75 |
| P75 | P75 | G77 |
| Y76 | Y76 | Q78 |
| G77 | G77 | N79 |
| Q78 | Q78 | Y81 |
| N79 | N79 | K82 |
| E80 | E80 | K83 |
| Y81 | Y81 | S84 |
| K82 | K82 | E114 |
| K83 | K83 | I116 |
| S84 | S84 | T185 |
| L85 | K106 | Y188 |
| V103 | W107 | Q202 |
| K106 | E114 | A204 |
| W107 | I116 | T206 |
| F113 | P183 | S209 |
| E114 | T185 | F211 |
| D115 | W187 | V213 |
| I116 | Y188 | P214 |
| T165 | Q202 | R215 |
| A182 | A204 | P218 |
| P183 | F205 | I219 |
| T185 | T206 | T220 |
| W187 | S209 | P221 |
| Y188 | F211 | D222 |
| G190 | V213 | Q223 |
| F200 | P214 | F224 |
| Q202 | R215 | G226 |
| A204 | P216 | K227 |
| F205 | K217 | I228 |
| T206 | P218 | P229 |
| S209 | I219 | I230 |
| T210 | T220 | K231 |
| F211 | P221 | E232 |
| V213 | D222 | A233 |
| P214 | Q223 | K235 |
| R215 | F224 | Y328 |
| P216 | K225 | N338 |
| K217 | G226 | Y339 |
| P218 | K227 | L340 |
| I219 | I228 | E441 |
| T220 | P229 | E446 |
| P221 | 1230 | |
| D222 | K231 | |
| Q223 | E232 | |
| F224 | A233 | |
| K225 | K235 | |
| G226 | Y328 | |
| K227 | N338 | |
| 1228 | Y339 | |
| P229 | L340 | |
| I230 | E441 | |
| K231 | N442 | |
| E232 | E446 | |
| A233 | | |
| D234 | | |
| K235 | | |
| V301 | | |
| Y328 | | |

### Table 37-2

**Table 37-2**

| Position at which 20% or more of mutations resulted in 54% or more improvement in yield | Position at which 30% or more of mutations resulted in 54% or more improvement in yield | Position at which 40% or more of mutations resulted in 54% or more improvement in yield |
|---|---|---|
| N338 | | |
| Y339 | | |
| L340 | | |
| I440 | | |
| E441 | | |
| N442 | | |
| E446 | | |

### (7) Preparation of double mutation strains

For the purpose of obtaining the strains capable of giving further enhanced yield, double mutation strains were made by mutually combining the mutation points by which the enhanced yield had been obtained (Table 37). For example, in the case of combining I157L and Y328F which were the mutation points which had contributed to enhanced yield of AMP, PCR and the transformation were performed by the methods described in Example 22 (2) using the primers used for introducing Y328F into A1/I157L, and the strains having the objective plasmid were selected using the ampicillin resistance as the indicator.

### (8) Measurement of specific activity in double mutation strain

The specific activity (U/mL) in the double mutation strains was calculated by the methods described in Example 22 (4), and is shown in Table 38.

### (9) Measurement of AMP yield in each double mutation strain in low concentration reaction solution

Based on the resulting specific activity data, the amount of the broth necessary for obtaining 2 U was calculated as to each mutant strain. Subsequently, the calculated amount of the broth was added to 1 mL of the low concentration reaction solution, and reacted at a temperature of 20°C at initial pH of 8.5. The amounts of produced AMP 25 and 45 minutes after the start of the reaction were quantified by HPLC, and the mutant strains listed on Table 38 exhibited higher yield than A1. It has been found out that these mutations contribute to the enhancement of yield when two of these mutations are combined.

### (10) Preparation of multiple mutation strains

For the purpose of obtaining the strains capable of exhibiting still more enhanced yield, the combinable mutation points each of which had contributed to AMP yield enhancement were mutually combined, to produce the multiple mutation strains (Table 38). For example, mutation points I157L with Y81A/Y328F, each of which had contributed to high AMP yield enhancement, were combined by PCR and transformation in accordance with the methods described in Example 22 (2) using the primers for introducing I157L into pA1/Y81A/Y328F, and the strains having the objective plasmid were selected using the ampicillin resistance as the indicator. The amounts of produced AMP 25 and 45 minutes after the start of the reaction were quantified by HPLC, and the mutants listed on Table 38 exhibited higher yield than A1. It has been found out that these mutations contribute to the enhancement of yield when three or more of these mutations are combined.

### Table 38-1

**Table 38-1**

| MUTATION ID | MUTATION | | | | | | RATIO TO A1 |
|---|---|---|---|---|---|---|---|
| MUTATION M1 | T69N | I157L | | | | | 1.09 |
| MUTATION M2 | T69Q | I157L | | | | | 1.28 |
| MUTATION M3 | T69S | I157L | | | | | 1.10 |
| MUTATION M4 | P70A | I157L | | | | | 1.15 |
| MUTATION M5 | P70G | I157L | | | | | 1.13 |
| MUTATION M6 | P701 | I157L | | | | | 1.06 |
| MUTATION M7 | P70L | I157L | | | | | 1.21 |
| MUTATION M8 | P70N | I157L | | | | | 1.13 |
| MUTATION M9 | P70S | I157L | | | | | 1.17 |
| MUTATION M10 | P70T | I157L | | | | | 1.33 |
| MUTATION M11 | P70T | T210L | | | | | 1.14 |
| MUTATION M12 | P70T | Y328F | | | | | 1.23 |
| MUTATION M13 | P70V | I157L | | | | | 1.24 |
| MUTATION M14 | A72E | G77S | | | | | 1.01 |
| MUTATION M15 | A72E | E80D | | | | | 1.08 |
| MUTATION M16 | A72E | Y81A | | | | | 1.09 |
| MUTATION M17 | A72E | S84D | | | | | 1.15 |
| MUTATION M18 | A72E | F113W | | | | | 1.15 |
| MUTATION M19 | A72E | l157L | | | | | 1.21 |
| MUTATION M20 | A72E | G161A | | | | | 1.11 |
| MUTATION M21 | A72E | F162L | | | | | 1.15 |
| MUTATION M22 | A72E | A184G | | | | | 1.05 |
| MUTATION M23 | A72E | W187F | | | | | 1.10 |
| MUTATION M24 | A72E | F200A | | | | | 1.06 |
| MUTATION M25 | A72E | A204S | | | | | 1.06 |
| MUTATION M26 | A72E | T210L | | | | | 1.10 |
| MUTATION M27 | A72E | F211 L | | | | | 1.19 |
| MUTATION M28 | A72E | F211W | | | | | 1.10 |
| MUTATION M29 | A72E | G226A | | | | | 1.14 |
| MUTATION M30 | A72E | 1228K | | | | | 1.08 |
| MUTATION M31 | A72E | A233D | | | | | 1.09 |
| MUTATION M32 | A72E | Y328F | | | | | 1.46 |
| MUTATION M33 | A72S | 1157L | | | | | 1.15 |
| MUTATION M34 | A72V | Y328F | | | | | 1.27 |
| MUTATION M35 | V73A | I157L | | | | | 1.10 |
| MUTATION M36 | V73I | I157L | | | | | 1.20 |
| MUTATION M37 | S74A | I157L | | | | | 1.30 |
| MUTATION M38 | S74N | I157L | | | | | 1.30 |
| MUTATION M39 | S74T | I157L | | | | | 1.20 |
| MUTATION M40 | S74V | I157L | | | | | 1.16 |
| MUTATION M41 | G77A | I157L | | | | | 1.31 |
| MUTATION M42 | G77F | I157L | | | | | 1.24 |
| MUTATION M43 | G77M | I157L | | | | | 1.30 |
| MUTATION M44 | G77P | I157L | | | | | 1.27 |
| MUTATION M45 | G77S | E80D | | | | | 1.06 |
| MUTATION M46 | G77S | Y81A | | | | | 1.05 |
| MUTATION M47 | G77S | S84D | | | | | 1.10 |
| MUTATION M48 | G77S | F113W | | | | | 1.12 |
| MUTATION M49 | G77S | I157L | | | | | 1.16 |
| MUTATION M50 | G77S | Y159N | | | | | 1.22 |
| MUTATION M51 | G77S | Y159S | | | | | 1.08 |
| MUTATION M52 | G77S | G161A | | | | | 1.02 |
| MUTATION M53 | G77S | F162L | | | | | 1.14 |

### Table 38-2

**Table 38-2**

| MUTATION ID | MUTATION | | | | | | RATIO TO A1 |
|---|---|---|---|---|---|---|---|
| MUTATION M54 | G77S | A184G | | | | | 1.07 |
| MUTATION M55 | G77S | W187F | | | | | 1.10 |
| MUTATION M56 | G77S | F200A | | | | | 1.00 |
| MUTATION M57 | G77S | A204S | | | | | 1.00 |
| MUTATION M58 | G77S | T210L | | | | | 1.03 |
| MUTATION M59 | G77S | F211L | | | | | 1.16 |
| MUTATION M60 | G77S | F211W | | | | | 1.13 |
| MUTATION M61 | G77S | I228K | | | | | 1.06 |
| MUTATION M62 | G77S | A233D | | | | | 1.11 |
| MUTATION M63 | G77S | R276A | | | | | 1.11 |
| MUTATION M64 | G77S | Y328F | | | | | 1.34 |
| MUTATION M65 | E80D | Y81A | | | | | 1.02 |
| MUTATION M66 | E80D | F113W | | | | | 1.07 |
| MUTATION M67 | E80D | I157L | | | | | 1.20 |
| MUTATION M68 | E80D | Y159N | | | | | 1.19 |
| MUTATION M69 | E80D | G161A | | | | | 1.08 |
| MUTATION M70 | E80D | A184G | | | | | 1.12 |
| MUTATION M71 | E80D | F211W | | | | | 1.07 |
| MUTATION M72 | E80D | Y328F | | | | | 1.17 |
| MUTATION M73 | E80S | I157L | | | | | 1.19 |
| MUTATION M74 | Y81A | F113W | | | | | 1.06 |
| MUTATION M75 | Y81A | I157L | | | | | 1.17 |
| MUTATION M76 | Y81A | Y159N | | | | | 1.14 |
| MUTATION M77 | Y81A | Y159S | | | | | 1.17 |
| MUTATION M78 | Y81A | G161A | | | | | 1.02 |
| MUTATION M79 | Y81A | A184G | | | | | 1.08 |
| MUTATION M80 | Y81A | W187F | | | | | 1.08 |
| MUTATION M81 | Y81A | F200A | | | | | 1.01 |
| MUTATION M82 | Y81A | T210L | | | | | 1.05 |
| MUTATION M83 | Y81A | F211W | | | | | 1.14 |
| MUTATION M84 | Y81A | F211Y | | | | | 1.16 |
| MUTATION M85 | Y81A | G226A | | | | | 1.06 |
| MUTATION M86 | Y81A | I228K | | | | | 1.02 |
| MUTATION M87 | Y81A | A233D | | | | | 1.05 |
| MUTATION M88 | Y81A | Y328F | | | | | 1.19 |
| MUTATION M89 | Y81H | I157L | | | | | 1.29 |
| MUTATION M90 | Y81N | I157L | | | | | 1.24 |
| MUTATION M91 | K83P | I157L | | | | | 1.23 |
| MUTATION M92 | S84A | I157L | | | | | 1.23 |
| MUTATION M93 | S84D | F113W | | | | | 1.04 |
| MUTATION M94 | S84D | I157L | | | | | 1.19 |
| MUTATION M95 | S84D | Y159N | | | | | 1.25 |
| MUTATION M96 | S84D | G161A | | | | | 1.03 |
| MUTATION M97 | S84D | A184G | | | | | 1.04 |
| MUTATION M98 | S84D | Y328F | | | | | 1.16 |
| MUTATION M99 | S84E | I157L | | | | | 1.16 |
| MUTATION M100 | S84F | I157L | | | | | 1.20 |
| MUTATION M101 | S84K | I157L | | | | | 1.26 |
| MUTATION M102 | L85F | I157L | | | | | 1.14 |
| MUTATION M103 | L851 | I157L | | | | | 1.27 |
| MUTATION M104 | L85P | I157L | | | | | 1.24 |
| MUTATION M105 | L85V | I157L | | | | | 1.36 |
| MUTATION M106 | N87A | I157L | | | | | 1.21 |
| MUTATION M107 | N87D | I157L | | | | | 1.22 |

### Table 38-3

**Table 38-3**

| MUTATION ID | MUTATION | | | | | | RATIO TO A1 |
|---|---|---|---|---|---|---|---|
| MUTATION M108 | N87E | I157L | | | | | 1.12 |
| MUTATION M109 | N87G | I157L | | | | | 1.30 |
| MUTATION M110 | N87Q | I157L | | | | | 1.18 |
| MUTATION M111 | N87S | I157L | | | | | 1.17 |
| MUTATION M112 | F88A | I157L | | | | | 1.11 |
| MUTATION M113 | F88D | I157L | | | | | 1.08 |
| MUTATION M114 | F88E | 1157L | | | | | 1.40 |
| MUTATION M115 | F88E | Y328F | | | | | 1.20 |
| MUTATION M116 | F88L | I157L | | | | | 1.00 |
| MUTATION M117 | F88T | I157L | | | | | 1.11 |
| MUTATION M118 | F88V | I157L | | | | | 1.08 |
| MUTATION M119 | F88Y | I157L | | | | | 1.18 |
| MUTATION M120 | K106H | I157L | | | | | 1.22 |
| MUTATION M121 | K106L | I157L | | | | | 1.22 |
| MUTATION M122 | K106M | I157L | | | | | 1.17 |
| MUTATION M123 | K106Q | I157L | | | | | 1.16 |
| MUTATION M124 | K106R | I157L | | | | | 1.20 |
| MUTATION M125 | K106S | I157L | | | | | 1.25 |
| MUTATION M126 | K106V | I157L | | | | | 1.37 |
| MUTATION M127 | W107A | I157L | | | | | 1.23 |
| MUTATION M128 | W107A | Y328F | | | | | 1.16 |
| MUTATION M129 | W107Y | I157L | | | | | 1.24 |
| MUTATION M130 | W107Y | T206Y | | | | | 1.01 |
| MUTATION M131 | W107Y | K217D | | | | | 1.04 |
| MUTATION M132 | W107Y | P218L | | | | | 1.04 |
| MUTATION M133 | W107Y | T220L | | | | | 1.03 |
| MUTATION M134 | W107Y | P221D | | | | | 1.02 |
| MUTATION M135 | W107Y | Y328F | | | | | 1.14 |
| MUTATION M136 | F113A | I157L | | | | | 1.12 |
| MUTATION M137 | F113H | I157L | | | | | 1.26 |
| MUTATION M138 | F113N | I157L | | | | | 1.14 |
| MUTATION M139 | F113V | I157L | | | | | 1.06 |
| MUTATION M140 | F113W | I157L | | | | | 1.19 |
| MUTATION M141 | F113W | Y159N | | | | | 1.09 |
| MUTATION M142 | F113W | Y159S | | | | | 1.12 |
| MUTATION M143 | F113W | G161A | | | | | 1.08 |
| MUTATION M144 | F113W | F162L | | | | | 1.13 |
| MUTATION M145 | F113W | A184G | | | | | 1.10 |
| MUTATION M146 | F113W | W187F | | | | | 1.05 |
| MUTATION M147 | F113W | F200A | | | | | 1.07 |
| MUTATION M148 | F113W | T206Y | | | | | 1.02 |
| MUTATION M149 | F113W | T210L | | | | | 1.08 |
| MUTATION M150 | F113W | F211L | | | | | 1.00 |
| MUTATION M151 | F113W | F211 W | | | | | 1.15 |
| MUTATION M152 | F113W | F211Y | | | | | 1.15 |
| MUTATION M153 | F113W | V213D | | | | | 1.02 |
| MUTATION M154 | F113W | K217D | | | | | 1.04 |
| MUTATION M155 | F113W | T220L | | | | | 1.06 |
| MUTATION M156 | F113W | P221 D | | | | | 1.06 |
| MUTATION M157 | F113W | G226A | | | | | 1.05 |
| MUTATION M158 | F113W | I228K | | | | | 1.11 |
| MUTATION M159 | F113W | A233D | | | | | 1.03 |
| MUTATION M160 | F113W | R276A | | | | | 1.05 |
| MUTATION M161 | F113Y | I157L | | | | | 1.20 |

### Table 38-4

**Table 38-4**

| MUTATION ID | MUTATION | | | | | | RATIO TO A1 |
|---|---|---|---|---|---|---|---|
| MUTATION M162 | F113Y | F211W | | | | | 1.13 |
| MUTATION M163 | E114D | I157L | | | | | 1.13 |
| MUTATION M164 | D115A | I157L | | | | | 1.15 |
| MUTATION M165 | D115E | I157L | | | | | 1.27 |
| MUTATION M166 | D115M | I157L | | | | | 1.08 |
| MUTATION M167 | D115N | I157L | | | | | 1.28 |
| MUTATION M168 | D115Q | I157L | | | | | 1.17 |
| MUTATION M169 | D115S | I157L | | | | | 1.21 |
| MUTATION M170 | D115V | I157L | | | | | 1.14 |
| MUTATION M171 | I157L | Y159I | | | | | 1.02 |
| MUTATION M172 | I157L | Y159L | | | | | 1.07 |
| MUTATION M173 | I157L | Y159N | | | | | 1.45 |
| MUTATION M174 | I157L | Y159S | | | | | 1.30 |
| MUTATION M175 | I157L | Y159V | | | | | 1.11 |
| MUTATION M176 | I157L | P160A | | | | | 1.03 |
| MUTATION M177 | I157L | P160S | | | | | 1.13 |
| MUTATION M178 | I157L | G161A | | | | | 1.28 |
| MUTATION M179 | I157L | F162L | | | | | 1.23 |
| MUTATION M180 | I157L | F162M | | | | | 1.34 |
| MUTATION M181 | I157L | F162N | | | | | 1.14 |
| MUTATION M182 | I157L | F162Y | | | | | 1.28 |
| MUTATION M183 | I157L | T165L | | | | | 1.23 |
| MUTATION M184 | I157L | T165V | | | | | 1.30 |
| MUTATION M185 | I157L | Q181A | | | | | 1.22 |
| MUTATION M186 | I157L | Q181F | | | | | 1.35 |
| MUTATION M187 | I157L | Q181N | | | | | 1.34 |
| MUTATION M188 | I157L | A184G | | | | | 1.35 |
| MUTATION M189 | I157L | A184L | | | | | 1.08 |
| MUTATION M190 | I157L | A184M | | | | | 1.04 |
| MUTATION M191 | I157L | A184S | | | | | 1.16 |
| MUTATION M192 | I157L | A184T | | | | | 1.22 |
| MUTATION M193 | I157L | W187F | | | | | 1.27 |
| MUTATION M194 | I157L | W187Y | | | | | 1.22 |
| MUTATION M195 | I157L | F193H | | | | | 1.31 |
| MUTATION M196 | I157L | F193I | | | | | 1.20 |
| MUTATION M197 | I157L | F193W | | | | | 1.17 |
| MUTATION M198 | I157L | F200A | | | | | 1.26 |
| MUTATION M199 | I157L | F200H | | | | | 1.37 |
| MUTATION M200 | I157L | F200L | | | | | 1.31 |
| MUTATION M201 | I157L | F200Y | | | | | 1.32 |
| MUTATION M202 | I157L | A204G | | | | | 1.38 |
| MUTATION M203 | I157L | A2041 | | | | | 1.37 |
| MUTATION M204 | I157L | A204L | | | | | 1.40 |
| MUTATION M205 | I157L | A204S | | | | | 1.21 |
| MUTATION M206 | I157L | A204T | | | | | 1.21 |
| MUTATION M207 | I157L | A204V | | | | | 1.20 |
| MUTATION M208 | I157L | F205A | | | | | 1.27 |
| MUTATION M209 | I157L | F2071 | | | | | 1.11 |
| MUTATION M210 | I157L | F207M | | | | | 1.26 |
| MUTATION M211 | I157L | F207V | | | | | 1.09 |
| MUTATION M212 | I157L | F207W | | | | | 1.19 |
| MUTATION M213 | I157L | F207Y | | | | | 1.24 |
| MUTATION M214 | I157L | M208A | | | | | 1.22 |
| MUTATION M215 | I157L | M208K | | | | | 1.34 |

### Table 38-5

**Table 38-5**

| MUTATION ID | MUTATION | | | | | | RATIO TO A1 |
|---|---|---|---|---|---|---|---|
| MUTATION M216 | I157L | M208L | | | | | 1.25 |
| MUTATION M217 | I157L | M208T | | | | | 1.25 |
| MUTATION M218 | I157L | M208V | | | | | 1.25 |
| MUTATION M219 | I157L | S209F | | | | | 1.19 |
| MUTATION M220 | I157L | S209N | | | | | 1.28 |
| MUTATION M221 | I157L | T210A | | | | | 1.28 |
| MUTATION M222 | I157L | T210L | | | | | 1.27 |
| MUTATION M223 | I157L | F211I | | | | | 1.20 |
| MUTATION M224 | I157L | F211L | | | | | 1.32 |
| MUTATION M225 | I157L | F211V | | | | | 1.17 |
| MUTATION M226 | I157L | F211W | | | | | 1.63 |
| MUTATION M227 | I157L | G212A | | | | | 1.16 |
| MUTATION M228 | I157L | G212D | | | | | 1.28 |
| MUTATION M229 | I157L | G212S | | | | | 1.17 |
| MUTATION M230 | I157L | R215K | | | | | 1.18 |
| MUTATION M231 | I157L | R215L | | | | | 1.17 |
| MUTATION M232 | I157L | R215T | | | | | 1.20 |
| MUTATION M233 | I157L | R215Y | | | | | 1.16 |
| MUTATION M234 | I157L | T220L | | | | | 1.23 |
| MUTATION M235 | I157L | G226A | | | | | 1.29 |
| MUTATION M236 | I157L | G226F | | | | | 1.24 |
| MUTATION M237 | I157L | I228K | | | | | 1.24 |
| MUTATION M238 | I157L | A233D | | | | | 1.21 |
| MUTATION M239 | I157L | R276A | | | | | 1.22 |
| MUTATION M240 | I157L | Y328A | | | | | 1.13 |
| MUTATION M241 | I157L | Y328F | | | | | 1.37 |
| MUTATION M242 | I157L | Y328H | | | | | 1.21 |
| MUTATION M243 | I157L | Y328I | | | | | 1.25 |
| MUTATION M244 | I157L | Y328L | | | | | 1.24 |
| MUTATION M245 | I157L | Y328P | | | | | 1.02 |
| MUTATION M246 | I157L | Y328V | | | | | 1.08 |
| MUTATION M247 | I157L | Y328W | | | | | 1.10 |
| MUTATION M248 | I157L | L340F | | | | | 1.12 |
| MUTATION M249 | I157L | L340I | | | | | 1.33 |
| MUTATION M250 | I157L | L340V | | | | | 1.31 |
| MUTATION M251 | I157L | V439A | | | | | 1.27 |
| MUTATION M252 | I157L | V439P | | | | | 1.26 |
| MUTATION M253 | I157L | R445A | | | | | 1.14 |
| MUTATION M254 | I157L | R445F | | | | | 1.06 |
| MUTATION M255 | I157L | R445G | | | | | 1.15 |
| MUTATION M256 | I157L | R445K | | | | | 1.17 |
| MUTATION M257 | I157L | R445V | | | | | 1.14 |
| MUTATION M258 | Y159N | G161A | | | | | 1.25 |
| MUTATION M259 | Y159N | A184G | | | | | 1.31 |
| MUTATION M260 | Y159N | A204S | | | | | 1.22 |
| MUTATION M261 | Y159N | T210L | | | | | 1.26 |
| MUTATION M262 | Y159N | F211W | | | | | 1.05 |
| MUTATION M263 | Y159N | F211Y | | | | | 1.03 |
| MUTATION M264 | Y159N | G226A | | | | | 1.33 |
| MUTATION M265 | Y159N | 1228K | | | | | 1.17 |
| MUTATION M266 | Y159N | A233D | | | | | 1.26 |
| MUTATION M267 | Y159N | Y328F | | | | | 1.25 |
| MUTATION M268 | Y159S | G161A | | | | | 1.41 |
| MUTATION M269 | Y159S | F211W | | | | | 1.25 |

### Table 38-6

**Table 38-6**

| MUTATION ID | MUTATION | | | | | | RATIO TO A1 |
|---|---|---|---|---|---|---|---|
| MUTATION M270 | G161A | F162L | | | | | 1.16 |
| MUTATION M271 | G161A | A184G | | | | | 1.17 |
| MUTATION M272 | G161A | W187F | | | | | 1.13 |
| MUTATION M273 | G161A | F200A | | | | | 1.15 |
| MUTATION M274 | G161A | A204S | | | | | 1.15 |
| MUTATION M275 | G161A | T210L | | | | | 1.11 |
| MUTATION M276 | G161A | F211L | | | | | 1.19 |
| MUTATION M277 | G161A | F211W | | | | | 1.21 |
| MUTATION M278 | G161A | G226A | | | | | 1.28 |
| MUTATION M279 | G161A | I228K | | | | | 1.13 |
| MUTATION M280 | G161A | A233D | | | | | 1.13 |
| MUTATION M281 | G161A | Y328F | | | | | 1.27 |
| MUTATION M282 | F162L | A184G | | | | | 1.11 |
| MUTATION M283 | F162L | F211W | | | | | 1.09 |
| MUTATION M284 | F162L | A233D | | | | | 1.01 |
| MUTATION M285 | P183A | Y328F | | | | | 1.19 |
| MUTATION M286 | A184G | W187F | | | | | 1.18 |
| MUTATION M287 | A184G | F200A | | | | | 1.14 |
| MUTATION M288 | A184G | A204S | | | | | 1.11 |
| MUTATION M289 | A184G | T210L | | | | | 1.02 |
| MUTATION M290 | A184G | F211L | | | | | 1.23 |
| MUTATION M291 | A184G | F211 W | | | | | 1.22 |
| MUTATION M292 | A184G | I228K | | | | | 1.12 |
| MUTATION M293 | A184G | A233D | | | | | 1.15 |
| MUTATION M294 | A184G | R276A | | | | | 1.08 |
| MUTATION M295 | V184G | Y328F | | | | | 1.30 |
| MUTATION M296 | T185A | Y328F | | | | | 1.11 |
| MUTATION M297 | T185N | Y328F | | | | | 1.14 |
| MUTATION M298 | W187F | F211W | | | | | 1.32 |
| MUTATION M299 | W187F | Y328F | | | | | 1.30 |
| MUTATION M300 | F193W | F211W | | | | | 1.02 |
| MUTATION M301 | F200A | F211W | | | | | 1.30 |
| MUTATION M302 | F200A | Y328F | | | | | 1.24 |
| MUTATION M303 | L201Q | Y328F | | | | | 1.01 |
| MUTATION M304 | L201S | Y328F | | | | | 1.14 |
| MUTATION M305 | A204S | F211 W | | | | | 1.22 |
| MUTATION M306 | A204S | Y328F | | | | | 1.18 |
| MUTATION M307 | T210L | F211W | | | | | 1.06 |
| MUTATION M308 | T210L | Y328F | | | | | 1.20 |
| MUTATION M309 | F211L | A233D | | | | | 1.02 |
| MUTATION M310 | F211L | Y328F | | | | | 1.23 |
| MUTATION M311 | F211W | 1228K | | | | | 1.19 |
| MUTATION M312 | F211W | A233D | | | | | 1.10 |
| MUTATION M313 | F211W | Y328F | | | | | 1.18 |
| MUTATION M314 | R215A | Y328F | | | | | 1.09 |
| MUTATION M315 | R215L | Y328F | | | | | 1.11 |
| MUTATION M316 | T220L | A233D | | | | | 1.03 |
| MUTATION M317 | T220L | D300N | | | | | 1.03 |
| MUTATION M318 | P221L | A233D | | | | | 1.02 |
| MUTATION M319 | P221L | Y328F | | | | | 1.15 |
| MUTATION M320 | F224A | A233D | | | | | 1.04 |
| MUTATION M321 | G226A | Y328F | | | | | 1.12 |
| MUTATION M322 | G226F | A233D | | | | | 1.06 |
| MUTATION M323 | G226F | Y328F | | | | | 1.11 |

### Table 38-7

**Table 38-7**

| MUTATION ID | MUTATION | | | | | | RATIO TO A1 |
|---|---|---|---|---|---|---|---|
| MUTATION M324 | I228K | Y328F | | | | | 1.15 |
| MUTATION M325 | A233D | K235D | | | | | 1.02 |
| MUTATION M326 | A233D | Y328F | | | | | 1.40 |
| MUTATION M327 | R276A | Y328F | | | | | 1.24 |
| MUTATION M328 | Y328F | Y339F | | | | | 1.14 |
| MUTATION M329 | A27T | Y81A | S84D | | | | 1.06 |
| MUTATION M330 | P70T | A72E | I157L | | | | 1.30 |
| MUTATION M331 | P70T | G77S | I157L | | | | 1.35 |
| MUTATION M332 | P70T | E80D | F88E | | | | 1.17 |
| MUTATION M333 | P70T | Y81A | I157L | | | | 1.21 |
| MUTATION M334 | P70T | S84D | I157L | | | | 1.17 |
| MUTATION M335 | P70T | F88E | Y328F | | | | 1.29 |
| MUTATION M336 | P70T | F113W | I157L | | | | 1.23 |
| MUTATION M337 | P70T | I157L | A204S | | | | 1.21 |
| MUTATION M338 | P70T | I157L | T210L | | | | 1.25 |
| MUTATION M339 | P70T | I157L | A233D | | | | 1.18 |
| MUTATION M340 | P70T | I157L | Y328F | | | | 1.34 |
| MUTATION M341 | P70T | I157L | V439P | | | | 1.23 |
| MUTATION M342 | P70T | I157L | I440F | | | | 1.25 |
| MUTATION M343 | P70T | G161A | T210L | | | | 1.29 |
| MUTATION M344 | P70T | G161A | Y328F | | | | 1.32 |
| MUTATION M345 | P70T | A184G | W187F | | | | 1.20 |
| MUTATION M346 | P70T | A204S | Y328F | | | | 1.25 |
| MUTATION M347 | P70T | F211W | Y328F | | | | 1.33 |
| MUTATION M348 | P70V | A72E | I157L | | | | 1.32 |
| MUTATION M349 | A72E | S74T | I157L | | | | 1.32 |
| MUTATION M350 | A72E | G77S | Y328F | | | | 1.24 |
| MUTATION M351 | A72E | E80D | Y328F | | | | 1.35 |
| MUTATION M352 | A72E | Y81H | I157L | | | | 1.28 |
| MUTATION M353 | A72E | K83P | I157L | | | | 1.35 |
| MUTATION M354 | A72E | S84D | Y328F | | | | 1.15 |
| MUTATION M355 | A72E | L85P | I157L | | | | 1.30 |
| MUTATION M356 | A72E | F113W | I157L | | | | 1.34 |
| MUTATION M357 | A72E | F113W | Y328F | | | | 1.30 |
| MUTATION M358 | A72E | F113Y | I157L | | | | 1.35 |
| MUTATION M359 | A72E | D115Q | I157L | | | | 1.31 |
| MUTATION M360 | A72E | I157L | G161A | | | | 1.21 |
| MUTATION M361 | A72E | I157L | F162L | | | | 1.26 |
| MUTATION M362 | A72E | I157L | A184G | | | | 1.52 |
| MUTATION M363 | A72E | I157L | F200A | | | | 1.20 |
| MUTATION M364 | A72E | I157L | A204S | | | | 1.28 |
| MUTATION M365 | A72E | I157L | A204T | | | | 1.29 |
| MUTATION M366 | A72E | I157L | T210L | | | | 1.30 |
| MUTATION M367 | A72E | I157L | F211W | | | | 1.17 |
| MUTATION M368 | A72E | I157L | G226A | | | | 1.31 |
| MUTATION M369 | A72E | I157L | A233D | | | | 1.43 |
| MUTATION M370 | A72E | I157L | Y328F | | | | 1.39 |
| MUTATION M371 | A72E | I157L | L340V | | | | 1.34 |
| MUTATION M372 | A72E | I157L | V439P | | | | 1.22 |
| MUTATION M373 | A72E | G161A | Y328F | | | | 1.45 |
| MUTATION M374 | A72E | F162L | Y328F | | | | 1.21 |
| MUTATION M375 | A72E | A184G | Y328F | | | | 1.31 |
| MUTATION M376 | A72E | W187F | Y328F | | | | 1.30 |
| MUTATION M377 | A72E | F200A | Y328F | | | | 1.23 |

### Table 38-8

**Table 38-8**

| MUTATION ID | MUTATION | | | | | | RATIO TO A1 |
|---|---|---|---|---|---|---|---|
| MUTATION M378 | A72E | A204S | Y328F | | | | 1.20 |
| MUTATION M379 | A72E | T210L | Y328F | | | | 1.15 |
| MUTATION M380 | A72E | I228K | Y328F | | | | 1.12 |
| MUTATION M381 | A72E | A233D | Y328F | | | | 1.16 |
| MUTATION M382 | A72E | Y328F | Y159N | | | | 1.26 |
| MUTATION M383 | A72E | Y328F | F211W | | | | 1.45 |
| MUTATION M384 | A72E | Y328F | F211Y | | | | 1.22 |
| MUTATION M385 | A72E | Y328F | G226A | | | | 1.22 |
| MUTATION M386 | A72V | Y81A | Y328F | | | | 1.01 |
| MUTATION M387 | A72V | G161A | Y328F | | | | 1.30 |
| MUTATION M388 | G77M | I157L | T210L | | | | 1.37 |
| MUTATION M389 | G77P | I157L | F162L | | | | 1.30 |
| MUTATION M390 | G77P | I157L | A184G | | | | 1.25 |
| MUTATION M391 | G77P | F211W | Y328F | | | | 1.28 |
| MUTATION M392 | G77S | Y81A | Y328F | | | | 1.34 |
| MUTATION M393 | G77S | S84D | I157L | | | | 1.29 |
| MUTATION M394 | G77S | F88E | I157L | | | | 1.25 |
| MUTATION M395 | G77S | F113W | I157L | | | | 1.16 |
| MUTATION M396 | G77S | F113Y | I157L | | | | 1.21 |
| MUTATION M397 | G77S | D115Q | I157L | | | | 1.22 |
| MUTATION M398 | G77S | I157L | G161A | | | | 1.21 |
| MUTATION M399 | G77S | I157L | F200A | | | | 1.33 |
| MUTATION M400 | G77S | I157L | A204S | | | | 1.30 |
| MUTATION M401 | G77S | I157L | T210L | | | | 1.20 |
| MUTATION M402 | G77S | I157L | F211W | | | | 1.49 |
| MUTATION M403 | G77S | I157L | G226A | | | | 1.38 |
| MUTATION M404 | G77S | I157L | A233D | | | | 1.39 |
| MUTATION M405 | G77S | I157L | L340V | | | | 1.38 |
| MUTATION M406 | G77S | I157L | V439P | | | | 1.33 |
| MUTATION M407 | G77S | G161A | Y328F | | | | 1.27 |
| MUTATION M408 | E80D | Y81A | Y328F | | | | 1.19 |
| MUTATION M409 | Y81A | S84D | Y328F | | | | 1.17 |
| MUTATION M410 | Y81A | F113W | Y328F | | | | 1.19 |
| MUTATION M411 | Y81A | I157L | T210L | | | | 1.14 |
| MUTATION M412 | Y81A | I157L | Y328F | | | | 1.32 |
| MUTATION M413 | Y81A | G161A | Y328F | | | | 1.17 |
| MUTATION M414 | Y81A | F162L | Y328F | | | | 1.20 |
| MUTATION M415 | Y81A | A184G | Y328F | | | | 1.27 |
| MUTATION M416 | Y81A | W187F | Y328F | | | | 1.19 |
| MUTATION M417 | Y81A | A204S | Y328F | | | | 1.11 |
| MUTATION M418 | Y81A | T210L | Y328F | | | | 1.22 |
| MUTATION M419 | Y81A | I228K | Y328F | | | | 1.27 |
| MUTATION M420 | Y81A | A233D | Y328F | | | | 1.19 |
| MUTATION M421 | Y81A | Y328F | Y159N | | | | 1.32 |
| MUTATION M422 | Y81A | Y328F | Y159S | | | | 1.20 |
| MUTATION M423 | Y81A | Y328F | F211W | | | | 1.24 |
| MUTATION M424 | Y81A | Y328F | F211Y | | | | 1.30 |
| MUTATION M425 | Y81A | Y328F | G226A | | | | 1.21 |
| MUTATION M426 | Y81A | Y328F | R276A | | | | 1.32 |
| MUTATION M427 | K83P | I157L | A184G | | | | 1.33 |
| MUTATION M428 | K83P | I157L | T210L | | | | 1.30 |
| MUTATION M429 | K83P | F211W | Y328F | | | | 1.24 |
| MUTATION M430 | S84D | F113W | I157L | | | | 1.34 |
| MUTATION M431 | S84D | I157L | T210L | | | | 1.33 |

### Table 38-9

**Table 38-9**

| MUTATION ID | MUTATION | | | | | | RATIO TO A1 |
|---|---|---|---|---|---|---|---|
| MUTATION M432 | F88E | I157L | F162L | | | | 1.24 |
| MUTATION M433 | F88E | I157L | A184G | | | | 1.31 |
| MUTATION M434 | F88E | I157L | F200A | | | | 1.21 |
| MUTATION M435 | F88E | I157L | T210L | | | | 1.37 |
| MUTATION M436 | F88E | I157L | Y328F | | | | 1.32 |
| MUTATION M437 | F88E | I157L | Y328Q | | | | 1.09 |
| MUTATION M438 | F88E | I157L | L340V | | | | 1.29 |
| MUTATION M439 | F88E | T210L | Y328F | | | | 1.19 |
| MUTATION M440 | F88E | F211W | Y328F | | | | 1.31 |
| MUTATION M441 | F113W | I157L | G161A | | | | 1.26 |
| MUTATION M442 | F113W | 1157L | A184G | | | | 1.36 |
| MUTATION M443 | F113W | I157L | W187F | | | | 1.20 |
| MUTATION M444 | F113W | I157L | F200A | | | | 1.33 |
| MUTATION M445 | F113W | I157L | A204S | | | | 1.33 |
| MUTATION M446 | F113W | I157L | A204T | | | | 1.29 |
| MUTATION M447 | F113W | I157L | T210L | | | | 1.16 |
| MUTATION M448 | F113W | I157L | F211W | | | | 1.48 |
| MUTATION M449 | F113W | I157L | G226A | | | | 1.31 |
| MUTATION M450 | F113W | I157L | A233D | | | | 1.35 |
| MUTATION M451 | F113W | I157L | Y328F | | | | 1.26 |
| MUTATION M452 | F113W | I157L | L340V | | | | 1.34 |
| MUTATION M453 | F113W | I157L | V439P | | | | 1.33 |
| MUTATION M454 | F113W | G161A | T210L | | | | 1.11 |
| MUTATION M455 | F113W | G161A | Y328F | | | | 1.27 |
| MUTATION M456 | F113W | A184G | W187F | | | | 1.11 |
| MUTATION M457 | F113Y | I157L | T210L | | | | 1.26 |
| MUTATION M458 | F113Y | I157L | Y328F | | | | 1.27 |
| MUTATION M459 | F113Y | G161A | T210L | | | | 1.08 |
| MUTATION M460 | D115Q | I157L | T210L | | | | 1.21 |
| MUTATION M461 | D115Q | I157L | Y328F | | | | 1.24 |
| MUTATION M462 | I157L | Y159N | T210L | | | | 1.34 |
| MUTATION M463 | I157L | Y159N | Y328F | | | | 1.49 |
| MUTATION M464 | I157L | G161A | W187F | | | | 1.19 |
| MUTATION M465 | I157L | G161A | F200A | | | | 1.01 |
| MUTATION M466 | I157L | G161A | A204S | | | | 1.20 |
| MUTATION M467 | I157L | G161A | T210L | | | | 1.20 |
| MUTATION M468 | I157L | G161A | A233D | | | | 1.22 |
| MUTATION M469 | I157L | G161A | Y328F | | | | 1.43 |
| MUTATION M470 | I157L | F162L | A184G | | | | 1.35 |
| MUTATION M471 | I157L | F162L | T210L | | | | 1.26 |
| MUTATION M472 | I157L | F162L | L340V | | | | 1.28 |
| MUTATION M473 | I157L | A184G | W187F | | | | 1.25 |
| MUTATION M474 | I157L | A184G | F200A | | | | 1.29 |
| MUTATION M475 | I157L | A184G | A204T | | | | 1.19 |
| MUTATION M476 | I157L | A184G | T210L | | | | 1.31 |
| MUTATION M477 | I157L | A184G | F211W | | | | 1.44 |
| MUTATION M478 | I157L | A184G | L340V | | | | 1.34 |
| MUTATION M479 | I157L | W187F | T210L | | | | 1.13 |
| MUTATION M480 | I157L | W187F | Y328F | | | | 1.27 |
| MUTATION M481 | I157L | F200A | T210L | | | | 1.18 |
| MUTATION M482 | I157L | F200A | Y328F | | | | 1.31 |
| MUTATION M483 | I157L | A204S | T210L | | | | 1.22 |
| MUTATION M484 | I157L | A204S | Y328F | | | | 1.30 |
| MUTATION M485 | I157L | A204T | T210L | | | | 1.22 |

### Table 38-10

**Table 38-10**

| MUTATION ID | MUTATION | | | | | | RATIO TO A1 |
|---|---|---|---|---|---|---|---|
| MUTATION M486 | I157L | A204T | Y328F | | | | 1.29 |
| MUTATION M487 | I157L | T210L | F211W | | | | 1.25 |
| MUTATION M488 | I157L | T210L | G212A | | | | 1.18 |
| MUTATION M489 | I157L | T210L | G226A | | | | 1.20 |
| MUTATION M490 | I157L | T210L | A233D | | | | 1.22 |
| MUTATION M491 | I157L | T210L | Y328F | | | | 1.34 |
| MUTATION M492 | I157L | T210L | L340V | | | | 1.37 |
| MUTATION M493 | 1157L | T210L | V439P | | | | 1.35 |
| MUTATION M494 | 1157L | F211W | Y328F | | | | 1.40 |
| MUTATION M495 | 1157L | G226A | Y328F | | | | 1.24 |
| MUTATION M496 | 1157L | A233D | Y328F | | | | 1.26 |
| MUTATION M497 | 1157L | Y328F | L340V | | | | 1.33 |
| MUTATION M498 | I157L | Y328F | V439P | | | | 1.27 |
| MUTATION M499 | Y159N | F211W | Y328F | | | | 1.16 |
| MUTATION M500 | G161A | A184G | W187F | | | | 1.25 |
| MUTATION M501 | G161A | T210L | Y328F | | | | 1.17 |
| MUTATION M502 | G161A | F211W | Y328F | | | | 1.17 |
| MUTATION M503 | A182G | P183A | Y328F | | | | 1.90 |
| MUTATION M504 | A182S | P183A | Y328F | | | | 1.18 |
| MUTATION M505 | A184G | W187F | F200A | | | | 1.10 |
| MUTATION M506 | A184G | W187F | A204S | | | | 1.16 |
| MUTATION M507 | A184G | W187F | F211W | | | | 1.15 |
| MUTATION M508 | A184G | W187F | 1228K | | | | 1.14 |
| MUTATION M509 | A184G | W187F | A233D | | | | 1.16 |
| MUTATION M510 | F200A | F211W | Y328F | | | | 1.31 |
| MUTATION M511 | A204S | F211W | Y328F | | | | 1.35 |
| MUTATION M512 | A204T | F211W | Y328F | | | | 1.28 |
| MUTATION M513 | F211 W | Y328F | L340V | | | | 1.26 |
| MUTATION M514 | P70T | A72E | I157L | Y328F | | | 1.65 |
| MUTATION M515 | P70T | A72E | T210L | Y328F | | | 1.39 |
| MUTATION M516 | P70T | G77M | I157L | Y328F | | | 1.32 |
| MUTATION M517 | P70T | Y81A | I157L | T210L | | | 1.19 |
| MUTATION M518 | P70T | Y81A | I157L | Y328F | | | 1.35 |
| MUTATION M519 | P70T | S84D | I157L | Y328F | | | 1.24 |
| MUTATION M520 | P70T | F88E | I157L | Y328F | | | 1.38 |
| MUTATION M521 | P70T | F88E | T210L | Y328F | | | 1.34 |
| MUTATION M522 | P70T | F113W | I157L | T210L | | | 1.37 |
| MUTATION M523 | P70T | F113W | G161A | Y328F | | | 1.17 |
| MUTATION M524 | P70T | F113Y | I157L | Y328F | | | 1.09 |
| MUTATION M525 | P70T | D115Q | I157L | T210L | | | 1.13 |
| MUTATION M526 | P70T | D115Q | I157L | Y328F | | | 1.27 |
| MUTATION M527 | P70T | I157L | G161A | T210L | | | 1.26 |
| MUTATION M528 | P70T | I157L | A184G | W187F | | | 1.33 |
| MUTATION M529 | P70T | I157L | A184G | T210L | | | 1.43 |
| MUTATION M530 | P70T | I157L | W187F | T210L | | | 1.34 |
| MUTATION M531 | P70T | I157L | W187F | Y328F | | | 1.34 |
| MUTATION M532 | P70T | I157L | A204T | T210L | | | 1.37 |
| MUTATION M533 | P70T | I157L | A204T | Y328F | | | 1.29 |
| MUTATION M534 | P70T | I157L | A204T | T210L | | | 1.22 |
| MUTATION M535 | P70T | I157L | T210L | F211W | | | 1.29 |
| MUTATION M536 | P70T | I157L | T210L | G226A | | | 1.27 |
| MUTATION M537 | P70T | I157L | T210L | A233D | | | 1.28 |
| MUTATION M538 | P70T | I157L | T210L | Y328F | | | 1.33 |
| MUTATION M539 | P70T | I157L | T210L | L340V | | | 1.37 |

### Table 38-11

**Table 38-11**

| MUTATION ID | MUTATION | | | | | | RATIO TO A1 |
|---|---|---|---|---|---|---|---|
| MUTATION M540 | P70T | I157L | T210L | V439P | | | 1.27 |
| MUTATION M541 | P70T | I157L | Y328F | V439P | | | 1.27 |
| MUTATION M542 | P70T | G161A | T210L | Y328F | | | 1.26 |
| MUTATION M543 | P70T | G161A | A233D | Y328F | | | 1.20 |
| MUTATION M544 | A72E | S74T | I157L | Y328F | | | 1.60 |
| MUTATION M545 | A72E | G77S | F113W | I157L | | | 1.07 |
| MUTATION M546 | A72E | Y81H | I157L | Y328F | | | 1.59 |
| MUTATION M547 | A72E | K83P | I157L | Y328F | | | 1.59 |
| MUTATION M548 | A72E | F88E | F113W | l157L | | | 1.28 |
| MUTATION M549 | A72E | F88E | l157L | Y328F | | | 1.59 |
| MUTATION M550 | A72E | F88E | G161A | Y328F | | | 1.45 |
| MUTATION M551 | A72E | F113W | 1157L | Y328F | | | 1.40 |
| MUTATION M552 | A72E | F113W | G161A | Y328F | | | 1.54 |
| MUTATION M553 | A72E | F113Y | I157L | Y328F | | | 1.67 |
| MUTATION M554 | A72E | F113Y | G161A | Y328F | | | 1.57 |
| MUTATION M555 | A72E | F113Y | G226A | Y328F | | | 1.49 |
| MUTATION M556 | A72E | I157L | G161A | Y328F | | | 1.47 |
| MUTATION M557 | A72E | I157L | F162L | Y328F | | | 1.56 |
| MUTATION M558 | A72E | I157L | A184G | Y328F | | | 1.45 |
| MUTATION M559 | A72E | I157L | F200A | Y328F | | | 1.59 |
| MUTATION M560 | A72E | I157L | A204T | Y328F | | | 1.37 |
| MUTATION M561 | A72E | I157L | F211W | Y328F | | | 1.74 |
| MUTATION M562 | A72E | I157L | F211Y | Y328F | | | 1.47 |
| MUTATION M563 | A72E | I157L | A233D | Y328F | | | 1.66 |
| MUTATION M564 | A72E | I157L | Y328F | L340V | | | 1.60 |
| MUTATION M565 | A72E | G161A | A204T | Y328F | | | 1.56 |
| MUTATION M566 | A72E | G161A | T210L | Y328F | | | 1.55 |
| MUTATION M567 | A72E | G161A | F211W | Y328F | | | 1.57 |
| MUTATION M568 | A72E | G161A | F211Y | Y328F | | | 1.57 |
| MUTATION M569 | A72E | G161A | A233D | Y328F | | | 1.54 |
| MUTATION M570 | A72E | G161A | Y328F | L340V | | | 1.48 |
| MUTATION M571 | A72E | A184G | W187F | Y328F | | | 1.30 |
| MUTATION M572 | A72E | T210L | Y328F | L340V | | | 1.23 |
| MUTATION M573 | A72V | l157L | W187F | Y328F | | | 1.40 |
| MUTATION M574 | G77P | I157L | T210L | Y328F | | | 1.33 |
| MUTATION M575 | Y81A | S84D | l157L | Y328F | | | 1.27 |
| MUTATION M576 | Y81A | F88E | l157L | Y328F | | | 1.24 |
| MUTATION M577 | Y81A | F113W | l157L | Y328F | | | 1.32 |
| MUTATION M578 | Y81A | l157L | G161A | Y328F | | | 1.32 |
| MUTATION M579 | Y81A | l157L | W187F | Y328F | | | 1.29 |
| MUTATION M580 | Y81A | I157L | A204S | Y328F | | | 1.28 |
| MUTATION M581 | Y81A | I157L | T210L | Y328F | | | 1.36 |
| MUTATION M582 | Y81A | I157L | A233D | Y328F | | | 1.30 |
| MUTATION M583 | Y81A | I157L | Y328F | V439P | | | 1.28 |
| MUTATION M584 | Y81A | A184G | W187F | Y328F | | | 1.25 |
| MUTATION M585 | F88E | I157L | T210L | Y328F | | | 1.30 |
| MUTATION M586 | F88E | I157L | A233D | Y328F | | | 1.25 |
| MUTATION M587 | F113W | I157L | A204T | T210L | | | 1.22 |
| MUTATION M588 | F113W | I157L | T210L | Y328F | | | 1.29 |
| MUTATION M589 | I157L | G161A | A184G | W187F | | | 1.34 |
| MUTATION M590 | I157L | G161A | T210L | Y328F | | | 1.33 |
| MUTATION M591 | I157L | A184G | W187F | T210L | | | 1.24 |
| MUTATION M592 | I157L | A204S | T210L | Y328F | | | 1.24 |
| MUTATION M593 | I157L | A204T | T210L | Y328F | | | 1.34 |

### Table 38-12

**Table 38-12**

| MUTATION ID | MUTATION | | | | | | RATIO TO A1 |
|---|---|---|---|---|---|---|---|
| MUTATION M594 | I157L | T210L | A233D | Y328F | | | 1.26 |
| MUTATION M595 | G161A | A184G | W187F | Y328F | | | 1.34 |
| MUTATION M596 | P70T | A72E | S84D | I157L | Y328F | | 1.41 |
| MUTATION M597 | P70T | A72E | A204S | I157L | Y328F | | 1.27 |
| MUTATION M598 | P70T | A72E | T210L | I157L | Y328F | | 1.35 |
| MUTATION M599 | P70T | A72E | G226A | I157L | Y328F | | 1.31 |
| MUTATION M600 | P70T | A72E | A233D | I157L | Y328F | | 1.36 |
| MUTATION M601 | P70T | Y81A | I157L | T210L | Y328F | | 1.38 |
| MUTATION M602 | P70T | Y81A | I157L | A233D | Y328F | | 1.10 |
| MUTATION M603 | P70T | Y81A | I157L | T210L | Y328F | | 1.37 |
| MUTATION M604 | P70T | Y81A | A233D | I157L | Y328F | | 1.23 |
| MUTATION M605 | P70T | S84D | I157L | T210L | Y328F | | 1.29 |
| MUTATION M606 | P70T | F113W | I157L | T210L | Y328F | | 1.33 |
| MUTATION M607 | P70T | I157L | A184G | W187F | A233D | | 1.30 |
| MUTATION M608 | P70T | I157L | W187F | T210L | Y328F | | 1.35 |
| MUTATION M609 | P70T | I157L | A204S | T210L | Y328F | | 1.31 |
| MUTATION M610 | P70T | G161A | A184G | W187F | Y328F | | 1.18 |
| MUTATION M611 | P70V | A72E | F113Y | I157L | Y328F | | 1.39 |
| MUTATION M612 | P70V | A72E | I157L | F211W | Y328F | | 1.53 |
| MUTATION M613 | A72E | S74T | F113Y | I157L | Y328F | | 1.31 |
| MUTATION M614 | A72E | S74T | I157L | F211W | Y328F | | 1.26 |
| MUTATION M615 | A72E | Y81H | I157L | F211W | Y328F | | 1.47 |
| MUTATION M616 | A72E | K83P | F113Y | I157L | Y328F | | 1.27 |
| MUTATION M617 | A72E | W17F | F113Y | I157L | Y328F | | 1.36 |
| MUTATION M618 | A72E | F113Y | D115Q | I157L | Y328F | | 1.32 |
| MUTATION M619 | A72E | F113Y | I157L | Y328F | L340V | | 1.35 |
| MUTATION M620 | A72E | F113Y | I157L | Y328F | V439P | | 1.38 |
| MUTATION M621 | A72E | F113Y | G161A | I157L | Y328F | | 1.44 |
| MUTATION M622 | A72E | F113Y | A204S | I157L | Y328F | | 1.41 |
| MUTATION M623 | A72E | F113Y | A204T | I157L | Y328F | | 1.39 |
| MUTATION M624 | A72E | F113Y | T210L | I157L | Y328F | | 1.40 |
| MUTATION M625 | A72E | F113Y | A233D | I157L | Y328F | | 1.38 |
| MUTATION M626 | A72E | I157L | G161A | F162L | Y328F | | 1.37 |
| MUTATION M627 | A72E | I157L | W187F | F211W | Y328F | | 1.09 |
| MUTATION M628 | A72E | I157L | A204S | F211W | Y328F | | 1.44 |
| MUTATION M629 | A72E | I157L | A204T | F211W | Y328F | | 1.43 |
| MUTATION M630 | A72E | I157L | F211W | Y328F | L340V | | 1.43 |
| MUTATION M631 | A72E | I157L | F211W | Y328F | V439P | | 1.48 |
| MUTATION M632 | A72E | I157L | G226A | F211W | Y328F | | 1.32 |
| MUTATION M633 | A72E | I157L | A233D | F211W | Y328F | | 1.43 |
| MUTATION M634 | Y81A | S84D | I157L | T210L | Y328F | | 1.24 |
| MUTATION M635 | Y81A | I157L | A184G | W187F | Y328F | | 1.35 |
| MUTATION M636 | Y81A | I157L | A184G | W187F | T210L | | 1.28 |
| MUTATION M637 | Y81A | I157L | A233D | T210L | Y328F | | 1.26 |
| MUTATION M638 | F88E | I157L | A184G | W187F | T210L | | 1.20 |
| MUTATION M639 | F113Y | I157L | Y159N | F211W | Y328F | | 1.30 |
| MUTATION M640 | I157L | A184G | W187F | T210L | Y328F | | 1.31 |
| MUTATION M641 | P70T | I157L | A184G | W187F | T210L | Y328F | 1.23 |
| MUTATION M642 | Y81A | I157L | A184G | W187F | T210L | Y328F | 1.39 |

### (11) Measurement of AMP yield in each mutant strain in high concentration reaction solution

Based on the resulting specific activity data, the amount of the broth necessary for obtaining 200 U was calculated as to each mutant strain. Subsequently, the calculated amount of the broth was concentrated to 5 mL. The concentrated broth of each mutant strain was added to 15 mL of the high concentration reaction solution (400 mM dimethyl aspartate, 600 mM phenylalanine), and reacted at a temperature of 22°C at initial pH of 8.5. As the reaction proceeds, the pH value was lowered, but pH was kept to 8.5 throughout the reaction by adding 6 M NaOH. The amounts of produced AMP 40, 60 and 80 minutes after the start of the reaction were quantified by HPLC. The mutants listed on Tables 39 and 40 exhibited higher yield than A1.

### Table 39

**Table 39**

| | RATIO TO A1 |
|---|---|
| A1 | 1.00 |
| P70T | 1.26 |
| A72E | 1.06 |
| G77S | 1.11 |
| G77P | 1.04 |
| E80D | 1.03 |
| Y81A | 1.00 |
| K83P | 1.00 |
| S84D | 1.05 |
| F88E | 1.10 |
| F113W | 1.09 |
| F113Y | 1.10 |
| D115Q | 1.04 |
| I157L | 1.37 |
| G161A | 1.20 |
| F162L | 1.09 |
| W187F | 1.05 |
| F200A | 1.12 |
| A204T | 1.14 |
| A204S | 1.09 |
| T210L | 1.15 |
| F211W | 1.11 |
| G226A | 1.06 |
| I228K | 1.00 |
| A233D | 1.09 |
| Y328F | 1.25 |
| L340V | 1.11 |
| V439P | 1.06 |

### Table 40-1

**Table 40-1**

| MUTATION | | | | | YIELD [%] |
|---|---|---|---|---|---|
| P70T | I157L | | | | 59.4 |
| P70T | T210L | | | | 56.4 |
| A72E | I157L | | | | 53.1 |
| A72E | Y328F | | | | 59.0 |
| G77M | I157L | | | | 44.1 |
| G77S | I157L | | | | 56.9 |
| G77S | Y328F | | | | 51.9 |
| E80D | I157L | | | | 54.2 |
| E80D | Y328F | | | | 54.6 |
| Y81A | I157L | | | | 56.9 |
| Y81A | Y328F | | | | 58.3 |
| S84D | I157L | | | | 55.7 |
| F88E | Y328F | | | | 58.1 |
| W107Y | Y328F | | | | 55.8 |
| F113W | I157L | | | | 56.3 |
| F113W | G161A | | | | 50.0 |
| I157L | G161A | | | | 58.5 |
| I157L | A184G | | | | 50.1 |
| I157L | W187F | | | | 57.7 |
| I157L | F200A | | | | 48.5 |
| I157L | A204S | | | | 53.7 |
| I157L | T210L | | | | 57.9 |
| I157L | G226A | | | | 56.8 |
| I157L | A233D | | | | 53.7 |
| I157L | Y328F | | | | 60.8 |
| I157L | L340V | | | | 59.4 |
| G161A | A204S | | | | 51.8 |
| G161A | T210L | | | | 54.2 |
| G161A | G226A | | | | 50.7 |
| G161A | Y328F | | | | 60.5 |
| A184G | W187F | | | | 53.5 |
| F200A | Y328F | | | | 50.0 |
| A204S | Y328F | | | | 59.2 |
| T210L | Y328F | | | | 56.6 |
| F211W | Y328F | | | | 52.5 |
| A233D | Y328F | | | | 57.7 |
| P70T | I157L | A204S | | | 58.5 |
| P70T | I157L | T210L | | | 64.7 |
| P70T | I157L | Y328F | | | 68.9 |
| P70T | G161A | Y328F | | | 64.8 |
| P70T | A184G | W187F | | | 47.5 |
| P70T | A204S | Y328F | | | 62.7 |
| A72E | I157L | Y328F | | | 62.9 |
| A72E | G161A | Y328F | | | 58.0 |
| A72E | A184G | Y328F | | | 48.5 |
| A72E | W187F | Y328F | | | 43.7 |
| A72E | F200A | Y328F | | | 43.5 |
| A72E | A204S | Y328F | | | 50.8 |
| A72E | G226A | Y328F | | | 51.2 |
| G77M | I157L | T210L | | | 43.9 |
| Y81 A | I157L | Y328F | | | 65.4 |
| Y81A | A184G | Y328F | | | 61.8 |
| Y81 A | F211W | Y328F | | | 58.0 |
| Y81A | G226A | Y328F | | | 55.5 |

### Table 40-2

**Table 40-2**

| MUTATION | | | | | YIELD [%] |
|---|---|---|---|---|---|
| S84D | I157L | T210L | | | 60.9 |
| F88E | I157L | T210L | | | 59.6 |
| F88E | I157L | Y328F | | | 64.9 |
| F113W | I157L | T210L | | | 57.3 |
| F113W | I157L | Y328F | | | 65.1 |
| F113Y | I157L | T210L | | | 58.8 |
| I157L | G161A | Y328F | | | 63.4 |
| I157L | A184G | W187F | | | 62.8 |
| I157L | A204S | Y328F | | | 61.2 |
| I157L | A204T | T210L | | | 59.9 |
| I157L | T210L | A233D | | | 59.2 |
| I157L | T210L | Y328F | | | 66.6 |
| I157L | A233D | Y328F | | | 65.0 |
| P70T | Y81A | I157L | Y328F | | 51.8 |
| A72E | Y81 H | I157L | Y328F | | 51.2 |
| Y81A | F88E | I157L | Y328F | | 49.3 |
| P70T | I157L | A204S | Y328F | | 64.5 |
| P70T | I157L | T210L | A233D | | 63.3 |
| P70T | I157L | T210L | Y328F | | 62.2 |
| Y81A | I157L | T210L | Y328F | | 67.6 |
| F88E | I157L | T210L | Y328F | | 61.1 |
| F113W | I157L | T210L | Y328F | | 68.0 |
| P70T | I157L | G226A | Y328F | | 66.9 |
| P70T | I157L | A233D | Y328F | | 66.8 |
| A72E | I157L | A233D | Y328F | | 58.4 |
| Y81A | I157L | A233D | Y328F | | 67.6 |
| P70T | I157L | Y328F | V439P | | 72.6 |
| I157L | G161A | T210L | Y328F | | 68.5 |
| P70T | G161A | A233D | Y328F | | 65.4 |
| I157L | G161A | A233D | Y328F | | 66.8 |
| I157L | A184G | W187F | T210L | | 55.9 |
| I157L | A184G | W187F | Y328F | | 69.7 |
| I157L | T210L | A233D | Y328F | | 66.4 |
| A72E | K83P | I157L | Y328F | | 52.6 |
| P70T | I157L | W187F | T210L | Y328F | 42.9 |
| Y81A | I157L | A184G | W187F | Y328F | 60.4 |
| Y81A | I157L | T210L | A233D | Y328F | 64.9 |
| I157L | A184G | W187F | T210L | Y328F | 63.2 |

### (Example 23)

### (F1) Production of dipeptide using rational mutations

The strains obtained in Example 22 (A1, A1/I157L, A1/G161A, A1/Y328F) were cultured by the method described in Example 6 (25). The cultured broth (5 µL or 10 µL) was added to 200 µL of borate buffer (pH 9.0) containing 50 mM Ala-OMe HCl, 100 mM L-amino acid and 10 mM EDTA, and reacted at 20°C for 30 minutes. The concentrations of dipeptides (Ala-X) synthesized 5, 10 and 30 minutes after the start of the reaction are shown in Table 41

### Table 41

**Table 41**

| | Reaction time [min] | SYNTHESIZED DIPEPTIDE CONCENTRATION [mM] | | | | | |
|---|---|---|---|---|---|---|---|
| | | Ala-Asp | Ala-Gln | Ala-Thr | Ala-Gly | Ala-Val | Ala-Ala |
| M35-4+V184A | 5 | 1.0 | 24.4 | 17.0 | 4.7 | 4.3 | 10.8 |
| | 10 | 1.6 | 28.8 | 22.5 | 6.3 | 7.5 | 12.3 |
| | 30 | 1.7 | 27.7 | 23.2 | 7.7 | 9.1 | 11.2 |
| M35-4/V184A/I157L | 5 | 0.4 | 17.6 | 11.9 | 4.1 | 3.5 | 7.9 |
| | 10 | 0.9 | 26.6 | 19.2 | 6.6 | 6.2 | 12.9 |
| | 30 | 1.6 | 31.5 | 24.2 | 9.2 | 9.3 | 16.2 |
| M35-4/V184A/G161A | 5 | 0.6 | 7.5 | 8.4 | 3.2 | 3.0 | 5.3 |
| | 10 | 1.2 | 14.3 | 14.2 | 5.5 | 5.1 | 8.9 |
| | 30 | 2.3 | 25.5 | 28.1 | 8.4 | 10.0 | 14.8 |
| M35-4/V184A/Y328F | 5 | 2.1 | 27.7 | 25.3 | 9.5 | 8.0 | 13.8 |
| | 10 | 3.2 | 33.3 | 30.2 | 11.7 | 11.3 | 17.8 |
| | 30 | 3.3 | 32.0 | 28.8 | 11.4 | 13.4 | 16.1 |

| | | | | | | | |
|---|---|---|---|---|---|---|---|
| substrate 50mM AlaOMe + 100mM X | | | | | | | |

### (Example 24) Construction of strains having high activity by combining mutations

### (F2) Construction of strains having combined mutation points by random screening

In order to construct strains having various combinations of mutation points, pSF_Sm_M35-4/V184A/I157L (A1/I157L) was used as the template of the site-directed mutagenesis using PCR.
The mutations were introduced by the same method as in Example 7 (29) using the primers (SEQ ID NOS:193, 195 to 198) corresponding to various enzymes to yield the library of the strains having the random combination.

### (F3) Screening of library having combined mutations

The library made in (F2) was cultured by the same method as in Example 3 (9). Using the cultured solution, two screenings for selection were performed (see the following (F4) and (F5)).

### (F4) Primary screening: A

The reaction solution (200 µL) (pH 8.2) containing 10 mM phenol, 6 mM AP, 5 mM Asp(OMe)₂, 30 mM Phe, 6.12 U/mL of peroxidase, 0.21 U/mL of alcohol oxidase, 10 mM EDTA and 100 mM borate was added to 5 µL of a resulting microbial solution, reacted at 25°C for about 20 minutes, and subsequently absorbance at 500 nm was measured to calculate the released amount of methanol.

### (F5) Primary screening: B

The reaction solution (200 µL) (pH 8.2) containing 10 mM phenol, 6 mM AP, 5 mM Asp(OMe)₂, 5 mM Ala-OEt, 30 mM Phe, 6.12 U/mL of peroxidase, 0.21 U/mL of alcohol oxidase, 10 mM EDTA and 100 mM borate was added to 5 µL of the resulting microbial solution, reacted at 25°C for about 20 minutes, and subsequently the absorbance at 500 nm was measured to calculate the released amount of methanol.
Both (F4) and (F5) were performed. Those having a larger value of (F4)/(F5) than that of the mother strain (A1+I157L) were selected as the enzymes which tend to produce AMP rather than Ala-Phe.

### (F6) Secondary screening

The strains screened and selected by the aforementioned primary screenings were cultured by the method described in Example 6 (25). The cultured broth (2 U) was suspended in 100 mM borate buffer (pH 8.5) containing 10 mM EDTA, 50 mM Asp(OMe)₂, and 75 mM Phe such that the final volume was 1 mL, and the amount of produced AMP was measured at 20°C. The strains which produced AMP abundantly were selected. The combination of the mutation points was specified by sequencing in the selected strains, and their mutation points are described in Table 34. The selected strain was described as F22, and the amounts of produced AMP in F22 are in shown in Table 42.

### Table 42

**Table 42**

| | AMP [mM] | |
|---|---|---|
| | 25 MIN | 50 MIN |
| A1/I157L | 25.4 | 24.2 |
| F22 | 18.2 | 30.3 |

### (F7) Combination with rational mutant strains

The mutation points Y328F, Y81A, and T210L which exhibited effect in Example 22 were introduced into F22 strain. The mutation was introduced by the same method as in (45) using the primers (SEQ ID NOS:201 to 206) corresponding to various mutant enzymes. The resulting strains were cultured by the method described in Example 6 (25). The cultured broth was suspended in the solution (18 U/mL reaction solution) containing 400 mM Asp(OMe)₂ monomethyl sulfate and 400 mM Phe, and reacted at 22°C with keeping pH 8.5 using NH₄OH. The yield of produced AMP was measured. The AMP yield in this reaction is shown in Table 43.

### Table 43

**Table 43**

| | AMP YIELD [%] | | | | | | |
|---|---|---|---|---|---|---|---|
| | 0 MIN | 10 min | 20 min | 30 min | 40 min | 60 min | 80 min |
| A1/I157L | 0 | 42.2 | 55.5 | 59.2 | 58.5 | 58.6 | 56.1 |
| F22 | 0 | 55.0 | 66.3 | 68.5 | 63.1 | 67.3 | 65.1 |
| F22/Y328F | 0 | 70.1 | 79.2 | 80.0 | 79.9 | 80.9 | 75.6 |
| F22/Y328F/Y81A | 0 | 69.4 | 84.2 | 85.6 | 84.9 | 82.7 | 79.7 |
| F22/Y328F/T210L | 0 | 65.9 | 86.6 | 85.7 | 84.9 | 86.3 | 69.4 |
| | | | | | | | |

| Strain | MUTATED PART | | | | | | |
|---|---|---|---|---|---|---|---|
| F22 Y328F | A1 L66F/E80K/I157L/A182G/P214H/L263M/Y328F | | | | | | |
| F22 Y328F/Y81A | A1 L66FIY81A/I157L/A182G/P214H/L263M/Y328F | | | | | | |
| F22 Y328F/T210L | A1 L66F/E80K/I157L/A182G/T210L/L263M/Y328F | | | | | | |

<List of abbreviations>
Asp(OMe)₂•HCl: L-aspartic acid-α,β-dimethyl ester hydrochloric acid
Ala-OEt: L-alanine ethyl ester
Ala-OMe: L-alanine methyl ester
Tyr-OMe: L-tyrosine methyl ester
Gly-OMe: glycine methyl ester
Phe-OMe: L-phenylalanine methyl ester
AMP: α-L-aspartyl-L-phenylalanine-β-ester
Ala-Gln: L-alanyl-L-glutamine
Ala-Phe: L-alanyl-L-phenylalanine
Phe-Met: L-phenylalanyl-L-methionine
Leu-Met: L-leucyl-L-methionine
Ile-Met: L-isoleucyl-L-methionine
Met-Met: L-methionyl-L-methionine
Pro-Met: L-prolyl-L-methionine
Trp-Met: L-tryptophyl-L-methionine
Val-Met: L-valyl-L-methionine
Asn-Met: L-asparaginyl-L-methionine
Cys-Met: L-cysteinyl-L-methionine
Gln-Met: L-glutaminyl-L-methionine
Gly-Met: glycyl-L-methionine
Ser-Met: L-seryl-L-methionine
Thr-Met: L-threonyl-L-methionine
Tyr-Met: L-tyrosyl-L-methionine
Asp-Met: L-aspartyl-L-methionine
Arg-Met: L-arginyl-L-methionine
His-Met: L-histidyl-L-methionine
Lys-Met: L-lysyl-L-methionine
Ala-Gly: L-alanyl-glycine
Ala-Thr: L-alanyl-L-threonine
Ala-Glu: L-alanyl*-*L-Glutamic acid
Ala-Ala: L-alanyl-L-alanine
Ala-Asp: L-alanyl-L-aspartic acid
Ala-Ser: L-alanyl-L-serine
Ala-Met: L-alanyl-L-methionine
Ala-Val: L-alanyl-L-valine
Ala-Lys: L-alanyl-L-lysine
Ala-Asn: L-alanyl-L-asparagine
Ala-Cys: L-alanyl-L-cysteine
Ala-Tyr: L-alanyl-L-tyrosine
Ala-Ile: L-alanyl-L-isoleucine
Arg-Gln: L-arginyl-L-glutamine
Gly-Ser: glycyl-L-serine
Gly-Ser(tBu): glycyl-L-(t-butyl)serine
HIL-Phe: (2S,3R,4S)-4-hydroxylisoleucyl-phenylalanine
AFA: L-alanyl-L-phenylalanyl-L-alanine
AGA: L-alanyl-glycyl-L-alanine
AHA: L-alanyl-L-histidyl-L-alanine
ALA: L-alanyl-L-leucyl-L-alanine
AAA: L-alanyl-L-alanyl-L-alanine
AAG: L-alanyl-L-alanyl-glycine
AAP: L-alanyl-L-alanyl-L-proline
AAQ: L-alanyl-L-alanyl-L-glutamine
AAY: L-alanyl-L-alanyl-L-tyrosine
GFA: glycyl-L-phenylalanyl-L-alanine
AGG: L-alanyl-glycyl-glycine
TGG: L-threonyl-glycyl-glycine
GGG: glycyl-glycyl-glycine
AFG: L-alanyl-L-phenylalanyl-glycine
GGFM: glycyl-glycyl-L-phenylalanyl-L-methionine
YGGFM: L-tyrosyl-glycyl-glycyl-L-phenylalanyl-L-methionine
AM: L-aspartic acid-β-methyl ester hydrochloric acid
AM(AM): L-aspartyl-L-aspartic acid-β,β-dimethyl ester
AP : 4-aminoantipyrine
OPT: 1,10-Phenanthoroline monohydrate

Single character codes of the amino acids at mutated positions and the codons used which correspond to the mutation introduction into the amino acid residues in the present specification are as shown in Table 44.

### Table 44

**Table 44**

| AMINO ACID RESIDUE | | CODON USED | |
|---|---|---|---|
| | | Forward | Reverse |
| Ala | A | GCT | AGC |
| Cys | C | TGC | GCA |
| Asp | D | GAC | GTC |
| Glu | E | GAA | TTC |
| Phe | F | TTC | GAA |
| Gly | G | GGT | ACC |
| His | H | CAC | GTG |
| Ile | I | ATC | GAT |
| Lys | K | AAA | TTT |
| Leu | L | CTG | CAG |
| Met | M | ATG | CAT |
| Asn | N | AAC | GTT |
| Pro | P | CCG | CGG |
| Gln | Q | CAG | CTG |
| Arg | R | CGT | ACG |
| Ser | S | TCT | AGA |
| Thr | T | ACC | GGT |
| Val | V | GTT | AAC |
| Trp | W | TGG | CCA |
| Tyr | Y | TAC | GTA |

[Sequence List Free Text]

List of primer sequences

### Table 45-1

**Table 45-1: PRIMER LIST (No. IN THE LIST INDICATES SEQUENCE NUMBER)**

| No. | Name | Sequence |
|---|---|---|
| 3 | 2458 EcoRl-S | CGCGAATTCATGAAAAATACAATTTCGTGC |
| 4 | 2458 Pstl-AS | CGCCTGCAGCTAATCTTTGAGGACAGAAAATTC |
| 5 | 2458 Ndel F | GGGAATTCCATATGAAAAATACAATTTCGT |
| 6 | 2458 Xbal R | GCTCTAGACTAATCTTTGAGGACAGAAAA |
| 7 | 2458 Check F2 | TGCTCAATAGAACGCCCTA |
| 8 | 2458 Check F3 | CCGAGCTTGAAGGCAGTCT |
| 9 | 2458 Check F4 | ACGCGGAAGATGCTTATGG |
| 10 | 2458 Check F5 | AAGTTCAACGTACAGATT |
| 11 | 2458 Check R4 | GGTATCCGTACTTTCATCGA |

### Table 45-2

**Table 45-2: PRIMER LIST (No. IN THE LIST INDICATES SEQUENCE NUMBER)**

| No. | INTRODUCED MUTATION | Sequence |
|---|---|---|
| 12 | S209D | GCA TTT ACA TTC ATG GAC ACC TTT GGT GTC CCT CG |
| 13 | Q441E | CAA GGT GGG TTA ATT GAA AAC CGA ACA CGG GAG |
| 14 | Q441K | CAA GGT GGG TTA ATT AAA AAC CGA ACA CGG GAG |
| 15 | N442K | GGT GGG TTA ATT CAA AAA CGA ACA CGG GAG TAT ATG |
| 16 | R445D | CAA AAC CGA ACA GAG GAG TAT ATG GTA GAT G |
| 17 | R445F | CAA AAC CGA ACA TTT GAG TAT ATG GTA GAT G |
| 18 | D203N | GTA TTG TTT CTT CAG AAT GCA TTT ACA TTC ATG |
| 19 | D203S | GTA TTG TTT CTT CAG TCT GCA TTT ACA TTC ATG |
| 20 | F207A | CAG GAT GCA TTT ACA GCC ATG TCA ACC TTT GGT G |
| 21 | F207S | CAG GA T GCA TTT ACA TCC ATG TCA ACC TTT GGT G |
| 22 | S209A | GCA TTT ACA TTC ATG GCA ACC TTT GGT GTC CCT C |
| 23 | Q441 N | CAA GGT GGG TTA ATT AAC AAC CGA ACA CGG GAG |
| 24 | Q441 D | CAA GGT GGG TTA ATT GAC AAC CGA ACA CGG GAG |
| 25 | K83A | CAG AAC GAA TAC AAA GCA AGT TTG GGA AAC |
| 26 | F207V | CAG GAT GCA TTT ACA GTC ATG TCA ACC TTT GGT G |
| 27 | F207G | CAG GAT GCA TTT ACA GGC ATG TCA ACC TTT GGT G |
| 28 | F207T | CAG GAT GCA TTT ACA ACC ATG TCA ACC TTT GGT G |
| 29 | M208A | GAT GCA TTT ACA TTC GCG TCA ACC TTT GGT GTC |
| 30 | S209G | GCA TTT ACA TTC ATG GGA AC C TTT GGT GTC CC |
| 31 | F207I | CAG GAT GCA TTT ACA ATC ATG TCA ACC TTT GGT G |
| 32 | R117A | GATTTTGAAGATATAGCTCCGACCACGTACAGC |
| 33 | F207V/S209A | CAG GAT GCA TTT ACA GTC ATG GCA ACC TTT GGT G |
| 34 | L439V | CAA GGT GGG GTA ATT CAA AAC |
| 35 | A537G | CGA TAA AGG GCA GGC CTT G |
| 36 | A301V | GCG GAA GAT GTT TAT GGA AC |
| 37 | G226S | CAA TTT AAG AGC AAA ATT C |
| 38 | V257I | GGT GAC TCC ATA CAA TTT TG |
| 39 | D619E | TTT CTG TCC TCA AA G AAT AG |
| 40 | Y339H | GAA GGA AAC CAT TTA GGT G |
| 41 | N607K | CAC GAT GTG AAG AAT GCC AC |
| 42 | A324V | TTT TAG TC G TG G GAC CTT G |
| 43 | Q229H | GCA AAA TT C AT A TCA AAG AAG |
| 44 | W327G | GCG GGA CCT GGG TAT CAT G |

### Table 45-3

**Table 45-3: PRIMER LIST (No. IN THE LIST INDICATES SEQUENCE NUMBER)**

| | Name | | Sequence |
|---|---|---|---|
| 45 | F207V F | | CAGGATGCATTTACAGTCATGTCAACCTTTGGTG |
| 46 | F207V R | | CACCAAAGGTTGACATGACTGTAAATGCATCCTG |
| 47 | 2458 K83A F | | GAACGAATACAAAGCAAGTTTGGGAAAC |
| 48 | 2458 K83A R | | GTTTCCCAAACTTGCTTTGTATTCGTTC |
| 49 | 2458 Q229H F | | GGGCAAAATTCATATCAAAGAAGCCG |
| 50 | 2458 Q229H R | | CGGCTTCTTTGATATGAATTTTGCCC |
| 51 | 2458 V257I F | | CTTTGGTGACTCCATACAATTTTGG |
| 52 | 2458 V257I R | | CCAAAATTGTATGGAGTCACCAAAG |
| 53 | 2458 A301V F | | GACGCGGAAGATGTTTATGGAACATTT |
| 54 | 2458 A301V R | | AAATGTTCCATAAACATCTTCCGCGTC |
| 55 | 2458 D313E F | | CCAATCGATTGAGGAAAAAAGCAAAAAAAAC |
| 56 | 2458 D313E R | | GTTTTTTTTGCTTTTTTCCTCAATCGATTGG |
| 57 | 2458 A324V F | | CTCGATTTTAGTCGTGGGACCTTGGTATC |
| 58 | 2458 A324V R | | GATACCAAGGTCCCACGACTAAAATCGAG |
| 59 | 2458 L439V F | | GCATCAAGGTGGGGTAATTCAAAACCG |
| 60 | 2458 L439V R | | CGGTTTTGAATTACCCCACCTTGATGC |
| 61 | 2458 Q441 E F | | GGTGGGTTAATTGAAAACCGAACAC |
| 62 | 2458 Q441E R | | GTGTTCGGTTTTCAATTAACCCACC |
| 63 | 2458 A537G F | | GGTTTCGATAAAGGGCAGGCCTTGAC |
| 64 | 2458 A537G R | | GTCAAGGCCTGCCCTTTATCGAAACC |
| 65 | 2458 N607K F | | CACGATGTGAAGAATGCCACATACATCG |
| 66 | 2458 N607K R | | CGATGTATGTGGCATTCTTCACATCGTG |
| 67 | T72A F | | GAACGCCCTACGCGGTTTCTCC |
| 68 | T72A R | | GGAGAAACCGCGTAGGGCGTTC |
| 69 | A137S F | | CGGATACCTATGATTCGCTTGAATGGTTAC |
| 70 | A137S R | | GTAACCATTCAAGCGAATCATAGGTATCCG |
| 71 | E551 K S | | AAG GTG AAT TTT AAA ATG CCA GAC GTT GCG |
| 72 | E551 K AS | | CGC AAC GTC TGG CAT TTT AAA ATT CAC CTT |
| 73 | M208A S | | catttacattcgcgtcaacctttggtgtcc |
| 74 | M208A AS | | ggacaccaaaggttgacgcgaatgtaaatg |
| 75 | 2458 G226S F | | CGGATCAATTTAAGAGCAAAATTCAG |
| 76 | 2458 G226S R | | CTGAATTTTGCTCTTAAATTGATCCG |
| 77 | F207H S | | aggatgcatttacacacatgtcaacctttg |
| 78 | F207H AS | | caaaggttgacatgtgtgtaaatgcatcct |

### Table 45-4

**Table 45-4: PRIMER LIST (No. in the list indicates sequence number)**

| No. | Name | MUTATION | Sequence |
|---|---|---|---|
| 79 | 2458 V184A F | V184A | CACAGGCTCCCGCAACAGACTGGTATATC |
| 80 | 2458 V184A R | | GATATACCAGTCTGTTGCGGGAGCCTGTG |
| 81 | 2458 V184C F | V184C | CACAGGCTCCCTGCACAGACTGGTATATC |
| 82 | 2458 V184C R | | GATATACCAGTCTGTGCAGGGAGCCTGTG |
| 83 | 2458 V184G F | V184G | CACAGGCTCCCGGCACAGACTGGTATATC |
| 84 | 2458 V184G R | | GATATACCAGTCTGTGCCGGGAGCCTGTG |
| 85 | 2458 V184I F | V184I | CACAGGCTCCCATTACAGACTGGTATATC |
| 86 | 2458 V184I R | | GATATACCAGTCTGTAATGGGAGCCTGTG |
| 87 | 2458 V184L F | V184L | CACAGGCTCCCCTAACAGACTGGTATATC |
| 88 | 2458 V184L R | | GATATACCAGTCTGTTAGGGGAGCCTGTG |
| 89 | 2458 V184M F | V184M | CACAGGCTCCCATGACAGACTGGTATATC |
| 90 | 2458 V184M R | | GATATACCAGTCTGTCATGGGAGCCTGTG |
| 91 | 2458 V184N F | V184N | CACAGGCTCCCAACACAGACTGGTATATC |
| 92 | 2458 V184N R | | GATATACCAGTCTGTGTTGGGAGCCTGTG |
| 93 | 2458 V184P F | V184P | CACAGGCTCCCCAACAGACTGGTATATC |
| 94 | 2458 V184P R | | GATATACCAGTCTGTTGGGGGAGCCTGTG |
| 95 | 2458 V184S F | V184S | CACAGGCTCCCTCAACAGACTGGTATATC |
| 96 | 2458 V184S R | | GATATACCAGTCTGTTGAGGGAGCCTGTG |
| 97 | 2458 V184T F | V184T | CACAGGCTCCCACAACAGACTGGTATATC |
| 98 | 2458 V184T R | | GATATACCAGTCTGTTGTGGGAGCCTGTG |

### Table 45-5

**Table 45-5: PRIMER LIST (No. IN THE LIST INDICATES SEQUENCE NUMBER)**

| No. | Name | Sequence |
|---|---|---|
| 99 | 2458 | GAACGAATACAAAGCAAGTTTGGGAAAC |
| | K83A F | |
| 100 | 2458 | GGGCAAAATTCATATCAAAGAAGCCG |
| | Q229H F | |
| 101 | 2458 | CTTTGGTGACTCCATACAATTTTGG |
| | V2571 F | |
| 102 | 2458 | GACGCGGAAGATGTTTATGGAACATTT |
| | A301V F | |
| 103 | 2458 | CCAATCGATTGAGGAAAAAAGCAAAAAAAAC |
| | D313E F | |
| 104 | 2458 | CTCGATTTTAGTCGTGGGACCTTGGTATC |
| | A324V F | |
| 105 | 2458 | GCATCAAGGTGGGGTAATTCAAAACCG |
| | L439V F | |
| 106 | 2458 | GGTGGGTTAATTGAAAACCGAACAC |
| | Q441E F | |
| 107 | 2458 | GGTTTCGATAAAGGGCAGGCCTTGAC |
| | A537G F | |
| 108 | 2458 | CACGATGTGAAGAATGCCACATACATCG |
| | N607K F | |
| 109 | T72 A F | GAACGCCCTACGCGGTTTCTCC |
| 110 | A137S F | CGGATACCTATGATTCGCTTGAATGGTTAC |
| 111 | Q229X F | GGGCAAAATTNNNATCAAAGAAGCCG |
| 112 | I228X F + Q229P F | CAATTTAAGGGCAAANNNCCTATCAAAGAAGCCG |
| 113 | I230X F + Q229P F | GGGCAAAATTCCTNNNAAAGAAGCCG |
| 114 | I228X F + Q229H F | CAATTTAAGGGCAAANNNCATATCAAAGAAGCCG |
| 115 | S256X F + V257I F | CTTTGGTGACNNNATACAATTTTGGAATG |
| 116 | A137X F | CGGATACCTATGATNNNCTTGAATGGTTAC |
| 117 | 2458 | GGGCAAAATTCCTATCAAAGAAGCCG |
| | Q229P F | |
| 118 | A324X F | CAACTCGATTTTAGTCNNNGGACCTTGGTATC |
| 119 | A301X F | CTTTGACGCGGAAGATNNNTATGGAACATTTAAG |
| 120 | A537X F | GAAATGGTTTCGATAAANNNCAGGCCTTGACTCC |

### Table 45-6

**Table 45-6: PRIMER LIST (No. IN THE LIST INDICATES SEQUENCE NUMBER)**

| No. | Name | Sequence |
|---|---|---|
| 121 | Esp-S1 | CCGTAAGGAGGAATGTAGATGAAAAATACAATTTCGTGCC |
| 122 | S-AS1 | GGC TGC AGT TTG CGG GAT GGA AGG CCG GC |
| 123 | E-S1 | CCT CTA GAA TTT TTT CAA TGT GAT TT |
| 124 | Esp-AS1 | GCAGGAAATTGTATTTTTCATCTACATTCCTCCTTACGGTGTTAT |
| 125 | EM1 | CTT ACA GAT GAC TAT AAT GTG ACT AAA AAC |
| 126 | EMR1 | GTT TTT AGT CAC ATT ATA GTC ATC TGT AAG |

### Table 45-7

**Table 45-7: PRIMER LIST (No. IN THE LIST INDICATES SEQUENCE NUMBER)**

| No. | Name | Sequence |
|---|---|---|
| 127 | pSFNde-cut-1 | cggtatttcacaccgcgtatggtgcactctcagtac |
| 128 | pSFNde-cut-2 | gtactgagagtgcaccatacgcggtgtgaaataccg |
| 129 | pSFNde-1 | ccgtaaggaggaatgcatatgaaaaatacaatttcg |
| 130 | pSFNde-2 | cgaaattgtatttttcatatgcattcctccttacgg |
| 131 | W187A/F | GCT CCC GTA ACA GAC GCG TAT ATC GGC GAC GAC |
| 132 | S209A/F | GCA TTT ACA TTC ATG GCA ACC TTT GGT GTC CCT C |
| 133 | S209G/F | GCA TTT ACA TTC ATG GGA ACC TTT GGT GTC CC |
| 134 | F211A/F | GCA TTT ACA TTC ATG TCA ACC GCT GGT GTC CCT CGT CC |
| 135 | T210K/F | GCA TTT ACA TTC ATG TCA AAG TTT GGT GTC CCT CG |
| 136 | N442D/F | GGT GGG TTA ATT CAA GAC CGA ACA CGG GAG TAT ATG |
| 137 | F211V/F | GCA TTT ACA TTC ATG TCA ACC GTT GGT GTC CCT CGT CC |
| 138 | 2458/V257A/F | CTTTGGTGACTCCGCACAATTTTGGAATG |
| 139 | 2458/V257A/R | CATTCCAAAATTGTGCGGAGTCACCAAAG |
| 140 | 2458/V257G/F | CTTTGGTGACTCCGGACAATTTTGGAATG |
| 141 | 2458/V257G/R | CATTCCAAAATTGTCCGGAGTCACCAAAG |
| 142 | 2458/V257H/F | CTTTGGTGACTCCCACCAATTTTGGAATG |
| 143 | 2458/V257H/R | CATTCCAAAATTGGTGGGAGTCACCAAAG |
| 144 | 2458/V257M/F | CTTTGGTGACTCCATGCAATTTTGGAATG |
| 145 | 2458/V257M/R | CATTCCAAAATTGCATGGAGTCACCAAAG |
| 146 | 2458/V257N/F | CTTTGGTGACTCCAACCAATTTTGGAATG |
| 147 | 2458/V257N/R | CATTCCAAAATTGGTTGGAGTCACCAAAG |
| 148 | 2458/V257Q/F | CTTTGGTGACTCCCAACAATTTTGGAATG |
| 149 | 2458/V257Q/R | CATTCCAAAATTGTTGGGAGTCACCAAAG |
| 150 | 2458/V257S/ F | CTTTGGTGACTCCTCACAATTTTGGAATG |
| 151 | 2458/V257S/R | CATTCCAAAATTGTGAGGAGTCACCAAAG |
| 152 | 2458/V257T/F | CTTTGGTGACTCCACACAATTTTGGAATG |
| 153 | 2458/V257T/R | CATTCCAAAATTGTGTGGAGTCACCAAAG |
| 154 | 2458/V257W/F | CTTTGGTGACTCCTGGCAATTTTGGAATG |
| 155 | 2458/V257W/R | CATTCCAAAATTGCCAGGAGTCACCAAAG |
| 156 | 2458/V257Y/F | CTTTGGTGACTCCTACCAATTTTGGAATG |

### Table 45-8

**Table 45-8: PRIMER LIST (No. IN THE LIST INDICATES SEQUENCE NUMBER)**

| No. | Name | Sequence |
|---|---|---|
| 157 | 2458/V257Y/R | CATTCCAAAATTGGTAGGAGTCACCAAAG |
| 158 | W187A/R | GTCGTCGCCGATATACGCGTCTGTTACGGGAGC |
| 159 | F211A/R | GGACGAGGGACACCAGCGGTTGACATGAATGTAAATGC |
| 160 | K47G/F | atgcgagatgggaaaggtttatttactgcgatc |
| 161 | K47G/R | gatcgcagtaaataaacctttcccatctcgca |
| 162 | K47E/F | atgcgagatgggaaagaattatttactgcgatc |
| 163 | K47E/R | gatcgcagtaaataattctttcccatctcgca |
| 164 | N442F/F | ggtgggttaattcaattccgaacacgggagtat |
| 165 | N442F/R | atactcccgtgttcggaattgaattaacccac |
| 166 | N607R/F | atttttcacgatgtgcgtaatgccacatacatc |
| 167 | N607R/R | gatgtatgtggcattacgcacatcgtgaaaaat |
| 168 | V184A+W187A/F | gctcccgcaacagacgcgtatatcggcgacgac |
| 169 | V184A+W187A/R | gtcgtcgccgatatacgcgtctgttgcgggagc |
| 170 | Q441K/R | gtgttcggtttttaattaacccacc |
| 171 | V184A/P183A F | CCCCACAGGCTGCAGCAACAGACTGG |
| 172 | V184A/P183AR | CCAGTCTGTTGCTGCAGCCTGTGGGG |
| 173 | V184A/T185A F | CAGGCTCCCGCAGCAGACTGGTATATC |
| 174 | V184A/T185A R | GATATACCAGTCTGCTGCGGGAGCCTG |
| 175 | V184A/T185N F | CAGGCTCCCGCAAACGACTGGTATATC |
| 176 | V184A/T185N R | GATATACCAGTCGTTTGCGGGAGCCTG |
| 177 | V184A/T185K F | CAGGCTCCCGCAAAAGACTGGTATATC |
| 178 | V184A/T185K R | GATATACCAGTCTTTTGCGGGAGCCTG |
| 179 | V184A/T185D F | CAGGCTCCCGCAGATGACTGGTATATC |
| 180 | V184A/T185D R | GATATACCAGTCATCTGCGGGAGCCTG |
| 181 | V184A/T185C F | CAGGCTCCCGCATGCGACTGGTATATC |
| 182 | V184A/T185C R | GATATACCAGTCGCATGCGGGAGCCTG |
| 183 | V184A/T185S F | CAGGCTCCCGCATCAGACTGGTATATC |
| 184 | V184A/T185S R | GATATACCAGTCTGATGCGGGAGCCTG |
| 185 | V184A/T185F F | CAGGCTCCCGCATTTGACTGGTATATC |
| 186 | V184A/T185F R | GATATACCAGTCAAATGCGGGAGCCTG |
| 187 | V184A/T185P F | CAGGCTCCCGCACCAGACTGGTATATC |
| 188 | V184A/T185P R | GATATACCAGTCTGGTGCGGGAGCCTG |

### Table 45-9

**Table 45-9: PRIMER LIST (No. IN THE LIST INDICATES SEQUENCE NUMBER)**

| No. | Name | Sequence |
|---|---|---|
| 189 | V184A/P183A/A182S F | GTCTCCCCACAGTCAGCAGCAACAGAC |
| 190 | V184A/P183A/A182S R | GTCTGTTGCTGCTGACTGTGGGGAGAC |
| 191 | V184A/P183A/A182G F | GTCTCCCCACAGGGTGCAGCAACAGAC |
| 192 | V184A/P183A /A182G R | GTCTGTTGCTGCACCCTGTGGGGAGAC |
| 193 | V184A/A182G F | CTCCCCACAGGGTCCCGCAACAG |
| 194 | V184A/A182GR | CTGTTGCGGGACCCTGTGGGGAG |
| 195 | L66F | CCAGTTTTGTTCAATAGAACGCC |
| 196 | E80K | CCTTATGGGCAGAACAAATACAAAAAAAG |
| 197 | P214H | CTTTGGTGTCCATCGTCCAAAACC |
| 198 | L263M | CAATTTTGGAATGACATGTTTAAGCATCC |
| 199 | Q441E+N442D/F | CAAGGTGGGTTAATTGAAGACCGAACACGGGAG |
| 200 | Q441E+N442D/R | CTCCCGTGTTCGGTCTTCAATTAACCCACCTTG |
| 201 | Y81A-F | TAT GGG CAG AAC GAA GCT AAA AAA AGT TTG GGA |
| 202 | Y81A-R | TCC CAA ACT TTT TTT AGC TTC GTT CTG CCC ATA |
| 203 | T210L-F | TTT ACA TTC ATG TCA CTG TTT GGT GTC CCT CGT |
| 204 | T210L-R | ACG AGG GAC ACC AAA CAG TGA CAT GAA TGT AAA |
| 205 | Y328F-F | GTC GTG GGA CCT TGG TTC CAT GGC GGC TGG GTT |
| 206 | Y328F-R | AAC CCA GCC GCC ATG GAA CCA AGG TCC CAC GAC |

### Table 46-1

**Table 46-1**

| RESIDUE | Forward PRIMER | Reverse PRIMER |
|---|---|---|
| N67 | TAT CCA GTT TTG CTC XXX AGA ACG CCC TAC GCG | CGC GTA GGG CGT TCT XXX GAG CAA AAC TGG ATA |
| R68 | CCA GTT TTG CTC AAT XXX ACG CCC TAC GCG GTT | AAC CGC GTA GGG CGT XXX ATT GAG CAA AAC TGG |
| T69 | GTT TTG CTC AAT AGA XXX CCC TAC GCG GTT TCT | AGA AAC CGC GTA GGG XXX TCT ATT GAG CAA AAC |
| P70 | TTG CTC AAT AGA ACG XXX TAC GCG GTT TCT CCT | AGG AGA AAC CGC GTA XXX CGT TCT ATT GAG CAA |
| Y71 | CTC AAT AGA ACG CCC XXX GCG GTT TCT CCT TAT | ATA AGG AGA AAC CGC XXX GGG CGT TCT ATT GAG |
| A72 | AAT AGA ACG CCC TAC XXX GTT TCT CCT TAT GGG | CCC ATA AGG AGA AAC XXX GTA GGG CGT TCT ATT |
| V73 | AGA ACG CCC TAC GCG XXX TCT CCT TAT GGG CAG | CTG CCC ATA AGG AGA XXX CGC GTA GGG CGT TCT |
| S74 | ACG CCC TAC GCG GTT XXX CCT TAT GGG CAG AAC | GTT CTG CCC ATA AGG XXX AAC CGC GTA GGG CGT |
| P75 | CCC TAC GCG GTT TCT XXX TAT GGG CAG AAC GAA | TTC GTT CTG CCC ATA XXX AGA AAC CGC GTA GGG |
| Y76 | TAC GCG GTT TCT CCT XXX GGG CAG AAC GAA TAC | GTA TTC GTT CTG CCC XXX AGG AGA AAC CGC GTA |
| G77 | GCG GTT TCT CCT TAT XXX CAG AAC GAA TAC AAA | TTT GTA TTC GTT CTG XXX ATA AGG AGA AAC CGC |
| Q78 | GTT TCT CCT TAT GGG XXX AAC GAA TAC AAA AAA | TTT TTT GTA TTC GTT XXX CCC ATA AGG AGA AAC |
| N79 | TCT CCT TAT GGG CAG XXX GAA TAC AAA AAA AGT | ACT TTT TTT GTA TTC XXX CTG CCC ATA AGG AGA |
| E80 | CCT TAT GGG CAG AAC XXX TAC AAA AAA AGT TTG | CAA ACT TTT TTT GTA XXX GTT CTG CCC ATA AGG |
| Y81 | TAT GGG CAG AAC GAA XXX AAA AAA AGT TTG GGA | TCC CAA ACT TTT TTT XXX TTC GTT CTG CCC ATA |
| K82 | GGG CAG AAC GAA TAC XXX AAA AGT TTG GGA AAC | GTT TCC CAA ACT TTT XXX GTA TTC GTT CTG CCC |
| K83 | CAG AAC GAA TAC AAA XXX AGT TTG GGA AAC TTT | AAA GTT TCC CAA ACT XXX TTT GTA TTC GTT CTG |
| S84 | AAC GAA TAC AAA AAA XXX TTG GGA AAC TTT CCC | GGG AAA GTT TCC CAA XXX TTT TTT GTA TTC GTT |
| L85 | GAA TAC AAA AAA AGT XXX GGA AAC TTT CCC CAA | TTG GGG AAA GTT TCC XXX ACT TTT TTT GTA TTC |
| G86 | TAC AAA AAA AGT TTG XXX AAC TTT CCC CAA ATG | CAT TTG GGG AAA GTT XXX CAA ACT TTT TTT GTA |
| N87 | AAA AAA AGT TTG GGA XXX TTT CCC CAA ATG ATG | CAT CAT TTG GGG AAA XXX TCC CAA ACT TTT TTT |
| F88 | AAA AGT TTG GGA AAC XXX CCC CAA ATG ATG CGT | ACG CAT CAT TTG GGG XXX GTT TCC CAA ACT TTT |
| Y100 | GGC TAT ATT TTC GTT XXX CAG GAT GTC CGT GGC | GCC ACG GAC ATC CTG XXX AAC GAA AAT ATA GCC |
| D102 | ATT TTC GTT TAC CAG XXX GTC CGT GGC AAG TGG | CCA CTT GCC ACG GAC XXX CTG GTA AAC GAA AAT |
| V103 | TTC GTT TAC CAG GAT XXX CGT GGC AAG TGG ATG | CAT CCA CTT GCC ACG XXX ATC CTG GTA AAC GAA |
| K106 | CAG GAT GTC CGT GGC XXX TGG ATG AGC GAA GGT | ACC TTC GCT CAT CCA XXX GCC ACG GAC ATC CTG |
| W107 | GAT GTC CGT GGC AAG XXX ATG AGC GAA GGT GAT | ATC ACC TTC GCT CAT XXX CTT GCC ACG GAC ATC |
| F113 | ATG AGC GAA GGT GAT XXX GAA GAT ATA CGT CCG | CGG ACG TAT ATC TTC XXX ATC ACC TTC GCT CAT |
| E114 | AGC GAA GGT GAT TTT XXX GAT ATA CGT CCG ACC | GGT CGG ACG TAT ATC XXX AAA ATC ACC TTC GCT |
| D115 | GAA GGT GAT TTT GAA XXX ATA CGT CCG ACC ACG | CGT GGT CGG ACG TAT XXX TTC AAA ATC ACC TTC |
| I116 | GGT GAT TTT GAA GAT XXX CGT CCG ACC ACG TAC | GTA CGT GGT CGG ACG XXX ATC TTC AAA ATC ACC |
| R117 | GAT TTT GAA GAT ATA XXX CCG ACC ACG TAC AGC | GCT GTA CGT GGT CGG XXX TAT ATC TTC AAA ATC |
| E130 | AAA AAA GCA ATC GAT XXX AGT ACG GAT ACC TAT | ATA GGT ATC CGT ACT XXX ATC GAT TGC TTT TTT |
| Y155 | GGC AAA GCC GGG CTC XXX GGG ATT TCC TAT CCA | TGG ATA GGA AAT CCC XXX GAG CCC GGC TTT GCC |

### Table 46-2

**Table 46-2**

| RESIDUE | Forward PRIMER | Reverse PRIMER |
|---|---|---|
| G156 | AAA GCC GGG CTC TAT XXX ATT TCC TAT CCA GGC | GCC TGG ATA GGA AAT XXX ATA GAG CCC GGC TTT |
| l157 | GCC GGG CTC TAT GGG XXX TCC TAT CCA GGC TTC | GAA GCC TGG ATA GGA XXX CCC ATA GAG CCC GGC |
| S158 | GGG CTC TAT GGG ATT XXX TAT CCA GGC TTC TAT | ATA GAA GCC TGG ATA XXX AAT CCC ATA GAG CCC |
| Y159 | CTC TAT GGG ATT TCC XXX CCA GGC TTC TAT TCT | AGA ATA GAA GCC TGG XXX GGA AAT CCC ATA GAG |
| P160 | TAT GGG ATT TCC TAT XXX GGC TTC TAT TCT ACC | GGT AGA ATA GAA GCC XXX ATA GGA AAT CCC ATA |
| G161 | GGG ATT TCC TAT CCA XXX TTC TAT TCT ACC GTC | GAC GGT AGA ATA GAA XXX TGG ATA GGA AAT CCC |
| F162 | ATT TCC TAT CCA GGC XXX TAT TCT ACC GTC GGA | TCC GAC GGT AGA ATA XXX GCC TGG ATA GGA AAT |
| Y163 | TCC TAT CCA GGC TTC XXX TCT ACC GTC GGA TTG | CAA TCC GAC GGT AGA XXX GAA GCC TGG ATA GGA |
| T165 | CCA GGC TTC TAT TCT XXX GTC GGA TTG GTC AAA | TTT GAC CAA TCC GAC XXX AGA ATA GAA GCC TGG |
| V166 | GGC TTC TAT TCT ACC XXX GGA TTG GTC AAA ACA | TGT TTT GAC CAA TCC XXX GGT AGA ATA GAA GCC |
| P180 | TTG AAG GCA GTC TCC XXX CAG GCT CCC GCA ACA | TGT TGC GGG AGC CTG XXX GGA GAC TGC CTT CAA |
| Q181 | AAG GCA GTC TCC CCA XXX GCT CCC GCA ACA GAC | GTC TGT TGC GGG AGC XXX TGG GGA GAC TGC CTT |
| A182 | GCA GTC TCC CCA CAG XXX CCC GCA ACA GAC TGG | CCA GTC TGT TGC GGG XXX CTG TGG GGA GAC TGC |
| P183 | GTC TCC CCA CAG GCT XXX GCA ACA GAC TGG TAT | ATA CCA GTC TGT TGC XXX AGC CTG TGG GGA GAC |
| A184 | TCC CCA CAG GCT CCC XXX ACA GAC TGG TAT ATC | GAT ATA CCA GTC TGT XXX GGG AGC CTG TGG GGA |
| T185 | CCA CAG GCT CCC GCA XXX GAC TGG TAT ATC GGC | GCC GAT ATA CCA GTC XXX TGC GGG AGC CTG TGG |
| D186 | CAG GCT CCC GCA ACA XXX TGG TAT ATC GGC GAC | GTC GCC GAT ATA CCA XXX TGT TGC GGG AGC CTG |
| W187 | GCT CCC GCA ACA GAC XXX TAT ATC GGC GAC GAC | GTC GTC GCC GAT ATA XXX GTC TGT TGC GGG AGC |
| Y188 | CCC GCA ACA GAC TGG XXX ATC GGC GAC GAC TTC | GAA GTC GTC GCC GAT XXX CCA GTC TGT TGC GGG |
| G190 | ACA GAC TGG TAT ATC XXX GAC GAC TTC CAC CAT | ATG GTG GAA GTC GTC XXX GAT ATA CCA GTC TGT |
| D191 | GAC TGG TAT ATC GGC XXX GAC TTC CAC CAT AAT | ATT ATG GTG GAA GTC XXX GCC GAT ATA CCA GTC |
| D192 | TGG TAT ATC GGC GAC XXX TTC CAC CAT AAT GGC | GCC ATT ATG GTG GAA XXX GTC GCC GAT ATA CCA |
| F193 | TAT ATC GGC GAC GAC XXX CAC CAT AAT GGC GTA | TAC GCC ATT ATG GTG XXX GTC GTC GCC GAT ATA |
| H194 | ATC GGC GAC GAC TTC XXX CAT AAT GGC GTA TTG | CAA TAC GCC ATT ATG XXX GAA GTC GTC GCC GAT |
| H195 | GGC GAC GAC TTC CAC XXX AAT GGC GTA TTG TTT | AAA CAA TAC GCC ATT XXX GTG GAA GTC GTC GCC |
| F200 | CAT AAT GGC GTA TTG XXX CTT CAG GAT GCA TTT | AAA TGC ATC CTG AAG XXX CAA TAC GCC ATT ATG |
| L201 | AAT GGC GTA TTG TTT XXX CAG GAT GCA TTT ACA | TGT AAA TGC ATC CTG XXX AAA CAA TAC GCC ATT |
| Q202 | GGC GTA TTG TTT CTT XXX GAT GCA TTT ACA TTC | GAA TGT AAA TGC ATC XXX AAG AAA CAA TAC GCC |
| D203 | GTA TTG TTT CTT CAG XXX GCA TTT ACA TTC ATG | CAT GAA TGT AAA TGC XXX CTG AAG AAA CAA TAC |
| A204 | TTG TTT CTT CAG GAT XXX TTT ACA TTC ATG TCA | TGA CAT GAA TGT AAA XXX ATC CTG AAG AAA CAA |
| F205 | TTT CTT CAG GAT GCA XXX ACA TTC ATG TCA ACC | GGT TGA CAT GAA TGT XXX TGC ATC CTG AAG AAA |
| T206 | CTT CAG GAT GCA TTT XXX TTC ATG TCA ACC TTT | AAA GGT TGA CAT GAA XXX AAA TGC ATC CTG AAG |
| F207 | CAG GAT GCA TTT ACA XXX ATG TCA ACC TTT GGT | ACC AAA GGT TGA CAT XXX TGT AAA TGC ATC CTG |
| M208 | GAT GCA TTT ACA TTC XXX TCA ACC TTT GGT GTC | GAC ACC AAA GGT TGA XXX GAA TGT AAA TGC ATC |
| S209 | GCA TTT ACA TTC ATG XXX ACC TTT GGT GTC CCT | AGG GAC ACC AAA GGT XXX CAT GAA TGT AAA TGC |

### Table 46-3

**Table 46-3**

| RESIDUE | Forward PRIMER | Reverse PRIMER |
|---|---|---|
| T210 | TTT ACA TTC ATG TCA XXX TTT GGT GTC CCT CGT | ACG AGG GAC ACC AAA XXX TGA CAT GAA TGT AAA |
| F211 | ACA TTC ATG TCA ACC XXX GGT GTC CCT CGT CCA | TGG ACG AGG GAC ACC XXX GGT TGA CAT GAA TGT |
| G212 | TTC ATG TCA ACC TTT XXX GTC CCT CGT CCA AAA | TTT TGG ACG AGG GAC XXX AAA GGT TGA CAT GAA |
| V213 | ATG TCA ACC TTT GGT XXX CCT CGT CCA AAA CCC | GGG TTT TGG ACG AGG XXX ACC AAA GGT TGA CAT |
| P214 | TCA ACC TTT GGT GTC XXX CGT CCA AAA CCC ATT | AAT GGG TTT TGG ACG XXX GAC ACC AAA GGT TGA |
| R215 | ACC TTT GGT GTC CCT XXX CCA AAA CCC ATT ACA | TGT AAT GGG TTT TGG XXX AGG GAC ACC AAA GGT |
| P216 | TTT GGT GTC CCT CGT XXX AAA CCC ATT ACA CCG | CGG TGT AAT GGG TTT XXX ACG AGG GAC ACC AAA |
| K217 | GGT GTC CCT CGT CCA XXX CCC ATT ACA CCG GAT | ATC CGG TGT AAT GGG XXX TGG ACG AGG GAC ACC |
| P218 | GTC CCT CGT CCA AAA XXX ATT ACA CCG GAT CAA | TTG ATC CGG TGT AAT XXX TTT TGG ACG AGG GAC |
| I219 | CCT CGT CCA AAA CCC XXX ACA CCG GAT CAA TTT | AAA TTG ATC CGG TGT XXX GGG TTT TGG ACG AGG |
| T220 | CGT CCA AAA CCC ATT XXX CCG GAT CAA TTT AAG | CTT AAA TTG ATC CGG XXX AAT GGG TTT TGG ACG |
| P221 | CCA AAA CCC ATT ACA XXX GAT CAA TTT AAG GGC | GCC CTT AAA TTG ATC XXX TGT AAT GGG TTT TGG |
| D222 | AAA CCC ATT ACA CCG XXX CAA TTT AAG GGC AAA | TTT GCC CTT AAA TTG XXX CGG TGT AAT GGG TTT |
| Q223 | CCC ATT ACA CCG GAT XXX TTT AAG GGC AAA ATT | AAT TTT GCC CTT AAA XXX ATC CGG TGT AAT GGG |
| F224 | ATT ACA CCG GAT CAA XXX AAG GGC AAA ATT CCT | AGG AAT TTT GCC CTT XXX TTG ATC CGG TGT AAT |
| K225 | ACA CCG GAT CAA TTT XXX GGC AAA ATT CCT ATC | GAT AGG AAT TTT GCC XXX AAA TTG ATC CGG TGT |
| G226 | CCG GAT CAA TTT AAG XXX AAA ATT CCT ATC AAA | TTT GAT AGG AAT TTT XXX CTT AAA TTG ATC CGG |
| K227 | GAT CAA TTT AAG GGC XXX ATT CCT ATC AAA GAA | TTC TTT GAT AGG AAT XXX GCC CTT AAA TTG ATC |
| I228 | CAA TTT AAG GGC AAA XXX CCT ATC AAA GAA GCC | GGC TTC TTT GAT AGG XXX TTT GCC CTT AAA TTG |
| P229 | TTT AAG GGC AAA ATT XXX ATC AAA GAA GCC GAT | ATC GGC TTC TTT GAT XXX AAT TTT GCC CTT AAA |
| 1230 | AAG GGC AAA ATT CCT XXX AAA GAA GCC GAT AAA | TTT ATC GGC TTC TTT XXX AGG AAT TTT GCC CTT |
| K231 | GGC AAA ATT CCT ATC XXX GAA GCC GAT AAA TAT | ATA TTT ATC GGC TTC XXX GAT AGG AAT TTT GCC |
| E232 | AAA ATT CCT ATC AAA XXX GCC GAT AAA TAT AAC | GTT ATA TTT ATC GGC XXX TTT GAT AGG AAT TTT |
| A233 | ATT CCT ATC AAA GAA XXX GAT AAA TAT AAC TTT | AAA GTT ATA TTT ATC XXX TTC TTT GAT AGG AAT |
| D234 | CCT ATC AAA GAA GCC XXX AAA TAT AAC TTT TTT | AAA AAA GTT ATA TTT XXX GGC TTC TTT GAT AGG |
| K235 | ATC AAA GAA GCC GAT XXX TAT AAC TTT TTT GCA | TGC AAA AAA GTT ATA XXX ATC GGC TTC TTT GAT |
| F259 | GGT GAC TCC ATA CAA XXX TGG AAT GAC CTG TTT | AAA CAG GTC ATT CCA XXX TTG TAT GGA GTC ACC |
| W273 | GAC TAT GAT GAT TTT XXX AAA TCG CGT GTG ATC | GAT CAC ACG CGA TTT XXX AAA ATC ATC ATA GTC |
| R276 | GAT TTT TGG AAA TCG XXX GTG ATC ACC AAT TCT | AGA ATT GGT GAT CAC XXX CGA TTT CCA AAA ATC |
| R278 | TGG AAA TCG CGT GTG XXX ACC AAT TCT TTA CAG | CTG TAA AGA ATT GGT XXX CAC ACG CGA TTT CCA |
| V292 | CCA GCT GTG ATG GTG XXX GGT GGT TTC TTT GAC | GTC AAA GAA ACC ACC XXX CAC CAT CAC AGC TGG |
| G293 | GCT GTG ATG GTG GTT XXX GGT TTC TTT GAC GCG | CGC GTC AAA GAA ACC XXX AAC CAC CAT CAC AGC |
| G294 | GTG ATG GTG GTT GGT XXX TTC TTT GAC GCG GAA | TTC CGC GTC AAA GAA XXX ACC AAC CAC CAT CAC |
| F296 | GTG GTT GGT GGT TTC XXX GAC GCG GAA GAT GTT | AAC ATC TTC CGC GTC XXX GAA ACC ACC AAC CAC |
| A298 | GGT GGT TTC TTT GAC XXX GAA GAT GTT TAT GGA | TCC ATA AAC ATC TTC XXX GTC AAA GAA ACC ACC |

### Table 46-4

**Table 46-4**

| RESIDUE | Forward PRIMER | Reverse PRIMER |
|---|---|---|
| E299 | GGT TTC TTT GAC GCG XXX GAT GTT TAT GGA ACA | TGT TCC ATA AAC ATC XXX CGC GTC AAA GAA ACC |
| D300 | TTC TTT GAC GCG GAA XXX GTT TAT GGA ACA TTT | AAA TGT TCC ATA AAC XXX TTC CGC GTC AAA GAA |
| V301 | TTT GAC GCG GAA GAT XXX TAT GGA ACA TTT AAG | CTT AAA TGT TCC ATA XXX ATC TTC CGC GTC AAA |
| Y302 | GAC GCG GAA GAT GTT XXX GGA ACA TTT AAG ACC | GGT CTT AAA TGT TCC XXX AAC ATC TTC CGC GTC |
| G303 | GCG GAA GAT GTT TAT XXX ACA TTT AAG ACC TAC | GTA GGT CTT AAA TGT XXX ATA AAC ATC TTC CGC |
| T304 | GAA GAT GTT TAT GGA XXX TTT AAG ACC TAC CAA | TTG GTA GGT CTT AAA XXX TCC ATA AAC ATC TTC |
| G325 | TCG ATT TTA GTC GTG XXX CCT TGG TAT CAT GGC | GCC ATG ATA CCA AGG XXX CAC GAC TAA AAT CGA |
| P326 | ATT TTA GTC GTG GGA XXX TGG TAT CAT GGC GGC | GCC GCC ATG ATA CCA XXX TCC CAC GAC TAA AAT |
| W327 | TTA GTC GTG GGA CCT XXX TAT CAT GGC GGC TGG | CCA GCC GCC ATG ATA XXX AGG TCC CAC GAC TAA |
| Y328 | GTC GTG GGA CCT TGG XXX CAT GGC GGC TGG GTT | AAC CCA GCC GCC ATG XXX CCA AGG TCC CAC GAC |
| H329 | GTG GGA CCT TGG TAT XXX GGC GGC TGG GTT CGT | ACG AAC CCA GCC GCC XXX ATA CCA AGG TCC CAC |
| G330 | GGA CCT TGG TAT CAT XXX GGC TGG GTT CGT GCA | TGC ACG AAC CCA GCC XXX ATG ATA CCA AGG TCC |
| G331 | CCT TGG TAT CAT GGC XXX TGG GTT CGT GCA GAA | TTC TGC ACG AAC CCA XXX GCC ATG ATA CCA AGG |
| W332 | TGG TAT CAT GGC GGC XXX GTT CGT GCA GAA GGA | TCC TTC TGC ACG AAC XXX GCC GCC ATG ATA CCA |
| V333 | TAT CAT GGC GGC TGG XXX CGT GCA GAA GGA AAC | GTT TCC TTC TGC ACG XXX CCA GCC GCC ATG ATA |
| R334 | CAT GGC GGC TGG GTT XXX GCA GAA GGA AAC TAT | ATA GTT TCC TTC TGC: XXX AAC CCA GCC GCC ATG |
| A335 | GGC GGC TGG GTT CGT XXX GAA GGA AAC TAT TTA | TAA ATA GTT TCC TTC XXX ACG AAC CCA GCC GCC |
| E336 | GGC TGG GTT CGT GCA XXX GGA AAC TAT TTA GGT | ACC TAA ATA GTT TCC, XXX TGC ACG AAC CCA GCC |
| G337 | TGG GTT CGT GCA GAA XXX AAC TAT TTA GGT GAT | ATC ACC TAA ATA GTT XXX TTC TGC ACG AAC CCA |
| N338 | GTT CGT GCA GAA GGA XXX TAT TTA GGT GAT ATC | GAT ATC ACC TAA ATA XXX TCC TTC TGC ACG AAC |
| Y339 | CGT GCA GAA GGA AAC XXX TTA GGT GAT ATC CAA | TTG GAT ATC ACC TAA XXX GTT TCC TTC TGC ACG |
| L340 | GCA GAA GGA AAC TAT XXX GGT GAT ATC CAA TTT | AAA TTG GAT ATC ACC XXX ATA GTT TCC TTC TGC |
| G437 | CCT GTT CCG CAT CAA XXX GGG GTA ATT GAA AAC | GTT TTC AAT TAC CCC XXX TTG ATG CGG AAC AGG |
| G438 | GTT CCG CAT CAA GGT XXX GTA ATT GAA AAC CGA | TCG GTT TTC AAT TAC XXX ACC TTG ATG CGG AAC |
| V439 | CCG CAT CAA GGT GGG XXX ATT GAA AAC CGA ACA | TGT TCG GTT TTC AAT XXX CCC ACC TTG ATG CGG |
| I440 | CAT CAA GGT GGG GTA XXX GAA AAC CGA ACA CGG | CCG TGT TCG GTT TTC XXX TAC CCC ACC TTG ATG |
| E441 | CAA GGT GGG GTA ATT XXX AAC CGA ACA CGG GAG | CTC CCG TGT TCG GTT XXX AAT TAC CCC ACC TTG |
| N442 | GGT GGG GTA ATT GAA XXX CGA ACA CGG GAG TAT | ATA CTC CCG TGT TCG XXX TTC AAT TAC CCC ACC |
| R443 | GGG GTA ATT GAA AAC XXX ACA CGG GAG TAT ATG | CAT ATA CTC CCG TGT XXX GTT TTC AAT TAC CCC |
| T444 | GTA ATT GAA AAC CGA XXX CGG GAG TAT ATG GTA | TAC CAT ATA CTC CCG XXX TCG GTT TTC AAT TAC |
| R445 | ATT GAA AAC CGA ACA XXX GAG TAT ATG GTA GAT | ATC TAC CAT ATA CTC XXX TGT TCG GTT TTC AAT |
| E446 | GAA AAC CGA ACA CGG XXX TAT ATG GTA GAT GAT | ATC ATC TAC CAT ATA XXX CCG TGT TCG GTT TTC |
| Y447 | AAC CGA ACA CGG GAG XXX ATG GTA GAT GAT CAA | TTG ATC ATC TAC CAT XXX CTC CCG TGT TCG GTT |

### BRIEF DESCRIPTION OF DRAWINGS

FIG. 1 is a view showing experimental results for pH stability.
FIG. 2 is a view showing experimental results for optimal reaction temperature.
FIG. 3 is a view showing experimental results for temperature stability.
FIG. 4 is a view showing a tertiary structure of a protein having an amino acid sequence of SEQ ID NO:209.
FIG. 5 is a tertiary structure of a protein having an amino acid sequence of SEQ ID NO:208.
FIG. 6-1 is a view showing atomic coordinates in the tertiary structure (three dimensional structure) of the protein having the amino acid sequence of SEQ ID NO:209.
FIG. 6-2 is a view showing atomic coordinates in the tertiary structure (three dimensional structure) of the protein having the amino acid sequence of SEQ ID NO:209.
FIG. 6-3 is a view showing atomic coordinates in the tertiary structure (three dimensional structure) of the protein having the amino acid sequence of SEQ ID NO:209.
FIG. 6-4 is a view showing atomic coordinates in the tertiary structure (three dimensional structure) of the protein having the amino acid sequence of SEQ ID NO:209.
FIG. 6-5 is a view showing atomic coordinates in the tertiary structure (three dimensional structure) of the protein having the amino acid sequence of SEQ ID NO:209.
FIG. 6-6 is a view showing atomic coordinates in the tertiary structure (three dimensional structure) of the protein having the amino acid sequence of SEQ ID NO:209.
FIG. 6-7 is a view showing atomic coordinates in the tertiary structure (three dimensional structure) of the protein having the amino acid sequence of SEQ ID NO:209.
FIG. 6-8 is a view showing atomic coordinates in the tertiary structure (three dimensional structure) of the protein having the amino acid sequence of SEQ ID NO:209.
FIG. 6-9 is a view showing atomic coordinates in the tertiary structure (three dimensional structure) of the protein having the amino acid sequence of SEQ ID NO:209.
FIG. 6-10 is a view showing atomic coordinates in the tertiary structure (three dimensional structure) of the protein having the amino acid sequence of SEQ ID NO:209.
FIG. 6-11 is a view showing atomic coordinates in the tertiary structure (three dimensional structure) of the protein having the amino acid sequence of SEQ ID NO:209.
FIG. 6-12 is a view showing atomic coordinates in the tertiary structure (three dimensional structure) of the protein having the amino acid sequence of SEQ ID NO:209.
FIG. 6-13 is a view showing atomic coordinates in the tertiary structure (three dimensional structure) of the protein having the amino acid sequence of SEQ ID NO:209.
FIG. 6-14 is a view showing atomic coordinates in the tertiary structure (three dimensional structure) of the protein having the amino acid sequence of SEQ ID NO:209.
FIG. 6-15 is a view showing atomic coordinates in the tertiary structure (three dimensional structure) of the protein having the amino acid sequence of SEQ ID NO:209.
FIG. 6-16 is a view showing atomic coordinates in the tertiary structure (three dimensional structure) of the protein having the amino acid sequence of SEQ ID NO:209.
FIG. 6-17 is a view showing atomic coordinates in the tertiary structure (three dimensional structure) of the protein having the amino acid sequence of SEQ ID NO:209.
FIG. 6-18 is a view showing atomic coordinates in the tertiary structure (three dimensional structure) of the protein having the amino acid sequence of SEQ ID NO:209.
FIG. 6-19 is a view showing atomic coordinates in the tertiary structure (three dimensional structure) of the protein having the amino acid sequence of SEQ ID NO:209.
FIG. 6-20 is a view showing atomic coordinates in the tertiary structure (three dimensional structure) of the protein having the amino acid sequence of SEQ ID NO:209.
FIG. 6-21 is a view showing atomic coordinates in the tertiary structure (three dimensional structure) of the protein having the amino acid sequence of SEQ ID NO:209.
FIG. 6-22 is a view showing atomic coordinates in the tertiary structure (three dimensional structure) of the protein having the amino acid sequence of SEQ ID NO:209.
FIG. 6-23 is a view showing atomic coordinates in the tertiary structure (three dimensional structure) of the protein having the amino acid sequence of SEQ ID NO:209.
FIG. 6-24 is a view showing atomic coordinates in the tertiary structure (three dimensional structure) of the protein having the amino acid sequence of SEQ ID NO:209.
FIG. 6-25 is a view showing atomic coordinates in the tertiary structure (three dimensional structure) of the protein having the amino acid sequence of SEQ ID NO:209.
FIG. 6-26 is a view showing atomic coordinates in the tertiary structure (three dimensional structure) of the protein having the amino acid sequence of SEQ ID NO:209.
FIG. 6-27 is a view showing atomic coordinates in the tertiary structure (three dimensional structure) of the protein having the amino acid sequence of SEQ ID NO:209.
FIG. 6-28 is a view showing atomic coordinates in the tertiary structure (three dimensional structure) of the protein having the amino acid sequence of SEQ ID NO:209.
FIG. 6-29 is a view showing atomic coordinates in the tertiary structure (three dimensional structure) of the protein having the amino acid sequence of SEQ ID NO:209.
FIG. 6-30 is a view showing atomic coordinates in the tertiary structure (three dimensional structure) of the protein having the amino acid sequence of SEQ ID NO:209.
FIG. 6-31 is a view showing atomic coordinates in the tertiary structure (three dimensional structure) of the protein having the amino acid sequence of SEQ ID NO:209.
FIG. 6-32 is a view showing atomic coordinates in the tertiary structure (three dimensional structure) of the protein having the amino acid sequence of SEQ ID NO:209.
FIG. 6-33 is a view showing atomic coordinates in the tertiary structure (three dimensional structure) of the protein having the amino acid sequence of SEQ ID NO:209.
FIG. 6-34 is a view showing atomic coordinates in the tertiary structure (three dimensional structure) of the protein having the amino acid sequence of SEQ ID NO:209.
FIG. 6-35 is a view showing atomic coordinates in the tertiary structure (three dimensional structure) of the protein having the amino acid sequence of SEQ ID NO:209.
FIG. 6-36 is a view showing atomic coordinates in the tertiary structure (three dimensional structure) of the protein having the amino acid sequence of SEQ ID NO:209.
FIG. 6-37 is a view showing atomic coordinates in the tertiary structure (three dimensional structure) of the protein having the amino acid sequence of SEQ ID NO:209.
FIG. 6-38 is a view showing atomic coordinates in the tertiary structure (three dimensional structure) of the protein having the amino acid sequence of SEQ ID NO:209.
FIG. 6-39 is a view showing atomic coordinates in the tertiary structure (three dimensional structure) of the protein having the amino acid sequence of SEQ ID NO:209.
FIG. 6-40 is a view showing atomic coordinates in the tertiary structure (three dimensional structure) of the protein having the amino acid sequence of SEQ ID NO:209.
FIG. 6-41 is a view showing atomic coordinates in the tertiary structure (three dimensional structure) of the protein having the amino acid sequence of SEQ ID NO:209.
FIG. 6-42 is a view showing atomic coordinates in the tertiary structure (three dimensional structure) of the protein having the amino acid sequence of SEQ ID NO:209.
FIG. 6-43 is a view showing atomic coordinates in the tertiary structure (three dimensional structure).of the protein having the amino acid sequence of SEQ ID NO:209.
FIG. 6-44 is a view showing atomic coordinates in the tertiary structure (three dimensional structure) of the protein having the amino acid sequence of SEQ ID NO:209.
FIG. 6-45 is a view showing atomic coordinates in the tertiary structure (three dimensional structure) of the protein having the amino acid sequence of SEQ ID NO:209.
FIG. 6-46 is a view showing atomic coordinates in the tertiary structure (three dimensional structure) of the protein having the amino acid sequence of SEQ ID NO:209.
FIG. 6-47 is a view showing atomic coordinates in the tertiary structure (three dimensional structure) of the protein having the amino acid sequence of SEQ ID NO:209.
FIG. 6-48 is a view showing atomic coordinates in the tertiary structure (three dimensional structure) of the protein having the amino acid sequence of SEQ ID NO:209.
FIG. 6-49 is a view showing atomic coordinates in the tertiary structure (three dimensional structure) of the protein having the amino acid sequence of SEQ ID NO:209.
FIG. 6-50 is a view showing atomic coordinates in the tertiary structure (three dimensional structure) of the protein having the amino acid sequence of SEQ ID NO:209.
FIG. 6-51 is a view showing atomic coordinates in the tertiary structure (three dimensional structure) of the protein having the amino acid sequence of SEQ ID NO:209.
FIG. 6-52 is a view showing atomic coordinates in the tertiary structure (three dimensional structure) of the protein having the amino acid sequence of SEQ ID NO:209.
FIG. 6-53 is a view showing atomic coordinates in the tertiary structure (three dimensional structure) of the protein having the amino acid sequence of SEQ ID NO:209.
FIG. 6-54 is a view showing atomic coordinates in the tertiary structure (three dimensional structure) of the protein having the amino acid sequence of SEQ ID NO:209.
FIG. 6-55 is a view showing atomic coordinates in the tertiary structure (three dimensional structure) of the protein having the amino acid sequence of SEQ ID NO:209.
FIG. 6-56 is a view showing atomic coordinates in the tertiary structure (three dimensional structure) of the protein having the amino acid sequence of SEQ ID NO:209.
FIG. 6-57 is a view showing atomic coordinates in the tertiary structure (three dimensional structure) of the protein having the amino acid sequence of SEQ ID NO:209.
FIG. 6-58 is a view showing atomic coordinates in the tertiary structure (three dimensional structure) of the protein having the amino acid sequence of SEQ ID NO:209.
FIG. 6-59 is a view showing atomic coordinates in the tertiary structure (three dimensional structure) of the protein having the amino acid sequence of SEQ ID NO:209.
FIG. 6-60 is a view showing atomic coordinates in the tertiary structure (three dimensional structure) of the protein having the amino acid sequence of SEQ ID NO:209.
FIG. 6-61 is a view showing atomic coordinates in the tertiary structure (three dimensional structure) of the protein having the amino acid sequence of SEQ ID NO:209.
FIG. 6-62 is a view showing atomic coordinates in the tertiary structure (three dimensional structure) of the protein having the amino acid sequence of SEQ ID NO:209.
FIG. 6-63 is a view showing atomic coordinates in the tertiary structure (three dimensional structure) of the protein having the amino acid sequence of SEQ ID NO:209.
FIG. 6-64 is a view showing atomic coordinates in the tertiary structure (three dimensional structure) of the protein having the amino acid sequence of SEQ ID NO:209.
FIG. 6-65 is a view showing atomic coordinates in the tertiary structure (three dimensional structure) of the protein having the amino acid sequence of SEQ ID NO:209.
FIG. 6-66 is a view showing atomic coordinates in the tertiary structure (three dimensional structure) of the protein having the amino acid sequence of SEQ ID NO:209.
FIG. 6-67 is a view showing atomic coordinates in the tertiary structure (three dimensional structure) of the protein having the amino acid sequence of SEQ ID NO:209.
FIG. 6-68 is a view showing atomic coordinates in the tertiary structure (three dimensional structure) of the protein having the amino acid sequence of SEQ ID NO:209.
FIG. 6-69 is a view showing atomic coordinates in the tertiary structure (three dimensional structure) of the protein having the amino acid sequence of SEQ ID NO:209.
FIG. 6-70 is a view showing atomic coordinates in the tertiary structure (three dimensional structure) of the protein having the amino acid sequence of SEQ ID NO:209.
FIG. 6-71 is a view showing atomic coordinates in the tertiary structure (three dimensional structure) of the protein having the amino acid sequence of SEQ ID NO:209.
FIG. 6-72 is a view showing atomic coordinates in the tertiary structure (three dimensional structure) of the protein having the amino acid sequence of SEQ ID NO:209.
FIG. 6-73 is a view showing atomic coordinates in the tertiary structure (three dimensional structure) of the protein having the amino acid sequence of SEQ ID NO:209.
FIG. 6-74 is a view showing atomic coordinates in the tertiary structure (three dimensional structure) of the protein having the amino acid sequence of SEQ ID NO:209.
FIG. 6-75 is a view showing atomic coordinates in the tertiary structure (three dimensional structure) of the protein having the amino acid sequence of SEQ ID NO:209.
FIG. 6-76 is a view showing atomic coordinates in the tertiary structure (three dimensional structure) of the protein having the amino acid sequence of SEQ ID NO:209.
FIG. 6-77 is a view showing atomic coordinates in the tertiary structure (three dimensional structure) of the protein having the amino acid sequence of SEQ ID NO:209.
FIG. 6-78 is a view showing atomic coordinates in the tertiary structure (three dimensional structure) of the protein having the amino acid sequence of SEQ ID NO:209.
FIG. 6-79 is a view showing atomic coordinates in the tertiary structure (three dimensional structure) of the protein having the amino acid sequence of SEQ ID NO:209.
FIG. 6-80 is a view showing atomic coordinates in the tertiary structure (three dimensional structure) of the protein having the amino acid sequence of SEQ ID NO:209.
FIG. 6-81 is a view showing atomic coordinates in the tertiary structure (three dimensional structure) of the protein having the amino acid sequence of SEQ ID NO:209.
FIG. 6-82 is a view showing atomic coordinates in the tertiary structure (three dimensional structure) of the protein having the amino acid sequence of SEQ ID NO:209.
FIG. 6-83 is a view showing atomic coordinates in the tertiary structure (three dimensional structure) of the protein having the amino acid sequence of SEQ ID NO:209.
FIG. 6-84 is a view showing atomic coordinates in the tertiary structure (three dimensional structure) of the protein having the amino acid sequence of SEQ ID NO:209.
FIG. 6-85 is a view showing atomic coordinates in the tertiary structure (three dimensional structure) of the protein having the amino acid sequence of SEQ ID NO:209.
FIG. 6-86 is a view showing atomic coordinates in the tertiary structure (three dimensional structure) of the protein having the amino acid sequence of SEQ ID NO:209.
FIG. 6-87 is a view showing atomic coordinates in the tertiary structure (three dimensional structure) of the protein having the amino acid sequence of SEQ ID NO:209.
FIG. 6-88 is a view showing atomic coordinates in the tertiary structure (three dimensional structure) of the protein having the amino acid sequence of SEQ ID NO:209.
FIG. 6-89 is a view showing atomic coordinates in the tertiary structure (three dimensional structure) of the protein having the amino acid sequence of SEQ ID NO:209.
FIG. 6-90 is a view showing atomic coordinates in the tertiary structure (three dimensional structure) of the protein having the amino acid sequence of SEQ ID NO:209.
FIG. 6-91 is a view showing atomic coordinates in the tertiary structure (three dimensional structure) of the protein having the amino acid sequence of SEQ ID NO:209.
FIG. 6-92 is a view showing atomic coordinates in the tertiary structure (three dimensional structure) of the protein having the amino acid sequence of SEQ ID NO:209.
FIG. 6-93 is a view showing atomic coordinates in the tertiary structure (three dimensional structure) of the protein having the amino acid sequence of SEQ ID NO:209.
FIG. 6-94 is a view showing atomic coordinates in the tertiary structure (three dimensional structure) of the protein having the amino acid sequence of SEQ ID NO:209.
FIG. 6-95 is a view showing atomic coordinates in the tertiary structure (three dimensional structure) of the protein having the amino acid sequence of SEQ ID NO:209.
FIG. 6-96 is a view showing atomic coordinates in the tertiary structure (three dimensional structure) of the protein having the amino acid sequence of SEQ ID NO:209.
FIG. 6-97 is a view showing atomic coordinates in the tertiary structure (three dimensional structure) of the protein having the amino acid sequence of SEQ ID NO:209.
FIG. 6-98 is a view showing atomic coordinates in the tertiary structure (three dimensional structure) of the protein having the amino acid sequence of SEQ ID NO:209.
FIG. 6-99 is a view showing atomic coordinates in the tertiary structure (three dimensional structure) of the protein having the amino acid sequence of SEQ ID NO:209.
FIG. 6-100 is a view showing atomic coordinates in the tertiary structure (three dimensional structure) of the protein having the amino acid sequence of SEQ ID NO:209.
FIG. 6-101 is a view showing atomic coordinates in the tertiary structure (three dimensional structure) of the protein having the amino acid sequence of SEQ ID NO:209.
FIG. 6-102 is a view showing atomic coordinates in the tertiary structure (three dimensional structure) of the protein having the amino acid sequence of SEQ ID NO:209.
FIG. 6-103 is a view showing atomic coordinates in the tertiary structure (three dimensional structure) of the protein having the amino acid sequence of SEQ ID NO:209.
FIG. 6-104 is a view showing atomic coordinates in the tertiary structure (three dimensional structure) of the protein having the amino acid sequence of SEQ ID NO:209.
FIG. 6-105 is a view showing atomic coordinates in the tertiary structure (three dimensional structure) of the protein having the amino acid sequence of SEQ ID NO:209.
FIG. 6-106 is a view showing atomic coordinates in the tertiary structure (three dimensional structure) of the protein having the amino acid sequence of SEQ ID NO:209.
FIG. 6-107 is a view showing atomic coordinates in the tertiary structure (three dimensional structure) of the protein having the amino acid sequence of SEQ ID NO:209.
FIG. 6-108 is a view showing atomic coordinates in the tertiary structure (three dimensional structure) of the protein having the amino acid sequence of SEQ ID NO:209.
FIG. 6-109 is a view showing atomic coordinates in the tertiary structure (three dimensional structure) of the protein having the amino acid sequence of SEQ ID NO:209.
FIG. 6-110 is a view showing atomic coordinates in the tertiary structure (three dimensional structure) of the protein having the amino acid sequence of SEQ ID NO:209.
FIG. 6-111 is a view showing atomic coordinates in the tertiary structure (three dimensional structure) of the protein having the amino acid sequence of SEQ ID NO:209.
FIG. 6-112 is a view showing atomic coordinates in the tertiary structure (three dimensional structure) of the protein having the amino acid sequence of SEQ ID NO:209.
FIG. 6-113 is a view showing atomic coordinates in the tertiary structure (three dimensional structure) of the protein having the amino acid sequence of SEQ ID NO:209.
FIG. 6-114 is a view showing atomic coordinates in the tertiary structure (three dimensional structure) of the protein having the amino acid sequence of SEQ ID NO:209.
FIG. 6-115 is a view showing atomic coordinates in the tertiary structure (three dimensional structure) of the protein having the amino acid sequence of SEQ ID NO:209.
FIG. 6-116 is a view showing atomic coordinates in the tertiary structure (three dimensional structure) of the protein having the amino acid sequence of SEQ ID NO:209.
FIG. 6-117 is a view showing atomic coordinates in the tertiary structure (three dimensional structure) of the protein having the amino acid sequence of SEQ ID NO:209.
FIG. 6-118 is a view showing atomic coordinates in the tertiary structure (three dimensional structure) of the protein having the amino acid sequence of SEQ ID NO:209.
FIG. 6-119 is a view showing atomic coordinates in the tertiary structure (three dimensional structure) of the protein having the amino acid sequence of SEQ ID NO:209.
FIG. 6-120 is a view showing atomic coordinates in the tertiary structure (three dimensional structure) of the protein having the amino acid sequence of SEQ ID NO:209.
FIG. 6-121 is a view showing atomic coordinates in the tertiary structure (three dimensional structure) of the protein having the amino acid sequence of SEQ ID NO:209.
FIG. 6-122 is a view showing atomic coordinates in the tertiary structure (three dimensional structure) of the protein having the amino acid sequence of SEQ ID NO:209.
FIG. 6-123 is a view showing atomic coordinates in the tertiary structure (three dimensional structure) of the protein having the amino acid sequence of SEQ ID NO:209.
FIG. 6-124 is a view showing atomic coordinates in the tertiary structure (three dimensional structure) of the protein having the amino acid sequence of SEQ ID NO:209.
FIG. 6-125 is a view showing atomic coordinates in the tertiary structure (three dimensional structure) of the protein having the amino acid sequence of SEQ ID NO:209.
FIG. 6-126 is a view showing atomic coordinates in the tertiary structure (three dimensional structure) of the protein having the amino acid sequence of SEQ ID NO:209.
FIG. 6-127 is a view showing atomic coordinates in the tertiary structure (three dimensional structure) of the protein having the amino acid sequence of SEQ ID NO:209.
FIG. 6-128 is a view showing atomic coordinates in the tertiary structure (three dimensional structure) of the protein having the amino acid sequence of SEQ ID NO:209.
FIG. 6-129 is a view showing atomic coordinates in the tertiary structure (three dimensional structure) of the protein having the amino acid sequence of SEQ ID NO:209.
FIG. 6-130 is a view showing atomic coordinates in the tertiary structure (three dimensional structure) of the protein having the amino acid sequence of SEQ ID NO:209.
FIG. 6-131 is a view showing atomic coordinates in the tertiary structure (three dimensional structure) of the protein having the amino acid sequence of SEQ ID NO:209.
FIG. 6-132 is a view showing atomic coordinates in the tertiary structure (three dimensional structure) of the protein having the amino acid sequence of SEQ ID N0:209.
FIG. 6-133 is a view showing atomic coordinates in the tertiary structure (three dimensional structure) of the protein having the amino acid sequence of SEQ ID NO:209.
FIG. 6-134 is a view showing atomic coordinates in the tertiary structure (three dimensional structure) of the protein having the amino acid sequence of SEQ ID NO:209.

### Industrial Applicability

The present invention is useful in a variety of fields concerning, e.g., a method for producing peptides.

## Claims

1. A mutant protein having an amino acid sequence comprising one or more mutations selected from any of the following mutations 1 to 68 in an amino acid sequence of SEQ ID NO:2:
mutation 1 F207V, mutation 2 Q441E, mutation 3 K83A,
mutation 4 A301V, mutation 5 V257I, mutation 6 A537G,
mutation 7 A324V, mutation 8 N607K, mutation 9 D313E,
mutation 10 Q229H, mutation 11 M208A, mutation 12 E551K
mutation 13 F207H, mutation 14 T72A, mutation 15 A137S,
mutation 16 L439V, mutation 17 G226S, mutation 18 D619E,
mutation 19 Y339H, mutation 20 W327G, mutation 21 V184A,
mutation 22 V184C, mutation 23 V184G, mutation 24 V184I,
mutation 25 V184L, mutation 26 V184M, mutation 27 V184P,
mutation 28 V184S, mutation 29 V184T, mutation 30 Q441K,
mutation 31 N442K, mutation 32 D203N, mutation 33 D203S,
mutation 34 F207A, mutation 35 F207S, mutation 36 Q441N,
mutation 37 F207T, mutation 38 F207I, mutation 39 T210K,
mutation 40 W187A, mutation 41 S209A, mutation 42 F211A,
mutation 43 F211V, mutation 44 V257A, mutation 45 V257G,
mutation 46 V257H, mutation 47 V257M, mutation 48 V257N,
mutation 49 V257Q, mutation 50 V257S, mutation 51 V257T,
mutation 52 V257W, mutation 53 V257Y, mutation 54 K47G,
mutation 55 K47E, mutation 56 N442F, mutation 57 N607R,
mutation 58 P214T, mutation 59 Q202E, mutation 60 Y494F,
mutation 61 R117A, mutation 62 F207G, mutation 63 S209D,
mutation 64 S209G, mutation 65 Q441D, mutation 66 R445D,
mutation 67 R445F, mutation 68 N442D.

2. The mutant protein according to claim 1 wherein, in said amino acid sequence comprising one or more mutations selected from any of the mutations 1 to 68, said amino acid sequence further comprises at other than the mutated position(s) one or several amino acid mutations selected from the group consisting of substitutions, deletions, insertions, additions and inversions, said mutant protein having a peptide-synthesizing activity.

3. The mutant protein according to claim 1 or 2 comprising at least the mutation 2.

4. The mutant protein according to any one of claim 1 to 3 comprising at least the mutation 14.

5. A mutant protein having an amino acid sequence comprising one or more mutations selected from any of the following mutations 239 to 290 and 324 to 377 in an amino acid sequence of SEQ ID NO:2:
mutation 239 F207V/Q441E
mutation 240 F207V/K83A
mutation 241 F207V/E551K
mutation 242 K83A/Q441E
mutation 243 M208A/E551K
mutation 244 V257I/Q441E
mutation 245 V257I/A537G
mutation 246 F207V/S209A
mutation 247 K83A/S209A
mutation 248 K83A/F207V/Q441E
mutation 249 L439V/F207V/Q441E
mutation 250 A537G/F207V/Q441E
mutation 251 A301V/F207V/Q441E
mutation 252 G226S/F207V/Q441E
mutation 253 V257I/F207V/Q441E
mutation 254 D619E/F207V/Q441E
mutation 255 Y339H/F207V/Q441E
mutation 256 N607K/F207V/Q441E
mutation 257 A324V/F207V/Q441E
mutation 258 Q229H/F207V/Q441E
mutation 259 W327G/F207V/Q441E
mutation 260 A301V/L439V/A537G/N607K
mutation 261 K83A/Q229H/A301V/D313E/A324V/L439V/A537G/N607K
mutation 262 Q229H/V257I/A301V/A324V/Q441E/A537G/N607K
mutation 263 Q229H/A301V/A324V/Q441E/A537G/N607K
mutation 264 Q229H/V257I/A301V/D313E/A324V/Q441E/A537G/N607K
mutation 265 T72A/A137S/A301V/L439V/Q441E/A537G/N607K
mutation 266 T72A/A137S/A301V/Q441E/A537G/N607K
mutation 267 T72A/A137S/Q229H/A301V/A324V/L439V/A537G/N607K
mutation 268 T72A/A137S/Q229H/A301V/A324V/L439V/Q441E/A537G/N607K
mutation 269 T72A/ Q229H/V257I/A301V/D313E/A324V/L439V/Q441E/A537G/N607K
mutation 270 T72A/ Q229H/V257I/A301V/D313E/A324V/Q441E/A537G/N607K
mutation 271 T72A/A137S/Q229P/A301V/L439V/Q441E/A537G/N607K
mutation 272 T72A/A137S/Q229L/A301V/L439V/Q441E/A537G/N607K
mutation 273 T72A/A137S/Q229G/A301V/L439V/Q441E/A537G/N607K
mutation 274 T72A/Q229I/V257I/A301V/D313E/A324V/L439V/Q441E/A537G/N607K
mutation 275 T72A/A137S/I228G/Q229P/A301V/L439V/Q441E/A537G/N607K
mutation 276 T72A/A137S/I228L/Q229P/A301V/L439V/Q441E/A537G/N607K
mutation 277 T72A/A137S/I228D/Q229P/A301V/L439V/Q441E/A537G/N607K
mutation 278 T72A/A137S/Q229P/I230D/A301V/L439V/Q441E/A537G/N607K
mutation 279 T72A/A137S/Q229P/I230V/A301V/L439V/Q441E/A537G/N607K
mutation 280 T72A/I228S/Q229H/V257I/A301V/D313E/A324V/L439V/Q441E/A537G/ N607K
mutation 281 T72A/Q229H/S256C/V257I/A301V/D313E/A324V/L439V/Q441E/A537G/ N607K
mutation 282 T72A/A137S/Q229P/V257I/A301V/A324V/L439V/Q441E/A537G/N607K
mutation 283 T72A/A137S/Q229P/A301V/A324V/L439V/Q441E/A537G/N607K
mutation 284 T72A/Q229P/V257I/A301G/D313E/A324V/Q441E/A537G/N607K
mutation 285 T72A/Q229P/V257I/A301V/D313E/A324V/Q441E/A537G/N607K
mutation 286 T72A/A137S/V184A/Q229P/V257I/A301V/A324V/L439V/Q441E/A537G/ N607K
mutation 287 T72A/A137S/V184G/Q229P/V257I/A301V/A324V/L439V/Q441E/A537G/ N607K
mutation 288 T72A/A137S/V184N/Q229P/V257I/A301V/A324V/L439V/Q441E/A537G/ N607K
mutation 289 T72A/A137S/V184S/Q229P/V257I/A301V/A324V/L439V/Q441E/A537G/ N607K
mutation 290 T72A/A137S/V184T/Q229P/V257I/A301V/A324V/L439V/Q441E/A537G/ N607K
mutation 324 V184A/V257Y
mutation 325 V184A/W187A
mutation 326 V184A/N442D
mutation 327 V184P/N442D
mutation 328 V184A/N442D/L439V
mutation 329 A301V/L439V/A537G/N607K/V184A
mutation 330 A301V/L439V/A537G/N607K/V184P
mutation 331 A301V/L439V/A537G/N607K/V257Y
mutation 332 A301V/L439V/A537G/N607K/W187A
mutation 333 A301V/L439V/A537G/N607K/F211A
mutation 334 A301V/L439V/A537G/N607K/Q441E
mutation 335 A301V/L439V/A537G/N607K/N442D
mutation 336 A301V/L439V/A537G/N607K/V184/V184/F207V
mutation 337 A301V/L439V/A537G/N607K/V184A/A182G
mutation 338 T72A/A137S/Q229P/V257I/A301V/A324V/L439V/A537G/N607K/V184A/ N442D
mutation 339 T72A/A137S/Q229P/V257I/A301V/A324V/L439V/A537G/N607K/V184A/ N442D/T185F
mutation 340 T72A/A137S/Q229P/V257I/A301V/A324V/L439V/Q441E/A537G/N607K/ V184A/K83A
mutation 341 T72A/A137S/Q229P/V257I/A301V/A324V/L439V/Q441E/A537G/N607K/ V184A/W187A
mutation 342 T72A/A137S/Q229P/V257I/A301V/A324V/L439V/Q441E/A537G/N607K/ V184A/F211A
mutation 343 T72A/A137S/Q229P/V257I/A301V/A324V/L439V/Q441E/A537G/N607K/ V184A/V178G
mutation 344 T72A/A137S/Q229P/V257I/A301V/A324V/L439V/Q441E/A537G/N607K/ V184A/T185A
mutation 345 T72A/A137S/Q229P/V257I/A301V/A324V/L439V/Q441E/A537G/N607K/ V184A/A182G
mutation 346 T72A/A137S/Q229P/V257I/A301V/A324V/L439V/Q441E/A537G/N607K/ V184A/K314R
mutation 347 T72A/A137S/Q229P/V257I/A301V/A324V/L439V/Q441E/A537G/N607K/ V184A/A515V
mutation 348 T72A/A137S/Q229P/V257I/A301V/A324V/L439V/Q441E/A537G/N607K/ V184A/L66F
mutation 349 T72A/A137S/Q229P/V257I/A301V/A324V/L439V/Q441E/A537G/N607K/ V184A/S315R
mutation 350 T72A/A137S/Q229P/V257I/A301V/A324V/L439V/Q441E/A537G/N607K/ V184A/K484I
mutation 351 T72A/A137S/Q229P/V257I/A301V/A324V/L439V/Q441E/A537G/N607K/ V184A/V213A
mutation 352 T72A/A137S/Q229P/V257I/A301V/A324V/L439V/Q441E/A537G/N607K/ V184A/A245S
mutation 353 T72A/Al37S/Q229P/V257I/A301V/A324V/L439V/Q441E/A537G/N607K/ V184A/P214H
mutation 354 T72A/A137S/Q229P/V257I /A301V/A324V/L439V/Q441E /A537G/N607K/V184A/L263M
mutation 355 T72A/A137S/Q229P/V257I /A301V/A324V/L439V/Q441E/A537G/N607K/V184A/P183A
mutation 356 T72A/A137S/Q229P/V257I/A301V/A324V/L439V/Q441E/A537G/N607K/ V184A/T185K
mutation 357 T72A/A137S/Q229P/V257I/A301V/A324V/L439V/Q441E/A537G/N607K/ V184A/T185D
mutation 358 T72A/A137S/Q229P/V257I/A301V/A324V/L439V/Q441E/A537G/N607K/ V184A/T185C
mutation 359 T72A/A137S/Q229P/V257I/A301V/A324V/L439V/Q441E/A537G/N607K/ V184A/T185S
mutation 360 T72A/A137S/Q229P/V257I/A301V/A324V/L439V/Q441E/A537G/N607K/ V184A/T185F
mutation 361 T72A/A137S/Q229P/V257I/A301V/A324V/L439V/Q441E/A537G/N607K/ V184A/T185P
mutation 362 T72A/A137S/Q229P/V257I/A301V/A324V/L439V/Q441E/A537G/N607K/ V184A/T185N
mutation 363 T72A/A137S/Q229P/V257I/A301V/A324V/L439V/Q441E/A537G/N607K/ V184A/P183A/A182G
mutation 364 T72A/A137S/Q229P/V257I/A301V/A324V/L439V/Q441E/A537G/N607K/ V184A/P183A/A182S
mutation 365 T72A/A137S/Q229P/V257I /A301V/A324V/L439V/Q441E/A537G/N607K/V184A/T185F/N442D
mutation 366 T72A/A137S/Q229P/V257I /A301V/A324V/L439V/Q441E/A537G/N607K/V184A/L66F/E80K/I157L/ A182G/P214H/L263M
mutation 367 T72A/A137S/Q229P/V257I/A301V/A324V/L439V/Q441E/A537G/N607K/ V184A/L66F/E80K/I157L/A182G/P214H/L263M/Y328F
mutation 368 T72A/A137S/Q229P/V257I /A301V/A324V/L439V/Q441E/A537G/N607K/V184A/L66F/Y81A/I157L/ A182G/P214H/L263M/Y328F
mutation 369 T72A/A137S/Q229P/V257I/A301V/A324V/L439V/Q441E/A537G/N607K/ V184A/L66F/E80K/I157L/A182G/T210L/L263M/Y328F
mutation 370 A301V/L439V/A537G/N607K/Q441K
mutation 371 T72A/A137S/Q229P/V257I/A301V/A324V/L439V/Q441E/A537G/N607K/ V184A/I157L
mutation 372 T72A/A137S/Q229P/V257I/A301V/A324V/L439V/Q441E/A537G/N607K/ V184A/G161A
mutation 373 T72A/A137S/Q229P/V257I/A301V/A324V/L439V/Q441E/A537G/N607K/ V184A/Y328F
mutation 374 F207V/G226S
mutation 375 F207V/W327G
mutation 376 F207V/Y339H
mutation 377 F207V/D619E.

6. The mutant protein according to claim 5 wherein, in said amino acid sequence comprising one or more mutations selected from any of the mutations 239 to 290 and 324 to 377, said amino acid sequence further comprises at other than the mutated position(s) one or more amino acid mutations selected from the group consisting of substitutions, deletions, insertions, additions and inversions, said mutant protein having a peptide-synthesizing activity.

7. The mutant protein according to claim 5 or 6 comprising at least the mutation 260.

8. The mutant protein according to any one of claim 5 to 7 comprising at least the mutation 286.

9. A polynucleotide encoding the amino acid sequence of the mutant protein according to any one of claim 1 to 8.

10. A recombinant polynucleotide comprising the polynucleotide according to claim 9.

11. transformed microorganism comprising the recombinant polynucleotide according to claim 10.

12. A method for producing a mutant protein comprising culturing the transformed microorganism according to claim 11 in a medium, to accumulate the mutant protein in the medium and/or the transformed microorganism.

13. A method for producing a peptide comprising performing a peptide-synthesizing reaction in the presence of the mutant protein according to any one of claim 1 to 8.

14. A method for producing a peptide comprising culturing the transformed microorganism according to claim 11 in a medium to accumulate the mutant protein in the medium and/or the transformed microorganism for performing a peptide-synthesizing reaction.

15. A method for producing α-L-aspartyl-L-phenylalanine-β-ester comprising reacting L-aspartic acid-α,β-diester and L-phenylalanine in the presence of the mutant protein according to any one of claim 1 to 8.

16. A method for producing α-L-aspartyl-L-phenylalanine-β-ester comprising culturing the transformed microorganism according to claim 11 in a medium to accumulate the mutant protein in the medium and/or the transformed microorganism for performing a reaction of L-aspartic acid-α,β-diester and L-phenylalanine.

17. A method for designing and producing a mutant protein having a peptide-synthesizing activity comprising:
analyzing a protein having an amino acid sequence of SEQ ID NO:208 by X-ray crystal structure analysis to obtain a tertiary structure thereof;
predicting a substrate binding site of the protein based on said tertiary structure; and
substituting, inserting or deleting an amino acid residue located at said substrate binding site.

18. A mutant protein having an amino acid sequence comprising one or more amino acid substitutions, insertions or deletions at positions 67 to 70, 72 to 88, 100, 102, 103, 106, 107, 113 to 117, 130, 155 to 163, 165, 166, 180 to 188, 190 to 195, 200 to 235, 259, 273, 276, 278, 292 to 294, 296, 298, 299, 300 to 304, 325 to 328, 330 to 340, and 437 to 447 in an amino acid sequence in a tertiary structure of a protein having an amino acid sequence of SEQ ID NO:208, and having a peptide-synthesizing activity.

19. A mutant protein of a protein having a peptide-synthesizing activity wherein:
three dimensional structures of the mutant protein and a protein having an amino acid sequence of SEQ ID NO:209 are similar as a result of determination by a threading method;
in alignment obtained upon the determination, at least one or more amino acid residues are substituted, inserted or deleted at positions corresponding to positions 67 to 70, 72 to 88, 100, 102, 103, 106, 107, 113 to 117, 130, 155 to 163, 165, 166, 180 to 188, 190 to 195, 200 to 235, 259, 273, 276, 278, 292 to 294, 296, 298, 299, 300 to 304, 325 to 328, 330 to 340 and 437 to 447 in the amino acid sequence of SEQ ID NO:209; and
said mutant protein has the peptide-synthesizing activity.

20. A mutant protein of a protein having a peptide-synthesizing activity wherein:
when an alignment of primary sequences of the mutant protein and a protein having an amino acid sequence of SEQ ID NO:209 or an alignment of three dimensional structures of the mutant protein and the protein having the amino acid sequence of SEQ ID NO:209 is performed, homology of the primary sequences is 25% or more, and at least one or more amino acid residues are substituted, inserted or deleted at positions corresponding to positions 67 to 70, 72 to 88, 100, 102, 103, 106, 107, 113 to 117, 130, 155 to 163, 165, 166, 180 to 188, 190 to 195, 200 to 235, 259, 273, 276, 278, 292 to 294, 296, 298, 299, 300 to 304, 325 to 328, 330 to 340 and 437 to 447 in the amino acid sequence of SEQ ID NO:209; and
said mutant protein has the peptide-synthesizing activity.

21. A mutant protein having one or more changes in a tertiary structure selected from the following (a) to (i) in the tertiary structure of a protein having an amino acid sequence of SEQ ID NO:208, said mutant protein having a peptide-synthesizing activity:
(a) at least one or more amino acid residue substitutions, insertions or deletions at any of positions 79 to 82 in the amino acid sequence of SEQ ID NO:208;
(b) at least one or more amino acid residue substitutions, insertions or deletions at any of positions 84, 88, 89 and 92 in the amino acid sequence of SEQ ID NO:208;
(c) at least one or more amino acid residue substitutions, insertions or deletions at any of positions 72 75 and 77 in the amino acid sequence of SEQ ID NO:208;
(d) at least one or more amino acid residue substitutions, insertions or deletions at any of positions 159, 161, 162, 184, 187 and 276 in the amino acid sequence of SEQ ID NO:208;
(e) at least one or more amino acid residue substitutions, insertions or deletions at any of positions 70, 106, 113, 115, 193, 207, 209 to 212, 216 and 259 in the amino acid sequence of SEQ ID NO:208;
(f) at least one or more amino acid residue substitutions, insertions or deletions at any of positions 200, 202 to 205, 207 and 228 in the amino acid sequence of SEQ ID NO:208;
(g) at least one or more amino acid residue substitutions, insertions or deletions at any of positions 233, 234 and 439 in the amino acid sequence of SEQ ID NO:208;
(h) at least one or more amino acid residue substitutions, insertions or deletions at any of positions 328, 339, 340, 445 and 446 in the amino acid sequence of SEQ ID NO:208; and
(i) at least one or more amino acid residue substitutions, insertions or deletions at any of positions 87, 155, 157 and 160 in the amino acid sequence of SEQ ID NO:208.

22. A mutant protein of a protein having a peptide-synthesizing activity wherein:
three dimensional structures of the mutant protein and a protein having an amino acid sequence of SEQ ID NO:209 are similar as a result of determination by a threading method, and in alignment obtained upon the determination, one or more changes selected from the following (a') to (i') are present; and
the mutant protein has a peptide-synthesizing activity:
(a') at least one or more amino acid residue substitutions, insertions or deletions in the tertiary structure corresponding to any of positions 79 to 82 in the amino acid sequence of SEQ ID NO:209;
(b') at least one or more amino acid residue substitutions, insertions or deletions in the tertiary structure corresponding to any of positions 84, 88, 89 and 92 in the amino acid sequence of SEQ ID NO:209;
(c') at least one or more amino acid residue substitutions, insertions or deletions in the tertiary structure corresponding to any of positions 72, 75 and 77 in the amino acid sequence of SEQ ID NO:209;
(d') at least one or more amino acid residue substitutions, insertions or deletions in the tertiary structure corresponding to any of positions 159, 161, 162, 184, 187 and 276 in the amino acid sequence of SEQ ID NO:209;
(e') at least one or more amino acid residue substitutions, insertions or deletions in the tertiary structure corresponding to any of positions 70, 106, 113, 115, 193, 207, 209 to 212, 216 and 259 in the amino acid sequence of SEQ ID NO:209;
(f') at least one or more amino acid residue substitutions, insertions or deletions in the tertiary structure corresponding to any of positions 200, 202 to 205, 207 and 228 in the amino acid sequence of SEQ ID NO:209;
(g') at least one or more amino acid residue substitutions, insertions or deletions in the tertiary structure corresponding to any of positions 233, 234 and 439 in the amino acid sequence of SEQ ID NO:209;
(h') at least one or more amino acid residue substitutions, insertions or deletions in the tertiary structure corresponding to any of positions 328, 339, 340, 445 and 446 in the amino acid sequence of SEQ ID NO:209; and
(i') at least one or more amino acid residue substitutions, insertions or deletions in the tertiary structure corresponding to any of positions 87, 155, 157 and 160 in the amino acid sequence of SEQ ID NO:209.

23. A mutant protein of a protein having a peptide-synthesizing activity wherein:
when an alignment of primary sequences of the mutant protein and a protein having an amino acid sequence of SEQ ID NO:209 or an alignment of three dimensional structures of the mutant protein and the protein having the amino acid sequence of SEQ ID NO:209 is performed, homology of the primary sequences is 25% or more, and one or more changes selected from the following (a'') to (i'') are present; and
said mutant protein has the peptide-synthesizing activity:
(a'') at least one or more amino acid residue substitutions, insertions or deletions in the tertiary structure corresponding to any of positions 79 to 82 in the amino acid sequence of SEQ ID NO:209;
(b'') at least one or more amino acid residue substitutions, insertions or deletions in the tertiary structure corresponding to any of positions 84, 88, 89 and 92 in the amino acid sequence of SEQ ID NO:209;
(c'') at least one or more amino acid residue substitutions, insertions or deletions in the tertiary structure corresponding to any of positions 72, 75 and 77 in the amino acid sequence of SEQ ID NO:209;
(d'') at least one or more amino acid residue substitutions, insertions or deletions in the tertiary structure corresponding to any of positions 159, 161, 162, 184, 187 and 276 in the amino acid sequence of SEQ ID NO:209;
(e'') at least one or more amino acid residue substitutions, insertions or deletions in the tertiary structure corresponding to any of positions 70, 106, 113, 115, 193, 207, 209 to 212, 216 and 259 in the amino acid sequence of SEQ ID NO:209;
(f'') at least one or more amino acid residue substitutions, insertions or deletions in the tertiary structure corresponding to any of positions 200, 202 to 205, 207 and 228 in the amino acid sequence of SEQ ID NO:209;
(g'') at least one or more amino acid residue substitutions, insertions or deletions in the tertiary structure corresponding to any of positions 233, 234 and 439 in the amino acid sequence of SEQ ID NO:209;
(h'') at least one or more amino acid residue substitutions, insertions or deletions in the tertiary structure corresponding to any of positions 328, 339, 340, 445 and 446 in the amino acid sequence of SEQ ID NO:209; and
(i'') at least one or more amino acid residue substitutions, insertions or deletions in the tertiary structure corresponding to any of positions 87, 155, 157 and 160 in the amino acid sequence of SEQ ID NO:209.

24. A mutant protein having at least one or more amino acid residue substitutions, insertions or deletions at positions 67, 69, 70, 72 to 85, 103, 106, 107, 113 to 116, 165, 182, 183, 185, 187, 188, 190, 200, 202, 204 to 206, 209 to 211, 213 to 235, 301, 328, 338 to 340, 440 to 442 and 446 in a tertiary structure of a protein having an amino acid sequence of SEQ ID NO:208, said mutant protein having a peptide-synthesizing activity.

25. A mutant protein having at least one or more amino acid residue substitutions, insertions or deletions at positions 67, 69, 70, 72 to 84, 106, 107, 114, 116, 183, 185, 187, 188, 202, 204 to 206, 209, 211, 213 to 233, 235, 328, 338 to 442 and 446 in a tertiary structure of a protein having an amino acid sequence of SEQ ID NO:208, said mutant protein having a peptide-synthesizing activity.

26. A mutant protein having at least one or more amino acid residue substitutions, insertions or deletions at positions 67, 70, 72 to 75, 77 to 79, 81 to 84, 114, 116, 185, 188, 202, 204, 206, 209, 211, 213 to 215, 218 to 224, 226 to 233, 235, 328, 338 to 441 and 446 in a tertiary structure of a protein having an amino acid sequence of SEQ ID NO:208, said mutant protein having a peptide-synthesizing activity.

27. A mutant protein having an amino acid sequence comprising one or more mutations selected from any of the following mutations L1 to L335 in an amino acid sequence of SEQ ID NO:208:
mutation L1 N67K
mutation L2 N67L
mutation L3 N67S
mutation L4 T69I
mutation L5 T69M
mutation L6 T69Q
mutation L7 T69R
mutation L8 T69V
mutation L9 P70G
mutation L10 P70N
mutation L11 P70S
mutation L12 P70T
mutation L13 P70V
mutation L14 A72C
mutation L15 A72D
mutation L16 A72E
mutation L17 A72I
mutation L18 A72L
mutation L19 A72M
mutation L20 A72N
mutation L21 A72Q
mutation L22 A72S
mutation L23 A72V
mutation L24 V73A
mutation L25 V73I
mutation L26 V73L
mutation L27 V73M
mutation L28 V73N
mutation L29 V73S
mutation L30 V73T
mutation L31 S74A
mutation L32 S74F
mutation L33 S74K
mutation L34 S74N
mutation L35 S74T
mutation L36 S74V
mutation L37 P75A
mutation L38 P75D
mutation L39 P75L
mutation L40 P75S
mutation L41 Y76F
mutation L42 Y76H
mutation L43 Y76I
mutation L44 Y76V
mutation L45 Y76W
mutation L46 G77A
mutation L47 G77F
mutation L48 G77K
mutation L49 G77M
mutation L50 G77N
mutation L51 G77P
mutation L52 G77S
mutation L53 G77T
mutation L54 Q78F
mutation L55 Q78L
mutation L56 N79D
mutation L57 N79L
mutation L58 N79R
mutation L59 N79S
mutation L60 E80D
mutation L61 E80F
mutation L62 E80L
mutation L63 E80P
mutation L64 E80S
mutation L65 Y81A
mutation L66 Y81C
mutation L67 Y81D
mutation L68 Y81E
mutation L69 Y81F
mutation L70 Y81H
mutation L71 Y81K
mutation L72 Y81L
mutation L73 Y81N
mutation L74 Y81S
mutation L75 Y81T
mutation L76 Y81W
mutation L77 K82D
mutation L78 K82L
mutation L79 K82P
mutation L80 K82S
mutation L81 K83D
mutation L82 K83F
mutation L83 K83L
mutation L84 K83P
mutation L85 K83S
mutation L86 K83V
mutation L87 S84D
mutation L88 S84F
mutation L89 S84K
mutation L90 S84L
mutation L91 S84N
mutation L92 S84Q
mutation L93 L85F
mutation L94 L85I
mutation L95 L85P
mutation L96 L85V
mutation L97 N87E
mutation L98 N87Q
mutation L99 F88E
mutation L100 V103I
mutation L101 V103L
mutation L102 K106A
mutation L103 K106F
mutation L104 K106L
mutation L105 K106Q
mutation L106 K106S
mutation L107 W107A
mutation L108 W107Y
mutation L109 F113A
mutation L110 F113W
mutation L111 F113Y
mutation L112 E114A
mutation L113 E114D
mutation L114 D115E
mutation L115 D115Q
mutation L116 D115S
mutation L117 I116F
mutation L118 I116K
mutation L119 I116L
mutation L120 I116M
mutation L121 I116N
mutation L122 I116T
mutation L123 I116V
mutation L124 I157K
mutation L125 I157L
mutation L126 Y159G
mutation L127 Y159N
mutation L128 Y159S
mutation L129 P160G
mutation L130 G161A
mutation L131 F162L
mutation L132 F162Y
mutation L133 Y163I
mutation L134 T165V
mutation L135 Q181F
mutation L136 A182G
mutation L137 A182S
mutation L138 P183A
mutation L139 P183G
mutation L140 P183S
mutation L141 T185A
mutation L142 T185G
mutation L143 T185V
mutation L144 W187A
mutation L145 W187F
mutation L146 W187H
mutation L147 W187Y
mutation L148 Y188F
mutation L149 Y188L
mutation L150 Y188W
mutation L151 G190A
mutation L152 G190D
mutation L153 F193W
mutation L154 H194D
mutation L155 F200A
mutation L156 F200L
mutation L157 F200S
mutation L158 F200V
mutation L159 L201Q
mutation L160 L201S
mutation L161 Q202A
mutation L162 Q202D
mutation L163 Q202F
mutation L164 Q202S
mutation L165 Q202T
mutation L166 Q202V
mutation L167 D203E
mutation L168 A204G
mutation L169 A204L
mutation L170 A204S
mutation L171 A204T
mutation L172 A204V
mutation L173 F205L
mutation L174 F205Q
mutation L175 F205V
mutation L176 F205W
mutation L177 T206F
mutation L178 T206K
mutation L179 T206L
mutation L180 F207I
mutation L181 F207W
mutation L182 F207Y
mutation L183 M208A
mutation L184 M208L
mutation L185 S209F
mutation L186 S209K
mutation L187 S209L
mutation L188 S209N
mutation L189 S209V
mutation L190 T210A
mutation L191 T210L
mutation L192 T210Q
mutation L193 T210V
mutation L194 F211A
mutation L195 F211I
mutation L196 F211L
mutation L197 F211M
mutation L198 F211V
mutation L199 F211W
mutation L200 F211Y
mutation L201 G212A
mutation L202 V213D
mutation L203 V213F
mutation L204 V213K
mutation L205 V213S
mutation L206 P214D
mutation L207 P214F
mutation L208 P214K
mutation L209 P214S
mutation L210 R215A
mutation L211 R215I
mutation L212 R215K
mutation L213 R215Q
mutation L214 R215S
mutation L215 R215T
mutation L216 R215Y
mutation L217 P216D
mutation L218 P216K
mutation L219 K217D
mutation L220 P218F
mutation L221 P218L
mutation L222 P218Q
mutation L223 P218S
mutation L224 I219D
mutation L225 I219F
mutation L226 I219K
mutation L227 T220A
mutation L228 T220D
mutation L229 T220F
mutation L230 T220K
mutation L231 T220L
mutation L232 T220S
mutation L233 P221A
mutation L234 P221D
mutation L235 P221F
mutation L236 P221K
mutation L237 P221L
mutation L238 P221S
mutation L239 D222A
mutation L240 D222F
mutation L241 D222L
mutation L242 D222R
mutation L243 Q223F
mutation L244 Q223K
mutation L245 Q223L
mutation L246 Q223S
mutation L247 F224A
mutation L248 F224D
mutation L249 F224G
mutation L250 F224K
mutation L251 F224L
mutation L252 K225D
mutation L253 K225G
mutation L254 K225S
mutation L255 G226A
mutation L256 G226F
mutation L257 G226L
mutation L258 G226N
mutation L259 G226S
mutation L260 K227D
mutation L261 K227F
mutation L262 K227S
mutation L263 I228A
mutation L264 I228F
mutation L265 I228K
mutation L266 I228S
mutation L267 P229A
mutation L268 P229D
mutation L269 P229K
mutation L270 P229L
mutation L271 P229S
mutation L272 I230A
mutation L273 I230F
mutation L274 I230K
mutation L275 I230S
mutation L276 K231F
mutation L277 K231L
mutation L278 K231S
mutation L279 E232D
mutation L280 E232F
mutation L281 E232G
mutation L282 E232L
mutation L283 E232S
mutation L284 A233D
mutation L285 A233F
mutation L286 A233H
mutation L287 A233K
mutation L288 A233L
mutation L289 A233N
mutation L290 A233S
mutation L291 D234L
mutation L292 D234S
mutation L293 K235D
mutation L294 K235F
mutation L295 K235L
mutation L296 K235S
mutation L297 F259Y
mutation L298 R276A
mutation L299 R276Q
mutation L300 A298S
mutation L301 D300N
mutation L302 V301M
mutation L303 Y328F
mutation L304 Y328H
mutation L305 Y328M
mutation L306 Y328W
mutation L307 W332H
mutation L308 E336A
mutation L309 N338A
mutation L310 N338F
mutation L311 Y339K
mutation L312 Y339L
mutation L313 Y339T
mutation L314 L340A
mutation L315 L340I
mutation L316 L340V
mutation L317 V439P
mutation L318 I440F
mutation L319 I440V
mutation L320 E441F
mutation L321 E441M
mutation L322 E441N
mutation L323 N442A
mutation L324 N442L
mutation L325 R443S
mutation L326 T444W
mutation L327 R445G
mutation L328 R445K
mutation L329 E446A
mutation L330 E446F
mutation L331 E446Q
mutation L332 E446S
mutation L333 E446T
mutation L334 Y447L
mutation L335 Y447S.

28. The mutant protein according to claim 20 wherein, in said amino acid sequence comprising one or more mutations selected from any of the mutations L1 to L335, said amino acid sequence further comprises at other than the mutated position(s) one or several amino acid mutations selected from the group consisting of substitutions, deletions, insertions, additions and inversions, said mutant protein having a peptide-synthesizing activity.

29. The mutant protein according to claim 27 or 28 comprising at least the mutation L124 or L125.

30. The mutant protein according to any one of claim 27 to 29 above comprising at least the mutation L303.

31. The mutant protein according to any one of claims 27 to 30 comprising at least the mutation L12.

32. The mutant protein according to any one of claims 27 to 31 comprising at least the mutation L127.

33. The mutant protein according to any one of claims 27 to 32 comprising at least the mutation L195 or L199.

34. The mutant protein according to any one of claims 27 to 33 comprising at least the mutation L130.

35. The mutant protein according to any one of claims 27 to 34 comprising at least the mutation L115.

36. The mutant protein according to any one of claims 27 to 35 comprising at least the mutation L316.

37. The mutant protein according to any one of claims 27 to 36 above comprising at least the mutation L99.

38. The mutant protein according to any one of claims 27 to 37 above comprising at least the mutation L15 or L16.

39. The mutant protein according to any one of claims 27 to 38 comprising at least the mutation L131.

40. The mutant protein according to any one of claims 27 to 39 comprising at least the mutation L284.

41. The mutant protein according to any one of claims 27 to 40 comprising at.least the mutation L191.

42. The mutant protein according to any one of claims 27 to 41 comprising at least the mutation L65.

43. The mutant protein according to any one of claims 27 to 42 comprising at least the mutation L265.

44. The mutant protein according to any one of claims 27 to 43 comprising at least the mutation L317.

45. The mutant protein according to any one of claims 27 to 44 comprising at least the mutation L255.

46. The mutant protein according to any one of claims 27 to 45 comprising at least the mutation L52.

47. The mutant protein according to any one of claims 27 to 46 comprising at least the mutation L155.

48. The mutant protein according to any one of claims 27 to 47 comprising at least the mutation L298.

49. The mutant protein according to any one of claims 27 to 48 comprising at least the mutation L201.

50. The mutant protein according to any one of claims 27 to 49 comprising at least the mutation L145.

51. The mutant protein according to any one of claims 27 to 50 comprising at least the mutation L170.

52. The mutant protein according to any one of claims 27 to 51 comprising at least the mutation L87.

53. The mutant protein according to any one of claims 27 to 52 comprising at least the mutation L60.

54. The mutant protein according to any one of claims 27 to 53 comprising at least the mutation L110.

55. A mutant protein having an amino acid sequence comprising one or more mutations selected from any of the following mutations M1 to M642 in an amino acid sequence of SEQ ID NO:208:
mutation M1 T69N/I157L
mutation M2 T69Q/I157L
mutation M3 T69S/I157L
mutation M4 P70A/I157L
mutation M5 P70G/I157L
mutation M6 P70I/I157L
mutation M7 P70L/I157L
mutation M8 P70N/I157L
mutation M9 P70S/I157L
mutation M10 P70T/I157L
mutation M11 P70T/T210L
mutation M12 P70T/Y328F
mutation M13 P70V/I157L
mutation M14 A72E/G77S
mutation M15 A72E/E80D
mutation M16 A72E/Y81A
mutation M17 A72E/S84D
mutation M18 A72E/F113W
mutation M19 A72E/I157L
mutation M20 A72E/G161A
mutation M21 A72E/F162L
mutation M22 A72E/A184G
mutation M23 A72E/W187F
mutation M24 A72E/F200A
mutation M25 A72E/A204S
mutation M26 A72E/T210L
mutation M27 A72E/F211L
mutation M28 A72E/F211W
mutation M29 A72E/G226A
mutation M30 A72E/I228K
mutation M31 A72E/A233D
mutation M32 A72E/Y328F
mutation M33 A72S/I157L
mutation M34 A72V/Y328F
mutation M35 V73A/I157L
mutation M36 V73I/I157L
mutation M37 S74A/I157L
mutation M38 S74N/I157L
mutation M39 S74T/I157L
mutation M40 S74V/I157L
mutation M41 G77A/I157L
mutation M42 G77F/I157L
mutation M43 G77M/I157L
mutation M44 G77P/I157L
mutation M45 G77S/E80D
mutation M46 G77S/Y81A
mutation M47 G77S/S84D
mutation M48 G77S/F113W
mutation M49 G77S/I157L
mutation M50 G77S/Y159N
mutation M51 G77S/Y159S
mutation M52 G77S/G161A
mutation M53 G77S/F162L
mutation M54 G77S/A184G
mutation M55 G77S/W187F
mutation M56 G77S/F200A
mutation M57 G77S/A204S
mutation M58 G77S/T210L
mutation M59 G77S/F211L
mutation M60 G77S/F211W
mutation M61 G77S/I228K
mutation M62 G77S/A233D
mutation M63 G77S/R276A
mutation M64 G77S/Y328F
mutation M65 E80D/Y81A
mutation M66 E80D/F113W
mutation M67 E80D/I157L
mutation M68 E80D/Y159N
mutation M69 E80D/G161A
mutation M70 E80D/A184G
mutation M71 E80D/F211W
mutation M72 E80D/Y328F
mutation M73 E80S/I157L
mutation M74 Y81A/F113W
mutation M75 Y81A/I157L
mutation M76 Y81A/Y159N
mutation M77 Y81A/Y159S
mutation M78 Y81A/G161A
mutation M79 Y81A/A184G
mutation M80 Y81A/W187F
mutation M81 Y81A/F200A
mutation M82 Y81A/T210L
mutation M83 Y81A/F211W
mutation M84 Y81A/F211Y
mutation M85 Y81A/G226A
mutation M86 Y81A/I228K
mutation M87 Y81A/A233D
mutation M88 Y81A/Y328F
mutation M89 Y81H/I157L
mutation M90 Y81N/I157L
mutation M91 K83P/I157L
mutation M92 S84A/I157L
mutation M93 S84D/F113W
mutation M94 S84D/I157L
mutation M95 S84D/Y159N
mutation M96 S84D/G161A
mutation M97 S84D/A184G
mutation M98 S84D/Y328F
mutation M99 S84E/I157L
mutation M100 S84F/I157L
mutation M101 S84K/I157L
mutation M102 L85F/I157L
mutation M103 L85I/I157L
mutation M104 L85P/I157L
mutation M105 L85V/I157L
mutation M106 N87A/I157L
mutation M107 N87D/I157L
mutation M108 N87E/I157L
mutation M109 N87G/I157L
mutation M110 N87Q/I157L
mutation M111 N87S/I157L
mutation M112 F88A/I157L
mutation M113 F88D/I157L
mutation M114 F88E/I157L
mutation M115 F88E/Y328F
mutation M116 F88L/I157L
mutation M117 F88T/I157L
mutation M118 F88V/I157L
mutation M119 F88Y/I157L
mutation M120 K106H/I157L
mutation M121 K106L/I157L
mutation M122 K106M/I157L
mutation M123 K106Q/I157L
mutation M124 K106R/I157L
mutation M125 K106S/I157L
mutation M126 K106V/I157L
mutation M127 W107A/I157L
mutation M128 W107A/Y328F
mutation M129 W107Y/I157L
mutation M130 W107Y/T206Y
mutation M131 W107Y/K217D
mutation M132 W107Y/P218L
mutation M133 W107Y/T220L
mutation M134 W107Y/P221D
mutation M135 W107Y/Y328F
mutation M136 F113A/I157L
mutation M137 F113H/I157L
mutation M138 F113N/I157L
mutation M139 F113V/I157L
mutation M140 F113W/I157L
mutation M141 F113W/Y159N
mutation M142 F113W/Y159S
mutation M143 F113W/G161A
mutation M144 F113W/F162L
mutation M145 F113W/A184G
mutation M146 F113W/W187F
mutation M147 F113W/F200A
mutation M148 F113W/T206Y
mutation M149 F113W/T210L
mutation M150 F113W/F211L
mutation M151 F113W/F211W
mutation M152 F113W/F211Y
mutation M153 F113W/V213D
mutation M154 F113W/K217D
mutation M155 F113W/T220L
mutation M156 F113W/P221D
mutation M157 F113W/G226A
mutation M158 F113W/I228K
mutation M159 F113W/A233D
mutation M160 F113W/R276A
mutation M161 F113Y/I157L
mutation M162 FI13Y/F211W
mutation M163 E114D/I157L
mutation M164 D115A/I157L
mutation M165 D115E/I157L
mutation M166 D115M/I157L
mutation M167 D115N/I157L
mutation M168 D115Q/I157L
mutation M169 D115S/I157L
mutation M170 D115V/I157L
mutation M171 I157L/Y159I
mutation M172 I157L/Y159L
mutation M173 I157L/Y159N
mutation M174 I157L/Y159S
mutation M175 I157L/Y159V
mutation M176 I157L/P160A
mutation M177 I157L/P160S
mutation M178 I157L/G161A
mutation M179 I157L/F162L
mutation M180 I157L/F162M
mutation M181 I157L/F162N
mutation M182 I157L/F162Y
mutation M183 I157L/T165L
mutation M184 I157L/T165V
mutation M185 I157L/Q181A
mutation M186 I157L/Q181F
mutation M187 I157L/Q181N
mutation M188 I157L/A184G
mutation M189 I157L/A184L
mutation M190 I157L/A184M
mutation M191 I157L/A184S
mutation M192 I157L/A184T
mutation M193 I157L/W187F
mutation M194 I157L/W187Y
mutation M195 I157L/F193H
mutation M196 I157L/F193I
mutation M197 I157L/F193W
mutation M198 I157L/F200A
mutation M199 I157L/F200H
mutation M200 I157L/F200L
mutation M201 I157L/F200Y
mutation M202 I157L/A204G
mutation M203 I157L/A204I
mutation M204 I157L/A204L
mutation M205 I157L/A204S
mutation M206 I157L/A204T
mutation M207 I157L/A204V
mutation M208 I157L/F205A
mutation M209 I157L/F207I
mutation M210 I157L/F207M
mutation M211 I157L/F207V
mutation M212 I157L/F207W
mutation M213 I157L/F207Y
mutation M214 I157L/M208A
mutation M215 I157L/M208K
mutation M216 I157L/M208L
mutation M217 I157L/M208T
mutation M218 I157L/M208V
mutation M219 I157L/S209F
mutation M220 I157L/S209N
mutation M221 I157L/T210A
mutation M222 I157L/T210L
mutation M223 I157L/F211I
mutation M224 I157L/F211L
mutation M225 I157L/F211V
mutation M226 I157L/F211W
mutation M227 I157L/G212A
mutation M228 I157L/G212D
mutation M229 I157L/G212S
mutation M230 I157L/R215K
mutation M231 I157L/R215L
mutation M232 I157L/R215T
mutation M233 I157L/R215Y
mutation M234 I157L/T220L
mutation M235 I157L/G226A
mutation M236 I157L/G226F
mutation M237 I157L/1228K
mutation M238 I157L/A233D
mutation M239 I157L/R276A
mutation M240 I157L/Y328A
mutation M241 I157L/Y328F
mutation M242 I157L/Y328H
mutation M243 I157L/Y328I
mutation M244 I157L/Y328L
mutation M245 I157L/Y328P
mutation M246 I157L/Y328V
mutation M247 I157L/Y328W
mutation M248 I157L/L340F
mutation M249 I157L/L340I
mutation M250 I157L/L340V
mutation M251 I157L/V439A
mutation M252 I157L/V439P
mutation M253 I157L/R445A
mutation M254 I157L/R445F
mutation M255 I157L/R445G
mutation M256 I157L/R445K
mutation M257 I157L/R445V
mutation M258 Y159N/G161A
mutation M259 Y159N/A184G
mutation M260 Y159N/A204S
mutation M261 Y159N/T210L
mutation M262 Y159N/F211W
mutation M263 Y159N/F211Y
mutation M264 Y159N/G226A
mutation M265 Y159N/I228K
mutation M266 Y159N/A233D
mutation M267 Y159N/Y328F
mutation M268 Y159S/G161A
mutation M269 Y159S/F211W
mutation M270 G161A/F162L
mutation M271 G161A/A184G
mutation M272 G161A/W187F
mutation M273 G161A/F200A
mutation M274 G161A/A204S
mutation M275 G161A/T210L
mutation M276 G161A/F211L
mutation M277 G161A/F211W
mutation M278 G161A/G226A
mutation M279 G161A/I228K
mutation M280 G161A/A233D
mutation M281 G161A/Y328F
mutation M282 F162L/A184G
mutation M283 F162L/F211W
mutation M284 F162L/A233D
mutation M285 P183A/Y328F
mutation M286 A184G/W187F
mutation M287 A184G/F200A
mutation M288 A184G/A204S
mutation M289 A184G/T210L
mutation M290 A184G/F211L
mutation M291 A184G/F211W
mutation M292 A184G/I228K
mutation M293 A184G/A233D
mutation M294 A184G/R276A
mutation M295 V184G/Y328F
mutation M296 T185A/Y328F
mutation M297 T185N/Y328F
mutation M298 W187F/F211W
mutation M299 W187F/Y328F
mutation M300 F193W/F211W
mutation M301 F200A/F211W
mutation M302 F200A/Y328F
mutation M303 L201Q/Y328F
mutation M304 L201S/Y328F
mutation M305 A204S/F211W
mutation M306 A204S/Y328F
mutation M307 T210L/F211W
mutation M308 T210L/Y328F
mutation M309 F211L/A233D
mutation M310 F211L/Y328F
mutation M311 F211W/I228K
mutation M312 F211W/A233D
mutation M313 F211W/Y328F
mutation M314 R215A/Y328F
mutation M315 R215L/Y328F
mutation M316 T220L/A233D
mutation M317 T220L/D300N
mutation M318 P221L/A233D
mutation M319 P221L/Y328F
mutation M320 F224A/A233D
mutation M321 G226A/Y328F
mutation M322 G226F/A233D
mutation M323 G226F/Y328F
mutation M324 I228K/Y328F
mutation M325 A233D/K235D
mutation M326 A233D/Y328F
mutation M327 R276A/Y328F
mutation M328 Y328F/Y339F
mutation M329 A27T/Y81A/S84D
mutation M330 P70T/A72E/I157L
mutation M331 P70T/G77S/I157L
mutation M332 P70T/E80D/F88E
mutation M333 P70T/Y81A/I157L
mutation M334 P70T/S84D/I157L
mutation M335 P70T/F88E/Y328F
mutation M336 P70T/F113W/I157L
mutation M337 P70T/I157L/A204S
mutation M338 P70T/I157L/T210L
mutation M339 P70T/I157L/A233D
mutation M340 P70T/I157L/Y328F
mutation M341 P70T/I157L/V439P
mutation M342 P70T/I157L/I440F
mutation M343 P70T/G161A/T210L
mutation M344 P70T/G161A/Y328F
mutation M345 P70T/A184G/W187F
mutation M346 P70T/A204S/Y328F
mutation M347 P70T/F211W/Y328F
mutation M348 P70V/A72E/I157L
mutation M349 A72E/S74T/I157L
mutation M350 A72E/G77S/Y328F
mutation M351 A72E/E80D/Y328F
mutation M352 A72E/Y81H/I157L
mutation M353 A72E/K83P/I157L
mutation M354 A72E/S84D/Y328F
mutation M355 A72E/L85P/I157L
mutation M356 A72E/F113W/I157L
mutation M357 A72E/F113W/Y328F
mutation M358 A72E/F113Y/I157L
mutation M359 A72E/D115Q/I157L
mutation M360 A72E/I157L/G161A
mutation M361 A72E/I157L/F162L
mutation M362 A72E/I157L/A184G
mutation M363 A72E/I157L/F200A
mutation M364 A72E/I157L/A204S
mutation M365 A72E/I157L/A204T
mutation M366 A72E/I157L/T210L
mutation M367 A72E/I157L/F211W
mutation M368 A72E/I157L/G226A
mutation M369 A72E/I157L/A233D
mutation M370 A72E/I157L/Y328F
mutation M371 A72E/I157L/L340V
mutation M372 A72E/I157L/V439P
mutation M373 A72E/G161A/Y328F
mutation M374 A72E/F162L/Y328F
mutation M375 A72E/A184G/Y328F
mutation M376 A72E/W187F/Y328F
mutation M377 A72E/F200A/Y328F
mutation M378 A72E/A204S/Y328F
mutation M379 A72E/T210L/Y328F
mutation M380 A72E/I228K/Y328F
mutation M381 A72E/A233D/Y328F
mutation M382 A72E/Y328F/Y159N
mutation M383 A72E/Y328F/F211W
mutation M384 A72E/Y328F/F211Y
mutation M385 A72E/Y328F/G226A
mutation M386 A72V/Y81A/Y328F
mutation M387 A72V/G161A/Y328F
mutation M388 G77M/I157L/T210L
mutation M389 G77P/I157L/F162L
mutation M390 G77P/I157L/A184G
mutation M391 G77P/F211W/Y328F
mutation M392 G77S/Y81A/Y328F
mutation M393 G77S/S84D/I157L
mutation M394 G77S/F88E/I157L
mutation M395 G77S/F113W/I157L
mutation M396 G77S/F113Y/I157L
mutation M397 G77S/D115Q/I157L
mutation M398 G77S/I157L/G161A
mutation M399 G77S/I157L/F200A
mutation M400 G77S/I157L/A204S
mutation M401 G77S/I157L/T210L
mutation M402 G77S/I157L/F211W
mutation M403 G77S/I157L/G226A
mutation M404 G77S/I157L/A233D
mutation M405 G77S/I157L/L340V
mutation M406 G77S/I157L/V439P
mutation M407 G77S/G161A/Y328F
mutation M408 E80D/Y81A/Y328F
mutation M409 Y81A/S84D/Y328F
mutation M410 Y81A/F113W/Y328F
mutation M411 Y81A/I157L/T210L
mutation M412 Y81A/I157L/Y328F
mutation M413 Y81A/G161A/Y328F
mutation M414 Y81A/F162L/Y328F
mutation M415 Y81A/A184G/Y328F
mutation M416 Y81A/W187F/Y328F
mutation M417 Y81A/A204S/Y328F
mutation M418 Y81A/T210L/Y328F
mutation M419 Y81A/I228K/Y328F
mutation M420 Y81A/A233D/Y328F
mutation M421 Y81A/Y328F/Y159N
mutation M422 Y81A/Y328F/Y159S
mutation M423 Y81A/Y328F/F211W
mutation M424 Y81A/Y328F/F211Y
mutation M425 Y81A/Y328F/G226A
mutation M426 Y81A/Y328F/R276A
mutation M427 K83P/I157L/A184G
mutation M428 K83P/I157L/T210L
mutation M429 K83P/F211W/Y328F
mutation M430 S84D/F113W/I157L
mutation M431 S84D/I157L/T210L
mutation M432 F88E/I157L/F162L
mutation M433 F88E/I157L/A184G
mutation M434 F88E/I157L/F200A
mutation M435 F88E/I157L/T210L
mutation M436 F88E/I157L/Y328F
mutation M437 F88E/I157L/Y328Q
mutation M438 F88E/I157L/L340V
mutation M439 F88E/T210L/Y328F
mutation M440 F88E/F211W/Y328F
mutation M441 F113W/I157L/G161A
mutation M441 F113W/I157L/A184G
mutation M442 F113W/I157L/A184G
mutation M443 F113W/I157L/W187F
mutation M444 F113W/I157L/F200A
mutation M445 F113W/I157L/A204S
mutation M446 F113W/I157L/A204T
mutation M447 F113W/I157L/T210L
mutation M448 F113W/I157L/F211W
mutation M449 F113W/I157L/G226A
mutation M450 F113W/I157L/A233D
mutation M451 F113W/I157L/Y328F
mutation M452 F113W/I157L/L340V
mutation M453 F113W/I157L/V439P
mutation M454 F113W/G161A/T210L
mutation M455 F113W/G161A/Y328F
mutation M456 F113W/A184G/W187F
mutation M457 F113Y/I157L/T210L
mutation M458 F113Y/I157L/Y328F
mutation M459 F113Y/G161A/T210L
mutation M460 D115Q/I157L/T210L
mutation M461 D115Q/I157L/Y328F
mutation M462 I157L/Y159N/T210L
mutation M463 I157L/Y159N/Y328F
mutation M464 I157L/G161A/W187F
mutation M465 I157L/G161A/F200A
mutation M466 I157L/G161A/A204S
mutation M467 I157L/G161A/T210L
mutation M468 I157L/G161A/A233D
mutation M469 I157L/G161A/Y328F
mutation M470 I157L/F162L/A184G
mutation M471 I157L/F162L/T210L
mutation M472 I157L/F162L/L340V
mutation M473 I157L/A184G/W187F
mutation M474 I157L/A184G/F200A
mutation M475 I157L/A184G/A204T
mutation M476 I157L/A184G/T210L
mutation M477 I157L/A184G/F211W
mutation M478 I157L/A184G/L340V
mutation M479 I157L/W187F/T210L
mutation M480 I157L/W187F/Y328F
mutation M481 I157L/F200A/T210L
mutation M482 I157L/F200A/Y328F
mutation M483 I157L/A204S/T210L
mutation M484 I157L/A204S/Y328F
mutation M485 I157L/A204T/T210L
mutation M486 I157L/A204T/Y328F
mutation M487 I157L/T210L/F211W
mutation M488 I157L/T210L/G212A
mutation M489 I157L/T210L/G226A
mutation M490 I157L/T210L/A233D
mutation M491 I157L/T210L/Y328F
mutation M492 I157L/T210L/L340V
mutation M493 I157L/T210L/V439P
mutation M494 I157L/F211W/Y328F
mutation M495 I157L/G226A/Y328F
mutation M496 I157L/A233D/Y328F
mutation M497 I157L/Y328F/L340V
mutation M498 I157L/Y328F/V439P
mutation M499 Y159N/F211W/Y328F
mutation M500 G161A/A184G/W187F
mutation M501 G161A/T210L/Y328F
mutation M502 G161A/F211W/Y328F
mutation M503 A182G/P183A/Y328F
mutation M504 A182S/P183A/Y328F
mutation M505 A184G/W187F/F200A
mutation M506 A184G/W187F/A204S
mutation M507 A184G/W187F/F211W
mutation M508 A184G/W187F/I228K
mutation M509 A184G/W187F/A233D
mutation M510 F200A/F211W/Y328F
mutation M511 A204S/F211W/Y328F
mutation M512 A204T/F211W/Y328F
mutation M513 F211W/Y328F/L340V
mutation M514 P70T/A72E/I157L/Y328F
mutation M515 P70T/A72E/T210L/Y328F
mutation M516 P70T/G77M/I157L/Y328F
mutation M517 P70T/Y81A/I157L/T210L
mutation M518 P70T/Y81A/I157L/Y328F
mutation M519 P70T/S84D/I157L/Y328F
mutation M520 P70T/F88E/I157L/Y328F
mutation M521 P70T/F88E/T210L/Y328F
mutation M522 P70T/F113W/I157L/T210L
mutation M523 P70T/F113W/G161A/Y328F
mutation M524 P70T/F113Y/I157L/Y328F
mutation M525 P70T/D115Q/I157L/T210L
mutation M526 P70T/D115Q/I157L/Y328F
mutation M527 P70T/I157L/G161A/T210L
mutation M528 P70T/I157L/A184G/W187F
mutation M529 P70T/I157L/A184G/T210L
mutation M530 P70T/I157L/W187F/T210L
mutation M531 P70T/I157L/W187F/Y328F
mutation M532 P70T/I157L/A204T/T210L
mutation M533 P70T/I157L/A204T/Y328F
mutation M534 P70T/I157L/A204T/T210L
mutation M535 P70T/I157L/T210L/F211W
mutation M536 P70T/I157L/T210L/G226A
mutation M537 P70T/I157L/T210L/A233D
mutation M538 P70T/I157L/T210L/Y328F
mutation M539 P70T/I157L/T210L/L340V
mutation M540 P70T/I157L/T210L/V439P
mutation M541 P70T/I157L/Y328F/V439P
mutation M542 P70T/G161A/T210L/Y328F
mutation M543 P70T/G161A/A233D/Y328F
mutation M544 A72E/S74T/I157L/Y328F
mutation M545 A72E/G77S/F113W/I157L
mutation M546 A72E/Y81H/I157L/Y328F
mutation M547 A72E/K83P/I157L/Y328F
mutation M548 A72E/F88E/F113W/I157L
mutation M549 A72E/F88E/I157L/Y328F
mutation M550 A72E/F88E/G161A/Y328F
mutation M551 A72E/F113W/I157L/Y328F
mutation M552 A72E/F113W/G161A/Y328F
mutation M553 A72E/F113Y/I157L/Y328F
mutation M554 A72E/F113Y/G161A/Y328F
mutation M555 A72E/F113Y/G226A/Y328F
mutation M556 A72E/I157L/G161A/Y328F
mutation M557 A72E/I157L/F162L/Y328F
mutation M558 A72E/I157L/A184G/Y328F
mutation M559 A72E/I157L/F200A/Y328F
mutation M560 A72E/I157L/A204T/Y328F
mutation M561 A72E/I157L/F211W/Y328F
mutation M562 A72E/I157L/F211Y/Y328F
mutation M563 A72E/I157L/A233D/Y328F
mutation M564 A72E/I157L/Y328F/L340V
mutation M565 A72E/G161A/A204T/Y328F
mutation M566 A72E/G161A/T210L/Y328F
mutation M567 A72E/G161A/F211W/Y328F
mutation M568 A72E/G161A/F211Y/Y328F
mutation M569 A72E/G161A/A233D/Y328F
mutation M570 A72E/G161A/Y328F/L340V
mutation M571 A72E/A184G/W187F/Y328F
mutation M572 A72E/T210L/Y328F/L340V
mutation M573 A72V/I157L/W187F/Y328F
mutation M574 G77P/I157L/T210L/Y328F
mutation M575 Y81A/S84D/I157L/Y328F
mutation M576 Y81A/F88E/I157L/Y328F
mutation M577 Y81A/F113W/I157L/Y328F
mutation M578 Y81A/I157L/G161A/Y328F
mutation M579 Y81A/I157L/W187F/Y328F
mutation M580 Y81A/I157L/A204S/Y328F
mutation M581 Y81A/I157L/T210L/Y328F
mutation M582 Y81A/I157L/A233D/Y328F
mutation M583 Y81A/I157L/Y328F/V439P
mutation M584 Y81A/A184G/W187F/Y328F
mutation M585 F88E/I157L/T210L/Y328F
mutation M586 F88E/I157L/A233D/Y328F
mutation M587 F113W/I157L/A204T/T210L
mutation M588 F113W/I157L/T210L/Y328F
mutation M589 I157L/G161A/A184G/W187F
mutation M590 I157L/G161A/T210L/Y328F
mutation M591 I157L/A184G/W187F/T210L
mutation M592 I157L/A204S/T210L/Y328F
mutation M593 I157L/A204T/T210L/Y328F
mutation M594 I157L/T210L/A233D/Y328F
mutation M595 G161A/A184G/W187F/Y328F
mutation M596 P70T/A72E/S84D/I157L/Y328F
mutation M597 P70T/A72E/A204S/I157L/Y328F
mutation M598 P70T/A72E/T210L/I157L/Y328F
mutation M599 P70T/A72E/G226A/I157L/Y328F
mutation M600 P70T/A72E/A233D/I157L/Y328F
mutation M601 P70T/Y81A/I157L/T210L/Y328F
mutation M602 P70T/Y81A/I157L/A233D/Y328F
mutation M603 P70T/Y81A/I157L/T210L/Y328F
mutation M604 P70T/Y81A/A233D/I157L/Y328F
mutation M605 P70T/S84D/I157L/T210L/Y328F
mutation M606 P70T/F113W/I157L/T210L/Y328F
mutation M607 P70T/I157L/A184G/W187F/A233D
mutation M608 P70T/I157L/W187F/T210L/Y328F
mutation M609 P70T/I157L/A204S/T210L/Y328F
mutation M610 P70T/G161A/A184G/W187F/Y328F
mutation M611 P70V/A72E/F113Y/I157L/Y328F
mutation M612 P70V/A72E/I157L/F211W/Y328F
mutation M613 A72E/S74T/F113Y/I157L/Y328F
mutation M614 A72E/S74T/I157L/F211W/Y328F
mutation M615 A72E/Y81H/I157L/F211W/Y328F
mutation M616 A72E/K83P/F113Y/I157L/Y328F
mutation M617 A72E/W17F/F113Y/I157L/Y328F
mutation M618 A72E/F113Y/D115Q/I157L/Y328F
mutation M619 A72E/F113Y/I157L/Y328F/L340V
mutation M620 A72E/F113Y/I157L/Y328F/V439P
mutation M621 A72E/F113Y/G161A/I157L/Y328F
mutation M622 A72E/F113Y/A204S/I157L/Y328F
mutation M623 A72E/F113Y/A204T/I157L/Y328F
mutation M624 A72E/F113Y/T210L/I157L/Y328F
mutation M625 A72E/F113Y/A233D/I157L/Y328F
mutation M626 A72E/I157L/G161A/F162L/Y328F
mutation M627 A72E/I157L/W187F/F211W/Y328F
mutation M628 A72E/I157L/A204S/F211W/Y328F
mutation M629 A72E/I157L/A204T/F211W/Y328F
mutation M630 A72E/I157L/F211W/Y328F/L340V
mutation M631 A72E/I157L/F211W/Y328F/V439P
mutation M632 A72E/I157L/G226A/F211W/Y328F
mutation M633 A72E/I157L/A233D/F211W/Y328F
mutation M634 Y81A/S84D/I157L/T210L/Y328F
mutation M635 Y81A/I157L/A184G/W187F/Y328F
mutation M636 Y81A/I157L/A184G/W187F/T210L
mutation M637 Y81A/I157L/A233D/T210L/Y328F
mutation M638 F88E/I157L/A184G/W187F/T210L
mutation M639 F113Y/I157L/Y159N/F211W/Y328F
mutation M640 I157L/A184G/W187F/T210L/Y328F
mutation M641 P70T/I157L/A184G/W187F/T210L/Y328F
mutation M642 Y81A/I157L/A184G/W187F/T210L/Y328F.

56. The mutant protein according to claim 55 wherein, in said amino acid sequence comprising one or more mutations selected from any of the mutations M1 to M642, said amino acid sequence further comprises at other than the mutated position(s) one or several amino acid mutations selected from the group consisting of substitutions, deletions, insertions, additions and inversions, said mutant protein having a peptide-synthesizing activity.

57. The mutant protein according to any one of claims 55 to 56 comprising at least the mutation M241.

58. The mutant protein according to any one of claims 55 to 57 comprising at least the mutation M340.

59. The mutant protein according to any one of claims 55 to 58 comprising at least the mutation M412.

60. The mutant protein according to any one of claims 55 to 59 comprising at least the mutation M491.

61. The mutant protein according to any one of claims 55 to 60 comprising at least the mutation M496.

62. The mutant protein according to any one of claims 55 to 61 comprising at least the mutation M581.

63. The mutant protein according to any one of claims 55 to 62 comprising at least the mutation M582.

64. The mutant protein according to any one of claims 55 to 63 comprising at least the mutation M594.

65. A polynucleotide encoding an amino acid sequence of the mutant protein according to any one of claims 18 to 64.

66. A recombinant polynucleotide comprising the polynucleotide according to claim 65.

67. A transformed microorganism comprising the recombinant polynucleotide according to claim 66.

68. A method for producing a mutant protein comprising culturing the transformed microorganism according to claim 67 in a medium, to accumulate the mutant protein in the medium and/or the transformed microorganism.

69. A method for producing a peptide comprising performing a peptide-synthesizing reaction in the presence of the mutant protein according to any one of claim 18 to 64.

70. A method for producing a peptide comprising culturing the transformed microorganism according to claim 67 in a medium to accumulate the mutant protein in the medium and/or the transformed microorganism for performing a peptide-synthesizing reaction.

71. A method for producing α-L-aspartyl-L-phenylalanine-β-ester comprising reacting L-aspartic acid-α,β-diester and L-phenylalanine in the presence of the mutant protein according to any one of claims 18 to 64.

72. A method for producing α-L-aspartyl-L-phenylalanine-β-ester comprising culturing the transformed microorganism according to claim 67 in a medium to accumulate the mutant protein in the medium and/or the transformed microorganism for performing a reaction of L-aspartic acid-α,β-diester and L-phenylalanine.
